(19) Europäisches Patentamt
European Patent Office
Office européen des brevets

(11) EP 3 680 251 A1

(12) EUROPEAN PATENT APPLICATION

(43) Date of publication:
15.07.2020 Bulletin 2020/29

(51) Int Cl.:
*C07K 16/00* (2006.01)

(21) Application number: 19197872.5

(22) Date of filing: 28.09.2012

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 30.09.2011 JP 2011217498
24.02.2012 PCT/JP2012/054624
24.08.2012 JP 2012185866

(62) Document number(s) of the earlier application(s) in accordance with Art. 76 EPC:
12835590.6 / 2 762 166

(71) Applicant: Chugai Seiyaku Kabushiki Kaisha
Kita-ku
Tokyo 115-8543 (JP)

(72) Inventors:
- IGAWA, Tomoyuki
Shizuoka 412-8513 (JP)
- MAEDA, Atsuhiko
Shizuoka 412-8513 (JP)
- HARAYA, Kenta
Shizuoka 412-8513 (JP)
- IWAYANAGI, Yuki
Shizuoka 412-8513 (JP)
- TACHIBANA, Tatsuhiko
Shizuoka 412-8513 (JP)

(74) Representative: Vossius & Partner
Patentanwälte Rechtsanwälte mbB
Siebertstrasse 3
81675 München (DE)

Remarks:
•This application was filed on 17.09.2019 as a divisional application to the application mentioned under INID code 62.
•Claims filed after the date of receipt of the divisional application (Rule 68(4) EPC).

# (54) ANTIGEN-BINDING MOLECULES FOR PROMOTING ELIMINATION OF ANTIGENS

(57) The present inventors created antigen-binding molecules containing an antigen-binding domain and an Fcγ-receptor-binding domain, wherein the molecules have human-FcRn-binding activity in an acidic pH range condition, the antigen-binding domain changes the antigen-binding activity of the antigen-binding molecules depending on the ion-concentration condition, and the Fcγ receptor-binding domain has higher binding activity to the Fcγ receptor in a neutral pH range condition than an Fc region of a native human IgG in which the sugar chain bound at position 297 (EU numbering) is a fucose-containing sugar chain.

EP 3 680 251 A1

SEQUENCE LISTING

<110> CHUGAI SEIYAKU KABUSHIKI KAISHA

<120> Antigen-binding molecule for promoting elimination of antigens

<130> X1534 EP/1 S3

<140>
<141>

<150> JP 2011-217498
<151> 2011-09-30

<150> PCT/JP2012/054624
<151> 2012-02-24

<150> JP 2012-185866
<151> 2012-08-24

<160> 164

<170> PatentIn version 3.4

<210> 1
<211> 468
<212> PRT
<213> Homo sapiens

<400> 1

```
Met Leu Ala Val Gly Cys Ala Leu Leu Ala Ala Leu Leu Ala Ala Pro
1               5                   10                  15

Gly Ala Ala Leu Ala Pro Arg Arg Cys Pro Ala Gln Glu Val Ala Arg
            20                  25                  30

Gly Val Leu Thr Ser Leu Pro Gly Asp Ser Val Thr Leu Thr Cys Pro
        35                  40                  45

Gly Val Glu Pro Glu Asp Asn Ala Thr Val His Trp Val Leu Arg Lys
    50                  55                  60

Pro Ala Ala Gly Ser His Pro Ser Arg Trp Ala Gly Met Gly Arg Arg
65                  70                  75                  80

Leu Leu Leu Arg Ser Val Gln Leu His Asp Ser Gly Asn Tyr Ser Cys
                85                  90                  95

Tyr Arg Ala Gly Arg Pro Ala Gly Thr Val His Leu Leu Val Asp Val
            100                 105                 110

Pro Pro Glu Glu Pro Gln Leu Ser Cys Phe Arg Lys Ser Pro Leu Ser
        115                 120                 125
```

Asn Val Val Cys Glu Trp Gly Pro Arg Ser Thr Pro Ser Leu Thr Thr
130 135 140

Lys Ala Val Leu Leu Val Arg Lys Phe Gln Asn Ser Pro Ala Glu Asp
145 150 155 160

Phe Gln Glu Pro Cys Gln Tyr Ser Gln Glu Ser Gln Lys Phe Ser Cys
165 170 175

Gln Leu Ala Val Pro Glu Gly Asp Ser Ser Phe Tyr Ile Val Ser Met
180 185 190

Cys Val Ala Ser Ser Val Gly Ser Lys Phe Ser Lys Thr Gln Thr Phe
195 200 205

Gln Gly Cys Gly Ile Leu Gln Pro Asp Pro Pro Ala Asn Ile Thr Val
210 215 220

Thr Ala Val Ala Arg Asn Pro Arg Trp Leu Ser Val Thr Trp Gln Asp
225 230 235 240

Pro His Ser Trp Asn Ser Ser Phe Tyr Arg Leu Arg Phe Glu Leu Arg
245 250 255

Tyr Arg Ala Glu Arg Ser Lys Thr Phe Thr Thr Trp Met Val Lys Asp
260 265 270

Leu Gln His His Cys Val Ile His Asp Ala Trp Ser Gly Leu Arg His
275 280 285

Val Val Gln Leu Arg Ala Gln Glu Glu Phe Gly Gln Gly Glu Trp Ser
290 295 300

Glu Trp Ser Pro Glu Ala Met Gly Thr Pro Trp Thr Glu Ser Arg Ser
305 310 315 320

Pro Pro Ala Glu Asn Glu Val Ser Thr Pro Met Gln Ala Leu Thr Thr
325 330 335

Asn Lys Asp Asp Asp Asn Ile Leu Phe Arg Asp Ser Ala Asn Ala Thr
340 345 350

Ser Leu Pro Val Gln Asp Ser Ser Ser Val Pro Leu Pro Thr Phe Leu
355 360 365

Val Ala Gly Gly Ser Leu Ala Phe Gly Thr Leu Leu Cys Ile Ala Ile
370 375 380

Val Leu Arg Phe Lys Lys Thr Trp Lys Leu Arg Ala Leu Lys Glu Gly
385 390 395 400

Lys Thr Ser Met His Pro Pro Tyr Ser Leu Gly Gln Leu Val Pro Glu
405 410 415

Arg Pro Arg Pro Thr Pro Val Leu Val Pro Leu Ile Ser Pro Pro Val
420 425 430

Ser Pro Ser Ser Leu Gly Ser Asp Asn Thr Ser Ser His Asn Arg Pro
435 440 445

Asp Ala Arg Asp Pro Arg Ser Pro Tyr Asp Ile Ser Asn Thr Asp Tyr
450 455 460

Phe Phe Pro Arg
465

<210> 2
<211> 1407
<212> DNA
<213> Homo sapiens

<400> 2
atgctggccg tcggctgcgc gctgctggct gccctgctgg ccgcgccggg agcggcgctg 60

gccccaaggc gctgccctgc gcaggaggtg gcgagaggcg tgctgaccag tctgccagga 120

gacagcgtga ctctgacctg cccgggggta gagccggaag acaatgccac tgttcactgg 180

gtgctcagga agccggctgc aggctcccac cccagcagat gggctggcat gggaaggagg 240

ctgctgctga ggtcggtgca gctccacgac tctggaaact attcatgcta ccgggccggc 300

cgcccagctg ggactgtgca cttgctggtg gatgttcccc ccgaggagcc ccagctctcc 360

tgcttccgga agagcccct cagcaatgtt gtttgtgagt ggggtcctcg gagcacccca 420

tccctgacga caaaggctgt gctcttggtg aggaagtttc agaacagtcc ggccgaagac 480

ttccaggagc cgtgccagta ttcccaggag tcccagaagt tctcctgcca gttagcagtc 540

ccggagggag acagctcttt ctacatagtg tccatgtgcg tcgccagtag tgtcgggagc 600

aagttcagca aaactcaaac ctttcagggt tgtggaatct tgcagcctga tccgcctgcc 660

aacatcacag tcactgccgt ggccagaaac ccccgctggc tcagtgtcac ctggcaagac 720

ccccactcct ggaactcatc tttctacaga ctacggtttg agctcagata tcgggctgaa 780

cggtcaaaga cattcacaac atggatggtc aaggacctcc agcatcactg tgtcatccac 840

gacgcctgga gcggcctgag gcacgtggtg cagcttcgtg cccaggagga gttcgggcaa 900

ggcgagtgga gcgagtggag cccggaggcc atgggcacgc cttggacaga atccaggagt 960

cctccagctg agaacgaggt gtccaccccc atgcaggcac ttactactaa taaagacgat 1020

gataatattc tcttcagaga ttctgcaaat gcgacaagcc tcccagtgca agattcttct 1080

tcagtaccac tgcccacatt cctggttgct ggagggagcc tggccttcgg aacgctcctc 1140

tgcattgcca ttgttctgag gttcaagaag acgtggaagc tgcgggctct gaaggaaggc 1200

aagacaagca tgcatccgcc gtactctttg gggcagctgg tcccggagag gcctcgaccc 1260

accccagtgc ttgttcctct catctcccca ccggtgtccc ccagcagcct ggggtctgac 1320

aatacctcga gccacaaccg accagatgcc agggacccac ggagccctta tgacatcagc 1380

aatacagact acttcttccc cagatag 1407

<210> 3
<211> 19
<212> PRT
<213> Artificial

<220>
<223> An artificially synthesized peptide sequence

<400> 3

Met Gly Trp Ser Cys Ile Ile Leu Phe Leu Val Ala Thr Ala Thr Gly
1 5 10 15

Val His Ser

<210> 4
<211> 214
<212> PRT
<213> Artificial

<220>
<223> An artificially synthesized peptide sequence

<400> 4

Glu Thr Thr Leu Thr Gln Ser Pro Ala Phe Met Ser Ala Thr Pro Gly
1 5 10 15

Asp Lys Val Asn Ile Ser Cys Lys Ala Ser Gln Asp Ile Asp Asp Asp
20 25 30

Met Asn Trp Tyr Gln Gln Lys Pro Gly Glu Ala Ala Ile Phe Ile Ile
35 40 45

Gln Glu Ala Thr Thr Leu Val Pro Gly Ile Ser Pro Arg Phe Ser Gly
50 55 60

Ser Gly Tyr Gly Thr Asp Phe Thr Leu Thr Ile Asn Asn Ile Glu Ser
65 70 75 80

Glu Asp Ala Ala Tyr Tyr Phe Cys Leu Gln His Asp Asn Phe Pro Tyr
85 90 95

Thr Phe Gly Gln Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 5
<211> 107
<212> PRT
<213> Artificial

<220>
<223> An artificially synthesized peptide sequence

<400> 5

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Ser Tyr
20 25 30

Leu Asn Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Ser Tyr Ser Thr Pro Phe
85 90 95

Thr Phe Gly Pro Gly Thr Lys Val Asp Ile Lys
100 105

<210> 6
<211> 112
<212> PRT
<213> Artificial

<220>
<223> An artificially synthesized peptide sequence

<400> 6

Asp Ile Val Met Thr Gln Ser Pro Leu Ser Leu Pro Val Thr Pro Gly
1 5 10 15

Glu Pro Ala Ser Ile Ser Cys Arg Ser Ser Gln Ser Leu Leu His Ser
20 25 30

Asn Gly Asp Asn Tyr Leu Asp Trp Tyr Leu Gln Lys Pro Gly Gln Ser
35 40 45

Pro Gln Leu Leu Ile Tyr Leu Gly Ser Asn Arg Ala Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Met Gln Val
85 90 95

Leu Arg Asn Pro Leu Thr Phe Gly Gly Gly Thr Lys Val Glu Ile Gln
100 105 110

<210> 7
<211> 107
<212> PRT
<213> Artificial

<220>
<223> An artificially synthesized peptide sequence

<400> 7

Glu Ile Val Met Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr
20 25 30

Leu Ala Trp Tyr Gln Gln Lys Pro Gly Gln Ala Pro Arg Leu Leu Ile
35 40 45

Tyr Asp Ala Ser Asn Arg Ala Thr Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Arg Ser Asn Trp Pro Pro
85 90 95

Thr Phe Gly Gln Gly Thr Lys Val Glu Ile Lys
100 105

<210> 8
<211> 112
<212> PRT
<213> Artificial

<220>
<223> An artificially synthesized peptide sequence

<400> 8

Asp Ile Val Met Thr Gln Ser Pro Glu Ser Leu Val Leu Ser Leu Gly
1 5 10 15

Gly Thr Ala Thr Ile Asn Cys Arg Ser Ser Gln Ser Val Leu Tyr Ser
20 25 30

Ser Asn Asn Lys Asn Tyr Leu Thr Trp Tyr Gln Gln Lys Pro Gly Gln
35 40 45

Pro Pro Thr Leu Leu Phe Ser Trp Ala Ser Ile Arg Asp Ser Gly Val
50 55 60

Pro Asp Arg Phe Ser Ala Ser Gly Ser Gly Thr Glu Phe Thr Leu Thr
65 70 75 80

Ile Ser Asp Leu Gln Ala Glu Asp Ala Ala Val Tyr Tyr Cys Gln Gln
85 90 95

Tyr Tyr Arg Ala Pro Ser Phe Gly Gln Gly Thr Lys Leu Gln Ile Lys
100 105 110

<210> 9
<211> 121
<212> PRT
<213> Artificial

<220>
<223> An artificially synthesized peptide sequence

<400> 9

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Tyr Met His Trp Val Arg Gln Ala Pro Gly Gln Gly Leu Glu Trp Met
35 40 45

Gly Ile Ile Asn Pro Ser Gly Gly Ser Thr Ser Tyr Ala Gln Lys Phe
50 55 60

Gln Gly Arg Val Thr Met Thr Arg Asp Thr Ser Thr Ser Thr Val Tyr
65 70 75 80

Met Glu Leu Ser Ser Leu Arg Ser Glu Asp Thr Ala Val Tyr Tyr Cys
85 90 95

Ala Arg Asp Asp Pro Gly Gly Gly Glu Tyr Tyr Phe Asp Tyr Trp Gly
100 105 110

Gln Gly Thr Leu Val Thr Val Ser Ser
115 120

<210> 10
<211> 126
<212> PRT
<213> Artificial

<220>
<223> An artificially synthesized peptide sequence

<400> 10

Glu Val Gln Leu Val Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Glu Met Asn Trp Val Arg Gln Ala Pro Gly Lys Gly Leu Glu Trp Val
35 40 45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Ala Pro Tyr Tyr Tyr Asp Ser Ser Gly Tyr Thr Asp Ala
            100                 105                 110

Phe Asp Ile Trp Gly Gln Gly Thr Met Val Thr Val Ser Ser
        115                 120                 125

<210>  11
<211>  330
<212>  PRT
<213>  Homo sapiens

<400>  11

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 12
<211> 326
<212> PRT
<213> Homo sapiens

<400> 12

Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Asn Phe Gly Thr Gln Thr
65 70 75 80

Tyr Thr Cys Asn Val Asp His Lys Pro Ser Asn Thr Lys Val Asp Lys
85 90 95

Thr Val Glu Arg Lys Cys Cys Val Glu Cys Pro Pro Cys Pro Ala Pro
100 105 110

Pro Val Ala Gly Pro Ser Val Phe Leu Phe Pro Pro Lys Pro Lys Asp
115 120 125

Thr Leu Met Ile Ser Arg Thr Pro Glu Val Thr Cys Val Val Val Asp
130 135 140

Val Ser His Glu Asp Pro Glu Val Gln Phe Asn Trp Tyr Val Asp Gly
145 150 155 160

Val Glu Val His Asn Ala Lys Thr Lys Pro Arg Glu Glu Gln Phe Asn
165 170 175

Ser Thr Phe Arg Val Val Ser Val Leu Thr Val Val His Gln Asp Trp
180 185 190

Leu Asn Gly Lys Glu Tyr Lys Cys Lys Val Ser Asn Lys Gly Leu Pro
195 200 205

Ala Pro Ile Glu Lys Thr Ile Ser Lys Thr Lys Gly Gln Pro Arg Glu
210 215 220

Pro Gln Val Tyr Thr Leu Pro Pro Ser Arg Glu Glu Met Thr Lys Asn
225 230 235 240

Gln Val Ser Leu Thr Cys Leu Val Lys Gly Phe Tyr Pro Ser Asp Ile
245 250 255

Ala Val Glu Trp Glu Ser Asn Gly Gln Pro Glu Asn Asn Tyr Lys Thr
260 265 270

Thr Pro Pro Met Leu Asp Ser Asp Gly Ser Phe Phe Leu Tyr Ser Lys

275 280 285

Leu Thr Val Asp Lys Ser Arg Trp Gln Gln Gly Asn Val Phe Ser Cys
290 295 300

Ser Val Met His Glu Ala Leu His Asn His Tyr Thr Gln Lys Ser Leu
305 310 315 320

Ser Leu Ser Pro Gly Lys
325

<210> 13
<211> 377
<212> PRT
<213> Homo sapiens

<400> 13

Ala Ser Thr Lys Gly Pro Ser Val Phe Pro Leu Ala Pro Cys Ser Arg
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Thr Cys Asn Val Asn His Lys Pro Ser Asn Thr Lys Val Asp Lys
85 90 95

Arg Val Glu Leu Lys Thr Pro Leu Gly Asp Thr Thr His Thr Cys Pro
100 105 110

Arg Cys Pro Glu Pro Lys Ser Cys Asp Thr Pro Pro Pro Cys Pro Arg
115 120 125

Cys Pro Glu Pro Lys Ser Cys Asp Thr Pro Pro Pro Cys Pro Arg Cys
130 135 140

Pro Glu Pro Lys Ser Cys Asp Thr Pro Pro Pro Cys Pro Arg Cys Pro
145 150 155 160

Ala Pro Glu Leu Leu Gly Gly Pro Ser Val Phe Leu Phe Pro Pro Lys

165 170 175

Pro Lys Asp Thr Leu Met Ile Ser Arg Thr Pro Glu Val Thr Cys Val
180 185 190

Val Val Asp Val Ser His Glu Asp Pro Glu Val Gln Phe Lys Trp Tyr
195 200 205

Val Asp Gly Val Glu Val His Asn Ala Lys Thr Lys Pro Arg Glu Glu
210 215 220

Gln Tyr Asn Ser Thr Phe Arg Val Val Ser Val Leu Thr Val Leu His
225 230 235 240

Gln Asp Trp Leu Asn Gly Lys Glu Tyr Lys Cys Lys Val Ser Asn Lys
245 250 255

Ala Leu Pro Ala Pro Ile Glu Lys Thr Ile Ser Lys Thr Lys Gly Gln
260 265 270

Pro Arg Glu Pro Gln Val Tyr Thr Leu Pro Pro Ser Arg Glu Glu Met
275 280 285

Thr Lys Asn Gln Val Ser Leu Thr Cys Leu Val Lys Gly Phe Tyr Pro
290 295 300

Ser Asp Ile Ala Val Glu Trp Glu Ser Ser Gly Gln Pro Glu Asn Asn
305 310 315 320

Tyr Asn Thr Thr Pro Pro Met Leu Asp Ser Asp Gly Ser Phe Phe Leu
325 330 335

Tyr Ser Lys Leu Thr Val Asp Lys Ser Arg Trp Gln Gln Gly Asn Ile
340 345 350

Phe Ser Cys Ser Val Met His Glu Ala Leu His Asn Arg Phe Thr Gln
355 360 365

Lys Ser Leu Ser Leu Ser Pro Gly Lys
370 375

<210> 14
<211> 327
<212> PRT
<213> Homo sapiens

<400> 14

Ala Ser Thr Lys Gly Pro Ser Val Phe Pro Leu Ala Pro Cys Ser Arg

1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Lys Thr
65 70 75 80

Tyr Thr Cys Asn Val Asp His Lys Pro Ser Asn Thr Lys Val Asp Lys
85 90 95

Arg Val Glu Ser Lys Tyr Gly Pro Pro Cys Pro Ser Cys Pro Ala Pro
100 105 110

Glu Phe Leu Gly Gly Pro Ser Val Phe Leu Phe Pro Pro Lys Pro Lys
115 120 125

Asp Thr Leu Met Ile Ser Arg Thr Pro Glu Val Thr Cys Val Val Val
130 135 140

Asp Val Ser Gln Glu Asp Pro Glu Val Gln Phe Asn Trp Tyr Val Asp
145 150 155 160

Gly Val Glu Val His Asn Ala Lys Thr Lys Pro Arg Glu Glu Gln Phe
165 170 175

Asn Ser Thr Tyr Arg Val Val Ser Val Leu Thr Val Leu His Gln Asp
180 185 190

Trp Leu Asn Gly Lys Glu Tyr Lys Cys Lys Val Ser Asn Lys Gly Leu
195 200 205

Pro Ser Ser Ile Glu Lys Thr Ile Ser Lys Ala Lys Gly Gln Pro Arg
210 215 220

Glu Pro Gln Val Tyr Thr Leu Pro Pro Ser Gln Glu Glu Met Thr Lys
225 230 235 240

Asn Gln Val Ser Leu Thr Cys Leu Val Lys Gly Phe Tyr Pro Ser Asp
245 250 255

Ile Ala Val Glu Trp Glu Ser Asn Gly Gln Pro Glu Asn Asn Tyr Lys
260 265 270

Thr Thr Pro Pro Val Leu Asp Ser Asp Gly Ser Phe Phe Leu Tyr Ser
275 280 285

Arg Leu Thr Val Asp Lys Ser Arg Trp Gln Glu Gly Asn Val Phe Ser
290 295 300

Cys Ser Val Met His Glu Ala Leu His Asn His Tyr Thr Gln Lys Ser
305 310 315 320

Leu Ser Leu Ser Leu Gly Lys
325

<210> 15
<211> 107
<212> PRT
<213> Artificial

<220>
<223> An artificially synthesized peptide sequence

<400> 15

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Ile Ser Ser Tyr
20 25 30

Leu Asn Trp Tyr Gln Gln Lys Pro Gly Lys Ala Pro Lys Leu Leu Ile
35 40 45

Tyr Tyr Thr Ser Arg Leu His Ser Gly Val Pro Ser Arg Phe Ser Gly
50 55 60

Ser Gly Ser Gly Thr Asp Phe Thr Phe Thr Ile Ser Ser Leu Gln Pro
65 70 75 80

Glu Asp Ile Ala Thr Tyr Tyr Cys Gln Gln Gly Asn Thr Leu Pro Tyr
85 90 95

Thr Phe Gly Gln Gly Thr Lys Val Glu Ile Lys
100 105

<210> 16
<211> 1125
<212> DNA
<213> Homo sapiens

<400> 16
atgtggttct tgacaactct gctcctttgg gttccagttg atgggcaagt ggacaccaca 60

aaggcagtga tcactttgca gcctccatgg gtcagcgtgt tccaagagga aaccgtaacc 120

ttgcactgtg aggtgctcca tctgcctggg agcagctcta cacagtggtt tctcaatggc 180

acagccactc agacctcgac ccccagctac agaatcacct ctgccagtgt caatgacagt 240

ggtgaataca ggtgccagag aggtctctca gggcgaagtg accccataca gctggaaatc 300

cacagaggct ggctactact gcaggtctcc agcagagtct tcacggaagg agaacctctg 360

gccttgaggt gtcatgcgtg gaaggataag ctggtgtaca atgtgcttta ctatcgaaat 420

ggcaaagcct ttaagttttt ccactggaat tctaacctca ccattctgaa aaccaacata 480

agtcacaatg gcacctacca ttgctcaggc atgggaaagc atcgctacac atcagcagga 540

atatctgtca ctgtgaaaga gctatttcca gctccagtgc tgaatgcatc tgtgacatcc 600

ccactcctgg aggggaatct ggtcaccctg agctgtgaaa caaagttgct cttgcagagg 660

cctggtttgc agctttactt ctccttctac atgggcagca agaccctgcg aggcaggaac 720

acatcctctg aataccaaat actaactgct agaagagaag actctgggtt atactggtgc 780

gaggctgcca cagaggatgg aaatgtcctt aagcgcagcc ctgagttgga gcttcaagtg 840

cttggcctcc agttaccaac tcctgtctgg tttcatgtcc ttttctatct ggcagtggga 900

ataatgtttt tagtgaacac tgttctctgg gtgacaatac gtaaagaact gaaaagaaag 960

aaaaagtggg atttagaaat ctctttggat tctggtcatg agaagaaggt aatttccagc 1020

cttcaagaag acagacattt agaagaagag ctgaaatgtc aggaacaaaa agaagaacag 1080

ctgcaggaag gggtgcaccg gaaggagccc caggggccca cgtag 1125

<210> 17
<211> 374
<212> PRT
<213> Homo sapiens

<400> 17

Met Trp Phe Leu Thr Thr Leu Leu Leu Trp Val Pro Val Asp Gly Gln
1 5 10 15

Val Asp Thr Thr Lys Ala Val Ile Thr Leu Gln Pro Pro Trp Val Ser
20 25 30

Val Phe Gln Glu Glu Thr Val Thr Leu His Cys Glu Val Leu His Leu
35 40 45

Pro Gly Ser Ser Ser Thr Gln Trp Phe Leu Asn Gly Thr Ala Thr Gln
50 55 60

Thr Ser Thr Pro Ser Tyr Arg Ile Thr Ser Ala Ser Val Asn Asp Ser
65 70 75 80

Gly Glu Tyr Arg Cys Gln Arg Gly Leu Ser Gly Arg Ser Asp Pro Ile
85 90 95

Gln Leu Glu Ile His Arg Gly Trp Leu Leu Leu Gln Val Ser Ser Arg
100 105 110

Val Phe Thr Glu Gly Glu Pro Leu Ala Leu Arg Cys His Ala Trp Lys
115 120 125

Asp Lys Leu Val Tyr Asn Val Leu Tyr Tyr Arg Asn Gly Lys Ala Phe
130 135 140

Lys Phe Phe His Trp Asn Ser Asn Leu Thr Ile Leu Lys Thr Asn Ile
145 150 155 160

Ser His Asn Gly Thr Tyr His Cys Ser Gly Met Gly Lys His Arg Tyr
165 170 175

Thr Ser Ala Gly Ile Ser Val Thr Val Lys Glu Leu Phe Pro Ala Pro
180 185 190

Val Leu Asn Ala Ser Val Thr Ser Pro Leu Leu Glu Gly Asn Leu Val
195 200 205

Thr Leu Ser Cys Glu Thr Lys Leu Leu Leu Gln Arg Pro Gly Leu Gln
210 215 220

Leu Tyr Phe Ser Phe Tyr Met Gly Ser Lys Thr Leu Arg Gly Arg Asn
225 230 235 240

Thr Ser Ser Glu Tyr Gln Ile Leu Thr Ala Arg Arg Glu Asp Ser Gly
245 250 255

Leu Tyr Trp Cys Glu Ala Ala Thr Glu Asp Gly Asn Val Leu Lys Arg
260 265 270

Ser Pro Glu Leu Glu Leu Gln Val Leu Gly Leu Gln Leu Pro Thr Pro
275 280 285

Val Trp Phe His Val Leu Phe Tyr Leu Ala Val Gly Ile Met Phe Leu
290 295 300

Val Asn Thr Val Leu Trp Val Thr Ile Arg Lys Glu Leu Lys Arg Lys
305 310 315 320

Lys Lys Trp Asp Leu Glu Ile Ser Leu Asp Ser Gly His Glu Lys Lys
325 330 335

Val Ile Ser Ser Leu Gln Glu Asp Arg His Leu Glu Glu Glu Leu Lys
340 345 350

Cys Gln Glu Gln Lys Glu Glu Gln Leu Gln Glu Gly Val His Arg Lys
355 360 365

Glu Pro Gln Gly Ala Thr
370

<210> 18
<211> 951
<212> DNA
<213> Homo sapiens

<400> 18
atgactatgg agacccaaat gtctcagaat gtatgtccca gaaacctgtg gctgcttcaa 60

ccattgacag ttttgctgct gctggcttct gcagacagtc aagctgctcc cccaaaggct 120

gtgctgaaac ttgagccccc gtggatcaac gtgctccagg aggactctgt gactctgaca 180

tgccaggggg ctcgcagccc tgagagcgac tccattcagt ggttccacaa tgggaatctc 240

attcccaccc acacgcagcc cagctacagg ttcaaggcca acaacaatga cagcggggag 300

tacacgtgcc agactggcca gaccagcctc agcgaccctg tgcatctgac tgtgctttcc 360

gaatggctgg tgctccagac ccctcacctg gagttccagg agggagaaac catcatgctg 420

aggtgccaca gctggaagga caagcctctg gtcaaggtca cattcttcca gaatggaaaa 480

tcccagaaat tctcccattt ggatcccacc ttctccatcc cacaagcaaa ccacagtcac 540

agtggtgatt accactgcac aggaaacata ggctacacgc tgttctcatc caagcctgtg 600

accatcactg tccaagtgcc cagcatgggc agctcttcac caatgggggt cattgtggct 660

gtggtcattg cgactgctgt agcagccatt gttgctgctg tagtggcctt gatctactgc 720

aggaaaaagc ggatttcagc caattccact gatcctgtga aggctgccca atttgagcca 780

cctggacgtc aaatgattgc catcagaaag agacaacttg aagaaaccaa caatgactat 840

gaaacagctg acggcggcta catgactctg aaccccaggg cacctactga cgatgataaa 900

aacatctacc tgactcttcc tcccaacgac catgtcaaca gtaataacta a 951

<210> 19
<211> 316
<212> PRT
<213> Homo sapiens

<400> 19

Met Thr Met Glu Thr Gln Met Ser Gln Asn Val Cys Pro Arg Asn Leu
1               5                   10                  15

Trp Leu Leu Gln Pro Leu Thr Val Leu Leu Leu Leu Ala Ser Ala Asp
            20                  25                  30

Ser Gln Ala Ala Pro Pro Lys Ala Val Leu Lys Leu Glu Pro Pro Trp
        35                  40                  45

Ile Asn Val Leu Gln Glu Asp Ser Val Thr Leu Thr Cys Gln Gly Ala
    50                  55                  60

Arg Ser Pro Glu Ser Asp Ser Ile Gln Trp Phe His Asn Gly Asn Leu
65                  70                  75                  80

Ile Pro Thr His Thr Gln Pro Ser Tyr Arg Phe Lys Ala Asn Asn Asn
                85                  90                  95

Asp Ser Gly Glu Tyr Thr Cys Gln Thr Gly Gln Thr Ser Leu Ser Asp
            100                 105                 110

Pro Val His Leu Thr Val Leu Ser Glu Trp Leu Val Leu Gln Thr Pro
        115                 120                 125

His Leu Glu Phe Gln Glu Gly Glu Thr Ile Met Leu Arg Cys His Ser
    130                 135                 140

Trp Lys Asp Lys Pro Leu Val Lys Val Thr Phe Phe Gln Asn Gly Lys
145                 150                 155                 160

Ser Gln Lys Phe Ser His Leu Asp Pro Thr Phe Ser Ile Pro Gln Ala
                165                 170                 175

Asn His Ser His Ser Gly Asp Tyr His Cys Thr Gly Asn Ile Gly Tyr
            180                 185                 190

Thr Leu Phe Ser Ser Lys Pro Val Thr Ile Thr Val Gln Val Pro Ser
        195                 200                 205

Met Gly Ser Ser Ser Pro Met Gly Val Ile Val Ala Val Val Ile Ala
    210                 215                 220

Thr Ala Val Ala Ala Ile Val Ala Ala Val Val Ala Leu Ile Tyr Cys
225                 230                 235                 240

Arg Lys Lys Arg Ile Ser Ala Asn Ser Thr Asp Pro Val Lys Ala Ala
                245                 250                 255

Gln Phe Glu Pro Pro Gly Arg Gln Met Ile Ala Ile Arg Lys Arg Gln
260 265 270

Leu Glu Glu Thr Asn Asn Asp Tyr Glu Thr Ala Asp Gly Gly Tyr Met
275 280 285

Thr Leu Asn Pro Arg Ala Pro Thr Asp Asp Asp Lys Asn Ile Tyr Leu
290 295 300

Thr Leu Pro Pro Asn Asp His Val Asn Ser Asn Asn
305 310 315

<210> 20
<211> 876
<212> DNA
<213> Homo sapiens

<400> 20
atgggaatcc tgtcattctt acctgtcctt gccactgaga gtgactgggc tgactgcaag 60

tccccccagc cttggggtca tatgcttctg tggacagctg tgctattcct ggctcctgtt 120

gctgggacac ctgcagctcc cccaaaggct gtgctgaaac tcgagcccca gtggatcaac 180

gtgctccagg aggactctgt gactctgaca tgccggggga ctcacagccc tgagagcgac 240

tccattcagt ggttccacaa tgggaatctc attcccaccc acacgcagcc cagctacagg 300

ttcaaggcca acaacaatga cagcggggag tacacgtgcc agactggcca gaccagcctc 360

agcgaccctg tgcatctgac tgtgctttct gagtggctgg tgctccagac ccctcacctg 420

gagttccagg agggagaaac catcgtgctg aggtgccaca gctggaagga caagcctctg 480

gtcaaggtca cattcttcca gaatggaaaa tccaagaaat ttcccgttc ggatcccaac 540

ttctccatcc cacaagcaaa ccacagtcac agtggtgatt accactgcac aggaaacata 600

ggctacacgc tgtactcatc caagcctgtg accatcactg tccaagctcc cagctcttca 660

ccgatgggga tcattgtggc tgtggtcact gggattgctg tagcggccat tgttgctgct 720

gtagtggcct tgatctactg caggaaaaag cggatttcag ccaatcccac taatcctgat 780

gaggctgaca aagttggggc tgagaacaca atcacctatt cacttctcat gcacccggat 840

gctctggaag agcctgatga ccagaaccgt atttag 876

<210> 21
<211> 291
<212> PRT
<213> Homo sapiens

<400> 21

Met Gly Ile Leu Ser Phe Leu Pro Val Leu Ala Thr Glu Ser Asp Trp
1 5 10 15

Ala Asp Cys Lys Ser Pro Gln Pro Trp Gly His Met Leu Leu Trp Thr
20 25 30

Ala Val Leu Phe Leu Ala Pro Val Ala Gly Thr Pro Ala Ala Pro Pro
35 40 45

Lys Ala Val Leu Lys Leu Glu Pro Gln Trp Ile Asn Val Leu Gln Glu
50 55 60

Asp Ser Val Thr Leu Thr Cys Arg Gly Thr His Ser Pro Glu Ser Asp
65 70 75 80

Ser Ile Gln Trp Phe His Asn Gly Asn Leu Ile Pro Thr His Thr Gln
85 90 95

Pro Ser Tyr Arg Phe Lys Ala Asn Asn Asn Asp Ser Gly Glu Tyr Thr
100 105 110

Cys Gln Thr Gly Gln Thr Ser Leu Ser Asp Pro Val His Leu Thr Val
115 120 125

Leu Ser Glu Trp Leu Val Leu Gln Thr Pro His Leu Glu Phe Gln Glu
130 135 140

Gly Glu Thr Ile Val Leu Arg Cys His Ser Trp Lys Asp Lys Pro Leu
145 150 155 160

Val Lys Val Thr Phe Phe Gln Asn Gly Lys Ser Lys Lys Phe Ser Arg
165 170 175

Ser Asp Pro Asn Phe Ser Ile Pro Gln Ala Asn His Ser His Ser Gly
180 185 190

Asp Tyr His Cys Thr Gly Asn Ile Gly Tyr Thr Leu Tyr Ser Ser Lys
195 200 205

Pro Val Thr Ile Thr Val Gln Ala Pro Ser Ser Ser Pro Met Gly Ile
210 215 220

Ile Val Ala Val Val Thr Gly Ile Ala Val Ala Ala Ile Val Ala Ala
225 230 235 240

Val Val Ala Leu Ile Tyr Cys Arg Lys Lys Arg Ile Ser Ala Asn Pro
245 250 255

Thr Asn Pro Asp Glu Ala Asp Lys Val Gly Ala Glu Asn Thr Ile Thr

260 265 270

Tyr Ser Leu Leu Met His Pro Asp Ala Leu Glu Glu Pro Asp Asp Gln
275 280 285

Asn Arg Ile
290

<210> 22
<211> 765
<212> DNA
<213> Homo sapiens

<400> 22
atgtggcagc tgctcctccc aactgctctg ctacttctag tttcagctgg catgcggact 60

gaagatctcc caaaggctgt ggtgttcctg gagcctcaat ggtacagggt gctcgagaag 120

gacagtgtga ctctgaagtg ccagggagcc tactcccctg aggacaattc cacacagtgg 180

tttcacaatg agagcctcat ctcaagccag gcctcgagct acttcattga cgctgccaca 240

gttgacgaca gtggagagta caggtgccag acaaacctct ccaccctcag tgacccggtg 300

cagctagaag tccatatcgg ctggctgttg ctccaggccc ctcggtgggt gttcaaggag 360

gaagacccta ttcacctgag gtgtcacagc tggaagaaca ctgctctgca taaggtcaca 420

tatttacaga atggcaaagg caggaagtat tttcatcata attctgactt ctacattcca 480

aaagccacac tcaaagacag cggctcctac ttctgcaggg ggcttgttgg gagtaaaaat 540

gtgtcttcag agactgtgaa catcaccatc actcaaggtt tgtcagtgtc aaccatctca 600

tcattctttc cacctgggta ccaagtctct ttctgcttgg tgatggtact cctttttgca 660

gtggacacag gactatattt ctctgtgaag acaaacattc gaagctcaac aagagactgg 720

aaggaccata aatttaaatg gagaaaggac cctcaagaca aatga 765

<210> 23
<211> 254
<212> PRT
<213> Homo sapiens

<400> 23

Met Trp Gln Leu Leu Leu Pro Thr Ala Leu Leu Leu Leu Val Ser Ala
1 5 10 15

Gly Met Arg Thr Glu Asp Leu Pro Lys Ala Val Val Phe Leu Glu Pro
20 25 30

Gln Trp Tyr Arg Val Leu Glu Lys Asp Ser Val Thr Leu Lys Cys Gln
35 40 45

```
Gly Ala Tyr Ser Pro Glu Asp Asn Ser Thr Gln Trp Phe His Asn Glu
    50                  55                  60

Ser Leu Ile Ser Ser Gln Ala Ser Ser Tyr Phe Ile Asp Ala Ala Thr
65                  70                  75                  80

Val Asp Asp Ser Gly Glu Tyr Arg Cys Gln Thr Asn Leu Ser Thr Leu
                85                  90                  95

Ser Asp Pro Val Gln Leu Glu Val His Ile Gly Trp Leu Leu Leu Gln
            100                 105                 110

Ala Pro Arg Trp Val Phe Lys Glu Glu Asp Pro Ile His Leu Arg Cys
        115                 120                 125

His Ser Trp Lys Asn Thr Ala Leu His Lys Val Thr Tyr Leu Gln Asn
    130                 135                 140

Gly Lys Gly Arg Lys Tyr Phe His His Asn Ser Asp Phe Tyr Ile Pro
145                 150                 155                 160

Lys Ala Thr Leu Lys Asp Ser Gly Ser Tyr Phe Cys Arg Gly Leu Val
                165                 170                 175

Gly Ser Lys Asn Val Ser Ser Glu Thr Val Asn Ile Thr Ile Thr Gln
            180                 185                 190

Gly Leu Ser Val Ser Thr Ile Ser Ser Phe Phe Pro Pro Gly Tyr Gln
        195                 200                 205

Val Ser Phe Cys Leu Val Met Val Leu Leu Phe Ala Val Asp Thr Gly
    210                 215                 220

Leu Tyr Phe Ser Val Lys Thr Asn Ile Arg Ser Ser Thr Arg Asp Trp
225                 230                 235                 240

Lys Asp His Lys Phe Lys Trp Arg Lys Asp Pro Gln Asp Lys
                245                 250
```

<210> 24
<211> 702
<212> DNA
<213> Homo sapiens

<400> 24

```
atgtggcagc tgctcctccc aactgctctg ctacttctag tttcagctgg catgcggact      60

gaagatctcc caaaggctgt ggtgttcctg gagcctcaat ggtacagcgt gcttgagaag     120

gacagtgtga ctctgaagtg ccagggagcc tactcccctg aggacaattc cacacagtgg     180
```

tttcacaatg agagcctcat ctcaagccag gcctcgagct acttcattga cgctgccaca 240

gtcaacgaca gtggagagta caggtgccag acaaacctct ccaccctcag tgacccggtg 300

cagctagaag tccatatcgg ctggctgttg ctccaggccc ctcggtgggt gttcaaggag 360

gaagacccta ttcacctgag gtgtcacagc tggaagaaca ctgctctgca taaggtcaca 420

tatttacaga atggcaaaga caggaagtat tttcatcata attctgactt ccacattcca 480

aaagccacac tcaaagatag cggctcctac ttctgcaggg ggcttgttgg gagtaaaaat 540

gtgtcttcag agactgtgaa catcaccatc actcaaggtt tggcagtgtc aaccatctca 600

tcattctctc cacctgggta ccaagtctct ttctgcttgg tgatggtact cctttttgca 660

gtggacacag gactatattt ctctgtgaag acaaacattt ga 702

<210> 25
<211> 233
<212> PRT
<213> Homo sapiens

<400> 25

Met Trp Gln Leu Leu Leu Pro Thr Ala Leu Leu Leu Leu Val Ser Ala
1 5 10 15

Gly Met Arg Thr Glu Asp Leu Pro Lys Ala Val Val Phe Leu Glu Pro
20 25 30

Gln Trp Tyr Ser Val Leu Glu Lys Asp Ser Val Thr Leu Lys Cys Gln
35 40 45

Gly Ala Tyr Ser Pro Glu Asp Asn Ser Thr Gln Trp Phe His Asn Glu
50 55 60

Ser Leu Ile Ser Ser Gln Ala Ser Ser Tyr Phe Ile Asp Ala Ala Thr
65 70 75 80

Val Asn Asp Ser Gly Glu Tyr Arg Cys Gln Thr Asn Leu Ser Thr Leu
85 90 95

Ser Asp Pro Val Gln Leu Glu Val His Ile Gly Trp Leu Leu Leu Gln
100 105 110

Ala Pro Arg Trp Val Phe Lys Glu Glu Asp Pro Ile His Leu Arg Cys
115 120 125

His Ser Trp Lys Asn Thr Ala Leu His Lys Val Thr Tyr Leu Gln Asn
130 135 140

Gly Lys Asp Arg Lys Tyr Phe His His Asn Ser Asp Phe His Ile Pro
145                 150                 155                 160

Lys Ala Thr Leu Lys Asp Ser Gly Ser Tyr Phe Cys Arg Gly Leu Val
                165                 170                 175

Gly Ser Lys Asn Val Ser Ser Glu Thr Val Asn Ile Thr Ile Thr Gln
            180                 185                 190

Gly Leu Ala Val Ser Thr Ile Ser Ser Phe Ser Pro Pro Gly Tyr Gln
        195                 200                 205

Val Ser Phe Cys Leu Val Met Val Leu Leu Phe Ala Val Asp Thr Gly
    210                 215                 220

Leu Tyr Phe Ser Val Lys Thr Asn Ile
225                 230

<210>  26
<211>  4
<212>  PRT
<213>  Artificial

<220>
<223>  An artificially synthesized peptide sequence

<400>  26

Gly Gly Gly Ser
1

<210>  27
<211>  4
<212>  PRT
<213>  Artificial

<220>
<223>  An artificially synthesized peptide sequence

<400>  27

Ser Gly Gly Gly
1

<210>  28
<211>  5
<212>  PRT
<213>  Artificial

<220>
<223>  An artificially synthesized peptide sequence

<400>  28

Gly Gly Gly Gly Ser

1 5

<210> 29
<211> 5
<212> PRT
<213> Artificial

<220>
<223> An artificially synthesized peptide sequence

<400> 29

Ser Gly Gly Gly Gly
1 5

<210> 30
<211> 6
<212> PRT
<213> Artificial

<220>
<223> An artificially synthesized peptide sequence

<400> 30

Gly Gly Gly Gly Gly Ser
1 5

<210> 31
<211> 6
<212> PRT
<213> Artificial

<220>
<223> An artificially synthesized peptide sequence

<400> 31

Ser Gly Gly Gly Gly Gly
1 5

<210> 32
<211> 7
<212> PRT
<213> Artificial

<220>
<223> An artificially synthesized peptide sequence

<400> 32

Gly Gly Gly Gly Gly Gly Ser
1 5

<210> 33
<211> 7
<212> PRT
<213> Artificial

<220>
<223>  An artificially synthesized peptide sequence

<400>  33

Ser Gly Gly Gly Gly Gly Gly
1               5

<210>  34
<211>  365
<212>  PRT
<213>  Homo sapiens

<400>  34

Met Gly Val Pro Arg Pro Gln Pro Trp Ala Leu Gly Leu Leu Leu Phe
1               5                   10                  15

Leu Leu Pro Gly Ser Leu Gly Ala Glu Ser His Leu Ser Leu Leu Tyr
            20                  25                  30

His Leu Thr Ala Val Ser Ser Pro Ala Pro Gly Thr Pro Ala Phe Trp
        35                  40                  45

Val Ser Gly Trp Leu Gly Pro Gln Gln Tyr Leu Ser Tyr Asn Ser Leu
    50                  55                  60

Arg Gly Glu Ala Glu Pro Cys Gly Ala Trp Val Trp Glu Asn Gln Val
65                  70                  75                  80

Ser Trp Tyr Trp Glu Lys Glu Thr Thr Asp Leu Arg Ile Lys Glu Lys
                85                  90                  95

Leu Phe Leu Glu Ala Phe Lys Ala Leu Gly Gly Lys Gly Pro Tyr Thr
            100                 105                 110

Leu Gln Gly Leu Leu Gly Cys Glu Leu Gly Pro Asp Asn Thr Ser Val
        115                 120                 125

Pro Thr Ala Lys Phe Ala Leu Asn Gly Glu Glu Phe Met Asn Phe Asp
    130                 135                 140

Leu Lys Gln Gly Thr Trp Gly Gly Asp Trp Pro Glu Ala Leu Ala Ile
145                 150                 155                 160

Ser Gln Arg Trp Gln Gln Gln Asp Lys Ala Ala Asn Lys Glu Leu Thr
                165                 170                 175

Phe Leu Leu Phe Ser Cys Pro His Arg Leu Arg Glu His Leu Glu Arg
            180                 185                 190

```
Gly Arg Gly Asn Leu Glu Trp Lys Glu Pro Pro Ser Met Arg Leu Lys
        195                 200                 205

Ala Arg Pro Ser Ser Pro Gly Phe Ser Val Leu Thr Cys Ser Ala Phe
    210                 215                 220

Ser Phe Tyr Pro Pro Glu Leu Gln Leu Arg Phe Leu Arg Asn Gly Leu
225                 230                 235                 240

Ala Ala Gly Thr Gly Gln Gly Asp Phe Gly Pro Asn Ser Asp Gly Ser
                245                 250                 255

Phe His Ala Ser Ser Ser Leu Thr Val Lys Ser Gly Asp Glu His His
            260                 265                 270

Tyr Cys Cys Ile Val Gln His Ala Gly Leu Ala Gln Pro Leu Arg Val
        275                 280                 285

Glu Leu Glu Ser Pro Ala Lys Ser Ser Val Leu Val Val Gly Ile Val
    290                 295                 300

Ile Gly Val Leu Leu Leu Thr Ala Ala Ala Val Gly Gly Ala Leu Leu
305                 310                 315                 320

Trp Arg Arg Met Arg Ser Gly Leu Pro Ala Pro Trp Ile Ser Leu Arg
                325                 330                 335

Gly Asp Asp Thr Gly Val Leu Leu Pro Thr Pro Gly Glu Ala Gln Asp
            340                 345                 350

Ala Asp Leu Lys Asp Val Asn Val Ile Pro Ala Thr Ala
        355                 360                 365


<210>  35
<211>  119
<212>  PRT
<213>  Homo sapiens

<400>  35

Met Ser Arg Ser Val Ala Leu Ala Val Leu Ala Leu Leu Ser Leu Ser
1               5                   10                  15

Gly Leu Glu Ala Ile Gln Arg Thr Pro Lys Ile Gln Val Tyr Ser Arg
            20                  25                  30

His Pro Ala Glu Asn Gly Lys Ser Asn Phe Leu Asn Cys Tyr Val Ser
        35                  40                  45
```

Gly Phe His Pro Ser Asp Ile Glu Val Asp Leu Leu Lys Asn Gly Glu
50 55 60

Arg Ile Glu Lys Val Glu His Ser Asp Leu Ser Phe Ser Lys Asp Trp
65 70 75 80

Ser Phe Tyr Leu Leu Tyr Tyr Thr Glu Phe Thr Pro Thr Glu Lys Asp
85 90 95

Glu Tyr Ala Cys Arg Val Asn His Val Thr Leu Ser Gln Pro Lys Ile
100 105 110

Val Lys Trp Asp Arg Asp Met
115

<210> 36
<211> 447
<212> PRT
<213> Artificial

<220>
<223> An artificially synthesized peptide sequence

<400> 36

Gln Val Gln Leu Gln Glu Ser Gly Pro Gly Leu Val Lys Pro Ser Glu
1 5 10 15

Thr Leu Ser Leu Thr Cys Ala Val Ser Gly Tyr Ser Ile Ser Asp Asp
20 25 30

Gln Ala Trp Ser Trp Val Arg Gln Pro Pro Gly Glu Gly Leu Glu Trp
35 40 45

Ile Gly Tyr Ile Ser Tyr Ser Gly Ile Thr Asn Tyr Asn Pro Ser Leu
50 55 60

Lys Gly Arg Val Thr Ile Ser Arg Asp Thr Ser Lys Asn Gln Phe Ser
65 70 75 80

Leu Lys Leu Ser Ser Val Thr Ala Ala Asp Thr Ala Ala Tyr Tyr Cys
85 90 95

Ala Arg Ser Leu Ala Arg Thr Thr Ala Met Asp Tyr Trp Gly Glu Gly
100 105 110

Thr Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
210 215 220

Thr His Thr Cys Pro Pro Cys Pro Ala Pro Glu Leu Leu Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro
435 440 445

<210> 37
<211> 214
<212> PRT
<213> Artificial

<220>
<223> An artificially synthesized peptide sequence

<400> 37

Asp Ile Gln Met Thr Gln Ser Pro Ser Ser Leu Ser Ala Ser Val Gly
1 5 10 15

Asp Ser Val Thr Ile Thr Cys Gln Ala Ser Gln Asp Ile Ser Ser Tyr
20 25 30

Leu Asn Trp Tyr Gln Gln Lys Pro Gly Lys Ala Pro Glu Leu Leu Ile
35 40 45

Tyr Tyr Gly Ser Glu Leu His Ser Gly Val Pro Ser Arg Phe Ser Gly
50 55 60

Ser Gly Ser Gly Thr Asp Phe Thr Phe Thr Ile Ser Ser Leu Glu Ala
65 70 75 80

Glu Asp Ala Ala Thr Tyr Tyr Cys Gln Gln Gly Asn Ser Leu Pro Tyr
85 90 95

Thr Phe Gly Gln Gly Thr Lys Val Glu Ile Glu Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 38
<211> 447
<212> PRT
<213> Artificial

<220>
<223> An artificially synthesized peptide sequence

<400> 38

Gln Val Gln Leu Gln Glu Ser Gly Pro Gly Leu Val Lys Pro Ser Glu
1 5 10 15

Thr Leu Ser Leu Thr Cys Ala Val Ser Gly His Ser Ile Ser His Asp
20 25 30

His Ala Trp Ser Trp Val Arg Gln Pro Pro Gly Glu Gly Leu Glu Trp
35 40 45

Ile Gly Phe Ile Ser Tyr Ser Gly Ile Thr Asn Tyr Asn Pro Ser Leu
50 55 60

Gln Gly Arg Val Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Ser Leu Ala Arg Thr Thr Ala Met Asp Tyr Trp Gly Glu Gly
100 105 110

Thr Leu Val Thr Val Ser Ser Ala Ser Thr Lys Gly Pro Ser Val Phe
115 120 125

```
Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380
```

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro
435 440 445

<210> 39
<211> 214
<212> PRT
<213> Artificial

<220>
<223> An artificially synthesized peptide sequence

<400> 39

Asp Ile Gln Met Thr Gln Ser Pro Ser Ser Leu Ser Ala Ser Val Gly
1 5 10 15

Asp Ser Val Thr Ile Thr Cys Gln Ala Ser Thr Asp Ile Ser Ser His
20 25 30

Leu Asn Trp Tyr Gln Gln Lys Pro Gly Lys Ala Pro Glu Leu Leu Ile
35 40 45

Tyr Tyr Gly Ser His Leu Leu Ser Gly Val Pro Ser Arg Phe Ser Gly
50 55 60

Ser Gly Ser Gly Thr Asp Phe Thr Phe Thr Ile Ser Ser Leu Glu Ala
65 70 75 80

Glu Asp Ala Ala Thr Tyr Tyr Cys Gly Gln Gly Asn Arg Leu Pro Tyr
85 90 95

Thr Phe Gly Gln Gly Thr Lys Val Glu Ile Glu Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 40
<211> 447
<212> PRT
<213> Artificial

<220>
<223> An artificially synthesized peptide sequence

<400> 40

Gln Val Gln Leu Gln Glu Ser Gly Pro Gly Leu Val Lys Pro Ser Glu
1 5 10 15

Thr Leu Ser Leu Thr Cys Ala Val Ser Gly His Ser Ile Ser His Asp
20 25 30

His Ala Trp Ser Trp Val Arg Gln Pro Pro Gly Glu Gly Leu Glu Trp
35 40 45

Ile Gly Phe Ile Ser Tyr Ser Gly Ile Thr Asn Tyr Asn Pro Ser Leu
50 55 60

Gln Gly Arg Val Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Ser Leu Ala Arg Thr Thr Ala Met Asp Tyr Trp Gly Glu Gly
100 105 110

Thr Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu

130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
210 215 220

Thr His Thr Cys Pro Pro Cys Pro Ala Pro Glu Leu Leu Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Asp Ala Tyr Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro
435 440 445

<210> 41
<211> 447
<212> PRT
<213> Artificial

<220>
<223> An artificially synthesized peptide sequence

<400> 41

Gln Val Gln Leu Gln Glu Ser Gly Pro Gly Leu Val Lys Pro Ser Glu
1 5 10 15

Thr Leu Ser Leu Thr Cys Ala Val Ser Gly His Ser Ile Ser His Asp
20 25 30

His Ala Trp Ser Trp Val Arg Gln Pro Pro Gly Glu Gly Leu Glu Trp
35 40 45

Ile Gly Phe Ile Ser Tyr Ser Gly Ile Thr Asn Tyr Asn Pro Ser Leu
50 55 60

Gln Gly Arg Val Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Ser Leu Ala Arg Thr Thr Ala Met Asp Tyr Trp Gly Glu Gly
100 105 110

Thr Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp

145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
210 215 220

Thr His Thr Cys Pro Pro Cys Pro Ala Pro Glu Leu Arg Gly Gly Pro
225 230 235 240

Lys Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro
435 440 445

<210> 42
<211> 214
<212> PRT
<213> Artificial

<220>
<223> An artificially synthesized peptide sequence

<400> 42

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Ile Ser Ser Tyr
20 25 30

Leu Asn Trp Tyr Gln Gln Lys Pro Gly Lys Ala Pro Lys Leu Leu Ile
35 40 45

Tyr Tyr Thr Ser Arg Leu His Ser Gly Val Pro Ser Arg Phe Ser Gly
50 55 60

Ser Gly Ser Gly Thr Asp Phe Thr Phe Thr Ile Ser Ser Leu Gln Pro
65 70 75 80

Glu Asp Ile Ala Thr Tyr Tyr Cys Gln Gln Gly Asn Thr Leu Pro Tyr
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser

165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 43
<211> 447
<212> PRT
<213> Artificial

<220>
<223> An artificially synthesized peptide sequence

<400> 43

Gln Val Gln Leu Gln Glu Ser Gly Pro Gly Leu Val Lys Pro Ser Glu
1 5 10 15

Thr Leu Ser Leu Thr Cys Ala Val Ser Gly His Ser Ile Ser His Asp
20 25 30

His Ala Trp Ser Trp Val Arg Gln Pro Pro Gly Glu Gly Leu Glu Trp
35 40 45

Ile Gly Phe Ile Ser Tyr Ser Gly Ile Thr Asn Tyr Asn Pro Ser Leu
50 55 60

Gln Gly Arg Val Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Ser Leu Ala Arg Thr Thr Ala Met Asp Tyr Trp Gly Glu Gly
100 105 110

Thr Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

```
Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Asp Ala Tyr Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
```

405 410 415

Ser Arg Trp Gln Gln Gly Asn Val Phe Ser Cys Ser Val Leu His Glu
420 425 430

Ala Leu His Ser His Tyr Thr Gln Lys Ser Leu Ser Leu Ser Pro
435 440 445

<210> 44
<211> 449
<212> PRT
<213> Artificial

<220>
<223> An artificially synthesized peptide sequence

<400> 44

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Tyr Met His Trp Val Arg Gln Ala Pro Gly Gln Gly Leu Glu Trp Met
35 40 45

Gly Ile Ile Asn Pro Ser Gly Gly Ser Thr Ser Tyr Ala Gln Lys Phe
50 55 60

Gln Gly Arg Val Thr Met Thr Arg Asp Thr Ser Thr Ser Thr Val Tyr
65 70 75 80

Met Glu Leu Ser Ser Leu Arg Ser Glu Asp Thr Ala Val Tyr Tyr Cys
85 90 95

Ala Arg Asp Asp Pro Gly Gly Gly Glu Tyr Tyr Phe Asp Tyr Trp Gly
100 105 110

Gln Gly Thr Leu Val Thr Val Ser Ser Ala Ser Thr Lys Gly Pro Ser
115 120 125

Val Phe Pro Leu Ala Pro Ser Ser Lys Ser Thr Ser Gly Gly Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Ser Leu Gly Thr Gln Thr Tyr Ile Cys Asn Val Asn His
195 200 205

Lys Pro Ser Asn Thr Lys Val Asp Lys Lys Val Glu Pro Lys Ser Cys
210 215 220

Asp Lys Thr His Thr Cys Pro Pro Cys Pro Ala Pro Glu Leu Leu Gly
225 230 235 240

Gly Pro Ser Val Phe Leu Phe Pro Pro Lys Pro Lys Asp Thr Leu Met
245 250 255

Ile Ser Arg Thr Pro Glu Val Thr Cys Val Val Val Asp Val Ser His
260 265 270

Glu Asp Pro Glu Val Lys Phe Asn Trp Tyr Val Asp Gly Val Glu Val
275 280 285

His Asn Ala Lys Thr Lys Pro Arg Glu Glu Gln Tyr Asn Ser Thr Tyr
290 295 300

Arg Val Val Ser Val Leu Thr Val Leu His Gln Asp Trp Leu Asn Gly
305 310 315 320

Lys Glu Tyr Lys Cys Lys Val Ser Asn Lys Ala Leu Pro Ala Pro Ile
325 330 335

Glu Lys Thr Ile Ser Lys Ala Lys Gly Gln Pro Arg Glu Pro Gln Val
340 345 350

Tyr Thr Leu Pro Pro Ser Arg Asp Glu Leu Thr Lys Asn Gln Val Ser
355 360 365

Leu Thr Cys Leu Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Tyr Ser Lys Leu Thr Val
405 410 415

Asp Lys Ser Arg Trp Gln Gln Gly Asn Val Phe Ser Cys Ser Val Met

420 425 430

His Glu Ala Leu His Asn His Tyr Thr Gln Lys Ser Leu Ser Leu Ser
435 440 445

Pro

<210> 45
<211> 217
<212> PRT
<213> Artificial

<220>
<223> An artificially synthesized peptide sequence

<400> 45

Gln Ser Val Leu Thr Gln Pro Pro Ser Val Ser Gly Ala Pro Gly Gln
1 5 10 15

Arg Val Thr Ile Ser Cys Thr Gly Ser Arg Ser Asn Met Gly Ala Gly
20 25 30

Tyr Asp Val His Trp Tyr Gln Leu Leu Pro Gly Ala Ala Pro Lys Leu
35 40 45

Leu Ile Ser His Asn Thr His Arg Pro Ser Gly Val Pro Asp Arg Phe
50 55 60

Ser Gly Ser Lys Ser Gly Ala Ser Ala Ser Leu Ala Ile Thr Gly Leu
65 70 75 80

Gln Ala Glu Asp Glu Ala Asp Tyr Tyr Cys Gln Ser His Asp Ser Ser
85 90 95

Leu Ser Ala Val Val Phe Gly Gly Gly Thr Lys Leu Thr Val Leu Ser
100 105 110

Gln Pro Lys Ala Ala Pro Ser Val Thr Leu Phe Pro Pro Ser Ser Glu
115 120 125

Glu Leu Gln Ala Asn Lys Ala Thr Leu Val Cys Leu Ile Ser Asp Phe
130 135 140

Tyr Pro Gly Ala Val Thr Val Ala Trp Lys Ala Asp Ser Ser Pro Val
145 150 155 160

Lys Ala Gly Val Glu Thr Thr Thr Pro Ser Lys Gln Ser Asn Asn Lys
165 170 175

Tyr Ala Ala Ser Ser Tyr Leu Ser Leu Thr Pro Glu Gln Trp Lys Ser
180 185 190

His Arg Ser Tyr Ser Cys Gln Val Thr His Glu Gly Ser Thr Val Glu
195 200 205

Lys Thr Val Ala Pro Thr Glu Cys Ser
210 215

<210> 46
<211> 453
<212> PRT
<213> Artificial

<220>
<223> An artificially synthesized peptide sequence

<400> 46

Gln Val Gln Leu Val Gln Ser Gly Ala Glu Val Lys Lys Pro Gly Ser
1 5 10 15

Ser Val Lys Val Ser Cys Lys Ala Ser Gly Gly Thr Phe Ser Ser Tyr
20 25 30

Ala Ile Ser Trp Val Arg Gln Ala Pro Gly Gln Gly Leu Glu Trp Met
35 40 45

Gly Gly Ile Ile Pro Ile Phe Gly Thr Ala Asn Tyr Ala Gln Lys Phe
50 55 60

Gln Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Glu Arg Asp Tyr Tyr Asp Ser Ser Gly Tyr Tyr Asp Ala Phe
100 105 110

Asp Ile Trp Gly Gln Gly Thr Met Val Thr Val Ser Ser Ala Ser Thr
115 120 125

Lys Gly Pro Ser Val Phe Pro Leu Ala Pro Ser Ser Lys Ser Thr Ser
130 135 140

Gly Gly Thr Ala Ala Leu Gly Cys Leu Val Lys Asp Tyr Phe Pro Glu
145 150 155 160

```
Pro Val Thr Val Ser Trp Asn Ser Gly Ala Leu Thr Ser Gly Val His
                165                 170                 175

Thr Phe Pro Ala Val Leu Gln Ser Ser Gly Leu Tyr Ser Leu Ser Ser
            180                 185                 190

Val Val Thr Val Pro Ser Ser Ser Leu Gly Thr Gln Thr Tyr Ile Cys
        195                 200                 205

Asn Val Asn His Lys Pro Ser Asn Thr Lys Val Asp Lys Lys Val Glu
    210                 215                 220

Pro Lys Ser Cys Asp Lys Thr His Thr Cys Pro Pro Cys Pro Ala Pro
225                 230                 235                 240

Glu Leu Leu Gly Gly Pro Ser Val Phe Leu Phe Pro Pro Lys Pro Lys
                245                 250                 255

Asp Thr Leu Met Ile Ser Arg Thr Pro Glu Val Thr Cys Val Val Val
            260                 265                 270

Asp Val Ser His Glu Asp Pro Glu Val Lys Phe Asn Trp Tyr Val Asp
        275                 280                 285

Gly Val Glu Val His Asn Ala Lys Thr Lys Pro Arg Glu Glu Gln Tyr
    290                 295                 300

Asn Ser Thr Tyr Arg Val Val Ser Val Leu Thr Val Leu His Gln Asp
305                 310                 315                 320

Trp Leu Asn Gly Lys Glu Tyr Lys Cys Lys Val Ser Asn Lys Ala Leu
                325                 330                 335

Pro Ala Pro Ile Glu Lys Thr Ile Ser Lys Ala Lys Gly Gln Pro Arg
            340                 345                 350

Glu Pro Gln Val Tyr Thr Leu Pro Pro Ser Arg Asp Glu Leu Thr Lys
        355                 360                 365

Asn Gln Val Ser Leu Thr Cys Leu Val Lys Gly Phe Tyr Pro Ser Asp
    370                 375                 380

Ile Ala Val Glu Trp Glu Ser Asn Gly Gln Pro Glu Asn Asn Tyr Lys
385                 390                 395                 400

Thr Thr Pro Pro Val Leu Asp Ser Asp Gly Ser Phe Phe Leu Tyr Ser
                405                 410                 415
```

```
Lys Leu Thr Val Asp Lys Ser Arg Trp Gln Gln Gly Asn Val Phe Ser
            420                 425                 430

Cys Ser Val Met His Glu Ala Leu His Asn His Tyr Thr Gln Lys Ser
        435                 440                 445

Leu Ser Leu Ser Pro
    450
```

<210> 47
<211> 214
<212> PRT
<213> Artificial

<220>
<223> An artificially synthesized peptide sequence

<400> 47

```
Glu Thr Thr Val Thr Gln Ser Pro Ser Phe Leu Ser Ala Ser Val Gly
1               5                   10                  15

Asp Arg Val Thr Ile Thr Cys Ile Thr Thr Thr Asp Ile Asp Asp Asp
            20                  25                  30

Met Asn Trp Phe Gln Gln Glu Pro Gly Lys Ala Pro Lys Leu Leu Ile
        35                  40                  45

Ser Glu Gly Asn Ile Leu Arg Pro Gly Val Pro Ser Arg Phe Ser Ser
    50                  55                  60

Ser Gly Tyr Gly Thr Asp Phe Thr Leu Thr Ile Ser Lys Leu Gln Pro
65                  70                  75                  80

Glu Asp Phe Ala Thr Tyr Tyr Cys Leu Gln Ser Asp Asn Leu Pro Phe
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160
```

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 48
<211> 106
<212> PRT
<213> Artificial

<220>
<223> An artificially synthesized peptide sequence

<400> 48

Glu Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr
20 25 30

Leu Ala Trp Tyr Gln Gln Lys Pro Gly Gln Ala Pro Arg Leu Leu Ile
35 40 45

Phe Asp Ala Ser Asn Arg Ala Ala Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Phe Asp Lys Trp Val Thr
85 90 95

Phe Gly Gly Gly Thr Thr Val Glu Ile Arg
100 105

<210> 49
<211> 454
<212> PRT
<213> Artificial

<220>
<223> An artificially synthesized peptide sequence

<400> 49

Glu Val Gln Leu Val Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Glu Met Asn Trp Val Arg Gln Ala Pro Gly Lys Gly Leu Glu Trp Val
35 40 45

Ser Tyr Ile Ser Ser Ser Gly Ser Thr Ile Tyr Tyr Ala Asp Ser Val
50 55 60

Lys Gly Arg Phe Thr Ile Ser Arg Asp Asn Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Asp Ala Pro Tyr Tyr Tyr Asp Ser Ser Gly Tyr Thr Asp Ala
100 105 110

Phe Asp Ile Trp Gly Gln Gly Thr Met Val Thr Val Ser Ser Ala Ser
115 120 125

Thr Lys Gly Pro Ser Val Phe Pro Leu Ala Pro Ser Ser Lys Ser Thr
130 135 140

Ser Gly Gly Thr Ala Ala Leu Gly Cys Leu Val Lys Asp Tyr Phe Pro
145 150 155 160

Glu Pro Val Thr Val Ser Trp Asn Ser Gly Ala Leu Thr Ser Gly Val
165 170 175

His Thr Phe Pro Ala Val Leu Gln Ser Ser Gly Leu Tyr Ser Leu Ser
180 185 190

Ser Val Val Thr Val Pro Ser Ser Ser Leu Gly Thr Gln Thr Tyr Ile
195 200 205

Cys Asn Val Asn His Lys Pro Ser Asn Thr Lys Val Asp Lys Lys Val
210 215 220

Glu Pro Lys Ser Cys Asp Lys Thr His Thr Cys Pro Pro Cys Pro Ala
225 230 235 240

Pro Glu Leu Leu Gly Gly Pro Ser Val Phe Leu Phe Pro Pro Lys Pro

245 250 255

Lys Asp Thr Leu Met Ile Ser Arg Thr Pro Glu Val Thr Cys Val Val
260 265 270

Val Asp Val Ser His Glu Asp Pro Glu Val Lys Phe Asn Trp Tyr Val
275 280 285

Asp Gly Val Glu Val His Asn Ala Lys Thr Lys Pro Arg Glu Glu Gln
290 295 300

Tyr Asn Ser Thr Tyr Arg Val Val Ser Val Leu Thr Val Leu His Gln
305 310 315 320

Asp Trp Leu Asn Gly Lys Glu Tyr Lys Cys Lys Val Ser Asn Lys Ala
325 330 335

Leu Pro Ala Pro Ile Glu Lys Thr Ile Ser Lys Ala Lys Gly Gln Pro
340 345 350

Arg Glu Pro Gln Val Tyr Thr Leu Pro Pro Ser Arg Asp Glu Leu Thr
355 360 365

Lys Asn Gln Val Ser Leu Thr Cys Leu Val Lys Gly Phe Tyr Pro Ser
370 375 380

Asp Ile Ala Val Glu Trp Glu Ser Asn Gly Gln Pro Glu Asn Asn Tyr
385 390 395 400

Lys Thr Thr Pro Pro Val Leu Asp Ser Asp Gly Ser Phe Phe Leu Tyr
405 410 415

Ser Lys Leu Thr Val Asp Lys Ser Arg Trp Gln Gln Gly Asn Val Phe
420 425 430

Ser Cys Ser Val Met His Glu Ala Leu His Asn His Tyr Thr Gln Lys
435 440 445

Ser Leu Ser Leu Ser Pro
450

<210> 50
<211> 107
<212> PRT
<213> Artificial

<220>
<223> An artificially synthesized peptide sequence

<400> 50

Arg Thr Val Ala Ala Pro Ser Val Phe Ile Phe Pro Pro Ser Asp Glu
1               5                   10                  15

Gln Leu Lys Ser Gly Thr Ala Ser Val Val Cys Leu Leu Asn Asn Phe
            20                  25                  30

Tyr Pro Arg Glu Ala Lys Val Gln Trp Lys Val Asp Asn Ala Leu Gln
        35                  40                  45

Ser Gly Asn Ser Gln Glu Ser Val Thr Glu Gln Asp Ser Lys Asp Ser
    50                  55                  60

Thr Tyr Ser Leu Ser Ser Thr Leu Thr Leu Ser Lys Ala Asp Tyr Glu
65                  70                  75                  80

Lys His Lys Val Tyr Ala Cys Glu Val Thr His Gln Gly Leu Ser Ser
                85                  90                  95

Pro Val Thr Lys Ser Phe Asn Arg Gly Glu Cys
            100                 105

<210> 51
<211> 443
<212> PRT
<213> Artificial

<220>
<223> An artificially synthesized peptide sequence

<400> 51

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Glu Met His Trp Val Arg Gln Ala Pro Gly Gln Gly Leu Glu Trp Met
        35                  40                  45

Gly Ala Leu Asp Pro Lys Thr Gly Asp Thr Ala Tyr Ser Gln Lys Phe
    50                  55                  60

Lys Gly Arg Val Thr Leu Thr Ala Asp Lys Ser Thr Ser Thr Ala Tyr
65                  70                  75                  80

Met Glu Leu Ser Ser Leu Thr Ser Glu Asp Thr Ala Val Tyr Tyr Cys
                85                  90                  95

Thr Arg Phe Tyr Ser Tyr Thr Tyr Trp Gly Gln Gly Thr Leu Val Thr
100 105 110

Val Ser Ser Ala Ser Thr Lys Gly Pro Ser Val Phe Pro Leu Ala Pro
115 120 125

Ser Ser Lys Ser Thr Ser Gly Gly Thr Ala Ala Leu Gly Cys Leu Val
130 135 140

Lys Asp Tyr Phe Pro Glu Pro Val Thr Val Ser Trp Asn Ser Gly Ala
145 150 155 160

Leu Thr Ser Gly Val His Thr Phe Pro Ala Val Leu Gln Ser Ser Gly
165 170 175

Leu Tyr Ser Leu Ser Ser Val Val Thr Val Pro Ser Ser Ser Leu Gly
180 185 190

Thr Gln Thr Tyr Ile Cys Asn Val Asn His Lys Pro Ser Asn Thr Lys
195 200 205

Val Asp Lys Lys Val Glu Pro Lys Ser Cys Asp Lys Thr His Thr Cys
210 215 220

Pro Pro Cys Pro Ala Pro Glu Leu Leu Gly Gly Pro Ser Val Phe Leu
225 230 235 240

Phe Pro Pro Lys Pro Lys Asp Thr Leu Met Ile Ser Arg Thr Pro Glu
245 250 255

Val Thr Cys Val Val Val Asp Val Ser His Glu Asp Pro Glu Val Lys
260 265 270

Phe Asn Trp Tyr Val Asp Gly Val Glu Val His Asn Ala Lys Thr Lys
275 280 285

Pro Arg Glu Glu Gln Tyr Asn Ser Thr Tyr Arg Val Val Ser Val Leu
290 295 300

Thr Val Leu His Gln Asp Trp Leu Asn Gly Lys Glu Tyr Lys Cys Lys
305 310 315 320

Val Ser Asn Lys Ala Leu Pro Ala Pro Ile Glu Lys Thr Ile Ser Lys
325 330 335

Ala Lys Gly Gln Pro Arg Glu Pro Gln Val Tyr Thr Leu Pro Pro Ser
340 345 350

Arg Asp Glu Leu Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro
        435                 440

<210> 52
<211> 219
<212> PRT
<213> Artificial

<220>
<223> An artificially synthesized peptide sequence

<400> 52

Asp Val Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Arg Asn Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Pro Thr Phe Gly Gln Gly Thr Lys Leu Glu Ile Lys
            100                 105                 110

Arg Thr Val Ala Ala Pro Ser Val Phe Ile Phe Pro Pro Ser Asp Glu
115 120 125

Gln Leu Lys Ser Gly Thr Ala Ser Val Val Cys Leu Leu Asn Asn Phe
130 135 140

Tyr Pro Arg Glu Ala Lys Val Gln Trp Lys Val Asp Asn Ala Leu Gln
145 150 155 160

Ser Gly Asn Ser Gln Glu Ser Val Thr Glu Gln Asp Ser Lys Asp Ser
165 170 175

Thr Tyr Ser Leu Ser Ser Thr Leu Thr Leu Ser Lys Ala Asp Tyr Glu
180 185 190

Lys His Lys Val Tyr Ala Cys Glu Val Thr His Gln Gly Leu Ser Ser
195 200 205

Pro Val Thr Lys Ser Phe Asn Arg Gly Glu Cys
210 215

<210> 53
<211> 454
<212> PRT
<213> Artificial

<220>
<223> An artificially synthesized peptide sequence

<400> 53

Glu Val Gln Leu Val Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Glu Met Asn Trp Val Arg Gln Ala Pro Gly Lys Gly Leu Glu Trp Val
35 40 45

Ser Tyr Ile Ser Ser Ser Gly Ser Thr Ile Tyr Tyr Ala Ala Ser Val
50 55 60

Lys Gly Arg Phe Thr Ile Ser Arg Asp Asn Ala Lys Asn Ser Leu Tyr
65 70 75 80

Leu Gln Met Asn Ser Leu Arg Ala Glu Asp Thr Ala Val Tyr Tyr Cys
85 90 95

```
Ala Arg Asp Ala Pro Tyr Tyr Tyr Asp Ser Ser Gly Tyr Thr Asp Ala
            100                 105                 110

Phe Asp Ile Trp Gly Gln Gly Thr Met Val Thr Val Ser Ser Ala Ser
        115                 120                 125

Thr Lys Gly Pro Ser Val Phe Pro Leu Ala Pro Ser Ser Lys Ser Thr
    130                 135                 140

Ser Gly Gly Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
            180                 185                 190

Ser Val Val Thr Val Pro Ser Ser Ser Leu Gly Thr Gln Thr Tyr Ile
        195                 200                 205

Cys Asn Val Asn His Lys Pro Ser Asn Thr Lys Val Asp Lys Lys Val
    210                 215                 220

Glu Pro Lys Ser Cys Asp Lys Thr His Thr Cys Pro Pro Cys Pro Ala
225                 230                 235                 240

Pro Glu Leu Leu Gly Gly Pro Ser Val Phe Leu Phe Pro Pro Lys Pro
                245                 250                 255

Lys Asp Thr Leu Met Ile Ser Arg Thr Pro Glu Val Thr Cys Val Val
            260                 265                 270

Val Asp Val Ser His Glu Asp Pro Glu Val Lys Phe Asn Trp Tyr Val
        275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Pro Ala Pro Ile Glu Lys Thr Ile Ser Lys Ala Lys Gly Gln Pro
            340                 345                 350
```

Arg Glu Pro Gln Val Tyr Thr Leu Pro Pro Ser Arg Asp Glu Leu Thr
        355                 360                 365

Lys Asn Gln Val Ser Leu Thr Cys Leu Val Lys Gly Phe Tyr Pro Ser
    370                 375                 380

Asp Ile Ala Val Glu Trp Glu Ser Asn Gly Gln Pro Glu Asn Asn Tyr
385                 390                 395                 400

Lys Thr Thr Pro Pro Val Leu Asp Ser Asp Gly Ser Phe Phe Leu Tyr
                405                 410                 415

Ser Lys Leu Thr Val Asp Lys Ser Arg Trp Gln Gln Gly Asn Val Phe
            420                 425                 430

Ser Cys Ser Val Met His Glu Ala Leu His Asn His Tyr Thr Gln Lys
        435                 440                 445

Ser Leu Ser Leu Ser Pro
    450

<210> 54
<211> 454
<212> PRT
<213> Artificial

<220>
<223> An artificially synthesized peptide sequence

<400> 54

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Glu Met Asn Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

```
Ala Arg Ala Ala Pro Tyr Tyr Tyr Asp Ser Ser Gly Tyr Thr Asp Ala
            100                 105                 110

Phe Asp Ile Trp Gly Gln Gly Thr Met Val Thr Val Ser Ser Ala Ser
        115                 120                 125

Thr Lys Gly Pro Ser Val Phe Pro Leu Ala Pro Ser Ser Lys Ser Thr
    130                 135                 140

Ser Gly Gly Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
            180                 185                 190

Ser Val Val Thr Val Pro Ser Ser Ser Leu Gly Thr Gln Thr Tyr Ile
        195                 200                 205

Cys Asn Val Asn His Lys Pro Ser Asn Thr Lys Val Asp Lys Lys Val
    210                 215                 220

Glu Pro Lys Ser Cys Asp Lys Thr His Thr Cys Pro Pro Cys Pro Ala
225                 230                 235                 240

Pro Glu Leu Leu Gly Gly Pro Ser Val Phe Leu Phe Pro Pro Lys Pro
                245                 250                 255

Lys Asp Thr Leu Met Ile Ser Arg Thr Pro Glu Val Thr Cys Val Val
            260                 265                 270

Val Asp Val Ser His Glu Asp Pro Glu Val Lys Phe Asn Trp Tyr Val
        275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Pro Ala Pro Ile Glu Lys Thr Ile Ser Lys Ala Lys Gly Gln Pro
            340                 345                 350
```

Arg Glu Pro Gln Val Tyr Thr Leu Pro Pro Ser Arg Asp Glu Leu Thr
355 360 365

Lys Asn Gln Val Ser Leu Thr Cys Leu Val Lys Gly Phe Tyr Pro Ser
370 375 380

Asp Ile Ala Val Glu Trp Glu Ser Asn Gly Gln Pro Glu Asn Asn Tyr
385 390 395 400

Lys Thr Thr Pro Pro Val Leu Asp Ser Asp Gly Ser Phe Phe Leu Tyr
405 410 415

Ser Lys Leu Thr Val Asp Lys Ser Arg Trp Gln Gln Gly Asn Val Phe
420 425 430

Ser Cys Ser Val Met His Glu Ala Leu His Asn His Tyr Thr Gln Lys
435 440 445

Ser Leu Ser Leu Ser Pro
450

<210> 55
<211> 454
<212> PRT
<213> Artificial

<220>
<223> An artificially synthesized peptide sequence

<400> 55

Glu Val Gln Leu Val Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Glu Met Asn Trp Val Arg Gln Ala Pro Gly Lys Gly Leu Glu Trp Val
35 40 45

Ser Tyr Ile Ser Ser Ser Gly Ser Thr Ile Tyr Tyr Ala Asp Ser Val
50 55 60

Lys Gly Arg Phe Thr Ile Ser Arg Asp Asn Ala Lys Asn Ser Leu Tyr
65 70 75 80

Leu Gln Met Asn Ser Leu Arg Ala Glu Asp Thr Ala Val Tyr Tyr Cys
85 90 95

```
Ala Arg Asp Ala Pro Tyr Tyr Tyr Ala Ser Ser Gly Tyr Thr Asp Ala
            100                 105                 110

Phe Asp Ile Trp Gly Gln Gly Thr Met Val Thr Val Ser Ser Ala Ser
        115                 120                 125

Thr Lys Gly Pro Ser Val Phe Pro Leu Ala Pro Ser Ser Lys Ser Thr
    130                 135                 140

Ser Gly Gly Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
            180                 185                 190

Ser Val Val Thr Val Pro Ser Ser Ser Leu Gly Thr Gln Thr Tyr Ile
        195                 200                 205

Cys Asn Val Asn His Lys Pro Ser Asn Thr Lys Val Asp Lys Lys Val
    210                 215                 220

Glu Pro Lys Ser Cys Asp Lys Thr His Thr Cys Pro Pro Cys Pro Ala
225                 230                 235                 240

Pro Glu Leu Leu Gly Gly Pro Ser Val Phe Leu Phe Pro Pro Lys Pro
                245                 250                 255

Lys Asp Thr Leu Met Ile Ser Arg Thr Pro Glu Val Thr Cys Val Val
            260                 265                 270

Val Asp Val Ser His Glu Asp Pro Glu Val Lys Phe Asn Trp Tyr Val
        275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Pro Ala Pro Ile Glu Lys Thr Ile Ser Lys Ala Lys Gly Gln Pro
            340                 345                 350
```

Arg Glu Pro Gln Val Tyr Thr Leu Pro Pro Ser Arg Asp Glu Leu Thr
355 360 365

Lys Asn Gln Val Ser Leu Thr Cys Leu Val Lys Gly Phe Tyr Pro Ser
370 375 380

Asp Ile Ala Val Glu Trp Glu Ser Asn Gly Gln Pro Glu Asn Asn Tyr
385 390 395 400

Lys Thr Thr Pro Pro Val Leu Asp Ser Asp Gly Ser Phe Phe Leu Tyr
405 410 415

Ser Lys Leu Thr Val Asp Lys Ser Arg Trp Gln Gln Gly Asn Val Phe
420 425 430

Ser Cys Ser Val Met His Glu Ala Leu His Asn His Tyr Thr Gln Lys
435 440 445

Ser Leu Ser Leu Ser Pro
450

<210> 56
<211> 454
<212> PRT
<213> Artificial

<220>
<223> An artificially synthesized peptide sequence

<400> 56

Glu Val Gln Leu Val Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Glu Met Asn Trp Val Arg Gln Ala Pro Gly Lys Gly Leu Glu Trp Val
35 40 45

Ser Tyr Ile Ser Ser Ser Gly Ser Thr Ile Tyr Tyr Ala Asp Ser Val
50 55 60

Lys Gly Arg Phe Thr Ile Ser Arg Asp Asn Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Asp Ala Pro Tyr Tyr Tyr Asp Ser Ser Gly Tyr Thr Ala Ala
100 105 110

Phe Asp Ile Trp Gly Gln Gly Thr Met Val Thr Val Ser Ser Ala Ser
115 120 125

Thr Lys Gly Pro Ser Val Phe Pro Leu Ala Pro Ser Ser Lys Ser Thr
130 135 140

Ser Gly Gly Thr Ala Ala Leu Gly Cys Leu Val Lys Asp Tyr Phe Pro
145 150 155 160

Glu Pro Val Thr Val Ser Trp Asn Ser Gly Ala Leu Thr Ser Gly Val
165 170 175

His Thr Phe Pro Ala Val Leu Gln Ser Ser Gly Leu Tyr Ser Leu Ser
180 185 190

Ser Val Val Thr Val Pro Ser Ser Ser Leu Gly Thr Gln Thr Tyr Ile
195 200 205

Cys Asn Val Asn His Lys Pro Ser Asn Thr Lys Val Asp Lys Lys Val
210 215 220

Glu Pro Lys Ser Cys Asp Lys Thr His Thr Cys Pro Pro Cys Pro Ala
225 230 235 240

Pro Glu Leu Leu Gly Gly Pro Ser Val Phe Leu Phe Pro Pro Lys Pro
245 250 255

Lys Asp Thr Leu Met Ile Ser Arg Thr Pro Glu Val Thr Cys Val Val
260 265 270

Val Asp Val Ser His Glu Asp Pro Glu Val Lys Phe Asn Trp Tyr Val
275 280 285

Asp Gly Val Glu Val His Asn Ala Lys Thr Lys Pro Arg Glu Glu Gln
290 295 300

Tyr Asn Ser Thr Tyr Arg Val Val Ser Val Leu Thr Val Leu His Gln
305 310 315 320

Asp Trp Leu Asn Gly Lys Glu Tyr Lys Cys Lys Val Ser Asn Lys Ala
325 330 335

Leu Pro Ala Pro Ile Glu Lys Thr Ile Ser Lys Ala Lys Gly Gln Pro
340 345 350

Arg Glu Pro Gln Val Tyr Thr Leu Pro Pro Ser Arg Asp Glu Leu Thr
355 360 365

Lys Asn Gln Val Ser Leu Thr Cys Leu Val Lys Gly Phe Tyr Pro Ser
370 375 380

Asp Ile Ala Val Glu Trp Glu Ser Asn Gly Gln Pro Glu Asn Asn Tyr
385 390 395 400

Lys Thr Thr Pro Pro Val Leu Asp Ser Asp Gly Ser Phe Phe Leu Tyr
405 410 415

Ser Lys Leu Thr Val Asp Lys Ser Arg Trp Gln Gln Gly Asn Val Phe
420 425 430

Ser Cys Ser Val Met His Glu Ala Leu His Asn His Tyr Thr Gln Lys
435 440 445

Ser Leu Ser Leu Ser Pro
450

<210> 57
<211> 454
<212> PRT
<213> Artificial

<220>
<223> An artificially synthesized peptide sequence

<400> 57

Glu Val Gln Leu Val Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Glu Met Asn Trp Val Arg Gln Ala Pro Gly Lys Gly Leu Glu Trp Val
35 40 45

Ser Tyr Ile Ser Ser Ser Gly Ser Thr Ile Tyr Tyr Ala Asp Ser Val
50 55 60

Lys Gly Arg Phe Thr Ile Ser Arg Asp Asn Ala Lys Asn Ser Leu Tyr
65 70 75 80

Leu Gln Met Asn Ser Leu Arg Ala Glu Asp Thr Ala Val Tyr Tyr Cys
85 90 95

Ala Arg Asp Ala Pro Tyr Tyr Tyr Asp Ser Ser Gly Tyr Thr Asp Ala
100 105 110

Phe Ala Ile Trp Gly Gln Gly Thr Met Val Thr Val Ser Ser Ala Ser
115 120 125

Thr Lys Gly Pro Ser Val Phe Pro Leu Ala Pro Ser Ser Lys Ser Thr
130 135 140

Ser Gly Gly Thr Ala Ala Leu Gly Cys Leu Val Lys Asp Tyr Phe Pro
145 150 155 160

Glu Pro Val Thr Val Ser Trp Asn Ser Gly Ala Leu Thr Ser Gly Val
165 170 175

His Thr Phe Pro Ala Val Leu Gln Ser Ser Gly Leu Tyr Ser Leu Ser
180 185 190

Ser Val Val Thr Val Pro Ser Ser Ser Leu Gly Thr Gln Thr Tyr Ile
195 200 205

Cys Asn Val Asn His Lys Pro Ser Asn Thr Lys Val Asp Lys Lys Val
210 215 220

Glu Pro Lys Ser Cys Asp Lys Thr His Thr Cys Pro Pro Cys Pro Ala
225 230 235 240

Pro Glu Leu Leu Gly Gly Pro Ser Val Phe Leu Phe Pro Pro Lys Pro
245 250 255

Lys Asp Thr Leu Met Ile Ser Arg Thr Pro Glu Val Thr Cys Val Val
260 265 270

Val Asp Val Ser His Glu Asp Pro Glu Val Lys Phe Asn Trp Tyr Val
275 280 285

Asp Gly Val Glu Val His Asn Ala Lys Thr Lys Pro Arg Glu Glu Gln
290 295 300

Tyr Asn Ser Thr Tyr Arg Val Val Ser Val Leu Thr Val Leu His Gln
305 310 315 320

Asp Trp Leu Asn Gly Lys Glu Tyr Lys Cys Lys Val Ser Asn Lys Ala
325 330 335

Leu Pro Ala Pro Ile Glu Lys Thr Ile Ser Lys Ala Lys Gly Gln Pro
340 345 350

Arg Glu Pro Gln Val Tyr Thr Leu Pro Pro Ser Arg Asp Glu Leu Thr
355 360 365

Lys Asn Gln Val Ser Leu Thr Cys Leu Val Lys Gly Phe Tyr Pro Ser
370 375 380

Asp Ile Ala Val Glu Trp Glu Ser Asn Gly Gln Pro Glu Asn Asn Tyr
385 390 395 400

Lys Thr Thr Pro Pro Val Leu Asp Ser Asp Gly Ser Phe Phe Leu Tyr
405 410 415

Ser Lys Leu Thr Val Asp Lys Ser Arg Trp Gln Gln Gly Asn Val Phe
420 425 430

Ser Cys Ser Val Met His Glu Ala Leu His Asn His Tyr Thr Gln Lys
435 440 445

Ser Leu Ser Leu Ser Pro
450

<210> 58
<211> 213
<212> PRT
<213> Artificial

<220>
<223> An artificially synthesized peptide sequence

<400> 58

Glu Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr
20 25 30

Leu Ala Trp Tyr Gln Gln Lys Pro Gly Gln Ala Pro Arg Leu Leu Ile
35 40 45

Phe Ala Ala Ser Asn Arg Ala Ala Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Phe Asp Lys Trp Val Thr
85 90 95

Phe Gly Gly Gly Thr Thr Val Glu Ile Arg Arg Thr Val Ala Ala Pro
100 105 110

Ser Val Phe Ile Phe Pro Pro Ser Asp Glu Gln Leu Lys Ser Gly Thr
115 120 125

Ala Ser Val Val Cys Leu Leu Asn Asn Phe Tyr Pro Arg Glu Ala Lys
130 135 140

Val Gln Trp Lys Val Asp Asn Ala Leu Gln Ser Gly Asn Ser Gln Glu
145 150 155 160

Ser Val Thr Glu Gln Asp Ser Lys Asp Ser Thr Tyr Ser Leu Ser Ser
165 170 175

Thr Leu Thr Leu Ser Lys Ala Asp Tyr Glu Lys His Lys Val Tyr Ala
180 185 190

Cys Glu Val Thr His Gln Gly Leu Ser Ser Pro Val Thr Lys Ser Phe
195 200 205

Asn Arg Gly Glu Cys
210

<210> 59
<211> 213
<212> PRT
<213> Artificial

<220>
<223> An artificially synthesized peptide sequence

<400> 59

Glu Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr
20 25 30

Leu Ala Trp Tyr Gln Gln Lys Pro Gly Gln Ala Pro Arg Leu Leu Ile
35 40 45

Phe Asp Ala Ser Asn Arg Ala Ala Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Phe Ala Lys Trp Val Thr

85 90 95

Phe Gly Gly Gly Thr Thr Val Glu Ile Arg Arg Thr Val Ala Ala Pro
100 105 110

Ser Val Phe Ile Phe Pro Pro Ser Asp Glu Gln Leu Lys Ser Gly Thr
115 120 125

Ala Ser Val Val Cys Leu Leu Asn Asn Phe Tyr Pro Arg Glu Ala Lys
130 135 140

Val Gln Trp Lys Val Asp Asn Ala Leu Gln Ser Gly Asn Ser Gln Glu
145 150 155 160

Ser Val Thr Glu Gln Asp Ser Lys Asp Ser Thr Tyr Ser Leu Ser Ser
165 170 175

Thr Leu Thr Leu Ser Lys Ala Asp Tyr Glu Lys His Lys Val Tyr Ala
180 185 190

Cys Glu Val Thr His Gln Gly Leu Ser Ser Pro Val Thr Lys Ser Phe
195 200 205

Asn Arg Gly Glu Cys
210

<210> 60
<211> 449
<212> PRT
<213> Artificial

<220>
<223> An artificially synthesized peptide sequence

<400> 60

Gln Val Gln Leu Gln Glu Ser Gly Pro Gly Leu Val Arg Pro Ser Gln
1 5 10 15

Thr Leu Ser Leu Thr Cys Thr Val Ser Gly Tyr Ser Ile Thr Ser Asp
20 25 30

His Ala Trp Ser Trp Val Arg Gln Pro Pro Gly Arg Gly Leu Glu Trp
35 40 45

Ile Gly Tyr Ile Ser Tyr Ser Gly Ile Thr Thr Tyr Asn Pro Ser Leu
50 55 60

Lys Ser Arg Val Thr Met Leu Arg Asp Thr Ser Lys Asn Gln Phe Ser
65 70 75 80

Leu Arg Leu Ser Ser Val Thr Ala Ala Asp Thr Ala Val Tyr Tyr Cys
85 90 95

Ala Arg Ser Leu Ala Arg Thr Thr Ala Met Asp Tyr Trp Gly Gln Gly
100 105 110

Ser Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
210 215 220

Thr His Thr Cys Pro Pro Cys Pro Ala Pro Glu Leu Leu Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Ile Glu Lys

325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Lys

<210> 61
<211> 214
<212> PRT
<213> Artificial

<220>
<223> An artificially synthesized peptide sequence

<400> 61

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Ile Ser Ser Tyr
20 25 30

Leu Asn Trp Tyr Gln Gln Lys Pro Gly Lys Ala Pro Lys Leu Leu Ile
35 40 45

Tyr Tyr Thr Ser Arg Leu His Ser Gly Val Pro Ser Arg Phe Ser Gly
50 55 60

Ser Gly Ser Gly Thr Asp Phe Thr Phe Thr Ile Ser Ser Leu Gln Pro
65 70 75 80

Glu Asp Ile Ala Thr Tyr Tyr Cys Gln Gln Gly Asn Thr Leu Pro Tyr
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 62
<211> 107
<212> PRT
<213> Artificial

<220>
<223> An artificially synthesized peptide sequence

<400> 62

Glu Thr Thr Leu Thr Gln Ser Pro Ala Phe Met Ser Ala Thr Pro Gly
1 5 10 15

Asp Lys Val Asn Ile Ser Cys Lys Ala Ser Gln Asp Ile Asp Asp Asp
20 25 30

Met Asn Trp Tyr Gln Gln Lys Pro Gly Glu Ala Ala Ile Phe Ile Ile
35 40 45

Gln Glu Ala Thr Thr Leu Val Pro Gly Ile Ser Pro Arg Phe Ser Gly
50 55 60

Ser Gly Tyr Gly Thr Asp Phe Thr Leu Thr Ile Asn Asn Ile Glu Ser
65                  70                  75                  80

Glu Asp Ala Ala Tyr Tyr Phe Cys Leu Gln His Asp Asn Phe Pro Tyr
                85                  90                  95

Thr Phe Gly Gln Gly Thr Lys Leu Glu Ile Lys
            100                 105

<210>  63
<211>  128
<212>  PRT
<213>  Artificial

<220>
<223>  An artificially synthesized peptide sequence

<400>  63

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ile Ile Asn Pro Ser Gly Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Asp Gly Thr Leu Tyr Asp Phe Trp Ser Gly Tyr Tyr Ser Tyr
            100                 105                 110

Asp Ala Phe Asp Ile Trp Gly Gln Gly Thr Met Val Thr Val Ser Ser
        115                 120                 125

<210>  64
<211>  122
<212>  PRT
<213>  Artificial

<220>
<223>  An artificially synthesized peptide sequence

<400> 64

Gln Met Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Leu Asp Thr Gly Pro Tyr Tyr Tyr Gly Met Asp Val Trp
            100                 105                 110

Gly Gln Gly Thr Met Val Thr Val Ser Ser
        115                 120

<210> 65
<211> 124
<212> PRT
<213> Artificial

<220>
<223> An artificially synthesized peptide sequence

<400> 65

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
85 90 95

Ala Arg Asp Ser Pro Val Pro Gly Val Tyr Tyr Tyr Tyr Gly Met Asp
100 105 110

Val Trp Gly Gln Gly Thr Met Val Thr Val Ser Ser
115 120

<210> 66
<211> 119
<212> PRT
<213> Artificial

<220>
<223> An artificially synthesized peptide sequence

<400> 66

Gln Val Gln Leu Val Gln Ser Gly Ala Glu Val Lys Lys Pro Gly Glu
1 5 10 15

Ser Leu Lys Ile Ser Cys Lys Gly Ser Gly Tyr Ser Phe Thr Ser Tyr
20 25 30

Trp Ile Gly Trp Val Arg Gln Met Pro Gly Lys Gly Leu Glu Trp Met
35 40 45

Gly Ile Ile Tyr Pro Gly Asp Ser Asp Thr Arg Tyr Ser Pro Ser Phe
50 55 60

Gln Gly Gln Val Thr Ile Ser Ala Asp Lys Ser Ile Ser Thr Ala Tyr
65 70 75 80

Leu Gln Trp Ser Ser Leu Lys Ala Ser Asp Thr Ala Met Tyr Tyr Cys
85 90 95

Ala Arg His Arg Ala Gly Asp Leu Gly Gly Asp Tyr Trp Gly Gln Gly
100 105 110

Thr Leu Val Thr Val Ser Ser
115

<210> 67
<211> 445
<212> PRT
<213> Artificial

<220>
<223> An artificially synthesized peptide sequence

<400> 67

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Asp Tyr Asn Pro Gln Phe
    50                  55                  60

Gln Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Leu Glu Thr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
```

245 250 255

Pro Glu Val Thr Cys Val Val Val Asp Val Ser Gln Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Gln Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Glu Gly Asn Val Phe Ser Cys Ser Val Met His Glu Ala Leu
420 425 430

His Asn His Tyr Thr Gln Lys Ser Leu Ser Leu Ser Pro
435 440 445

<210> 68
<211> 214
<212> PRT
<213> Artificial

<220>
<223> An artificially synthesized peptide sequence

<400> 68

Glu Thr Thr Leu Thr Gln Ser Pro Ala Phe Met Ser Ala Thr Pro Gly
1 5 10 15

Asp Lys Val Asn Ile Ser Cys Lys Ala Ser Gln Asp Ile Asp Asp Asp
            20                  25                  30

Met Asn Trp Tyr Gln Gln Lys Pro Gly Glu Ala Ala Ile Phe Ile Ile
        35                  40                  45

Gln Glu Ala Thr Thr Leu Val Pro Gly Ile Pro Pro Arg Phe Ser Gly
    50                  55                  60

Ser Gly Tyr Gly Thr Asp Phe Thr Leu Thr Ile Asn Asn Ile Glu Ser
65                  70                  75                  80

Glu Asp Ala Ala Tyr Tyr Phe Cys Leu Gln His Asp Asn Phe Pro Leu
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 69
<211> 214
<212> PRT
<213> Artificial

<220>
<223> An artificially synthesized peptide sequence

<400> 69

```
Glu Thr Thr Leu Thr Gln Ser Pro Ala Phe Met Ser Ala Thr Pro Gly
1               5                   10                  15

Asp Lys Val Asn Ile Ser Cys Lys Ala Ser Gln Asp Ile Glu Asp Asp
            20                  25                  30

Met Asn Trp Tyr Gln Gln Lys Pro Gly Glu Ala Ala Ile Phe Ile Ile
        35                  40                  45

Gln Glu Ala Thr Thr Leu Val Pro Gly Ile Ser Pro Arg Phe Ser Gly
    50                  55                  60

Ser Gly Tyr Gly Thr Asp Phe Thr Leu Thr Ile Asn Asn Ile Glu Ser
65                  70                  75                  80

Glu Asp Ala Ala Tyr Tyr Phe Cys Leu Gln His Asp Asn Phe Pro Leu
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 70
<211> 214
<212> PRT
<213> Artificial

<220>

<223> An artificially synthesized peptide sequence

<400> 70

Glu Thr Thr Leu Thr Gln Ser Pro Ala Phe Met Ser Ala Thr Pro Gly
1               5                   10                  15

Asp Lys Val Thr Ile Ser Cys Lys Ala Ser Gln Asp Ile Asp Asp Asp
            20                  25                  30

Met Asn Trp Tyr Gln Gln Lys Pro Gly Glu Ala Ala Ile Phe Ile Ile
        35                  40                  45

Gln Glu Ala Thr Thr Leu Val Pro Gly Ile Ser Pro Arg Phe Ser Gly
    50                  55                  60

Ser Gly Tyr Gly Thr Asp Phe Thr Leu Thr Ile Asn Asn Ile Glu Ser
65                  70                  75                  80

Glu Asp Ala Ala Tyr Tyr Phe Cys Leu Gln His Asp Asn Phe Pro Tyr
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 71
<211> 107
<212> PRT

<213>  Artificial

<220>
<223>  An artificially synthesized peptide sequence

<400>  71

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Ile Asp Asp Asp
            20                  25                  30

Met Asn Trp Tyr Gln Gln Lys Pro Gly Lys Ala Pro Lys Leu Leu Ile
        35                  40                  45

Tyr Glu Ala Thr Thr Leu Val Pro Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Gln His Asp Asn Phe Pro Tyr
                85                  90                  95

Thr Phe Gly Gln Gly Thr Lys Val Glu Ile Lys
            100                 105

<210>  72
<211>  107
<212>  PRT
<213>  Artificial

<220>
<223>  An artificially synthesized peptide sequence

<400>  72

Asp Ile Val Met Thr Gln Ser Pro Leu Ser Leu Pro Val Thr Pro Gly
1               5                   10                  15

Glu Pro Ala Ser Ile Ser Cys Lys Ala Ser Gln Asp Ile Asp Asp Asp
            20                  25                  30

Met Asn Trp Tyr Leu Gln Lys Pro Gly Gln Ser Pro Gln Leu Leu Ile
        35                  40                  45

Tyr Glu Ala Thr Thr Leu Val Pro Gly Val Pro Asp Arg Phe Ser Gly
    50                  55                  60

Ser Gly Ser Gly Thr Asp Phe Thr Leu Lys Ile Ser Arg Val Glu Ala
65                  70                  75                  80

Glu Asp Val Gly Val Tyr Tyr Cys Leu Gln His Asp Asn Phe Pro Tyr
85 90 95

Thr Phe Gly Gln Gly Thr Lys Leu Glu Ile Lys
100 105

<210> 73
<211> 107
<212> PRT
<213> Artificial

<220>
<223> An artificially synthesized peptide sequence

<400> 73

Glu Ile Val Leu Thr Gln Ser Pro Gly Thr Leu Ser Leu Ser Pro Gly
1 5 10 15

Glu Arg Ala Thr Leu Ser Cys Lys Ala Ser Gln Asp Ile Asp Asp Asp
20 25 30

Met Asn Trp Tyr Gln Gln Lys Pro Gly Gln Ala Pro Arg Leu Leu Ile
35 40 45

Tyr Glu Ala Thr Thr Leu Val Pro Gly Ile Pro Asp Arg Phe Ser Gly
50 55 60

Ser Gly Ser Gly Thr Asp Phe Thr Leu Thr Ile Ser Arg Leu Glu Pro
65 70 75 80

Glu Asp Phe Ala Val Tyr Tyr Cys Leu Gln His Asp Asn Phe Pro Tyr
85 90 95

Thr Phe Gly Gln Gly Thr Lys Val Glu Ile Lys
100 105

<210> 74
<211> 107
<212> PRT
<213> Artificial

<220>
<223> An artificially synthesized peptide sequence

<400> 74

Asp Ile Val Met Thr Gln Ser Pro Asp Ser Leu Ala Val Ser Leu Gly
1 5 10 15

Glu Arg Ala Thr Ile Asn Cys Lys Ala Ser Gln Asp Ile Asp Asp Asp

20 25 30

Met Asn Trp Tyr Gln Gln Lys Pro Gly Gln Pro Pro Lys Leu Leu Ile
35 40 45

Tyr Glu Ala Thr Thr Leu Val Pro Gly Val Pro Asp Arg Phe Ser Gly
50 55 60

Ser Gly Ser Gly Thr Asp Phe Thr Leu Thr Ile Ser Ser Leu Gln Ala
65 70 75 80

Glu Asp Val Ala Val Tyr Tyr Cys Leu Gln His Asp Asn Phe Pro Tyr
85 90 95

Thr Phe Gly Gln Gly Thr Lys Leu Glu Ile Lys
100 105

<210> 75
<211> 214
<212> PRT
<213> Artificial

<220>
<223> An artificially synthesized peptide sequence

<400> 75

Glu Thr Thr Leu Thr Gln Ser Pro Ala Phe Met Ser Ala Thr Pro Gly
1 5 10 15

Asp Lys Val Thr Ile Ser Cys Lys Ala Ser Gln Asp Ile Ala Asp Asp
20 25 30

Met Asn Trp Tyr Gln Gln Lys Pro Gly Glu Ala Ala Ile Phe Ile Ile
35 40 45

Gln Glu Ala Thr Thr Leu Val Pro Gly Ile Ser Pro Arg Phe Ser Gly
50 55 60

Ser Gly Tyr Gly Thr Asp Phe Thr Leu Thr Ile Asn Asn Ile Glu Ser
65 70 75 80

Glu Asp Ala Ala Tyr Tyr Phe Cys Leu Gln His Asp Asn Phe Pro Tyr
85 90 95

Thr Phe Gly Gln Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

```
Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210


<210>  76
<211>  214
<212>  PRT
<213>  Artificial

<220>
<223>  An artificially synthesized peptide sequence

<400>  76

Glu Thr Thr Leu Thr Gln Ser Pro Ala Phe Met Ser Ala Thr Pro Gly
1               5                   10                  15

Asp Lys Val Thr Ile Ser Cys Lys Ala Ser Gln Asp Ile Asp Ala Asp
            20                  25                  30

Met Asn Trp Tyr Gln Gln Lys Pro Gly Glu Ala Ala Ile Phe Ile Ile
        35                  40                  45

Gln Glu Ala Thr Thr Leu Val Pro Gly Ile Ser Pro Arg Phe Ser Gly
    50                  55                  60

Ser Gly Tyr Gly Thr Asp Phe Thr Leu Thr Ile Asn Asn Ile Glu Ser
65                  70                  75                  80

Glu Asp Ala Ala Tyr Tyr Phe Cys Leu Gln His Asp Asn Phe Pro Tyr
                85                  90                  95

Thr Phe Gly Gln Gly Thr Lys Leu Glu Ile Lys Arg Thr Val Ala Ala
            100                 105                 110
```

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 77
<211> 214
<212> PRT
<213> Artificial

<220>
<223> An artificially synthesized peptide sequence

<400> 77

Glu Thr Thr Leu Thr Gln Ser Pro Ala Phe Met Ser Ala Thr Pro Gly
1 5 10 15

Asp Lys Val Thr Ile Ser Cys Lys Ala Ser Gln Asp Ile Asp Asp Ala
20 25 30

Met Asn Trp Tyr Gln Gln Lys Pro Gly Glu Ala Ala Ile Phe Ile Ile
35 40 45

Gln Glu Ala Thr Thr Leu Val Pro Gly Ile Ser Pro Arg Phe Ser Gly
50 55 60

Ser Gly Tyr Gly Thr Asp Phe Thr Leu Thr Ile Asn Asn Ile Glu Ser
65 70 75 80

Glu Asp Ala Ala Tyr Tyr Phe Cys Leu Gln His Asp Asn Phe Pro Tyr
85 90 95

Thr Phe Gly Gln Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 78
<211> 214
<212> PRT
<213> Artificial

<220>
<223> An artificially synthesized peptide sequence

<400> 78

Glu Thr Thr Leu Thr Gln Ser Pro Ala Phe Met Ser Ala Thr Pro Gly
1 5 10 15

Asp Lys Val Thr Ile Ser Cys Lys Ala Ser Gln Asp Ile Asp Asp Asp
20 25 30

Met Asn Trp Tyr Gln Gln Lys Pro Gly Glu Ala Ala Ile Phe Ile Ile
35 40 45

Gln Ala Ala Thr Thr Leu Val Pro Gly Ile Ser Pro Arg Phe Ser Gly
50 55 60

Ser Gly Tyr Gly Thr Asp Phe Thr Leu Thr Ile Asn Asn Ile Glu Ser
65 70 75 80

Glu Asp Ala Ala Tyr Tyr Phe Cys Leu Gln His Asp Asn Phe Pro Tyr

```
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210


<210>  79
<211>  214
<212>  PRT
<213>  Artificial

<220>
<223>  An artificially synthesized peptide sequence

<400>  79

Glu Thr Thr Leu Thr Gln Ser Pro Ala Phe Met Ser Ala Thr Pro Gly
1               5                   10                  15

Asp Lys Val Thr Ile Ser Cys Lys Ala Ser Gln Asp Ile Ala Asp Ala
            20                  25                  30

Met Asn Trp Tyr Gln Gln Lys Pro Gly Glu Ala Ala Ile Phe Ile Ile
        35                  40                  45

Gln Glu Ala Thr Thr Leu Val Pro Gly Ile Ser Pro Arg Phe Ser Gly
    50                  55                  60

Ser Gly Tyr Gly Thr Asp Phe Thr Leu Thr Ile Asn Asn Ile Glu Ser
65                  70                  75                  80
```

Glu Asp Ala Ala Tyr Tyr Phe Cys Leu Gln His Asp Asn Phe Pro Tyr
85 90 95

Thr Phe Gly Gln Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 80
<211> 214
<212> PRT
<213> Artificial

<220>
<223> An artificially synthesized peptide sequence

<400> 80

Glu Thr Thr Leu Thr Gln Ser Pro Ala Phe Met Ser Ala Thr Pro Gly
1 5 10 15

Asp Lys Val Thr Ile Ser Cys Lys Ala Ser Gln Asp Ile Ala Asp Asp
20 25 30

Met Asn Trp Tyr Gln Gln Lys Pro Gly Glu Ala Ala Ile Phe Ile Ile
35 40 45

Gln Ala Ala Thr Thr Leu Val Pro Gly Ile Ser Pro Arg Phe Ser Gly
50 55 60

Ser Gly Tyr Gly Thr Asp Phe Thr Leu Thr Ile Asn Asn Ile Glu Ser
65 70 75 80

Glu Asp Ala Ala Tyr Tyr Phe Cys Leu Gln His Asp Asn Phe Pro Tyr
85 90 95

Thr Phe Gly Gln Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 81
<211> 214
<212> PRT
<213> Artificial

<220>
<223> An artificially synthesized peptide sequence

<400> 81

Glu Thr Thr Leu Thr Gln Ser Pro Ala Phe Met Ser Ala Thr Pro Gly
1 5 10 15

Asp Lys Val Thr Ile Ser Cys Lys Ala Ser Gln Asp Ile Ala Asp Ala
20 25 30

Met Asn Trp Tyr Gln Gln Lys Pro Gly Glu Ala Ala Ile Phe Ile Ile
35 40 45

Gln Ala Ala Thr Thr Leu Val Pro Gly Ile Ser Pro Arg Phe Ser Gly
50 55 60

Ser Gly Tyr Gly Thr Asp Phe Thr Leu Thr Ile Asn Asn Ile Glu Ser
65 70 75 80

Glu Asp Ala Ala Tyr Tyr Phe Cys Leu Gln His Asp Asn Phe Pro Tyr
85 90 95

Thr Phe Gly Gln Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 82
<211> 214
<212> PRT
<213> Artificial

<220>
<223> An artificially synthesized peptide sequence

<400> 82

Glu Thr Thr Leu Thr Gln Ser Pro Ala Phe Met Ser Ala Thr Pro Gly
1 5 10 15

Asp Lys Val Thr Ile Ser Cys Lys Ala Ser Gln Asp Ile Asp Asp Asp
20 25 30

Met Asn Trp Tyr Gln Gln Lys Pro Gly Glu Ala Ala Ile Phe Ile Ile

35 40 45

Gln Glu Ala Thr Thr Leu Val Pro Gly Ile Ser Pro Arg Phe Ser Gly
50 55 60

Ser Gly Tyr Gly Thr Asp Phe Thr Leu Thr Ile Asn Asn Ile Glu Ser
65 70 75 80

Glu Asp Ala Ala Tyr Tyr Phe Cys Leu Gln His Ala Asn Phe Pro Tyr
85 90 95

Thr Phe Gly Gln Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 83
<211> 214
<212> PRT
<213> Artificial

<220>
<223> An artificially synthesized peptide sequence

<400> 83

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Asp Asp Asp
20 25 30

```
Leu Asn Trp Tyr Gln Gln Lys Pro Gly Lys Ala Pro Lys Leu Leu Ile
        35                  40                  45

Tyr Glu Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Asp Ser Thr Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210


<210>  84
<211>  214
<212>  PRT
<213>  Artificial

<220>
<223>  An artificially synthesized peptide sequence

<400>  84

Asp Ile Gln Met Thr Gln Ser Pro Ser Ser Leu Ser Ala Ser Val Gly
1               5                   10                  15
```

Asp Arg Val Thr Ile Thr Cys Arg Ala Ser Gln Ser Ile Ser Ser Tyr
20 25 30

Leu Asn Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Ser Tyr Ser Thr Pro Tyr
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 85
<211> 214
<212> PRT
<213> Artificial

<220>
<223> An artificially synthesized peptide sequence

<400> 85

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ala Asp Asp
            20                  25                  30

Leu Asn Trp Tyr Gln Gln Lys Pro Gly Lys Ala Pro Lys Leu Leu Ile
        35                  40                  45

Tyr Glu Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Asp Ser Thr Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 86
<211> 214
<212> PRT
<213> Artificial

<220>
<223> An artificially synthesized peptide sequence

<400> 86

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Asp Ala Asp
            20                  25                  30

Leu Asn Trp Tyr Gln Gln Lys Pro Gly Lys Ala Pro Lys Leu Leu Ile
        35                  40                  45

Tyr Glu Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Asp Ser Thr Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 87
<211> 214
<212> PRT
<213> Artificial

<220>
<223>  An artificially synthesized peptide sequence

<400>  87

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Asp Asp Ala
            20                  25                  30

Leu Asn Trp Tyr Gln Gln Lys Pro Gly Lys Ala Pro Lys Leu Leu Ile
        35                  40                  45

Tyr Glu Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Asp Ser Thr Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  88

<211> 214
<212> PRT
<213> Artificial

<220>
<223> An artificially synthesized peptide sequence

<400> 88

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Asp Asp
            20                  25                  30

Leu Asn Trp Tyr Gln Gln Lys Pro Gly Lys Ala Pro Lys Leu Leu Ile
        35                  40                  45

Tyr Glu Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Asp Ser Thr Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 89
<211> 456
<212> PRT
<213> Artificial

<220>
<223> An artificially synthesized peptide sequence

<400> 89

```
Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Ser
            20                  25                  30

Ser Tyr Tyr Trp Gly Trp Ile Arg Gln Pro Pro Gly Lys Gly Leu Glu
        35                  40                  45

Trp Ile Gly Ser Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Val Pro Pro Tyr Ser Ser Ser Ser Tyr Tyr Tyr Tyr Tyr
            100                 105                 110

Tyr Tyr Met Asp Val Trp Gly Lys Gly Thr Thr Val Thr Val Ser Ser
        115                 120                 125

Ala Ser Thr Lys Gly Pro Ser Val Phe Pro Leu Ala Pro Ser Ser Lys
    130                 135                 140

Ser Thr Ser Gly Gly Thr Ala Ala Leu Gly Cys Leu Val Lys Asp Tyr
145                 150                 155                 160

Phe Pro Glu Pro Val Thr Val Ser Trp Asn Ser Gly Ala Leu Thr Ser
                165                 170                 175

Gly Val His Thr Phe Pro Ala Val Leu Gln Ser Ser Gly Leu Tyr Ser
            180                 185                 190

Leu Ser Ser Val Val Thr Val Pro Ser Ser Ser Leu Gly Thr Gln Thr
        195                 200                 205
```

Tyr Ile Cys Asn Val Asn His Lys Pro Ser Asn Thr Lys Val Asp Lys
    210                 215                 220

Lys Val Glu Pro Lys Ser Cys Asp Lys Thr His Thr Cys Pro Pro Cys
225                 230                 235                 240

Pro Ala Pro Glu Leu Leu Gly Gly Pro Ser Val Phe Leu Phe Pro Pro
                245                 250                 255

Lys Pro Lys Asp Thr Leu Met Ile Ser Arg Thr Pro Glu Val Thr Cys
            260                 265                 270

Val Val Val Asp Val Ser His Glu Asp Pro Glu Val Lys Phe Asn Trp
        275                 280                 285

Tyr Val Asp Gly Val Glu Val His Asn Ala Lys Thr Lys Pro Arg Glu
    290                 295                 300

Glu Gln Tyr Asn Ser Thr Tyr Arg Val Val Ser Val Leu Thr Val Leu
305                 310                 315                 320

His Gln Asp Trp Leu Asn Gly Lys Glu Tyr Lys Cys Lys Val Ser Asn
                325                 330                 335

Lys Ala Leu Pro Ala Pro Ile Glu Lys Thr Ile Ser Lys Ala Lys Gly
            340                 345                 350

Gln Pro Arg Glu Pro Gln Val Tyr Thr Leu Pro Pro Ser Arg Asp Glu
        355                 360                 365

Leu Thr Lys Asn Gln Val Ser Leu Thr Cys Leu Val Lys Gly Phe Tyr
    370                 375                 380

Pro Ser Asp Ile Ala Val Glu Trp Glu Ser Asn Gly Gln Pro Glu Asn
385                 390                 395                 400

Asn Tyr Lys Thr Thr Pro Pro Val Leu Asp Ser Asp Gly Ser Phe Phe
                405                 410                 415

Leu Tyr Ser Lys Leu Thr Val Asp Lys Ser Arg Trp Gln Gln Gly Asn
            420                 425                 430

Val Phe Ser Cys Ser Val Met His Glu Ala Leu His Asn His Tyr Thr
        435                 440                 445

Gln Lys Ser Leu Ser Leu Ser Pro
    450                 455

<210> 90
<211> 452
<212> PRT
<213> Artificial

<220>
<223> An artificially synthesized peptide sequence

<400> 90

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala Pro Gly Ile Gln Leu Trp Leu Arg Pro Ser Tyr Phe Asp
            100                 105                 110

Tyr Trp Gly Gln Gly Thr Leu Val Thr Val Ser Ser Ala Ser Thr Lys
        115                 120                 125

Gly Pro Ser Val Phe Pro Leu Ala Pro Ser Ser Lys Ser Thr Ser Gly
    130                 135                 140

Gly Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val
            180                 185                 190

Val Thr Val Pro Ser Ser Ser Leu Gly Thr Gln Thr Tyr Ile Cys Asn
        195                 200                 205

```
Val Asn His Lys Pro Ser Asn Thr Lys Val Asp Lys Lys Val Glu Pro
    210                 215                 220

Lys Ser Cys Asp Lys Thr His Thr Cys Pro Pro Cys Pro Ala Pro Glu
225                 230                 235                 240

Leu Leu Gly Gly Pro Ser Val Phe Leu Phe Pro Pro Lys Pro Lys Asp
                245                 250                 255

Thr Leu Met Ile Ser Arg Thr Pro Glu Val Thr Cys Val Val Val Asp
            260                 265                 270

Val Ser His Glu Asp Pro Glu Val Lys Phe Asn Trp Tyr Val Asp Gly
        275                 280                 285

Val Glu Val His Asn Ala Lys Thr Lys Pro Arg Glu Glu Gln Tyr Asn
    290                 295                 300

Ser Thr Tyr Arg Val Val Ser Val Leu Thr Val Leu His Gln Asp Trp
305                 310                 315                 320

Leu Asn Gly Lys Glu Tyr Lys Cys Lys Val Ser Asn Lys Ala Leu Pro
                325                 330                 335

Ala Pro Ile Glu Lys Thr Ile Ser Lys Ala Lys Gly Gln Pro Arg Glu
            340                 345                 350

Pro Gln Val Tyr Thr Leu Pro Pro Ser Arg Asp Glu Leu Thr Lys Asn
        355                 360                 365

Gln Val Ser Leu Thr Cys Leu Val Lys Gly Phe Tyr Pro Ser Asp Ile
    370                 375                 380

Ala Val Glu Trp Glu Ser Asn Gly Gln Pro Glu Asn Asn Tyr Lys Thr
385                 390                 395                 400

Thr Pro Pro Val Leu Asp Ser Asp Gly Ser Phe Phe Leu Tyr Ser Lys
                405                 410                 415

Leu Thr Val Asp Lys Ser Arg Trp Gln Gln Gly Asn Val Phe Ser Cys
            420                 425                 430

Ser Val Met His Glu Ala Leu His Asn His Tyr Thr Gln Lys Ser Leu
        435                 440                 445

Ser Leu Ser Pro
    450
```

<210> 91
<211> 447
<212> PRT
<213> Artificial

<220>
<223> An artificially synthesized peptide sequence

<400> 91

Gln Val Gln Leu Gln Glu Ser Gly Pro Gly Leu Val Lys Pro Trp Glu
1 5 10 15

Thr Leu Ser Leu Thr Cys Thr Val Ala Gly Asp Ser Ile Lys Tyr Ser
20 25 30

Ser Asp Tyr Trp Gly Trp Val Arg Gln Ser Pro Gly Lys Gly Leu Glu
35 40 45

Trp Ile Gly Ser Ser Tyr Leu Ser Gly Thr Thr Gln Tyr Asn Pro Ser
50 55 60

Leu Lys Ser Arg Val Thr Met Ser Val Asp Thr Ser Lys Asn Gln Phe
65 70 75 80

Ser Leu Arg Leu Ser Ser Val Thr Ala Ala Asp Thr Ala Val Tyr Tyr
85 90 95

Cys Ala Arg His Arg Gly Pro Thr Gly Val Asp Gln Trp Gly Gln Gly
100 105 110

Thr Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  92
<211>  449

<212> PRT
<213> Artificial

<220>
<223> An artificially synthesized peptide sequence

<400> 92

Glu Val Gln Leu Val Gln Ser Gly Gly Gly Leu Val Gln Pro Gly Arg
1               5                   10                  15

Ser Leu Thr Leu Ser Cys Val Gly Tyr Gly Phe Thr Phe His Glu Asn
            20                  25                  30

Asp Met His Trp Leu Arg Gln Pro Leu Gly Lys Gly Leu Glu Trp Val
        35                  40                  45

Ser His Ile Gly Trp Asn Asn Asn Arg Val Ala Tyr Ala Asp Ser Val
    50                  55                  60

Lys Gly Arg Phe Ala Val Ser Arg Asp Asn Ala Lys Asn Ser Leu Phe
65                  70                  75                  80

Leu His Met Asn Ser Leu Arg Pro Asp Asp Thr Ala Leu Tyr Tyr Cys
                85                  90                  95

Ala Lys Asp Leu Gly Asn Pro Ile Tyr Asp Val Phe Asp Val Trp Gly
            100                 105                 110

Gln Gly Thr Met Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro

<210> 93
<211> 452

<212> PRT
<213> Artificial

<220>
<223> An artificially synthesized peptide sequence

<400> 93

```
Gln Pro Ala Leu Ala Gln Met Gln Leu Val Glu Ser Gly Gly Gly Leu
1               5                   10                  15

Val Gln Pro Gly Arg Ser Leu Arg Leu Ser Cys Ala Ala Ser Gly Phe
            20                  25                  30

Thr Phe Asp Asp Tyr Ala Met His Trp Val Arg Gln Ala Pro Gly Lys
        35                  40                  45

Gly Leu Glu Trp Val Ser Gly Ile Ser Trp Asn Ser Gly Ser Ile Gly
    50                  55                  60

Tyr Ala Asp Ser Val Lys Gly Arg Phe Thr Ile Ser Arg Asp Asn Ala
65                  70                  75                  80

Lys Asn Ser Leu Tyr Leu Gln Met Asn Ser Leu Arg Ala Glu Asp Thr
                85                  90                  95

Ala Leu Tyr Tyr Cys Ala Arg Glu Gly Val Leu Gly Asp Ala Phe Asp
            100                 105                 110

Ile Trp Gly Gln Gly Thr Met Val Thr Val Ser Ser Ala Ser Thr Lys
        115                 120                 125

Gly Pro Ser Val Phe Pro Leu Ala Pro Ser Ser Lys Ser Thr Ser Gly
    130                 135                 140

Gly Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val
            180                 185                 190

Val Thr Val Pro Ser Ser Ser Leu Gly Thr Gln Thr Tyr Ile Cys Asn
        195                 200                 205

Val Asn His Lys Pro Ser Asn Thr Lys Val Asp Lys Lys Val Glu Pro
    210                 215                 220
```

Lys Ser Cys Asp Lys Thr His Thr Cys Pro Pro Cys Pro Ala Pro Glu
225 230 235 240

Leu Leu Gly Gly Pro Ser Val Phe Leu Phe Pro Pro Lys Pro Lys Asp
245 250 255

Thr Leu Met Ile Ser Arg Thr Pro Glu Val Thr Cys Val Val Val Asp
260 265 270

Val Ser His Glu Asp Pro Glu Val Lys Phe Asn Trp Tyr Val Asp Gly
275 280 285

Val Glu Val His Asn Ala Lys Thr Lys Pro Arg Glu Glu Gln Tyr Asn
290 295 300

Ser Thr Tyr Arg Val Val Ser Val Leu Thr Val Leu His Gln Asp Trp
305 310 315 320

Leu Asn Gly Lys Glu Tyr Lys Cys Lys Val Ser Asn Lys Ala Leu Pro
325 330 335

Ala Pro Ile Glu Lys Thr Ile Ser Lys Ala Lys Gly Gln Pro Arg Glu
340 345 350

Pro Gln Val Tyr Thr Leu Pro Pro Ser Arg Asp Glu Leu Thr Lys Asn
355 360 365

Gln Val Ser Leu Thr Cys Leu Val Lys Gly Phe Tyr Pro Ser Asp Ile
370 375 380

Ala Val Glu Trp Glu Ser Asn Gly Gln Pro Glu Asn Asn Tyr Lys Thr
385 390 395 400

Thr Pro Pro Val Leu Asp Ser Asp Gly Ser Phe Phe Leu Tyr Ser Lys
405 410 415

Leu Thr Val Asp Lys Ser Arg Trp Gln Gln Gly Asn Val Phe Ser Cys
420 425 430

Ser Val Met His Glu Ala Leu His Asn His Tyr Thr Gln Lys Ser Leu
435 440 445

Ser Leu Ser Pro
450

<210> 94
<211> 454

<212> PRT
<213> Artificial

<220>
<223> An artificially synthesized peptide sequence

<400> 94

```
Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Arg Val Arg Ser Gly Ser Tyr Tyr Tyr Tyr Gly
            100                 105                 110

Met Asp Val Trp Gly Gln Gly Thr Thr Val Thr Val Ser Ser Ala Ser
        115                 120                 125

Thr Lys Gly Pro Ser Val Phe Pro Leu Ala Pro Ser Ser Lys Ser Thr
    130                 135                 140

Ser Gly Gly Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
            180                 185                 190

Ser Val Val Thr Val Pro Ser Ser Ser Leu Gly Thr Gln Thr Tyr Ile
        195                 200                 205

Cys Asn Val Asn His Lys Pro Ser Asn Thr Lys Val Asp Lys Lys Val
    210                 215                 220
```

Glu Pro Lys Ser Cys Asp Lys Thr His Thr Cys Pro Pro Cys Pro Ala
225                 230                 235                 240

Pro Glu Leu Leu Gly Gly Pro Ser Val Phe Leu Phe Pro Pro Lys Pro
                245                 250                 255

Lys Asp Thr Leu Met Ile Ser Arg Thr Pro Glu Val Thr Cys Val Val
            260                 265                 270

Val Asp Val Ser His Glu Asp Pro Glu Val Lys Phe Asn Trp Tyr Val
        275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Pro Ala Pro Ile Glu Lys Thr Ile Ser Lys Ala Lys Gly Gln Pro
            340                 345                 350

Arg Glu Pro Gln Val Tyr Thr Leu Pro Pro Ser Arg Asp Glu Leu Thr
        355                 360                 365

Lys Asn Gln Val Ser Leu Thr Cys Leu Val Lys Gly Phe Tyr Pro Ser
    370                 375                 380

Asp Ile Ala Val Glu Trp Glu Ser Asn Gly Gln Pro Glu Asn Asn Tyr
385                 390                 395                 400

Lys Thr Thr Pro Pro Val Leu Asp Ser Asp Gly Ser Phe Phe Leu Tyr
                405                 410                 415

Ser Lys Leu Thr Val Asp Lys Ser Arg Trp Gln Gln Gly Asn Val Phe
            420                 425                 430

Ser Cys Ser Val Met His Glu Ala Leu His Asn His Tyr Thr Gln Lys
        435                 440                 445

Ser Leu Ser Leu Ser Pro
    450

<210> 95
<211> 450

<212>  PRT
<213>  Artificial

<220>
<223>  An artificially synthesized peptide sequence

<400>  95

```
Gln Val Gln Leu Gln Glu Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Ile Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Lys Asp Pro Arg Val Trp Ala Phe Asp Ile Trp
            100                 105                 110

Gly Gln Gly Thr Met Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220
```

Cys Asp Lys Thr His Thr Cys Pro Pro Cys Pro Ala Pro Glu Leu Leu
225 230 235 240

Gly Gly Pro Ser Val Phe Leu Phe Pro Pro Lys Pro Lys Asp Thr Leu
245 250 255

Met Ile Ser Arg Thr Pro Glu Val Thr Cys Val Val Val Asp Val Ser
260 265 270

His Glu Asp Pro Glu Val Lys Phe Asn Trp Tyr Val Asp Gly Val Glu
275 280 285

Val His Asn Ala Lys Thr Lys Pro Arg Glu Glu Gln Tyr Asn Ser Thr
290 295 300

Tyr Arg Val Val Ser Val Leu Thr Val Leu His Gln Asp Trp Leu Asn
305 310 315 320

Gly Lys Glu Tyr Lys Cys Lys Val Ser Asn Lys Ala Leu Pro Ala Pro
325 330 335

Ile Glu Lys Thr Ile Ser Lys Ala Lys Gly Gln Pro Arg Glu Pro Gln
340 345 350

Val Tyr Thr Leu Pro Pro Ser Arg Asp Glu Leu Thr Lys Asn Gln Val
355 360 365

Ser Leu Thr Cys Leu Val Lys Gly Phe Tyr Pro Ser Asp Ile Ala Val
370 375 380

Glu Trp Glu Ser Asn Gly Gln Pro Glu Asn Asn Tyr Lys Thr Thr Pro
385 390 395 400

Pro Val Leu Asp Ser Asp Gly Ser Phe Phe Leu Tyr Ser Lys Leu Thr
405 410 415

Val Asp Lys Ser Arg Trp Gln Gln Gly Asn Val Phe Ser Cys Ser Val
420 425 430

Met His Glu Ala Leu His Asn His Tyr Thr Gln Lys Ser Leu Ser Leu
435 440 445

Ser Pro
450

<210> 96
<211> 449

<212> PRT
<213> Artificial

<220>
<223> An artificially synthesized peptide sequence

<400> 96

```
Glu Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Arg Ile Ile Pro Val Leu Ala Ile Ala Asn Tyr Ala Gln Lys Phe
    50                  55                  60

Gln Gly Arg Val Thr Ile Thr Ala Asp Lys Ser Thr His Thr Ala Tyr
65                  70                  75                  80

Met Glu Leu Ser Ser Leu Arg Ser Gly Asp Thr Ala Val Tyr Tyr Cys
                85                  90                  95

Ala Arg Asp Ser Gly Tyr Ser Ala Gly Tyr Gly Gly Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220
```

Asp Lys Thr His Thr Cys Pro Pro Cys Pro Ala Pro Glu Leu Leu Gly
225 230 235 240

Gly Pro Ser Val Phe Leu Phe Pro Pro Lys Pro Lys Asp Thr Leu Met
245 250 255

Ile Ser Arg Thr Pro Glu Val Thr Cys Val Val Val Asp Val Ser His
260 265 270

Glu Asp Pro Glu Val Lys Phe Asn Trp Tyr Val Asp Gly Val Glu Val
275 280 285

His Asn Ala Lys Thr Lys Pro Arg Glu Glu Gln Tyr Asn Ser Thr Tyr
290 295 300

Arg Val Val Ser Val Leu Thr Val Leu His Gln Asp Trp Leu Asn Gly
305 310 315 320

Lys Glu Tyr Lys Cys Lys Val Ser Asn Lys Ala Leu Pro Ala Pro Ile
325 330 335

Glu Lys Thr Ile Ser Lys Ala Lys Gly Gln Pro Arg Glu Pro Gln Val
340 345 350

Tyr Thr Leu Pro Pro Ser Arg Asp Glu Leu Thr Lys Asn Gln Val Ser
355 360 365

Leu Thr Cys Leu Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Tyr Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro

<210> 97
<211> 447

<212> PRT
<213> Artificial

<220>
<223> An artificially synthesized peptide sequence

<400> 97

Glu Val Gln Leu Val Glu Ser Gly Gly Asp Leu Val Gln Pro Gly Gly
1 5 10 15

Ser Leu Arg Leu Ser Cys Ala Ala Ser Gly Phe Thr Phe Lys Thr Tyr
20 25 30

Trp Met Asn Trp Val Arg Gln Ala Pro Gly Lys Gly Leu Glu Trp Val
35 40 45

Ala Asn Ile Arg Ala Asp Gly Gly Gln Met Tyr Tyr Ala Asp Ser Val
50 55 60

Lys Gly Arg Phe Thr Ile Ser Arg Asp Asn Ala Lys Asp Ser Leu Tyr
65 70 75 80

Leu Gln Met Ile Ser Leu Arg Pro Glu Asp Thr Ala Leu Tyr Tyr Cys
85 90 95

Ala Arg Asp Pro Phe Ala Ser Gly Gly Leu Asp Gln Trp Gly Gln Gly
100 105 110

Thr Met Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
210 215 220

Thr His Thr Cys Pro Pro Cys Pro Ala Pro Glu Leu Leu Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro
435 440 445

<210> 98
<211> 450
<212> PRT
<213> Artificial

<220>

<223> An artificially synthesized peptide sequence

<400> 98

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met Glu Trp Val Arg Gln Ala Pro Gly Lys Gly Leu Glu Trp Val
        35                  40                  45

Ala Leu Ile Ser His Asp Gly Asn Asn Lys Tyr Tyr Ala Asp Ser Val
    50                  55                  60

Glu Gly Arg Phe Thr Ile Ser Arg Asp Asn Ser Arg Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Val Arg Tyr Phe Asp Ser Tyr Gly Met Asp Val Trp
            100                 105                 110

Gly His Gly Thr Thr Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

```
Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro
    450
```

<210> 99
<211> 449
<212> PRT
<213> Artificial

<220>

<223> An artificially synthesized peptide sequence

<400> 99

Glu Val Gln Leu Val Gln Ser Gly Ala Glu Val Lys Lys Pro Gly Glu
1 5 10 15

Ser Leu Arg Ile Ser Cys Lys Gly Ser Gly Tyr Ser Phe Thr Ser Phe
20 25 30

Trp Ile Asn Trp Val Arg Gln Met Pro Gly Lys Gly Leu Glu Trp Met
35 40 45

Gly Asn Ile Asp Pro Ser Asp Ser Tyr Thr Asn Tyr Ser Pro Ser Phe
50 55 60

Gln Gly His Val Ala Ile Ser Ala Asp Lys Ser Ile Ser Thr Ala Tyr
65 70 75 80

Leu His Trp Asn Ser Leu Lys Ala Ser Asp Thr Ala Met Tyr Tyr Cys
85 90 95

Ala Arg His Arg Tyr Leu Gly Gln Leu Ala Pro Phe Asp Pro Trp Gly
100 105 110

Gln Gly Thr Leu Val Thr Val Ser Ser Ala Ser Thr Lys Gly Pro Ser
115 120 125

Val Phe Pro Leu Ala Pro Ser Ser Lys Ser Thr Ser Gly Gly Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Ser Leu Gly Thr Gln Thr Tyr Ile Cys Asn Val Asn His
195 200 205

Lys Pro Ser Asn Thr Lys Val Asp Lys Lys Val Glu Pro Lys Ser Cys
210 215 220

Asp Lys Thr His Thr Cys Pro Pro Cys Pro Ala Pro Glu Leu Leu Gly
225 230 235 240

Gly Pro Ser Val Phe Leu Phe Pro Pro Lys Pro Lys Asp Thr Leu Met
245 250 255

Ile Ser Arg Thr Pro Glu Val Thr Cys Val Val Val Asp Val Ser His
260 265 270

Glu Asp Pro Glu Val Lys Phe Asn Trp Tyr Val Asp Gly Val Glu Val
275 280 285

His Asn Ala Lys Thr Lys Pro Arg Glu Glu Gln Tyr Asn Ser Thr Tyr
290 295 300

Arg Val Val Ser Val Leu Thr Val Leu His Gln Asp Trp Leu Asn Gly
305 310 315 320

Lys Glu Tyr Lys Cys Lys Val Ser Asn Lys Ala Leu Pro Ala Pro Ile
325 330 335

Glu Lys Thr Ile Ser Lys Ala Lys Gly Gln Pro Arg Glu Pro Gln Val
340 345 350

Tyr Thr Leu Pro Pro Ser Arg Asp Glu Leu Thr Lys Asn Gln Val Ser
355 360 365

Leu Thr Cys Leu Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Tyr Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro

<210> 100
<211> 214
<212> PRT
<213> Artificial

<220>

<223> An artificially synthesized peptide sequence

<400> 100

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Glu Asp Asp
20 25 30

Leu Ala Trp Tyr Gln Gln Lys Pro Gly Lys Ala Pro Lys Leu Leu Ile
35 40 45

Tyr Glu Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Arg Asp Asn Tyr Pro Leu
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 101
<211> 214
<212> PRT

<213> Artificial

<220>
<223> An artificially synthesized peptide sequence

<400> 101

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Glu Asp Asp
            20                  25                  30

Leu Ala Trp Tyr Gln Gln Lys Pro Gly Lys Ala Pro Lys Leu Leu Ile
        35                  40                  45

Tyr His Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Asp Ser Ser Pro Leu
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 102
<211> 214
<212> PRT
<213> Artificial

<220>
<223> An artificially synthesized peptide sequence

<400> 102

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Glu Asp Asp
            20                  25                  30

Leu Ala Trp Tyr Gln Gln Lys Pro Gly Lys Ala Pro Lys Leu Leu Ile
        35                  40                  45

Tyr Glu Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Ser Asn Tyr Pro Leu
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
```

210

<210> 103
<211> 214
<212> PRT
<213> Artificial

<220>
<223> An artificially synthesized peptide sequence

<400> 103

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Glu Asp Asp
            20                  25                  30

Leu Ala Trp Tyr Gln Gln Lys Pro Gly Lys Ala Pro Lys Leu Leu Ile
        35                  40                  45

Tyr Glu Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Asp Gly Tyr Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205
```

Phe Asn Arg Gly Glu Cys
210

<210> 104
<211> 214
<212> PRT
<213> Artificial

<220>
<223> An artificially synthesized peptide sequence

<400> 104

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Glu Asp Asp
20 25 30

Leu Asn Trp Tyr Gln Gln Lys Pro Gly Lys Ala Pro Lys Leu Leu Ile
35 40 45

Tyr Glu Ala Ser Asn Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln His Ser Ser Ser Pro Tyr
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 105
<211> 214
<212> PRT
<213> Artificial

<220>
<223> An artificially synthesized peptide sequence

<400> 105

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Glu Asp Asp
20 25 30

Leu Ala Trp Tyr Gln Gln Lys Pro Gly Lys Ala Pro Lys Leu Leu Ile
35 40 45

Tyr Glu Ala Ser Asn Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Asp Ser Tyr Pro Tyr
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 106
<211> 214
<212> PRT
<213> Artificial

<220>
<223> An artificially synthesized peptide sequence

<400> 106

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Asp Asp
20 25 30

Leu Ala Trp Tyr Gln Gln Lys Pro Gly Lys Ala Pro Lys Leu Leu Ile
35 40 45

Tyr His Ala Ser His Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Asp Asn Ser Pro Leu
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser

165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 107
<211> 214
<212> PRT
<213> Artificial

<220>
<223> An artificially synthesized peptide sequence

<400> 107

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Asp Asp
20 25 30

Leu Ala Trp Tyr Gln Gln Lys Pro Gly Lys Ala Pro Lys Leu Leu Ile
35 40 45

Tyr His Ala Ser Asn Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Asp Asn Tyr Pro Tyr
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 108
<211> 214
<212> PRT
<213> Artificial

<220>
<223> An artificially synthesized peptide sequence

<400> 108

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Glu Asp Asp
20 25 30

Leu Asn Trp Tyr Gln Gln Lys Pro Gly Lys Ala Pro Lys Leu Leu Ile
35 40 45

Tyr Glu Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Arg Asp Ser Ser Pro Tyr
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

```
Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  109
<211>  214
<212>  PRT
<213>  Artificial

<220>
<223>  An artificially synthesized peptide sequence

<400>  109

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Glu Asp Asp
            20                  25                  30

Leu Asn Trp Tyr Gln Gln Lys Pro Gly Lys Ala Pro Lys Leu Leu Ile
        35                  40                  45

Tyr Glu Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Asn Asp Tyr Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125
```

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  110
<211>  214
<212>  PRT
<213>  Artificial

<220>
<223>  An artificially synthesized peptide sequence

<400>  110

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Glu Asp Asp
            20                  25                  30

Leu Ala Trp Tyr Gln Gln Lys Pro Gly Lys Ala Pro Lys Leu Leu Ile
        35                  40                  45

Tyr Glu Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln His Asp Asn Tyr Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly

```
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  111
<211>  453
<212>  PRT
<213>  Artificial

<220>
<223>  An artificially synthesized peptide sequence

<400>  111

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ala Ala Phe Leu Glu Trp Pro Ile Trp Gly Ser Glu Tyr Phe
            100                 105                 110
```

Gln His Trp Gly Gln Gly Thr Leu Val Thr Val Ser Ser Ala Ser Thr
115 120 125

Lys Gly Pro Ser Val Phe Pro Leu Ala Pro Ser Ser Lys Ser Thr Ser
130 135 140

Gly Gly Thr Ala Ala Leu Gly Cys Leu Val Lys Asp Tyr Phe Pro Glu
145 150 155 160

Pro Val Thr Val Ser Trp Asn Ser Gly Ala Leu Thr Ser Gly Val His
165 170 175

Thr Phe Pro Ala Val Leu Gln Ser Ser Gly Leu Tyr Ser Leu Ser Ser
180 185 190

Val Val Thr Val Pro Ser Ser Ser Leu Gly Thr Gln Thr Tyr Ile Cys
195 200 205

Asn Val Asn His Lys Pro Ser Asn Thr Lys Val Asp Lys Lys Val Glu
210 215 220

Pro Lys Ser Cys Asp Lys Thr His Thr Cys Pro Pro Cys Pro Ala Pro
225 230 235 240

Glu Leu Leu Gly Gly Pro Ser Val Phe Leu Phe Pro Pro Lys Pro Lys
245 250 255

Asp Thr Leu Met Ile Ser Arg Thr Pro Glu Val Thr Cys Val Val Val
260 265 270

Asp Val Ser His Glu Asp Pro Glu Val Lys Phe Asn Trp Tyr Val Asp
275 280 285

Gly Val Glu Val His Asn Ala Lys Thr Lys Pro Arg Glu Glu Gln Tyr
290 295 300

Asn Ser Thr Tyr Arg Val Val Ser Val Leu Thr Val Leu His Gln Asp
305 310 315 320

Trp Leu Asn Gly Lys Glu Tyr Lys Cys Lys Val Ser Asn Lys Ala Leu
325 330 335

Pro Ala Pro Ile Glu Lys Thr Ile Ser Lys Ala Lys Gly Gln Pro Arg
340 345 350

Glu Pro Gln Val Tyr Thr Leu Pro Pro Ser Arg Asp Glu Leu Thr Lys

355 360 365

Asn Gln Val Ser Leu Thr Cys Leu Val Lys Gly Phe Tyr Pro Ser Asp
370 375 380

Ile Ala Val Glu Trp Glu Ser Asn Gly Gln Pro Glu Asn Asn Tyr Lys
385 390 395 400

Thr Thr Pro Pro Val Leu Asp Ser Asp Gly Ser Phe Phe Leu Tyr Ser
405 410 415

Lys Leu Thr Val Asp Lys Ser Arg Trp Gln Gln Gly Asn Val Phe Ser
420 425 430

Cys Ser Val Met His Glu Ala Leu His Asn His Tyr Thr Gln Lys Ser
435 440 445

Leu Ser Leu Ser Pro
450

<210> 112
<211> 214
<212> PRT
<213> Artificial

<220>
<223> An artificially synthesized peptide sequence

<400> 112

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Glu Asp Asp
20 25 30

Leu Ala Trp Tyr Gln Gln Lys Pro Gly Lys Ala Pro Lys Leu Leu Ile
35 40 45

Tyr His Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Arg Asp Ser Tyr Pro Leu
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 113
<211> 451
<212> PRT
<213> Artificial

<220>
<223> An artificially synthesized peptide sequence

<400> 113

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Gly Ile Ser Trp Val Arg Gln Ala Pro Gly Gln Gly Leu Glu Trp Met
35 40 45

Gly Trp Ile Ser Ala Tyr Asn Gly Asn Thr Asn Tyr Ala Gln Lys Leu
50 55 60

Gln Gly Arg Val Thr Met Thr Thr Asp Thr Ser Thr Ser Thr Ala Tyr
65 70 75 80

Met Glu Leu Arg Ser Leu Arg Ser Asp Asp Thr Ala Val Tyr Tyr Cys
85 90 95

Ala Arg Ala Thr Tyr Tyr Tyr Asp Ser Ser Ala Pro Ala Phe Asp Ile
100 105 110

Trp Gly Gln Gly Thr Met Val Thr Val Ser Ser Ala Ser Thr Lys Gly
115 120 125

Pro Ser Val Phe Pro Leu Ala Pro Ser Ser Lys Ser Thr Ser Gly Gly
130 135 140

Thr Ala Ala Leu Gly Cys Leu Val Lys Asp Tyr Phe Pro Glu Pro Val
145 150 155 160

Thr Val Ser Trp Asn Ser Gly Ala Leu Thr Ser Gly Val His Thr Phe
165 170 175

Pro Ala Val Leu Gln Ser Ser Gly Leu Tyr Ser Leu Ser Ser Val Val
180 185 190

Thr Val Pro Ser Ser Ser Leu Gly Thr Gln Thr Tyr Ile Cys Asn Val
195 200 205

Asn His Lys Pro Ser Asn Thr Lys Val Asp Lys Lys Val Glu Pro Lys
210 215 220

Ser Cys Asp Lys Thr His Thr Cys Pro Pro Cys Pro Ala Pro Glu Leu
225 230 235 240

Leu Gly Gly Pro Ser Val Phe Leu Phe Pro Pro Lys Pro Lys Asp Thr
245 250 255

Leu Met Ile Ser Arg Thr Pro Glu Val Thr Cys Val Val Val Asp Val
260 265 270

Ser His Glu Asp Pro Glu Val Lys Phe Asn Trp Tyr Val Asp Gly Val
275 280 285

Glu Val His Asn Ala Lys Thr Lys Pro Arg Glu Glu Gln Tyr Asn Ser
290 295 300

Thr Tyr Arg Val Val Ser Val Leu Thr Val Leu His Gln Asp Trp Leu
305 310 315 320

Asn Gly Lys Glu Tyr Lys Cys Lys Val Ser Asn Lys Ala Leu Pro Ala
325 330 335

Pro Ile Glu Lys Thr Ile Ser Lys Ala Lys Gly Gln Pro Arg Glu Pro
340 345 350

Gln Val Tyr Thr Leu Pro Pro Ser Arg Asp Glu Leu Thr Lys Asn Gln
355 360 365

Val Ser Leu Thr Cys Leu Val Lys Gly Phe Tyr Pro Ser Asp Ile Ala
370 375 380

Val Glu Trp Glu Ser Asn Gly Gln Pro Glu Asn Asn Tyr Lys Thr Thr
385 390 395 400

Pro Pro Val Leu Asp Ser Asp Gly Ser Phe Phe Leu Tyr Ser Lys Leu
405 410 415

Thr Val Asp Lys Ser Arg Trp Gln Gln Gly Asn Val Phe Ser Cys Ser
420 425 430

Val Met His Glu Ala Leu His Asn His Tyr Thr Gln Lys Ser Leu Ser
435 440 445

Leu Ser Pro
450

<210> 114
<211> 214
<212> PRT
<213> Artificial

<220>
<223> An artificially synthesized peptide sequence

<400> 114

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Glu Asp Asp
20 25 30

Leu Ala Trp Tyr Gln Gln Lys Pro Gly Lys Ala Pro Lys Leu Leu Ile
35 40 45

Tyr Glu Ala Ser Thr Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln His Asp Asp Ser Pro Leu
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 115
<211> 452
<212> PRT
<213> Artificial

<220>
<223> An artificially synthesized peptide sequence

<400> 115

Glu Val Gln Leu Val Gln Ser Gly Ala Glu Val Lys Lys Pro Gly Ser
1 5 10 15

Ser Val Lys Val Ser Cys Lys Ala Ser Gly Gly Thr Phe Ser Ser Tyr
20 25 30

Ala Ile Ser Trp Val Arg Gln Ala Pro Gly Gln Gly Leu Glu Trp Met
35 40 45

Gly Gly Ile Ile Pro Ile Phe Gly Thr Ala Asn Tyr Ala Gln Lys Phe
50 55 60

Gln Gly Arg Val Thr Ile Thr Ala Asp Glu Ser Thr Ser Thr Ala Tyr
65 70 75 80

```
Met Glu Leu Ser Ser Leu Arg Ser Glu Asp Thr Ala Val Tyr Tyr Cys
                85                  90                  95

Ala Arg Asp Arg Gly Ser Gly Phe Asn Trp Gly Asn Tyr Ala Phe Asp
            100                 105                 110

Ile Trp Gly Gln Gly Phe Ser Val Thr Val Ser Ser Ala Ser Thr Lys
        115                 120                 125

Gly Pro Ser Val Phe Pro Leu Ala Pro Ser Ser Lys Ser Thr Ser Gly
    130                 135                 140

Gly Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val
            180                 185                 190

Val Thr Val Pro Ser Ser Ser Leu Gly Thr Gln Thr Tyr Ile Cys Asn
        195                 200                 205

Val Asn His Lys Pro Ser Asn Thr Lys Val Asp Lys Lys Val Glu Pro
    210                 215                 220

Lys Ser Cys Asp Lys Thr His Thr Cys Pro Pro Cys Pro Ala Pro Glu
225                 230                 235                 240

Leu Leu Gly Gly Pro Ser Val Phe Leu Phe Pro Pro Lys Pro Lys Asp
                245                 250                 255

Thr Leu Met Ile Ser Arg Thr Pro Glu Val Thr Cys Val Val Val Asp
            260                 265                 270

Val Ser His Glu Asp Pro Glu Val Lys Phe Asn Trp Tyr Val Asp Gly
        275                 280                 285

Val Glu Val His Asn Ala Lys Thr Lys Pro Arg Glu Glu Gln Tyr Asn
    290                 295                 300

Ser Thr Tyr Arg Val Val Ser Val Leu Thr Val Leu His Gln Asp Trp
305                 310                 315                 320

Leu Asn Gly Lys Glu Tyr Lys Cys Lys Val Ser Asn Lys Ala Leu Pro
                325                 330                 335
```

Ala Pro Ile Glu Lys Thr Ile Ser Lys Ala Lys Gly Gln Pro Arg Glu
340 345 350

Pro Gln Val Tyr Thr Leu Pro Pro Ser Arg Asp Glu Leu Thr Lys Asn
355 360 365

Gln Val Ser Leu Thr Cys Leu Val Lys Gly Phe Tyr Pro Ser Asp Ile
370 375 380

Ala Val Glu Trp Glu Ser Asn Gly Gln Pro Glu Asn Asn Tyr Lys Thr
385 390 395 400

Thr Pro Pro Val Leu Asp Ser Asp Gly Ser Phe Phe Leu Tyr Ser Lys
405 410 415

Leu Thr Val Asp Lys Ser Arg Trp Gln Gln Gly Asn Val Phe Ser Cys
420 425 430

Ser Val Met His Glu Ala Leu His Asn His Tyr Thr Gln Lys Ser Leu
435 440 445

Ser Leu Ser Pro
450

<210> 116
<211> 214
<212> PRT
<213> Artificial

<220>
<223> An artificially synthesized peptide sequence

<400> 116

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Glu Asp Asp
20 25 30

Leu Ala Trp Tyr Gln Gln Lys Pro Gly Lys Ala Pro Lys Leu Leu Ile
35 40 45

Tyr Glu Ala Ser Asn Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Asp Arg Tyr Pro Tyr
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 117
<211> 458
<212> PRT
<213> Artificial

<220>
<223> An artificially synthesized peptide sequence

<400> 117

Glu Val Gln Leu Val Gln Ser Gly Ala Glu Val Lys Lys Pro Gly Ser
1 5 10 15

Ser Val Lys Val Ser Cys Lys Ala Ser Gly Gly Thr Phe Ser Ser Tyr
20 25 30

Ala Ile Ser Trp Val Arg Gln Ala Pro Gly Gln Gly Leu Glu Trp Met
35 40 45

Gly Gly Ile Ile Pro Ile Phe Gly Thr Ala Asn Tyr Ala Gln Lys Phe
50 55 60

Gln Gly Arg Val Thr Ile Thr Ala Asp Glu Ser Thr Ser Thr Ala Tyr

65 70 75 80

Met Glu Leu Ser Ser Leu Arg Ser Glu Asp Thr Ala Val Tyr Tyr Cys
85 90 95

Ala Arg Ala Ser Ile Tyr Cys Ser Ser Thr Ser Cys Tyr Glu Pro Pro
100 105 110

Tyr Tyr Tyr Tyr Met Asp Val Trp Gly Lys Gly Thr Met Val Thr Val
115 120 125

Ser Ser Ala Ser Thr Lys Gly Pro Ser Val Phe Pro Leu Ala Pro Ser
130 135 140

Ser Lys Ser Thr Ser Gly Gly Thr Ala Ala Leu Gly Cys Leu Val Lys
145 150 155 160

Asp Tyr Phe Pro Glu Pro Val Thr Val Ser Trp Asn Ser Gly Ala Leu
165 170 175

Thr Ser Gly Val His Thr Phe Pro Ala Val Leu Gln Ser Ser Gly Leu
180 185 190

Tyr Ser Leu Ser Ser Val Val Thr Val Pro Ser Ser Ser Leu Gly Thr
195 200 205

Gln Thr Tyr Ile Cys Asn Val Asn His Lys Pro Ser Asn Thr Lys Val
210 215 220

Asp Lys Lys Val Glu Pro Lys Ser Cys Asp Lys Thr His Thr Cys Pro
225 230 235 240

Pro Cys Pro Ala Pro Glu Leu Leu Gly Gly Pro Ser Val Phe Leu Phe
245 250 255

Pro Pro Lys Pro Lys Asp Thr Leu Met Ile Ser Arg Thr Pro Glu Val
260 265 270

Thr Cys Val Val Val Asp Val Ser His Glu Asp Pro Glu Val Lys Phe
275 280 285

Asn Trp Tyr Val Asp Gly Val Glu Val His Asn Ala Lys Thr Lys Pro
290 295 300

Arg Glu Glu Gln Tyr Asn Ser Thr Tyr Arg Val Val Ser Val Leu Thr
305 310 315 320

Val Leu His Gln Asp Trp Leu Asn Gly Lys Glu Tyr Lys Cys Lys Val
325 330 335

Ser Asn Lys Ala Leu Pro Ala Pro Ile Glu Lys Thr Ile Ser Lys Ala
340 345 350

Lys Gly Gln Pro Arg Glu Pro Gln Val Tyr Thr Leu Pro Pro Ser Arg
355 360 365

Asp Glu Leu Thr Lys Asn Gln Val Ser Leu Thr Cys Leu Val Lys Gly
370 375 380

Phe Tyr Pro Ser Asp Ile Ala Val Glu Trp Glu Ser Asn Gly Gln Pro
385 390 395 400

Glu Asn Asn Tyr Lys Thr Thr Pro Pro Val Leu Asp Ser Asp Gly Ser
405 410 415

Phe Phe Leu Tyr Ser Lys Leu Thr Val Asp Lys Ser Arg Trp Gln Gln
420 425 430

Gly Asn Val Phe Ser Cys Ser Val Met His Glu Ala Leu His Asn His
435 440 445

Tyr Thr Gln Lys Ser Leu Ser Leu Ser Pro
450 455

<210> 118
<211> 214
<212> PRT
<213> Artificial

<220>
<223> An artificially synthesized peptide sequence

<400> 118

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Ser His
20 25 30

Leu Ala Trp Tyr Gln Gln Lys Pro Gly Lys Ala Pro Lys Leu Leu Ile
35 40 45

Tyr Ala Ala Ser His Leu Gln Ser Gly Val Pro Ser Arg Phe Ser Gly
50 55 60

Ser Gly Ser Gly Thr Asp Phe Thr Leu Thr Ile Ser Ser Leu Gln Pro

65 70 75 80

Glu Asp Phe Ala Thr Tyr Tyr Cys Gln Gln Ser His Asn Tyr Pro Leu
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 119
<211> 447
<212> PRT
<213> Artificial

<220>
<223> An artificially synthesized peptide sequence

<400> 119

Gln Val Gln Leu Val Gln Ser Gly Ala Glu Val Lys Lys Pro Gly Ser
1 5 10 15

Ser Val Lys Val Ser Cys Lys Ala Ser Gly Gly Thr Phe Ser Ser Tyr
20 25 30

Ala Ile Ser Trp Val Arg Gln Ala Pro Gly Gln Gly Leu Glu Trp Met
35 40 45

Gly Gly Ile Ile Pro Ile Phe Gly Thr Ala Asn Tyr Ala Gln Lys Phe
50 55 60

Gln Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Met Ile Asn Gly Val Trp Glu Gly Gly Met Asp Val Trp Gly Gln Gly
100 105 110

Thr Thr Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
210 215 220

Thr His Thr Cys Pro Pro Cys Pro Ala Pro Glu Leu Leu Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu

305 310 315 320

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro
435 440 445

<210> 120
<211> 214
<212> PRT
<213> Artificial

<220>
<223> An artificially synthesized peptide sequence

<400> 120

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile His Ser His
20 25 30

Leu Asn Trp Tyr Gln Gln Lys Pro Gly Lys Ala Pro Lys Leu Leu Ile
35 40 45

Tyr His Ala Ser Lys Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln His Gly Asn Ser Pro Leu
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 121
<211> 451
<212> PRT
<213> Artificial

<220>
<223> An artificially synthesized peptide sequence

<400> 121

Glu Val Gln Leu Val Gln Ser Gly Gly Gly Leu Val Gln Pro Gly Gly
1 5 10 15

Ser Leu Arg Leu Ser Cys Ala Ala Ser Gly Phe Thr Phe Ser Asn Ala
20 25 30

Trp Met Ile Trp Val Arg Gln Ala Pro Gly Lys Gly Leu Glu Trp Val
35 40 45

Gly Arg Ile Lys Ser Lys Val Asp Gly Gly Thr Thr Asp Tyr Ala Ala
50 55 60

Pro Val Lys Gly Arg Phe Thr Ile Ser Arg Asp Asp Ser Lys Asn Thr
65 70 75 80

Leu Tyr Leu Gln Met Asp Ser Leu Lys Thr Glu Asp Thr Ala Val Tyr
85 90 95

Tyr Cys Val Ala Asp Val Pro Ala Ser Asn Pro Tyr Gly Phe Asp Tyr
100 105 110

Trp Gly Gln Gly Thr Leu Val Thr Val Ser Ser Ala Ser Thr Lys Gly
115 120 125

Pro Ser Val Phe Pro Leu Ala Pro Ser Ser Lys Ser Thr Ser Gly Gly
130 135 140

Thr Ala Ala Leu Gly Cys Leu Val Lys Asp Tyr Phe Pro Glu Pro Val
145 150 155 160

Thr Val Ser Trp Asn Ser Gly Ala Leu Thr Ser Gly Val His Thr Phe
165 170 175

Pro Ala Val Leu Gln Ser Ser Gly Leu Tyr Ser Leu Ser Ser Val Val
180 185 190

Thr Val Pro Ser Ser Ser Leu Gly Thr Gln Thr Tyr Ile Cys Asn Val
195 200 205

Asn His Lys Pro Ser Asn Thr Lys Val Asp Lys Lys Val Glu Pro Lys
210 215 220

Ser Cys Asp Lys Thr His Thr Cys Pro Pro Cys Pro Ala Pro Glu Leu
225 230 235 240

Leu Gly Gly Pro Ser Val Phe Leu Phe Pro Pro Lys Pro Lys Asp Thr
245 250 255

Leu Met Ile Ser Arg Thr Pro Glu Val Thr Cys Val Val Val Asp Val
260 265 270

Ser His Glu Asp Pro Glu Val Lys Phe Asn Trp Tyr Val Asp Gly Val
275 280 285

Glu Val His Asn Ala Lys Thr Lys Pro Arg Glu Glu Gln Tyr Asn Ser
290 295 300

Thr Tyr Arg Val Val Ser Val Leu Thr Val Leu His Gln Asp Trp Leu
305 310 315 320

Asn Gly Lys Glu Tyr Lys Cys Lys Val Ser Asn Lys Ala Leu Pro Ala
325 330 335

Pro Ile Glu Lys Thr Ile Ser Lys Ala Lys Gly Gln Pro Arg Glu Pro
340 345 350

Gln Val Tyr Thr Leu Pro Pro Ser Arg Asp Glu Leu Thr Lys Asn Gln
355 360 365

Val Ser Leu Thr Cys Leu Val Lys Gly Phe Tyr Pro Ser Asp Ile Ala
370 375 380

Val Glu Trp Glu Ser Asn Gly Gln Pro Glu Asn Asn Tyr Lys Thr Thr
385 390 395 400

Pro Pro Val Leu Asp Ser Asp Gly Ser Phe Phe Leu Tyr Ser Lys Leu
405 410 415

Thr Val Asp Lys Ser Arg Trp Gln Gln Gly Asn Val Phe Ser Cys Ser
420 425 430

Val Met His Glu Ala Leu His Asn His Tyr Thr Gln Lys Ser Leu Ser
435 440 445

Leu Ser Pro
450

<210> 122
<211> 214
<212> PRT
<213> Artificial

<220>
<223> An artificially synthesized peptide sequence

<400> 122

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Asn Ser His
20 25 30

Leu Asn Trp Tyr Gln Gln Lys Pro Gly Lys Ala Pro Lys Leu Leu Ile
35 40 45

Tyr Asp Ala Ser His Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln His Asn Asn Tyr Pro Leu
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 123
<211> 447
<212> PRT
<213> Artificial sequence

<220>
<223> An artificially synthesized peptide sequence

<400> 123

Gln Val Gln Leu Gln Glu Ser Gly Pro Gly Leu Val Lys Pro Ser Glu
1 5 10 15

Thr Leu Ser Leu Thr Cys Ala Val Ser Gly His Ser Ile Ser His Asp
20 25 30

His Ala Trp Ser Trp Val Arg Gln Pro Pro Gly Glu Gly Leu Glu Trp
35 40 45

```
Ile Gly Phe Ile Ser Tyr Ser Gly Ile Thr Asn Tyr Asn Pro Ser Leu
    50                  55                  60

Gln Gly Arg Val Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Leu Ala Arg Thr Thr Ala Met Asp Tyr Trp Gly Glu Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300
```

Val Ser Val Leu Thr Val Leu His Gln Asp Trp Leu Asn Gly Lys Glu
305 310 315 320

Tyr Lys Cys Lys Val Ser Asn Lys Ala Tyr Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro
435 440 445

<210> 124
<211> 447
<212> PRT
<213> Artificial sequence

<220>
<223> An artificially synthesized peptide sequence

<400> 124

Gln Val Gln Leu Gln Glu Ser Gly Pro Gly Leu Val Lys Pro Ser Glu
1 5 10 15

Thr Leu Ser Leu Thr Cys Ala Val Ser Gly His Ser Ile Ser His Asp
20 25 30

His Ala Trp Ser Trp Val Arg Gln Pro Pro Gly Glu Gly Leu Glu Trp
35 40 45

Ile Gly Phe Ile Ser Tyr Ser Gly Ile Thr Asn Tyr Asn Pro Ser Leu
50 55 60

```
Gln Gly Arg Val Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Leu Ala Arg Thr Thr Ala Met Asp Tyr Trp Gly Glu Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Asp Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320
```

Tyr Lys Cys Lys Val Ser Asn Lys Ala Leu Pro Ala Pro Glu Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro
435 440 445

<210> 125
<211> 447
<212> PRT
<213> Artificial sequence

<220>
<223> An artificially synthesized peptide sequence

<400> 125

Gln Val Gln Leu Gln Glu Ser Gly Pro Gly Leu Val Lys Pro Ser Glu
1 5 10 15

Thr Leu Ser Leu Thr Cys Ala Val Ser Gly His Ser Ile Ser His Asp
20 25 30

His Ala Trp Ser Trp Val Arg Gln Pro Pro Gly Glu Gly Leu Glu Trp
35 40 45

Ile Gly Phe Ile Ser Tyr Ser Gly Ile Thr Asn Tyr Asn Pro Ser Leu
50 55 60

Gln Gly Arg Val Thr Ile Ser Arg Asp Asn Ser Lys Asn Thr Leu Tyr
65 70 75 80

```
Leu Gln Met Asn Ser Leu Arg Ala Glu Asp Thr Ala Val Tyr Tyr Cys
                85                  90                  95

Ala Arg Ser Leu Ala Arg Thr Thr Ala Met Asp Tyr Trp Gly Glu Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Tyr Pro Ala Pro Ile Glu Lys
                325                 330                 335
```

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Leu His Glu
420 425 430

Ala Leu His Ser His Tyr Thr Gln Lys Ser Leu Ser Leu Ser Pro
435 440 445

<210> 126
<211> 447
<212> PRT
<213> Artificial sequence

<220>
<223> An artificially synthesized peptide sequence

<400> 126

Gln Val Gln Leu Gln Glu Ser Gly Pro Gly Leu Val Lys Pro Ser Glu
1 5 10 15

Thr Leu Ser Leu Thr Cys Ala Val Ser Gly His Ser Ile Ser His Asp
20 25 30

His Ala Trp Ser Trp Val Arg Gln Pro Pro Gly Glu Gly Leu Glu Trp
35 40 45

Ile Gly Phe Ile Ser Tyr Ser Gly Ile Thr Asn Tyr Asn Pro Ser Leu
50 55 60

Gln Gly Arg Val Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Ser Leu Ala Arg Thr Thr Ala Met Asp Tyr Trp Gly Glu Gly
100 105 110

Thr Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
210 215 220

Thr His Thr Cys Pro Pro Cys Pro Ala Pro Glu Leu Leu Gly Gly Pro
225 230 235 240

Asp Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Glu Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Leu His Glu
420 425 430

Ala Leu His Ser His Tyr Thr Gln Lys Ser Leu Ser Leu Ser Pro
435 440 445

<210> 127
<211> 447
<212> PRT
<213> Artificial sequence

<220>
<223> An artificially synthesized peptide sequence

<400> 127

Gln Val Gln Leu Gln Glu Ser Gly Pro Gly Leu Val Lys Pro Ser Glu
1 5 10 15

Thr Leu Ser Leu Thr Cys Ala Val Ser Gly His Ser Ile Ser His Asp
20 25 30

His Ala Trp Ser Trp Val Arg Gln Pro Pro Gly Glu Gly Leu Glu Trp
35 40 45

Ile Gly Phe Ile Ser Tyr Ser Gly Ile Thr Asn Tyr Asn Pro Ser Leu
50 55 60

Gln Gly Arg Val Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Ser Leu Ala Arg Thr Thr Ala Met Asp Tyr Trp Gly Glu Gly
100 105 110

Thr Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
210 215 220

Thr His Thr Cys Pro Pro Cys Pro Ala Pro Glu Leu Leu Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Tyr Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Ala His Tyr Thr Gln Lys Ser Leu Ser Leu Ser Pro
435 440 445

<210> 128
<211> 443
<212> PRT
<213> Mus musculus

<400> 128

Gln Val Gln Leu Gln Glu Ser Gly Pro Gly Leu Val Lys Pro Ser Glu
1 5 10 15

Thr Leu Ser Leu Thr Cys Ala Val Ser Gly His Ser Ile Ser His Asp
20 25 30

His Ala Trp Ser Trp Val Arg Gln Pro Pro Gly Glu Gly Leu Glu Trp
35 40 45

Ile Gly Phe Ile Ser Tyr Ser Gly Ile Thr Asn Tyr Asn Pro Ser Leu
50 55 60

Gln Gly Arg Val Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Ser Leu Ala Arg Thr Thr Ala Met Asp Tyr Trp Gly Glu Gly
100 105 110

Thr Leu Val Thr Val Ser Ser Ala Lys Thr Thr Pro Pro Ser Val Tyr
115 120 125

Pro Leu Ala Pro Gly Ser Ala Ala Gln Thr Asn Ser Met Val Thr Leu
130 135 140

```
Gly Cys Leu Val Lys Gly Tyr Phe Pro Glu Pro Val Thr Val Thr Trp
145                 150                 155                 160

Asn Ser Gly Ser Leu Ser Ser Gly Val His Thr Phe Pro Ala Val Leu
                165                 170                 175

Gln Ser Asp Leu Tyr Thr Leu Ser Ser Ser Val Thr Val Pro Ser Ser
            180                 185                 190

Thr Trp Pro Ser Glu Thr Val Thr Cys Asn Val Ala His Pro Ala Ser
        195                 200                 205

Ser Thr Lys Val Asp Lys Lys Ile Val Pro Arg Asp Cys Gly Cys Lys
    210                 215                 220

Pro Cys Ile Cys Thr Val Pro Glu Val Ser Ser Val Phe Ile Phe Pro
225                 230                 235                 240

Pro Lys Pro Lys Asp Val Leu Thr Ile Thr Leu Thr Pro Lys Val Thr
                245                 250                 255

Cys Val Val Val Asp Ile Ser Lys Asp Asp Pro Glu Val Gln Phe Ser
            260                 265                 270

Trp Phe Val Asp Asp Val Glu Val His Thr Ala Gln Thr Gln Pro Arg
        275                 280                 285

Glu Glu Gln Phe Asn Ser Thr Phe Arg Ser Val Ser Glu Leu Pro Ile
    290                 295                 300

Met His Gln Asp Trp Leu Asn Gly Lys Glu Phe Lys Cys Arg Val Asn
305                 310                 315                 320

Ser Ala Ala Phe Pro Ala Pro Ile Glu Lys Thr Ile Ser Lys Thr Lys
                325                 330                 335

Gly Arg Pro Lys Ala Pro Gln Val Tyr Thr Ile Pro Pro Pro Lys Glu
            340                 345                 350

Gln Met Ala Lys Asp Lys Val Ser Leu Thr Cys Met Ile Thr Asp Phe
        355                 360                 365

Phe Pro Glu Asp Ile Thr Val Glu Trp Gln Trp Asn Gly Gln Pro Ala
    370                 375                 380

Glu Asn Tyr Lys Asn Thr Gln Pro Ile Met Asp Thr Asp Gly Ser Tyr
385                 390                 395                 400
```

Phe Val Tyr Ser Lys Leu Asn Val Gln Lys Ser Asn Trp Glu Ala Gly
405 410 415

Asn Thr Phe Thr Cys Ser Val Leu His Glu Gly Leu His Asn His His
420 425 430

Thr Glu Lys Ser Leu Ser His Ser Pro Gly Lys
435 440

<210> 129
<211> 214
<212> PRT
<213> Mus musculus

<400> 129

Asp Ile Gln Met Thr Gln Ser Pro Ser Ser Leu Ser Ala Ser Val Gly
1 5 10 15

Asp Ser Val Thr Ile Thr Cys Gln Ala Ser Thr Asp Ile Ser Ser His
20 25 30

Leu Asn Trp Tyr Gln Gln Lys Pro Gly Lys Ala Pro Glu Leu Leu Ile
35 40 45

Tyr Tyr Gly Ser His Leu Leu Ser Gly Val Pro Ser Arg Phe Ser Gly
50 55 60

Ser Gly Ser Gly Thr Asp Phe Thr Phe Thr Ile Ser Ser Leu Glu Ala
65 70 75 80

Glu Asp Ala Ala Thr Tyr Tyr Cys Gly Gln Gly Asn Arg Leu Pro Tyr
85 90 95

Thr Phe Gly Gln Gly Thr Lys Val Glu Ile Glu Arg Ala Asp Ala Ala
100 105 110

Pro Thr Val Ser Ile Phe Pro Pro Ser Ser Glu Gln Leu Thr Ser Gly
115 120 125

Gly Ala Ser Val Val Cys Phe Leu Asn Asn Phe Tyr Pro Lys Asp Ile
130 135 140

Asn Val Lys Trp Lys Ile Asp Gly Ser Glu Arg Gln Asn Gly Val Leu
145 150 155 160

Asn Ser Trp Thr Asp Gln Asp Ser Lys Asp Ser Thr Tyr Ser Met Ser
165 170 175

```
Ser Thr Leu Thr Leu Thr Lys Asp Glu Tyr Glu Arg His Asn Ser Tyr
            180                 185                 190

Thr Cys Glu Ala Thr His Lys Thr Ser Thr Ser Pro Ile Val Lys Ser
        195                 200                 205

Phe Asn Arg Asn Glu Cys
    210


<210>  130
<211>  443
<212>  PRT
<213>  Artificial sequence

<220>
<223>  An artificially synthesized peptide sequence

<400>  130

Gln Val Gln Leu Gln Glu Ser Gly Pro Gly Leu Val Lys Pro Ser Glu
1               5                   10                  15

Thr Leu Ser Leu Thr Cys Ala Val Ser Gly His Ser Ile Ser His Asp
            20                  25                  30

His Ala Trp Ser Trp Val Arg Gln Pro Pro Gly Glu Gly Leu Glu Trp
        35                  40                  45

Ile Gly Phe Ile Ser Tyr Ser Gly Ile Thr Asn Tyr Asn Pro Ser Leu
    50                  55                  60

Gln Gly Arg Val Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Leu Ala Arg Thr Thr Ala Met Asp Tyr Trp Gly Glu Gly
            100                 105                 110

Thr Leu Val Thr Val Ser Ser Ala Lys Thr Thr Pro Pro Ser Val Tyr
        115                 120                 125

Pro Leu Ala Pro Gly Ser Ala Ala Gln Thr Asn Ser Met Val Thr Leu
    130                 135                 140

Gly Cys Leu Val Lys Gly Tyr Phe Pro Glu Pro Val Thr Val Thr Trp
145                 150                 155                 160
```

Asn Ser Gly Ser Leu Ser Ser Gly Val His Thr Phe Pro Ala Val Leu
165 170 175

Gln Ser Asp Leu Tyr Thr Leu Ser Ser Ser Val Thr Val Pro Ser Ser
180 185 190

Thr Trp Pro Ser Glu Thr Val Thr Cys Asn Val Ala His Pro Ala Ser
195 200 205

Ser Thr Lys Val Asp Lys Lys Ile Val Pro Arg Asp Cys Gly Cys Lys
210 215 220

Pro Cys Ile Cys Thr Val Pro Glu Val Ser Ser Val Phe Ile Phe Pro
225 230 235 240

Pro Lys Pro Lys Asp Val Leu Thr Ile Thr Leu Thr Pro Lys Val Thr
245 250 255

Cys Val Val Val Asp Ile Ser Lys Asp Asp Pro Glu Val Gln Phe Ser
260 265 270

Trp Phe Val Asp Asp Val Glu Val His Thr Ala Gln Thr Gln Pro Arg
275 280 285

Glu Glu Gln Phe Asn Ser Thr Phe Arg Ser Val Ser Glu Leu Pro Ile
290 295 300

Met His Gln Asp Trp Leu Asn Gly Lys Glu Phe Lys Cys Arg Val Asn
305 310 315 320

Ser Ala Asp Phe Pro Ala Pro Ile Glu Lys Thr Ile Ser Lys Thr Lys
325 330 335

Gly Arg Pro Lys Ala Pro Gln Val Tyr Thr Ile Pro Pro Pro Lys Glu
340 345 350

Gln Met Ala Lys Asp Lys Val Ser Leu Thr Cys Met Ile Thr Asp Phe
355 360 365

Phe Pro Glu Asp Ile Thr Val Glu Trp Gln Trp Asn Gly Gln Pro Ala
370 375 380

Glu Asn Tyr Lys Asn Thr Gln Pro Ile Met Asp Thr Asp Gly Ser Tyr
385 390 395 400

Phe Val Tyr Ser Lys Leu Asn Val Gln Lys Ser Asn Trp Glu Ala Gly
405 410 415

Asn Thr Phe Thr Cys Ser Val Leu His Glu Gly Leu His Asn His His
420 425 430

Thr Glu Lys Ser Leu Ser His Ser Pro Gly Lys
435 440

<210> 131
<211> 443
<212> PRT
<213> Artificial sequence

<220>
<223> An artificially synthesized peptide sequence

<400> 131

Gln Val Gln Leu Gln Glu Ser Gly Pro Gly Leu Val Lys Pro Ser Glu
1 5 10 15

Thr Leu Ser Leu Thr Cys Ala Val Ser Gly His Ser Ile Ser His Asp
20 25 30

His Ala Trp Ser Trp Val Arg Gln Pro Pro Gly Glu Gly Leu Glu Trp
35 40 45

Ile Gly Phe Ile Ser Tyr Ser Gly Ile Thr Asn Tyr Asn Pro Ser Leu
50 55 60

Gln Gly Arg Val Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Ser Leu Ala Arg Thr Thr Ala Met Asp Tyr Trp Gly Glu Gly
100 105 110

Thr Leu Val Thr Val Ser Ser Ala Lys Thr Thr Pro Pro Ser Val Tyr
115 120 125

Pro Leu Ala Pro Gly Ser Ala Ala Gln Thr Asn Ser Met Val Thr Leu
130 135 140

Gly Cys Leu Val Lys Gly Tyr Phe Pro Glu Pro Val Thr Val Thr Trp
145 150 155 160

Asn Ser Gly Ser Leu Ser Ser Gly Val His Thr Phe Pro Ala Val Leu
165 170 175

Gln Ser Asp Leu Tyr Thr Leu Ser Ser Ser Val Thr Val Pro Ser Ser
180 185 190

Thr Trp Pro Ser Glu Thr Val Thr Cys Asn Val Ala His Pro Ala Ser
195 200 205

Ser Thr Lys Val Asp Lys Lys Ile Val Pro Arg Asp Cys Gly Cys Lys
210 215 220

Pro Cys Ile Cys Thr Val Pro Glu Val Ser Asp Val Phe Ile Phe Pro
225 230 235 240

Pro Lys Pro Lys Asp Val Leu Thr Ile Thr Leu Thr Pro Lys Val Thr
245 250 255

Cys Val Val Val Asp Ile Ser Lys Asp Asp Pro Glu Val Gln Phe Ser
260 265 270

Trp Phe Val Asp Asp Val Glu Val His Thr Ala Gln Thr Gln Pro Arg
275 280 285

Glu Glu Gln Phe Asn Ser Thr Phe Arg Ser Val Ser Glu Leu Pro Ile
290 295 300

Met His Gln Asp Trp Leu Asn Gly Lys Glu Phe Lys Cys Arg Val Asn
305 310 315 320

Ser Ala Asp Phe Pro Ala Pro Ile Glu Lys Thr Ile Ser Lys Thr Lys
325 330 335

Gly Arg Pro Lys Ala Pro Gln Val Tyr Thr Ile Pro Pro Pro Lys Glu
340 345 350

Gln Met Ala Lys Asp Lys Val Ser Leu Thr Cys Met Ile Thr Asp Phe
355 360 365

Phe Pro Glu Asp Ile Thr Val Glu Trp Gln Trp Asn Gly Gln Pro Ala
370 375 380

Glu Asn Tyr Lys Asn Thr Gln Pro Ile Met Asp Thr Asp Gly Ser Tyr
385 390 395 400

Phe Val Tyr Ser Lys Leu Asn Val Gln Lys Ser Asn Trp Glu Ala Gly
405 410 415

Asn Thr Phe Thr Cys Ser Val Leu His Glu Gly Leu His Asn His His
420 425 430

Thr Glu Lys Ser Leu Ser His Ser Pro Gly Lys
        435                 440

<210>  132
<211>  119
<212>  PRT
<213>  Artificial sequence

<220>
<223>  An artificially synthesized peptide sequence

<400>  132

Gln Val Gln Leu Gln Glu Ser Gly Pro Gly Leu Val Lys Pro Ser Glu
1               5                   10                  15

Thr Leu Ser Leu Thr Cys Ala Val Ser Gly His Ser Ile Ser His Asp
            20                  25                  30

His Ala Trp Ser Trp Val Arg Gln Pro Pro Gly Glu Gly Leu Glu Trp
        35                  40                  45

Ile Gly Phe Ile Ser Tyr Ser Gly Ile Thr Asn Tyr Asn Pro Ser Leu
    50                  55                  60

Gln Gly Arg Val Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Leu Ala Arg Thr Thr Ala Met Asp Tyr Trp Gly Glu Gly
            100                 105                 110

Thr Leu Val Thr Val Ser Ser
        115

<210>  133
<211>  447
<212>  PRT
<213>  Artificial sequence

<220>
<223>  An artificially synthesized peptide sequence

<400>  133

Gln Val Gln Leu Gln Glu Ser Gly Pro Gly Leu Val Lys Pro Ser Glu
1               5                   10                  15

Thr Leu Ser Leu Thr Cys Ala Val Ser Gly His Ser Ile Ser His Asp

20 25 30

His Ala Trp Ser Trp Val Arg Gln Pro Pro Gly Glu Gly Leu Glu Trp
35 40 45

Ile Gly Phe Ile Ser Tyr Ser Gly Ile Thr Asn Tyr Asn Pro Ser Leu
50 55 60

Gln Gly Arg Val Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Ser Leu Ala Arg Thr Thr Ala Met Asp Tyr Trp Gly Glu Gly
100 105 110

Thr Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
210 215 220

Thr His Thr Cys Pro Pro Cys Pro Ala Pro Glu Leu Leu Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro
435 440 445

<210> 134
<211> 447
<212> PRT
<213> Artificial sequence

<220>
<223> An artificially synthesized peptide sequence

<400> 134

Gln Val Gln Leu Gln Glu Ser Gly Pro Gly Leu Val Lys Pro Ser Glu
1 5 10 15

Thr Leu Ser Leu Thr Cys Ala Val Ser Gly His Ser Ile Ser His Asp
20 25 30

His Ala Trp Ser Trp Val Arg Gln Pro Pro Gly Glu Gly Leu Glu Trp

35 40 45

Ile Gly Phe Ile Ser Tyr Ser Gly Ile Thr Asn Tyr Asn Pro Ser Leu
50 55 60

Gln Gly Arg Val Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Ser Leu Ala Arg Thr Thr Ala Met Asp Tyr Trp Gly Glu Gly
100 105 110

Thr Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
210 215 220

Thr His Thr Cys Pro Pro Cys Pro Ala Pro Glu Leu Leu Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Glu His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Phe Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro
435 440 445

<210> 135
<211> 214
<212> PRT
<213> Artificial sequence

<220>
<223> An artificially synthesized peptide sequence

<400> 135

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Ile Ser Ser Tyr
20 25 30

Leu Asn Trp Tyr Gln Gln Lys Pro Gly Lys Ala Pro Lys Leu Leu Ile
35 40 45

Tyr Tyr Thr Ser Arg Leu His Ser Gly Val Pro Ser Arg Phe Ser Gly

50 55 60

Ser Gly Ser Gly Thr Asp Phe Thr Phe Thr Ile Ser Ser Leu Gln Pro
65 70 75 80

Glu Asp Ile Ala Thr Tyr Tyr Cys Gln Gln Gly Asn Thr Leu Pro Tyr
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 136
<211> 449
<212> PRT
<213> Artificial sequence

<220>
<223> An artificially synthesized peptide sequence

<400> 136

Glu Val Gln Leu Val Glu Ser Gly Gly Gly Leu Val Gln Pro Gly Gly
1 5 10 15

Ser Leu Arg Leu Ser Cys Ala Val Ser Gly Tyr Ser Ile Thr Ser Gly
20 25 30

Tyr Ser Trp Asn Trp Ile Arg Gln Ala Pro Gly Lys Gly Leu Glu Trp
35 40 45

```
Val Ala Ser Ile Thr Tyr Asp Gly Ser Thr Asn Tyr Asn Pro Ser Val
    50                  55                  60

Lys Gly Arg Ile Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ser His Tyr Phe Gly His Trp His Phe Ala Val Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
```

290 295 300

Arg Val Val Ser Val Leu Thr Val Leu His Gln Asp Trp Leu Asn Gly
305 310 315 320

Lys Glu Tyr Lys Cys Lys Val Ser Asn Lys Ala Leu Pro Ala Pro Ile
325 330 335

Glu Lys Thr Ile Ser Lys Ala Lys Gly Gln Pro Arg Glu Pro Gln Val
340 345 350

Tyr Thr Leu Pro Pro Ser Arg Asp Glu Leu Thr Lys Asn Gln Val Ser
355 360 365

Leu Thr Cys Leu Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Tyr Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro

<210> 137
<211> 218
<212> PRT
<213> Artificial sequence

<220>
<223> An artificially synthesized peptide sequence

<400> 137

Asp Ile Gln Leu Thr Gln Ser Pro Ser Ser Leu Ser Ala Ser Val Gly
1 5 10 15

Asp Arg Val Thr Ile Thr Cys Arg Ala Ser Gln Ser Val Asp Tyr Asp
20 25 30

Gly Asp Ser Tyr Met Asn Trp Tyr Gln Gln Lys Pro Gly Lys Ala Pro
35 40 45

Lys Leu Leu Ile Tyr Ala Ala Ser Tyr Leu Glu Ser Gly Val Pro Ser
    50                  55                  60

Arg Phe Ser Gly Ser Gly Ser Gly Thr Asp Phe Thr Leu Thr Ile Ser
65                  70                  75                  80

Ser Leu Gln Pro Glu Asp Phe Ala Thr Tyr Tyr Cys Gln Gln Ser His
                85                  90                  95

Glu Asp Pro Tyr Thr Phe Gly Gln Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210> 138
<211> 449
<212> PRT
<213> Artificial sequence

<220>
<223> An artificially synthesized peptide sequence

<400> 138

Glu Val Gln Leu Val Glu Ser Gly Gly Gly Leu Val Gln Pro Gly Gly
1               5                   10                  15

Ser Leu Arg Leu Ser Cys Ala Val Ser Gly Tyr Ser Ile Thr Ser Gly
            20                  25                  30

```
Tyr Ser Trp Asn Trp Ile Arg Gln Ala Pro Gly Lys Gly Leu Glu Trp
        35                  40                  45

Val Ala Ser Ile Thr Tyr Asp Gly Ser Thr Asn Tyr Asn Pro Ser Val
    50                  55                  60

Lys Gly Arg Ile Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ser His Tyr Phe Gly His Trp His Phe Ala Val Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Glu His
            260                 265                 270

Glu Asp Pro Glu Val Lys Phe Asn Trp Tyr Val Asp Gly Val Glu Val
        275                 280                 285
```

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Phe Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro

<210>  139
<211>  447
<212>  PRT
<213>  Artificial sequence

<220>
<223>  An artificially synthesized peptide sequence

<400>  139

Gln Val Gln Leu Gln Glu Ser Gly Pro Gly Leu Val Lys Pro Ser Glu
1               5                   10                  15

Thr Leu Ser Leu Thr Cys Ala Val Ser Gly His Ser Ile Ser His Asp
            20                  25                  30

```
His Ala Trp Ser Trp Val Arg Gln Pro Pro Gly Glu Gly Leu Glu Trp
        35                  40                  45

Ile Gly Phe Ile Ser Tyr Ser Gly Ile Thr Asn Tyr Asn Pro Ser Leu
    50                  55                  60

Gln Gly Arg Val Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Leu Ala Arg Thr Thr Ala Met Asp Tyr Trp Gly Glu Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Tyr Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285
```

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Tyr His Tyr Thr Gln Lys Ser Leu Ser Leu Ser Pro
        435                 440                 445

<210> 140
<211> 449
<212> PRT
<213> Artificial sequence

<220>
<223> An artificially synthesized peptide sequence

<400> 140

Glu Val Gln Leu Val Glu Ser Gly Gly Gly Leu Val Gln Pro Gly Gly
1               5                   10                  15

Ser Leu Arg Leu Ser Cys Ala Val Ser Gly Tyr Ser Ile Thr Ser Gly
            20                  25                  30

Tyr Ser Trp Asn Trp Ile Arg Gln Ala Pro Gly Lys Gly Leu Glu Trp
        35                  40                  45

```
Val Ala Ser Ile Thr Tyr Asp Gly Ser Thr Asn Tyr Asn Pro Ser Val
    50                  55                  60

Lys Gly Arg Ile Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ser His Tyr Phe Gly His Trp His Phe Ala Val Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Asp Leu Leu Gly
225                 230                 235                 240

Asp Asp Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser Asp
            260                 265                 270

Glu Asp Gly Glu Val Lys Phe Asn Trp Tyr Val Asp Gly Val Glu Val
        275                 280                 285

His Asn Ala Lys Thr Lys Pro Arg Glu Glu Gln Asp Asn Ser Thr Tyr
    290                 295                 300
```

Arg Val Val Ser Val Leu Thr Val Leu His Gln Asp Trp Leu Asn Gly
305 310 315 320

Lys Glu Tyr Lys Cys Lys Val Ser Asn Lys Ala Leu Pro Arg Pro Ile
325 330 335

Glu Lys Thr Ile Ser Lys Ala Lys Gly Gln Pro Arg Glu Pro Gln Val
340 345 350

Tyr Thr Leu Pro Pro Ser Arg Asp Glu Leu Thr Lys Asn Gln Val Ser
355 360 365

Leu Thr Cys Leu Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Tyr Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Glu Ser Leu Ser Leu Ser
435 440 445

Pro

<210> 141
<211> 447
<212> PRT
<213> Artificial sequence

<220>
<223> An artificially synthesized peptide sequence

<400> 141

Gln Val Gln Leu Gln Glu Ser Gly Pro Gly Leu Val Lys Pro Ser Glu
1 5 10 15

Thr Leu Ser Leu Thr Cys Ala Val Ser Gly His Ser Ile Ser His Asp
20 25 30

His Ala Trp Ser Trp Val Arg Gln Pro Pro Gly Glu Gly Leu Glu Trp
35 40 45

```
Ile Gly Phe Ile Ser Tyr Ser Gly Ile Thr Asn Tyr Asn Pro Ser Leu
    50                  55                  60

Gln Gly Arg Val Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Leu Ala Arg Thr Thr Ala Met Asp Tyr Trp Gly Glu Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Tyr Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300
```

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Tyr His Tyr Thr Gln Lys Ser Leu Ser Leu Ser Pro
435 440 445

<210> 142
<211> 447
<212> PRT
<213> Artificial sequence

<220>
<223> An artificially synthesized peptide sequence

<400> 142

Gln Val Gln Leu Gln Glu Ser Gly Pro Gly Leu Val Lys Pro Ser Glu
1 5 10 15

Thr Leu Ser Leu Thr Cys Ala Val Ser Gly His Ser Ile Ser His Asp
20 25 30

His Ala Trp Ser Trp Val Arg Gln Pro Pro Gly Glu Gly Leu Glu Trp
35 40 45

Ile Gly Phe Ile Ser Tyr Ser Gly Ile Thr Asn Tyr Asn Pro Ser Leu
50 55 60

Gln Gly Arg Val Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Ser Leu Ala Arg Thr Thr Ala Met Asp Tyr Trp Gly Glu Gly
100 105 110

Thr Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
210 215 220

Thr His Thr Cys Pro Pro Cys Pro Ala Pro Glu Leu Leu Gly Gly Asp
225 230 235 240

Ser Val Phe Leu Phe Pro Pro Lys Pro Lys Asp Thr Leu Tyr Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Tyr His Tyr Thr Gln Lys Ser Leu Ser Leu Ser Pro
435 440 445

<210> 143
<211> 447
<212> PRT
<213> Artificial sequence

<220>
<223> An artificially synthesized peptide sequence

<400> 143

Gln Val Gln Leu Gln Glu Ser Gly Pro Gly Leu Val Lys Pro Ser Glu
1 5 10 15

Thr Leu Ser Leu Thr Cys Ala Val Ser Gly His Ser Ile Ser His Asp
20 25 30

His Ala Trp Ser Trp Val Arg Gln Pro Pro Gly Glu Gly Leu Glu Trp
35 40 45

Ile Gly Phe Ile Ser Tyr Ser Gly Ile Thr Asn Tyr Asn Pro Ser Leu
50 55 60

Gln Gly Arg Val Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Ser Leu Ala Arg Thr Thr Ala Met Asp Tyr Trp Gly Glu Gly
100 105 110

Thr Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
210 215 220

Thr His Thr Cys Pro Pro Cys Pro Ala Pro Glu Leu Leu Gly Gly Asp
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Ile Glu Lys
325 330 335

```
Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445


<210>  144
<211>  447
<212>  PRT
<213>  Artificial sequence

<220>
<223>  An artificially synthesized peptide sequence

<400>  144

Gln Val Gln Leu Gln Glu Ser Gly Pro Gly Leu Val Lys Pro Ser Glu
1               5                   10                  15

Thr Leu Ser Leu Thr Cys Ala Val Ser Gly His Ser Ile Ser His Asp
            20                  25                  30

His Ala Trp Ser Trp Val Arg Gln Pro Pro Gly Glu Gly Leu Glu Trp
        35                  40                  45

Ile Gly Phe Ile Ser Tyr Ser Gly Ile Thr Asn Tyr Asn Pro Ser Leu
    50                  55                  60

Gln Gly Arg Val Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95
```

Ala Arg Ser Leu Ala Arg Thr Thr Ala Met Asp Tyr Trp Gly Glu Gly
100 105 110

Thr Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
210 215 220

Thr His Thr Cys Pro Pro Cys Pro Ala Pro Glu Leu Leu Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Glu Ser Leu Ser Leu Ser Pro
435 440 445

<210> 145
<211> 464
<212> PRT
<213> Artificial sequence

<220>
<223> An artificially synthesized peptide sequence

<400> 145

Gln Val Gln Leu Gln Glu Ser Gly Pro Gly Leu Val Arg Pro Ser Gln
1 5 10 15

Thr Leu Ser Leu Thr Cys Thr Val Ser Gly Tyr Ser Ile Thr Ser Asp
20 25 30

His Ala Trp Ser Trp Val Arg Gln Pro Pro Gly Arg Gly Leu Glu Trp
35 40 45

Ile Gly Tyr Ile Ser Tyr Ser Gly Ile Thr Thr Tyr Asn Pro Ser Leu
50 55 60

Lys Ser Arg Val Thr Met Leu Arg Asp Thr Ser Lys Asn Gln Phe Ser
65 70 75 80

Leu Arg Leu Ser Ser Val Thr Ala Ala Asp Thr Ala Val Tyr Tyr Cys
85 90 95

Ala Arg Ser Leu Ala Arg Thr Thr Ala Met Asp Tyr Trp Gly Gln Gly
100 105 110

```
Ser Leu Val Thr Val Ser Ser Ala Ser Pro Thr Ser Pro Lys Val Phe
        115                 120                 125

Pro Leu Ser Leu Cys Ser Thr Gln Pro Asp Gly Asn Val Val Ile Ala
    130                 135                 140

Cys Leu Val Gln Gly Phe Phe Pro Gln Glu Pro Leu Ser Val Thr Trp
145                 150                 155                 160

Ser Glu Ser Gly Gln Gly Val Thr Ala Arg Asn Phe Pro Pro Ser Gln
                165                 170                 175

Asp Ala Ser Gly Asp Leu Tyr Thr Thr Ser Ser Gln Leu Thr Leu Pro
            180                 185                 190

Ala Thr Gln Cys Leu Ala Gly Lys Ser Val Thr Cys His Val Lys His
        195                 200                 205

Tyr Thr Asn Pro Ser Gln Asp Val Thr Val Pro Cys Pro Val Pro Ser
    210                 215                 220

Thr Pro Pro Thr Pro Ser Pro Ser Thr Pro Pro Thr Pro Ser Pro Ser
225                 230                 235                 240

Cys Cys His Pro Arg Leu Ser Leu His Arg Pro Ala Leu Glu Asp Leu
                245                 250                 255

Leu Leu Gly Ser Glu Ala Asn Leu Thr Cys Thr Leu Thr Gly Leu Arg
            260                 265                 270

Asp Ala Ser Gly Val Thr Phe Thr Trp Thr Pro Ser Ser Gly Lys Ser
        275                 280                 285

Ala Val Gln Gly Pro Pro Glu Arg Asp Leu Cys Gly Cys Tyr Ser Val
    290                 295                 300

Ser Ser Val Leu Pro Gly Cys Ala Glu Pro Trp Asn His Gly Lys Thr
305                 310                 315                 320

Phe Thr Cys Thr Ala Ala Tyr Pro Glu Ser Lys Thr Pro Leu Thr Ala
                325                 330                 335

Thr Leu Ser Lys Ser Gly Asn Thr Phe Arg Pro Glu Val His Leu Leu
            340                 345                 350

Pro Pro Pro Ser Glu Glu Leu Ala Leu Asn Glu Leu Val Thr Leu Thr
        355                 360                 365
```

Cys Leu Ala Arg Gly Phe Ser Pro Lys Asp Val Leu Val Arg Trp Leu
370 375 380

Gln Gly Ser Gln Glu Leu Pro Arg Glu Lys Tyr Leu Thr Trp Ala Ser
385 390 395 400

Arg Gln Glu Pro Ser Gln Gly Thr Thr Thr Phe Ala Val Thr Ser Ile
405 410 415

Leu Arg Val Ala Ala Glu Asp Trp Lys Lys Gly Asp Thr Phe Ser Cys
420 425 430

Met Val Gly His Glu Ala Leu Pro Leu Ala Phe Thr Gln Lys Thr Ile
435 440 445

Asp Arg Leu Ala Gly Lys Glu Gln Lys Leu Ile Ser Glu Glu Asp Leu
450 455 460

<210> 146
<211> 451
<212> PRT
<213> Artificial sequence

<220>
<223> An artificially synthesized peptide sequence

<400> 146

Gln Val Gln Leu Val Gln Ser Gly Ala Glu Val Lys Lys Pro Gly Ser
1 5 10 15

Ser Val Lys Val Ser Cys Lys Ala Ser Gly Gly Thr Phe Ser Ser Tyr
20 25 30

Ala Ile Ser Trp Val Arg Gln Ala Pro Gly Gln Gly Leu Glu Trp Met
35 40 45

Gly Gly Ile Ile Pro Ile Phe Gly Thr Ala Asn Tyr Ala Gln Lys Phe
50 55 60

Gln Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Pro Arg Trp Glu Thr Ala Ile Ser Ser Asp Ala Phe Asp Ile
100 105 110

```
Trp Gly Gln Gly Thr Met Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Asp Glu Leu Thr Lys Asn Gln
        355                 360                 365
```

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro
    450

<210> 147
<211> 214
<212> PRT
<213> Artificial sequence

<220>
<223> An artificially synthesized peptide sequence

<400> 147

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Asp Asp
            20                  25                  30

Leu Asn Trp Tyr Gln Gln Lys Pro Gly Lys Ala Pro Lys Leu Leu Ile
        35                  40                  45

Tyr Glu Ala Ser Asn Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln His Ser Ser Ser Pro Leu
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 148
<211> 451
<212> PRT
<213> Artificial sequence

<220>
<223> An artificially synthesized peptide sequence

<400> 148

Gln Val Gln Leu Val Gln Ser Gly Ala Glu Val Lys Lys Pro Gly Ser
1 5 10 15

Ser Val Lys Val Ser Cys Lys Ala Ser Gly Gly Thr Phe Ser Ser Tyr
20 25 30

Ala Ile Ser Trp Val Arg Gln Ala Pro Gly Gln Gly Leu Glu Trp Met
35 40 45

Gly Gly Ile Ile Pro Ile Phe Gly Thr Ala Asn Tyr Ala Gln Lys Phe
50 55 60

Gln Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

```
Ala Arg Pro Arg Trp Glu Thr Ala Ile Ser Ser Asp Ala Phe Asp Ile
            100                 105                 110

Trp Gly Gln Gly Thr Met Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Tyr Pro Ala
                325                 330                 335

Pro Ile Glu Lys Thr Ile Ser Lys Ala Lys Gly Gln Pro Arg Glu Pro
            340                 345                 350
```

Gln Val Tyr Thr Leu Pro Pro Ser Arg Asp Glu Leu Thr Lys Asn Gln
355 360 365

Val Ser Leu Thr Cys Leu Val Lys Gly Phe Tyr Pro Ser Asp Ile Ala
370 375 380

Val Glu Trp Glu Ser Asn Gly Gln Pro Glu Asn Asn Tyr Lys Thr Thr
385 390 395 400

Pro Pro Val Leu Asp Ser Asp Gly Ser Phe Phe Leu Tyr Ser Lys Leu
405 410 415

Thr Val Asp Lys Ser Arg Trp Gln Gln Gly Asn Val Phe Ser Cys Ser
420 425 430

Val Met His Glu Ala Leu His Asn His Tyr Thr Gln Lys Ser Leu Ser
435 440 445

Leu Ser Pro
450

<210> 149
<211> 543
<212> PRT
<213> Homo sapiens

<400> 149

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Glu Met His Trp Val Arg Gln Ala Pro Gly Gln Gly Leu Glu Trp Met
35 40 45

Gly Ala Leu Asp Pro Lys Thr Gly Asp Thr Ala Tyr Ser Gln Lys Phe
50 55 60

Lys Gly Arg Val Thr Leu Thr Ala Asp Lys Ser Thr Ser Thr Ala Tyr
65 70 75 80

Met Glu Leu Ser Ser Leu Thr Ser Glu Asp Thr Ala Val Tyr Tyr Cys
85 90 95

Thr Arg Phe Tyr Ser Tyr Thr Tyr Trp Gly Gln Gly Thr Leu Val Thr
100 105 110

Val Ser Ser Ala Ser Thr Gln Ser Pro Ser Val Phe Pro Leu Thr Arg
        115                 120                 125

Cys Cys Lys Asn Ile Pro Ser Asn Ala Thr Ser Val Thr Leu Gly Cys
    130                 135                 140

Leu Ala Thr Gly Tyr Phe Pro Glu Pro Val Met Val Thr Trp Asp Thr
145                 150                 155                 160

Gly Ser Leu Asn Gly Thr Thr Met Thr Leu Pro Ala Thr Thr Leu Thr
                165                 170                 175

Leu Ser Gly His Tyr Ala Thr Ile Ser Leu Leu Thr Val Ser Gly Ala
            180                 185                 190

Trp Ala Lys Gln Met Phe Thr Cys Arg Val Ala His Thr Pro Ser Ser
        195                 200                 205

Thr Asp Trp Val Asp Asn Lys Thr Phe Ser Val Cys Ser Arg Asp Phe
    210                 215                 220

Thr Pro Pro Thr Val Lys Ile Leu Gln Ser Ser Cys Asp Gly Gly Gly
225                 230                 235                 240

His Phe Pro Pro Thr Ile Gln Leu Leu Cys Leu Val Ser Gly Tyr Thr
                245                 250                 255

Pro Gly Thr Ile Asn Ile Thr Trp Leu Glu Asp Gly Gln Val Met Asp
            260                 265                 270

Val Asp Leu Ser Thr Ala Ser Thr Thr Gln Glu Gly Glu Leu Ala Ser
        275                 280                 285

Thr Gln Ser Glu Leu Thr Leu Ser Gln Lys His Trp Leu Ser Asp Arg
    290                 295                 300

Thr Tyr Thr Cys Gln Val Thr Tyr Gln Gly His Thr Phe Glu Asp Ser
305                 310                 315                 320

Thr Lys Lys Cys Ala Asp Ser Asn Pro Arg Gly Val Ser Ala Tyr Leu
                325                 330                 335

Ser Arg Pro Ser Pro Phe Asp Leu Phe Ile Arg Lys Ser Pro Thr Ile
            340                 345                 350

Thr Cys Leu Val Val Asp Leu Ala Pro Ser Lys Gly Thr Val Asn Leu
        355                 360                 365

Thr Trp Ser Arg Ala Ser Gly Lys Pro Val Asn His Ser Thr Arg Lys
370 375 380

Glu Glu Lys Gln Arg Asn Gly Thr Leu Thr Val Thr Ser Thr Leu Pro
385 390 395 400

Val Gly Thr Arg Asp Trp Ile Glu Gly Glu Thr Tyr Gln Cys Arg Val
405 410 415

Thr His Pro His Leu Pro Arg Ala Leu Met Arg Ser Thr Thr Lys Thr
420 425 430

Ser Gly Pro Arg Ala Ala Pro Glu Val Tyr Ala Phe Ala Thr Pro Glu
435 440 445

Trp Pro Gly Ser Arg Asp Lys Arg Thr Leu Ala Cys Leu Ile Gln Asn
450 455 460

Phe Met Pro Glu Asp Ile Ser Val Gln Trp Leu His Asn Glu Val Gln
465 470 475 480

Leu Pro Asp Ala Arg His Ser Thr Thr Gln Pro Arg Lys Thr Lys Gly
485 490 495

Ser Gly Phe Phe Val Phe Ser Arg Leu Glu Val Thr Arg Ala Glu Trp
500 505 510

Glu Gln Lys Asp Glu Phe Ile Cys Arg Ala Val His Glu Ala Ala Ser
515 520 525

Pro Ser Gln Thr Val Gln Arg Ala Val Ser Val Asn Pro Gly Lys
530 535 540

<210> 150
<211> 219
<212> PRT
<213> Homo sapiens

<400> 150

Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Arg Asn Thr Tyr Leu His Trp Tyr Leu Gln Lys Pro Gly Gln Ser
35 40 45

Pro Gln Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys
100 105 110

Arg Thr Val Ala Ala Pro Ser Val Phe Ile Phe Pro Pro Ser Asp Glu
115 120 125

Gln Leu Lys Ser Gly Thr Ala Ser Val Val Cys Leu Leu Asn Asn Phe
130 135 140

Tyr Pro Arg Glu Ala Lys Val Gln Trp Lys Val Asp Asn Ala Leu Gln
145 150 155 160

Ser Gly Asn Ser Gln Glu Ser Val Thr Glu Gln Asp Ser Lys Asp Ser
165 170 175

Thr Tyr Ser Leu Ser Ser Thr Leu Thr Leu Ser Lys Ala Asp Tyr Glu
180 185 190

Lys His Lys Val Tyr Ala Cys Glu Val Thr His Gln Gly Leu Ser Ser
195 200 205

Pro Val Thr Lys Ser Phe Asn Arg Gly Glu Cys
210 215

<210> 151
<211> 443
<212> PRT
<213> Artificial sequence

<220>
<223> An artificially synthesized peptide sequence

<400> 151

Gln Ser Leu Glu Glu Ser Gly Gly Arg Leu Val Thr Pro Gly Thr Pro
1 5 10 15

Leu Thr Leu Thr Cys Thr Ala Ser Gly Phe Ser Leu Ser Ser Tyr His
20 25 30

```
Met Ser Trp Val Arg Gln Ala Pro Gly Lys Gly Leu Glu Trp Ile Gly
        35                  40                  45

Val Ile Asn Ser Ala Gly Asn Thr Tyr Tyr Ala Ser Trp Ala Lys Gly
    50                  55                  60

Arg Phe Thr Val Ser Lys Thr Ser Thr Thr Val Asp Leu Asn Leu Thr
65                  70                  75                  80

Ser Leu Thr Thr Glu Asp Thr Ala Thr Tyr Phe Cys Ala Arg Tyr Val
                85                  90                  95

Phe Ser Ser Gly Ser His Asp Ile Trp Gly Pro Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Leu Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285
```

Pro Arg Glu Glu Gln Tyr Asn Ser Thr Tyr Arg Val Val Ser Val Leu
290 295 300

Thr Val Leu His Gln Asp Trp Leu Asn Gly Lys Glu Tyr Lys Cys Lys
305 310 315 320

Val Ser Asn Lys Ala Leu Pro Ala Pro Ile Glu Lys Thr Ile Ser Lys
325 330 335

Ala Lys Gly Gln Pro Arg Glu Pro Gln Val Tyr Thr Leu Pro Pro Ser
340 345 350

Arg Glu Glu Met Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Val Leu Asp Ser Asp Gly
385 390 395 400

Ser Phe Phe Leu Tyr Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Lys Ser Leu Ser Leu Ser Pro
435 440

<210> 152
<211> 217
<212> PRT
<213> Artificial sequence

<220>
<223> An artificially synthesized peptide sequence

<400> 152

Ala Tyr Asp Met Thr Gln Thr Pro Ala Ser Val Glu Val Ala Val Gly
1 5 10 15

Gly Thr Val Thr Ile Lys Cys Gln Ala Ser Gln Ser Ile Gly Ser Trp
20 25 30

Leu Ala Trp Tyr Gln Gln Lys Pro Gly Gln Pro Pro Lys Glu Leu Ile
35 40 45

Tyr Gly Thr Ser Thr Leu Glu Ser Gly Val Pro Ser Arg Phe Ile Gly
    50                  55                  60

Ser Gly Ser Gly Thr Glu Phe Thr Leu Thr Ile Ser Gly Val Glu Cys
65                  70                  75                  80

Ala Asp Ala Ala Thr Tyr Tyr Cys Gln Gln Gly Tyr Ser Glu Asp Asn
                85                  90                  95

Ile Asp Asn Ala Phe Gly Gly Gly Thr Glu Val Val Val Lys Arg Thr
            100                 105                 110

Val Ala Ala Pro Ser Val Phe Ile Phe Pro Pro Ser Asp Glu Gln Leu
        115                 120                 125

Lys Ser Gly Thr Ala Ser Val Val Cys Leu Leu Asn Asn Phe Tyr Pro
    130                 135                 140

Arg Glu Ala Lys Val Gln Trp Lys Val Asp Asn Ala Leu Gln Ser Gly
145                 150                 155                 160

Asn Ser Gln Glu Ser Val Thr Glu Gln Asp Ser Lys Asp Ser Thr Tyr
                165                 170                 175

Ser Leu Ser Ser Thr Leu Thr Leu Ser Lys Ala Asp Tyr Glu Lys His
            180                 185                 190

Lys Val Tyr Ala Cys Glu Val Thr His Gln Gly Leu Ser Ser Pro Val
        195                 200                 205

Thr Lys Ser Phe Asn Arg Gly Glu Cys
    210                 215

<210> 153
<211> 32
<212> PRT
<213> Artificial sequence

<220>
<223> An artificially synthesized peptide sequence

<400> 153

Val Asp Asp Ala Pro Gly Asn Ser Gln Gln Ala Thr Pro Lys Asp Asn
1               5                   10                  15

Glu Ile Ser Thr Phe His Asn Leu Gly Asn Val His Ser Pro Leu Lys
            20                  25                  30

<210> 154
<211> 443
<212> PRT
<213> Artificial sequence

<220>
<223> An artificially synthesized peptide sequence

<400> 154

Gln Ser Leu Glu Glu Ser Gly Gly Arg Leu Val Thr Pro Gly Thr Pro
1 5 10 15

Leu Thr Leu Thr Cys Thr Ala Ser Gly Phe Ser Leu Ser Ser Tyr His
20 25 30

Met Ser Trp Val Arg Gln Ala Pro Gly Lys Gly Leu Glu Trp Ile Gly
35 40 45

Val Ile Asn Ser Ala Gly Asn Thr Tyr Tyr Ala Ser Trp Ala Lys Gly
50 55 60

Arg Phe Thr Val Ser Lys Thr Ser Thr Thr Val Asp Leu Asn Leu Thr
65 70 75 80

Ser Leu Thr Thr Glu Asp Thr Ala Thr Tyr Phe Cys Ala Arg Tyr Val
85 90 95

Phe Ser Ser Gly Ser His Asp Ile Trp Gly Pro Gly Thr Leu Val Thr
100 105 110

Val Ser Ser Ala Ser Thr Lys Gly Pro Ser Val Phe Pro Leu Ala Pro
115 120 125

Ser Ser Lys Ser Thr Ser Gly Gly Thr Ala Ala Leu Gly Cys Leu Val
130 135 140

Lys Asp Tyr Phe Pro Glu Pro Val Thr Val Ser Trp Asn Ser Gly Ala
145 150 155 160

Leu Thr Ser Gly Val His Thr Phe Pro Ala Val Leu Gln Ser Ser Gly
165 170 175

Leu Tyr Ser Leu Ser Ser Val Val Thr Val Pro Ser Ser Ser Leu Gly
180 185 190

Thr Gln Thr Tyr Ile Cys Asn Val Asn His Lys Pro Ser Asn Thr Lys
195 200 205

Val Asp Lys Lys Val Glu Pro Lys Ser Cys Asp Lys Thr His Thr Cys

210 215 220

Pro Pro Cys Pro Ala Pro Glu Leu Leu Gly Gly Pro Ser Val Phe Leu
225 230 235 240

Phe Pro Pro Lys Pro Lys Asp Thr Leu Met Ile Ser Arg Thr Pro Glu
245 250 255

Val Thr Cys Val Val Val Asp Val Ser His Glu Asp Pro Glu Val Lys
260 265 270

Phe Asn Trp Tyr Val Asp Gly Val Glu Val His Asn Ala Lys Thr Lys
275 280 285

Pro Arg Glu Glu Gln Tyr Asn Ser Thr Tyr Arg Val Val Ser Val Leu
290 295 300

Thr Val Leu His Gln Asp Trp Leu Asn Gly Lys Glu Tyr Lys Cys Lys
305 310 315 320

Val Ser Asn Lys Ala Tyr Pro Ala Pro Ile Glu Lys Thr Ile Ser Lys
325 330 335

Ala Lys Gly Gln Pro Arg Glu Pro Gln Val Tyr Thr Leu Pro Pro Ser
340 345 350

Arg Asp Glu Leu Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Val Leu Asp Ser Asp Gly
385 390 395 400

Ser Phe Phe Leu Tyr Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Lys Ser Leu Ser Leu Ser Pro
435 440

<210> 155
<211> 543
<212> PRT
<213> Artificial Sequence

<220>
<223> An artificially synthesized peptide sequence

<400> 155

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Glu Met His Trp Val Arg Gln Ala Pro Gly Gln Gly Leu Glu Trp Met
35 40 45

Gly Ala Leu Asp Pro Lys Thr Gly Asp Thr Ala Tyr Ser Gln Lys Phe
50 55 60

Lys Gly Arg Val Thr Leu Thr Ala Asp Lys Ser Thr Ser Thr Ala Tyr
65 70 75 80

Met Glu Leu Ser Ser Leu Thr Ser Glu Asp Thr Ala Val Tyr Tyr Cys
85 90 95

Thr Arg Phe Tyr Ser Tyr Thr Tyr Trp Gly Gln Gly Thr Leu Val Thr
100 105 110

Val Ser Ser Ala Ser Thr Gln Ser Pro Ser Val Phe Pro Leu Thr Arg
115 120 125

Cys Cys Lys Asn Ile Pro Ser Asp Ala Thr Ser Val Thr Leu Gly Cys
130 135 140

Leu Ala Thr Gly Tyr Phe Pro Glu Pro Val Met Val Thr Trp Asp Thr
145 150 155 160

Gly Ser Leu Asp Gly Thr Thr Met Thr Leu Pro Ala Thr Thr Leu Thr
165 170 175

Leu Ser Gly His Tyr Ala Thr Ile Ser Leu Leu Thr Val Ser Gly Ala
180 185 190

Trp Ala Lys Gln Met Phe Thr Cys Arg Val Ala His Thr Pro Ser Ser
195 200 205

Thr Asp Trp Val Asp Asp Lys Thr Phe Ser Val Cys Ser Arg Asp Phe
210 215 220

Thr Pro Pro Thr Val Lys Ile Leu Gln Ser Ser Cys Asp Gly Gly Gly

225 230 235 240

His Phe Pro Pro Thr Ile Gln Leu Leu Cys Leu Val Ser Gly Tyr Thr
245 250 255

Pro Gly Thr Ile Asp Ile Thr Trp Leu Glu Asp Gly Gln Val Met Asp
260 265 270

Val Asp Leu Ser Thr Ala Ser Thr Thr Gln Glu Gly Glu Leu Ala Ser
275 280 285

Thr Gln Ser Glu Leu Thr Leu Ser Gln Lys His Trp Leu Ser Asp Arg
290 295 300

Thr Tyr Thr Cys Gln Val Thr Tyr Gln Gly His Thr Phe Glu Asp Ser
305 310 315 320

Thr Lys Lys Cys Ala Asp Ser Asn Pro Arg Gly Val Ser Ala Tyr Leu
325 330 335

Ser Arg Pro Ser Pro Phe Asp Leu Phe Ile Arg Lys Ser Pro Thr Ile
340 345 350

Thr Cys Leu Val Val Asp Leu Ala Pro Ser Lys Gly Thr Val Asp Leu
355 360 365

Thr Trp Ser Arg Ala Ser Gly Lys Pro Val Asp His Ser Thr Arg Lys
370 375 380

Glu Glu Lys Gln Arg Asn Gly Thr Leu Thr Val Thr Ser Thr Leu Pro
385 390 395 400

Val Gly Thr Arg Asp Trp Ile Glu Gly Glu Thr Tyr Gln Cys Arg Val
405 410 415

Thr His Pro His Leu Pro Arg Ala Leu Met Arg Ser Thr Thr Lys Thr
420 425 430

Ser Gly Pro Arg Ala Ala Pro Glu Val Tyr Ala Phe Ala Thr Pro Glu
435 440 445

Trp Pro Gly Ser Arg Asp Lys Arg Thr Leu Ala Cys Leu Ile Gln Asn
450 455 460

Phe Met Pro Glu Asp Ile Ser Val Gln Trp Leu His Asn Glu Val Gln
465 470 475 480

Leu Pro Asp Ala Arg His Ser Thr Thr Gln Pro Arg Lys Thr Lys Gly
485 490 495

Ser Gly Phe Phe Val Phe Ser Arg Leu Glu Val Thr Arg Ala Glu Trp
500 505 510

Glu Gln Lys Asp Glu Phe Ile Cys Arg Ala Val His Glu Ala Ala Ser
515 520 525

Pro Ser Gln Thr Val Gln Arg Ala Val Ser Val Asn Pro Gly Lys
530 535 540

<210> 156
<211> 443
<212> PRT
<213> Artificial sequence

<220>
<223> An artificially synthesized peptide sequence

<400> 156

Gln Ser Leu Glu Glu Ser Gly Gly Arg Leu Val Thr Pro Gly Thr Pro
1 5 10 15

Leu Thr Leu Thr Cys Thr Ala Ser Gly Phe Ser Leu Ser Ser Tyr His
20 25 30

Met Ser Trp Val Arg Gln Ala Pro Gly Lys Gly Leu Glu Trp Ile Gly
35 40 45

Val Ile Asn Ser Ala Gly Asn Thr Tyr Tyr Ala Ser Trp Ala Lys Gly
50 55 60

Arg Phe Thr Val Ser Lys Thr Ser Thr Thr Val Asp Leu Asn Leu Thr
65 70 75 80

Ser Leu Thr Thr Glu Asp Thr Ala Thr Tyr Phe Cys Ala Arg Tyr Val
85 90 95

Phe Ser Ser Gly Ser His Asp Ile Trp Gly Pro Gly Thr Leu Val Thr
100 105 110

Val Ser Ser Ala Ser Thr Lys Gly Pro Ser Val Phe Pro Leu Ala Pro
115 120 125

Ser Ser Lys Ser Thr Ser Gly Gly Thr Ala Ala Leu Gly Cys Leu Val
130 135 140

Lys Asp Tyr Phe Pro Glu Pro Val Thr Val Ser Trp Asn Ser Gly Ala

145 150 155 160

Leu Thr Ser Gly Val His Thr Phe Pro Ala Val Leu Gln Ser Ser Gly
165 170 175

Leu Tyr Ser Leu Ser Ser Val Val Thr Val Pro Ser Ser Ser Leu Gly
180 185 190

Thr Gln Thr Tyr Ile Cys Asn Val Asn His Lys Pro Ser Asn Thr Lys
195 200 205

Val Asp Lys Lys Val Glu Pro Lys Ser Cys Asp Lys Thr His Thr Cys
210 215 220

Pro Pro Cys Pro Ala Pro Glu Leu Leu Gly Gly Pro Ser Val Phe Leu
225 230 235 240

Phe Pro Pro Lys Pro Lys Asp Thr Leu Met Ile Ser Arg Thr Pro Glu
245 250 255

Val Thr Cys Val Val Val Asp Val Ser His Glu Asp Pro Glu Val Lys
260 265 270

Phe Asn Trp Tyr Val Asp Gly Val Glu Val His Asn Ala Lys Thr Lys
275 280 285

Pro Arg Glu Glu Gln Tyr Asn Ser Thr Tyr Arg Val Val Ser Val Leu
290 295 300

Thr Val Leu His Gln Asp Trp Leu Asn Gly Lys Glu Tyr Lys Cys Lys
305 310 315 320

Val Ser Asn Asp Ala Tyr Pro Ala Pro Ile Glu Lys Thr Ile Ser Lys
325 330 335

Ala Lys Gly Gln Pro Arg Glu Pro Gln Val Tyr Thr Leu Pro Pro Ser
340 345 350

Arg Asp Glu Leu Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Val Leu Asp Ser Asp Gly
385 390 395 400

Ser Phe Phe Leu Tyr Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Lys Ser Leu Ser Leu Ser Pro
435 440

<210> 157
<211> 443
<212> PRT
<213> Artificial sequence

<220>
<223> An artificially synthesized peptide sequence

<400> 157

Gln Ser Leu Glu Glu Ser Gly Gly Arg Leu Val Thr Pro Gly Thr Pro
1 5 10 15

Leu Thr Leu Thr Cys Thr Ala Ser Gly Phe Ser Leu Ser Ser Tyr His
20 25 30

Met Ser Trp Val Arg Gln Ala Pro Gly Lys Gly Leu Glu Trp Ile Gly
35 40 45

Val Ile Asn Ser Ala Gly Asn Thr Tyr Tyr Ala Ser Trp Ala Lys Gly
50 55 60

Arg Phe Thr Val Ser Lys Thr Ser Thr Thr Val Asp Leu Asn Leu Thr
65 70 75 80

Ser Leu Thr Thr Glu Asp Thr Ala Thr Tyr Phe Cys Ala Arg Tyr Val
85 90 95

Phe Ser Ser Gly Ser His Asp Ile Trp Gly Pro Gly Thr Leu Val Thr
100 105 110

Val Ser Ser Ala Ser Thr Lys Gly Pro Ser Val Phe Pro Leu Ala Pro
115 120 125

Ser Ser Lys Ser Thr Ser Gly Gly Thr Ala Ala Leu Gly Cys Leu Val
130 135 140

Lys Asp Tyr Phe Pro Glu Pro Val Thr Val Ser Trp Asn Ser Gly Ala
145 150 155 160

Leu Thr Ser Gly Val His Thr Phe Pro Ala Val Leu Gln Ser Ser Gly

165 170 175

Leu Tyr Ser Leu Ser Ser Val Val Thr Val Pro Ser Ser Ser Leu Gly
180 185 190

Thr Gln Thr Tyr Ile Cys Asn Val Asn His Lys Pro Ser Asn Thr Lys
195 200 205

Val Asp Lys Lys Val Glu Pro Lys Ser Cys Asp Lys Thr His Thr Cys
210 215 220

Pro Pro Cys Pro Ala Pro Glu Leu Arg Gly Gly Pro Lys Val Phe Leu
225 230 235 240

Phe Pro Pro Lys Pro Lys Asp Thr Leu Met Ile Ser Arg Thr Pro Glu
245 250 255

Val Thr Cys Val Val Val Asp Val Ser His Glu Asp Pro Glu Val Lys
260 265 270

Phe Asn Trp Tyr Val Asp Gly Val Glu Val His Asn Ala Lys Thr Lys
275 280 285

Pro Arg Glu Glu Gln Tyr Asn Ser Thr Tyr Arg Val Val Ser Val Leu
290 295 300

Thr Val Leu His Gln Asp Trp Leu Asn Gly Lys Glu Tyr Lys Cys Lys
305 310 315 320

Val Ser Asn Lys Ala Leu Pro Ala Pro Ile Glu Lys Thr Ile Ser Lys
325 330 335

Ala Lys Gly Gln Pro Arg Glu Pro Gln Val Tyr Thr Leu Pro Pro Ser
340 345 350

Arg Asp Glu Leu Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Val Leu Asp Ser Asp Gly
385 390 395 400

Ser Phe Phe Leu Tyr Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Lys Ser Leu Ser Leu Ser Pro
435 440

<210> 158
<211> 545
<212> PRT
<213> Artificial Sequence

<220>
<223> An artificially synthesized peptide sequence

<400> 158

Gln Pro Pro Pro Pro Pro Pro Asp Ala Thr Cys His Gln Val Arg Ser
1 5 10 15

Phe Phe Gln Arg Leu Gln Pro Gly Leu Lys Trp Val Pro Glu Thr Pro
20 25 30

Val Pro Gly Ser Asp Leu Gln Val Cys Leu Pro Lys Gly Pro Thr Cys
35 40 45

Cys Ser Arg Lys Met Glu Glu Lys Tyr Gln Leu Thr Ala Arg Leu Asn
50 55 60

Met Glu Gln Leu Leu Gln Ser Ala Ser Met Glu Leu Lys Phe Leu Ile
65 70 75 80

Ile Gln Asn Ala Ala Val Phe Gln Glu Ala Phe Glu Ile Val Val Arg
85 90 95

His Ala Lys Asn Tyr Thr Asn Ala Met Phe Lys Asn Asn Tyr Pro Ser
100 105 110

Leu Thr Pro Gln Ala Phe Glu Phe Val Gly Glu Phe Phe Thr Asp Val
115 120 125

Ser Leu Tyr Ile Leu Gly Ser Asp Ile Asn Val Asp Asp Met Val Asn
130 135 140

Glu Leu Phe Asp Ser Leu Phe Pro Val Ile Tyr Thr Gln Leu Met Asn
145 150 155 160

Pro Gly Leu Pro Asp Ser Ala Leu Asp Ile Asn Glu Cys Leu Arg Gly
165 170 175

Ala Arg Arg Asp Leu Lys Val Phe Gly Asn Phe Pro Lys Leu Ile Met

180 185 190

Thr Gln Val Ser Lys Ser Leu Gln Val Thr Arg Ile Phe Leu Gln Ala
195 200 205

Leu Asn Leu Gly Ile Glu Val Ile Asn Thr Thr Asp His Leu Lys Phe
210 215 220

Ser Lys Asp Cys Gly Arg Met Leu Thr Arg Met Trp Tyr Cys Ser Tyr
225 230 235 240

Cys Gln Gly Leu Met Met Val Lys Pro Cys Gly Gly Tyr Cys Asn Val
245 250 255

Val Met Gln Gly Cys Met Ala Gly Val Val Glu Ile Asp Lys Tyr Trp
260 265 270

Arg Glu Tyr Ile Leu Ser Leu Glu Glu Leu Val Asn Gly Met Tyr Arg
275 280 285

Ile Tyr Asp Met Glu Asn Val Leu Leu Gly Leu Phe Ser Thr Ile His
290 295 300

Asp Ser Ile Gln Tyr Val Gln Lys Asn Ala Gly Lys Leu Thr Thr Thr
305 310 315 320

Ile Gly Lys Leu Cys Ala His Ser Gln Gln Arg Gln Tyr Arg Ser Ala
325 330 335

Tyr Tyr Pro Glu Asp Leu Phe Ile Asp Lys Lys Val Leu Lys Val Ala
340 345 350

His Val Glu His Glu Glu Thr Leu Ser Ser Arg Arg Arg Glu Leu Ile
355 360 365

Gln Lys Leu Lys Ser Phe Ile Ser Phe Tyr Ser Ala Leu Pro Gly Tyr
370 375 380

Ile Cys Ser His Ser Pro Val Ala Glu Asn Asp Thr Leu Cys Trp Asn
385 390 395 400

Gly Gln Glu Leu Val Glu Arg Tyr Ser Gln Lys Ala Ala Arg Asn Gly
405 410 415

Met Lys Asn Gln Phe Asn Leu His Glu Leu Lys Met Lys Gly Pro Glu
420 425 430

Pro Val Val Ser Gln Ile Ile Asp Lys Leu Lys His Ile Asn Gln Leu
        435                 440                 445

Leu Arg Thr Met Ser Met Pro Lys Gly Arg Val Leu Asp Lys Asn Leu
    450                 455                 460

Asp Glu Glu Gly Phe Glu Ala Gly Asp Cys Gly Asp Asp Glu Asp Glu
465                 470                 475                 480

Cys Ile Gly Gly Ala Gly Asp Gly Met Ile Lys Val Lys Asn Gln Leu
                485                 490                 495

Arg Phe Leu Ala Glu Leu Ala Tyr Asp Leu Asp Val Asp Asp Ala Pro
            500                 505                 510

Gly Asn Ser Gln Gln Ala Thr Pro Lys Asp Asn Glu Ile Ser Thr Phe
        515                 520                 525

His Asn Leu Gly Asn Val His Ser Pro Leu Lys His His His His His
    530                 535                 540

His
545

<210> 159
<211> 443
<212> PRT
<213> Artificial sequence

<220>
<223> An artificially synthesized peptide sequence

<400> 159

Gln Val Gln Leu Val Gln Ser Gly Ala Glu Val Lys Lys Pro Gly Ala
1               5                   10                  15

Ser Val Thr Val Ser Cys Lys Ala Ser Gly Tyr Thr Phe Thr Asp Tyr
            20                  25                  30

Glu Met His Trp Ile Arg Gln Pro Pro Gly Glu Gly Leu Glu Trp Ile
        35                  40                  45

Gly Ala Ile Asp Pro Lys Thr Gly Asp Thr Ala Tyr Ser Glu Ser Phe
    50                  55                  60

Gln Asp Arg Val Thr Leu Thr Ala Asp Lys Ser Thr Ser Thr Ala Tyr
65                  70                  75                  80

Met Glu Leu Ser Ser Leu Thr Ser Glu Asp Thr Ala Val Tyr Tyr Cys

85 90 95

Thr Arg Phe Tyr Ser Tyr Thr Tyr Trp Gly Gln Gly Thr Leu Val Thr
100 105 110

Val Ser Ser Ala Ser Thr Lys Gly Pro Ser Val Phe Pro Leu Ala Pro
115 120 125

Ser Ser Lys Ser Thr Ser Gly Gly Thr Ala Ala Leu Gly Cys Leu Val
130 135 140

Lys Asp Tyr Phe Pro Glu Pro Val Thr Val Ser Trp Asn Ser Gly Ala
145 150 155 160

Leu Thr Ser Gly Val His Thr Phe Pro Ala Val Leu Gln Ser Ser Gly
165 170 175

Leu Tyr Ser Leu Ser Ser Val Val Thr Val Pro Ser Ser Ser Leu Gly
180 185 190

Thr Gln Thr Tyr Ile Cys Asn Val Asn His Lys Pro Ser Asn Thr Lys
195 200 205

Val Asp Lys Lys Val Glu Pro Lys Ser Cys Asp Lys Thr His Thr Cys
210 215 220

Pro Pro Cys Pro Ala Pro Glu Leu Leu Gly Gly Pro Ser Val Phe Leu
225 230 235 240

Phe Pro Pro Lys Pro Lys Asp Thr Leu Met Ile Ser Arg Thr Pro Glu
245 250 255

Val Thr Cys Val Val Val Asp Val Ser His Glu Asp Pro Glu Val Lys
260 265 270

Phe Asn Trp Tyr Val Asp Gly Val Glu Val His Asn Ala Lys Thr Lys
275 280 285

Pro Arg Glu Glu Gln Tyr Asn Ser Thr Tyr Arg Val Val Ser Val Leu
290 295 300

Thr Val Leu His Gln Asp Trp Leu Asn Gly Lys Glu Tyr Lys Cys Lys
305 310 315 320

Val Ser Asn Lys Ala Leu Pro Ala Pro Ile Glu Lys Thr Ile Ser Lys
325 330 335

```
Ala Lys Gly Gln Pro Arg Glu Pro Gln Val Tyr Thr Leu Pro Pro Ser
            340                 345                 350

Arg Asp Glu Leu Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Glu Ser Leu Ser Leu Ser Pro
        435                 440


<210>  160
<211>  219
<212>  PRT
<213>  Artificial sequence

<220>
<223>  An artificially synthesized peptide sequence

<400>  160

Asp Ile Val Met Thr Gln Ser Pro Leu Ser Leu Pro Val Thr Pro Gly
1               5                   10                  15

Glu Pro Ala Ser Ile Ser Cys Gln Ala Ser Glu Ser Leu Val His Ser
            20                  25                  30

Asn Arg Asn Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Pro Thr Phe Gly Gln Gly Thr Lys Val Glu Ile Glu
```

100 105 110

Arg Thr Val Ala Ala Pro Ser Val Phe Ile Phe Pro Pro Ser Asp Glu
115 120 125

Gln Leu Lys Ser Gly Thr Ala Ser Val Val Cys Leu Leu Asn Asn Phe
130 135 140

Tyr Pro Arg Glu Ala Lys Val Gln Trp Lys Val Asp Asn Ala Leu Gln
145 150 155 160

Ser Gly Asn Ser Gln Glu Ser Val Thr Glu Gln Asp Ser Lys Asp Ser
165 170 175

Thr Tyr Ser Leu Ser Ser Thr Leu Thr Leu Ser Lys Ala Asp Tyr Glu
180 185 190

Lys His Lys Val Tyr Ala Cys Glu Val Thr His Gln Gly Leu Ser Ser
195 200 205

Pro Val Thr Lys Ser Phe Asn Arg Gly Glu Cys
210 215

<210> 161
<211> 328
<212> PRT
<213> Artificial sequence

<220>
<223> An artificially synthesized peptide sequence

<400> 161

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

```
Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro
                325

<210>  162
```

<211> 325
<212> PRT
<213> Artificial sequence

<220>
<223> An artificially synthesized peptide sequence

<400> 162

```
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220
```

Glu Pro Gln Val Tyr Thr Leu Pro Pro Ser Gln Glu Glu Met Thr Lys
225 230 235 240

Asn Gln Val Ser Leu Thr Cys Leu Val Lys Gly Phe Tyr Pro Ser Asp
245 250 255

Ile Ala Val Glu Trp Glu Ser Asn Gly Gln Pro Glu Asn Asn Tyr Lys
260 265 270

Thr Thr Pro Pro Val Leu Asp Ser Asp Gly Ser Phe Phe Leu Tyr Ser
275 280 285

Arg Leu Thr Val Asp Lys Ser Arg Trp Gln Glu Gly Asn Val Phe Ser
290 295 300

Cys Ser Val Met His Glu Ala Leu His Asn His Tyr Thr Gln Lys Ser
305 310 315 320

Leu Ser Leu Ser Leu
325

<210> 163
<211> 328
<212> PRT
<213> Artificial Sequence

<220>
<223> An artificially synthesized peptide sequence

<400> 163

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

```
Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro
                325
```

<210> 164
<211> 432

<212> PRT
<213> Artificial Sequence

<220>
<223> An artificially synthesized peptide sequence

<400> 164

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Ser Tyr
            20                  25                  30

Leu Asn Trp Tyr Gln Gln Lys Pro Gly Lys Ala Pro Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Gly Asn Thr Leu Pro Tyr
                85                  90                  95

Thr Phe Gly Gln Gly Thr Lys Val Glu Ile Lys Ala Ser Thr Lys Gly
            100                 105                 110

Pro Ser Val Phe Pro Leu Ala Pro Cys Ser Arg Ser Thr Ser Glu Ser
        115                 120                 125

Thr Ala Ala Leu Gly Cys Leu Val Lys Asp Tyr Phe Pro Glu Pro Val
    130                 135                 140

Thr Val Ser Trp Asn Ser Gly Ala Leu Thr Ser Gly Val His Thr Phe
145                 150                 155                 160

Pro Ala Val Leu Gln Ser Ser Gly Leu Tyr Ser Leu Ser Ser Val Val
                165                 170                 175

Thr Val Pro Ser Ser Ser Leu Gly Thr Lys Thr Tyr Thr Cys Asn Val
            180                 185                 190

Asp His Lys Pro Ser Asn Thr Lys Val Asp Lys Arg Val Glu Ser Lys
        195                 200                 205

Tyr Gly Pro Pro Cys Pro Pro Cys Pro Ala Pro Glu Phe Leu Gly Gly
    210                 215                 220
```

```
Pro Ser Val Phe Leu Phe Pro Pro Lys Pro Lys Asp Thr Leu Met Ile
225                 230                 235                 240

Ser Arg Thr Pro Glu Val Thr Cys Val Val Val Asp Val Ser Gln Glu
                245                 250                 255

Asp Pro Glu Val Gln Phe Asn Trp Tyr Val Asp Gly Val Glu Val His
            260                 265                 270

Asn Ala Lys Thr Lys Pro Arg Glu Glu Gln Phe Asn Ser Thr Tyr Arg
        275                 280                 285

Val Val Ser Val Leu Thr Val Leu His Gln Asp Trp Leu Asn Gly Lys
    290                 295                 300

Glu Tyr Lys Cys Lys Val Ser Asn Lys Gly Leu Pro Ser Ser Ile Glu
305                 310                 315                 320

Lys Thr Ile Ser Lys Ala Lys Gly Gln Pro Arg Glu Pro Gln Val Tyr
                325                 330                 335

Thr Leu Pro Pro Ser Gln Glu Glu Met Thr Lys Asn Gln Val Ser Leu
            340                 345                 350

Thr Cys Leu Val Lys Gly Phe Tyr Pro Ser Asp Ile Ala Val Glu Trp
        355                 360                 365

Glu Ser Asn Gly Gln Pro Glu Asn Asn Tyr Lys Thr Thr Pro Pro Val
    370                 375                 380

Leu Asp Ser Asp Gly Ser Phe Phe Leu Tyr Ser Arg Leu Thr Val Asp
385                 390                 395                 400

Lys Ser Arg Trp Gln Glu Gly Asn Val Phe Ser Cys Ser Val Met His
                405                 410                 415

Glu Ala Leu His Asn His Tyr Thr Gln Lys Ser Leu Ser Leu Ser Leu
            420                 425                 430
```

## Description

### Technical Field

The present invention provides antigen-binding molecules with enhanced intracellular uptake of a bound antigen, antigen-binding molecules in which the number of antigens that can be bound per single molecule is increased, antigen-binding molecules with improved pharmacokinetics, antigen-binding molecules with enchanced intracellular dissociation of extracellularly bound antigen, antigen-binding molecules with enhanced extracellular release in the antigen-unbound state, antigen-binding molecules having the function of decreasing the total antigen concentration or the free antigen concentration in plasma, pharmaceutical compositions comprising such an antigen-binding molecules, and methods for producing them.

### Background Art

Antibodies are drawing attention as pharmaceuticals as they are highly stable in plasma and have few side effects. A number of IgG-type antibody pharmaceuticals are now available on the market and many antibody pharmaceuticals are currently under development (Non-patent Documents 1 and 2). Meanwhile, various technologies applicable to second-generation antibody pharmaceuticals have been reported, including those that enhance effector function, antigen-binding ability, pharmacokinetics and stability, and those that reduce the risk of immunogenicity (Non-patent Document 3). In general, the requisite dose of an antibody pharmaceutical is very high. This in turn has led to problems such as high production cost as well as difficulty in producing subcutaneous formulations. In theory, the dose of an antibody pharmaceutical may be reduced by improving antibody pharmacokinetics or improving the affinity between antibodies and antigens.

Literature has reported methods for improving antibody pharmacokinetics using artificial substitution of amino acids in constant regions (Non-patent Documents 4 and 5). Similarly, affinity maturation has been reported as a technology for enhancing antigen-binding ability or antigen-neutralizing activity (Non-patent Document 6). This technology enables enhancement of antigen-binding activity by introduction of amino acid mutations into the CDR of a variable region or such. The enhancement of antigen-binding ability enables improvement of *in vitro* biological activity or reduction of dosage, and further enables improvement of *in vivo* efficacy (Non-patent Document 7).

The antigen-neutralizing capacity of a single antibody molecule depends on its affinity. By increasing the affinity, an antigen can be neutralized by a smaller amount of an antibody. Various methods can be used to enhance antibody affinity (Non-patent Document 6). Furthermore, if affinity could be made infinite by covalently binding an antibody to an antigen, a single antibody molecule could neutralize one antigen molecule (a divalent antibody can neutralize two antigen molecules). However, the stoichiometric neutralization of one antibody against one antigen (one divalent antibody against two antigens) is the limit of pre-existing methods, and thus it is impossible to completely neutralize an antigen with an amount of antibody smaller than the amount of antigen. In other words, the affinity enhancing effect has a limit (Non-patent Document 9). To prolong the neutralization effect of a neutralizing antibody for a certain period, the antibody must be administered at a dose higher than the amount of antigen produced in the body during the same period. With just the improvement of antibody pharmacokinetics or affinity maturation technology described above, there is thus a limitation in the reduction of the required antibody dose. Accordingly, in order to sustain an antibody's antigen-neutralizing effect for a target period with an amount of antibody smaller than the amount of antigen, a single antibody must neutralize multiple antigens. An antibody that binds to an antigen in a pH-dependent manner has recently been reported as a novel method for achieving the above objective (Patent Document 1). pH-dependent antigen-binding antibodies, which bind strongly to an antigen under neutral conditions in plasma and dissociate from the antigen under acidic conditions in the endosome, can dissociate from the antigen in the endosome. When a pH-dependent antigen-binding antibody dissociates from the antigen is recycled to the plasma by FcRn, it can bind to another antigen again. Thus, a single pH-dependent antigen-binding antibody can bind to a number of antigens repeatedly.

In addition, plasma retention of an antigen is very short as compared to antibodies recycled via FcRn binding. When an antibody with such long plasma retention binds to the antigen, the plasma retention time of the antigen-antibody complex is prolonged to the same retention time as that of the antibody. Thus, plasma retention of the antigen is prolonged by binding to the antibody, and thus the plasma antigen concentration is increased.

Accordingly, pH-dependent antigen-binding antibodies have effects that could not be accomplished by normal antibodies, since they can promote elimination of antigens from plasma compared to normal antibodies, by binding of a single antibody to a plurality of antigens. However, antibody engineering methods for improving the effects of pH-dependent antigen-binding antibodies that can bind repeatedly to antigens and that promote elimination of antigens from plasma have not been reported to date.

IgG antibodies have long retentivity in plasma due to their binding to FcRn. Binding between IgG and FcRn is observed only under acidic conditions (pH6.0), and the binding is hardly observed under neutral conditions (pH7.4). IgG antibodies are taken up into cells non-specifically, but upon binding to FcRn in the endosome under an intra-endosome acidic condition, they return to the cell surface, and dissociate from FcRn under neutral conditions in plasma. When mutations are introduced into an Fc region of IgG so that binding to FcRn in an acidic pH range condition is lost, recycling of the antibody from endosome to plasma does not take place and plasma retentivity of the antibodies is significantly impaired. A method for improving FcRn binding in an acidic pH range condition has been reported as a method for improving plasma retentivity of an IgG antibody. Improving binding to FcRn in an acidic pH range condition by introducing amino acid substitutions to an Fc region of IgG antibody leads to increased efficiency of antibody recycling from endosome to plasma, and as a result, plasma retentivity is improved.

Many studies have been carried out so far on antibody-dependent cellular cytotoxicity (hereinafter denoted as ADCC) and complement-dependent cytotoxicity (hereinafter denoted as CDC), which are effector functions of IgG class antibodies. It has been reported that in the human IgG class, antibodies of the IgG1 subclass have the highest ADCC activity and CDC activity (Non-Patent Document 13). Furthermore, antibody-dependent cell-mediated phagocytosis (ADCP), which is phagocytosis of target cells mediated by IgG class antibodies, is also suggested to be one of the antibody effector functions (Non-Patent Documents 14 and 15). Since IgG1 subclass antibodies can exert these effector functions against tumors, IgG1 subclass antibodies are used for most antibody pharmaceuticals against cancer antigens.

In order for IgG antibodies to mediate ADCC and ADCP activities, the Fc region of the IgG antibodies must bind to antibody receptors (hereinafter denoted as Fcγ receptor or FcγR) that are present on the surface of effector cells such as killer cells, natural killer cells, and activated macrophages. In humans, isoforms FcγRIa, FcγRIIa, FcγRIIb, FcγRIIIa, and FcγRIIIb have been reported as members of the Fcγ receptor protein family, and their respective allotypes have been reported as well (Non-Patent Document 16).

Enhancement of cytotoxic effector functions such as ADCC and ADCP has been drawing attention as a promising means for enhancing the antitumor effects of anticancer antibodies. Importance of Fcγ receptor-mediated effector functions aimed for antitumor effects of antibodies has been reported using mouse models (Non-Patent Documents 17 and 18). Furthermore, it was observed that clinical effects in humans correlated with the high-affinity polymorphic allotype (V158) and the low-affinity polymorphic allotype (F158) of FcγRIIIa (Non-Patent Document 19). These reports suggest that antibodies with an Fc region optimized for binding to a specific Fcγ receptor mediates stronger effector functions, and thereby exert more effective antitumor effects. The balance between the affinity of antibodies against the activating receptors including FcγRIa, FcγRIIa, FcγRIIIa, and FcγRIIIb, and the inhibitory receptors including FcγRIIb is an important factor in optimizing antibody effector functions. Enhancing the affinity to activating receptors may give antibodies a property to mediate stronger effector functions (Non-Patent Document 20), and therefore has been reported in various reports to date as an antibody engineering technique for improving or enhancing the antitumor activity of antibody pharmaceuticals against cancer antigens.

Regarding binding between the Fc region and Fcγ receptor, several amino acid residues in the antibody hinge region and the CH2 domain, and a sugar chain added to Asn at position 297 (EU numbering) bound to the CH2 domain have been shown as being important (Non-Patent Documents 13, 21, and 22). Focusing on this binding site, studies have so far been carried out on mutants of the Fc region having various Fcγ receptor binding properties, and Fc region mutants with higher affinity to activating Fcγ receptor have been obtained (Patent Documents 2 and 3). For example, Lazar *et al.* have succeeded in increasing the binding of human IgG1 to human FcγRIIIa (V158) by approximately 370 fold by substituting Ser at position 239, Ala at position 330, and Ile at position 332 (EU numbering) of human IgG1 with Asp, Leu, and Glu, respectively (Non-Patent Document 19 and Patent Document 2). The ratio of binding to FcγRIIIa and FcγRIIb (A/I ratio) for this mutant was approximately 9-fold that of the wild type. Furthermore, Shinkawa *et al.* have succeeded in increasing the binding to FcγRIIIa up to approximately 100 fold by removing fucose from the sugar chain added to Asn at position 297 (EU numbering) (Non-Patent Document 24). These methods can greatly improve the ADCC activity of human IgG1 compared to that of naturally-occurring human IgG1.

Thus, since Fcγ-receptor-binding activity plays an important role in cytotoxic activity in antibodies targeting membrane-type antigens, an isotype of human IgG1 with high FcγR-binding activity is used when cytotoxic activity is needed. Improvement of cytotoxic activity by enhancing Fcγ-receptor-binding activity is also widely used technique. On the other hand, the role played by Fcγ-receptor-binding activity in antibodies targeting soluble antigens is not known, and it has been thought that there is no difference between human IgG1 with high Fcγ-receptor-binding activity and human IgG2 and human IgG4 with low FcγR-binding activity. Therefore, to date, enhancement of Fcγ-receptor-binding activity has not been attempted for antibodies targeting soluble antigens, and their effects have not been reported.

[Prior Art Documents]

Patent Documents

[Patent Document 1] WO 2009/125825
[Patent Document 2] WO 2000/042072
[Patent Document 3] WO 2006/019447

Non-patent Documents

[Non-patent Document 1] Janice M Reichert, Clark J Rosensweig, Laura B Faden & Matthew C Dewitz, Monoclonal antibody successes in the clinic., Nat. Biotechnol. (2005) 23, 1073-1078

[Non-patent Document 2] Pavlou AK, Belsey MJ., The therapeutic antibodies market to 2008., Eur J Pharm Biopharm. (2005) 59 (3), 389-396

[Non-patent Document 3] Kim SJ, Park Y, Hong HJ., Antibody engineering for the development of therapeutic antibodies., Mol Cells. (2005) 20 (1), 17-29

[Non-patent Document 4] Hinton PR, Xiong JM, Johlfs MG, Tang MT, Keller S, Tsurushita N., An engineered human IgG1 antibody with longer serum half-life., J. Immunol. (2006) 176 (1), 346-356

[Non-patent Document 5] Ghetie V, Popov S, Borvak J, Radu C, Matesoi D, Medesan C, Ober RJ, Ward ES., Increasing the serum persistence of an IgG fragment by random mutagenesis., Nat. Biotechnol. (1997) 15 (7), 637-640

[Non-patent Document 6] Rajpal A, Beyaz N, Haber L, Cappuccilli G, Yee H, Bhatt RR, Takeuchi T, Lerner RA, Crea R., A general method for greatly improving the affinity of antibodies by using combinatorial libraries., Proc. Natl. Acad. Sci. U. S. A. (2005) 102 (24), 8466-8471

[Non-patent Document 7] Wu H, Pfarr DS, Johnson S, Brewah YA, Woods RM, Patel NK, White WI, Young JF, Kiener PA., Development of Motavizumab, an Ultra-potent Antibody for the Prevention of Respiratory Syncytial Virus Infection in the Upper and Lower Respiratory Tract., J. Mol. Biol. (2007) 368, 652-665

[Non-patent Document 8] Hanson CV, Nishiyama Y, Paul S., Catalytic antibodies and their applications., Curr Opin Biotechnol. (2005) 16 (6), 631-636

[Non-patent Document 9] Rathanaswami P, Roalstad S, Roskos L, Su QJ, Lackie S, Babcook J., Demonstration of an in vivo generated sub-picomolar affinity fully human monoclonal antibody to interleukin-8., Biochem. Biophys. Res. Commun. (2005) 334 (4), 1004-1013

[Non-patent Document 10] Dall'Acqua WF, Woods RM, Ward ES, Palaszynski SR, Patel NK, Brewah YA, Wu H, Kiener PA, Langermann S., Increasing the affinity of a human IgG1 for the neonatal Fc receptor: biological consequences., J. Immunol. (2002) 169 (9), 5171-5180

[Non-patent Document 11] Yeung YA, Leabman MK, Marvin JS, Qiu J, Adams CW, Lien S, Starovasnik MA, Lowman HB., Engineering human IgG1 affinity to human neonatal Fc receptor: impact of affinity improvement on pharmacokinetics in primates., J. Immunol. (2009) 182 (12), 7663-7671

[Non-patent Document 12] Datta-Mannan A, Witcher DR, Tang Y, Watkins J, Wroblewski VJ., Monoclonal antibody clearance. Impact of modulating the interaction of IgG with the neonatal Fc receptor., J. Biol. Chem. (2007) 282 (3), 1709-1717

[Non-patent Document 13] Clark, M., Antibody Engineering IgG Effector Mechanisms., Chemical Immunology (1997) 65, 88-110

[Non-patent Document 14] Horton HM, Bernett MJ, Pong E, Peipp M, Karki S, Chu SY, Richards JO, Vostiar I, Joyce PF, Repp R, Desjarlais JR, Zhukovsky EA., Potent in vitro and in vivo activity of an Fc-engineered anti-CD 19 monoclonal antibody against lymphoma and leukemia., Cancer Res. (2008) 68, 8049-8057

[Non-patent Document 15] Zalevsky J, Leung IW, Karki S, Chu SY, Zhukovsky EA, Desjarlais JR, Carmichael DF, Lawrence CE., The impact of Fc engineering on an anti-CD19 antibody: increased Fcγ receptor affinity enhances B-cell clearing in nonhuman primates., Blood (2009) 113, 3735-3743

[Non-patent Document 16] Jefferis R, Lund J., Interaction sites on human IgG-Fc for FcgammaR: current models., Immunol. Lett. (2002) 82, 57-65

[Non-patent Document 17] Clynes, R., Yoshizumi, T., Moroi, Y., Houghton, A.N., and Ravetch, J.V., Fc Receptors are required for passive and active immunity to melanoma., Proc. Natl. Acad. Sci. U. S. A. (1998) 95, 652-656

[Non-patent Document 18] Clynes RA, Towers TL, Presta LG, Ravetch JV., Inhibitory Fc receptors modulate in vivo cytoxicity against tumor targets., Nat. Med. (2000) 6, 443-446

[Non-patent Document 19] Cartron G, Dacheux L, Salles G, Solal-Celigny P, Bardos P, Colombat P, Watier H., Therapeutic activity of humanized anti-CD20 monoclonal antibody and polymorphism in IgG Fc receptor FcgammaRIIIa gene., Blood (2002) 99, 754-758

[Non-patent Document 20] Nimmerjahn F, Ravetch JV., Divergent immunoglobulin g subclass activity through selective Fc receptor binding., Science (2005) 310, 1510-1512

[Non-patent Document 21] Greenwood J, Clark M, Waldmann H., Structural motifs involved in human IgG antibody effector functions., Eur. J. Immunol. (1993) 23, 1098-1104
[Non-patent Document 22] Morgan A, Jones ND, Nesbitt AM, Chaplin L, Bodmer MW, Emtage JS., The N-terminal end of the CH2 domain of chimeric human IgG1 anti-HLA-DR is necessary for C1q, Fc gamma RI and Fc gamma RIII binding., Immunology (1995) 86, 319-324
[Non-patent Document 23] Lazar GA, Dang W, Karki S, Vafa O, Peng JS, Hyun L, Chan C, Chung HS, Eivazi A, Yoder SC, Vielmetter J, Carmichael DF, Hayes RJ, Dahiyat BI., Engineered antibody Fc variants with enhanced effector function., Proc. Nat. Acad. Sci. U. S. A. (2006) 103, 4005-4010
[Non-patent Document 24] Shinkawa T, Nakamura K, Yamane N, Shoji-Hosaka E, Kanda Y, Sakurada M, Uchida K, Anazawa H, Satoh M, Yamasaki M, Hanai N, Shitara K., The absence of fucose but not the presence of galactose or bisecting N-acetylglucosamine of human IgG1 complex-type oligosaccharides shows the critical role of enhancing antibody-dependent cellular cytotoxicity., J. Biol. Chem. (2003) 278, 3466-3473

## Summary of the Invention

### [Problems to be Solved by the Invention]

The present invention was achieved in view of the above circumstances. An objective of the present invention is to provide antigen-binding molecule with enhanced intracellular uptake of a bound antigen, antigen-binding molecules in which the number of antigens that can be bound per single molecule is increased, antigen-binding molecules with improved pharmacokinetics, antigen-binding molecules with enchanced intracellular dissociation of extracellularly bound antigen, antigen-binding molecules with enhanced extracellular release in the antigen-unbound state, antigen-binding molecules having the function of decreasing the total antigen concentration or the free antigen concentration in plasma, pharmaceutical compositions comprising such an antigen-binding molecules, and methods for producing them.

### [Means for Solving the Problems]

As a result of conducting dedicated research to accomplish the above-mentioned objectives, the present inventors created an antigen-binding molecule containing an antigen-binding domain having human-FcRn-binding activity in an acidic pH range condition and in which the antigen-binding activity of an antigen-binding molecule changes depending on ion-concentration, and an Fcγ receptor-binding domain having a binding activity higher to the Fcγ receptor in a neutral pH range condition than the Fcy-receptor-binding domain of an Fc region of a native human IgG in which the sugar chain bonded at position 297 (EU numbering) is a fucose-containing sugar chain. Furthermore, the present inventors discovered a method for enhancing intracellular uptake of bound antigens, a method for increasing the number of antigens that can bind to a single antigen-binding molecule, a method for improving pharmacokinetics of an antigen-binding molecule, a method for promoting intracellular dissociation of an antigen, which is extracellularly bound to the antigen-binding molecule, from an antigen-binding molecule, a method for promoting extracellular release of the antigen-binding molecule not bound to an antigen, and a method for decreasing total antigen concentration or free antigen concentration in plasma, wherein the methods comprises contacting the antigen-binding molecule with an Fcy-receptor-expressing cell *in vivo* or *in vitro*. Furthermore, the inventors discovered methods for producing antigen-binding molecules having the above-mentioned properties, and also discovered the utility of pharmaceutical compositions containing, as an active ingredient, such an antigen-binding molecule or an antigen-binding molecule produced by the production method of the present invention, and thereby completed the present invention.
That is, more specifically, the present invention provides [1] to [46] below:

[1] A pharmaceutical composition which comprises an antigen-binding molecule comprising an antigen-binding domain and an Fcy-receptor-binding domain, wherein the antigen-binding molecule has human-FcRn-binding activity in an acidic pH range condition, and wherein the antigen-binding domain has antigen-binding activity that changes depending on an ion-concentration condition, and the Fcy-receptor-binding domain has higher binding activity to the Fcγ receptor in a neutral pH range condition than an Fc region of a native human IgG in which the sugar chain bound at position 297 according to EU numbering is a fucose-containing sugar chain.
[2] The pharmaceutical composition of [1], wherein the antigen is a soluble antigen.
[3] The pharmaceutical composition of [1] or [2], wherein the ion concentration is calcium ion concentration.
[4] The pharmaceutical composition of [3], wherein the antigen-binding domain is an antigen-binding domain in which binding activity to the antigen under a high-calcium-ion concentration condition is higher than that under a low-calcium-ion concentration condition.
[5] The pharmaceutical composition of [1] or [2], wherein the ion-concentration condition is a pH condition.
[6] The pharmaceutical composition of [5], wherein the antigen-binding domain is an antigen-binding domain in which binding activity to the antigen in a neutral pH range condition is higher than that in an acidic pH range condition.

[7] The pharmaceutical composition of any one of [1] to [6], wherein the antigen-binding molecule has neutralizing activity against the antigen.

[8] The pharmaceutical composition of any one of [1] to [7], wherein the Fcγ receptor-binding domain comprises an antibody Fc region.

[9] The pharmaceutical composition of [8], wherein the Fc region is an Fc region in which at least one or more amino acids selected from the group consisting of amino acids at positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, and 440 in the Fc region site according to EU numbering are different from amino acids at corresponding sites in a native Fc region.

[10] The pharmaceutical composition of [9], wherein the Fc region is an Fc region which comprises at least one or more amino acids selected from the group consisting of:

either Lys or Tyr at amino acid position 221;
any one of Phe, Trp, Glu, and Tyr at amino acid position 222;
any one of Phe, Trp, Glu, and Lys at amino acid position 223;
any one of Phe, Trp, Glu, and Tyr at amino acid position 224;
any one of Glu, Lys, and Trp at amino acid position 225;
any one of Glu, Gly, Lys, and Tyr at amino acid position 227;
any one of Glu, Gly, Lys, and Tyr at amino acid position 228;
any one of Ala, Glu, Gly, and Tyr at amino acid position 230;
any one of Glu, Gly, Lys, Pro, and Tyr at amino acid position 231;
any one of Glu, Gly, Lys, and Tyr at amino acid position 232;
any one of Ala, Asp, Phe, Gly, His, Ile, Lys, Leu, Met, Asn, Gln, Arg, Ser, Thr, Val, Trp, and Tyr at amino acid position 233;
any one of Ala, Asp, Glu, Phe, Gly, His, Ile, Lys, Met, Asn, Pro, Gln, Arg, Ser, Thr, Val, Trp, and Tyr at amino acid position 234;
any one of Ala, Asp, Glu, Phe, Gly, His, Ile, Lys, Met, Asn, Pro, Gln, Arg, Ser, Thr, Val, Trp, and Tyr at amino acid position 235;
any one of Ala, Asp, Glu, Phe, His, Ile, Lys, Leu, Met, Asn, Pro, Gln, Arg, Ser, Thr, Val, Trp, and Tyr at amino acid position 236;
any one of Asp, Glu, Phe, His, Ile, Lys, Leu, Met, Asn, Pro, Gln, Arg, Ser, Thr, Val, Trp, and Tyr at amino acid position 237;
any one of Asp, Glu, Phe, Gly, His, Ile, Lys, Leu, Met, Asn, Gln, Arg, Ser, Thr, Val, Trp, and Tyr at amino acid position 238;
any one of Asp, Glu, Phe, Gly, His, Ile, Lys, Leu, Met, Asn, Pro, Gln, Arg, Thr, Val, Trp, and Tyr at amino acid position 239;
any one of Ala, Ile, Met, and Thr at amino acid position 240;
any one of Asp, Glu, Leu, Arg, Trp, and Tyr at amino acid position 241;
any one of Leu, Glu, Leu, Gln, Arg, Trp, and Tyr at amino acid position 243;
His at amino acid position 244;
Ala at amino acid position 245;
any one of Asp, Glu, His, and Tyr at amino acid position 246;
any one of Ala, Phe, Gly, His, Ile, Leu, Met, Thr, Val, and Tyr at amino acid position 247;
any one of Glu, His, Gln, and Tyr at amino acid position 249;
either Glu or Gln at amino acid position 250;
Phe at amino acid position 251;
any one of Phe, Met, and Tyr at amino acid position 254;
any one of Glu, Leu, and Tyr at amino acid position 255;
any one of Ala, Met, and Pro at amino acid position 256;
any one of Asp, Glu, His, Ser, and Tyr at amino acid position 258;
any one of Asp, Glu, His, and Tyr at amino acid position 260;
any one of Ala, Glu, Phe, Ile, and Thr at amino acid position 262;
any one of Ala, Ile, Met, and Thr at amino acid position 263;
any one of Asp, Glu, Phe, Gly, His, Ile, Lys, Leu, Met, Asn, Pro, Gln, Arg, Ser, Thr, Trp, and Tyr at amino acid position 264;
any one of Ala, Leu, Phe, Gly, His, Ile, Lys, Leu, Met, Asn, Pro, Gln, Arg, Ser, Thr, Val, Trp, and Tyr at amino acid position 265;
any one of Ala, Ile, Met, and Thr at amino acid position 266;
any one of Asp, Glu, Phe, His, Ile, Lys, Leu, Met, Asn, Pro, Gln, Arg, Thr, Val, Trp, and Tyr at amino acid position 267;
any one of Asp, Glu, Phe, Gly, Ile, Lys, Leu, Met, Pro, Gln, Arg, Thr, Val, and Trp at amino acid position 268;
any one of Phe, Gly, His, Ile, Lys, Leu, Met, Asn, Pro, Arg, Ser, Thr, Val, Trp, and Tyr at amino acid position 269;
any one of Glu, Phe, Gly, His, Ile, Leu, Met, Pro, Gln, Arg, Ser, Thr, Trp, and Tyr at amino acid position 270;
any one of Ala, Asp, Glu, Phe, Gly, His, Ile, Lys, Leu, Met, Asn, Gln, Arg, Ser, Thr, Val, Trp, and Tyr at amino acid position 271;
any one of Asp, Phe, Gly, His, Ile, Lys, Leu, Met, Pro, Arg, Ser, Thr, Val, Trp, and Tyr at amino acid position 272;
either Phe or Ile at amino acid position 273;
any one of Asp, Glu, Phe, Gly, His, Ile, Leu, Met, Asn, Pro, Arg, Ser, Thr, Val, Trp, and Tyr at amino acid position 274;
either Leu or Trp at amino acid position 275;
any one of Asp, Glu, Phe, Gly, His, Ile, Leu, Met, Pro, Arg, Ser, Thr, Val, Trp, and Tyr at amino acid position 276;
any one of Asp, Glu, Gly, His, Ile, Lys, Leu, Met, Asn, Pro, Gln, Arg, Ser, Thr, Val, and Trp at amino acid position 278;
Ala at amino acid position 279;
any one of Ala, Gly, His, Lys, Leu, Pro, Gln, Trp, and Tyr at amino acid position 280;
any one of Asp, Lys, Pro, and Tyr at amino acid position 281;
any one of Glu, Gly, Lys, Pro, and Tyr at amino acid position 282;
any one of Ala, Gly, His, Ile, Lys, Leu, Met, Pro, Arg, and Tyr at amino acid position 283;
any one of Asp, Glu, Leu, Asn, Thr, and Tyr at amino acid position 284;
any one of Asp, Glu, Lys, Gln, Trp, and Tyr at amino acid position 285;
any one of Glu, Gly, Pro, and Tyr at amino acid position 286;
any one of Asn, Asp, Glu, and Tyr at amino acid position 288;
any one of Asp, Gly, His, Leu, Asn, Ser, Thr, Trp, and Tyr at amino acid position 290;
any one of Asp, Glu, Gly, His, Ile, Gln, and Thr at amino acid position 291;
any one of Ala, Asp, Glu, Pro, Thr, and Tyr at amino acid position 292;
any one of Phe, Gly, His, Ile, Leu, Met, Asn, Pro, Arg, Ser, Thr, Val, Trp, and Tyr at amino acid position 293;
any one of Phe, Gly, His, Ile, Lys, Leu, Met, Asn, Pro, Arg, Ser, Thr, Val, Trp, and Tyr at amino acid position 294;
any one of Asp, Glu, Phe, Gly, His, Ile, Lys, Met, Asn, Pro, Arg, Ser, Thr, Val, Trp, and Tyr at amino acid position 295;
any one of Ala, Asp, Glu, Gly, His, Ile, Lys, Leu, Met, Asn, Gln, Arg, Ser, Thr, and Val at amino acid position 296;
any one of Asp, Glu, Phe, Gly, His, Ile, Lys, Leu, Met, Pro, Gln, Arg, Ser, Thr, Val, Trp, and Tyr at amino acid position 297;
any one of Ala, Asp, Glu, Phe, His, Ile, Lys, Met, Asn, Gln, Arg, Thr, Val, Trp, and Tyr at amino acid position 298;
any one of Ala, Asp, Glu, Phe, Gly, His, Ile, Lys, Leu, Met, Asn, Pro, Gln, Arg, Ser, Val, Trp, and Tyr at amino acid position 299;
any one of Ala, Asp, Glu, Gly, His, Ile, Lys, Leu, Met, Asn, Pro, Gln, Arg, Ser, Thr, Val, and Trp at amino acid position 300;
any one of Asp, Glu, His, and Tyr at amino acid position 301;
Ile at amino acid position 302;
any one of Asp, Gly, and Tyr at amino acid position 303;
any one of Asp, His, Leu, Asn, and Thr at amino acid position 304;
any one of Glu, Ile, Thr, and Tyr at amino acid position 305;
any one of Ala, Asp, Asn, Thr, Val, and Tyr at amino acid position 311;
Phe at amino acid position 313;
Leu at amino acid position 315;
either Glu or Gln at amino acid position 317;
any one of His, Leu, Asn, Pro, Gln, Arg, Thr, Val, and Tyr at amino acid position 318;
any one of Asp, Phe, Gly, His, Ile, Leu, Asn, Pro, Ser, Thr, Val, Trp, and Tyr at amino acid position 320;
any one of Ala, Asp, Phe, Gly, His, Ile, Pro, Ser, Thr, Val, Trp, and Tyr at amino acid position 322;
Ile at amino acid position 323;
any one of Asp, Phe, Gly, His, Ile, Leu, Met, Pro, Arg, Thr, Val, Trp, and Tyr at amino acid position 324;
any one of Ala, Asp, Glu, Phe, Gly, His, Ile, Lys, Leu, Met, Pro, Gln, Arg, Ser, Thr, Val, Trp, and Tyr at amino acid position 325;
any one of Ala, Asp, Glu, Gly, Ile, Leu, Met, Asn, Pro, Gln, Ser, Thr, Val, Trp, and Tyr at amino acid position 326;
any one of Ala, Asp, Glu, Phe, Gly, His, Ile, Lys, Leu, Met, Asn, Pro, Arg, Thr, Val, Trp, and Tyr at amino acid position 327;
any one of Ala, Asp, Glu, Phe, Gly, His, Ile, Lys, Met, Asn, Pro, Gln, Arg, Ser, Thr, Val, Trp, and Tyr at amino acid position 328;
any one of Asp, Glu, Phe, Gly, His, Ile, Lys, Leu, Met, Asn, Gln, Arg, Ser, Thr, Val, Trp, and Tyr at amino acid position 329;
any one of Cys, Glu, Phe, Gly, His, Ile, Lys, Leu, Met, Asn, Pro, Arg, Ser, Thr, Val, Trp, and Tyr at amino acid position 330;
any one of Asp, Phe, His, Ile, Leu, Met, Gln, Arg, Thr, Val, Trp, and Tyr at amino acid position 331;
any one of Ala, Asp, Glu, Phe, Gly, His, Lys, Leu, Met, Asn, Pro, Gln, Arg, Ser, Thr, Val, Trp, and Tyr at amino acid position 332;
any one of Ala, Asp, Glu, Phe, Gly, His, Ile, Leu, Met, Pro, Ser, Thr, Val, and Tyr at amino acid position 333;
any one of Ala, Glu, Phe, Ile, Leu, Pro, and Thr at amino acid position 334;
any one of Asp, Phe, Gly, His, Ile, Leu, Met, Asn, Pro, Arg, Ser, Val, Trp, and Tyr at amino acid position 335;
any one of Glu, Lys, and Tyr at amino acid position 336;
any one of Glu, His, and Asn at amino acid position 337;
any one of Asp, Phe, Gly, Ile, Lys, Met, Asn, Gln, Arg, Ser, and Thr at amino acid position 339; either Ala or Val at amino acid position 376;
either Gly or Lys at amino acid position 377;
Asp at amino acid position 378;
Asn at amino acid position 379;
any one of Ala, Asn, and Ser at amino acid position 380;
either Ala or Ile at amino acid position 382;
Glu at amino acid position 385;
Thr at amino acid position 392;
Leu at amino acid position 396;
Lys at amino acid position 421;
Asn at amino acid position 427;
either Phe or Leu at amino acid position 428;
Met at amino acid position 429;
Trp at amino acid position 434;
Ile at amino acid position 436; and
any one of Gly, His, Ile, Leu, and Tyr at amino acid position 440,
in the Fc region site according to EU numbering.

[11] The pharmaceutical composition of any one of [1] to [10], wherein the Fc region of a native human IgG in which the sugar chain bound at position 297 according to EU numbering is a fucose-containing sugar chain, is an Fc region of any one of native human IgG1, native human IgG2, native human IgG3, and native human IgG4 in which the sugar chain bound at position 297 according to EU numbering is a fucose-containing sugar chain.
[12] The pharmaceutical composition of any one of [1] to [11], wherein the human Fcγ receptor is FcyRIa, FcyRIIa(R), FcγRIIa(H), FcγRIIb, FcγRIIIa(V), or FcγRIIIa(F).
[13] The pharmaceutical composition of any one of [1] to [11], wherein the human Fcγ receptor is FcγRIIb.
[14] The pharmaceutical composition of any one of [8] to [13], wherein the Fc region is an Fc region which comprises at least one or more of

Asp at amino acid position 238, and
Glu at amino acid position 328
in the Fc region site according to EU numbering.

[15] A method comprising the step of contacting an antigen-binding molecule with an Fcy-receptor-expressing cell *in vivo* or *ex vivo,* wherein the antigen-binding molecule has human-FcRn-binding activity in an acidic pH range condition and comprises an antigen-binding domain whose antigen-binding activity changes depending on the ion concentration condition and an Fcy-receptor-binding domain that has higher binding activity to the Fcγ receptor in a neutral pH range condition compared to an Fc region of a native human IgG in which the sugar chain bound at position 297 according to EU numbering is a fucose-containing sugar chain, which is a method of any one of:

(i) a method for increasing the number of antigens that can bind to a single antigen-binding molecule;
(ii) a method for eliminating plasma antigens;
(iii) a method for improving antigen-binding molecule pharmacokinetics;
(iv) a method for promoting intracellular dissociation of an antigen from an antigen-binding molecule, wherein the antigen has been extracellularly bound to the antigen-binding molecule;
(v) a method for promoting extracellular release of an antigen-binding molecule not bound to an antigen; and
(vi) a method for decreasing a total antigen concentration or free antigen concentration in plasma.

[16] The method of [15], wherein the antigen is a soluble antigen.
[17] The method of [15] or [16], wherein the ion concentration is a calcium ion concentration.
[18] The method of [17], wherein the antigen-binding domain is an antigen-binding domain in which binding activity to the antigen under a high-calcium-ion concentration condition is higher than that under a low-calcium-ion concentration condition.
[19] The method of [15] or [16], wherein the ion concentration condition is a pH condition.
[20] The method of [19], wherein the antigen-binding domain is an antigen-binding domain in which binding activity to the antigen in a neutral pH range condition is higher than that in an acidic pH range condition.
[21] The method of any one of [15] to [20], wherein the antigen-binding molecule has neutralizing activity against the antigen.
[22] The method of any one of [15] to [21], wherein the Fcγ receptor-binding domain comprises an antibody Fc region.
[23] The method of [22], wherein the Fc region is an Fc region in which at least one or more amino acids selected from the group consisting of amino acids at positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, and 440 in the Fc region site according to EU numbering are different from the amino acids at corresponding sites in the native Fc region.
[24] The method of [23], wherein the Fc region is an Fc region which comprises at least one or more amino acids selected from the group consisting of:

either Lys or Tyr at amino acid position 221;
any one of Phe, Trp, Glu, and Tyr at amino acid position 222;
any one of Phe, Trp, Glu, and Lys at amino acid position 223;
any one of Phe, Trp, Glu, and Tyr at amino acid position 224;
any one of Glu, Lys, and Trp at amino acid position 225;
any one of Glu, Gly, Lys, and Tyr at amino acid position 227;
any one of Glu, Gly, Lys, and Tyr at amino acid position 228;
any one of Ala, Glu, Gly, and Tyr at amino acid position 230;
any one of Glu, Gly, Lys, Pro, and Tyr at amino acid position 231;
any one of Glu, Gly, Lys, and Tyr at amino acid position 232;
any one of Ala, Asp, Phe, Gly, His, Ile, Lys, Leu, Met, Asn, Gln, Arg, Ser, Thr, Val, Trp, and Tyr at amino acid position 233;
any one of Ala, Asp, Glu, Phe, Gly, His, Ile, Lys, Met, Asn, Pro, Gln, Arg, Ser, Thr, Val, Trp, and Tyr at amino acid position 234;
any one of Ala, Asp, Glu, Phe, Gly, His, Ile, Lys, Met, Asn, Pro, Gln, Arg, Ser, Thr, Val, Trp, and Tyr at amino acid position 235;
any one of Ala, Asp, Glu, Phe, His, Ile, Lys, Leu, Met, Asn, Pro, Gln, Arg, Ser, Thr, Val, Trp, and Tyr at amino acid position 236;
any one of Asp, Glu, Phe, His, Ile, Lys, Leu, Met, Asn, Pro, Gln, Arg, Ser, Thr, Val, Trp, and Tyr at amino acid position 237;
any one of Asp, Glu, Phe, Gly, His, Ile, Lys, Leu, Met, Asn, Gln, Arg, Ser, Thr, Val, Trp, and Tyr at amino acid position 238;
any one of Asp, Glu, Phe, Gly, His, Ile, Lys, Leu, Met, Asn, Pro, Gln, Arg, Thr, Val, Trp, and Tyr at amino acid position 239;
any one of Ala, Ile, Met, and Thr at amino acid position 240;
any one of Asp, Glu, Leu, Arg, Trp, and Tyr at amino acid position 241;
any one of Leu, Glu, Leu, Gln, Arg, Trp, and Tyr at amino acid position 243;
His at amino acid position 244;

Ala at amino acid position 245;
any one of Asp, Glu, His, and Tyr at amino acid position 246;
any one of Ala, Phe, Gly, His, Ile, Leu, Met, Thr, Val, and Tyr at amino acid position 247;
any one of Glu, His, Gln, and Tyr at amino acid position 249;
either Glu or Gln at amino acid position 250;
Phe at amino acid position 251;
any one of Phe, Met, and Tyr at amino acid position 254;
any one of Glu, Leu, and Tyr at amino acid position 255;
any one of Ala, Met, and Pro at amino acid position 256;
any one of Asp, Glu, His, Ser, and Tyr at amino acid position 258;
any one of Asp, Glu, His, and Tyr at amino acid position 260;
any one of Ala, Glu, Phe, Ile, and Thr at amino acid position 262;
any one of Ala, Ile, Met, and Thr at amino acid position 263;
any one of Asp, Glu, Phe, Gly, His, Ile, Lys, Leu, Met, Asn, Pro, Gln, Arg, Ser, Thr, Trp, and Tyr at amino acid position 264;
any one of Ala, Leu, Phe, Gly, His, Ile, Lys, Leu, Met, Asn, Pro, Gln, Arg, Ser, Thr, Val, Trp, and Tyr at amino acid position 265;
any one of Ala, Ile, Met, and Thr at amino acid position 266;
any one of Asp, Glu, Phe, His, Ile, Lys, Leu, Met, Asn, Pro, Gln, Arg, Thr, Val, Trp, and Tyr at amino acid position 267;
any one of Asp, Glu, Phe, Gly, Ile, Lys, Leu, Met, Pro, Gln, Arg, Thr, Val, and Trp at amino acid position 268;
any one of Phe, Gly, His, Ile, Lys, Leu, Met, Asn, Pro, Arg, Ser, Thr, Val, Trp, and Tyr at amino acid position 269;
any one of Glu, Phe, Gly, His, Ile, Leu, Met, Pro, Gln, Arg, Ser, Thr, Trp, and Tyr at amino acid position 270;
any one of Ala, Asp, Glu, Phe, Gly, His, Ile, Lys, Leu, Met, Asn, Gln, Arg, Ser, Thr, Val, Trp, and Tyr at amino acid position 271;
any one of Asp, Phe, Gly, His, Ile, Lys, Leu, Met, Pro, Arg, Ser, Thr, Val, Trp, and Tyr at amino acid position 272;
either Phe or Ile at amino acid position 273;
any one of Asp, Glu, Phe, Gly, His, Ile, Leu, Met, Asn, Pro, Arg, Ser, Thr, Val, Trp, and Tyr at amino acid position 274;
either Leu or Trp at amino acid position 275;
any one of Asp, Glu, Phe, Gly, His, Ile, Leu, Met, Pro, Arg, Ser, Thr, Val, Trp, and Tyr at amino acid position 276;
any one of Asp, Glu, Gly, His, Ile, Lys, Leu, Met, Asn, Pro, Gln, Arg, Ser, Thr, Val, and Trp at amino acid position 278;
Ala at amino acid position 279;
any one of Ala, Gly, His, Lys, Leu, Pro, Gln, Trp, and Tyr at amino acid position 280;
any one of Asp, Lys, Pro, and Tyr at amino acid position 281;
any one of Glu, Gly, Lys, Pro, and Tyr at amino acid position 282;
any one of Ala, Gly, His, Ile, Lys, Leu, Met, Pro, Arg, and Tyr at amino acid position 283;
any one of Asp, Glu, Leu, Asn, Thr, and Tyr at amino acid position 284;
any one of Asp, Glu, Lys, Gln, Trp, and Tyr at amino acid position 285;
any one of Glu, Gly, Pro, and Tyr at amino acid position 286;
any one of Asn, Asp, Glu, and Tyr at amino acid position 288;
any one of Asp, Gly, His, Leu, Asn, Ser, Thr, Trp, and Tyr at amino acid position 290;
any one of Asp, Glu, Gly, His, Ile, Gln, and Thr at amino acid position 291;
any one of Ala, Asp, Glu, Pro, Thr, and Tyr at amino acid position 292;
any one of Phe, Gly, His, Ile, Leu, Met, Asn, Pro, Arg, Ser, Thr, Val, Trp, and Tyr at amino acid position 293;
any one of Phe, Gly, His, Ile, Lys, Leu, Met, Asn, Pro, Arg, Ser, Thr, Val, Trp, and Tyr at amino acid position 294;
any one of Asp, Glu, Phe, Gly, His, Ile, Lys, Met, Asn, Pro, Arg, Ser, Thr, Val, Trp, and Tyr at amino acid position 295;
any one of Ala, Asp, Glu, Gly, His, Ile, Lys, Leu, Met, Asn, Gln, Arg, Ser, Thr, and Val at amino acid position 296;
any one of Asp, Glu, Phe, Gly, His, Ile, Lys, Leu, Met, Pro, Gln, Arg, Ser, Thr, Val, Trp, and Tyr at amino acid position 297;
any one of Ala, Asp, Glu, Phe, His, Ile, Lys, Met, Asn, Gln, Arg, Thr, Val, Trp, and Tyr at amino acid position 298;
any one of Ala, Asp, Glu, Phe, Gly, His, Ile, Lys, Leu, Met, Asn, Pro, Gln, Arg, Ser, Val, Trp, and Tyr at amino acid position 299;
any one of Ala, Asp, Glu, Gly, His, Ile, Lys, Leu, Met, Asn, Pro, Gln, Arg, Ser, Thr, Val, and Trp at amino acid position 300;
any one of Asp, Glu, His, and Tyr at amino acid position 301;

Ile at amino acid position 302;
any one of Asp, Gly, and Tyr at amino acid position 303;
any one of Asp, His, Leu, Asn, and Thr at amino acid position 304;
any one of Glu, Ile, Thr, and Tyr at amino acid position 305;
any one of Ala, Asp, Asn, Thr, Val, and Tyr at amino acid position 311;
Phe at amino acid position 313;
Leu at amino acid position 315;
either Glu or Gln at amino acid position 317;
any one of His, Leu, Asn, Pro, Gln, Arg, Thr, Val, and Tyr at amino acid position 318;
any one of Asp, Phe, Gly, His, Ile, Leu, Asn, Pro, Ser, Thr, Val, Trp, and Tyr at amino acid position 320;
any one of Ala, Asp, Phe, Gly, His, Ile, Pro, Ser, Thr, Val, Trp, and Tyr at amino acid position 322;
Ile at amino acid position 323;
any one of Asp, Phe, Gly, His, Ile, Leu, Met, Pro, Arg, Thr, Val, Trp, and Tyr at amino acid position 324;
any one of Ala, Asp, Glu, Phe, Gly, His, Ile, Lys, Leu, Met, Pro, Gln, Arg, Ser, Thr, Val, Trp, and Tyr at amino acid position 325;
any one of Ala, Asp, Glu, Gly, Ile, Leu, Met, Asn, Pro, Gln, Ser, Thr, Val, Trp, and Tyr at amino acid position 326;
any one of Ala, Asp, Glu, Phe, Gly, His, Ile, Lys, Leu, Met, Asn, Pro, Arg, Thr, Val, Trp, and Tyr at amino acid position 327;
any one of Ala, Asp, Glu, Phe, Gly, His, Ile, Lys, Met, Asn, Pro, Gln, Arg, Ser, Thr, Val, Trp, and Tyr at amino acid position 328;
any one of Asp, Glu, Phe, Gly, His, Ile, Lys, Leu, Met, Asn, Gln, Arg, Ser, Thr, Val, Trp, and Tyr at amino acid position 329;
any one of Cys, Glu, Phe, Gly, His, Ile, Lys, Leu, Met, Asn, Pro, Arg, Ser, Thr, Val, Trp, and Tyr at amino acid position 330;
any one of Asp, Phe, His, Ile, Leu, Met, Gln, Arg, Thr, Val, Trp, and Tyr at amino acid position 331;
any one of Ala, Asp, Glu, Phe, Gly, His, Lys, Leu, Met, Asn, Pro, Gln, Arg, Ser, Thr, Val, Trp, and Tyr at amino acid position 332;
any one of Ala, Asp, Glu, Phe, Gly, His, Ile, Leu, Met, Pro, Ser, Thr, Val, and Tyr at amino acid position 333;
any one of Ala, Glu, Phe, Ile, Leu, Pro, and Thr at amino acid position 334;
any one of Asp, Phe, Gly, His, Ile, Leu, Met, Asn, Pro, Arg, Ser, Val, Trp, and Tyr at amino acid position 335;
any one of Glu, Lys, and Tyr at amino acid position 336;
any one of Glu, His, and Asn at amino acid position 337;
any one of Asp, Phe, Gly, Ile, Lys, Met, Asn, Gln, Arg, Ser, and Thr at amino acid position 339; either Ala or Val at amino acid position 376;
either Gly or Lys at amino acid position 377;
Asp at amino acid position 378;
Asn at amino acid position 379;
any one of Ala, Asn, and Ser at amino acid position 380;
either Ala or Ile at amino acid position 382;
Glu at amino acid position 385;
Thr at amino acid position 392;
Leu at amino acid position 396;
Lys at amino acid position 421;
Asn at amino acid position 427;
either Phe or Leu at amino acid position 428;
Met at amino acid position 429;
Trp at amino acid position 434;
Ile at amino acid position 436; and
any one of Gly, His, Ile, Leu, and Tyr at amino acid position 440,
in the Fc region site according to EU numbering.

[25] The method of any one of [15] to [24], wherein the Fc region of a native human IgG in which the sugar chain bound at position 297 according to EU numbering is a fucose-containing sugar chain, is an Fc region of any one of native human IgG1, native human IgG2, native human IgG3, and native human IgG4 in which the sugar chain bound at position 297 according to EU numbering is a fucose-containing sugar chain.
[26] The method of any one of [15] to [25], wherein the human Fcγ receptor is FcyRIa, FcγRIIa(R), FcγRIIa(H), FcγRIIb, FcγRIIIa(V), or FcγRIIIa(F).
[27] The method of any one of [15] to [25], wherein the human Fcγ receptor is FcγRIIb.

[28] The method of any one of [22] to [27], wherein the Fc region is an Fc region which comprises at least one or more of

Asp at amino acid position 238, and
Glu at amino acid position 328
in the Fc region site according to EU numbering.

[29] A method comprising the step of enhancing Fcy-receptor-binding activity in a neutral pH range condition of the Fcy-receptor-binding domain in an antigen-binding molecule compared to that of a native human IgG Fc region in which the sugar chain bound at position 297 according to EU numbering is a fucose-containing sugar chain, wherein the antigen-binding molecule has human-FcRn-binding activity in an acidic pH range condition and comprises an Fcγ receptor-binding domain and an antigen-binding domain whose antigen-binding activity changes depending on the ion concentration condition, which is a method of any one of:

(i) a method for altering an antigen-binding molecule, wherein the intracellular uptake of the antigen to which it binds is enhanced;
(ii) a method for increasing the number of antigens that can bind to a single molecule of antigen-binding molecule;
(iii) a method for increasing the ability of an antigen-binding molecule to eliminate plasma antigens;
(iv) a method for improving antigen-binding molecule pharmacokinetics;
(v) a method for promoting intracellular dissociation of an antigen from an antigen-binding molecule, wherein the antigen has been extracellularly bound to the antigen-binding molecule;
(vi) a method for promoting extracellular release of an antigen-binding molecule not bound to an antigen, wherein the antigen-binding molecule had been taken up into a cell in an antigen-bound form; and
(vii) a method for altering an antigen-binding molecule, which can decrease a total antigen concentration or free antigen concentration in plasma.

[30] The method of [29], wherein the antigen is a soluble antigen.
[31] The method of [29] or [30], wherein the ion concentration is a calcium ion concentration.
[32] The method of [31], wherein the antigen-binding domain is an antigen-binding domain in which binding activity to the antigen under high calcium ion concentration conditions is higher than that under low calcium ion concentration conditions.
[33] The method of [29] or [30], wherein the ion concentration condition is a pH condition.
[34] The method of [33], wherein the antigen-binding domain is an antigen-binding domain in which binding activity to the antigen in a neutral pH range condition is higher than that in an acidic pH range condition.
[35] The method of any one of [29] to [34], wherein the antigen-binding molecule has neutralizing activity against the antigen.
[36] The method of any one of [29] to [35], wherein the Fcγ receptor-binding domain comprises an antibody Fc region.
[37] The method of [36], wherein the Fc region is an Fc region in which at least one or more amino acids selected from the group consisting of amino acids at positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270,271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, and 440 in the Fc region site according to EU numbering are different from the amino acids at corresponding sites in the native Fc region.
[38] The method of [33], wherein the Fc region is an Fc region comprising at least one or more amino acids selected from the group consisting of:

either Lys or Tyr at amino acid position 221;
any one of Phe, Trp, Glu, and Tyr at amino acid position 222;
any one of Phe, Trp, Glu, and Lys at amino acid position 223;
any one of Phe, Trp, Glu, and Tyr at amino acid position 224;
any one of Glu, Lys, and Trp at amino acid position 225;
any one of Glu, Gly, Lys, and Tyr at amino acid position 227;
any one of Glu, Gly, Lys, and Tyr at amino acid position 228;
any one of Ala, Glu, Gly, and Tyr at amino acid position 230;
any one of Glu, Gly, Lys, Pro, and Tyr at amino acid position 231;
any one of Glu, Gly, Lys, and Tyr at amino acid position 232;
any one of Ala, Asp, Phe, Gly, His, Ile, Lys, Leu, Met, Asn, Gln, Arg, Ser, Thr, Val, Trp, and Tyr at amino acid position 233;
any one of Ala, Asp, Glu, Phe, Gly, His, Ile, Lys, Met, Asn, Pro, Gin, Arg, Ser, Thr, Val, Trp, and Tyr at amino acid position 234;
any one of Ala, Asp, Glu, Phe, Gly, His, Ile, Lys, Met, Asn, Pro, Gln, Arg, Ser, Thr, Val, Trp, and Tyr at amino acid position 235;
any one of Ala, Asp, Glu, Phe, His, Ile, Lys, Leu, Met, Asn, Pro, Gin, Arg, Ser, Thr, Val, Trp, and Tyr at amino acid position 236;
any one of Asp, Glu, Phe, His, Ile, Lys, Leu, Met, Asn, Pro, Gln, Arg, Ser, Thr, Val, Trp, and Tyr at amino acid position 237;
any one of Asp, Glu, Phe, Gly, His, Ile, Lys, Leu, Met, Asn, Gln, Arg, Ser, Thr, Val, Trp, and Tyr at amino acid position 238;
any one of Asp, Glu, Phe, Gly, His, Ile, Lys, Leu, Met, Asn, Pro, Gln, Arg, Thr, Val, Trp, and Tyr at amino acid position 239;
any one of Ala, Ile, Met, and Thr at amino acid position 240;
any one of Asp, Glu, Leu, Arg, Trp, and Tyr at amino acid position 241;
any one of Leu, Glu, Leu, Gln, Arg, Trp, and Tyr at amino acid position 243;
His at amino acid position 244;
Ala at amino acid position 245;
any one of Asp, Glu, His, and Tyr at amino acid position 246;
any one of Ala, Phe, Gly, His, Ile, Leu, Met, Thr, Val, and Tyr at amino acid position 247;
any one of Glu, His, Gln, and Tyr at amino acid position 249;
either Glu or Gln at amino acid position 250;
Phe at amino acid position 251;
any one of Phe, Met, and Tyr at amino acid position 254;
any one of Glu, Leu, and Tyr at amino acid position 255;
any one of Ala, Met, and Pro at amino acid position 256;
any one of Asp, Glu, His, Ser, and Tyr at amino acid position 258;
any one of Asp, Glu, His, and Tyr at amino acid position 260;
any one of Ala, Glu, Phe, Ile, and Thr at amino acid position 262;
any one of Ala, Ile, Met, and Thr at amino acid position 263;
any one of Asp, Glu, Phe, Gly, His, Ile, Lys, Leu, Met, Asn, Pro, Gln, Arg, Ser, Thr, Trp, and Tyr at amino acid position 264;
any one of Ala, Leu, Phe, Gly, His, Ile, Lys, Leu, Met, Asn, Pro, Gln, Arg, Ser, Thr, Val, Trp, and Tyr at amino acid position 265;
any one of Ala, Ile, Met, and Thr at amino acid position 266;
any one of Asp, Glu, Phe, His, Ile, Lys, Leu, Met, Asn, Pro, Gln, Arg, Thr, Val, Trp, and Tyr at amino acid position 267;
any one of Asp, Glu, Phe, Gly, Ile, Lys, Leu, Met, Pro, Gln, Arg, Thr, Val, and Trp at amino acid position 268;
any one of Phe, Gly, His, Ile, Lys, Leu, Met, Asn, Pro, Arg, Ser, Thr, Val, Trp, and Tyr at amino acid position 269;
any one of Glu, Phe, Gly, His, Ile, Leu, Met, Pro, Gln, Arg, Ser, Thr, Trp, and Tyr at amino acid position 270;
any one of Ala, Asp, Glu, Phe, Gly, His, Ile, Lys, Leu, Met, Asn, Gln, Arg, Ser, Thr, Val, Trp, and Tyr at amino acid position 271;
any one of Asp, Phe, Gly, His, Ile, Lys, Leu, Met, Pro, Arg, Ser, Thr, Val, Trp, and Tyr at amino acid position 272;
either Phe or Ile at amino acid position 273;
any one of Asp, Glu, Phe, Gly, His, Ile, Leu, Met, Asn, Pro, Arg, Ser, Thr, Val, Trp, and Tyr at amino acid position 274;
either Leu or Trp at amino acid position 275;
any one of Asp, Glu, Phe, Gly, His, Ile, Leu, Met, Pro, Arg, Ser, Thr, Val, Trp, and Tyr at amino acid position 276;
any one of Asp, Glu, Gly, His, Ile, Lys, Leu, Met, Asn, Pro, Gln, Arg, Ser, Thr, Val, and Trp at amino acid position 278;
Ala at amino acid position 279;
any one of Ala, Gly, His, Lys, Leu, Pro, Gln, Trp, and Tyr at amino acid position 280;
any one of Asp, Lys, Pro, and Tyr at amino acid position 281;
any one of Glu, Gly, Lys, Pro, and Tyr at amino acid position 282;
any one of Ala, Gly, His, Ile, Lys, Leu, Met, Pro, Arg, and Tyr at amino acid position 283;
any one of Asp, Glu, Leu, Asn, Thr, and Tyr at amino acid position 284;
any one of Asp, Glu, Lys, Gln, Trp, and Tyr at amino acid position 285;
any one of Glu, Gly, Pro, and Tyr at amino acid position 286;

any one of Asn, Asp, Glu, and Tyr at amino acid position 288;
any one of Asp, Gly, His, Leu, Asn, Ser, Thr, Trp, and Tyr at amino acid position 290;
any one of Asp, Glu, Gly, His, Ile, Gln, and Thr at amino acid position 291;
any one of Ala, Asp, Glu, Pro, Thr, and Tyr at amino acid position 292;
any one of Phe, Gly, His, Ile, Leu, Met, Asn, Pro, Arg, Ser, Thr, Val, Trp, and Tyr at amino acid position 293;
any one of Phe, Gly, His, Ile, Lys, Leu, Met, Asn, Pro, Arg, Ser, Thr, Val, Trp, and Tyr at amino acid position 294;
any one of Asp, Glu, Phe, Gly, His, Ile, Lys, Met, Asn, Pro, Arg, Ser, Thr, Val, Trp, and Tyr at amino acid position 295;
any one of Ala, Asp, Glu, Gly, His, Ile, Lys, Leu, Met, Asn, Gln, Arg, Ser, Thr, and Val at amino acid position 296;
any one of Asp, Glu, Phe, Gly, His, Ile, Lys, Leu, Met, Pro, Gln, Arg, Ser, Thr, Val, Trp, and Tyr at amino acid position 297;
any one of Ala, Asp, Glu, Phe, His, Ile, Lys, Met, Asn, Gln, Arg, Thr, Val, Trp, and Tyr at amino acid position 298;
any one of Ala, Asp, Glu, Phe, Gly, His, Ile, Lys, Leu, Met, Asn, Pro, Gln, Arg, Ser, Val, Trp, and Tyr at amino acid position 299;
any one of Ala, Asp, Glu, Gly, His, Ile, Lys, Leu, Met, Asn, Pro, Gln, Arg, Ser, Thr, Val, and Trp at amino acid position 300;
any one of Asp, Glu, His, and Tyr at amino acid position 301;
Ile at amino acid position 302;
any one of Asp, Gly, and Tyr at amino acid position 303;
any one of Asp, His, Leu, Asn, and Thr at amino acid position 304;
any one of Glu, Ile, Thr, and Tyr at amino acid position 305;
any one of Ala, Asp, Asn, Thr, Val, and Tyr at amino acid position 311;
Phe at amino acid position 313;
Leu at amino acid position 315;
either Glu or Gln at amino acid position 317;
any one of His, Leu, Asn, Pro, Gln, Arg, Thr, Val, and Tyr at amino acid position 318;
any one of Asp, Phe, Gly, His, Ile, Leu, Asn, Pro, Ser, Thr, Val, Trp, and Tyr at amino acid position 320;
any one of Ala, Asp, Phe, Gly, His, Ile, Pro, Ser, Thr, Val, Trp, and Tyr at amino acid position 322;
Ile at amino acid position 323;
any one of Asp, Phe, Gly, His, Ile, Leu, Met, Pro, Arg, Thr, Val, Trp, and Tyr at amino acid position 324;
any one of Ala, Asp, Glu, Phe, Gly, His, Ile, Lys, Leu, Met, Pro, Gln, Arg, Ser, Thr, Val, Trp, and Tyr at amino acid position 325;
any one of Ala, Asp, Glu, Gly, Ile, Leu, Met, Asn, Pro, Gln, Ser, Thr, Val, Trp, and Tyr at amino acid position 326;
any one of Ala, Asp, Glu, Phe, Gly, His, Ile, Lys, Leu, Met, Asn, Pro, Arg, Thr, Val, Trp, and Tyr at amino acid position 327;
any one of Ala, Asp, Glu, Phe, Gly, His, Ile, Lys, Met, Asn, Pro, Gln, Arg, Ser, Thr, Val, Trp, and Tyr at amino acid position 328;
any one of Asp, Glu, Phe, Gly, His, Ile, Lys, Leu, Met, Asn, Gln, Arg, Ser, Thr, Val, Trp, and Tyr at amino acid position 329;
any one of Cys, Glu, Phe, Gly, His, Ile, Lys, Leu, Met, Asn, Pro, Arg, Ser, Thr, Val, Trp, and Tyr at amino acid position 330;
any one of Asp, Phe, His, Ile, Leu, Met, Gln, Arg, Thr, Val, Trp, and Tyr at amino acid position 331;
any one of Ala, Asp, Glu, Phe, Gly, His, Lys, Leu, Met, Asn, Pro, Gln, Arg, Ser, Thr, Val, Trp, and Tyr at amino acid position 332;
any one of Ala, Asp, Glu, Phe, Gly, His, Ile, Leu, Met, Pro, Ser, Thr, Val, and Tyr at amino acid position 333;
any one of Ala, Glu, Phe, Ile, Leu, Pro, and Thr at amino acid position 334;
any one of Asp, Phe, Gly, His, Ile, Leu, Met, Asn, Pro, Arg, Ser, Val, Trp, and Tyr at amino acid position 335;
any one of Glu, Lys, and Tyr at amino acid position 336;
any one of Glu, His, and Asn at amino acid position 337;
any one of Asp, Phe, Gly, Ile, Lys, Met, Asn, Gln, Arg, Ser, and Thr at amino acid position 339; either Ala or Val at amino acid position 376;
either Gly or Lys at amino acid position 377;
Asp at amino acid position 378;
Asn at amino acid position 379;
any one of Ala, Asn, and Ser at amino acid position 380;
either Ala or Ile at amino acid position 382;
Glu at amino acid position 385;
Thr at amino acid position 392;

Leu at amino acid position 396;
Lys at amino acid position 421;
Asn at amino acid position 427;
either Phe or Leu at amino acid position 428;
Met at amino acid position 429;
Trp at amino acid position 434;
Ile at amino acid position 436; and
any one of Gly, His, Ile, Leu, and Tyr at amino acid position 440,
in the Fc region site according to EU numbering.

[39] The method of any one of [29] to [38], wherein the Fc region of a native human IgG in which the sugar chain bound at position 297 according to EU numbering is a fucose-containing sugar chain, is an Fc region of any one of native human IgG1, native human IgG2, native human IgG3, and native human IgG4 in which the sugar chain bound at position 297 according to EU numbering is a fucose-containing sugar chain.
[40] The method of any one of [29] to [39], wherein the human Fcγ receptor is FcyRIa, FcγRIIa(R), FcγRIIa(H), FcγRIIb, FcγRIIIa(V), or FcγRIIIa(F).
[41] The method of any one of [29] to [39], wherein the human Fcγ receptor is FcγRIIb.
[42] The method of any one of [36] to [41], wherein the Fc region is an Fc region which comprises at least one or more of:

Asp at amino acid position 238, and
Glu at amino acid position 328
in the Fc region site according to EU numbering.

[43] A method for producing an antigen-binding molecule, which comprises the steps of:

(a) determining the antigen-binding activity of an antigen-binding domain under a high-calcium-ion concentration condition;
(b) determining the antigen-binding activity of an antigen-binding domain under a low-calcium-ion concentration condition;
(c) selecting the antigen-binding domain for which the antigen-binding activity determined in (a) is higher than the antigen-binding activity determined in (b);
(d) linking a polynucleotide encoding the antigen-binding domain selected in (c) to a polynucleotide encoding an Fcγ receptor-binding domain having human-FcRn-binding activity in an acidic pH range condition and in which binding activity to the Fcγ receptor in a neutral pH range condition is higher than that of an Fc region of a native human IgG in which the sugar chain bound at position 297 according to EU numbering is a fucose-containing sugar chain;
(e) culturing cells introduced with a vector in which the polynucleotide obtained in (d) is operably linked; and
(f) collecting antigen-binding molecules from the cell culture of (e).

[44] A method for producing an antigen-binding molecule, which comprises the steps of:

(a) determining the antigen-binding activity of an antibody under a high-calcium-ion concentration condition;
(b) determining the antigen-binding activity of an antibody under a low-calcium-ion concentration condition;
(c) selecting the antibody for which the antigen-binding activity determined in (a) is higher than the antigen-binding activity determined in (b);
(d) linking a polynucleotide encoding the antigen-binding domain of the antibody selected in (c) to a polynucleotide encoding an Fcγ receptor-binding domain having human-FcRn-binding activity in an acidic pH range, and in which binding activity to the Fcγ receptor in a neutral pH range condition is higher than that of an Fc region of a native human IgG in which the sugar chain bound at position 297 according to EU numbering is a fucose-containing sugar chain;
(e) culturing cells introduced with a vector in which the polynucleotide obtained in (d) is operably linked; and
(f) collecting antigen-binding molecules from the cell culture of (e).

[45] A method for producing an antigen-binding molecule, which comprises the steps of:

(a) determining the antigen-binding activity of an antigen-binding domain in a neutral pH range condition;
(b) determining the antigen-binding activity of an antigen-binding domain in an acidic pH range condition;
(c) selecting the antigen-binding domain for which the antigen-binding activity determined in (a) is higher than the antigen-binding activity determined in (b);
(d) linking a polynucleotide encoding the antigen-binding domain selected in (c) to a polynucleotide encoding an Fcγ receptor-binding domain having human-FcRn-binding activity in an acidic pH range condition, and in which binding activity to the Fcγ receptor in a neutral pH range condition is higher than that of an Fc region of a native human IgG in which the sugar chain bound at position 297 according to EU numbering is a fucose-containing sugar chain;
(e) culturing cells introduced with a vector in which the polynucleotide obtained in (d) is operably linked; and
(f) collecting antigen-binding molecules from the cell culture of (e).

[46] A method for producing an antigen-binding molecule, which comprises the steps of:

(a) determining the antigen-binding activity of an antibody in a neutral pH range condition;
(b) determining the antigen-binding activity of an antibody in an acidic pH range condition;
(c) selecting the antibody for which the antigen-binding activity determined in (a) is higher than the antigen-binding activity determined in (b);
(d) linking a polynucleotide encoding the antigen-binding domain of the antibody selected in (c) to a polynucleotide encoding an Fcγ receptor-binding domain having human-FcRn-binding activity in an acidic pH range condition, in which binding activity to the Fcγ receptor in a neutral pH range condition is higher than that of an Fc region of a native human IgG in which the sugar chain bound at position 297 according to EU numbering is a fucose-containing sugar chain;
(e) culturing cells introduced with a vector in which the polynucleotide obtained in (d) is operably linked; and
(f) collecting antigen-binding molecules from the cell culture of (e).

[47] The production method of any one of [43] to [46], wherein the antigen is a soluble antigen.
[48] The production method of any one of [43] to [47], wherein the Fcγ receptor-binding domain comprises an antibody Fc region.
[49] The production method of [48], wherein the Fc region is an Fc region in which at least one or more amino acids selected from the group consisting of amino acids at positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, and 440 in the Fc region site according to EU numbering are different from the amino acids at corresponding sites in the native Fc region.
[50] The production method of [49], wherein the Fc region comprises at least one or more amino acids selected from the group consisting of:

either Lys or Tyr at amino acid position 221;
any one of Phe, Trp, Glu, and Tyr at amino acid position 222;
any one of Phe, Trp, Glu, and Lys at amino acid position 223;
any one of Phe, Trp, Glu, and Tyr at amino acid position 224;
any one of Glu, Lys, and Trp at amino acid position 225;
any one of Glu, Gly, Lys, and Tyr at amino acid position 227;
any one of Glu, Gly, Lys, and Tyr at amino acid position 228;
any one of Ala, Glu, Gly, and Tyr at amino acid position 230;
any one of Glu, Gly, Lys, Pro, and Tyr at amino acid position 231;
any one of Glu, Gly, Lys, and Tyr at amino acid position 232;
any one of Ala, Asp, Phe, Gly, His, Ile, Lys, Leu, Met, Asn, Gln, Arg, Ser, Thr, Val, Trp, and Tyr at amino acid position 233;
any one of Ala, Asp, Glu, Phe, Gly, His, Ile, Lys, Met, Asn, Pro, Gln, Arg, Ser, Thr, Val, Trp, and Tyr at amino acid position 234;
any one of Ala, Asp, Glu, Phe, Gly, His, Ile, Lys, Met, Asn, Pro, Gln, Arg, Ser, Thr, Val, Trp, and Tyr at amino acid position 235;
any one of Ala, Asp, Glu, Phe, His, Ile, Lys, Leu, Met, Asn, Pro, Gln, Arg, Ser, Thr, Val, Trp, and Tyr at amino acid position 236;
any one of Asp, Glu, Phe, His, Ile, Lys, Leu, Met, Asn, Pro, Gln, Arg, Ser, Thr, Val, Trp, and Tyr at amino acid position 237;
any one of Asp, Glu, Phe, Gly, His, Ile, Lys, Leu, Met, Asn, Gln, Arg, Ser, Thr, Val, Trp, and Tyr at amino acid position 238;
any one of Asp, Glu, Phe, Gly, His, Ile, Lys, Leu, Met, Asn, Pro, Gln, Arg, Thr, Val, Trp, and Tyr at amino acid position 239;
any one of Ala, Ile, Met, and Thr at amino acid position 240;
any one of Asp, Glu, Leu, Arg, Trp, and Tyr at amino acid position 241;
any one of Leu, Glu, Leu, Gln, Arg, Trp, and Tyr at amino acid position 243;
His at amino acid position 244;
Ala at amino acid position 245;
any one of Asp, Glu, His, and Tyr at amino acid position 246;
any one of Ala, Phe, Gly, His, Ile, Leu, Met, Thr, Val, and Tyr at amino acid position 247;
any one of Glu, His, Gln, and Tyr at amino acid position 249;
either Glu or Gln at amino acid position 250;
Phe at amino acid position 251;
any one of Phe, Met, and Tyr at amino acid position 254;
any one of Glu, Leu, and Tyr at amino acid position 255;
any one of Ala, Met, and Pro at amino acid position 256;
any one of Asp, Glu, His, Ser, and Tyr at amino acid position 258;
any one of Asp, Glu, His, and Tyr at amino acid position 260;
any one of Ala, Glu, Phe, Ile, and Thr at amino acid position 262;
any one of Ala, Ile, Met, and Thr at amino acid position 263;
any one of Asp, Glu, Phe, Gly, His, Ile, Lys, Leu, Met, Asn, Pro, Gln, Arg, Ser, Thr, Trp, and Tyr at amino acid position 264;
any one of Ala, Leu, Phe, Gly, His, Ile, Lys, Leu, Met, Asn, Pro, Gln, Arg, Ser, Thr, Val, Trp, and Tyr at amino acid position 265;
any one of Ala, Ile, Met, and Thr at amino acid position 266;
any one of Asp, Glu, Phe, His, Ile, Lys, Leu, Met, Asn, Pro, Gln, Arg, Thr, Val, Trp, and Tyr at amino acid position 267;
any one of Asp, Glu, Phe, Gly, Ile, Lys, Leu, Met, Pro, Gln, Arg, Thr, Val, and Trp at amino acid position 268;
any one of Phe, Gly, His, Ile, Lys, Leu, Met, Asn, Pro, Arg, Ser, Thr, Val, Trp, and Tyr at amino acid position 269;
any one of Glu, Phe, Gly, His, Ile, Leu, Met, Pro, Gln, Arg, Ser, Thr, Trp, and Tyr at amino acid position 270;
any one of Ala, Asp, Glu, Phe, Gly, His, Ile, Lys, Leu, Met, Asn, Gln, Arg, Ser, Thr, Val, Trp, and Tyr at amino acid position 271;
any one of Asp, Phe, Gly, His, Ile, Lys, Leu, Met, Pro, Arg, Ser, Thr, Val, Trp, and Tyr at amino acid position 272;
either Phe or Ile at amino acid position 273;
any one of Asp, Glu, Phe, Gly, His, Ile, Leu, Met, Asn, Pro, Arg, Ser, Thr, Val, Trp, and Tyr at amino acid position 274;
either Leu or Trp at amino acid position 275;
any one of Asp, Glu, Phe, Gly, His, Ile, Leu, Met, Pro, Arg, Ser, Thr, Val, Trp, and Tyr at amino acid position 276;
any one of Asp, Glu, Gly, His, Ile, Lys, Leu, Met, Asn, Pro, Gln, Arg, Ser, Thr, Val, and Trp at amino acid position 278;
Ala at amino acid position 279;
any one of Ala, Gly, His, Lys, Leu, Pro, Gln, Trp, and Tyr at amino acid position 280;
any one of Asp, Lys, Pro, and Tyr at amino acid position 281;
any one of Glu, Gly, Lys, Pro, and Tyr at amino acid position 282;
any one of Ala, Gly, His, Ile, Lys, Leu, Met, Pro, Arg, and Tyr at amino acid position 283;
any one of Asp, Glu, Leu, Asn, Thr, and Tyr at amino acid position 284;
any one of Asp, Glu, Lys, Gln, Trp, and Tyr at amino acid position 285;
any one of Glu, Gly, Pro, and Tyr at amino acid position 286;
any one of Asn, Asp, Glu, and Tyr at amino acid position 288;
any one of Asp, Gly, His, Leu, Asn, Ser, Thr, Trp, and Tyr at amino acid position 290;
any one of Asp, Glu, Gly, His, Ile, Gln, and Thr at amino acid position 291;
any one of Ala, Asp, Glu, Pro, Thr, and Tyr at amino acid position 292;
any one of Phe, Gly, His, Ile, Leu, Met, Asn, Pro, Arg, Ser, Thr, Val, Trp, and Tyr at amino acid position 293;
any one of Phe, Gly, His, Ile, Lys, Leu, Met, Asn, Pro, Arg, Ser, Thr, Val, Trp, and Tyr at amino acid position 294;
any one of Asp, Glu, Phe, Gly, His, Ile, Lys, Met, Asn, Pro, Arg, Ser, Thr, Val, Trp, and Tyr at amino acid position 295;
any one of Ala, Asp, Glu, Gly, His, Ile, Lys, Leu, Met, Asn, Gln, Arg, Ser, Thr, and Val at amino acid position 296;
any one of Asp, Glu, Phe, Gly, His, Ile, Lys, Leu, Met, Pro, Gln, Arg, Ser, Thr, Val, Trp, and Tyr at amino acid position 297;
any one of Ala, Asp, Glu, Phe, His, Ile, Lys, Met, Asn, Gln, Arg, Thr, Val, Trp, and Tyr at amino acid position 298;
any one of Ala, Asp, Glu, Phe, Gly, His, Ile, Lys, Leu, Met, Asn, Pro, Gln, Arg, Ser, Val, Trp, and Tyr at amino acid position 299;
any one of Ala, Asp, Glu, Gly, His, Ile, Lys, Leu, Met, Asn, Pro, Gln, Arg, Ser, Thr, Val, and Trp at amino acid position 300;
any one of Asp, Glu, His, and Tyr at amino acid position 301;
Ile at amino acid position 302;
any one of Asp, Gly, and Tyr at amino acid position 303;
any one of Asp, His, Leu, Asn, and Thr at amino acid position 304;
any one of Glu, Ile, Thr, and Tyr at amino acid position 305;
any one of Ala, Asp, Asn, Thr, Val, and Tyr at amino acid position 311;
Phe at amino acid position 313;
Leu at amino acid position 315;
either Glu or Gln at amino acid position 317;
any one of His, Leu, Asn, Pro, Gln, Arg, Thr, Val, and Tyr at amino acid position 318;
any one of Asp, Phe, Gly, His, Ile, Leu, Asn, Pro, Ser, Thr, Val, Trp, and Tyr at amino acid position 320;
any one of Ala, Asp, Phe, Gly, His, Ile, Pro, Ser, Thr, Val, Trp, and Tyr at amino acid position 322;
Ile at amino acid position 323;
any one of Asp, Phe, Gly, His, Ile, Leu, Met, Pro, Arg, Thr, Val, Trp, and Tyr at amino acid position 324;
any one of Ala, Asp, Glu, Phe, Gly, His, Ile, Lys, Leu, Met, Pro, Gln, Arg, Ser, Thr, Val, Trp, and Tyr at amino acid position 325;
any one of Ala, Asp, Glu, Gly, Ile, Leu, Met, Asn, Pro, Gln, Ser, Thr, Val, Trp, and Tyr at amino acid position 326;
any one of Ala, Asp, Glu, Phe, Gly, His, Ile, Lys, Leu, Met, Asn, Pro, Arg, Thr, Val, Trp, and Tyr at amino acid position 327;
any one of Ala, Asp, Glu, Phe, Gly, His, Ile, Lys, Met, Asn, Pro, Gln, Arg, Ser, Thr, Val, Trp, and Tyr at amino acid position 328;
any one of Asp, Glu, Phe, Gly, His, Ile, Lys, Leu, Met, Asn, Gln, Arg, Ser, Thr, Val, Trp, and Tyr at amino acid position 329;
any one of Cys, Glu, Phe, Gly, His, Ile, Lys, Leu, Met, Asn, Pro, Arg, Ser, Thr, Val, Trp, and Tyr at amino acid position 330;
any one of Asp, Phe, His, Ile, Leu, Met, Gln, Arg, Thr, Val, Trp, and Tyr at amino acid position 331;
any one of Ala, Asp, Glu, Phe, Gly, His, Lys, Leu, Met, Asn, Pro, Gln, Arg, Ser, Thr, Val, Trp, and Tyr at amino acid position 332;
any one of Ala, Asp, Glu, Phe, Gly, His, Ile, Leu, Met, Pro, Ser, Thr, Val, and Tyr at amino acid position 333;
any one of Ala, Glu, Phe, Ile, Leu, Pro, and Thr at amino acid position 334;
any one of Asp, Phe, Gly, His, Ile, Leu, Met, Asn, Pro, Arg, Ser, Val, Trp, and Tyr at amino acid position 335;
any one of Glu, Lys, and Tyr at amino acid position 336;
any one of Glu, His, and Asn at amino acid position 337;
any one of Asp, Phe, Gly, Ile, Lys, Met, Asn, Gln, Arg, Ser, and Thr at amino acid position 339; either Ala or Val at amino acid position 376;
either Gly or Lys at amino acid position 377;
Asp at amino acid position 378;
Asn at amino acid position 379;
any one of Ala, Asn, and Ser at amino acid position 380;
either Ala or Ile at amino acid position 382;
Glu at amino acid position 385;
Thr at amino acid position 392;
Leu at amino acid position 396;
Lys at amino acid position 421;
Asn at amino acid position 427;
either Phe or Leu at amino acid position 428;
Met at amino acid position 429;
Trp at amino acid position 434;
Ile at amino acid position 436; and
any one of Gly, His, Ile, Leu, and Tyr at amino acid position 440,
in the Fc region site according to EU numbering.

[51] The production method of any one of [43] to [50], wherein the Fcγ receptor binding domain is an Fc region of any one of native human IgG1, native human IgG2, native human IgG3, and native human IgG4 in which the sugar chain bound at position 297 according to EU numbering is a fucose-containing sugar chain.
[52] The production method of any one of [43] to [51], wherein the human Fcγ receptor is FcyRIa, FcγRIIa(R), FcγRIIa(H), FcγRIIb, FcγRIIIa(V), or FcγRIIIa(F).
[53] The production method of any one of [43] to [51], wherein the human Fcγ receptor is FcγRIIb.
[54] The production method of any one of [48] to [53], wherein the Fc region comprises at least one or more amino acids of:

Asp at amino acid position 238, and
Glu at amino acid position 328
in the Fc region site according to EU numbering.

Brief Description of the Drawings

Fig. 1 shows a non-limiting action mechanism for the elimination of soluble antigen from plasma by administering an antibody that binds to an antigen in an ion concentration-dependent manner and whose Fcγ receptor binding is enhanced at a neutral pH as compared to existing neutralizing antibodies.
Fig. 2 shows a time course of human IL-6 receptor concentration in the plasma of human FcRn transgenic mice administered with Fv4-IgG1 which binds to human IL-6 receptor in a pH-dependent manner or H54/L28-IgG1.
Fig. 3 shows a time course of human IL-6 receptor concentration in the plasma of human FcRn transgenic mice administered with Fv4-IgG1 which binds to human IL-6 receptor in a pH-dependent manner, Fv4-IgG1-F760 which is an Fv4-IgG1 variant that lacks mouse FcyR binding, Fv4-IgG1-F1022 which is an Fv4-IgG1 variant with enhanced mouse FcyR binding, or Fv4-IgG1-Fuc which is an Fv4-IgG1 antibody with low fucose content.
Fig. 4 shows a time course of human IL-6 receptor concentration in the plasma of human FcRn transgenic mice administered with Fv4-IgG1 or antigen-binding molecules comprising as the heavy chain, Fv4-IgG1-F1022 or Fv4-IgG1-F1093 which is a Fv4-IgG1-F1022 variant with improved FcRn binding in an acidic pH range.
Fig. 5 shows a concentration time course of the administered antigen-binding molecules in the plasma of human FcRn transgenic mice administered with Fv4-IgG1 or antigen-binding molecules comprising as the heavy chain, Fv4-IgG1-F1022 or Fv4-IgG1-F1093 which is a Fv4-IgG1-F1022 variant with improved FcRn binding in an acidic pH range.
Fig. 6 shows a time course of human IL-6 receptor concentration in the plasma of human FcRn transgenic mice administered with Fv4-IgG1, Fv4-IgG1-F1087 which is an Fv4-IgG1 variant with enhanced mouse FcyR binding (in particular, enhanced mouse FcγRIIb binding and mouse FcγRIII binding), and Fv4-IgG1-F1182 which is an Fv4-IgG1 variant with enhanced mouse FcyR binding (in particular, enhanced mouse FcyRI binding and mouse FcyRIV binding).
Fig. 7 shows a concentration time course of the administered antigen-binding molecules in the plasma of human FcRn transgenic mice administered with Fv4-IgG1, Fv4-IgG1-F1087, and Fv4-IgG1-F1180 and Fv4-IgG1-F1412 which are Fv4-IgG1-F1087 variants with improved FcRn binding in an acidic pH range.
Fig. 8 shows a concentration time course of the administered antigen-binding molecules in the plasma of human FcRn transgenic mice administered with Fv4-IgG1, Fv4-IgG1-F1182, and Fv4-IgG1-F1181 which is an Fv4-IgG1-F1182 variant with improved FcRn binding in an acidic pH range.
Fig. 9 shows a time course of human IL-6 receptor concentration in the plasma of human FcRn transgenic mice administered with Fv4-IgG1, Fv4-IgG1-F1087, and Fv4-IgG1-F1180 and Fv4-IgG1-F1412 which are Fv4-IgG1-F1087 variants with improved FcRn binding in an acidic pH range.
Fig. 10 shows a time course of human IL-6 receptor concentration in the plasma of human FcRn transgenic mice administered with Fv4-IgG1, Fv4-IgG1-F1182, and Fv4-IgG1-F1181 which is an Fv4-IgG1-F1182 variant with improved FcRn binding in an acidic pH range.
Fig. 11 shows the results of change in plasma concentration of Fv4-IgG1, Fv4-IgG1-F1782, or Fv4-IgG1-F1087 in a human FcRn transgenic mouse when Fv4-IgG1, Fv4-IgG1-F1782, or Fv4-IgG1-F1087 is administered to the mouse.
Fig. 12 shows the results of change in plasma concentration of a soluble human IL-6 receptor in a human FcRn transgenic mouse when Fv4-IgG1, Fv4-IgG1-F1782, or Fv4-IgG1-F1087 is administered to the mouse.
Fig. 13 shows a time course of human IL-6 receptor concentration in the plasma of normal mice administered with Fv4-mIgG1, Fv4-mIgG1-mF44 which is an Fv4-mIgG1 variant with enhanced mouse FcγRIIb binding and mouse FcγRIII binding, and Fv4-mIgG1-mF46 which is an Fv4-mIgG1 variant with further enhanced mouse FcγRIIb binding and mouse FcγRIII binding.

Fig. 14 shows a time course of human IL-6 receptor concentration in the plasma of FcyRIII-deficient mice administered with Fv4-mIgG1, Fv4-mIgG1-mF44 which is an Fv4-mIgG1 variant with enhanced mouse FcγRIIb binding and mouse FcγRIII binding, and Fv4-mIgG1-mF46 which is an Fv4-mIgG1 variant with further enhanced mouse FcγRIIb binding and mouse FcγRIII binding.

Fig. 15 shows a time course of human IL-6 receptor concentration in the plasma of Fc receptor γ chain-deficient mice administered with Fv4-mIgG1, Fv4-mIgG1-mF44 which is an Fv4-mIgG1 variant with enhanced mouse FcγRIIb binding and mouse FcγRIII binding, and Fv4-mIgG1-mF46 which is an Fv4-mIgG1 variant with further enhanced mouse FcγRIIb binding and mouse FcγRIII binding.

Fig. 16 shows a time course of human IL-6 receptor concentration in the plasma of FcyRIIb-deficient mice administered with Fv4-mIgG1, Fv4-mIgG1-mF44 which is an Fv4-mIgG1 variant with enhanced mouse FcγRIIb binding and mouse FcγRIII binding, and Fv4-mIgG1-mF46 which is an Fv4-mIgG1 variant with further enhanced mouse FcγRIIb binding and mouse FcγRIII binding.

Fig. 17 shows a result of evaluating the platelet aggregation ability of the omalizumab-G1d-v3/IgE immunocomplex by platelet aggregation assay using platelets derived from donors with FcγRIIa allotype (R/H).

Fig. 18 shows a result of evaluating the platelet aggregation ability of the omalizumab-G1d-v3/IgE immunocomplex by platelet aggregation assay using platelets derived from donors with FcγRIIa allotype (H/H).

Fig. 19 shows a result of assessing CD62p expression on the membrane surface of washed platelets. The black-filled area in the graph indicates a result of ADP stimulation after reaction with PBS. The area that is not filled in the graph indicates a result of ADP stimulation after reaction with the immunocomplex.

Fig. 20 shows a result of assessing the expression of active integrin on the membrane surface of washed platelets. The black-filled area in the graph indicates a result of ADP stimulation after reaction with PBS. The area that is not filled in the graph indicates a result of ADP stimulation after reaction with the immunocomplex.

Fig. 21 shows the results of evaluating platelet aggregation activity induced by the omalizumab-BP230/IgE immunocomplex and the omalizumab-G1d-v3/IgE immunocomplex in a platelet aggregation assay using platelets derived from a donor with an FcγRIIa polymorphism (R/H).

Fig. 22 shows the results of evaluating CD62p expression on the surface of the membrane of washed platelets. The graph shaded with grey indicates the result when stimulation by adding ADP was performed after reaction with PBS, the solid line and the dotted line indicate the results when stimulation by ADP was performed after reaction with the omalizumab-G1d-v3/IgE immunocomplex and the omalizumab-BP230/IgE immunocomplex, respectively.

Fig. 23 shows the results of evaluating activating integrin expression on the surface of the membrane of washed platelets. The graph shaded with grey indicates the result when stimulation by adding ADP was performed after reaction with PBS, the solid line and the dotted line indicate the results when stimulation by ADP was performed after reaction with the omalizumab-G1d-v3/IgE immunocomplex and the omalizumab-BP230/IgE immunocomplex, respectively.

Fig. 24 shows a graph in which the horizontal axis shows the relative value of FcyRIIb-binding activity of each PD variant, and the vertical axis shows the relative value of FcγRIIa type R-binding activity of each PD variant. The value for the amount of binding of each PD variant to each FcyR was divided by the value for the amount of binding of IL6R-F652 / IL6R-L, which is a control antibody prior to introduction of the alteration (IL6R-F652, defined by SEQ ID NO: 142, is an antibody heavy chain comprising an altered Fc with substitution of Pro at position 238 (EU numbering) with Asp), to each FcyR; and then the obtained value was multiplied by 100, and used as the relative binding activity value for each PD variant to each FcyR. The F652 plot in the figure shows the value for IL6R-F652/IL6R-L.

Fig. 25 shows a graph in which the vertical axis shows the relative value of FcyRIIb-binding activity of variants produced by introducing each alteration into GpH7-B3 (SEQ ID NO: 159) / GpL16-k0 (SEQ ID NO: 160) which does not have the P238D alteration, and the horizontal axis shows the relative value of FcγRIIb-binding activity of variants produced by introducing each alteration into IL6R-F652 (SEQ ID NO: 142) / IL6R-L which has the P238D alteration. The value for the amount of FcγRIIb binding of each variant was divided by the value for the amount of FcγRIIb binding of the pre-altered antibody; and then the obtained value was multiplied by 100, and used as the value of relative binding activity. Here, region A contains alterations that exhibit the effect of enhancing FcγRIIb binding in both cases where an alteration is introduced into GpH7-B3 / GpL16-k0 which does not have P238D and where an alteration is introduced into IL6R-F652 / IL6R-L which has P238D. Region B contains alterations that exhibit the effect of enhancing FcγRIIb binding when introduced into GpH7-B3 / GpL16-k0 which does not have P238D, but do not exhibit the effect of enhancing FcγRIIb binding when introduced into IL6R-F652 / IL6R-L which has P238D.

Fig. 26 shows a crystal structure of the Fc(P238D) / FcγRIIb extracellular region complex.

Fig. 27 shows an image of superimposing the crystal structure of the Fc(P238D) / FcγRIIb extracellular region complex and the model structure of the Fc(WT) / FcγRIIb extracellular region complex, with respect to the FcγRIIb extracellular region and the Fc CH2 domain A by the least squares fitting based on the Cα atom pair distances.

Fig. 28 shows comparison of the detailed structure around P238D after superimposing the crystal structure of the Fc(P238D) / FcγRIIb extracellular region complex and the model structure of the Fc(WT) / FcγRIIb extracellular region complex with respect to the only Fc CH2 domain A or the only Fc CH2 domain B by the least squares fitting based on the Cα atom pair distances.

Fig. 29 shows that a hydrogen bond can be found between the main chain of Gly at position 237 (indicated by EU numbering) in Fc CH2 domain A, and Tyr at position 160 in FcγRIIb in the crystal structure of the Fc(P238D) / FcγRIIb extracellular region complex.

Fig. 30 shows that an electrostatic interaction can be found between Asp at position 270 (indicated by EU numbering) in Fc CH2 domain B, and Arg at position 131 in FcγRIIb in the crystal structure of the Fc(P238D) / FcγRIIb extracellular region complex.

Fig. 31 shows a graph in which the horizontal axis shows the relative value of FcγRIIb-binding activity of each 2B variant, and the vertical axis shows the relative value of FcγRIIa type R-binding activity of each 2B variant. The value for the amount of binding of each 2B variant to each FcγR was divided by the value for the amount of binding of a control antibody prior to alteration (altered Fc with substitution of Pro at position 238 (indicated by EU numbering) with Asp) to each FcγR; and then the obtained value was multiplied by 100, and used as the value of relative binding activity of each 2B variant towards each FcγR.

Fig. 32 shows Glu at position 233 (indicated by EU numbering) in Fc Chain A and the surrounding residues in the extracellular region of FcγRIIb in the crystal structure of the Fc(P238D) / FcγRIIb extracellular region complex.

Fig. 33 shows Ala at position 330 (indicated by EU numbering) in Fc Chain A and the surrounding residues in the extracellular region of FcγRIIb in the crystal structure of the Fc(P238D) / FcγRIIb extracellular region complex.

Fig. 34 shows the structures of Pro at position 271 (EU numbering) of Fc Chain B after superimposing the crystal structures of the Fc(P238D) / FcγRIIb extracellular region complex and the Fc(WT) / FcγRIIIa extracellular region complex by the least squares fitting based on the Cα atom pair distances with respect to Fc Chain B.

Fig. 35 shows an image of the Fc (P208)/FcγRIIb extracellular region complex determined by X-ray crystal structure analysis. For each of the CH2 and CH3 domains in the Fc portion, those on the left side are referred to as domain A and those on the right side are referred to as domain B.

Fig. 36 shows comparison after superimposing the structures of Fc (P208)/FcγRIIb extracellular region complex and Fc (WT)/FcγRIIa extracellular region complex (PDB code: 3RY6) determined by X-ray crystal structure analysis with respect to the CH2 domain A of the Fc portion by the least squares fitting based on the Cα atom pair distances. In the diagram, the structure drawn with heavy line shows the Fc (P208)/FcγRIIb extracellular region complex, while the structure drawn with thin line indicates the structure of Fc (WT)/FcγRIIa extracellular region complex. Only the CH2 domain A of the Fc portion is drawn for the Fc (WT)/FcyRIIa extracellular region complex.

Fig. 37 shows in the X-ray crystal structure of the Fc (P208)/FcγRIIb extracellular region complex, a detailed structure around Asp at position 237 (EU numbering) in the CH2 domain A of the Fc portion, which forms a hydrogen bond with Tyr at position 160 in FcγRIIb at the main chain moiety.

Fig. 38 shows in the X-ray crystal structure of the Fc (P208)/FcγRIIb extracellular region complex, the structure of amino acid residues around Asp at position 237 (EU numbering) in the CH2 domain A of the Fc portion, which forms a hydrogen bond with Tyr at position 160 in FcγRIIb at the main chain moiety.

Fig. 39 shows comparison around the loop at positions 266 to 271 (EU numbering) after superimposing the X-ray crystal structures of the Fc (P238D)/FcγRIIb extracellular region complex shown in Example 10 and the Fc (P208)/FcγRIIb extracellular region complex with respect to the CH2 domain B of the Fc portion by the least squares fitting based on the Cα atom pair distances. When compared to Fc (P238D), Fc (P208) has the H268D alteration at position 268 (EU numbering) and the P271G alteration at position 271 (EU numbering) in the loop.

Fig. 40 is a diagram showing the structure around Ser239 in the CH2 domain B of the Fc portion in the X-ray crystal structure of the Fc (P208)/FcγRIIb extracellular region complex, along with the electron density determined by X-ray crystal structure analysis with 2Fo-Fc coefficient.

Fig. 41 shows comparison after superimposing the three-dimensional structures of the Fc (P208)/FcγRIIaR extracellular region complex and Fc (P208)/FcγRIIb extracellular region complex determined by X-ray crystal structure analysis by the least squares fitting based on the Cα atom pair distances.

Fig. 42 shows comparison around Asp at position 237 (EU numbering) in the CH2 domain A of the Fc portion between the X-ray crystal structures of the Fc (P208)/FcγRIIaR extracellular region complex and the Fc (P208)/FcγRIIb extracellular region complex, along with the electron density determined by X-ray crystal structure analysis with 2Fo-Fc coefficient.

Fig. 43 shows comparison around Asp at position 237 (EU numbering) in the CH2 domain B of the Fc portion between the X-ray crystal structures of the Fc (P208)/FcγRIIaR extracellular region complex and the Fc (P208)/FcγRIIb extracellular region complex, along with the electron density determined by X-ray crystal structure analysis with 2Fo-Fc coefficient.

Fig. 44 shows comparison between the constant-region sequences of G1d and G4d. In the diagram, the amino acids boxed with thick-frame indicate positions with different amino acid residues between G1d and G4d.

Fig. 45 shows the change in plasma antibody concentration of GA2-IgG1 and GA2-F1087 in normal mice.

Fig. 46 shows the change in plasma hIgA concentration in normal mice administered with GA2-IgG1 and GA2-F1087.

Fig. 47 shows the change in plasma antibody concentration of 278-IgG1 and 278-F1087 in C57BL/6J mice.

Fig. 48 shows the change in plasma hIgE (Asp6) concentration in C57BL/6J mice administerd with 278-IgG1 and 278-F1087.

Fig. 49 shows the structure of the heavy chain CDR3 of the 6RL#9 antibody Fab fragment determined by X-ray crystal structure analysis. (i) shows the crystal structure of the heavy chain CDR3 obtained under a crystallization condition in the presence of calcium ion. (ii) shows the crystal structure of the heavy chain CDR3 obtained under a crystallization condition in the absence of calcium ion.

Fig. 50 shows a time course of the plasma concentration of each antibody in normal mice administered with antibody H54/L28-IgG1, FH4-IgG1, or 6RL#9-IgG1.

Fig. 51 shows a time course of the plasma concentration of soluble human IL-6 receptor (hsIL-6R) in normal mice administered with antibody H54/L28-IgG1, FH4-IgG1, or 6RL#9-IgG1.

Fig. 52 shows ion-exchange chromatograms for an antibody having human Vk5-2 sequence and an antibody having h Vk5-2_L65 sequence which has an altered glycosylation sequence in the human Vk5-2 sequence. Solid line indicates a chromatogram for an antibody having human Vk5-2 sequence (heavy chain: CIM_H (SEQ ID NO: 67); light chain: hVk5-2 (SEQ ID NO: 4)); broken line indicates a chromatogram for an antibody having hVk5-2_L65 sequence (heavy chain: CIM_H (SEQ ID NO: 67); light chain: hVk5-2_L65 (SEQ ID NO: 70)).

Fig. 53A shows ion-exchange chromatograms for an antibody having LfVk1_Ca sequence (heavy chain: GC_H (SEQ ID NO: 51); light chain: LfYk1_Ca (SEQ ID NO: 83)) and an antibody having a sequence in which Asp (D) in the LfVk1_Ca sequence is substituted with Ala (A) after storage at 5°C (solid line) or 50°C (dotted line). After storage at 5°C, the highest peak in the chromatogram for each antibody is defined as a main peak, and the y axis of each ion-exchange chromatogram was normalized to the main peak. The graph shows a chromatogram for an antibody having LfVk1_Ca (SEQ ID NO: 83) as the light chain.

Fig. 53B shows a chromatogram for an antibody having LfVk1_Ca1 (SEQ ID NO: 85) as the light chain.

Fig. 53C shows a chromatogram for an antibody having LfVk1_Ca2 (SEQ ID NO: 86) as the light chain.

Fig. 53D shows a chromatogram for an antibody having LfVk1_Ca3 (SEQ ID NO: 87) as the light chain.

Fig. 54A shows ion-exchange chromatograms for an antibody having LfVk1_Ca sequence (heavy chain: GC_H (SEQ ID NO: 51); light chain: LfVk1_Ca (SEQ ID NO: 83)) and an antibody having LfVk1_Ca6 sequence (heavy chain: GC_H (SEQ ID NO: 51); light chain: LfVk1_Ca6 (SEQ ID NO: 88)) in which Asp (D) at position 30 (Kabat numbering) in the LfVk1_Ca sequence is substituted with Ser (S) after storage at 5°C (solid line) or 50°C (dotted line). After storage at 5°C, the highest peak in the chromatogram for each antibody is defined as a main peak, and the y axis of each ion-exchange chromatogram was normalized to the main peak. The graph shows a chromatogram for an antibody having LfVk1_Ca (SEQ ID NO: 83) as the light chain.

Fig. 54B shows a chromatogram for an antibody having LfVk1_Ca6 (SEQ ID NO: 88) as the light chain.

Fig. 55 shows the relationship between designed amino acid distribution (indicated with "Design") and amino acid distribution for sequence information on 290 clones isolated from E. *coli* introduced with a gene library of antibodies that bind to antigens in a Ca-dependent manner (indicated with "Library"). The horizontal axis indicates amino acid position (Kabat numbering). The vertical axis indicates percentage in amino acid distribution.

Fig. 56 shows sensorgrams for anti-IL-6R antibody (tocilizumab), antibody 6RC1IgG_010, antibody 6RC1IgG_012, and antibody 6RC1IgG_019 under a high calcium ion concentration (1.2 mM) condition. The horizontal axis shows time, and the vertical axis shows RU value.

Fig. 57 shows sensorgrams for anti-IL-6R antibody (tocilizumab), antibody 6RC1IgG_010, antibody 6RC1IgG_012, and antibody 6RC1IgG_019 under a low calcium ion concentration (3 μM) condition. The horizontal axis shows time, and the vertical axis shows RU value.

Fig. 58 shows the relationship between designed amino acid distribution (indicated with "Design") and amino acid distribution for sequence information on 132 clones isolated from *E. coli* introduced with a gene library of antibodies that bind to antigens in a pH-dependent manner (indicated with "Library"). The horizontal axis shows amino acid position (Kabat numbering). The vertical axis indicates percentage in amino acid distribution.

Fig. 59 shows sensorgrams for anti-IL-6R antibody (tocilizumab), antibody 6RpH#01, antibody 6RpH#02, and antibody 6RpH#03 at pH 7.4. The horizontal axis shows time, and the vertical axis shows RU value.

Fig. 60 shows sensorgrams for anti-IL-6R antibody (tocilizumab), antibody 6RpH#01, antibody 6RpH#02, and antibody 6RpH#03 at pH 6.0. The horizontal axis shows time, and the vertical axis shows RU value.

Fig. 61A depicts a graph of ECL responses to native Fc and altered Fc from sera isolated from 15 to 30 independent rheumatism patients. Graphs of ECL responses to native Fc (Fig. 61A), Fv4-YTE (Fig. 61B), Fv4-F1166 (= YTE + Q438R/S440E) (Fig. 61C), Fv4-F1167 (= YTE + S424N) (Fig. 61D), Fv4-LS (Fig. 61E), Fv4-F1170 (= LS + Q438R/S440E) (Fig. 61F), Fv4-F1171 (= LS + S424N) (Fig. 61G), Fv4-N434H (Fig. 61H), Fv4-F1172 (= N434H + Q438R/S440E) (Fig. 61I), Fv4-F1173 (= N434H + S424N) (Fig. 61J) are shown, respectively.
Fig. 61B is a continuation of Fig. 61A.
Fig. 61C is a continuation of Fig. 61B.
Fig. 61D is a continuation of Fig. 61C.
Fig. 61E is a continuation of Fig. 61D.
Fig. 61F is a continuation of Fig. 61E.
Fig. 61G is a continuation of Fig. 61F.
Fig. 61H is a continuation of Fig. 61G.
Fig. 61I is a continuation of Fig. 61H.
Fig. 61J is a continuation of Fig. 61I.
Fig. 62A depicts a graph of ECL responses to altered Fc from sera isolated from 30 independent rheumatism patients. Graphs of ECL responses to Fv4-LS (Fig. 62A), Fv4-F1380 (Fig. 62B), Fv4-F1384 (Fig. 62C), Fv4-F1385 (Fig. 62D), Fv4-F1386 (Fig. 62E), Fv4-F1388 (Fig. 62F), and Fv4-F1389 (Fig. 62G) are shown, respectively.
Fig. 62B is a continuation of Fig. 62A.
Fig. 62C is a continuation of Fig. 62B.
Fig. 62D is a continuation of Fig. 62C.
Fig. 62E is a continuation of Fig. 62D.
Fig. 62F is a continuation of Fig. 62E.
Fig. 62G is a continuation of Fig. 62F.

[Mode for Carrying Out the Invention]

The definitions and detailed description below are provided to help the understanding of the present invention illustrated herein.

Amino acids

Herein, amino acids are described in one- or three-letter codes or both, for example, Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, or Val/V.

Alteration of amino acids

For amino acid alterations in the amino acid sequence of an antigen-binding molecule, known methods such as site-directed mutagenesis methods (Kunkel et al. (Proc. Natl. Acad. Sci. USA (1985) 82, 488-492)) and overlap extension PCR may be appropriately employed. Additions, deletions, and/or substitutions of an amino acid are added appropriately by these known methods. Substituting amino acid residues means substituting an amino acid residue with another amino acid residue for the purpose of altering aspects such as the following:

(a) backbone structure of a polypeptide in a helical structure region or a sheet structure region;
(b) charge or hydrophobicity at a target site; or
(c) length of a side chain.

Amino acid residues are classified into the following groups based on the properties of side chains included in their structures:

(1) hydrophobic: norleucine, Met, Ala, Val, Leu, and Ile;
(2) neutral hydrophilic: Cys, Ser, Thr, Asn, and Gln:
(3) acidic: Asp and Glu;
(4) basic: His, Lys, and Arg;
(5) residues that affect the orientation of the chain: Gly and Pro; and
(6) aromatic: Trp, Tyr, and Phe.

Substitution between amino acid residues within each of these groups is referred to as conservative substitution. On the other hand, substitution between amino acid residues from different amino acid groups is referred to as non-conservative substitution. Substitutions in the present invention may be conservative substitutions or non-conservative substitutions, or a combination of conservative and non-conservative substitutions. Furthermore, a plurality of known methods may be employed as amino acid alteration methods for substitution to non-native amino acids (Annu. Rev. Biophys. Biomol. Struct. (2006) 35, 225-249; and Proc. Natl. Acad. Sci. U.S.A. (2003) 100 (11), 6353-6357). For example, a cell-free translation system (Clover Direct (Protein Express)) containing a tRNA which has the non-native amino acid bound to a complementary amber suppressor tRNA of the UAG codon (amber codon), which is one of the stop codons, is suitably used.

Furthermore, an expression that uses one-letter amino-acid codes of the amino acid before alteration and the amino acid after the alteration before and after a number indicating a specific position, respectively, may be used appropriately as an expression for an amino acid alteration. For example, the alteration P238D, which is used when substituting an amino acid of the Fc region included in an antibody constant region, expresses substitution of Pro at position 238 (according to EU numbering) with Asp. That is, the number shows the position of the amino acid according to EU numbering, the one-letter amino-acid code written before the number shows the amino acid before substitution, and the one-letter amino-acid code written after the number shows the amino acid after substitution.

## And/or

As used herein, the term "and/or" means a combination of the terms before and after the set phrase "and/or", and includes every combination where "and" and "or" are suitably combined. Specifically, for example, "the amino acids at positions 326, 328, and/or 428 are substituted" includes a variation of alterations of the following amino acids:
amino acid(s) at (a) position 326, (b) position 328, (c) position 428, (d) positions 326 and 328, (e) positions 326 and 428, (f) positions 328 and 428, and (g) positions 326, 328, and 428.

## Antigens

As used herein, the structure of an "antigen" is not particularly limited to a specific structure as long as it includes an epitope which is bound by an antigen-binding domain. In another meaning, an antigen may be an inorganic matter or an organic matter, and it is preferably a soluble antigen which is present in the body fluid of an organism and which is in an embodiment which may be bound by an antigen-binding molecule of the present invention.
The following molecules are examples of the antigens:
17-IA, 4-1BB, 4Dc, 6-keto-PGF1a, 8-iso-PGF2a, 8-oxo-dG, A1 adenosine receptor, A33, ACE, ACE-2, activin, activin A, activin AB, activin B, activin C, activin RIA, activin RIAALK-2, activin RIB ALK-4, activin RIIA, activin RIIB, ADAM, ADAM10, ADAM12, ADAM15, ADAM17/TACE, ADAM8, ADAM9, ADAMTS, ADAMTS4, ADAMTS5, addressin, aFGF, ALCAM, ALK, ALK-1, ALK-7, alpha-1-antitrypsin, alpha-V/beta-1 antagonist, ANG, Ang, APAF-1, APE, APJ, APP, APRIL, AR, ARC, ART, artemin, anti-Id, ASPARTIC, atrial natriuretic peptide, av/b3 integrin, Axl, b2M, B7-1, B7-2, B7-H, B-lymphocyte stimulating factor (BlyS), BACE, BACE-1, Bad, BAFF, BAFF-R, Bag-1, BAK, Bax, BCA-1, BCAM, Bcl, BCMA, BDNF, b-ECGF, bFGF, BID, Bik, BIM, BLC, BL-CAM, BLK, BMP, BMP-2 BMP-2a, BMP-3 Osteogenin, BMP-4 BMP-2b, BMP-5, BMP-6 Vgr-1, BMP-7 (OP-1), BMP-8 (BMP-8a, OP-2), BMPR, BMPR-IA (ALK-3), BMPR-IB (ALK-6), BRK-2, RPK-1, BMPR-II (BRK-3), BMP, b-NGF, BOK, bombesin, bone-derived neurotrophic factor, BPDE, BPDE-DNA, BTC, complement factor 3 (C3), C3a, C4, C5, C5a, C10, CA125, CAD-8, calcitonin, cAMP, carcinoembryonic antigen (CEA), cancer associated antigen, cathepsin A, cathepsin B, cathepsin C/DPPI, cathepsin D, cathepsin E, cathepsin H, cathepsin L, cathepsin O, cathepsin S, cathepsin V, cathepsin X/Z/P, CBL, CCI, CCK2, CCL, CCL1, CCL11, CCL12, CCL13, CCL14, CCL15, CCL16, CCL17, CCL18, CCL19, CCL2, CCL20, CCL21, CCL22, CCL23, CCL24, CCL25, CCL26, CCL27, CCL28, CCL3, CCL4, CCL5, CCL6, CCL7, CCL8, CCL9/10, CCR, CCR1, CCR10, CCR10, CCR2, CCR3, CCR4, CCR5, CCR6, CCR7, CCR8, CCR9, CD1, CD2, CD3, CD3E, CD4, CD5, CD6, CD7, CD8, CD10, CD11a, CD11b, CD11c, CD13, CD14, CD15, CD16, CD18, CD19, CD20, CD21, CD22, CD23, CD25, CD27L, CD28, CD29, CD30, CD30L, CD32, CD33 (p67 protein), CD34, CD38, CD40, CD40L, CD44, CD45, CD46, CD49a, CD52, CD54, CD55, CD56, CD61, CD64, CD66e, CD74, CD80 (B7-1), CD89, CD95, CD123, CD137, CD138, CD140a, CD146, CD147, CD148, CD152, CD164, CEACAM5, CFTR, cGMP, CINC, Botulinum toxin, Clostridium perfringens toxin, CKb8-1, CLC, CMV, CMV UL, CNTF, CNTN-1, COX, C-Ret, CRG-2, CT-1, CTACK, CTGF, CTLA-4, CX3CL1, CX3CR1, CXCL, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCR, CXCR1, CXCR2, CXCR3, CXCR4, CXCR5, CXCR6,cytokeratin tumor associated antigen, DAN, DCC, DcR3, DC-SIGN, complement regulatory factor (Decay accelerating factor), des (1-3)-IGF-I (brain IGF-1), Dhh, digoxin, DNAM-1, Dnase, Dpp, DPPIV/CD26, Dtk, ECAD, EDA, EDA-A1, EDA-A2, EDAR, EGF, EGFR (ErbB-1), EMA, EMMPRIN, ENA, endothelin receptor, enkephalinase, eNOS, Eot, eotaxin 1, EpCAM, ephrin B2/EphB4, EPO, ERCC, E-selectin, ET-1, factor IIa, factor VII, factor VIIIc, factor IX, fibroblast activation protein (FAP), Fas, FcR1, FEN-1, ferritin, FGF, FGF-19, FGF-2, FGF3, FGF-8, FGFR, FGFR-3, fibrin, FL, FLIP, Flt-3, Flt-4, follicle stimulating hormone, fractalkine, FZD1, FZD2, FZD3, FZD4, FZD5, FZD6, FZD7, FZD8, FZD9, FZD10, G250, Gas6, GCP-2, GCSF, GD2, GD3, GDF, GDF-1, GDF-3 (Vgr-2), GDF-5 (BMP-14, CDMP-1), GDF-6 (BMP-13, CDMP-2), GDF-7 (BMP-12, CDMP-3), GDF-8 (myostatin), GDF-9, GDF-15 (MIC-1), GDNF, GDNF, GFAP, GFRa-1, GFR-alpha1, GFR-alpha2, GFR-alpha3, GITR, glucagon, Glut4, glycoprotein IIb/IIIa (GPIIb/IIIa), GM-CSF, gp130, gp72, GRO, growth hormone releasing hormone, hapten (NP-cap or NIP-cap), HB-EGF, HCC, HCMV gB envelope glycoprotein, HCMV gH envelope glycoprotein, HCMV UL, hematopoietic growth factor (HGF), Hep B gp120, heparanase, Her2, Her2/neu (ErbB-2), Her3 (ErbB-3), Her4 (ErbB-4), herpes simplex virus (HSV) gB glycoprotein, HSV gD glycoprotein, HGFA, high molecular weight melanoma-associated antigen (HMW-MAA), HIV gp120, HIV IIIB gp 120 V3 loop, HLA, HLA-DR, HM1.24, HMFG PEM, HRG, Hrk, human cardiac myosin, human cytomegalovirus (HCMV), human growth hormone (HGH), HVEM, 1-309, IAP, ICAM, ICAM-1, ICAM-3, ICE, ICOS, IFNg, Ig, IgA receptor, IgE, IGF, IGF binding protein, IGF-1R, IGFBP, IGF-I, IGF-II, IL, IL-1, IL-1R, IL-2, IL-2R, IL-4, IL-4R, IL-5, IL-5R, IL-6, IL-6R, IL-8, IL-9, IL-10, IL-12, IL-13, IL-15, IL-18, IL-18R, IL-23, interferon (INF)-alpha, INF-beta, INF-gamma, inhibin, iNOS, insulin A chain, insulin B chain, insulin-like growth factor1, integrin alpha2, integrin alpha3, integrin alpha4, integrin alpha4/beta1, integrin alpha4/beta7, integrin alpha5 (alpha V), integrin alpha5/beta1, integrin alpha5/beta3, integrin alpha6, integrin beta1, integrin beta2,interferon gamma, IP-10, I-TAC, JE, kallikrein 2, kallikrein 5, kallikrein 6, kallikrein 11, kallikrein 12, kallikrein 14, kallikrein 15, kallikrein L1, kallikrein L2, kallikrein L3, kallikrein L4, KC, KDR, keratinocyte growth factor (KGF), laminin 5, LAMP, LAP, LAP (TGF-1), latent TGF-1, latent TGF-1 bp1, LBP, LDGF, LECT2, lefty, Lewis-Y antigen, Lewis-Y associated antigen, LFA-1, LFA-3, Lfo, LIF, LIGHT, lipoprotein, LIX, LKN, Lptn, L-selectin, LT-a, LT-b, LTB4, LTBP-1, lung surface, luteinizing hormone, lymphotoxin beta receptor, Mac-1, MAdCAM, MAG, MAP2, MARC, MCAM, MCAM, MCK-2, MCP, M-CSF, MDC, Mer, METALLOPROTEASES, MGDF receptor, MGMT, MHC (HLA-DR), MIF, MIG, MIP, MIP-1-alpha, MK, MMAC1, MMP, MMP-1, MMP-10, MMP-11, MMP-12, MMP-13, MMP-14, MMP-15, MMP-2, MMP-24, MMP-3, MMP-7, MMP-8, MMP-9, MPIF, Mpo, MSK, MSP, mucin (Muc1), MUC18, Mullerian-inhibiting substance, Mug, MuSK, NAIP, NAP, NCAD, N-C adherin, NCA 90, NCAM, NCAM, neprilysin, neurotrophin-3, -4, or -6, neurturin, nerve growth factor (NGF), NGFR, NGF-beta, nNOS, NO, NOS, Npn, NRG-3, NT, NTN, OB, OGG1, OPG, OPN, OSM, OX40L, OX40R, p150, p95, PADPr, parathyroid hormone, PARC, PARP, PBR, PBSF, PCAD, P-cadherin, PCNA, PDGF, PDGF, PDK-1, PECAM, PEM, PF4, PGE, PGF, PGI2, PGJ2, PIN, PLA2, placental alkaline phosphatase (PLAP), PIGF, PLP, PP14, proinsulin, prorelaxin, protein C, PS, PSA, PSCA, prostate-specific membrane antigen (PSMA), PTEN, PTHrp, Ptk, PTN, R51, RANK, RANKL, RANTES, RANTES, relaxin A chain, relaxin B chain, renin, respiratory syncytial virus (RSV) F, RSV Fgp, Ret, Rheumatoid factor, RLIP76, RPA2, RSK, S100, SCF/KL, SDF-1, SERINE, serum albumin, sFRP-3, Shh, SIGIRR, SK-1, SLAM, SLPI, SMAC, SMDF, SMOH, SOD, SPARC, Stat, STEAP, STEAP-II, TACE, TACI, TAG-72 (tumor-associated glycoprotein-72), TARC, TCA-3, T-cell receptor (for example, T-cell receptor alpha/beta), TdT, TECK, TEM1, TEM5, TEM7, TEM8, TERT, testis PLAP-like alkaline phosphatase, TfR, TGF, TGF-alpha, TGF-beta, TGF-beta Pan Specific, TGF-betaRI (ALK-5), TGF-betaRII, TGF-betaRIIb, TGF-betaRIII, TGF-beta1, TGF-beta2, TGF-beta3, TGF-beta4, TGF-beta5, thrombin, thymus Ck-1, thyroid-stimulating hormone, Tie, TIMP, TIQ, tissue factor, TMEFF2, Tmpo, TMPRSS2, TNF, TNF-alpha, TNF-alphabeta, TNF-beta2, TNFc, TNF-RI, TNF-RII, TNFRSF10A (TRAIL R1 Apo-2, DR4), TNFRSF10B (TRAIL R2 DR5, KILLER, TRICK-2A, TRICK-B), TNFRSF10C (TRAIL R3 DcR1, LIT, TRID), TNFRSF10D (TRAIL R4 DcR2, TRUNDD), TNFRSF11A (RANK ODF R, TRANCE R), TNFRSF11B (OPG OCIF, TR1), TNFRSF12 (TWEAK R FN14), TNFRSF13B (TACI), TNFRSF13C (BAFF R), TNFRSF14 (HVEM ATAR, HveA, LIGHT R, TR2), TNFRSF16 (NGFR p75NTR), TNFRSF17 (BCMA), TNFRSF18 (GITR AITR), TNFRSF19 (TROY TAJ, TRADE), TNFRSF19L (RELT), TNFRSF1A (TNF RI CD120a, p55-60), TNFRSF1B (TNF RII CD120b, p75-80), TNFRSF26 (TNFRH3), TNFRSF3 (LTbR TNF RIII, TNFC R), TNFRSF4 (OX40 ACT35, TXGP1 R), TNFRSF5 (CD40 p50), TNFRSF6 (Fas Apo-1, APT1, CD95), TNFRSF6B (DcR3 M68, TR6), TNFRSF7 (CD27), TNFRSF8 (CD30), TNFRSF9 (4-1BB CD137, ILA), TNFRSF21 (DR6), TNFRSF22 (DcTRAIL R2 TNFRH2), TNFRST23 (DcTRAIL R1 TNFRH1), TNFRSF25 (DR3 Apo-3, LARD, TR-3, TRAMP, WSL-1), TNFSF10 (TRAIL Apo-2 ligand, TL2), TNFSF11 (TRANCE/RANK ligand ODF, OPG ligand), TNFSF12 (TWEAK Apo-3 ligand, DR3 ligand), TNFSF13 (APRIL TALL2), TNFSF13B (BAFF BLYS, TALL1, THANK, TNFSF20), TNFSF14 (LIGHT HVEM ligand, LTg), TNFSF15 (TL1A/VEGI), TNFSF18 (GITR ligand AITR ligand, TL6), TNFSF1A (TNF-a Conectin, DIF, TNFSF2), TNFSF1B (TNF-b LTa, TNFSF1), TNFSF3 (LTb TNFC, p33), TNFSF4 (OX40 ligand gp34, TXGP1), TNFSF5 (CD40 ligand CD154, gp39, HIGM1, IMD3, TRAP), TNFSF6 (Fas ligand Apo-1 ligand, APT1 ligand), TNFSF7 (CD27 ligand CD70), TNFSF8 (CD30 ligand CD153), TNFSF9 (4-1BB ligand CD137 ligand), TP-1, t-PA, Tpo, TRAIL, TRAIL R, TRAIL-R1, TRAIL-R2, TRANCE, transferrin receptor, TRF, Trk, TROP-2, TSG, TSLP, tumor associated antigen CA125, tumor associated antigen expressing Lewis-Y associated carbohydrates, TWEAK, TXB2, Ung, uPAR, uPAR-1, urokinase, VCAM, VCAM-1, VECAD, VE-Cadherin, VE-cadherin-2, VEFGR-1 (flt-1), VEGF, VEGFR, VEGFR-3 (flt-4), VEGI, VIM, virus antigen, VLA, VLA-1, VLA-4, VNR integrin, von Willebrand factor, WIF-1, WNT1, WNT2, WNT2B/13, WNT3, WNT3A, WNT4, WNT5A, WNT5B, WNT6, WNT7A, WNT7B, WNT8A, WNT8B, WNT9A, WNT9A, WNT9B, WNT10A, WNT10B, WNT11, WNT16, XCL1, XCL2, XCR1, XCR1, XEDAR, XIAP, XPD, HMGB1, IgA, Aβ, CD81, CD97, CD98, DDR1, DKK1, EREG, Hsp90, IL-17/IL-17R, IL-20/IL-20R, oxidized LDL, PCSK9, prekallikrein, RON, TMEM16F, SOD1, Chromogranin A, Chromogranin B, tau, VAP1, high molecular weight kininogen, IL-31, IL-31R, Nav1.1, Nav1.2, Nav1.3, Nav1.4, Nav1.5, Nav1.6, Nav1.7, Nav1.8, Nav1.9, EPCR, C1, C1q, C1r, C1s, C2, C2a, C2b, C3, C3a, C3b, C4, C4a, C4b, C5, C5a, C5b, C6, C7, C8, C9, factor B, factor D, factor H, properdin, sclerostin, fibrinogen, fibrin, prothrombin, thrombin, tissue factor, factor V, factor Va, factor VII, factor VIIa, factor VIII, factor VIIIa, factor IX, factor IXa, factor X, factor Xa, factor XI, factor XIa, factor XII, factor XIIa, factor XIII, factor XIIIa, TFPI, antithrombin III, EPCR, thrombomodulin, TAPI, tPA, plasminogen, plasmin, PAI-1, PAI-2, GPC3, Syndecan-1, Syndecan-2, Syndecan-3, Syndecan-4, LPA, SIP, Acetylcholine receptor, AdipoR1, AdipoR2, ADP ribosyl cyclase-1, alpha-4/beta-7 integrin, alpha-5/beta-1 integrin, alpha-v/beta-6 integrin, alphavbeta1 integrin, Angiopoietin ligand-2, Angptl2, Anthrax, Cadherin, Carbonic anhydrase-IX, CD105, CD155, CD158a, CD37, CD49b, CD51, CD70, CD72, Claudin 18, Clostridium difficile toxin, CS1, Delta-like protein ligand 4, DHICA oxidase, Dickkopf-1 ligand, Dipeptidyl peptidase IV, EPOR, F protein of RSV, Factor Ia, FasL, Folate receptor alpha, Glucagon receptor, Glucagon-like peptide 1 receptor, Glutamate carboxypeptidase II, GMCSFR, Hepatitis C virus E2 glycoprotein, Hepcidin, IL-17 receptor, IL-22 receptor, IL-23 receptor, IL-3 receptor, Kit tyrosine kinase, Leucine Rich Alpha-2-Glycoprotein 1 (LRG1), Lysosphingolipid receptor, Membrane glycoprotein OX2, Mesothelin, MET, MICA, MUC-16, Myelin associated glycoprotein, Neuropilin-1, Neuropilin-2, Nogo receptor, PLXNA1, PLXNA2, PLXNA3, PLXNA4A, PLXNA4B, PLXNB1, PLXNB2, PLXNB3, PLXNC1, PLXND1, Programmed cell death ligand 1, Proprotein convertase PC9, P-selectin glycoprotein ligand-1, RAGE, Reticulon 4, RF, RON-8, SEMA3A, SEMA3B, SEMA3C, SEMA3D, SEMA3E, SEMA3F, SEMA3G, SEMA4A, SEMA4B, SEMA4C, SEMA4D, SEMA4F, SEMA4G, SEMA5A, SEMA5B, SEMA6A, SEMA6B, SEMA6C, SEMA6D, SEMA7A, Shiga like toxin II, Sphingosine-1-phosphate receptor-1, ST2, Staphylococcal lipoteichoic acid, Tenascin, TG2, Thymic stromal lymphoprotein receptor, TNF superfamily receptor 12A, Transmembrane glycoprotein NMB, TREM-1, TREM-2, Trophoblast glycoprotein, TSH receptor, TTR, Tubulin, and ULBP2, and receptors for growth factors and hormones, molecules that exist in their soluble form and are not anchored to cells in the body fluid of organisms. For example, among the receptors, soluble antigens present in the body fluid of an organism due to some mechanism including protease-mediated digestion of receptors or such expressed on a cell surface are also suitable examples of the soluble antigens of the present invention. Examples of such molecules may include the soluble IL-6R molecule (J. Immunol. (1994) 152, 4958-4968) and CD20, as well as CD52 (Br. J. Haematol. (2003) 123 (5), 850-857), described herein. Furthermore, not only the molecules inherently expressed in a living organism, but also soluble antigens existing in the body fluid of an organism, which are infectious molecules such as prions or antigens presented by infectious organisms such as viruses or presented on such organisms are also examples of the soluble antigens of the present invention. Suitable examples of the body fluid include blood, plasma, serum, urine, lymph, saliva, and tear fluid.

Epitope

"Epitope" means an antigenic determinant in an antigen, and refers to an antigen site to which the antigen-binding domain of an antigen-binding molecule disclosed herein binds. Thus, for example, the epitope can be defined according to its structure. Alternatively, the epitope may be defined according to the antigen-binding activity of an antigen-binding molecule that recognizes the epitope. When the antigen is a peptide or polypeptide, the epitope can be specified by the amino acid residues forming the epitope. Alternatively, when the epitope is a sugar chain, the epitope can be specified by its specific sugar chain structure.

A linear epitope is an epitope that contains an epitope whose primary amino acid sequence is recognized. Such a linear epitope typically contains at least three and most commonly at least five, for example, about 8 to about 10 or 6 to 20 amino acids in its specific sequence.

In contrast to the linear epitope, "conformational epitope" is an epitope in which the primary amino acid sequence containing the epitope is not the only determinant of the recognized epitope (for example, the primary amino acid sequence of a conformational epitope is not necessarily recognized by an epitope-defining antibody). Conformational epitopes may contain a greater number of amino acids compared to linear epitopes. A conformational epitope-recognizing antibody recognizes the three-dimensional structure of a peptide or protein. For example, when a protein molecule folds and forms a three-dimensional structure, amino acids and/or polypeptide main chains that form a conformational epitope become aligned, and the epitope is made recognizable by the antibody. Methods for determining epitope conformations include, for example, X ray crystallography, two-dimensional nuclear magnetic resonance, site-specific spin labeling, and electron paramagnetic resonance, but are not limited thereto. See, for example, Epitope Mapping Protocols in Methods in Molecular Biology (1996), Vol. 66, Morris (ed.).

Binding Activity

Examples of a method for assessing the epitope binding by a test antigen-binding molecule containing an IL-6R antigen-binding domain are described below. According to the examples below, methods for assessing the epitope binding by a test antigen-binding molecule containing an antigen-binding domain for an antigen other than IL-6R, can also be appropriately conducted.

For example, whether a test antigen-binding molecule containing an IL-6R antigen-binding domain recognizes a linear epitope in the IL-6R molecule can be confirmed for example as mentioned below. A linear peptide comprising an amino acid sequence forming the extracellular domain of IL-6R is synthesized for the above purpose. The peptide can be synthesized chemically, or obtained by genetic engineering techniques using a region encoding the amino acid sequence corresponding to the extracellular domain in an IL-6R cDNA. Then, a test antigen-binding molecule containing an IL-6R antigen-binding domain is assessed for its binding activity towards a linear peptide comprising the amino acid sequence forming the extracellular domain. For example, an immobilized linear peptide can be used as an antigen by ELISA to evaluate the binding activity of the antigen-binding molecule towards the peptide. Alternatively, the binding activity towards a linear peptide can be assessed based on the level that the linear peptide inhibits the binding of the antigen-binding molecule to IL-6R-expressing cells. These tests can demonstrate the binding activity of the antigen-binding molecule towards the linear peptide.

Whether a test antigen-binding molecule containing an IL-6R antigen-binding domain recognizes a conformational epitope can be assessed as follows. IL-6R-expressing cells are prepared for the above purpose. A test antigen-binding molecule containing an IL-6R antigen-binding domain can be determined to recognize a conformational epitope when it strongly binds to IL-6R-expressing cells upon contact, but does not substantially bind to an immobilized linear peptide comprising an amino acid sequence forming the extracellular domain of IL-6R. Herein, "not substantially bind" means that the binding activity is 80% or less, generally 50% or less, preferably 30% or less, and particularly preferably 15% or less compared to the binding activity towards cells expressing human IL-6R.

Methods for assaying the binding activity of a test antigen-binding molecule containing an IL-6R antigen-binding domain towards IL-6R-expressing cells include, for example, the methods described in Antibodies: A Laboratory Manual (Ed Harlow, David Lane, Cold Spring Harbor Laboratory (1988) 359-420). Specifically, the assessment can be performed based on the principle of ELISA or fluorescence activated cell sorting (FACS) using IL-6R-expressing cells as antigen.

In the ELISA format, the binding activity of a test antigen-binding molecule containing an IL-6R antigen-binding domain towards IL-6R-expressing cells can be assessed quantitatively by comparing the levels of signal generated by enzymatic reaction. Specifically, a test antigen-binding molecule is added to an ELISA plate onto which IL-6R-expressing cells are immobilized. Then, the test antigen-binding molecule bound to the cells is detected using an enzyme-labeled antibody that recognizes the test antigen-binding molecule. Alternatively, when FACS is used, a dilution series of a test antigen-binding molecule is prepared, and the antibody binding titer for IL-6R-expressing cells can be determined to compare the binding activity of the test antigen-binding molecule towards IL-6R-expressing cells.

The binding of a test antigen-binding molecule towards an antigen expressed on the surface of cells suspended in buffer or the like can be detected using a flow cytometer. Known flow cytometers include, for example, the following devices:

FACSCanto™ II
FACSAria™
FACSArray™
FACSVantage™ SE
FACSCalibur™ (all are trade names of BD Biosciences)
EPICS ALTRA HyPerSort
Cytomics FC 500
EPICS XL-MCL ADC EPICS XL ADC
Cell Lab Quanta/Cell Lab Quanta SC (all are trade names of Beckman Coulter).

Preferable methods for assaying the binding activity of a test antigen-binding molecule containing an IL-6R antigen-binding domain towards an antigen include, for example, the following method. First, IL-6R-expressing cells are reacted with a test antigen-binding molecule, and then this is stained with an FITC-labeled secondary antibody that recognizes the antigen-binding molecule. The test antigen-binding molecule is appropriately diluted with a suitable buffer to prepare the molecule at a desired concentration. For example, the molecule can be used at a concentration within the range of 10 μg/ml to 10 ng/ml. Then, the fluorescence intensity and cell count are determined using FACSCalibur (BD). The fluorescence intensity obtained by analysis using the CELL QUEST Software (BD), *i.e.,* the Geometric Mean value, reflects the quantity of antibody bound to cells. That is, the binding activity of a test antigen-binding molecule, which is represented by the quantity of the test antigen-binding molecule bound, can be determined by measuring the Geometric Mean value.

Whether a test antigen-binding molecule containing an IL-6R antigen-binding domain shares a common epitope with another antigen-binding molecule can be assessed based on the competition between the two molecules for the same epitope. The competition between antigen-binding molecules can be detected by cross-blocking assay or the like. For example, the competitive ELISA assay is a preferred cross-blocking assay.

Specifically, in cross-blocking assay, the IL-6R protein immobilized to the wells of a microtiter plate is pre-incubated in the presence or absence of a candidate competitor antigen-binding molecule, and then a test antigen-binding molecule is added thereto. The quantity of test antigen-binding molecule bound to the IL-6R protein in the wells is indirectly correlated with the binding ability of a candidate competitor antigen-binding molecule that competes for the binding to the same epitope. That is, the greater the affinity of the competitor antigen-binding molecule for the same epitope, the lower the binding activity of the test antigen-binding molecule towards the IL-6R protein-coated wells.

The quantity of the test antigen-binding molecule bound to the wells *via* the IL-6R protein can be readily determined by labeling the antigen-binding molecule in advance. For example, a biotin-labeled antigen-binding molecule is measured using an avidin/peroxidase conjugate and appropriate substrate. In particular, cross-blocking assay that uses enzyme labels such as peroxidase is called "competitive ELISA assay". The antigen-binding molecule can also be labeled with other labeling substances that enable detection or measurement. Specifically, radiolabels, fluorescent labels, and such are known.

When the candidate competitor antigen-binding molecule can block the binding by a test antigen-binding molecule containing an IL-6R antigen-binding domain by at least 20%, preferably at least 20 to 50%, and more preferably at least 50% compared to the binding activity in a control experiment conducted in the absence of the competitor antigen-binding molecule, the test antigen-binding molecule is determined to substantially bind to the same epitope bound by the competitor antigen-binding molecule, or compete for the binding to the same epitope.

When the structure of an epitope bound by a test antigen-binding molecule containing an IL-6R antigen-binding domain has already been identified, whether the test and control antigen-binding molecules share a common epitope can be assessed by comparing the binding activities of the two antigen-binding molecules towards a peptide prepared by introducing amino acid mutations into the peptide forming the epitope.

To measure the above binding activities, for example, the binding activities of test and control antigen-binding molecules towards a linear peptide into which a mutation is introduced are compared in the above ELISA format. Besides the ELISA methods, the binding activity towards the mutant peptide bound to a column can be determined by flowing test and control antigen-binding molecules in the column, and then quantifying the antigen-binding molecule eluted in the elution solution. Methods for adsorbing a mutant peptide to a column, for example, in the form of a GST fusion peptide, are known.

Alternatively, when the identified epitope is a conformational epitope, whether test and control antigen-binding molecules share a common epitope can be assessed by the following method. First, IL-6R-expressing cells and cells expressing IL-6R with a mutation introduced into the epitope are prepared. The test and control antigen-binding molecules are added to a cell suspension prepared by suspending these cells in an appropriate buffer such as PBS. Then, the cell suspensions are appropriately washed with a buffer, and an FITC-labeled antibody that recognizes the test and control antigen-binding molecules is added thereto. The fluorescence intensity and number of cells stained with the labeled antibody are determined using FACSCalibur (BD). The test and control antigen-binding molecules are appropriately diluted using a suitable buffer, and used at desired concentrations. For example, they may be used at a concentration within the range of 10 μg/ml to 10 ng/ml. The fluorescence intensity determined by analysis using the CELL QUEST Software (BD), *i.e.,* the Geometric Mean value, reflects the quantity of labeled antibody bound to cells. That is, the binding activities of the test and control antigen-binding molecules, which are represented by the quantity of labeled antibody bound, can be determined by measuring the Geometric Mean value.

In the above method, whether an antigen-binding molecule does "not substantially bind to cells expressing mutant IL-6R" can be assessed, for example, by the following method. First, the test and control antigen-binding molecules bound to cells expressing mutant IL-6R are stained with a labeled antibody. Then, the fluorescence intensity of the cells is determined. When FACSCalibur is used for fluorescence detection by flow cytometry, the determined fluorescence intensity can be analyzed using the CELL QUEST Software. From the Geometric Mean values in the presence and absence of the antigen-binding molecule, the comparison value (ΔGeo-Mean) can be calculated according to the following formula to determine the ratio of increase in fluorescence intensity as a result of the binding by the antigen-binding molecule.

ΔGeo-Mean = Geo-Mean (in the presence of the antigen-binding molecule)/Geo-Mean (in the absence of the antigen-binding molecule)

The Geometric Mean comparison value (ΔGeo-Mean value for the mutant IL-6R molecule) determined by the above analysis, which reflects the quantity of a test antigen-binding molecule bound to cells expressing mutant IL-6R, is compared to the ΔGeo-Mean comparison value that reflects the quantity of the test antigen-binding molecule bound to IL-6R-expressing cells. In this case, the concentrations of the test antigen-binding molecule used to determine the ΔGeo-Mean comparison values for IL-6R-expressing cells and cells expressing mutant IL-6R are particularly preferably adjusted to be equal or substantially equal. An antigen-binding molecule that has been confirmed to recognize an epitope in IL-6R is used as a control antigen-binding molecule.

If the ΔGeo-Mean comparison value of a test antigen-binding molecule for cells expressing mutant IL-6R is smaller than the ΔGeo-Mean comparison value of the test antigen-binding molecule for IL-6R-expressing cells by at least 80%, preferably 50%, more preferably 30%, and particularly preferably 15%, then the test antigen-binding molecule "does not substantially bind to cells expressing mutant IL-6R". The formula for determining the Geo-Mean (Geometric Mean) value is described in the CELL QUEST Software User's Guide (BD biosciences). When the comparison shows that the comparison values are substantially equivalent, the epitope for the test and control antigen-binding molecules can be determined to be the same.

Antigen-binding domain

Herein, an "antigen-binding domain" may be of any structure as long as it binds to an antigen of interest. Such domains preferably include, for example:

antibody heavy-chain and light-chain variable regions;
a module of about 35 amino acids called A domain which is contained in the *in vivo* cell membrane protein Avimer (WO 2004/044011, WO 2005/040229);
Adnectin containing the 10Fn3 domain which binds to the protein moiety of fibronectin, a glycoprotein expressed on cell membrane (WO 2002/032925);
Affibody which is composed of a 58-amino acid three-helix bundle based on the scaffold of the IgG-binding domain of Protein A (WO 1995/001937);
Designed Ankyrin Repeat proteins (DARPins) which are a region exposed on the molecular surface of ankyrin repeats (AR) having a structure in which a subunit consisting of a turn comprising 33 amino acid residues, two antiparallel helices, and a loop is repeatedly stacked (WO 2002/020565);
Anticalins and such, which are domains consisting of four loops that support one side of a barrel structure composed of eight circularly arranged antiparallel strands that are highly conserved among lipocalin molecules such as neutrophil gelatinase-associated lipocalin (NGAL) (WO 2003/029462); and
the concave region formed by the parallel-sheet structure inside the horseshoe-shaped structure constituted by stacked repeats of the leucine-rich-repeat (LRR) module of the variable lymphocyte receptor (VLR) which does not have the immunoglobulin structure and is used in the system of acquired immunity in jawless vertebrate such as lampery and hagfish (WO 2008/016854). Preferred antigen-binding domains of the present invention include, for example, those having antibody heavy-chain and light-chain variable regions. Preferred examples of antigen-binding domains include "single chain Fv (scFv)", "single chain antibody", "Fv", "single chain Fv 2 (scFv2)", "Fab", and "F(ab')2".

The antigen-binding domains of antigen-binding molecules of the present invention can bind to an identical epitope. Such epitope can be present, for example, in a protein comprising the amino acid sequence of SEQ ID NO: 1. Alternatively, the epitope can be present in the protein comprising the amino acids at positions 20 to 365 in the amino acid sequence of SEQ ID NO: 1. Alternatively, each of the antigen-binding domains of antigen-binding molecules of the present invention can bind to a different epitope. Herein, the different epitope can be present in, for example, a protein comprising the amino acid sequence of SEQ ID NO: 1. Alternatively, the epitope can be present in the protein comprising the amino acids at positions 20 to 365 in the amino acid sequence of SEQ ID NO: 1.

Specificity

"Specific" means that one of molecules that specifically binds to does not show any significant binding to molecules other than a single or a number of binding partner molecules. Furthermore, "specific" is also used when an antigen-binding domain is specific to a particular epitope among multiple epitopes in an antigen. When an epitope bound by an antigen-binding domain is contained in multiple different antigens, antigen-binding molecules containing the antigen-binding domain can bind to various antigens that have the epitope.

Antibodies

Herein, "antibody" refers to a natural immunoglobulin or an immunoglobulin produced by partial or complete synthesis. Antibodies can be isolated from natural sources such as naturally-occurring plasma and serum, or culture supernatants of antibody-producing hybridomas. Alternatively, antibodies can be partially or completely synthesized using techniques such as genetic recombination. Preferred antibodies include, for example, antibodies of an immunoglobulin isotype or subclass belonging thereto. Known human immunoglobulins include antibodies of the following nine classes (isotypes): IgG1, IgG2, IgG3, IgG4, IgA1, IgA2, IgD, IgE, and IgM. Of these isotypes, antibodies of the present invention include IgG1, IgG2, IgG3, and IgG4. IgG constant regions include mutants naturally formed therefrom. A number of allotype sequences due to genetic polymorphism are described in "Sequences of proteins of immunological interest", NIH Publication No.91-3242, for the constant regions of human IgG1, human IgG2, human IgG3, and human IgG4 antibodies, and any one of them may be used in the present invention. In particular for the human IgG1 sequence, the amino acid sequence of positions 356 to 358 (EU numbering) may be either DEL or EEM.

Methods for producing an antibody with desired binding activity are known to those skilled in the art. Below is an example that describes a method for producing an antibody that binds to IL-6R (anti-IL-6R antibody). Antibodies that bind to an antigen other than IL-6R can also be produced according to the example described below.

Anti-IL-6R antibodies can be obtained as polyclonal or monoclonal antibodies using known methods. The anti-IL-6R antibodies preferably produced are monoclonal antibodies derived from mammals. Such mammal-derived monoclonal antibodies include antibodies produced by hybridomas or host cells transformed with an expression vector carrying an antibody gene by genetic engineering techniques. "Humanized antibodies" or "chimeric antibodies" are included in the monoclonal antibodies of the present invention.

Monoclonal antibody-producing hybridomas can be produced using known techniques, for example, as described below. Specifically, mammals are immunized by conventional immunization methods using an IL-6R protein as a sensitizing antigen. Resulting immune cells are fused with known parental cells by conventional cell fusion methods. Then, hybridomas producing an anti-IL-6R antibody can be selected by screening for monoclonal antibody-producing cells using conventional screening methods.

Specifically, monoclonal antibodies are prepared as mentioned below. First, the IL-6R gene whose nucleotide sequence is disclosed in SEQ ID NO: 2 can be expressed to produce an IL-6R protein shown in SEQ ID NO: 1, which will be used as a sensitizing antigen for antibody preparation. That is, a gene sequence encoding IL-6R is inserted into a known expression vector, and appropriate host cells are transformed with this vector. The desired human IL-6R protein is purified from the host cells or their culture supernatants by known methods. In order to obtain soluble IL-6R from culture supernatants, for example, a protein consisting of the amino acids at positions 1 to 357 in the IL-6R polypeptide sequence of SEQ ID NO: 1, such as described in Mullberg et al. (J. Immunol. (1994) 152 (10), 4958-4968), is expressed as a soluble IL-6R, instead of the IL-6R protein of SEQ ID NO: 1. Purified natural IL-6R protein can also be used as a sensitizing antigen.

The purified IL-6R protein can be used as a sensitizing antigen for immunization of mammals. A partial IL-6R peptide may also be used as a sensitizing antigen. In this case, a partial peptide can be prepared by chemical synthesis based on the amino acid sequence of human IL-6R, or by inserting a partial IL-6R gene into an expression vector for expression. Alternatively, a partial peptide can be produced by degrading an IL-6R protein with a protease. The length and region of the partial IL-6R peptide are not limited to particular embodiments. A preferred region can be arbitrarily selected from the amino acid sequence at amino acid positions 20 to 357 in the amino acid sequence of SEQ ID NO: 1. The number of amino acids forming a peptide to be used as a sensitizing antigen is preferably at least five or more, six or more, or seven or more. More specifically, a peptide of 8 to 50 residues, more preferably 10 to 30 residues can be used as a sensitizing antigen.

For sensitizing antigen, alternatively it is possible to use a fusion protein prepared by fusing a desired partial polypeptide or peptide of the IL-6R protein with a different polypeptide. For example, antibody Fc fragments and peptide tags are preferably used to produce fusion proteins to be used as sensitizing antigens. Vectors for expression of such fusion proteins can be constructed by fusing in frame genes encoding two or more desired polypeptide fragments and inserting the fusion gene into an expression vector as described above. Methods for producing fusion proteins are described in Molecular Cloning 2nd ed. (Sambrook, J et al., Molecular Cloning 2nd ed., 9.47-9.58 (1989) Cold Spring Harbor Lab. Press). Methods for preparing IL-6R to be used as a sensitizing antigen, and immunization methods using IL-6R are specifically described in WO 2003/000883, WO 2004/022754, WO 2006/006693, and such.

There is no particular limitation on the mammals to be immunized with the sensitizing antigen. However, it is preferable to select the mammals by considering their compatibility with the parent cells to be used for cell fusion. In general, rodents such as mice, rats, and hamsters, rabbits, and monkeys are preferably used.

The above animals are immunized with a sensitizing antigen by known methods. Generally performed immunization methods include, for example, intraperitoneal or subcutaneous injection of a sensitizing antigen into mammals. Specifically, a sensitizing antigen is appropriately diluted with PBS (Phosphate-Buffered Saline), physiological saline, or the like. If desired, a conventional adjuvant such as Freund's complete adjuvant is mixed with the antigen, and the mixture is emulsified. Then, the sensitizing antigen is administered to a mammal several times at 4- to 21-day intervals. Appropriate carriers may be used in immunization with the sensitizing antigen. In particular, when a low-molecular-weight partial peptide is used as the sensitizing antigen, it is sometimes desirable to couple the sensitizing antigen peptide to a carrier protein such as albumin or keyhole limpet hemocyanin for immunization.

Alternatively, hybridomas producing a desired antibody can be prepared using DNA immunization as mentioned below. DNA immunization is an immunization method that confers immunostimulation by expressing a sensitizing antigen in an animal immunized as a result of administering a vector DNA constructed to allow expression of an antigen protein-encoding gene in the animal. As compared to conventional immunization methods in which a protein antigen is administered to animals to be immunized, DNA immunization is expected to be superior in that:

- immunostimulation can be provided while retaining the structure of a membrane protein such as IL-6R; and
- there is no need to purify the antigen for immunization.

In order to prepare a monoclonal antibody of the present invention using DNA immunization, first, a DNA expressing an IL-6R protein is administered to an animal to be immunized. The IL-6R-encoding DNA can be synthesized by known methods such as PCR. The obtained DNA is inserted into an appropriate expression vector, and then this is administered to an animal to be immunized. Preferably used expression vectors include, for example, commercially-available expression vectors such as pcDNA3.1. Vectors can be administered to an organism using conventional methods. For example, DNA immunization is performed by using a gene gun to introduce expression vector-coated gold particles into cells in the body of an animal to be immunized. Antibodies that recognized IL-6R can also be produced by the methods described in WO 2003/104453.

After immunizing a mammal as described above, an increase in the titer of an IL-6R-binding antibody is confirmed in the serum. Then, immune cells are collected from the mammal, and then subjected to cell fusion. In particular, splenocytes are preferably used as immune cells.

A mammalian myeloma cell is used as a cell to be fused with the above-mentioned immune cells. The myeloma cells preferably comprise a suitable selection marker for screening. A selection marker confers characteristics to cells for their survival (or death) under a specific culture condition. Hypoxanthine-guanine phosphoribosyltransferase deficiency (hereinafter abbreviated as HGPRT deficiency) and thymidine kinase deficiency (hereinafter abbreviated as TK deficiency) are known as selection markers. Cells with HGPRT or TK deficiency have hypoxanthine-aminopterin-thymidine sensitivity (hereinafter abbreviated as HAT sensitivity). HAT-sensitive cells cannot synthesize DNA in a HAT selection medium, and are thus killed. However, when the cells are fused with normal cells, they can continue DNA synthesis using the salvage pathway of the normal cells, and therefore they can grow even in the HAT selection medium.

HGPRT-deficient and TK-deficient cells can be selected in a medium containing 6-thioguanine, 8-azaguanine (hereinafter abbreviated as 8AG), or 5'-bromodeoxyuridine, respectively. Normal cells are killed because they incorporate these pyrimidine analogs into their DNA. Meanwhile, cells that are deficient in these enzymes can survive in the selection medium, since they cannot incorporate these pyrimidine analogs. In addition, a selection marker referred to as G418 resistance provided by the neomycin-resistant gene confers resistance to 2-deoxystreptamine antibiotics (gentamycin analogs). Various types of myeloma cells that are suitable for cell fusion are known.

For example, myeloma cells including the following cells can be preferably used:

P3(P3x63Ag8.653) (J. Immunol. (1979) 123 (4), 1548-1550);
P3x63Ag8U.1 (Current Topics in Microbiology and Immunology (1978)81, 1-7);
NS-1 (C. Eur. J. Immunol. (1976)6 (7), 511-519);
MPC-11 (Cell (1976) 8 (3), 405-415);
SP2/0 (Nature (1978) 276 (5685), 269-270);
FO (J. Immunol. Methods (1980) 35 (1-2), 1-21);
S194/5.XX0.BU.1 (J. Exp. Med. (1978) 148 (1), 313-323);
R210 (Nature (1979) 277 (5692), 131-133), etc.

Cell fusions between the immunocytes and myeloma cells are essentially carried out using known methods, for example, a method by Kohler and Milstein et al. (Methods Enzymol. (1981) 73: 3-46).

More specifically, cell fusion can be carried out, for example, in a conventional culture medium in the presence of a cell fusion-promoting agent. The fusion-promoting agents include, for example, polyethylene glycol (PEG) and Sendai virus (HVJ). If required, an auxiliary substance such as dimethyl sulfoxide is also added to improve fusion efficiency.

The ratio of immune cells to myeloma cells may be determined at one's own discretion, preferably, for example, one myeloma cell for every one to ten immunocytes. Culture media to be used for cell fusions include, for example, media that are suitable for the growth of myeloma cell lines, such as RPMI1640 medium and MEM medium, and other conventional culture medium used for this type of cell culture. In addition, serum supplements such as fetal calf serum (FCS) may be preferably added to the culture medium.

For cell fusion, predetermined amounts of the above immune cells and myeloma cells are mixed well in the above culture medium. Then, a PEG solution (for example, the average molecular weight is about 1,000 to 6,000) prewarmed to about 37°C is added thereto at a concentration of generally 30% to 60% (w/v). This is gently mixed to produce desired fusion cells (hybridomas). Then, an appropriate culture medium mentioned above is gradually added to the cells, and this is repeatedly centrifuged to remove the supernatant. Thus, cell fusion agents and such which are unfavorable to hybridoma growth can be removed.

The hybridomas thus obtained can be selected by culture using a conventional selective medium, for example, HAT medium (a culture medium containing hypoxanthine, aminopterin, and thymidine). Cells other than the desired hybridomas (non-fused cells) can be killed by continuing culture in the above HAT medium for a sufficient period of time. Typically, the period is several days to several weeks. Then, hybridomas producing the desired antibody are screened and singly cloned by conventional limiting dilution methods.

The hybridomas thus obtained can be selected using a selection medium based on the selection marker possessed by the myeloma used for cell fusion. For example, HGPRT- or TK-deficient cells can be selected by culture using the HAT medium (a culture medium containing hypoxanthine, aminopterin, and thymidine). Specifically, when HAT-sensitive myeloma cells are used for cell fusion, cells successfully fused with normal cells can selectively proliferate in the HAT medium. Cells other than the desired hybridomas (non-fused cells) can be killed by continuing culture in the above HAT medium for a sufficient period of time. Specifically, desired hybridomas can be selected by culture for generally several days to several weeks. Then, hybridomas producing the desired antibody are screened and singly cloned by conventional limiting dilution methods.

Desired antibodies can be preferably selected and singly cloned by screening methods based on known antigen/antibody reaction. For example, an IL-6R-binding monoclonal antibody can bind to IL-6R expressed on the cell surface. Such a monoclonal antibody can be screened by fluorescence activated cell sorting (FACS). FACS is a system that assesses the binding of an antibody to cell surface by analyzing cells contacted with a fluorescent antibody using laser beam, and measuring the fluorescence emitted from individual cells.

To screen for hybridomas that produce a monoclonal antibody of the present invention by FACS, IL-6R-expressing cells are first prepared. Cells preferably used for screening are mammalian cells in which IL-6R is forcedly expressed. As control, the activity of an antibody to bind to cell-surface IL-6R can be selectively detected using non-transformed mammalian cells as host cells. Specifically, hybridomas producing an anti-IL-6R monoclonal antibody can be isolated by selecting hybridomas that produce an antibody which binds to cells forced to express IL-6R, but not to host cells.

Alternatively, the activity of an antibody to bind to immobilized IL-6R-expressing cells can be assessed based on the principle of ELISA. For example, IL-6R-expressing cells are immobilized to the wells of an ELISA plate. Culture supernatants of hybridomas are contacted with the immobilized cells in the wells, and antibodies that bind to the immobilized cells are detected. When the monoclonal antibodies are derived from mouse, antibodies bound to the cells can be detected using an anti-mouse immunoglobulin antibody. Hybridomas producing a desired antibody having the antigen-binding ability are selected by the above screening, and they can be cloned by a limiting dilution method or the like.

Monoclonal antibody-producing hybridomas thus prepared can be passaged in a conventional culture medium, and stored in liquid nitrogen for a long period.

The above hybridomas are cultured by a conventional method, and desired monoclonal antibodies can be prepared from the culture supernatants. Alternatively, the hybridomas are administered to and grown in compatible mammals, and monoclonal antibodies are prepared from the ascites. The former method is suitable for preparing antibodies with high purity.

Antibodies encoded by antibody genes that are cloned from antibody-producing cells such as the above hybridomas can also be preferably used. A cloned antibody gene is inserted into an appropriate vector, and this is introduced into a host to express the antibody encoded by the gene. Methods for isolating antibody genes, inserting the genes into vectors, and transforming host cells have already been established, for example, by Vandamme et al. (Eur. J. Biochem. (1990) 192(3), 767-775). Methods for producing recombinant antibodies are also known as described below.

For example, a cDNA encoding the variable region (V region) of an anti-IL-6R antibody is prepared from hybridoma cells expressing the anti-IL-6R antibody. For this purpose, total RNA is first extracted from hybridomas. Methods used for extracting mRNAs from cells include, for example:

- the guanidine ultracentrifugation method (Biochemistry (1979) 18(24), 5294-5299), and
- the AGPC method (Anal. Biochem. (1987) 162(1), 156-159)

Extracted mRNAs can be purified using the mRNA Purification Kit (GE Healthcare Bioscience) or such. Alternatively, kits for extracting total mRNA directly from cells, such as the QuickPrep mRNA Purification Kit (GE Healthcare Bioscience), are also commercially available. mRNAs can be prepared from hybridomas using such kits. cDNAs encoding the antibody V region can be synthesized from the prepared mRNAs using a reverse transcriptase. cDNAs can be synthesized using the AMV Reverse Transcriptase First-strand cDNA Synthesis Kit (Seikagaku Co.) or such. Furthermore, the SMART RACE cDNA amplification kit (Clontech) and the PCR-based 5'-RACE method (Proc. Natl. Acad. Sci. USA (1988) 85(23), 8998-9002; Nucleic Acids Res. (1989) 17(8), 2919-2932) can be appropriately used to synthesize and amplify cDNAs. In such a cDNA synthesis process, appropriate restriction enzyme sites described below may be introduced into both ends of a cDNA.

The cDNA fragment of interest is purified from the resulting PCR product, and then this is ligated to a vector DNA. A recombinant vector is thus constructed, and introduced into E. *coli* or such. After colony selection, the desired recombinant vector can be prepared from the colony-forming *E. coli.* Then, whether the recombinant vector has the cDNA nucleotide sequence of interest is tested by a known method such as the dideoxy nucleotide chain termination method.

The 5'-RACE method which uses primers to amplify the variable region gene is conveniently used for isolating the gene encoding the variable region. First, a 5'-RACE cDNA library is constructed by cDNA synthesis using RNAs extracted from hybridoma cells as a template. A commercially available kit such as the SMART RACE cDNA amplification kit is appropriately used to synthesize the 5'-RACE cDNA library.

The antibody gene is amplified by PCR using the prepared 5'-RACE cDNA library as a template. Primers for amplifying the mouse antibody gene can be designed based on known antibody gene sequences. The nucleotide sequences of the primers vary depending on the immunoglobulin subclass. Therefore, it is preferable that the subclass is determined in advance using a commercially available kit such as the Iso Strip mouse monoclonal antibody isotyping kit (Roche Diagnostics).

Specifically, for example, primers that allow amplification of genes encoding γ1, γ2a, γ2b, and γ3 heavy chains and κ and λ light chains are used to isolate mouse IgG-encoding genes. In general, a primer that anneals to a constant region site close to the variable region is used as a 3'-side primer to amplify an IgG variable region gene. Meanwhile, a primer attached to a 5' RACE cDNA library construction kit is used as a 5'-side primer.

PCR products thus amplified are used to reshape immunoglobulins composed of a combination of heavy and light chains. A desired antibody can be selected using the IL-6R-binding activity of a reshaped immunoglobulin as an indicator. For example, when the objective is to isolate an antibody against IL-6R, it is more preferred that the binding of the antibody to IL-6R is specific. An IL-6R-binding antibody can be screened, for example, by the following steps:

(1) contacting an IL-6R-expressing cell with an antibody comprising the V region encoded by a cDNA isolated from a hybridoma;
(2) detecting the binding of the antibody to the IL-6R-expressing cell; and
(3) selecting an antibody that binds to the IL-6R-expressing cell.

Methods for detecting the binding of an antibody to IL-6R-expressing cells are known. Specifically, the binding of an antibody to IL-6R-expressing cells can be detected by the above-described techniques such as FACS. Immobilized samples of IL-6R-expressing cells are appropriately used to assess the binding activity of an antibody.

Preferred antibody screening methods that use the binding activity as an indicator also include panning methods using phage vectors. Screening methods using phage vectors are advantageous when the antibody genes are isolated from heavy-chain and light-chain subclass libraries from a polyclonal antibody-expressing cell population. Genes encoding the heavy-chain and light-chain variable regions can be linked by an appropriate linker sequence to form a single-chain Fv (scFv). Phages presenting scFv on their surface can be produced by inserting a gene encoding scFv into a phage vector. The phages are contacted with an antigen of interest. Then, a DNA encoding scFv having the binding activity of interest can be isolated by collecting phages bound to the antigen. This process can be repeated as necessary to enrich scFv having the binding activity of interest.

After isolation of the cDNA encoding the V region of the anti-IL-6R antibody of interest, the cDNA is digested with restriction enzymes that recognize the restriction sites introduced into both ends of the cDNA. Preferred restriction enzymes recognize and cleave a nucleotide sequence that occurs in the nucleotide sequence of the antibody gene at a low frequency. Furthermore, a restriction site for an enzyme that produces a sticky end is preferably introduced into a vector to insert a single-copy digested fragment in the correct orientation. The cDNA encoding the V region of the anti-IL-6R antibody is digested as described above, and this is inserted into an appropriate expression vector to construct an antibody expression vector. In this case, if a gene encoding the antibody constant region (C region) and a gene encoding the above V region are fused in-frame, a chimeric antibody is obtained. Herein, "chimeric antibody" means that the origin of the constant region is different from that of the variable region. Thus, in addition to mouse/human heterochimeric antibodies, human/human allochimeric antibodies are included in the chimeric antibodies of the present invention. A chimeric antibody expression vector can be constructed by inserting the above V region gene into an expression vector that already has the constant region. Specifically, for example, a recognition sequence for a restriction enzyme that excises the above V region gene can be appropriately placed on the 5' side of an expression vector carrying a DNA encoding a desired antibody constant region (C region). A chimeric antibody expression vector is constructed by fusing in frame the two genes digested with the same combination of restriction enzymes.

To produce an anti-IL-6R monoclonal antibody, antibody genes are inserted into an expression vector so that the genes are expressed under the control of an expression regulatory region. The expression regulatory region for antibody expression includes, for example, enhancers and promoters. Furthermore, an appropriate signal sequence may be attached to the amino terminus so that the expressed antibody is secreted to the outside of cells. In the Examples described later, a peptide having the amino acid sequence MGWSCIILFLVATATGVHS (SEQ ID NO: 3) are used as a signal sequence. Meanwhile, other appropriate signal sequences may be attached. The expressed polypeptide is cleaved at the carboxyl terminus of the above sequence, and the resulting polypeptide is secreted to the outside of cells as a mature polypeptide. Then, appropriate host cells are transformed with the expression vector, and recombinant cells expressing the anti-IL-6R antibody-encoding DNA are obtained.

DNAs encoding the antibody heavy chain (H chain) and light chain (L chain) are separately inserted into different expression vectors to express the antibody gene. An antibody molecule having the H and L chains can be expressed by co-transfecting the same host cell with vectors into which the H-chain and L-chain genes are respectively inserted. Alternatively, host cells can be transformed with a single expression vector into which DNAs encoding the H and L chains are inserted (see WO 1994/011523).

There are various known host cell/expression vector combinations for antibody preparation by introducing isolated antibody genes into appropriate hosts. All of these expression systems are applicable to isolation of the antigen-binding domains of the present invention. Appropriate eukaryotic cells used as host cells include animal cells, plant cells, and fungal cells. Specifically, the animal cells include, for example, the following cells.

(1) mammalian cells: CHO, COS, myeloma, baby hamster kidney (BHK), HeLa, Vero, human embryonic kidney (HEK) 293, Freestyle™293, or such;
(2) amphibian cells: Xenopus oocytes, or such; and
(3) insect cells: sf9, sf21, Tn5, or such.

In addition, as a plant cell, an antibody gene expression system using cells derived from the *Nicotiana* genus such as *Nicotiana tabacum* is known. Callus cultured cells can be appropriately used to transform plant cells.

Furthermore, the following cells can be used as fungal cells:

- yeasts: the *Saccharomyces* genus such as *Saccharomyces serevisiae,* and the *Pichia* genus such as *Pichia pastoris;* and
- filamentous fungi: the *Aspergillus* genus such as *Aspergillus niger.*

Furthermore, antibody gene expression systems that utilize prokaryotic cells are also known. For example, when using bacterial cells, *E. coli* cells, *Bacillus subtilis* cells, and such can suitably be utilized in the present invention. Expression vectors carrying the antibody genes of interest are introduced into these cells by transfection. The transfected cells are cultured *in vitro,* and the desired antibody can be prepared from the culture of transformed cells.

In addition to the above-described host cells, transgenic animals can also be used to produce a recombinant antibody. That is, the antibody can be obtained from an animal into which the gene encoding the antibody of interest is introduced. For example, the antibody gene can be constructed as a fusion gene by inserting in frame into a gene that encodes a protein produced specifically in milk. Goat β-casein or such can be used, for example, as the protein secreted in milk. DNA fragments containing the fused gene inserted with the antibody gene is injected into a goat embryo, and then this embryo is introduced into a female goat. Desired antibodies can be obtained as a protein fused with the milk protein from milk produced by the transgenic goat born from the embryo-recipient goat (or progeny thereof). In addition, to increase the volume of milk containing the desired antibody produced by the transgenic goat, hormones can be administered to the transgenic goat as necessary (Ebert, K. M. et al., Bio/Technology (1994) 12 (7), 699-702).

When an antigen-binding molecule described herein is administered to human, an antigen-binding domain derived from a genetically recombinant antibody that has been artificially modified to reduce the heterologous antigenicity against human and such, can be appropriately used as the antigen-binding domain of the molecule. Such genetically recombinant antibodies include, for example, humanized antibodies. These modified antibodies are appropriately produced by known methods.

An antibody variable region used to produce the antigen-binding domain of an antigen-binding molecule described herein is generally formed by three complementarity-determining regions (CDRs) that are separated by four framework regions (FRs). CDR is a region that substantially determines the binding specificity of an antibody. The amino acid sequences of CDRs are highly diverse. On the other hand, the FR-forming amino acid sequences often have high identity even among antibodies with different binding specificities. Therefore, generally, the binding specificity of a certain antibody can be introduced to another antibody by CDR grafting.

A humanized antibody is also called a reshaped human antibody. Specifically, humanized antibodies prepared by grafting the CDR of a non-human animal antibody such as a mouse antibody to a human antibody and such are known. Common genetic engineering techniques for obtaining humanized antibodies are also known. Specifically, for example, overlap extension PCR is known as a method for grafting a mouse antibody CDR to a human FR. In overlap extension PCR, a nucleotide sequence encoding a mouse antibody CDR to be grafted is added to primers for synthesizing a human antibody FR. Primers are prepared for each of the four FRs. It is generally considered that when grafting a mouse CDR to a human FR, selecting a human FR that has high identity to a mouse FR is advantageous for maintaining the CDR function. That is, it is generally preferable to use a human FR comprising an amino acid sequence which has high identity to the amino acid sequence of the FR adjacent to the mouse CDR to be grafted.

Nucleotide sequences to be ligated are designed so that they will be connected to each other in frame. Human FRs are individually synthesized using the respective primers. As a result, products in which the mouse CDR-encoding DNA is attached to the individual FR-encoding DNAs are obtained. Nucleotide sequences encoding the mouse CDR of each product are designed so that they overlap with each other. Then, complementary strand synthesis reaction is conducted to anneal the overlapping CDR of the products synthesized using a human antibody gene as template. Human FRs are ligated *via* the mouse CDR sequences by this reaction.

The full length V region gene, in which three CDRs and four FRs are ultimately ligated, is amplified using primers that anneal to its 5'- or 3'-end, which are added with suitable restriction enzyme recognition sequences. An expression vector for humanized antibody can be produced by inserting the DNA obtained as described above and a DNA that encodes a human antibody C region into an expression vector so that they will ligate in frame. After the recombinant vector is transfected into a host to establish recombinant cells, the recombinant cells are cultured, and the DNA encoding the humanized antibody is expressed to produce the humanized antibody in the cell culture (see, European Patent Publication No. EP 239400 and International Patent Publication No. WO 1996/002576).

By qualitatively or quantitatively measuring and evaluating the antigen-binding activity of the humanized antibody produced as described above, one can suitably select human antibody FRs that allow CDRs to form a favorable antigen-binding site when ligated through the CDRs. Amino acid residues in FRs may be substituted as necessary, so that the CDRs of a reshaped human antibody form an appropriate antigen-binding site. For example, amino acid sequence mutations can be introduced into FRs by applying the PCR method used for grafting a mouse CDR into a human FR. More specifically, partial nucleotide sequence mutations can be introduced into primers that anneal to the FR. Nucleotide sequence mutations are introduced into the FRs synthesized by using such primers. Mutant FR sequences having the desired characteristics can be selected by measuring and evaluating the activity of the amino acid-substituted mutant antibody to bind to the antigen by the above-mentioned method (Cancer Res. (1993) 53: 851-856).

Alternatively, desired human antibodies can be obtained by immunizing transgenic animals having the entire repertoire of human antibody genes (see WO 1993/012227; WO 1992/003918; WO 1994/002602; WO 1994/025585; WO 1996/034096; WO 1996/033735) by DNA immunization.

Furthermore, techniques for preparing human antibodies by panning using human antibody libraries are also known. For example, the V region of a human antibody is expressed as a single-chain antibody (scFv) on phage surface by the phage display method. Phages expressing an scFv that binds to the antigen can be selected. The DNA sequence encoding the human antibody V region that binds to the antigen can be determined by analyzing the genes of selected phages. The DNA sequence of the scFv that binds to the antigen is determined. An expression vector is prepared by fusing the V region sequence in frame with the C region sequence of a desired human antibody, and inserting this into an appropriate expression vector. The expression vector is introduced into cells appropriate for expression such as those described above. The human antibody can be produced by expressing the human antibody-encoding gene in the cells. These methods are already known (see WO 1992/001047; WO 1992/020791; WO 1993/006213; WO 1993/011236; WO 1993/019172; WO 1995/001438; WO 1995/015388).

In addition to the techniques described above, techniques of B cell cloning (identification of each antibody-encoding sequence, cloning and its isolation; use in constructing expression vector in order to prepare each antibody (IgG1, IgG2, IgG3, or IgG4 in particular); and such) such as described in Bernasconi et al. (Science (2002) 298: 2199-2202) or in WO 2008/081008 can be appropriately used to isolate antibody genes.

EU numbering system and Kabat's numbering system

According to the methods used in the present invention, amino acid positions assigned to antibody CDR and FR are specified according to Kabat's numbering (Sequences of Proteins of Immunological Interest (National Institute of Health, Bethesda, Md., 1987 and 1991)). Herein, when an antigen-binding molecule is an antibody or antigen-binding fragment, variable region amino acids are indicated according to Kabat's numbering system, while constant region amino acids are indicated according to EU numbering system based on Kabat's amino acid positions.

Conditions of ion concentration

Conditions of metal ion concentration

In one embodiment of the present invention, the ion concentration refers to a metal ion concentration. "Metal ions" refer to ions of group I elements except hydrogen such as alkaline metals and copper group elements, group II elements such as alkaline earth metals and zinc group elements, group III elements except boron, group IV elements except carbon and silicon, group VIII elements such as iron group and platinum group elements, elements belonging to subgroup A of groups V, VI, and VII, and metal elements such as antimony, bismuth, and polonium. Metal atoms have the property of releasing valence electrons to become cations. This is referred to as ionization tendency. Metals with strong ionization tendency are deemed to be chemically active.

In the present invention, preferred metal ions include, for example, calcium ion. Calcium ion is involved in modulation of many biological phenomena, including contraction of muscles such as skeletal, smooth, and cardiac muscles; activation of movement, phagocytosis, and the like of leukocytes; activation of shape change, secretion, and the like of platelets; activation of lymphocytes; activation of mast cells including secretion of histamine; cell responses mediated by catecholamine α receptor or acetylcholine receptor; exocytosis; release of transmitter substances from neuron terminals; and axoplasmic flow in neurons. Known intracellular calcium ion receptors include troponin C, calmodulin, parvalbumin, and myosin light chain, which have several calcium ion-binding sites and are believed to be derived from a common origin in terms of molecular evolution. There are also many known calcium-binding motifs. Such well-known motifs include, for example, cadherin domains, EF-hand of calmodulin, C2 domain of Protein kinase C, Gla domain of blood coagulation protein Factor IX, C-type lectins of acyaroglycoprotein receptor and mannose-binding receptor, A domains of LDL receptors, annexin, thrombospondin type 3 domain, and EGF-like domains.

In the present invention, when the metal ion is calcium ion, the conditions of calcium ion concentration include low calcium ion concentrations and high calcium ion concentrations. "The binding activity varies depending on calcium ion concentrations" means that the antigen-binding activity of an antigen-binding molecule varies due to the difference in the conditions between low and high calcium ion concentrations. For example, the antigen-binding activity of an antigen-binding molecule may be higher at a high calcium ion concentration than at a low calcium ion concentration. Alternatively, the antigen-binding activity of an antigen-binding molecule may be higher at a low calcium ion concentration than at a high calcium ion concentration.

Herein, the high calcium ion concentration is not particularly limited to a specific value; however, the concentration may preferably be selected between 100 μM and 10 mM. In another embodiment, the concentration may be selected between 200 μM and 5 mM. In an alternative embodiment, the concentration may be selected between 500 μM and 2.5 mM. In still another embodiment, the concentration may be selected between 200 μM and 2 mM. Furthermore, the concentration may be selected between 400 μM and 1.5 mM. In particular, a concentration selected between 500 μM and 2.5 mM, which is close to the plasma (blood) concentration of calcium ion *in vivo,* is preferred.

Herein, the low calcium ion concentration is not particularly limited to a specific value; however, the concentration may preferably be selected between 0.1 μM and 30 μM. In another embodiment, the concentration may be selected between 0.2 μM and 20 μM. In still another embodiment, the concentration may be selected between 0.5 μM and 10 μM. In an alternative embodiment, the concentration may be selected between 1 μM and 5 μM. Furthermore, the concentration may be selected between 2 μM and 4 μM. In particular, a concentration selected between 1 μM and 5 μM, which is close to the concentration of ionized calcium in early endosomes *in vivo,* is preferred.

Herein, "the antigen-binding activity is lower at a low calcium ion concentration than at a high calcium ion concentration" means that the antigen-binding activity of an antigen-binding molecule is weaker at a calcium ion concentration selected between 0.1 μM and 30 μM than at a calcium ion concentration selected between 100 μM and 10 mM. Preferably, it means that the antigen-binding activity of an antigen-binding molecule is weaker at a calcium ion concentration selected between 0.5 μM and 10 μM than at a calcium ion concentration selected between 200 μM and 5 mM. It particularly preferably means that the antigen-binding activity at the calcium ion concentration in the early endosome *in vivo* is weaker than that at the *in vivo* plasma calcium ion concentration; and specifically, it means that the antigen-binding activity of an antigen-binding molecule is weaker at a calcium ion concentration selected between 1 μM and 5 μM than at a calcium ion concentration selected between 500 μM and 2.5 mM.

Whether the antigen-binding activity of an antigen-binding molecule is changed depending on metal ion concentrations can be determined, for example, by the use of known measurement methods such as those described in the section "Binding Activity" above. For example, in order to confirm that the antigen-binding activity of an antigen-binding molecule becomes higher at a high calcium ion concentration than at a low calcium ion concentration, the antigen-binding activity of the antigen-binding molecule at low and high calcium ion concentrations is compared.

In the present invention, the expression "the antigen-binding activity is lower at a low calcium ion concentration than at a high calcium ion concentration" can also be expressed as "the antigen-binding activity of an antigen-binding molecule is higher at a high calcium ion concentration than at a low calcium ion concentration". In the present invention, "the antigen-binding activity is lower at a low calcium ion concentration than at a high calcium ion concentration" is sometimes written as "the antigen-binding ability is weaker at a low calcium ion concentration than at a high calcium ion concentration". Also, "the antigen-binding activity at a low calcium ion concentration is reduced to be lower than that at a high calcium ion concentration" may be written as "the antigen-binding ability at a low calcium ion concentration is made weaker than that at a high calcium ion concentration".

When determining the antigen-binding activity, the conditions other than calcium ion concentration can be appropriately selected by those skilled in the art, and are not particularly limited. For example, the activity can be determined at 37°C in HEPES buffer. For example, Biacore (GE Healthcare) or such can be used for the determination. When the antigen is a soluble antigen, the antigen-binding activity of an antigen-binding molecule can be assessed by flowing the antigen as an analyte over a chip onto which the antigen-binding molecule is immobilized. When the antigen is a membrane antigen, the binding activity of an antigen-binding molecule to the membrane antigen can be assessed by flowing the antigen-binding molecule as an analyte over a chip onto which the antigen is immobilized.

As long as the antigen-binding activity of an antigen-binding molecule of the present invention at a low calcium ion concentration is weaker than that at a high calcium ion concentration, the ratio of the antigen-binding activity between that at a low calcium ion concentration and at a high calcium ion concentration is not particularly limited; and the value of KD (Ca 3μM) / KD (Ca 2 mM), which is the ratio of the dissociation constant (KD) for an antigen at a low calcium ion concentration to the KD at a high calcium ion concentration, is preferably 2 or more; more preferably the value of KD (Ca 3μM) / KD (Ca 2 mM) is 10 or more; and still more preferably the value of KD (Ca 3μM) / KD (Ca 2 mM) is 40 or more. The upper limit of KD (Ca 3μM) / KD (Ca 2 mM) value is not particularly limited, and may be any value such as 400, 1000, or 10000, as long as the molecule can be produced by the techniques of those skilled in the art. Alternatively, the value of KD (Ca 3μM) / KD (Ca 1.2 mM) is specified. That is, the value of KD (Ca 3μM) / KD (Ca 1.2 mM) is 2 or more; more preferably the value of KD (Ca 3μM) / KD (Ca 1.2 mM) is 10 or more; and still more preferably the value of KD (Ca 3μM) / KD (Ca 1.2 mM) is 40 or more. The upper limit of KD (Ca 3μM) / KD (Ca 1.2 mM) value is not particularly limited, and may be any value such as 400, 1000, or 10000, as long as the molecule can be produced by the techniques of those skilled in the art.

When the antigen is a soluble antigen, KD (dissociation constant) can be used to represent the antigen-binding activity. Meanwhile, when the antigen is a membrane antigen, apparent KD (apparent dissociation constant) can be used to represent the activity. KD (dissociation constant) and apparent KD (apparent dissociation constant) can be determined by methods known to those skilled in the art, for example, using Biacore (GE healthcare), Scatchard plot, or flow cytometer.

Alternatively, for example, the dissociation rate constant (kd) can also be preferably used as an index to represent the ratio of the antigen-binding activity of an antigen-binding molecule of the present invention between low and high calcium concentrations. When the dissociation rate constant (kd) is used instead of the dissociation constant (KD) as an index to represent the binding activity ratio, the ratio of the dissociation rate constant (kd) between low and high calcium concentrations, i.e. the value of kd (low calcium concentration)/kd (high calcium concentration), is preferably 2 or more, more preferably 5 or more, still more preferably 10 or more, and yet more preferably 30 or more. The upper limit of the Kd (low calcium concentration)/kd (high calcium concentration) value is not particularly limited, and can be any value such as 50, 100, or 200 as long as the molecule can be produced by techniques known to those skilled in the art.

When the antigen is a soluble antigen, kd (dissociation rate constant) can be used to represent the antigen-binding activity. Meanwhile, when the antigen is a membrane antigen, apparent kd (apparent dissociation rate constant) can be used to represent the antigen-binding activity. The kd (dissociation rate constant) and apparent kd (apparent dissociation rate constant) can be determined by methods known to those skilled in the art, for example, using Biacore (GE healthcare) or flow cytometer. In the present invention, when the antigen-binding activity of an antigen-binding molecule is determined at different calcium ion concentrations, it is preferable to use the same conditions except for the calcium concentrations.

For example, an antigen-binding domain or antibody whose antigen-binding activity is lower at a low calcium ion concentration than at a high calcium ion concentration, which is one embodiment of the present invention, can be obtained via screening of antigen-binding domains or antibodies including the steps of:

(a) determining the antigen-binding activity of an antigen-binding domain or antibody at a low calcium concentration;
(b) determining the antigen-binding activity of an antigen-binding domain or antibody at a high calcium concentration; and
(c) selecting an antigen-binding domain or antibody whose antigen-binding activity is lower at a low calcium concentration than at a high calcium concentration.

Moreover, an antigen-binding domain or antibody whose antigen-binding activity is lower at a low calcium ion concentration than at a high calcium ion concentration, which is one embodiment of the present invention, can be obtained via screening of antigen-binding domains or antibodies, or a library thereof, including the steps of:

(a) contacting an antigen with an antigen-binding domain or antibody, or a library thereof at a high calcium concentration;
(b) incubating at a low calcium concentration an antigen-binding domain or antibody that has bound to the antigen in step (a); and
(c) isolating an antigen-binding domain or antibody dissociated in step (b).

Furthermore, an antigen-binding domain or antibody whose antigen-binding activity is lower at a low calcium ion concentration than at a high calcium ion concentration, which is one embodiment of the present invention, can be obtained via screening of antigen-binding domains or antibodies, or a library thereof, including the steps of:

(a) contacting an antigen with a library of antigen-binding domains or antibodies at a low calcium concentration;
(b) selecting an antigen-binding domain or antibody which does not bind to the antigen in step (a);

(c) allowing the antigen-binding domain or antibody selected in step (b) to bind to the antigen at a high calcium concentration ; and
(d) isolating an antigen-binding domain or antibody that has bound to the antigen in step (c).

In addition, an antigen-binding domain or antibody whose antigen-binding activity is lower at a low calcium ion concentration than at a high calcium ion concentration, which is one embodiment of the present invention, can be obtained by a screening method comprising the steps of:

(a) contacting at a high calcium concentration a library of antigen-binding domains or antibodies with a column onto which an antigen is immobilized;
(b) eluting an antigen-binding domain or antibody that has bound to the column in step (a) from the column at a low calcium concentration; and
(c) isolating the antigen-binding domain or antibody eluted in step (b).

Furthermore, an antigen-binding domain or antibody whose antigen-binding activity is lower at a low calcium ion concentration than at a high calcium ion concentration, which is one embodiment of the present invention, can be obtained by a screening method comprising the steps of:

(a) allowing at a low calcium concentration a library of antigen-binding domains or antibodies to pass through a column onto which an antigen is immobilized;
(b) collecting an antigen-binding domain or antibody that has been eluted without binding to the column in step (a);
(c) allowing the antigen-binding domain or antibody collected in step (b) to bind to the antigen at a high calcium concentration; and
(d) isolating an antigen-binding domain or antibody that has bound to the antigen in step (c).

Moreover, an antigen-binding domain or antibody whose antigen-binding activity is lower at a low calcium ion concentration than at a high calcium ion concentration, which is one embodiment of the present invention, can be obtained by a screening method comprising the steps of:

(a) contacting an antigen with a library of antigen-binding domains or antibodies at a high calcium concentration;
(b) obtaining an antigen-binding domain or antibody that has bound to the antigen in step (a);
(c) incubating at a low calcium concentration the antigen-binding domain or antibody obtained in step (b); and
(d) isolating an antigen-binding domain or antibody whose antigen-binding activity in step (c) is weaker than the criterion for the selection of step (b).

The above-described steps may be repeated twice or more times. Thus, the present invention provides antigen-binding domains or antibodies whose antigen-binding activity is lower at a low calcium ion concentration than at a high calcium ion concentration, which are obtained by screening methods that further comprises the step of repeating twice or more times steps (a) to (c) or (a) to (d) in the above-described screening methods. The number of cycles of steps (a) to (c) or (a) to (d) is not particularly limited, but generally is 10 or less.

In the screening methods of the present invention, the antigen-binding activity of an antigen-binding domain or antibody at a low calcium concentration is not particularly limited as long as it is antigen-binding activity at an ionized calcium concentration of between 0.1 μM and 30 μM, but preferably is antigen-binding activity at an ionized calcium concentration of between 0.5 μM and 10 μM. More preferably, it is antigen-binding activity at the ionized calcium concentration in the early endosome *in vivo,* specifically, between 1 μM and 5 μM. Meanwhile, the antigen-binding activity of an antigen-binding domain or antibody at a high calcium concentration is not particularly limited, as long as it is antigen-binding activity at an ionized calcium concentration of between 100 μM and 10 mM, but preferably is antigen-binding activity at an ionized calcium concentration of between 200 μM and 5 mM. More preferably, it is antigen-binding activity at the ionized calcium concentration in plasma *in vivo,* specifically, between 0.5 mM and 2.5 mM.

The antigen-binding activity of an antigen-binding domain or antibody can be measured by methods known to those skilled in the art. Conditions other than the ionized calcium concentration can be determined by those skilled in the art. The antigen-binding activity of an antigen-binding domain or antibody can be evaluated as a dissociation constant (KD), apparent dissociation constant (apparent KD), dissociation rate constant (kd), apparent dissociation constant (apparent kd), and such. These can be determined by methods known to those skilled in the art, for example, using Biacore (GE healthcare), Scatchard plot, or FACS.

In the present invention, the step of selecting an antigen-binding domain or antibody whose antigen-binding activity is higher at a high calcium concentration than at a low calcium concentration is synonymous with the step of selecting an antigen-binding domain or antibody whose antigen-binding activity is lower at a low calcium concentration than at a high calcium concentration.

As long as the antigen-binding activity is higher at a high calcium concentration than at a low calcium concentration, the difference in the antigen-binding activity between high and low calcium concentrations is not particularly limited; however, the antigen-binding activity at a high calcium concentration is preferably twice or more, more preferably 10 times or more, and still more preferably 40 times or more than that at a low calcium concentration.

Antigen-binding domains or antibodies of the present invention to be screened by the screening methods described above may be any antigen-binding domains and antibodies. For example, it is possible to screen the above-described antigen-binding domains or antibodies. For example, antigen-binding domains or antibodies having natural sequences or substituted amino acid sequences may be screened.

Libraries

In an embodiment, an antigen-binding domain or antibody of the present invention can be obtained from a library that is mainly composed of a plurality of antigen-binding molecules whose sequences are different from one another and whose antigen-binding domains have at least one amino acid residue that alters the antigen-binding activity of the antigen-binding molecules depending on ion concentrations. The ion concentrations preferably include, for example, metal ion concentration and hydrogen ion concentration.

Herein, a "library" refers to a plurality of antigen-binding molecules or a plurality of fusion polypeptides containing antigen-binding molecules, or nucleic acids or polynucleotides encoding their sequences. The sequences of a plurality of antigen-binding molecules or a plurality of fusion polypeptides containing antigen-binding molecules in a library are not identical, but are different from one another.

Herein, the phrase "sequences are different from one another" in the expression "a plurality of antigen-binding molecules whose sequences are different from one another" means that the sequences of antigen-binding molecules in a library are different from one another. Specifically, in a library, the number of sequences different from one another reflects the number of independent clones with different sequences, and may also be referred to as "library size". The library size of a conventional phage display library ranges from $10^6$ to $10^{12}$. The library size can be increased up to $10^{14}$ by the use of known techniques such as ribosome display. However, the actual number of phage particles used in panning selection of a phage library is in general 10-10000 times greater than the library size. This excess multiplicity is also referred to as "the number of library equivalents", and means that there are 10 to 10,000 individual clones that have the same amino acid sequence. Thus, in the present invention, the phrase "sequences are different from one another" means that the sequences of independent antigen-binding molecules in a library, excluding library equivalents, are different from one another. More specifically, the above means that there are $10^6$ to $10^{14}$ antigen-binding molecules whose sequences are different from one another, preferably $10^7$ to $10^{12}$ molecules, more preferably $10^8$ to $10^{11}$ molecules, and particularly preferably $10^8$ to $10^{12}$ molecules whose sequences are different from one another.

Herein, the phrase "a plurality of" in the expression "a library mainly composed of a plurality of antigen-binding molecules" generally refers to, in the case of, for example, antigen-binding molecules, fusion polypeptides, polynucleotide molecules, vectors, or viruses of the present invention, a group of two or more types of the substance. For example, when two or more substances are different from one another in a particular characteristic, this means that there are two or more types of the substance. Such examples may include, for example, mutant amino acids observed at specific amino acid positions in an amino acid sequence. For example, when there are two or more antigen-binding molecules of the present invention whose sequences are substantially the same or preferably the same except for flexible residues or except for particular mutant amino acids at hypervariable positions exposed on the surface, there are a plurality of antigen-binding molecules of the present invention. In another example, when there are two or more polynucleotide molecules whose sequences are substantially the same or preferably the same except for nucleotides encoding flexible residues or nucleotides encoding mutant amino acids of hypervariable positions exposed on the surface, there are a plurality of polynucleotide molecules of the present invention.

In addition, herein, the phrase "mainly composed of" in the expression "a library mainly composed of a plurality of antigen-binding molecules" reflects the number of antigen-binding molecules whose antigen-binding activity varies depending on ion concentrations, among independent clones with different sequences in a library. Specifically, it is preferable that there are at least $10^4$ antigen-binding molecules having such binding activity in a library. More preferably, antigen-binding domains of the present invention can be obtained from a library containing at least $10^5$ antigen-binding molecules having such binding activity. Still more preferably, antigen-binding domains of the present invention can be obtained from a library containing at least $10^6$ antigen-binding molecules having such binding activity. Particularly preferably, antigen-binding domains of the present invention can be obtained from a library containing at least $10^7$ antigen-binding molecules having such binding activity. Yet more preferably, antigen-binding domains of the present invention can be obtained from a library containing at least $10^8$ antigen-binding molecules having such binding activity. Alternatively, this may also be preferably expressed as the ratio of the number of antigen-binding molecules whose antigen-binding activity varies depending on ion concentrations with respect to the number of independent clones having different sequences in a library. Specifically, antigen-binding domains of the present invention can be obtained from a library in which antigen-binding molecules having such binding activity account for 0.1% to 80%, preferably 0.5% to 60%, more preferably 1% to 40%, still more preferably 2% to 20%, and particularly preferably 4% to 10% of independent clones with different sequences in the library. In the case of fusion polypeptides, polynucleotide molecules, or vectors, similar expressions may be possible using the number of molecules or the ratio to the total number of molecules. In the case of viruses, similar expressions may also be possible using the number of virions or the ratio to total number of virions.

Amino acids that alter the antigen-binding activity of antigen-binding domains depending on calcium ion concentrations

Antigen-binding domains or antibodies of the present invention to be screened by the above-described screening methods may be prepared in any manner. For example, when the metal ion is calcium ion, it is possible to use preexisting antibodies, preexisting libraries (phage library, etc.), antibodies or libraries prepared from hybridomas obtained by immunizing animals or from B cells of immunized animals, antibodies or libraries obtained by introducing amino acids capable of chelating calcium (for example, aspartic acid and glutamic acid) or unnatural amino acid mutations into the above-described antibodies or libraries (calcium-cheletable amino acids (such as aspartic acid and glutamic acid), libraries with increased content of unnatural amino acids, libraries prepared by introducing calcium-chelatable amino acids (such as aspartic acid and glutamic acid) or unnatural amino acid mutations at particular positions, or the like.

Examples of the amino acids that alter the antigen-binding activity of antigen-binding molecules depending on ion concentrations as described above may be any types of amino acids as long as the amino acids form a calcium-binding motif. Calcium-binding motifs are well known to those skilled in the art and have been described in details (for example, Springer et al. (Cell (2000) 102, 275-277); Kawasaki and Kretsinger (Protein Prof. (1995) 2, 305-490); Moncrief et al. (J. Mol. Evol. (1990) 30, 522-562); Chauvaux et al. (Biochem. J. (1990) 265, 261-265); Bairoch and Cox (FEBS Lett. (1990) 269, 454-456); Davis (New Biol. (1990) 2, 410-419); Schaefer et al. (Genomics (1995) 25, 638-643); Economou et al. (EMBO J. (1990) 9, 349-354); Wurzburg et al. (Structure. (2006) 14, 6, 1049-1058)). Specifically, any known calcium-binding motifs, including type C lectins such as ASGPR, CD23, MBR, and DC-SIGN, can be included in antigen-binding molecules of the present invention. Preferred examples of such preferred calcium-binding motifs also include, in addition to those described above, for example, the calcium-binding motif in the antigen-binding domain of SEQ ID NO: 62.

Furthermore, as amino acids that alter the antigen-binding activity of antigen-binding molecules depending on calcium ion concentrations, for example, amino acids having metal-chelating activity may also be preferably used. Examples of such metal-chelating amino acids include, for example, serine (Ser(S)), threonine (Thr(T)), asparagine (Asn(N)), glutamine (Gln(Q)), aspartic acid (Asp(D)), and glutamic acid (Glu(E)).

Positions in the antigen-binding domains at which the above-described amino acids are contained are not particularly limited to particular positions, and may be any positions within the heavy chain variable region or light chain variable region that forms an antigen-binding domain, as long as they alter the antigen-binding activity of antigen-binding molecules depending on calcium ion concentrations. Specifically, antigen-binding domains of the present invention can be obtained from a library mainly composed of antigen-binding molecules whose sequences are different from one another and whose heavy chain antigen-binding domains contain amino acids that alter the antigen-binding activity of the antigen-binding molecules depending on calcium ion concentrations. In another non-limiting embodiment, antigen-binding domains of the present invention can be obtained from a library mainly composed of antigen-binding molecules whose sequences are different from one another and whose heavy chain CDR3 domains contain the above-mentioned amino acids. In still another non-limiting embodiment, antigen-binding domains of the present invention can be obtained from a library mainly composed of antigen-binding molecules whose sequences are different from one another and whose heavy chain CDR3 domains contain the above-mentioned amino acids at positions 95, 96, 100a, and/or 101 as indicated according to the Kabat numbering system.

Meanwhile, in a non-limiting embodiment of the present invention, antigen-binding domains of the present invention can be obtained from a library mainly composed of antigen-binding molecules whose sequences are different from one another and whose light chain antigen-binding domains contain amino acids that alter the antigen-binding activity of antigen-binding molecules depending on calcium ion concentrations. In another embodiment, antigen-binding domains of the present invention can be obtained from a library mainly composed of antigen-binding molecules whose sequences are different from one another and whose light chain CDR1 domains contain the above-mentioned amino acids. In still another embodiment, antigen-binding domains of the present invention can be obtained from a library mainly composed of antigen-binding molecules whose sequences are different from one another and whose light chain CDR1 domains contain the above-mentioned amino acids at positions 30, 31, and/or 32 as indicated according to the Kabat numbering system.

In another non-limiting embodiment, antigen-binding domains of the present invention can be obtained from a library mainly composed of antigen-binding molecules whose sequences are different from one another and whose light chain CDR2 domains contain the above-mentioned amino acid residues. In yet another embodiment, the present invention provides libraries mainly composed of antigen-binding molecules whose sequences are different from one another and whose light chain CDR2 domains contain the above-mentioned amino acid residues at position 50 as indicated according to the Kabat numbering system.

In still another non-limiting embodiment of the present invention, antigen-binding domains of the present invention can be obtained from a library mainly composed of antigen-binding molecules whose sequences are different from one another and whose light chain CDR3 domains contain the above-mentioned amino acid residues. In an alternative embodiment, antigen-binding domains of the present invention can be obtained from a library mainly composed of antigen-binding molecules whose sequences are different from one another and whose light chain CDR3 domains contain the above-mentioned amino acid residues at position 92 as indicated according to the Kabat numbering system.

Furthermore, in a different embodiment of the present invention, antigen-binding domains of the present invention can be obtained from a library mainly composed of antigen-binding molecules whose sequences are different from one another and in which two or three CDRs selected from the above-described light chain CDR1, CDR2, and CDR3 contain the aforementioned amino acid residues. Moreover, antigen-binding domains of the present invention can be obtained from a library mainly composed of antigen-binding molecules whose sequences are different from one another and whose light chains contain the aforementioned amino acid residues at any one or more of positions 30, 31, 32, 50, and/or 92 as indicated according to the Kabat numbering system.

In a particularly preferred embodiment, the framework sequences of the light chain and/or heavy chain variable region of an antigen-binding molecule preferably contain human germ line framework sequences. Thus, in an embodiment of the present invention, when the framework sequences are completely human sequences, it is expected that when such an antigen-binding molecule of the present invention is administered to humans (for example, to treat diseases), it induces little or no immunogenic response. In the above sense, the phrase "containing a germ line sequence" in the present invention means that a part of the framework sequences of the present invention is identical to a part of any human germ line framework sequences. For example, when the heavy chain FR2 sequence of an antigen-binding molecule of the present invention is a combination of heavy chain FR2 sequences of different human germ line framework sequences, such a molecule is also an antigen-binding molecule of the present invention "containing a germ line sequence".

Preferred examples of the frameworks include, for example, fully human framework region sequences currently known, which are included in the website of V-Base (http://vbase.mrc-cpe.cam.ac.uk/) or others. Those framework region sequences can be appropriately used as a germ line sequence contained in an antigen-binding molecule of the present invention. The germ line sequences may be categorized according to their similarity (Tomlinson et al. (J. Mol. Biol. (1992) 227, 776-798); Williams and Winter (Eur. J. Immunol. (1993) 23, 1456-1461); Cox et al. (Nat. Genetics (1994) 7, 162-168)). Appropriate germ line sequences can be selected from Vκ, which is grouped into seven subgroups; Vλ, which is grouped into ten subgroups; and VH, which is grouped into seven subgroups.

Fully human VH sequences preferably include, but are not limited to, for example, VH sequences of:

subgroup VH1 (for example, VH1-2, VH1-3, VH1-8, VH1-18, VH1-24, VH1-45, VH1-46, VH1-58, and VH1-69);
subgroup VH2 (for example, VH2-5, VH2-26, and VH2-70);
subgroup VH3 (VH3-7, VH3-9, VH3-11, VH3-13, VH3-15, VH3-16, VH3-20, VH3-21, VH3-23, VH3-30, VH3-33, VH3-35, VH3-38, VH3-43, VH3-48, VH3-49, VH3-53, VH3-64, VH3-66, VH3-72, VH3-73, and VH3-74);
subgroup VH4 (VH4-4, VH4-28, VH4-31, VH4-34, VH4-39, VH4-59, and VH4-61);
subgroup VH5 (VH5-51);
subgroup VH6 (VH6-1); and
subgroup VH7 (VH7-4 and VH7-81).

These are also described in known documents (Matsuda et al. (J. Exp. Med. (1998) 188, 1973-1975)) and such, and thus persons skilled in the art can appropriately design antigen-binding molecules of the present invention based on the information of these sequences. It is also preferable to use other fully human frameworks or framework sub-regions.

Fully human VK sequences preferably include, but are not limited to, for example:

A20, A30, L1, L4, L5, L8, L9, L11, L12, L14, L15, L18, L19, L22, L23, L24, O2, O4, O8, 012, 014, and 018, grouped into subgroup Vk1;
A1, A2, A3, A5, A7, A17, A18, A19, A23, O1, and O11, grouped into subgroup Vk2;
A11, A27, L2, L6, L10, L16, L20, and L25, grouped into subgroup Vk3;
B3, grouped into subgroup Vk4;
B2 (herein also referred to as Vk5-2), grouped into subgroup Vk5; and
A10, A14, and A26, grouped into subgroup VK6
(Kawasaki et al. (Eur. J. Immunol. (2001) 31, 1017-1028); Schable and Zachau (Biol. Chem. Hoppe Seyler (1993) 374, 1001-1022); Brensing-Kuppers et al. (Gene (1997) 191, 173-181)).

Fully human VL sequences preferably include, but are not limited to, for example:

V1-2, V1-3, V1-4, V1-5, V1-7, V1-9, V1-11, V1-13, V1-16, V1-17, V1-18, V1-19, V1-20, and V1-22, grouped into subgroup VL1;
V2-1, V2-6, V2-7, V2-8, V2-11, V2-13, V2-14, V2-15, V2-17, and V2-19, grouped into subgroup VL1;
V3-2, V3-3, and V3-4, grouped into subgroup VL3;
V4-1, V4-2, V4-3, V4-4, and V4-6, grouped into subgroup VL4; and
V5-1, V5-2, V5-4, and V5-6, grouped into subgroup VL5 (Kawasaki et al. (Genome Res. (1997) 7, 250-261)).

Normally, these framework sequences are different from one another at one or more amino acid residues. These framework sequences can be used in combination with "at least one amino acid residue that alters the antigen-binding activity of an antigen-binding molecule depending on ion concentrations" of the present invention. Other examples of the fully human frameworks used in combination with "at least one amino acid residue that alters the antigen-binding activity of an antigen-binding molecule depending on ion concentrations" of the present invention include, but are not limited to, for example, KOL, NEWM, REI, EU, TUR, TEI, LAY, and POM (for example, Kabat *et al.* (1991) *supra;* Wu et al. (J. Exp. Med. (1970) 132, 211-250)).

Without being bound by a particular theory, one reason for the expectation that the use of germ line sequences precludes adverse immune responses in most individuals is believed to be as follows. As a result of the process of affinity maturation during normal immune responses, somatic mutation occurs frequently in the variable regions of immunoglobulin. Such mutations mostly occur around CDRs whose sequences are hypervariable, but also affect residues of framework regions. Such framework mutations do not exist on the germ line genes, but they are less likely to be immunogenic in patients. This is because the normal human population is exposed to most of the framework sequences expressed from the germ line genes, and as a result of immunotolerance, these germ line frameworks are expected to have low or no immunogenicity in patients. To maximize the possibility of immunotolerance, variable region-encoding genes may be selected from a group of commonly occurring functional germ line genes.

Known methods such as site-directed mutagenesis (Kunkel et al. (Proc. Natl. Acad. Sci. USA (1985) 82, 488-492)) and overlap extension PCR can be appropriately employed to produce antigen-binding molecules of the present invention in which the above-described framework sequences contain amino acids that alter the antigen-binding activity of the antigen-binding molecules depending on calcium ion concentrations.

For example, a library which contains a plurality of antigen-binding molecules of the present invention whose sequences are different from one another can be constructed by combining heavy chain variable regions prepared as a randomized variable region sequence library with a light chain variable region selected as a framework sequence originally containing at least one amino acid residue that alters the antigen-binding activity of the antigen-binding molecule depending on calcium ion concentrations. As a non-limiting example, when the ion concentration is calcium ion concentration, such preferred libraries include, for example, those constructed by combining the light chain variable region sequence belonging to the Vk5-2 family represented by the light chain variable region sequence of SEQ ID NO: 62 (Vk5-2) and the heavy chain variable region produced as a randomized variable region sequence library.

Alternatively, a light chain variable region sequence selected as a framework region originally containing at least one amino acid residue that alters the antigen-binding activity of an antigen-binding molecule as mentioned above can be design to contain various amino acid residues other than the above amino acid residues. Herein, such residues are referred to as flexible residues. The number and position of flexible residues are not particularly limited as long as the antigen-binding activity of the antigen-binding molecule of the present invention varies depending on ion concentrations. Specifically, the CDR sequences and/or FR sequences of the heavy chain and/or light chain may contain one or more flexible residues. For example, when the ion concentration is calcium ion concentration, non-limiting examples of flexible residues to be introduced into the light chain variable region sequence of SEQ ID NO: 62 (Vk5-2) include the amino acid residues listed in Tables 1 or 2.

[Table 1]

| CDR | Kabat NUMBERING | 70 % OF AMINO ACID OF THE TOTAL | | | | |
|---|---|---|---|---|---|---|
| CDR1 | 28 | S : 100% | | | | |
| | 29 | I : 100% | | | | |
| | 30 | E : 72% | N : 14% | S : 14% | | |
| | 31 | D : 100% | | | | |
| | 32 | D : 100% | | | | |
| | 33 | L : 100% | | | | |
| | 34 | A : 70% | N : 30% | | | |
| CDR2 | 50 | E : 100% | | | | |
| | 51 | A : 100% | | | | |
| | 52 | S : 100% | | | | |
| | 53 | H : 5% | N : 25% | S : 45% | T : 25% | |
| | 54 | L : 100% | | | | |
| | 55 | Q : 100% | | | | |
| | 56 | S : 100% | | | | |
| CDR3 | 90 | Q : 100% | | | | |
| | 91 | H : 25% | S : 15% | R : 15% | Y : 45% | |
| | 92 | D : 80% | N : 10% | S : 10% | | |
| | 93 | D : 5% | G : 10% | N : 25% | S : 50% | R : 10% |
| | 94 | S : 50% | Y : 50% | | | |
| | 95 | P : 100% | | | | |
| | 96 | L : 50% | Y : 50% | | | |

[Table 2]

| CDR | Kabat NUMBERING | 30 % OF AMINO ACID OF THE TOTAL | | | | |
|---|---|---|---|---|---|---|
| CDR1 | 28 | S : 100% | | | | |
| | 29 | I : 100% | | | | |
| | 30 | E : 83% | S : 17% | | | |
| | 31 | D : 100% | | | | |
| | 32 | D : 100% | | | | |
| | 33 | L : 100% | | | | |
| | 34 | A : 70% | N : 30% | | | |
| CDR2 | 50 | H : 100% | | | | |
| | 51 | A : 100% | | | | |
| | 52 | S : 100% | | | | |
| | 53 | H : 5% | N : 25% | S : 45% | T : 25% | |
| | 54 | L : 100% | | | | |
| | 55 | Q : 100% | | | | |
| | 56 | S : 100% | | | | |

(continued)

| CDR | Kabat NUMBERING | 30 % OF AMINO ACID OF THE TOTAL | | | | |
|---|---|---|---|---|---|---|
| CDR3 | 90 | Q : 100% | | | | |
| | 91 | H : 25% | S : 15% | R : 15% | Y : 45% | |
| | 92 | D : 80% | N : 10% | S : 10% | | |
| | 93 | D : 5% | G : 10% | N : 25% | S : 50% | R : 10% |
| | 94 | S : 50% | Y : 50% | | | |
| | 95 | P : 100% | | | | |
| | 96 | L : 50% | Y : 50% | | | |

Herein, flexible residues refer to amino acid residue variations present at hypervariable positions at which several different amino acids are present on the light chain and heavy chain variable regions when the amino acid sequences of known and/or native antibodies or antigen-binding domains are compared. Hypervariable positions are generally located in the CDR. In an embodiment, the data provided by Kabat, Sequences of Proteins of Immunological Interest (National Institute of Health Bethesda Md.) (1987 and 1991) is useful to determine hypervariable positions in known and/or native antibodies. Furthermore, databases on the Internet (http://vbase.mrc-cpe.cam.ac.uk/, http://www.bioinf.org.uk/abs/index.html) provide the collected sequences of many human light chains and heavy chains and their locations. The information on the sequences and locations is useful to determine hypervariable positions in the present invention. According to the present invention, when a certain amino acid position has preferably about 2 to about 20 possible amino acid residue variations, preferably about 3 to about 19, preferably about 4 to about 18, preferably 5 to 17, preferably 6 to 16, preferably 7 to 15, preferably 8 to 14, preferably 9 to 13, and preferably 10 to 12 possible amino acid residue variations, the position is hypervariable. In some embodiments, a certain amino acid position may have preferably at least about 2, preferably at least about 4, preferably at least about 6, preferably at least about 8, preferably about 10, and preferably about 12 amino acid residue variations.

Alternatively, a library containing a plurality of antigen-binding molecules of the present invention whose sequences are different from one another can be constructed by combining heavy chain variable regions produced as a randomized variable region sequence library with light chain variable regions into which at least one amino acid residue that alters the antigen-binding activity of antigen-binding molecules depending on ion concentrations as mentioned above is introduced. When the ion concentration is calcium ion concentration, non-limiting examples of such libraries preferably include, for example, libraries in which heavy chain variable regions produced as a randomized variable region sequence library are combined with light chain variable region sequences in which a particular residue(s) in a germ line sequence such as SEQ ID NO: 5 (Vk1), SEQ ID NO: 6 (Vk2), SEQ ID NO: 7 (Vk3), or SEQ ID NO: 8 (Vk4) has been substituted with at least one amino acid residue that alters the antigen-binding activity of an antigen-binding molecule depending on calcium ion concentrations. Non-limiting examples of such amino acid residues include amino acid residues in light chain CDR1. Furthermore, non-limiting examples of such amino acid residues include amino acid residues in light chain CDR2. In addition, non-limiting, other examples of such amino acid residues also include amino acid residues in light chain CDR3.

Non-limiting examples of such amino acid residues contained in light chain CDR1 include those at positions 30, 31, and/or 32 in the CDR1 of light chain variable region as indicated by Kabat numbering. Furthermore, non-limiting examples of such amino acid residues contained in light chain CDR2 include an amino acid residue at position 50 in the CDR2 of light chain variable region as indicated by Kabat numbering. Moreover, non-limiting examples of such amino acid residues contained in light chain CDR3 include an amino acid residue at position 92 in the CDR3 of light chain variable region as indicated by Kabat numbering. These amino acid residues can be contained alone or in combination as long as they form a calcium-binding motif and/or as long as the antigen-binding activity of an antigen-binding molecule varies depending on calcium ion concentrations. Meanwhile, as troponin C, calmodulin, parvalbumin, and myosin light chain, which have several calcium ion-binding sites and are believed to be derived from a common origin in terms of molecular evolution, are known, the light chain CDR1, CDR2, and/or CDR3 can be designed to have their binding motifs. For example, it is possible to use cadherin domains, EF hand of calmodulin, C2 domain of Protein kinase C, Gla domain of blood coagulation protein FactorIX, C type lectins of acyaroglycoprotein receptor and mannose-binding receptor, A domains of LDL receptors, annexin, thrombospondin type 3 domain, and EGF-like domains in an appropriate manner for the above purposes.

When heavy chain variable regions produced as a randomized variable region sequence library and light chain variable regions into which at least one amino acid residue that alters the antigen-binding activity of an antigen-binding molecule depending on ion concentrations has been introduced are combined as described above, the sequences of the light chain variable regions can be designed to contain flexible residues in the same manner as described above. The number and position of such flexible residues are not particularly limited to particular embodiments as long as the antigen-binding activity of antigen-binding molecules of the present invention varies depending on ion concentrations. Specifically, the CDR sequences and/or FR sequences of heavy chain and/or light chain can contain one or more flexible residues. When the ion concentration is calcium ion concentration, non-limiting examples of flexible residues to be introduced into the sequence of light chain variable region include the amino acid residues listed in Tables 1 and 2.

The preferred heavy chain variable regions to be combined include, for example, randomized variable region libraries. Known methods are combined as appropriate to produce a randomized variable region library . In a non-limiting embodiment of the present invention, an immune library constructed based on antibody genes derived from lymphocytes of animals immunized with a specific antigen, patients with infections, persons with an elevated antibody titer in blood as a result of vaccination, cancer patients, or auto immune disease patients, may be preferably used as a randomized variable region library.

In another non-limiting embodiment of the present invention, a synthetic library produced by replacing the CDR sequences of V genes in genomic DNA or functional reshaped V genes with a set of synthetic oligonucleotides containing sequences encoding codon sets of an appropriate length can also be preferably used as a randomized variable region library. In this case, since sequence diversity is observed in the heavy chain CDR3 sequence, it is also possible to replace the CDR3 sequence only. A criterion of giving rise to diversity in amino acids in the variable region of an antigen-binding molecule is that diversity is given to amino acid residues at surface-exposed positions in the antigen-binding molecule. The surface-exposed position refers to a position that is considered to be able to be exposed on the surface and/or contacted with an antigen, based on structure, ensemble of structures, and/or modeled structure of an antigen-binding molecule. In general, such positions are CDRs. Preferably, surface-exposed positions are determined using coordinates from a three-dimensional model of an antigen-binding molecule using a computer program such as the InsightII program (Accelrys). Surface-exposed positions can be determined using algorithms known in the art (for example, Lee and Richards (J. Mol. Biol. (1971) 55, 379-400); Connolly (J. Appl. Cryst. (1983) 16, 548-558)). Determination of surface-exposed positions can be performed using software suitable for protein modeling and three-dimensional structural information obtained from an antibody. Software that can be used for these purposes preferably includes SYBYL Biopolymer Module software (Tripos Associates). Generally or preferably, when an algorithm requires a user input size parameter, the "size" of a probe which is used in the calculation is set at about 1.4 Angstrom or smaller in radius. Furthermore, methods for determining surface-exposed regions and areas using software for personal computers are described by Pacios (Comput. Chem. (1994) 18 (4), 377-386; J. Mol. Model. (1995) 1, 46-53).

In another non-limiting embodiment of the present invention, a naive library, which is constructed from antibody genes derived from lymphocytes of healthy persons and whose repertoire consists of naive sequences, which are antibody sequences with no bias, can also be particularly preferably used as a randomized variable region library (Gejima et al. (Human Antibodies (2002) 11, 121-129); Cardoso et al. (Scand. J. Immunol. (2000) 51, 337-344)). Herein, an amino acid sequence comprising a naive sequence refers to an amino acid sequence obtained from such a naive library.

In one embodiment of the present invention, an antigen-binding domain of the present invention can be obtained from a library containing a plurality of antigen-binding molecules of the present invention whose sequences are different from one another, prepared by combining light chain variable regions constructed as a randomized variable region sequence library with a heavy chain variable region selected as a framework sequence that originally contains "at least one amino acid residue that alters the antigen-binding activity of an antigen-binding molecule depending on ion concentrations". When the ion concentration is calcium ion concentration, non-limiting examples of such libraries preferably include those constructed by combining light chain variable regions constructed as a randomized variable region sequence library with the sequence of heavy chain variable region of SEQ ID NO: 9 (6RL#9-IgG1) or SEQ ID NO: 10 (6KC4-1#85-IgG1). Alternatively, such a library can be constructed by selecting appropriate light chain variable regions from those having germ line sequences, instead of light chain variable regions constructed as a randomized variable region sequence library. Such preferred libraries include, for example, those in which the sequence of heavy chain variable region of SEQ ID NO: 9 (6RL#9-IgG1) or SEQ ID NO: 10 (6KC4-1#85-IgG1) is combined with light chain variable regions having germ line sequences.

Alternatively, the sequence of an heavy chain variable region selected as a framework sequence that originally contains "at least one amino acid residue that alters the antigen-binding activity of an antigen-binding molecule" as mentioned above can be designed to contain flexible residues. The number and position of the flexible residues are not particularly limited as long as the antigen-binding activity of an antigen-binding molecule of the present invention varies depending on ion concentrations. Specifically, the CDR and/or FR sequences of heavy chain and/or light chain can contain one or more flexible residues. When the ion concentration is calcium ion concentration, non-limiting examples of flexible residues to be introduced into the sequence of heavy chain variable region of SEQ ID NO: 9 (6RL#9-IgG1) include all amino acid residues of heavy chain CDR1 and CDR2 and the amino acid residues of the heavy chain CDR3 except those at positions 95, 96, and/or 100a. Alternatively, non-limiting examples of flexible residues to be introduced into the sequence of heavy chain variable region of SEQ ID NO: 10 (6KC4-1#85-IgG1) include all amino acid residues of heavy chain CDR1 and CDR2 and the amino acid residues of the heavy chain CDR3 except those at amino acid positions 95 and/or 101.

Alternatively, a library containing a plurality of antigen-binding molecules whose sequences are different from one another can be constructed by combining light chain variable regions constructed as a randomized variable region sequence library or light chain variable regions having germ line sequences with heavy chain variable regions into which "at least one amino acid residue responsible for the ion concentration-dependent change in the antigen-binding activity of an antigen-binding molecule" has been introduced as mentioned above. When the ion concentration is calcium ion concentration, non-limiting examples of such libraries preferably include those in which light chain variable regions constructed as a randomized variable region sequence library or light chain variable regions having germ line sequences are combined with the sequence of a heavy chain variable region in which a particular residue(s) has been substituted with at least one amino acid residue that alters the antigen-binding activity of an antigen-binding molecule depending on calcium ion concentrations. Non-limiting examples of such amino acid residues include amino acid residues of the heavy chain CDR1. Further non-limiting examples of such amino acid residues include amino acid residues of the heavy chain CDR2. In addition, non-limiting examples of such amino acid residues also include amino acid residues of the heavy chain CDR3. Non-limiting examples of such amino acid residues of heavy chain CDR3 include the amino acids of positions 95, 96, 100a, and/or 101 in the CDR3 of heavy chain variable region as indicated by the Kabat numbering. Furthermore, these amino acid residues can be contained alone or in combination as long as they form a calcium-binding motif and/or the antigen-binding activity of an antigen-binding molecule varies depending on calcium ion concentrations.

When light chain variable regions constructed as a randomized variable region sequence library or light chain variable regions having germ line sequence are combined with a heavy chain variable region into which at least one amino acid residue that alter the antigen-binding activity of an antigen-binding molecule depending on ion concentrations as mentioned above has been introduced, the sequence of the heavy chain variable region can also be designed to contain flexible residues in the same manner as described above. The number and position of flexible residues are not particularly limited as long as the antigen-binding activity of an antigen-binding molecule of the present invention varies depending on ion concentrations. Specifically, the heavy chain CDR and/or FR sequences may contain one or more flexible residues. Furthermore, randomized variable region libraries can be preferably used as amino acid sequences of CDR1, CDR2, and/or CDR3 of the heavy chain variable region other than the amino acid residues that alter the antigen-binding activity of an antigen-binding molecule. When germ line sequences are used as light chain variable regions, non-limiting examples of such sequences include those of SEQ ID NO: 5 (Vk1), SEQ ID NO: 6 (Vk2), SEQ ID NO: 7 (Vk3), and SEQ ID NO: 8 (Vk4).

Any of the above-described amino acids that alter the antigen-binding activity of an antigen-binding molecule depending on calcium ion concentrations can be preferably used, as long as they form a calcium-binding motif. Specifically, such amino acids include electron-donating amino acids. Preferred examples of such electron-donating amino acids include serine, threonine, asparagine, glutamic acid, aspartic acid, and glutamic acid.

Condition of hydrogen ion concentrations

In an embodiment of the present invention, the condition of ion concentrations refers to the condition of hydrogen ion concentrations or pH condition. In the present invention, the concentration of proton, i.e., the nucleus of hydrogen atom, is treated as synonymous with hydrogen index (pH). When the activity of hydrogen ion in an aqueous solution is represented as aH+, pH is defined as -log10aH+. When the ionic strength of the aqueous solution is low (for example, lower than $10^{-3}$), aH+ is nearly equal to the hydrogen ion strength. For example, the ionic product of water at 25°C and 1 atmosphere is $Kw=aH+aOH=10^{-14}$, and therefore in pure water, $aH+=aOH=10^{-7}$. In this case, pH=7 is neutral; an aqueous solution whose pH is lower than 7 is acidic or whose pH is greater than 7 is alkaline.

In the present invention, when pH condition is used as the ion concentration condition, pH conditions include high hydrogen ion concentrations or low pHs, i.e., an acidic pH range, and low hydrogen ion concentrations or high pHs, i.e., a neutral pH range. "The binding activity varies depending on pH condition" means that the antigen-binding activity of an antigen-binding molecule varies due to the difference in conditions of a high hydrogen ion concentration or low pH (an acidic pH range) and a low hydrogen ion concentration or high pH (a neutral pH range). This includes, for example, the case where the antigen-binding activity of an antigen-binding molecule is higher in a neutral pH range than in an acidic pH range and the case where the antigen-binding activity of an antigen-binding molecule is higher in an acidic pH range than in a neutral pH range.

In the present specification, neutral pH range is not limited to a specific value and is preferably selected from between pH 6.7 and pH 10.0. In another embodiment, the pH can be selected from between pH 6.7 and pH 9.5. In still another embodiment, the pH can be selected from between pH 7.0 and pH 9.0. In yet another embodiment, the pH can be selected from between pH7.0 and pH 8.0. In particular, the preferred pH includes pH 7.4, which is close to the pH of plasma (blood) *in vivo.*

In the present specification, an acidic pH range is not limited to a specific value and is preferably selected from between pH 4.0 and pH 6.5. In another embodiment, the pH can be selected from between pH 4.5 and pH 6.5. In still another embodiment, the pH can be selected from between pH 5.0 and pH 6.5. In yet another embodiment, the pH can be selected from between pH5.5 and pH 6.5. In particular, the preferred pH includes pH 5.8, which is close to the ionized calcium concentration in the early endosome *in vivo.*

In the present invention, "the antigen-binding activity of an antigen-binding molecule at a high hydrogen ion concentration or low pH (an acidic pH range) is lower than that at a low hydrogen ion concentration or high pH (a neutral pH range)" means that the antigen-binding activity of an antigen-binding molecule at a pH selected from between pH 4.0 and pH 6.5 is weaker than that at a pH selected from between pH6.7 and pH 10.0; preferably means that the antigen-binding activity of an antigen-binding molecule at a pH selected from between pH 4.5 and pH 6.5 is weaker than that at a pH selected from between pH 6.7 and pH 9.5; more preferably, means that the antigen-binding activity of an antigen-binding molecule at a pH selected from between pH 5.0 and pH 6.5 is weaker than that at a pH selected from between pH 7.0 and pH 9.0; still more preferably means that the antigen-binding activity of an antigen-binding molecule at a pH selected from between pH5.5 and pH6.5 is weaker than that at a pH selected from between pH 7.0 and pH 8.0; particularly preferably means that the antigen-binding activity at the pH in the early endosome *in vivo* is weaker than the antigen-binding activity at the pH of plasma *in vivo;* and specifically means that the antigen-binding activity of an antigen-binding molecule at pH 5.8 is weaker than the antigen-binding activity at pH 7.4.

Whether the antigen-binding activity of an antigen-binding molecule has changed by the pH condition can be determined, for example, by the use of known measurement methods such as those described in the section "Binding Activity" above. Specifically, the binding activity is measured under different pH conditions using the measurement methods described above. For example, the antigen-binding activity of an antigen-binding molecule is compared under the conditions of acidic pH range and neutral pH range to confirm that the antigen-binding activity of the antigen-binding molecule changes to be higher under the condition of neutral pH range than that under the condition of acidic pH range.

Furthermore, in the present invention, the expression "the antigen-binding activity at a high hydrogen ion concentration or low pH, i.e., in an acidic pH range, is lower than that at a low hydrogen ion concentration or high pH, i.e., in a neutral pH range" can also be expressed as "the antigen-binding activity of an antigen-binding molecule at a low hydrogen ion concentration or high pH, i.e., in a neutral pH range, is higher than that at a high hydrogen ion concentration or low pH, i.e., in an acidic pH range". In the present invention, "the antigen-binding activity at a high hydrogen ion concentration or low pH, i.e., in an acidic pH range, is lower than that at a low hydrogen ion concentration or high pH, i.e., in a neutral pH range" may be described as "the antigen-binding activity at a high hydrogen ion concentration or low pH, i.e., in an acidic pH range, is weaker than the antigen-binding ability at a low hydrogen ion concentration or high pH, i.e., in a neutral pH range". Alternatively, "the antigen-binding activity at a high hydrogen ion concentration or low pH, i.e., in an acidic pH range, is reduced to be lower than that at a low hydrogen ion concentration or high pH, i.e., in a neutral pH range" may be described as "the antigen-binding activity at a high hydrogen ion concentration or low pH, i.e., in an acidic pH range, is reduced to be weaker than the antigen-binding ability at a low hydrogen ion concentration or high pH, i.e., in a neutral pH range".

The conditions other than hydrogen ion concentration or pH for measuring the antigen-binding activity may be suitably selected by those skilled in the art and are not particularly limited. Measurements can be carried out, for example, at 37°C using HEPES buffer. Measurements can be carried out, for example, using Biacore (GE Healthcare). When the antigen is a soluble antigen, the antigen-binding activity of an antigen-binding molecule can be determined by assessing the binding activity to the soluble antigen by pouring the antigen as an analyte into a chip immobilized with the antigen-binding molecule. When the antigen is a membrane antigen, the binding activity to the membrane antigen can be assessed by pouring the antigen-binding molecule as an analyte into a chip immobilized with the antigen.

As long as the antigen-binding activity of an antigen-binding molecule of the present invention at a high hydrogen ion concentration or low pH, i.e., in an acidic pH range is weaker than that at a low hydrogen ion concentration or high pH, i.e., in a neutral pH range, the ratio of the antigen-binding activity between that at a high hydrogen ion concentration or low pH, i.e., an acidic pH range, and at a low hydrogen ion concentration or high pH, i.e., a neutral pH range is not particularly limited, and the value of KD (pH 5.8) / KD (pH 7.4), which is the ratio of the dissociation constant (KD) for an antigen at a high hydrogen ion concentration or low pH, i.e., in an acidic pH range to the KD at a low hydrogen ion concentration or high pH, i.e., in a neutral pH range, is preferably 2 or more; more preferably the value of KD (pH 5.8) / KD (pH 7.4) is 10 or more; and still more preferably the value of KD (pH 5.8) / KD (pH 7.4) is 40 or more. The upper limit of KD (pH 5.8) / KD (pH 7.4) value is not particularly limited, and may be any value such as 400, 1000, or 10000, as long as the molecule can be produced by the techniques of those skilled in the art.

When the antigen is a soluble antigen, the dissociation constant (KD) can be used as the value for antigen-binding activity. Meanwhile, when the antigen is a membrane antigen, the apparent dissociation constant (KD) can be used. The dissociation constant (KD) and apparent dissociation constant (KD) can be measured by methods known to those skilled in the art, and Biacore (GE healthcare), Scatchard plot, flow cytometer, and such can be used.

Alternatively, for example, the dissociation rate constant (kd) can be suitably used as an index for indicating the ratio of the antigen-binding activity of an antigen-binding molecule of the present invention between that at a high hydrogen ion concentration or low pH, i.e., an acidic pH range and a low hydrogen ion concentration or high pH, i.e., a neutral pH range. When kd (dissociation rate constant) is used as an index for indicating the binding activity ratio instead of KD (dissociation constant), the value of kd (in an acidic pH range) / kd (in a neutral pH range), which is the ratio of kd (dissociation rate constant) for the antigen at a high hydrogen ion concentration or low pH, i.e., in an acidic pH range to kd (dissociation rate constant) at a low hydrogen ion concentration or high pH, i.e., in a neutral pH range, is preferably 2 or more, more preferably 5 or more, still more preferably 10 or more, and yet more preferably 30 or more. The upper limit of kd (in an acidic pH range) / kd (in a neutral pH range) value is not particularly limited, and may be any value such as 50, 100, or 200, as long as the molecule can be produced by the techniques of those skilled in the art.

When the antigen is a soluble antigen, the dissociation rate constant (kd) can be used as the value for antigen-binding activity and when the antigen is a membrane antigen, the apparent dissociation rate constant (kd) can be used. The dissociation rate constant (kd) and apparent dissociation rate constant (kd) can be determined by methods known to those skilled in the art, and Biacore (GE healthcare), flow cytometer, and such may be used. In the present invention, when the antigen-binding activity of an antigen-binding molecule is measured at different hydrogen ion concentrations, i.e., pHs, conditions other than the hydrogen ion concentration, i.e., pH, are preferably the same.

For example, an antigen-binding domain or antibody whose antigen-binding activity at a high hydrogen ion concentration or low pH, i.e., in an acidic pH range is lower than that at a low hydrogen ion concentration or high pH, i.e., in a neutral pH range, which is one embodiment provided by the present invention, can be obtained via screening of antigen-binding domains or antibodies, comprising the following steps (a) to (c):

(a) obtaining the antigen-binding activity of an antigen-binding domain or antibody in an acidic pH range;
(b) obtaining the antigen-binding activity of an antigen-binding domain or antibody in a neutral pH range; and
(c) selecting an antigen-binding domain or antibody whose antigen-binding activity in the acidic pH range is lower than that in the neutral pH range.

Alternatively, an antigen-binding domain or antibody whose antigen-binding activity at a high hydrogen ion concentration or low pH, i.e., in an acidic pH range, is lower than that at a low hydrogen ion concentration or high pH, i.e., in a neutral pH range, which is one embodiment provided by the present invention, can be obtained via screening of antigen-binding domains or antibodies, or a library thereof, comprising the following steps (a) to (c):

(a) contacting an antigen-binding domain or antibody, or a library thereof, in a neutral pH range with an antigen;
(b) placing in an acidic pH range the antigen-binding domain or antibody bound to the antigen in step (a); and
(c) isolating the antigen-binding domain or antibody dissociated in step (b).

An antigen-binding domain or antibody whose antigen-binding activity at a high hydrogen ion concentration or low pH, i.e., in an acidic pH range is lower than that at a low hydrogen ion concentration or high pH, i.e., in a neutral pH range, which is another embodiment provided by the present invention, can be obtained *via* screening of antigen-binding domains or antibodies, or a library thereof, comprising the following steps (a) to (d):

(a) contacting in an acidic pH range an antigen with a library of antigen-binding domains or antibodies;
(b) selecting the antigen-binding domain or antibody which does not bind to the antigen in step (a);
(c) allowing the antigen-binding domain or antibody selected in step (b) to bind with the antigen in a neutral pH range; and
(d) isolating the antigen-binding domain or antibody bound to the antigen in step (c).

An antigen-binding domain or antibody whose antigen-binding activity at a high hydrogen ion concentration or low pH, i.e., in an acidic pH range, is lower than that at a low hydrogen ion concentration or high pH, i.e., in a neutral pH range, which is even another embodiment provided by the present invention, can be obtained by a screening method comprising the following steps (a) to (c):

(a) contacting in a neutral pH range a library of antigen-binding domains or antibodies with a column immobilized with an antigen;
(b) eluting in an acidic pH range from the column the antigen-binding domain or antibody bound to the column in step (a); and
(c) isolating the antigen-binding domain or antibody eluted in step (b).

An antigen-binding domain or antibody whose antigen-binding activity at a high hydrogen ion concentration or low pH, i.e., in an acidic pH, range is lower than that at a low hydrogen ion concentration or high pH, i.e., in a neutral pH range, which is still another embodiment provided by the present invention, can be obtained by a screening method comprising the following steps (a) to (d):

(a) allowing, in an acidic pH range, a library of antigen-binding domains or antibodies to pass a column immobilized with an antigen;
(b) collecting the antigen-binding domain or antibody eluted without binding to the column in step (a);
(c) allowing the antigen-binding domain or antibody collected in step (b) to bind with the antigen in a neutral pH range; and
(d) isolating the antigen-binding domain or antibody bound to the antigen in step (c).

An antigen-binding domain or antibody whose antigen-binding activity at a high hydrogen ion concentration or low pH, i.e., in an acidic pH range, is lower than that at a low hydrogen ion concentration or high pH, i.e., in a neutral pH range, which is yet another embodiment provided by the present invention, can be obtained by a screening method comprising the following steps (a) to (d):

(a) contacting an antigen with a library of antigen-binding domains or antibodies in a neutral pH range;
(b) obtaining the antigen-binding domain or antibody bound to the antigen in step (a);
(c) placing in an acidic pH range the antigen-binding domain or antibody obtained in step (b); and
(d) isolating the antigen-binding domain or antibody whose antigen-binding activity in step (c) is weaker than the standard selected in step (b).

The above-described steps may be repeated twice or more times. Thus, the present invention provides antigen-binding domains and antibodies whose antigen-binding activity in an acidic pH range is lower than that in a neutral pH range, which are obtained by a screening method that further comprises the steps of repeating, twice or more times, steps (a) to (c) or (a) to (d) in the above-described screening methods. The number of times that steps (a) to (c) or (a) to (d) is repeated is not particularly limited; however, the number is 10 or less in general.

In the screening methods of the present invention, the antigen-binding activity of an antigen-binding domain or antibody at a high hydrogen ion concentration or low pH, i.e., in an acidic pH range, is not particularly limited, as long as it is the antigen-binding activity at a pH of between 4.0 and 6.5, and includes the antigen-binding activity at a pH of between 4.5 and 6.6 as the preferred pH. The antigen-binding activity also includes that at a pH of between 5.0 and 6.5, and that at a pH of between 5.5 and 6.5 as another preferred pH. The antigen-binding activity also includes that at the pH in the early endosome *in vivo* as the more preferred pH, and specifically, that at pH5.8. Meanwhile, the antigen-binding activity of an antigen-binding domain or antibody at a low hydrogen ion concentration or high pH, i.e., in a neutral pH range, is not particularly limited, as long as it is the antigen-binding activity at a pH of between 6.7 and 10, and includes the antigen-binding activity at a pH of between 6.7 and 9.5 as the preferred pH. The antigen-binding activity also includes that at a pH of between 7.0 and 9.5 and that at a pH of between 7.0 and 8.0 as another preferred pH. The antigen-binding activity also includes that at the pH of plasma *in vivo* as the more preferred pH, and specifically, that at pH 7.4.

The antigen-binding activity of an antigen-binding domain or antibody can be measured by methods known to those skilled in the art. Those skilled in the art can suitably determine conditions other than ionized calcium concentration. The antigen-binding activity of an antigen-binding domain or antibody can be assessed based on the dissociation constant (KD), apparent dissociation constant (KD), dissociation rate constant (kd), apparent dissociation rate constant (kd), and such. These can be determined by methods known to those skilled in the art, for example, using Biacore (GE healthcare), Scatchard plot, or FACS.

Herein, the step of selecting an antigen-binding domain or antibody whose antigen-binding activity at a low hydrogen ion concentration or high pH, i.e., in a neutral pH range, is higher than that at a high hydrogen ion concentration or low pH, i.e., in an acidic pH range, is synonymous with the step of selecting an antigen-binding domain or antibody whose antigen-binding activity at a high hydrogen ion concentration or low pH, i.e., in an acidic pH range, is lower than that at a low hydrogen ion concentration or high pH, i.e., in a neutral pH range.

As long as the antigen-binding activity at a low hydrogen ion concentration or high pH, i.e., in a neutral pH range, is higher than that at a high hydrogen ion concentration or low pH, i.e., in an acidic pH range, the difference between the antigen-binding activity at a low hydrogen ion concentration or high pH, i.e., a neutral pH range, and that at a high hydrogen ion concentration or low pH, i.e., an acidic pH range, is not particularly limited; however, the antigen-binding activity at a low hydrogen ion concentration or high pH, i.e., in a neutral pH range, is preferably twice or more, more preferably 10 times or more, and still more preferably 40 times or more than that at a high hydrogen ion concentration or low pH, i.e., in an acidic pH range.

The antigen binding domain or antibody of the present invention screened by the screening methods described above may be any antigen-binding domain or antibody, and the above-mentioned antigen-binding domain or antibody may be screened. For example, antigen-binding domain or antibody having the native sequence may be screened, and antigen-binding domain or antibody in which their amino acid sequences have been substituted may be screened.

The antigen-binding domain or antibody of the present invention to be screened by the above-described screening methods may be prepared in any manner. For example, conventional antibodies, conventional libraries (phage library, etc.), antibodies or libraries prepared from B cells of immunized animals or from hybridomas obtained by immunizing animals, antibodies or libraries (libraries with increased content of amino acids with a side chain pKa of 4.0-8.0 (for example, histidine and glutamic acid) or unnatural amino acids, libraries introduced with amino acids with a side chain pKa of 4.0-8.0 (for example, histidine and glutamic acid) or unnatural amino acid mutations at specific positions, etc.) obtained by introducing amino acids with a side chain pKa of 4.0-8.0 (for example, histidine and glutamic acid) or unnatural amino acid mutations into the above-described antibodies or libraries may be used.

Methods for obtaining an antigen-binding domain or antibody whose antigen-binding activity at a low hydrogen ion concentration or high pH, i.e., in a neutral pH range, is higher than that at a high hydrogen ion concentration or low pH, i.e., in an acidic pH range, from an antigen-binding domains or antibodies prepared from hybridomas obtained by immunizing animals or from B cells of immunized animals preferably include, for example, the antigen-binding molecule or antibody in which at least one of the amino acids of the antigen-binding domain or antibody is substituted with an amino acid with a side chain pKa of 4.0-8.0 (for example, histidine and glutamic acid) or an unnatural amino acid mutation, or the antigen-binding domain or antibody inserted with an amino acid with a side chain pKa of 4.0-8.0 (for example, histidine and glutamic acid) or unnatural amino acid, such as those described in WO 2009/125825.

The sites of introducing mutations of amino acids with a side chain pKa of 4.0-8.0 (for example, histidine and glutamic acid) or unnatural amino acids are not particularly limited, and may be any position as long as the antigen-binding activity in an acidic pH range becomes weaker than that in a neutral pH range (the value of KD (in an acidic pH range) / KD (in a neutral pH range) or kd (in an acidic pH range) / kd (in a neutral pH range) is increased) as compared to before substitution or insertion. For example, when the antigen-binding molecule is an antibody, antibody variable region and CDRs are suitable. Those skilled in the art can appropriately determine the number of amino acids to be substituted with or the number of amino acids with a side chain pKa of 4.0-8.0 (for example, histidine and glutamic acid) or unnatural amino acids to be inserted. It is possible to substitute with a single amino acid having a side chain pKa of 4.0-8.0 (for example, histidine and glutamic acid) or a single unnatural amino acid; it is possible to insert a single amino acid having a side chain pKa of 4.0-8.0 (for example, histidine and glutamic acid) or a single unnatural amino acid; it is possible to substitute with two or more amino acids having a side chain pKa of 4.0-8.0 (for example, histidine and glutamic acid) or two or more unnatural amino acids; and it is possible to insert two or more amino acids having a side chain pKa of 4.0-8.0 (for example, histidine and glutamic acid) or two or more unnatural amino acids. Alternatively, other amino acids can be deleted, added, inserted, and/or substituted concomitantly, aside from the substitution into amino acids having a side chain pKa of 4.0-8.0 (for example, histidine and glutamic acid) or unnatural amino acids, or the insertion of amino acids having a side chain pKa of 4.0-8.0 (for example, histidine and glutamic acid) or unnatural amino acids. Substitution into or insertion of amino acids with a side chain pKa of 4.0-8.0 (for example, histidine and glutamic acid) or unnatural amino acids can performed randomly by methods such as histidine scanning, in which the alanine of alanine scanning known to those skilled in the art is replaced with histidine. Antigen-binding molecules exhibiting a greater value of KD (in an acidic pH range) / KD (in a neutral pH range) or kd (in an acidic pH range) / kd (in a neutral pH range) as compared to before the mutation can be selected from antigen-binding domains or antibodies introduced with random insertions or substitution mutations of amino acids with a side chain pKa of 4.0-8.0 (for example, histidine and glutamic acid) or unnatural amino acids.

Preferred examples of antigen-binding molecules containing the mutation into amino acids with a side chain pKa of 4.0-8.0 (for example, histidine and glutamic acid) or unnatural amino acids as described above and whose antigen-binding activity in an acidic pH range is lower than that in a neutral pH range include, antigen-binding molecules whose antigen-binding activity in the neutral pH range after the mutation into amino acids with a side chain pKa of 4.0-8.0 (for example, histidine and glutamic acid) or unnatural amino acids is comparable to that before the mutation into amino acids with a side chain pKa of 4.0-8.0 (for example, histidine and glutamic acid) or unnatural amino acids. Herein, "an antigen-binding molecule after the mutation with amino acids having a side chain pKa of 4.0-8.0 (for example, histidine and glutamic acid) or unnatural amino acids has an antigen-binding activity comparable to that before the mutation with amino acids having a side chain pKa of 4.0-8.0 (for example, histidine and glutamic acid) or unnatural amino acids" means that, when taking the antigen-binding activity of an antigen-binding molecule before the mutation with amino acids having a side chain pKa of 4.0-8.0 (for example, histidine and glutamic acid) or unnatural amino acids as 100%, the antigen-binding activity of an antigen-binding molecule after the mutation with amino acids having a side chain pKa of 4.0-8.0 (for example, histidine and glutamic acid) or unnatural amino acids is at least 10% or more, preferably 50% or more, more preferably 80% or more, and still more preferably 90% or more. The antigen-binding activity after the mutation of amino acids with a side chain pKa of 4.0-8.0 (for example, histidine and glutamic acid) or unnatural amino acids at pH 7.4 may be higher than that before the mutation of amino acids with a side chain pKa of 4.0-8.0 (for example, histidine and glutamic acid) or unnatural amino acids at pH 7.4. If the antigen-binding activity of an antigen-binding molecule is decreased due to insertion of or substitution into amino acids with a side chain pKa of 4.0-8.0 (for example, histidine and glutamic acid) or unnatural amino acids, the antigen-binding activity can be made to be comparable to that before the insertion of or substitution into amino acids with a side chain pKa of 4.0-8.0 (for example, histidine and glutamic acid) or unnatural amino acids, by introducing a substitution, deletion, addition, and/or insertion of one or more amino acids of the antigen-binding molecule. The present invention also includes antigen-binding molecules whose binding activity has been adjusted to be comparable by substitution, deletion, addition, and/or insertion of one or more amino acids after substitution or insertion of amino acids with a side chain pKa of 4.0-8.0 (for example, histidine and glutamic acid) or unnatural amino acids.

Meanwhile, when an antigen-binding molecule is a substance containing an antibody constant region, preferred embodiments of antigen-binding molecules whose antigen-binding activity at an acidic pH range is lower than that in a neutral pH range include methods in which the antibody constant regions contained in the antigen-binding molecules have been modified. Specific examples of modified antibody constant regions preferably include the constant regions of SEQ ID NOs: 11, 12, 13, and 14.

Amino acids that alter the antigen-binding activity of antigen-binding domain depending on the hydrogen ion concentration conditions

Antigen-binding domains or antibodies of the present invention to be screened by the above-described screening methods may be prepared in any manner. For example, when ion concentration condition is hydrogen ion concentration condition or pH condition, conventional antibodies, conventional libraries (phage library, etc.), antibodies or libraries prepared from B cells of immunized animals or from hybridomas obtained by immunizing animals, antibodies or libraries (libraries with increased content of amino acids with a side chain pKa of 4.0-8.0 (for example, histidine and glutamic acid) or unnatural amino acids, libraries introduced with mutations of amino acids with a side chain pKa of 4.0-8.0 (for example, histidine and glutamic acid) or unnatural amino acids at specific positions, etc.) obtained by introducing mutations of amino acids with a side chain pKa of 4.0-8.0 (for example, histidine and glutamic acid) or unnatural amino acids into the above-described antibodies or libraries may be used.

In one non-limiting embodiment of the present invention, a library containing multiple antigen-binding molecules of the present invention whose sequences are different from one another can also be constructed by combining heavy chain variable regions, produced as a randomized variable region sequence library, with light chain variable regions introduced with "at least one amino acid residue that changes the antigen-binding activity of an antigen-binding molecule depending on the hydrogen ion concentration condition".

Such amino acid residues include, but are not limited to, for example, amino acid residues contained in the light chain CDR1. The amino acid residues also include, but are not limited to, for example, amino acid residues contained in the light chain CDR2. The amino acid residues also include, but are not limited to, for example, amino acid residues contained in the light chain CDR3.

The above-described amino acid residues contained in the light chain CDR1 include, but are not limited to, for example, amino acid residues of positions 24, 27, 28, 31, 32, and/or 34 according to Kabat numbering in the CDR1 of light chain variable region. Meanwhile, the amino acid residues contained in the light chain CDR2 include, but are not limited to, for example, amino acid residues of positions 50, 51, 52, 53, 54, 55, and/or 56 according to Kabat numbering in the CDR2 of light chain variable region. Furthermore, the amino acid residues in the light chain CDR3 include, but are not limited to, for example, amino acid residues of positions 89, 90, 91, 92, 93, 94, and/or 95A according to Kabat numbering in the CDR3 of light chain variable region. Moreover, the amino acid residues can be contained alone or can be contained in combination of two or more amino acids as long as they allow the change in the antigen-binding activity of an antigen-binding molecule depending on the hydrogen ion concentration.

Even when the heavy chain variable region produced as a randomized variable region sequence library is combined with the above-described light chain variable region introduced with "at least one amino acid residue that changes the antigen-binding activity of an antigen-binding molecule depending on the hydrogen ion concentration condition", it is possible to design so that the flexible residues are contained in the sequence of the light chain variable region in the same manner as described above. The number and position of the flexible residues are not particularly limited to a specific embodiment, as long as the antigen-binding activity of an antigen-binding molecule of the present invention changes depending on the hydrogen ion concentration condition. Specifically, the CDR and/or FR sequences of heavy chain and/or light chain can contain one or more flexible residues. For example, flexible residues to be introduced into the sequences of the light chain variable regions include, but are not limited to, for example, the amino acid residues listed in Tables 3 and 4. Meanwhile, amino acid sequences of light chain variable regions other than the flexible residues and amino acid residues that change the antigen-binding activity of an antigen-binding molecule depending on the hydrogen ion concentration condition suitably include, but are not limited to, germ line sequences such as Vk1 (SEQ ID NO: 5), Vk2 (SEQ ID NO: 6), Vk3 (SEQ ID NO: 7), and Vk4 (SEQ ID NO: 8).

[Table 3]

| POSITION | AMINO ACID | | | | | | | | |
|---|---|---|---|---|---|---|---|---|---|
| CDR1 | | | | | | | | | |
| 28 | S:100% | | | | | | | | |
| 29 | I:100% | | | | | | | | |
| 30 | N:25% | S:25% | R:25% | H:25% | | | | | |
| 31 | S:100% | | | | | | | | |
| 32 | H:100% | | | | | | | | |
| 33 | L:100% | | | | | | | | |
| 34 | A:50% | N:50% | | | | | | | |
| CDR2 | | | | | | | | | |
| 50 | H:100% | | | | **OR** | A:25% | D:25% | G:25% | K:25% |
| 51 | A:100% | | | | | A:100% | | | |
| 52 | S:100% | | | | | S:100% | | | |
| 53 | K:33.3% | N:33.3 % | S:33.3 % | | | H:100% | | | |
| 54 | L:100% | | | | | L:100% | | | |
| 55 | Q:100% | | | | | Q:100% | | | |
| 56 | S:100% | | | | | S:100% | | | |
| CDR3 | | | | | | | | | |
| 90 | Q:100% | | | | **OR** | Q:100% | | | |
| 91 | H:100% | | | | | S:33.3% | R:33.3 % | Y:33.3 % | |
| 92 | G:25% | N:25% | S:25% | Y:25% | | H:100% | | | |
| 93 | H:33.3% | N:33.3 % | S:33.3 % | | | H:33.3 % | N:33.3 % | S:33.3 % | |
| 94 | S:50% | Y:50% | | | | S:50% | Y:50% | | |
| 95 | P:100% | | | | | P:100% | | | |
| 96 | L:50% | Y:50% | | | | L:50% | Y:50% | | |
| (Position indicates Kabat numbering) | | | | | | | | | |

[Table 4]

| CDR | POSITION | AMINO ACID | | | | |
|---|---|---|---|---|---|---|
| CDR1 | 28 | S:100% | | | | |
| | 29 | I:100% | | | | |
| | 30 | H:30% | N: 10% | S:50% | R:10% | |
| | 31 | N:35% | S:65% | | | |
| | 32 | H:40% | N:20% | Y:40% | | |
| | 33 | L:100% | | | | |
| | 34 | A:70% | N:30% | | | |

(continued)

| CDR | POSITION | AMINO ACID | | | | |
|---|---|---|---|---|---|---|
| CDR2 | 50 | A:25% | D:15% | G:25% | H:30% | K:5% |
| | 51 | A:100% | | | | |
| | 52 | S:100% | | | | |
| | 53 | H:30% | K:10% | N: 15% | S : 45% | |
| | 54 | L: 100% | | | | |
| | 55 | Q: 100% | | | | |
| | 56 | S:100% | | | | |
| CDR3 | 90 | Q:100% | | | | |
| | 91 | H:30% | S:15% | R:10% | Y:45% | |
| | 92 | G:20% | H:30% | N:20% | S:15% | Y: 15% |
| | 93 | H:30% | N:25% | S:45% | | |
| | 94 | S:50% | Y:50% | | | |
| | 95 | P:100% | | | | |
| | 96 | L:50% | Y:50% | | | |
| (Position indicates Kabat numbering) | | | | | | |

Any amino acid residue may be suitably used as the above-described amino acid residues that change the antigen-binding activity of an antigen-binding molecule depending on the hydrogen ion concentration condition. Specifically, such amino acid residues include amino acids with a side chain pKa of 4.0-8.0. Such electron-releasing amino acids preferably include, for example, naturally occurring amino acids such as histidine and glutamic acid, as well as unnatural amino acids such as histidine analogs (US 20090035836), m-NO2-Tyr (pKa 7.45), 3,5-Br2-Tyr (pKa 7.21), and 3,5-I2-Tyr (pKa 7.38) (Bioorg. Med. Chem. (2003) 11 (17), 3761-2768). Particularly preferred amino acid residues include, for example, amino acids with a side chain pKa of 6.0-7.0. Such electron-releasing amino acid residues preferably include, for example, histidine.

Known methods such as site-directed mutagenesis (Kunkel et al. (Proc. Natl. Acad. Sci. USA (1985) 82, 488-492)) and Overlap extension PCR can be appropriately employed to modify the amino acids of antigen-binding domains. Furthermore, various known methods can also be used as an amino acid modification method for substituting amino acids by those other than natural amino acids (Annu. Rev. Biophys. Biomol. Struct. (2006) 35, 225-249; Proc. Natl. Acad. Sci. U.S.A. (2003) 100 (11), 6353-6357). For example, a cell-free translation system (Clover Direct (Protein Express)) containing tRNAs in which amber suppressor tRNA, which is complementary to UAG codon (amber codon) that is a stop codon, is linked with an unnatural amino acid may be suitably used.

The preferred heavy chain variable region that is used in combination includes, for example, randomized variable region libraries. Known methods are appropriately combined as a method for producing a randomized variable region library. In a non-limiting embodiment of the present invention, an immune library constructed based on antibody genes derived from animals immunized with specific antigens, patients with infection or persons with an elevated antibody titer in blood as a result of vaccination, cancer patients, or lymphocytes of auto immune diseases may be suitably used as a randomized variable region library.

In another non-limiting embodiment of the present invention, in the same manner as described above, a synthetic library in which the CDR sequences of V genes from genomic DNA or functional reconstructed V genes are replaced with a set of synthetic oligonucleotides containing the sequences encoding codon sets of an appropriate length can also be suitably used as a randomized variable region library. In this case, the CDR3 sequence alone may be replaced because variety in the gene sequence of heavy chain CDR3 is observed. The basis for giving rise to amino acid variations in the variable region of an antigen-binding molecule is to generate variations of amino acid residues of surface-exposed positions of the antigen-binding molecule. The surface-exposed position refers to a position where an amino acid is exposed on the surface and/or contacted with an antigen based on the conformation, structural ensemble, and/or modeled structure of an antigen-binding molecule, and in general, such positions are the CDRs. The surface-exposed positions are preferably determined using the coordinates derived from a three-dimensional model of the antigen-binding molecule using computer programs such as InsightII program (Accelrys). The surface-exposed positions can be determined using algorithms known in the art (for example, Lee and Richards (J. Mol. Biol. (1971) 55, 379-400); Connolly (J. Appl. Cryst. (1983) 16, 548-558)). The surface-exposed positions can be determined based on the information on the three dimensional structure of antibodies using software suitable for protein modeling. Software which is suitably used for this purpose includes the SYBYL biopolymer module software (Tripos Associates). When the algorithm requires the input size parameter from the user, the "size" of probe for use in computation is generally or preferably set at about 1.4 angstrom or less in radius. Furthermore, a method for determining surface-exposed region and area using personal computer software is described by Pacios (Comput. Chem. (1994) 18 (4), 377-386; and J. Mol. Model. (1995) 1, 46-53).

In still another non-limiting embodiment of the present invention, a naive library constructed from antibody genes derived from lymphocytes of healthy persons and consisting of naive sequences, which are unbiased repertoire of antibody sequences, can also be particularly suitably used as a randomized variable region library (Gejima et al. (Human Antibodies (2002) 11, 121-129); and Cardoso et al. (Scand. J. Immunol. (2000) 51, 337-344)).

Neutralizing activity

A non-limiting embodiment of the present invention provides an antigen-binding molecule having human-FcRn-binding activity in an acidic pH range including an antigen-binding domain and an Fcγ receptor-binding domain, and having neutralizing activity against an antigen, wherein the antigen-binding domain has antigen-binding activity that changes depending on the ion-concentration condition, and the Fcγ receptor-binding domain has higher binding activity to the Fcγ receptor in a neutral pH range condition than an Fc region of a native human IgG in which the sugar chain bonded at position 297 (EU numbering) is a fucose-containing sugar chain; and a pharmaceutical composition comprising the antigen-binding molecule. Generally, neutralizing activity refers to activity of inhibiting the biological activity of a ligand, such as viruses and toxins, having biological activity on cells. Thus, substances having neutralizing activity refer to substances that bind to the ligand or the receptor to which the ligand binds, and inhibits the binding between the ligand and the receptor. Receptors blocked from binding with the ligand by the neutralizing activity will not be able to exhibit biological activity through this receptor. When the antigen-binding molecule is an antibody, such an antibody having neutralizing activity is generally called a neutralizing antibody. Neutralizing activity of a test substance may be measured by comparing the biological activity in the presence of a ligand between when the test substance is present and absent.

For example, major possible ligands for the IL-6 receptor preferably include IL-6 as shown in SEQ ID NO: 15. The IL-6 receptor, which is an I-type membrane protein with its amino terminus forming the extracellular domain, forms a hetero-tetramer with a gp130 receptor which has been induced to dimerize by IL-6 (Heinrich et al. (Biochem. J. (1998) 334, 297-314)). Formation of the heterotetramer activates Jak which is associated with the gp130 receptor. Jak undergoes autophosphorylation and phosphorylates the receptor. The phosphorylation site of the receptor and Jak serves as a binding site for SH2-carrying molecules belonging to the Stat family such as Stat3; MAP kinase; PI3/Akt; and other SH2-carrying proteins and adapters. Next, Stat bound to the gp130 receptor is phosphorylated by Jak. The phosphorylated Stat dimerizes and moves into the nucleus, and regulates the transcription of target genes. Jak or Stat can also be involved in signal cascades via receptors of other classes. Deregulated IL-6 signal cascades are observed in inflammation and pathological conditions of autoimmune diseases, and cancers such as prostate cancer and multiple myeloma. Stat3 which may act as an oncogene is constitutively activated in many cancers. In prostate cancer and multiple myeloma, there is a crosstalk between the signaling cascade via the IL-6 receptor and the signaling cascade via the epithelial growth factor receptor (EGFR) family members (Ishikawa et al. (J. Clin. Exp. Hematopathol. (2006) 46 (2), 55-66)).

Such intracellular signaling cascades are different for each cell type; therefore, appropriate target molecules can be determined for each target cell of interest, and are not limited to the above-mentioned factors. Neutralization activity can be evaluated by measuring the activation of *in vivo* signaling. Furthermore, the activation of *in vivo* signaling can be detected by using as an index the action of inducing the transcription of a target gene that exists downstream of the *in vivo* signaling cascade. Change in the transcription activity of the target gene can be detected by the principle of reporter assays. Specifically, a reporter gene such as green fluorescence protein (GFP) or luciferase is placed downstream of a promoter region or a transcription factor of the target gene, its reporter activity is measured, and thereby change in the transcription activity can be measured as the reporter activity. Commercially available kits for measuring the activation of *in vivo* signaling can be used appropriately (for example, Mercury Pathway Profiling Luciferase System (Clontech)).

Furthermore, for methods of measuring the activity of neutralizing receptors/ligands of the EGF receptor family and such, which normally act on signaling cascades that work toward promoting cell proliferation, the neutralization activity of neutralizing antibodies can be evaluated by measuring the proliferation activity of target cells. For example, when cells are promoted to proliferate by growth factors of the EGF family such as HB-EGF, the inhibitory effect on the proliferation of such cells based on the neutralizing activity of an anti-HB-EGF antibody can be suitably evaluated or measured by the following methods: For evaluating or measuring the cell proliferation inhibitory activity *in vitro,* a method of measuring the incorporation of [$^3$H]-labeled thymidine added to the medium by viable cells as an index of DNA replication ability is used. As more convenient methods, a dye exclusion method, in which the ability of a cell to exclude a dye such as trypan blue from the cell is measured under the microscope, and the MTT method, are used. The latter method makes use of the ability of viable cells to convert MTT (3-(4,5-dimethylthiazol-2-yl)-2,5-diphenyl tetrazolium bromide), which is a tetrazolium salt, to a blue formazan product. More specifically, a test antibody is added as well as a ligand to the culture solution of a test cell, and after a certain period of time, the MTT solution is added to the culture solution, and this is left to stand for a while for incorporation of MTT into the cell. As a result, MTT, which is a yellow compound, is converted to a blue compound by the action of succinate dehydrogenase in the mitochondria of the cell. After dissolving this blue product for coloration, its absorbance is measured and used as an index for the number of viable cells. In addition to MTT, reagents such as MTS, XTT, WST-1, and WST-8 are also commercially available (Nacalai Tesque, and such) and can be suitably used. For measuring the activity, a binding antibody which is of the same isotype as the anti-HB-EGF antibody but does not have the cell proliferation inhibitory activity can be used as a control antibody in the same manner as the anti-HB-EGF antibody, and the activity can be determined when the anti-HB-EGF antibody shows stronger cell proliferation inhibitory activity than the control antibody.

Cells that can be preferably used for evaluating the activity include, for example, cells promoted to proliferate by HB-EGF such as the ovarian cancer cell line RMG-1, and mouse Ba/F3 cells which have been transformed by a vector for expressing a gene encoding hEGFR/mG-CSFR, which is a fusion protein in which the extracellular domain of human EGFR is fused in frame with the intracellular domain of the mouse GCSF receptor. In this way, those skilled in the art can appropriately select cells to be used for evaluating the activity and use them to measure the cell proliferation activity as mentioned above.

Since the antigen-binding molecule provided by the present invention can eliminate antigens from plasma, the antigen-binding molecule itself does not necessarily have to have neutralizing activity. However, it is more favorable to block the function of the antigen present in plasma by exerting neutralizing activity against the antigen until the antigen is taken up with the antigen-binding molecule into Fcy-receptor-expressing cells by Fcy-receptor-mediated endocytosis.

Furthermore, since the antigen-binding molecule provided by the present invention can promote intracellular dissociation of an antigen, which has been extracellularly bound to the antigen-binding molecule, from an antigen-binding molecule, the antigen that dissociated from the antigen-binding molecule inside the cell is degraded in the lysosome. Therefore, the antigen-binding molecule itself does not necessarily have to have neutralizing activity. However, it is more favorable to block the function of the antigen present in plasma by exerting neutralizing activity against the antigen until the antigen is taken up with the antigen-binding molecule into Fcy-receptor-expressing cells by Fcy-receptor-mediated endocytosis.

Furthermore, since the antigen-binding molecule provided by the present invention can decrease the total antigen concentration or free antigen concentration in plasma, the antigen-binding molecule itself does not necessarily have to have neutralizing activity. However, it is more favorable to block the function of the antigen present in plasma by exerting neutralizing activity against the antigen until the antigen is taken up with the antigen-binding molecule into Fcy-receptor-expressing cells by Fcy-receptor-mediated endocytosis.

Fcγ receptor

Fcγ receptor (FcγR) refers to a receptor capable of binding to the Fc region of monoclonal IgG1, IgG2, IgG3, or IgG4 antibodies, and includes all members belonging to the family of proteins substantially encoded by an Fcγ receptor gene. In humans, the family includes FcγRI (CD64) including isoforms FcγRIa, FcγRIb and FcγRIc; FcγRII (CD32) including isoforms FcγRIIa (including allotype H131 and R131), FcγRIIb (including FcγRIIb-1 and FcγRIIb-2), and FcγRIIc; and FcγRIII (CD16) including isoform FcγRIIIa (including allotype V158 and F158) and FcγRIIIb (including allotype FcγRIIIb-NA1 and FcγRIIIb-NA2); as well as all unidentified human FcγRs, FcγR isoforms, and allotypes thereof. However, Fcγ receptor is not limited to these examples. Without being limited thereto, FcγR includes those derived from humans, mice, rats, rabbits, and monkeys. FcγR may be derived from any organism. Mouse FcγR includes, without being limited to, FcγRI (CD64), FcγRII (CD32), FcγRIII (CD16), and FcγRIII-2 (FcγRIV, CD16-2), as well as all unidentified mouse FcγRs, FcγR isoforms, and allotypes thereof. Such preferred Fcγ receptors include, for example, human FcγRI (CD64), FcγRIIa (CD32), FcγRIIb (CD32), FcγRIIIa (CD16), and/or FcγRIIIb (CD16). The polynucleotide sequence and amino acid sequence of human FcγRI are shown in SEQ ID NOs: 16 (NM_000566.3) and 17 (NP_000557.1), respectively; the polynucleotide sequence and amino acid sequence of human FcγRIIa (allotype H131) are shown in SEQ ID NOs: 18 (BC020823.1) and 19 (AAH20823.1) (allotype R131 is a sequence in which amino acid at position 166 of SEQ ID NO: 19 is substituted with Arg), respectively; the polynucleotide sequence and amino acid sequence of FcγIIB are shown in SEQ ID NOs: 20 (BC146678.1) and 21 (AAI46679.1), respectively; the polynucleotide sequence and amino acid sequence of FcγRIIIa are shown in SEQ ID NOs: 22 (BC033678.1) and 23 (AAH33678.1), respectively; and the polynucleotide sequence and amino acid sequence of FcγRIIIb are shown in SEQ ID NOs: 24 (BC128562.1) and 25 (AAI28563.1), respectively (RefSeq accession number is shown in each parentheses). Whether an Fcγ receptor has binding activity to the Fc region of a monoclonal IgG1, IgG2, IgG3, or IgG4 antibody can be assessed by ALPHA screen (Amplified Luminescent Proximity Homogeneous Assay), surface plasmon resonance (SPR)-based BIACORE method, and others (Proc. Natl. Acad. Sci. USA (2006) 103(11), 4005-4010), in addition to the above-described FACS and ELISA formats.

Meanwhile, "Fc ligand" or "effector ligand" refers to a molecule and preferably a polypeptide that binds to an antibody Fc region, forming an Fc/Fc ligand complex. The molecule may be derived from any organism. The binding of an Fc ligand to Fc preferably induces one or more effector functions. Such Fc ligands include, but are not limited to, Fc receptors, FcγR, FcαR, FcεR, FcRn, C1q, and C3, mannan-binding lectin, mannose receptor, *Staphylococcus* Protein A, *Staphylococcus* Protein G, and viral FcγRs. The Fc ligands also include Fc receptor homologs (FcRH) (Davis et al., (2002) Immunological Reviews 190, 123-136) or FCRL (Annu Rev Immunol. 2007; 25: 525-60), which are a family of Fc receptors homologous to FcγR. The Fc ligands also include unidentified molecules that bind to Fc.

In FcγRI (CD64) including FcγRIa, FcγRIb, and FcγRIc, and FcγRIII (CD16) including isoforms FcγRIIIa (including allotypes V158 and F158) and FcγRIIIb (including allotypes FcγRIIIb-NA1 and FcγRIIIb-NA2), α chain that binds to the Fc portion of IgG is associated with common γ chain having ITAM responsible for transduction of intracellular activation signal. Meanwhile, the cytoplasmic domain of FcγRII (CD32) including isoforms FcγRIIa (including allotypes H131 and R131) and FcγRIIc contains ITAM. These receptors are expressed on many immune cells such as macrophages, mast cells, and antigen-presenting cells. The activation signal transduced upon binding of these receptors to the Fc portion of IgG results in enhancement of the phagocytic activity and inflammatory cytokine production of macrophages, mast cell degranulation, and the enhanced function of antigen-presenting cells. Fcγ receptors having the ability to transduce the activation signal as described above are also referred to as activating Fcγ receptors.

Meanwhile, the intracytoplasmic domain of FcγRIIb (including FcγRIIb-1 and FcγRIIb-2) contains ITIM responsible for transduction of inhibitory signals. The crosslinking between FcγRIIb and B cell receptor (BCR) on B cells suppresses the activation signal from BCR, which results in suppression of antibody production via BCR. The crosslinking of FcγRIII and FcγRIIb on macrophages suppresses the phagocytic activity and inflammatory cytokine production. Fcγ receptors having the ability to transduce the inhibitory signal as described above are also referred to as inhibitory Fcγ receptors.

## Binding activity to the Fcγ receptor

The binding activity of an FcγR-binding domain, which is included in an antigen-binding molecule of the present invention, to any of the human Fcγ receptors, FcγRI, FcγRIIa, FcγRIIb, FcγRIIIa, and/or FcγRIIIb, can be confirmed by the above-described FACS and ELISA format, as well as ALPHA Screen (Amplified Luminescent Proximity Homogeneous Assay), a BIACORE method using the surface plasmon resonance (SPR) phenomena, and such (Proc. Natl. Acad. Sci. USA (2006) 103 (11), 4005-4010). The extracellular domain of a human Fcγ receptor may be used as the soluble antigen in these assays.

ALPHA screen is performed by the ALPHA technology based on the principle described below using two types of beads: donor and acceptor beads. A luminescent signal is detected only when molecules linked to the donor beads interact biologically with molecules linked to the acceptor beads and when the two beads are located in close proximity. Excited by laser beam, the photosensitizer in a donor bead converts oxygen around the bead into excited singlet oxygen. When the singlet oxygen diffuses around the donor beads and reaches the acceptor beads located in close proximity, a chemiluminescent reaction within the acceptor beads is induced. This reaction ultimately results in light emission. If molecules linked to the donor beads do not interact with molecules linked to the acceptor beads, the singlet oxygen produced by donor beads do not reach the acceptor beads and chemiluminescent reaction does not occur.

For example, a biotin-labeled antigen-binding molecule comprising Fc region is immobilized to the donor beads and glutathione S-transferase (GST)-tagged Fcγ receptor is immobilized to the acceptor beads. In the absence of an antigen-binding molecule comprising a competitive Fc region variant, Fcγ receptor interacts with a antigen-binding molecule comprising a native Fc region, inducing a signal of 520 to 620 nm as a result. The antigen-binding molecule having a non-tagged Fc region variant competes with the antigen-binding molecule comprising a native Fc region for the interaction with Fcγ receptor. The relative binding affinity can be determined by quantifying the reduction of fluorescence as a result of competition. Methods for biotinylating the antigen-binding molecules such as antibodies using Sulfo-NHS-biotin or the like are known. Appropriate methods for adding the GST tag to an Fcγ receptor include methods that involve fusing polypeptides encoding Fcγ and GST in-frame, expressing the fused gene using cells introduced with a vector to which the gene is operablye linked, and then purifying using a glutathione column. The induced signal can be preferably analyzed, for example, by fitting to a one-site competition model based on nonlinear regression analysis using software such as GRAPHPAD PRISM (GraphPad; San Diego).

One of the substances for observing their interaction is immobilized as a ligand onto the gold thin layer of a sensor chip. When light is shed on the rear surface of the sensor chip so that total reflection occurs at the interface between the gold thin layer and glass, the intensity of reflected light is partially reduced at a certain site (SPR signal). The other substance for observing their interaction is injected as an analyte onto the surface of the sensor chip. The mass of immobilized ligand molecule increases when the analyte binds to the ligand. This alters the refraction index of solvent on the surface of the sensor chip. The change in refraction index causes a positional shift of SPR signal (conversely, the dissociation shifts the signal back to the original position). In the Biacore system, the amount of shift described above (*i.e.,* the change of mass on the sensor chip surface) is plotted on the vertical axis, and thus the change of mass over time is shown as measured data (sensorgram). Kinetic parameters (association rate constant (ka) and dissociation rate constant (kd)) are determined from the curve of sensorgram, and affinity (KD) is determined from the ratio between these two constants. Inhibition assay is preferably used in the BIACORE methods. Examples of such inhibition assay are described in Proc. Natl. Acad. Sci. USA (2006) 103(11), 4005-4010.

Fcy-receptor-binding domain

An Fcy-receptor-binding domain having higher Fcy-receptor-binding activity than an Fc region of a native human IgG in which the sugar chain bonded at position 297 (EU numbering) is a fucose-containing sugar chain, may be produced by altering the amino acid of the native human IgG Fc region. Furthermore, any structure of the antigen-binding domain described previously, which is characterized by being bound to an Fcγ receptor, may be used for the Fcγ-receptor-binding domain. In such a case, the Fcγ-receptor-binding domain may be produced without the need for introducing amino acid alterations, or affinity to the Fcγ receptor may be increased by introducing further alterations. Examples of such an Fcy-receptor-binding domain include an Fab fragment antibody that binds to FcγRIIIa, which is described in Protein Eng Des Sel. 2009 Mar;22(3):175-88, Protein Eng Des Sel. 2008 Jan;21(1):1-10 and J Immunol. 2002 Jul 1;169(1):137-44, a camel-derived single domain antibody and a single-chain Fv antibody, and an FcγRI-binding cyclic peptide described in FASEB J. 2009 Feb;23(2):575-85. Whether or not the FcγR-binding activity of the Fcγ-receptor-binding domain is higher than that of the Fc region of a native human IgG in which the sugar chain bonded at position 297 (EU numbering) is a fucose-containing sugar chain may be determined appropriately using the method described in the above-mentioned section on binding activity.

In the present invention, a human IgG Fc region is a suitable example of a starting-material Fcγ-receptor-binding domain. In the present invention, "altering the amino acid" or "amino acid alteration" of the Fc region includes altering the amino acid sequence of the starting-material Fc region to a different amino acid sequence. As long as the modified variant of the starting-material Fc region can bind to a human Fcγ receptor in a neutral pH range, any Fc region may be used as the starting-material Fc region. Furthermore, an Fc region produced by further altering an already altered Fc region used as a starting Fc region may also be preferably used as the Fc region of the present invention. The "starting Fc region" can refer to the polypeptide itself, a composition comprising the starting Fc region, or an amino acid sequence encoding the starting Fc region. Starting Fc regions can comprise a known Fc region produced *via* recombination described briefly in section "Antibodies". The origin of starting Fc regions is not limited, and they may be obtained from human or any nonhuman organisms. Such organisms preferably include mice, rats, guinea pigs, hamsters, gerbils, cats, rabbits, dogs, goats, sheep, bovines, horses, camels and organisms selected from nonhuman primates. In another embodiment, starting Fcγ receptor binding domains can also be obtained from cynomolgus monkeys, marmosets, rhesus monkeys, chimpanzees, or humans. Starting Fc regions can be obtained preferably from human IgG1; however, they are not limited to any particular IgG class. This means that an Fc region of human IgG1, IgG2, IgG3, or IgG4 can be used appropriately as a starting Fc region, and herein also means that an Fc region of an arbitrary IgG class or subclass derived from any organisms described above can be preferably used as a starting Fc region. Examples of naturally-occurring IgG variants or modified forms are described in published documents (Curr. Opin. Biotechnol. (2009) 20 (6): 685-91; Curr. Opin. Immunol. (2008) 20 (4), 460-470; Protein Eng. Des. Sel. (2010) 23 (4): 195-202; WO 2009/086320; WO 2008/092117; WO 2007/041635; and WO 2006/105338); however, they are not limited to the examples.

Examples of alterations include those with one or more mutations, for example, mutations by substitution of different amino acid residues for amino acids of starting Fc regions, by insertion of one or more amino acid residues into starting Fc regions, or by deletion of one or more amino acids from starting Fc region. Preferably, the amino acid sequences of altered Fc regions comprise at least a part of the amino acid sequence of a non-native Fc region. Such variants necessarily have sequence identity or similarity less than 100% to their starting Fc region. In a preferred embodiment, the variants have amino acid sequence identity or similarity about 75% to less than 100%, more preferably about 80% to less than 100%, even more preferably about 85% to less than 100%, still more preferably about 90% to less than 100%, and yet more preferably about 95% to less than 100% to the amino acid sequence of their starting Fc region. In a non-limiting embodiment of the present invention, at least one amino acid is different between a modified Fc region of the present invention and its starting Fc region. Amino acid difference between a modified Fc region of the present invention and its starting Fc region can also be preferably specified based on amino acid differences at above-described particular amino acid positions according to EU numbering.

Known methods such as site-directed mutagenesis (Kunkel et al. (Proc. Natl. Acad. Sci. USA (1985) 82, 488-492)) and overlap extension PCR can be appropriately employed to modify the amino acids of Fc regions. Furthermore, various known methods can also be used as an amino acid modification method for substituting amino acids by those other than natural amino acids (Annu. Rev. Biophys. Biomol. Struct. (2006) 35, 225-249; Proc. Natl. Acad. Sci. U.S.A. (2003) 100 (11), 6353-6357). For example, a cell-free translation system (Clover Direct (Protein Express)) containing tRNAs in which amber suppressor tRNA, which is complementary to UAG codon (amber codon) which is a stop codon, is linked with an unnatural amino acid may be suitably used.

An Fc region having Fcγ receptor-binding activity in a neutral pH range that is contained in the antigen-binding molecules of the present invention may be obtained by any method, but specifically, an Fc region having Fcγ receptor-binding activity in the neutral pH range may be obtained by altering amino acids of human IgG immunoglobulin used as a starting Fc region. Preferred IgG immunoglobulin Fc regions to be altered include, for example, the Fc regions of human IgG (IgG1, IgG2, IgG3, or IgG4, and their variants). IgG Fc regions include mutants naturally formed therefrom. A number of allotype sequences due to genetic polymorphism are described in "Sequences of proteins of immunological interest", NIH Publication No.91-3242, for the Fc regions of human IgG1, human IgG2, human IgG3, and human IgG4 antibodies, and any one of them may be used in the present invention. In particular for the human IgG1 sequence, the amino acid sequence of positions 356 to 358 (EU numbering) may be either DEL or EEM.

Amino acids at any positions may be altered to other amino acids as long as the Fc region has Fcγ receptor-binding activity in a neutral pH range, or its Fcγ receptor-binding activity in a neutral range can be enhanced. When an antigen-binding molecule contains the Fc region of human IgG1, it is preferred to include alterations that result in enhancement of Fcγ receptor-binding in a neutral pH range compared to the binding activity of the starting Fc region of human IgG1. Amino acid alterations for enhancing Fcγ receptor-binding activity in a neutral pH range have been reported, for example, in WO 2007/024249, WO 2007/021841, WO 2006/031370, WO 2000/042072, WO 2004/029207, WO 2004/099249, WO 2006/105338, WO 2007/041635, WO 2008/092117, WO 2005/070963, WO 2006/020114, WO 2006/116260, and WO 2006/023403.

Examples of such amino acids that can be altered include at least one or more amino acids selected from the group consisting of those at positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, and 440 according to EU numbering. Alteration of these amino acids enhances the Fcγ receptor-binding of an IgG immunoglobulin Fc region in a neutral pH range.

Particularly preferred alterations for use in the present invention include the following alterations:

the amino acid at position 221 to either Lys or Tyr;
the amino acid at position 222 to any one of Phe, Trp, Glu, and Tyr;
the amino acid at position 223 to any one of Phe, Trp, Glu, and Lys;
the amino acid at position 224 to any one of Phe, Trp, Glu, and Tyr;
the amino acid at position 225 to any one of Glu, Lys, and Trp;
the amino acid at position 227 to any one of Glu, Gly, Lys, and Tyr;
the amino acid at position 228 to any one of Glu, Gly, Lys, and Tyr;
the amino acid at position 230 to any one of Ala, Glu, Gly, and Tyr;
the amino acid at position 231 to any one of Glu, Gly, Lys, Pro, and Tyr;
the amino acid at position 232 to any one of Glu, Gly, Lys, and Tyr;
the amino acid at position 233 to any one of Ala, Asp, Phe, Gly, His, Ile, Lys, Leu, Met, Asn, Gln, Arg, Ser, Thr, Val, Trp, and Tyr;
the amino acid at position 234 to any one of Ala, Asp, Glu, Phe, Gly, His, Ile, Lys, Met, Asn, Pro, Gln, Arg, Ser, Thr, Val, Trp, and Tyr;
the amino acid at position 235 to any one of Ala, Asp, Glu, Phe, Gly, His, Ile, Lys, Met, Asn, Pro, Gln, Arg, Ser, Thr, Val, Trp, and Tyr;
the amino acid at position 236 to any one of Ala, Asp, Glu, Phe, His, Ile, Lys, Leu, Met, Asn, Pro, Gln, Arg, Ser, Thr, Val, Trp, and Tyr;
the amino acid at position 237 to any one of Asp, Glu, Phe, His, Ile, Lys, Leu, Met, Asn, Pro, Gln, Arg, Ser, Thr, Val, Trp, and Tyr;
the amino acid at position 238 to any one of Asp, Glu, Phe, Gly, His, Ile, Lys, Leu, Met, Asn, Gln, Arg, Ser, Thr, Val, Trp, and Tyr;
the amino acid at position 239 to any one of Asp, Glu, Phe, Gly, His, Ile, Lys, Leu, Met, Asn, Pro, Gln, Arg, Thr, Val, Trp, and Tyr;
the amino acid at position 240 to any one of Ala, Ile, Met, and Thr;
the amino acid at position 241 to any one of Asp, Glu, Leu, Arg, Trp, and Tyr;
the amino acid at position 243 to any one of Leu, Glu, Leu, Gln, Arg, Trp, and Tyr;
the amino acid at position 244 to His;
the amino acid at position 245 to Ala;

the amino acid at position 246 to any one of Asp, Glu, His, and Tyr;
the amino acid at position 247 to any one of Ala, Phe, Gly, His, Ile, Leu, Met, Thr, Val, and Tyr;
the amino acid at position 249 to any one of Glu, His, Gln, and Tyr;
the amino acid at position 250 to either Glu or Gln;
the amino acid at position 251 to Phe;
the amino acid at position 254 to any one of Phe, Met, and Tyr;
the amino acid at position 255 to any one of Glu, Leu, and Tyr;
the amino acid at position 256 to any one of Ala, Met, and Pro;
the amino acid at position 258 to any one of Asp, Glu, His, Ser, and Tyr;
the amino acid at position 260 to any one of Asp, Glu, His, and Tyr;
the amino acid at position 262 to any one of Ala, Glu, Phe, Ile, and Thr;
the amino acid at position 263 to any one of Ala, Ile, Met, and Thr;
the amino acid at position 264 to any one of Asp, Glu, Phe, Gly, His, Ile, Lys, Leu, Met, Asn, Pro, Gln, Arg, Ser, Thr, Trp, and Tyr;
the amino acid at position 265 to any one of Ala, Leu, Phe, Gly, His, Ile, Lys, Leu, Met, Asn, Pro, Gln, Arg, Ser, Thr, Val, Val, Trp, and Tyr;
the amino acid at position 266 to any one of Ala, Ile, Met, and Thr;
the amino acid at position 267 to any one of Asp, Glu, Phe, His, Ile, Lys, Leu, Met, Asn, Pro, Gln, Arg, Thr, Val, Trp, and Tyr;
the amino acid at position 268 to any one of Asp, Glu, Phe, Gly, Ile, Lys, Leu, Met, Pro, Gln, Arg, Thr, Val, and Trp;
the amino acid at position 269 to any one of Phe, Gly, His, Ile, Lys, Leu, Met, Asn, Pro, Arg, Ser, Thr, Val, Trp, and Tyr;
the amino acid at position 270 to any one of Glu, Phe, Gly, His, Ile, Leu, Met, Pro, Gln, Arg, Ser, Thr, Trp, and Tyr;
the amino acid at position 271 to any one of Ala, Asp, Glu, Phe, Gly, His, Ile, Lys, Leu, Met, Asn, Gln, Arg, Ser, Thr, Val, Trp, and Tyr;
the amino acid at position 272 to any one of Asp, Phe, Gly, His, Ile, Lys, Leu, Met, Pro, Arg, Ser, Thr, Val, Trp, and Tyr;
the amino acid at position 273 to either Phe or Ile;
the amino acid at position 274 to any one of Asp, Glu, Phe, Gly, His, Ile, Leu, Met, Asn, Pro, Arg, Ser, Thr, Val, Trp, and Tyr;
the amino acid at position 275 to either Leu or Trp;
the amino acid at position 276 to any one of Asp, Glu, Phe, Gly, His, Ile, Leu, Met, Pro, Arg, Ser, Thr, Val, Trp, and Tyr;
the amino acid at position 278 to any one of Asp, Glu, Gly, His, Ile, Lys, Leu, Met, Asn, Pro, Gln, Arg, Ser, Thr, Val, and Trp;
the amino acid at position 279 to Ala;
the amino acid at position 280 to any one of Ala, Gly, His, Lys, Leu, Pro, Gln, Trp, and Tyr;
the amino acid at position 281 to any one of Asp, Lys, Pro, and Tyr;
the amino acid at position 282 to any one of Glu, Gly, Lys, Pro, and Tyr;
the amino acid at position 283 to any one of Ala, Gly, His, Ile, Lys, Leu, Met, Pro, Arg, and Tyr;
the amino acid at position 284 to any one of Asp, Glu, Leu, Asn, Thr, and Tyr;
the amino acid at position 285 to any one of Asp, Glu, Lys, Gln, Trp, and Tyr;
the amino acid at position 286 to any one of Glu, Gly, Pro, and Tyr;
the amino acid at position 288 to any one of Asn, Asp, Glu, and Tyr;
the amino acid at position 290 to any one of Asp, Gly, His, Leu, Asn, Ser, Thr, Trp, and Tyr;
the amino acid at position 291 to any one of Asp, Glu, Gly, His, Ile, Gln, and Thr;
the amino acid at position 292 to any one of Ala, Asp, Glu, Pro, Thr, and Tyr;
the amino acid at position 293 to any one of Phe, Gly, His, Ile, Leu, Met, Asn, Pro, Arg, Ser, Thr, Val, Trp, and Tyr;
the amino acid at position 294 to any one of Phe, Gly, His, Ile, Lys, Leu, Met, Asn, Pro, Arg, Ser, Thr, Val, Trp, and Tyr;
the amino acid at position 295 to any one of Asp, Glu, Phe, Gly, His, Ile, Lys, Met, Asn, Pro, Arg, Ser, Thr, Val, Trp, and Tyr;
the amino acid at position 296 to any one of Ala, Asp, Glu, Gly, His, Ile, Lys, Leu, Met, Asn, Gln, Arg, Ser, Thr, and Val;
the amino acid at position 297 to any one of Asp, Glu, Phe, Gly, His, Ile, Lys, Leu, Met, Pro, Gln, Arg, Ser, Thr, Val, Trp, and Tyr;
the amino acid at position 298 to any one of Ala, Asp, Glu, Phe, His, Ile, Lys, Met, Asn, Gln, Arg, Thr, Val, Trp, and Tyr;
the amino acid at position 299 to any one of Ala, Asp, Glu, Phe, Gly, His, Ile, Lys, Leu, Met, Asn, Pro, Gln, Arg, Ser, Val, Trp, and Tyr;
the amino acid at position 300 to any one of Ala, Asp, Glu, Gly, His, Ile, Lys, Leu, Met, Asn, Pro, Gln, Arg, Ser, Thr, Val, and Trp;
the amino acid at position 301 to any one of Asp, Glu, His, and Tyr;
the amino acid at position 302 to Ile;

the amino acid at position 303 to any one of Asp, Gly, and Tyr;
the amino acid at position 304 to any one of Asp, His, Leu, Asn, and Thr;
the amino acid at position 305 to any one of Glu, Ile, Thr, and Tyr;
the amino acid at position 311 to any one of Ala, Asp, Asn, Thr, Val, and Tyr;
the amino acid at position 313 to Phe;
the amino acid at position 315 to Leu;
the amino acid at position 317 to either Glu or Gln;
the amino acid at position 318 to any one of His, Leu, Asn, Pro, Gln, Arg, Thr, Val, and Tyr;
the amino acid at position 320 to any one of Asp, Phe, Gly, His, Ile, Leu, Asn, Pro, Ser, Thr, Val, Trp, and Tyr;
the amino acid at position 322 to any one of Ala, Asp, Phe, Gly, His, Ile, Pro, Ser, Thr, Val, Trp, and Tyr;
the amino acid at position 323 to Ile;
the amino acid at position 324 to any one of Asp, Phe, Gly, His, Ile, Leu, Met, Pro, Arg, Thr, Val, Trp, and Tyr;
the amino acid at position 325 to any one of Ala, Asp, Glu, Phe, Gly, His, Ile, Lys, Leu, Met, Pro, Gln, Arg, Ser, Thr, Val, Trp, and Tyr;
the amino acid at position 326 to any one of Ala, Asp, Glu, Gly, Ile, Leu, Met, Asn, Pro, Gln, Ser, Thr, Val, Trp, and Tyr;
the amino acid at position 327 to any one of Ala, Asp, Glu, Phe, Gly, His, Ile, Lys, Leu, Met, Asn, Pro, Arg, Thr, Val, Trp, and Tyr;
the amino acid at position 328 to any one of Ala, Asp, Glu, Phe, Gly, His, Ile, Lys, Met, Asn, Pro, Gln, Arg, Ser, Thr, Val, Trp, and Tyr;
the amino acid at position 329 to any one of Asp, Glu, Phe, Gly, His, Ile, Lys, Leu, Met, Asn, Gln, Arg, Ser, Thr, Val, Trp, and Tyr;
the amino acid at position 330 to any one of Cys, Glu, Phe, Gly, His, Ile, Lys, Leu, Met, Asn, Pro, Arg, Ser, Thr, Val, Trp, and Tyr;
the amino acid at position 331 to any one of Asp, Phe, His, Ile, Leu, Met, Gln, Arg, Thr, Val, Trp, and Tyr;
the amino acid at position 332 to any one of Ala, Asp, Glu, Phe, Gly, His, Lys, Leu, Met, Asn, Pro, Gln, Arg, Ser, Thr, Val, Trp, and Tyr;
the amino acid at position 333 to any one of Ala, Asp, Glu, Phe, Gly, His, Ile, Leu, Met, Pro, Ser, Thr, Val, and Tyr;
the amino acid at position 334 to any one of Ala, Glu, Phe, Ile, Leu, Pro, and Thr;
the amino acid at position 335 to any one of Asp, Phe, Gly, His, Ile, Leu, Met, Asn, Pro, Arg, Ser, Val, Trp, and Tyr;
the amino acid at position 336 to any one of Glu, Lys, and Tyr;
the amino acid at position 337 to any one of Glu, His, and Asn;
the amino acid at position 339 to any one of Asp, Phe, Gly, Ile, Lys, Met, Asn, Gln, Arg, Ser, and Thr;
the amino acid at position 376 to either Ala or Val;
the amino acid at position 377 to either Gly or Lys;
the amino acid at position 378 to Asp;
the amino acid at position 379 to Asn;
the amino acid at position 380 to any one of Ala, Asn, and Ser;
the amino acid at position 382 to either Ala or Ile;
the amino acid at position 385 to Glu;
the amino acid at position 392 to Thr;
the amino acid at position 396 to Leu;
the amino acid at position 421 to Lys;
the amino acid at position 427 to Asn;
the amino acid at position 428 to either Phe or Leu;
the amino acid at position 429 to Met;
the amino acid at position 434 to Trp;
the amino acid at position 436 to Ile; and
the amino acid at position 440 to any one of Gly, His, Ile, Leu, and Tyr,

according to EU numbering in the Fc region.
The number of amino acids that are altered is not particularly limited. An amino acid at one position only may be altered, or amino acids at two or more positions may be altered. Examples of combinations of amino acid alterations at two or more positions include the combinations shown in Table 5 (Tables 5-1 to 5-3).

[Table 5-1]

| COMBINATION OF AMINO ACIDS | COMBINATION OF AMINO ACIDS |
|---|---|
| K370E/P396L/D270E | S239Q/I332Q |

(continued)

| COMBINATION OF AMINO ACIDS | COMBINATION OF AMINO ACIDS |
|---|---|
| Q419H/P396L/D270E | S267D/I332E |
| V240A/P396L/D270E | S267E/I332E |
| R255L/P396L/D270E | S267L/A327S |
| R255L/P396L/D270E | S267Q/A327S |
| R255L/P396L/D270E/R292G | S298A/I332E |
| R255L/P396L/D270E | S304T/I332E |
| R255L/P396L/D270E/Y300L | S324G/I332D |
| F243L/D270E/K392N/P396L | S324G/I332E |
| F243L/R255L/D270E/P396L | S324I/I332D |
| F243L/R292P/Y300L/V305I/P396L | S324I/I332E |
| F243L/R292P/Y300L/P396L | T260H/I332E |
| F243L/R292P/Y300L | T335D/I332E |
| F243L/R292P/P396L | V240I/V266I |
| F243L/R292P/V305I | V264I/I332E |
| F243L/R292P | D265F/N297E/I332E |
| S298A/E333A/K334A | D265Y/N297D/I332E |
| E380A/T307A | F243L/V262I/V264W |
| K326M/E333S | N297D/A330Y/I332E |
| K326A/E333A | N297D/T299E/I332E |
| S317A/K353A | N297D/T299F/I332E |
| A327D/I332E | N297D/T299H/I332E |
| A330L/I332E | N297D/T299I/I332E |
| A330Y/I332E | N297D/T299L/I332E |
| E258H/I332E | N297D/T299V/I332E |
| E272H/I332E | P230A/E233D/I332E |
| E272I/N276D | P244H/P245A/P247V |
| E272R/I332E | S239D/A330L/I332E |
| E283H/I332E | S239D/A330Y/I332E |
| E293R/I332E | S239D/H268E/A330Y |
| F241L/V262I | S239D/I332E/A327A |
| F241W/F243W | S239D/I332E/A330I |

[Table 5-2]

| | |
|---|---|
| F243L/V264I | S239D/N297D/I332E |
| H268D/A330Y | S239D/S298A/I332E |
| H268E/A330Y | S239D/V264I/I332E |
| K246H/I332E | S239E/N297D/I332E |

(continued)

| | |
|---|---|
| L234D/I332E | S239E/V264I/I332E |
| L234E/I332E | S239N/A330L/I332E |
| L234G/I332E | S239N/A330Y/I332E |
| L234I/I332E | S239N/S298A/I332E |
| L234I/L235D | S239Q/V264I/I332E |
| L234Y/I332E | V264E/N297D/I332E |
| L235D/I332E | V264I/A330L/I332E |
| L235E/I332E | V264I/A330Y/I332E |
| L235I/I332E | V264I/S298A/I332E |
| L235S/I332E | Y296D/N297D/I332E |
| L328A/I332D | Y296E/N297D/I332E |
| L328D/I332D | Y296H/N297D/I332E |
| L328D/I332E | Y296N/N297D/I332E |
| L328E/I332D | Y296Q/N297D/I332E |
| L328E/I332E | Y296T/N297D/I332E |
| L328F/I332D | D265Y/N297D/T299L/I332E |
| L328F/I332E | F241E/F243Q/V262T/V264E |
| L328H/I332E | F241E/F243R/V262E/V264R |
| L328I/I332D | F241E/F243Y/V262T/V264R |
| L328I/I332E | F241L/F243L/V262I/V264I |
| L328M/I332D | F241R/F243Q/V262T/V264R |
| L328M/I332E | F241S/F243H/V262T/V264T |
| L328N/I332D | F241W/F243W/V262A/V264A |
| L328N/I332E | F241Y/F243Y/V262T/V264T |
| L328Q/I332D | I332E/A330Y/H268E/A327A |
| L328Q/I332E | N297D/I332E/S239D/A330L |
| L328T/I332D | N297D/S298A/A330Y/I332E |
| L328T/I332E | S239D/A330Y/I332E/ K326E |
| L328V/I332D | S239D/A330Y/I332E/ K326T |
| L328V/I332E | S239D/A330Y/I332E/L234I |
| L328Y/I332D | S239D/A330Y/I332E/L235D |

[Table 5-3]

| | |
|---|---|
| L328Y/I332E | S239D/A330Y/I332E/V240I |
| N297D/I332E | S239D/A330Y/I332E/V264T |
| N297E/I332E | S239D/A330Y/I332E/V266I |
| N297S/I332E | S239D/D265F/N297D/I332E |
| P227G/I332E | S239D/D265H/N297D/I332E |

(continued)

| | |
|---|---|
| P230A/E233D | S239D/D265I/N297D/I332E |
| Q295E/I332E | S239D/D265L/N297D/I332E |
| R255Y/I332E | S239D/ D265T/N297D/ I332E |
| S239D/I332D | S239D/D265V/N297D/I332E |
| S239D/I332E | S239D/D265Y/N297D/I332E |
| S239D/I332N | S239D/I332E/A330Y/A327A |
| S239D/I332Q | S239D/I332E/H268E/A327A |
| S239E/D265G | S239D/I332E/H268E/A330Y |
| S239E/D265N | S239D/N297D/I332E/A330Y |
| S239E/D265Q | S239D/N297D/I332E/K326E |
| S239E/I332D | S239D/N297D/I332E/L235D |
| S239E/I332E | S239D/V264I/A330L/I332E |
| S239E/I332N | S239D/V264I/S298A/I332E |
| S239E/I332Q | S239E/V264I/A330Y/I332E |
| S239N/I332D | F241E/F243Q/V262T/V264E/I332E |
| S239N/I332E | F241E/F243R/V262E/V264R/I332E |
| S239N/I332N | F241E/F243Y/V262T/V264R/I332E |
| S239N/I332Q | F241R/F243Q/V262T/V264R/I332E |
| S239Q/I332D | S239D/I332E/H268E/A330Y/A327A |
| S239Q/I332E | S239E/V264I/S298A/A330Y/I332E |
| S239Q/I332N | F241Y/F243Y/V262T/V264T/N297D/I332E |
| S267E/L328F | G236D/S267E |
| S239D/S267E | |

For the pH conditions to measure the binding activity of the Fcγ receptor-binding domain contained in the antigen-binding molecule of the present invention and the Fcγ receptor, conditions in an acidic pH range or in a neutral pH range may be suitably used. The neutral pH range, as a condition to measure the binding activity of the Fcγ receptor-binding domain contained in the antigen-binding molecule of the present invention and the Fcγ receptor, generally indicates pH 6.7 to pH 10.0. Preferably, it is a range indicated with arbitrary pH values between pH 7.0 and pH8.0; and preferably, it is selected from pH 7.0, pH 7.1, pH 7.2, pH 7.3, pH 7.4, pH 7.5, pH 7.6, pH 7.7, pH 7.8, pH 7.9, and pH 8.0; and particularly preferably, it is pH 7.4, which is close to the pH of plasma (blood) *in vivo*. Herein, the acidic pH range, as a condition for having a binding activity of the Fcγ receptor-binding domain contained in the antigen-binding molecule of the present invention and the Fcγ receptor, generally indicates pH 4.0 to pH 6.5. Preferably, it indicates pH 5.5 to pH 6.5, and particularly preferably, it indicates pH 5.8 to pH 6.0, which is close to the pH in the early endosome *in vivo*. With regard to the temperature used as measurement condition, the binding affinity between the Fcγ receptor-binding domain and the human Fcγ receptor can be evaluated at any temperature between 10°C and 50°C. Preferably, a temperature between 15°C and 40°C is used to determine the binding affinity between the human Fcγ receptor-binding domain and the Fcγ receptor. More preferably, any temperature between 20°C and 35°C, such as any from 20°C, 21°C, 22°C, 23°C, 24°C, 25°C, 26°C, 27°C, 28°C, 29°C, 30°C, 31°C, 32°C, 33°C, 34°C, or 35°C, can similarly be used to determine the binding affinity between the Fcγ receptor-binding domain and the Fcγ receptor. A temperature of 25°C is a non-limiting example in an embodiment of the present invention.

Herein, "the binding activity of the Fcγ receptor-binding domain toward Fcγ receptor is higher than the binding activity of the native Fc region toward activating Fcγ receptor" means that the binding activity of the Fcγ receptor-binding domain toward any of the human Fcγ receptors of FcγRI, FcγRIIa, FcγRIIb, FcγRIIIa, and/or FcγRIIIb is higher than the binding activity of the native Fcγ receptor-binding domain toward these human Fcγ receptors.

For example, it refers to the binding activity of the antigen-binding molecule including an Fcγ receptor-binding domain being 105% or more, preferably 110% or more, 115% or more, 120% or more, 125% or more, particularly preferably 130% or more, 135% or more, 140% or more, 145% or more, 150% or more, 155% or more, 160% or more, 165% or more, 170% or more, 175% or more, 180% or more, 185% or more, 190% or more, 195% or more, two-times or more, 2.5 times or more, three times or more, 3.5 times or more, four times or more, 4.5 times or more, five times or more, 7.5 times or more, ten times or more, 20 times or more, 30 times or more, 40 times or more, 50 times or more, 60 times or more, 70 times or more, 80 times or more, 90 times or more, or 100 times or more compared to the binding activity of the antigen-binding molecule including an Fc region of a native human IgG which is used as a control, based on the above-mentioned analysis method. For the native Fcγ receptor-binding domain, the starting-material Fcγ receptor-binding domain may be used, and the Fcγ receptor-binding domain of a native antibody belonging to the same subclass may also be used.

In the present invention, in particular, an Fc region of a native human IgG, in which the sugar chain bonded to an amino acid at position 297 (EU numbering) is a fucose-containing sugar chain, is suitably used as the Fc region of a native human IgG to be used as a control. Whether or not the sugar chain bonded to an amino acid at position 297 (EU numbering) is a fucose-containing sugar chain can be determined using the technique described in Non-Patent Document 24. For example, a method such the following enables determination of whether the sugar chain bonded to the native human IgG Fc region is a fucose-containing sugar chain. By reaction of *N*-glycosidase F (Roche diagnostics) with a native human IgG to be tested, a sugar chain is dissociated from the native human IgG to be tested (Weitzhandler et al. (J. Pharma. Sciences (1994) 83, 12, 1670-1675)). Next, a concentrated inspissated material of a reaction solution from which the proteins have been removed by reaction with ethanol (Schenk et al. (J. Clin. Investigation (2001) 108 (11) 1687-1695)) is fluorescence labeled by 2-aminopyridine (Bigge et al. (Anal. Biochem. (1995) 230 (2) 229-238)). Fluorescence-labeled 2-AB-modified sugar chain produced by removal of reagent by solid-phase extraction using a cellulose cartridge, is analyzed by normal-phase chromatography. Observation of the detected chromatogram peak enables determination of whether or not the sugar chain bonded to the native human IgG Fc region is a fucose-containing sugar chain. Non-limiting examples of such an Fc region of a native human IgG, in which the sugar chain bonded to an amino acid at position 297 (EU numbering) is a fucose-containing sugar chain, include Fc regions included in antibodies obtained by expressing in CHO cells, such as CHO-K1 (American Type Culture Collection, ATCC No. CRL-61) or DXB11 (American Type Culture Collection, ATCC No. CRL-11397), genes encoding antibodies containing a native human IgG Fc region. Using the Fcy-receptor-binding activities of Fc regions included in such antibodies as controls, the Fcy-receptor-binding activities of Fc regions of the present invention were compared. This enables determination of whether the Fcy-receptor-binding domain of the present invention has higher Fcy-receptor-binding activity than the Fc region of a native human IgG in which the sugar chain bonded at position 297 (EU numbering) is a fucose-containing sugar chain. Furthermore, the amount of fucose bonded to the sugar chain included in the compared Fc regions can be compared by determining the amount of fucose in the sugar chain bonded to the Fc regions included in these antibodies and the amount of fucose in the sugar chain bonded to the Fc regions of the present invention using methods such as those mentioned above.

As antigen-binding molecule containing an Fc region of the same subclass native antibody that is used as a control, antigen-binding molecules having an Fc region of a monoclonal IgG antibody may be suitably used. The structures of such Fc regions are shown in SEQ ID NO: 11 (A is added to the N terminus of RefSeq Accession No. AAC82527.1), SEQ ID NO: 12 (A is added to the N terminus of RefSeq Accession No. AAB59393.1), SEQ ID NO: 13 (RefSeq Accession No. CAA27268.1), and SEQ ID NO: 14 (A is added to the N terminus of RefSeq Accession No. AAB59394.1). Further, when an antigen-binding molecule containing an Fc region of a particular antibody isotype is used as the test substance, the effect of the binding activity of the antigen-binding molecule containing a test Fc region toward the Fcγ receptor is tested by using as a control an antigen-binding molecule having an Fc region of a monoclonal IgG antibody of that particular isotype. In this way, antigen-binding molecules containing an Fc region whose binding activity toward the Fcγ receptor was demonstrated to be high are suitably selected.

Examples of Fcy-receptor-binding domains suitable for use in the present invention include Fcγ-receptor-binding domains with the property of having higher binding activity to certain Fcγ receptors than to other Fcγ receptors (Fcγ-receptor-binding domains with selective Fcγ-receptor-binding activity). When an antibody is used as the antigen-binding molecule (when an Fc region is used as the Fcγ-receptor-binding domain), since a single antibody molecule can only bind to a single Fcγ receptor, a single antigen-binding molecule which is bound to an inhibitory Fcγ receptor cannot bind to another activating FcγR, and a single antigen-binding molecule which is bound to an activating Fcγ receptor cannot bind to another activating Fcγ receptor nor an inhibitory Fcγ receptor.

As described above, suitable examples of the activating Fcγ receptor are FcyRI (CD64) including FcγRIa, FcγRIb, and FcγRIc, and FcγRIII (CD16) including isoforms FcγRIIIa (including allotypes V158 and F158) and FcγRIIIb (including allotypes FcγRIIIb-NA1 and FcγRIIIb-NA2). FcγRIIb (including FcγRIIb-1 and FcγRIIb-2) is a suitable example of the inhibitory Fcγ receptor.

FcyR-binding domain having selective binding activity to an Fcγ receptor

Whether or not an FcγR-binding domain of the present invention has selective binding activity can be confirmed by comparing binding activities to the respective Fcγ receptors, determined by the method described in the above-mentioned section on binding activity to Fcγ receptors. An FcγR-binding domain with higher binding activity to inhibitory Fcγ receptors than to activating Fcγ receptors may be used as the selective FcγR-binding domain included in the antigen-binding molecule provided by the present invention. In a non-limiting embodiment, an FcγR-binding domain with higher binding activity to FcγRIIb (including FcγRIIb-1 and FcγRIIb-2) than to an activating Fcγ receptor selected from the group consisting of FcγRI(CD64) including FcγRIa, FcγRIb, FcγRIc, FcγRIII(CD16) including isoforms FcγRIIIa (including allotypes V158 and F158) and FcyRIIIb (including allotypes FcγRIIIb-NA1 and FcγRIIIb-NA2), and FcγRII(CD32) including isoforms FcγRIIa and FcγRIIc (including allotypes H131 and R131) may be used as a selective FcγR-binding domain included in an antigen-binding molecule provided by the present invention. Furthermore, in a non-limiting embodiment of the present invention, an FcγR-binding domain with higher binding activity to FcγRIIb-1 and/or FcγRIIb-2 than to FcγRIa, FcγRIb, and FcyRIc, FcγRIIIa including allotype V158, FcγRIIIa including allotype F158, FcγRIIIb including allotype FcγRIIIb-NA1, FcγRIIIb including allotype FcγRIIIb-NA2, FcγRIIa including allotype H131, FcγRIIa including allotype R131, and/or FcγRIIc may be used as a selective FcγR-binding domain included in an antigen-binding molecule provided by the present invention. Whether an FcγR-binding domain to be tested has selective binding activity to Fcγ receptors can be determined by comparing the value (ratio) obtained by dividing the KD values of the FcγR-binding domain for FcγRIa, FcγRIb, FcγRIc, FcγRIIIa including allotype V158, FcγRIIIa including allotype F158, FcγRIIIb including allotype FcγRIIIb-NA1, FcγRIIIb including allotype FcγRIIIb-NA2, FcγRIIa including allotype H131, FcγRIIa including allotype R131, and/or FcγRIIc by the KD values for FcγRIIb-1 and/or FcγRIIb-2, wherein the KD values are determined by the method described in the above-mentioned section on binding activity to Fcγ receptors, or more specifically, by comparing the FcγR selectivity indices shown in Equation 1.

[Equation 1]

$$\text{Fc}\gamma\text{R selectivity index} = \text{KD value for activating Fc}\gamma\text{R} / \text{KD value for inhibitory Fc}\gamma\text{R}$$

In Equation 1 mentioned above, "activating FcγR" refers to FcγRIa, FcγRIb, FcγRIc, FcγRIIIa including allotype V158, FcγRIIIa including allotype F158, FcyRIIIb including allotype FcγRIIIb-NA1, FcγRIIIb including allotype FcγRIIIb-NA2, FcγRIIa including allotype H131, FcγRIIa including allotype R131, and/or FcγRIIc, and inhibitory FcγR refers to FcγRIIb-1 and/or FcγRIIb-2. Although the activating FcγR and inhibitory FcγR used for the KD value measurements may be selected from any combination, in a non-limiting embodiment, a value (ratio) obtained by dividing the KD value for FcyRIIa including allotype H131 by the KD value for FcγRIIb-1 and/or FcγRIIb-2 may be used.

For example, the FcγR selectivity indices have values of, 1.2 or greater, 1.3 or greater, 1.4 or greater, 1.5 or greater, 1.6 or greater, 1.7 or greater, 1.8 or greater, 1.9 or greater, 2 or greater, 3 or greater, 5 or greater, 6 or greater, 7 or greater, 8 or greater, 9 or greater, 10 or greater, 15 or greater, 20 or greater, 25 or greater, 30 or greater, 35 or greater, 40 or greater, 45 or greater, 50 or greater, 55 or greater, 60 or greater, 65 or greater, 70 or greater, 75 or greater, 80 or greater, 85 or greater, 90 or greater, 95 or greater, 100 or greater, 110 or greater, 120 or greater, 130 or greater, 140 or greater, 150 or greater, 160 or greater, 170 or greater, 180 or greater, 190 or greater, 200 or greater, 210 or greater, 220 or greater, 230 or greater, 240 or greater, 250 or greater, 260 or greater, 270 or greater, 280 or greater, 290 or greater, 300 or greater, 310 or greater, 320 or greater, 330 or greater, 340 or greater, 350 or greater, 360 or greater, 370 or greater, 380 or greater, 390 or greater, 400 or greater, 410 or greater, 420 or greater, 430 or greater, 440 or greater, 450 or greater, 460 or greater, 470 or greater, 480 or greater, 490 or greater, 500 or greater, 520 or greater, 540 or greater, 560 or greater, 580 or greater, 600 or greater, 620 or greater, 640 or greater, 660 or greater, 680 or greater, 700 or greater, 720 or greater, 740 or greater, 760 or greater, 780 or greater, 800 or greater, 820 or greater, 840 or greater, 860 or greater, 880 or greater, 900 or greater, 920 or greater, 940 or greater, 960 or greater, 980 or greater, 1000 or greater, 1500 or greater, 2000 or greater, 2500 or greater, 3000 or greater, 3500 or greater, 4000 or greater, 4500 or greater, 5000 or greater, 5500 or greater, 6000 or greater, 6500 or greater, 7000 or greater, 7500 or greater, 8000 or greater, 8500 or greater, 9000 or greater, 9500 or greater, 10000 or greater, or 100000 or greater.

A non-limiting embodiment of the selective FcyR-binding domain in an antigen-binding molecule of the present invention includes, for example, Fc regions produced by modifying the FcyR-binding domain included in an Fc region (an Fc region of the IgG class refers to the region from cysteine at position 226 (EU numbering) to the C terminus, or from proline at position 230 (EU numbering) to the C terminus, but is not limited thereto) constituting a portion of a constant region presented as human IgG1 (SEQ ID NO: 14), IgG2 (SEQ ID NO: 15), IgG3 (SEQ ID NO: 16), or IgG4 (SEQ ID NO: 17). An example of a method for producing the modified Fc regions includes the method described in the above-mentioned section on amino acid alterations. Examples of such altered Fc regions include an Fc region in which amino acid at position 238 (EU numbering) is Asp or an Fc region in which amino acid at position 328 (EU numbering) is Glu in a human IgG (IgG1, IgG2, IgG3, or IgG4). An Fc region in which amino acid at position 238 (EU numbering) is Asp or an Fc region in which amino acid at position 328 (EU numbering) is Glu in a human IgG (IgG1, IgG2, IgG3, or IgG4), and antigen-binding molecules containing such an Fc region show higher binding activity to FcγRIIb-1 and/or FcγRIIb-2 than to FcγRIa, FcγRIb, FcγRIc, FcγRIIIa including allotype V158, FcγRIIIa including allotype F158, FcγRIIIb including allotype FcγRIIIb-NA1, FcγRIIIb including allotype FcγRIIIb-NA2, FcγRIIa including allotype H131, FcγRIIa including allotype R131, and/or FcγRIIc.

Constant regions containing a selective FcγR-binding domain which are included in the antigen-binding molecules of the present invention and antigen-binding molecules containing such a constant region may also be Fc regions and antigen-binding molecules containing such an Fc region which maintains or shows reduced binding activity to activating FcγR (FcγRIa, FcγRIb, FcγRIc, FcγRIIIa including allotype V158, FcγRIIIa including allotype F158, FcγRIIIb including allotype FcγRIIIb-NA1, FcγRIIIb including allotype FcγRIIIb-NA2, FcγRIIa including allotype H131, FcγRIIa including allotype R131, and/or FcγRIIc) when compared to an Fc region (an Fc region of the IgG class refers to the region from cysteine at position 226 (EU numbering) to the C terminus, or from proline at position 230 (EU numbering) to the C terminus, but is not limited thereto) constituting a portion of human IgG1 (SEQ ID NO: 14), IgG2 (SEQ ID NO: 15), IgG3 (SEQ ID NO: 16), or IgG4 (SEQ ID NO: 17) (hereinafter referred to as a wild-type Fc region) and an antigen-binding molecule containing such a wild-type Fc region.

Compared to a wild-type Fc region and an antigen-binding molecule containing a wild-type Fc region, the degree of the aforementioned reduction in binding activity to activating FcγR of an Fc region containing a selective FcγR-binding domain included in an antigen-binding molecule of the present invention, and an antigen-binding molecule containing such an Fc region is, for example, 99% or less, 98% or less, 97% or less, 96% or less, 95% or less, 94% or less, 93% or less, 92% or less, 91% or less, 90% or less, 88% or less, 86% or less, 84% or less, 82% or less, 80% or less, 78% or less, 76% or less, 74% or less, 72% or less, 70% or less, 68% or less, 66% or less, 64% or less, 62% or less, 60% or less, 58% or less, 56% or less, 54% or less, 52% or less, 50% or less, 45% or less, 40% or less, 35% or less, 30% or less, 25% or less, 20% or less, 15% or less, 10% or less, 5% or less, 4% or less, 3% or less, 2% or less, 1% or less, 0.5% or less, 0.4% or less, 0.3% or less, 0.2% or less, 0.1% or less, 0.05% or less, 0.01% or less, or 0.005% or less.

The Fc regions containing a selective FcyR-binding domain and constant regions containing such an Fc region, and antigen-binding molecules containing such a constant region, which are included in the antigen-binding molecules of the present invention, may also be Fc regions and antigen-binding molecules containing such an Fc region which shows enhanced binding activity to inhibitory FcγR (FcγRIIb-1 and/or FcγRIIb-2) when compared to an Fc region (an Fc region of the IgG class refers to the region from cysteine at position 226 (EU numbering) to the C terminus, or from proline at position 230 (EU numbering) to the C terminus, but is not limited thereto) constituting a portion of a constant region presented as human IgG1 (SEQ ID NO: 14), IgG2 (SEQ ID NO: 15), IgG3 (SEQ ID NO: 16), or IgG4 (SEQ ID NO: 17) (hereinafter referred to as a wild-type Fc region) and an antigen-binding molecule containing such a wild-type Fc region.

Compared to a wild-type Fc region and an antigen-binding molecule containing a wild-type Fc region, the degree of the aforementioned enhancement in binding activity to inhibitory FcγR of an Fc region containing a selective FcγR-binding domain included in an antigen-binding molecule of the present invention and an antigen-binding molecule containing such an Fc region is, for example, 101% or greater, 102% or greater, 103% or greater, 104% or greater, 105% or greater, 106% or greater, 107% or greater, 108% or greater, 109% or greater, 110% or greater, 112% or greater, 114% or greater, 116% or greater, 118% or greater, 120% or greater, 122% or greater, 124% or greater, 126% or greater, 128% or greater, 130% or greater, 132% or greater, 134% or greater, 136% or greater, 138% or greater, 140% or greater, 142% or greater, 144% or greater, 146% or greater, 148% or greater, 150% or greater, 155% or greater, 160% or greater, 165% or greater, 170% or greater, 175% or greater, 180% or greater, 185% or greater, 190% or greater, 195% or greater, 2-fold or greater, 3-fold or greater, 4-fold or greater, 5-fold or greater, 6-fold or greater, 7-fold or greater, 8-fold or greater, 9-fold or greater, 10-fold or greater, 20-fold or greater, 30-fold or greater, 40-fold or greater, 50-fold or greater, 60-fold or greater, 70-fold or greater, 80-fold or greater, 90-fold or greater, 100-fold or greater, 200-fold or greater, 300-fold or greater, 400-fold or greater, 500-fold or greater, 600-fold or greater, 700-fold or greater, 800-fold or greater, 900-fold or greater, 1000-fold or greater, 10000-fold or greater, or 100000-fold or greater.

Furthermore, the Fc region containing a selective FcyR-binding domain included in an antigen-binding molecule of the present invention and the antigen-binding molecule containing such an Fc region may be an Fc region and an antigen-binding molecule containing such an Fc region which maintains or shows reduced binding activity to activating FcyR (FcyRIa, FcyRIb, FcyRIc, FcγRIIIa including allotype V158, FcγRIIIa including allotype F158, FcyRIIIb including allotype FcγRIIIb-NA1, FcyRIIIb including allotype FcγRIIIb-NA2, FcyRIIa including allotype H131, FcyRIIa including allotype R131, and/or FcγRIIc) when compared to an Fc region (an Fc region of the IgG class refers to, for example, the region from cysteine at position 226 (EU numbering) to the C terminus or from proline at position 230 (EU numbering) to the C terminus, but is not limited thereto) constituting a portion of a constant region presented as human IgG1 (SEQ ID NO: 14), IgG2 (SEQ ID NO: 15), IgG3 (SEQ ID NO: 16), or IgG4 (SEQ ID NO: 17) (hereinafter referred to as a wild-type Fc region) and an antigen-binding molecule containing such a wild-type Fc region; and shows enhanced binding activity to inhibitory FcγR (FcγRIIb-1 and/or FcγRIIb-2) when compared to an Fc region (an Fc region of the IgG class refers to, for example, the region from cysteine at position 226 (EU numbering) to the C terminus or from proline at position 230 (EU numbering) to the C terminus, but is not limited thereto) constituting a portion of a constant region presented as human IgG1 (SEQ ID NO: 14), IgG2 (SEQ ID NO: 15), IgG3 (SEQ ID NO: 16), or IgG4 (SEQ ID NO: 17) (hereinafter referred to as a wild-type Fc region) and an antigen-binding molecule containing such a wild-type Fc region.

Furthermore, the Fc region containing a selective FcγR-binding domain included in an antigen-binding molecule of the present invention and the antigen-binding molecule containing such an Fc region may be an Fc region and an antigen-binding molecule containing such an Fc region with higher degree of enhancement of binding activity to an inhibitory Fcγ receptor (FcγRIIb-1 and/or FcγRIIb-2) than to an activating Fcγ receptor (FcγRIa, FcγRIb, FcγRIc, FcγRIIIa including allotype V158, FcγRIIIa including allotype F158, FcγRIIIb including allotype FcγRIIIb-NA1, FcγRIIIb including allotype FcγRIIIb-NA2, FcγRIIa including allotype H131, FcγRIIa including allotype R131), when compared to an Fc region (an Fc region of the IgG class refers to, for example, the region from cysteine at position 226 (EU numbering) to the C terminus or from proline at position 230 (EU numbering) to the C terminus, but is not limited thereto) constituting a portion of a constant region presented as human IgG1 (SEQ ID NO: 14), IgG2 (SEQ ID NO: 15), IgG3 (SEQ ID NO: 16), or IgG4 (SEQ ID NO: 17) (hereinafter referred to as a wild-type Fc region) and an antigen-binding molecule containing such a wild-type Fc region.

In the present invention, at least another alteration to the Fc region may be added to the Fc region in which amino acid at position 238 (EU numbering) is Asp and the Fc region in which amino acid at position 328 (EU numbering) is Glu, by the embodiments and such described in the aforementioned section on amino acid alterations. In addition to these alterations, additional alterations may also be added. The additional alterations can be selected from any of substitutions, deletions, and modifications of an amino acid, and combinations thereof. For example, alterations that enhance binding activity to FcγRIIb while maintaining or reducing binding activity to FcγRIIa (H type) and FcγRIIa (R type) may be added. Addition of such alterations improves binding selectivity to FcγRIIb over FcγRIIa.

Among these, alterations that improve binding selectivity to FcγRIIb over FcγRIIa (R type) is favorable, and alterations that improve binding selectivity to FcγRIIb over FcγRIIa (H type) is more favorable. Examples of preferred amino acid substitutions for such alterations include: an alteration by substituting Gly at position 237 (EU numbering) with Trp; an alteration by substituting Gly at position 237 (EU numbering) with Phe; an alteration by substituting Pro at position 238 (EU numbering) with Phe; an alteration by substituting Asn at position 325 (EU numbering) with Met; an alteration by substituting Ser at position 267 (EU numbering) with Ile; an alteration by substituting Leu at position 328 (EU numbering) with Asp; an alteration by substituting Ser at position 267 (EU numbering) with Val; an alteration by substituting Leu at position 328 (EU numbering) with Trp; an alteration by substituting Ser at position 267 (EU numbering) with Gln; an alteration by substituting Ser at position 267 (EU numbering) with Met; an alteration by substituting Gly at position 236 (EU numbering) with Asp; an alteration by substituting Ala at position 327 (EU numbering) with Asn; an alteration by substituting Asn at position 325 (EU numbering) with Ser; an alteration by substituting Leu at position 235 (EU numbering) with Tyr; an alteration by substituting Val at position 266 (EU numbering) with Met; an alteration by substituting Leu at position 328 (EU numbering) with Tyr; an alteration by substituting Leu at position 235 (EU numbering) with Trp; an alteration by substituting Leu at position 235 (EU numbering) with Phe; an alteration by substituting Ser at position 239 (EU numbering) with Gly; an alteration by substituting Ala at position 327 (EU numbering) with Glu; an alteration by substituting Ala at position 327 (EU numbering) with Gly; an alteration by substituting Pro at position 238 (EU numbering) with Leu; an alteration by substituting Ser at position 239 (EU numbering) with Leu; an alteration by substituting Leu at position 328 (EU numbering) with Thr; an alteration by substituting Leu at position 328 (EU numbering) with Ser; an alteration by substituting Leu at position 328 (EU numbering) with Met; an alteration by substituting Pro at position 331 (EU numbering) with Trp; an alteration by substituting Pro at position 331 (EU numbering) with Tyr; an alteration by substituting Pro at position 331 (EU numbering) with Phe; an alteration by substituting Ala at position 327 (EU numbering) with Asp; an alteration by substituting Leu at position 328 (EU numbering) with Phe; an alteration by substituting Pro at position 271 (EU numbering) with Leu; an alteration by substituting Ser at position 267 (EU numbering) with Glu; an alteration by substituting Leu at position 328 (EU numbering) with Ala; an alteration by substituting Leu at position 328 (EU numbering) with Ile; an alteration by substituting Leu at position 328 (EU numbering) with Gln; an alteration by substituting Leu at position 328 (EU numbering) with Val; an alteration by substituting Lys at position 326 (EU numbering) with Trp; an alteration by substituting Lys at position 334 (EU numbering) with Arg; an alteration by substituting His at position 268 (EU numbering) with Gly; an alteration by substituting His at position 268 (EU numbering) with Asn; an alteration by substituting Ser at position 324 (EU numbering) with Val; an alteration by substituting Val at position 266 (EU numbering) with Leu; an alteration by substituting Pro at position 271 (EU numbering) with Gly; an alteration by substituting Ile at position 332 (EU numbering) with Phe; an alteration by substituting Ser at position 324 (EU numbering) with Ile; an alteration by substituting Glu at position 333 (EU numbering) with Pro; an alteration by substituting Tyr at position 300 (EU numbering) with Asp; an alteration by substituting Ser at position 337 (EU numbering) with Asp; an alteration by substituting Tyr at position 300 (EU numbering) with Gln; an alteration by substituting Thr at position 335 (EU numbering) with Asp; an alteration by substituting Ser at position 239 (EU numbering) with Asn; an alteration by substituting Lys at position 326 (EU numbering) with Leu; an alteration by substituting Lys at position 326 (EU numbering) with Ile; an alteration by substituting Ser at position 239 (EU numbering) with Glu; an alteration by substituting Lys at position 326 (EU numbering) with Phe; an alteration by substituting Lys at position 326 (EU numbering) with Val; an alteration by substituting Lys at position 326 (EU numbering) with Tyr; an alteration by substituting Ser at position 267 (EU numbering) with Asp; an alteration by substituting Lys at position 326 (EU numbering) with Pro; an alteration by substituting Lys at position 326 (EU numbering) with His; an alteration by substituting Lys at position 334 (EU numbering) with Ala; an alteration by substituting Lys at position 334 (EU numbering) with Trp; an alteration by substituting His at position 268 (EU numbering) with Gln; an alteration by substituting Lys at position 326 (EU numbering) with Gln; an alteration by substituting Lys at position 326 (EU numbering) with Glu; an alteration by substituting Lys at position 326 (EU numbering) with Met; an alteration by substituting Val at position 266 (EU numbering) with Ile; an alteration by substituting Lys at position 334 (EU numbering) with Glu; an alteration by substituting Tyr at position 300 (EU numbering) with Glu; an alteration by substituting Lys at position 334 (EU numbering) with Met; an alteration by substituting Lys at position 334 (EU numbering) with Val; an alteration by substituting Lys at position 334 (EU numbering) with Thr; an alteration by substituting Lys at position 334 (EU numbering) with Ser; an alteration by substituting Lys at position 334 (EU numbering) with His; an alteration by substituting Lys at position 334 (EU numbering) with Phe; an alteration by substituting Lys at position 334 (EU numbering) with Gln; an alteration by substituting Lys at position 334 (EU numbering) with Pro; an alteration by substituting Lys at position 334 (EU numbering) with Tyr; an alteration by substituting Lys at position 334 (EU numbering) with Ile; an alteration by substituting Gln at position 295 (EU numbering) with Leu; an alteration by substituting Lys at position 334 (EU numbering) with Leu; an alteration by substituting Lys at position 334 (EU numbering) with Asn; an alteration by substituting His at position 268 (EU numbering) with Ala; an alteration by substituting Ser at position 239 (EU numbering) with Asp; an alteration by substituting Ser at position 267 (EU numbering) with Ala; an alteration by substituting Leu at position 234 (EU numbering) with Trp; an alteration by substituting Leu at position 234 (EU numbering) with Tyr; an alteration by substituting Gly at position 237 (EU numbering) with Ala; an alteration by substituting Gly at position 237 (EU numbering) with Asp; an alteration by substituting Gly at position 237 (EU numbering) with Glu; an alteration by substituting Gly at position 237 (EU numbering) with Leu; an alteration by substituting Gly at position 237 (EU numbering) with Met; an alteration by substituting Gly at position 237 (EU numbering) with Tyr; an alteration by substituting Ala at position 330 (EU numbering) with Lys; an alteration by substituting Ala at position 330 (EU numbering) with Arg; an alteration by substituting Glu at position 233 (EU numbering) with Asp; an alteration by substituting His at position 268 (EU numbering) with Asp; an alteration by substituting His at position 268 (EU numbering) with Glu; an alteration by substituting Lys at position 326 (EU numbering) with Asp; an alteration by substituting Lys at position 326 (EU numbering) with Ser; an alteration by substituting Lys at position 326 (EU numbering) with Thr; an alteration by substituting Val at position 323 (EU numbering) with Ile; an alteration by substituting Val at position 323 (EU numbering) with Leu; an alteration by substituting Val at position 323 (EU numbering) with Met; an alteration by substituting Tyr at position 296 (EU numbering) with Asp; an alteration by substituting Lys at position 326 (EU numbering) with Ala; an alteration by substituting Lys at position 326 (EU numbering) with Asn; and an alteration by substituting Ala at position 330 (EU numbering) with Met.

Favorable amino acid substitutions among these alterations are, for example, an alteration by substituting Gly at position 237 (EU numbering) with Trp; an alteration by substituting Gly at position 237 (EU numbering) with Phe; an alteration by substituting Ser at position 267 (EU numbering) with Val; an alteration by substituting Ser at position 267 (EU numbering) with Gln; an alteration by substituting His at position 268 (EU numbering) with Asn; an alteration by substituting Pro at position 271 (EU numbering) with Gly; an alteration by substituting Lys at position 326 (EU numbering) with Leu; an alteration by substituting Lys at position 326 (EU numbering) with Gln; an alteration by substituting Lys at position 326 (EU numbering) with Glu; an alteration by substituting Lys at position 326 (EU numbering) with Met; an alteration by substituting Ser at position 239 (EU numbering) with Asp; an alteration by substituting Ser at position 267 (EU numbering) with Ala; an alteration by substituting Leu at position 234 (EU numbering) with Trp; an alteration by substituting Leu at position 234 (EU numbering) with Tyr; an alteration by substituting Gly at position 237 (EU numbering) with Ala; an alteration by substituting Gly at position 237 (EU numbering) with Asp; an alteration by substituting Gly at position 237 (EU numbering) with Glu; an alteration by substituting Gly at position 237 (EU numbering) with Leu; an alteration by substituting Gly at position 237 (EU numbering) with Met; an alteration by substituting Gly at position 237 (EU numbering) with Tyr; an alteration by substituting Ala at position 330 (EU numbering) with Lys; an alteration by substituting Ala at position 330 (EU numbering) with Arg; an alteration by substituting Glu at position 233 (EU numbering) with Asp; an alteration by substituting His at position 268 (EU numbering) with Asp; an alteration by substituting His at position 268 (EU numbering) with Glu; an alteration by substituting Lys at position 326 (EU numbering) with Asp; an alteration by substituting Lys at position 326 (EU numbering) with Ser; an alteration by substituting Lys at position 326 (EU numbering) with Thr; an alteration by substituting Val at position 323 (EU numbering) with Ile; an alteration by substituting Val at position 323 (EU numbering) with Leu; an alteration by substituting Val at position 323 (EU numbering) with Met; an alteration by substituting Tyr at position 296 (EU numbering) with Asp; an alteration by substituting Lys at position 326 (EU numbering) with Ala; an alteration by substituting Lys at position 326 (EU numbering) with Asn; and an alteration by substituting Ala at position 330 (EU numbering) with Met.

The above-mentioned alteration may be at one position, or alterations at two or more positions may be combined. Favorable examples of such alterations are those described in Tables 14 to 15, Tables 17 to 24, and Tables 26 to 28.

Fc region produced by altering the FcyR-binding domain included in the Fc region presented as human IgG1 (SEQ ID NO: 14), IgG2 (SEQ ID NO: 15), IgG3 (SEQ ID NO: 16), or IgG4 (SEQ ID NO: 17) can be given as an example of another non-limiting embodiment of the selective FcyR-binding domain included in the antigen-binding molecules of the present invention. A method for producing the modified Fc regions is, for example, the method described in the above-mentioned section on amino acid alterations. Examples of such altered Fc regions include an Fc region in which amino acid at position 238 (EU numbering) is Asp and amino acid at position at 271 (EU numbering) is Gly in a human IgG (IgG1, IgG2, IgG3, or IgG4). An Fc region in which amino acid at position 238 (EU numbering) is Asp and amino acid at position at 271 (EU numbering) is Gly in a human IgG (IgG1, IgG2, IgG3, or IgG4), and antigen-binding molecules containing such an Fc region show higher binding activity to FcγRIIb-1 and/or FcγRIIb-2 than to FcγRIa, FcγRIb, FcγRIc, FcγRIIIa including allotype V158, FcγRIIIa including allotype F158, FcγRIIIb including allotype FcγRIIIb-NA1, FcγRIIIb including allotype FcγRIIIb-NA2, FcγRIIa including allotype H131, FcγRIIa including allotype R131, and/or FcyRIIc.

In the present invention, at least another alteration to the Fc region may be added to the Fc region in which amino acid at position 238 (EU numbering) is Asp and the amino acid at position 271 (EU numbering) is Gly, by the embodiments and such described in the aforementioned section on amino acid alterations. In addition to these alterations, additional alterations may also be added. The additional alterations can be selected from any of substitutions, deletions, and modifications of an amino acid, and combinations thereof. For example, alterations that maintain or reduce binding activity to activating Fcγ receptors (FcγRIa, FcγRIb, FcγRIc, FcγRIIIa including allotype V158, FcγRIIIa including allotype F158, FcγRIIIb including allotype FcγRIIIb-NA1, FcγRIIIb including allotype FcγRIIIb-NA2, FcγRIIa including allotype H131, FcγRIIa including allotype R131) can be added. Alterations that enhance binding activity to inhibitory Fcγ receptors (FcγRIIb-1 and/or FcγRIIb-2) while maintaining or reducing binding activity to FcγRIIa (H type) and FcγRIIa (R type) may be added. Furthermore, alterations where the degree of enhancement of binding activity to inhibitory Fcγ receptors (FcγRIIb-1 and/or FcγRIIb-2) is higher than the degree of enhancement of binding activity to activating Fcγ receptors (FcγRIa, FcγRIb, FcγRIc, FcγRIIIa including allotype V158, FcγRIIIa including allotype F158, FcγRIIIb including allotype FcγRIIIb-NA1, FcγRIIIb including allotype FcγRIIIb-NA2, FcγRIIa including allotype H131, FcγRIIa including allotype R131) may also be added. Addition of such alterations improves binding selectivity to FcγRIIb over FcγRIIa.

An example of a non-limiting embodiment of the altered Fc region comprising a selective FcγR-binding domain includes an altered Fc region in which any one or more of positions 233, 234, 237, 264, 265, 266, 267, 268, 269, 272, 296, 326, 327, 330, 331, 332, 333, and 396 (EU numbering) are substituted in the Fc region in which amino acid at position 238 (EU numbering) is Asp and amino acid at position 271 (EU numbering) is Gly in a human IgG (IgG1, IgG2, IgG3, or IgG4).

In addition, an example of a non-limiting embodiment of the altered Fc region comprising a selective FcγR-binding domain is an altered Fc region comprising any one or more of

Asp at amino acid position 233,
Tyr at amino acid position 234,
Asp at amino acid position 237,
Ile at amino acid position 264,
Glu at amino acid position 265,
any one of Phe, Met, and Leu at amino acid position 266,
any one of Ala, Glu, Gly, and Gln at amino acid position 267,
Asp or Glu at amino acid position 268,
Asp at amino acid position 269,
any one of Asp, Phe, Ile, Met, Asn, and Gln at amino acid position 272,
Asp at amino acid position 296,
Ala or Asp at amino acid position 326,
Gly at amino acid position 327,
Lys or Arg at amino acid position 330,
Ser at amino acid position 331,
Thr at amino acid position 332,
any one of Thr, Lys, and Arg at amino acid position 333,
any one of Asp, Glu, Phe, Ile, Lys, Leu, Met, Gln, Arg, and Tyr at amino acid position 396, shown by EU numbering, in the Fc region in which amino acid at position 238 is Asp and amino acid at position 271 (EU numbering) is Gly in a human IgG (IgG1, IgG2, IgG3, or IgG4).

Examples of a non-limiting embodiment of Fc region which further comprises at least another alteration to the Fc region and further comprises additional alterations mentioned above include Fc regions shown in Tables 6-1 to 6-7.

[Table 6-1]

| ALTERED Fc REGION | ALTERED AMINO ACID (EU NUMBERING) |
|---|---|
| BP208 | E233D/G237D/P238D/H268D/P271G/A330R |
| BP209 | G237D/P238D/H268D/P271G/K326A/A330R |
| BP210 | G237D/P238D/H268D/P271G/A330R |
| BP211 | E233D/P238D/H268D/P271G/K326A/A330R |
| BP212 | E233D/P238D/H268D/P271G/Y296D/A330R |
| BP213 | E233D/P238D/H268D/P271G/A330R |
| BP214 | E233D/L234Y/G237D/P238D/Y296D/K326D/A330K |
| BP215 | G237D/P238D/H268D/P271G/Y296D/A330K |
| BP216 | G237D/P238D/S267Q/H268D/P271G/A330K |
| BP217 | G237D/P238D/S267Q/H268D/P271G/Y296D/A330K |
| BP218 | G237D/P238D/H268D/P271G/K326D/A330K |
| BP219 | L234Y/G237D/P238D/H268D/P271G/A330K |
| BP220 | E233D/G237D/P238D/H268D/P271G/Y296D/A330K |
| BP221 | L234Y/G237D/P238D/Y296D/K326A/A330R |
| BP222 | L234Y/G237D/P238D/P271G/K326A/A330R |
| BP223 | L234Y/G237D/P238D/H268D/P271G/K326A/A330R |
| BP224 | L234Y/G237D/P238D/S267Q/H268D/P271G/K326A/A330R |
| BP225 | L234Y/G237D/P238D/K326D/A330R |
| BP226 | L234Y/G237D/P238D/P271G/K326D/A330R |
| BP227 | L234Y/G237D/P238D/H268D/P271G/K326D/A330R |
| BP228 | L234Y/G237D/P238D/S267Q/H268D/P271G/K326D/A330R |
| BP229 | E233D/L234Y/G237D/P238D/P271G/K326A/A330R |
| BP230 | E233D/G237D/P238D/H268D/P271G/Y296D/A330R |
| BP231 | G237D/P238D/H268D/P271G/Y296D/A330R |
| BP232 | L234Y/G237D/P238D/P271G/K326A/A330K |
| BP233 | L234Y/G237D/P238D/P271G/A330K |
| BP234 | E233D/L234Y/G237D/P238D/S267Q/H268D/P271G/Y296D/K326D/A330K |
| BP235 | E233D/L234Y/G237D/P238D/H268D/P271G/Y296D/K326D/A330R |
| BP236 | E233D/L234Y/G237D/P238D/S267Q/H268D/P271G/Y296D/K326D/A330R |
| BP237 | E233D/L234Y/G237D/P238D/S267Q/H268D/P271G/Y296D/K326A/A330K |

(Table 6-2 is a continuation table of Table 6-1.)

[Table 6-2]

| ALTERED Fc REGION | ALTERED AMINO ACID (EU NUMBERING) |
|---|---|
| BP238 | E233D/L234Y/G237D/P238D/H268D/P271G/Y296D/K326A/A330R |
| BP239 | E233D/L234Y/G237D/P238D/S267Q/H268D/P271G/Y296D/K326A/A330R |

(continued)

| ALTERED Fc REGION | ALTERED AMINO ACID (EU NUMBERING) |
|---|---|
| BP240 | E233D/G237D/P238D/S267Q/H268D/P271G/A330R |
| BP241 | E233D/G237D/P238D/H268D/P271G/K326D/A330R |
| BP242 | E233D/G237D/P238D/H268D/P271G/K326A/A330R |
| BP243 | E233D/L234Y/G237D/P238D/H268D/P271G/A330R |
| BP244 | E233D/G237D/P238D/S267Q/H268D/P271G/Y296D/A330R |
| BP245 | E233D/G237D/P238D/S267Q/H268D/P271G/Y296D/K326D/A330R |
| BP246 | E233D/G237D/P238D/S267Q/H268D/P271G/Y296D/K326A/A330R |
| BP247 | E233D/G237D/P238D/H268D/P271G/Y296D/K326D/A330R |
| BP248 | E233D/G237D/P238D/H268D/P271G/Y296D/K326A/A330R |
| BP249 | E233D/L234Y/G237D/P238D/H268D/P271G/Y296D/A330R |
| BP262 | G237D/P238D/H268E/P271G |
| BP264 | E233D/G237D/P238D/H268E/P271G/Y296D/A330R |
| BP265 | G237D/P238D/H268E/P271G/Y296D/A330R |
| BP266 | E233D/G237D/P238D/H268E/P271G/A330R |
| BP267 | E233D/G237D/P238D/H268E/P271G |
| BP268 | E233D/G237D/P238D/H268E/P271G/Y296D |
| BP269 | G237D/P238D/H268E/P271G/Y296D |
| BP300 | E233D/G237D/P238D/V264I/H268E/P271G |
| BP313 | E233D/G237D/P238D/D265E/H268E/P271G |
| BP333 | E233D/G237D/P238D/V266F/H268E/P271G |
| BP338 | E233D/G237D/P238D/V266L/H268E/P271G |
| BP339 | E233D/G237D/P238D/V266M/H268E/P271G |
| BP348 | E233D/G237D/P238D/S267A/H268E/P271G |
| BP350 | E233D/G237D/P238D/S267E/H268E/P271G |
| BP352 | E233D/G237D/P238D/S267G/H268E/P271G |
| BP367 | E233D/G237D/P238D/H268E/E269D/P271G |
| BP384 | E233D/G237D/P238D/H268D/P271G/Y296D/ A330R/K334R |
| BP390 | E233D/G237D/P238D/H268D/P271G/Y296D/A330R/I332S |
| BP391 | E233D/G237D/P238D/H268D/P271G/Y296D/A330R/I332T |

(Table 6-3 is a continuation table of Table 6-2.)

[Table 6-3]

| ALTERED Fc REGION | ALTERED AMINO ACID (EU NUMBERING) |
|---|---|
| BP392 | E233D/G237D/P238D/H268D/P271G/Y296D/A330R/I332K |
| BP393 | E233D/G237D/P238D/H268D/P271G/Y296D/A330R/I332R |
| BP423 | E233D/G237D/P238D/S267A/H268E/P271G/A330R |
| BP425 | E233D/G237D/P238D/V266L/S267A/H268E/P271G/A330R |
| BP426 | E233D/G237D/P238D/S267A/H268E/E269D/P271G/A330R |

(continued)

| ALTERED Fc REGION | ALTERED AMINO ACID (EU NUMBERING) |
|---|---|
| BP427 | E233D/G237D/P238D/S267A/H268E/E269Y/P271G/A330R |
| BP428 | E233D/G237D/P238D/S267G/H268E/P271G/A330R |
| BP429 | E233D/G237D/P238D/V264I/S267G/H268E/P271G/A330R |
| BP430 | E233D/G237D/P238D/V266L/S267G/H268E/P271G/A330R |
| BP431 | E233D/G237D/P238D/S267G/H268E/E269D/P271G/A330R |
| BP432 | E233D/G237D/P238D/S267G/H268E/E269Y/P271G/A330R |
| BP433 | E233D/G237D/P238D/H268D/P271G/Y296D/A330K/I332T |
| BP434 | E233D/G237D/P238D/H268D/P271G/Y296D/K326D/A330R/I332T |
| BP435 | E233D/G237D/P238D/H268D/P271G/Y296D/K326A/A330R/I332T |
| BP436 | E233D/G237D/P238D/S267A/H268E/P271G/Y296D/A330R/I332T |
| BP437 | G237D/P238D/S267A/H268E/P271G/Y296D/A330R/I332T |
| BP438 | E233D/G237D/P238D/S267A/H268E/P271G/A330R/I332T |
| BP439 | E233D/G237D/P238D/V264I/V266L/S267A/H268E/P271G/A330R |
| BP440 | E233D/G237D/P238D/V264I/H268E/P271G/A330R |
| BP441 | E233D/G237D/P238D/V266L/H268E/P271G/A330R |
| BP442 | E233D/G237D/P238D/H268E/E269D/P271G/A330R |
| BP443 | E233D/G237D/P238D/V266L/H268E/E269D/P271G/A330R |
| BP444 | E233D/G237D/P238D/H268E/E269N/P271G/A330R |
| BP445 | E233D/G237D/P238D/V264I/S267A/H268E/P271G/A330R |
| BP446 | E233D/G237D/P238D/S267A/H268E/E269N/P271G/A330R |
| BP447 | E233D/G237D/P238D/S267A/H268E/P271G/A330R/P396A |
| BP448 | E233D/G237D/P238D/S267A/H268E/P271G/A330R/P396D |
| BP449 | E233D/G237D/P238D/S267A/H268E/P271G/A330R/P396E |
| BP450 | E233D/G237D/P238D/S267A/H268E/P271G/A330R/P396F |
| BP451 | E233D/G237D/P238D/S267A/H268E/P271G/A330R/P396G |
| BP452 | E233D/G237D/P238D/S267A/H268E/P271G/A330R/P396H |

(Table 6-4 is a continuation table of Table 6-3.)

[Table 6-4]

| ALTERED Fc REGION | ALTERED AMINO ACID (EU NUMBERING) |
|---|---|
| BP453 | E233D/G237D/P238D/S267A/H268E/P271G/A330R/P396I |
| BP454 | E233D/G237D/P238D/S267A/H268E/P271G/A330R/P396K |
| BP455 | E233D/G237D/P238D/S267A/H268E/P271G/A330R/P396L |
| BP456 | E233D/G237D/P238D/S267A/H268E/P271G/A330R/P396M |
| BP457 | E233D/G237D/P238D/S267A/H268E/P271G/A330R/P396N |
| BP458 | E233D/G237D/P238D/S267A/H268E/P271G/A330R/P396Q |

(continued)

| ALTERED Fc REGION | ALTERED AMINO ACID (EU NUMBERING) |
|---|---|
| BP459 | E233D/G237D/P238D/S267A/H268E/P271G/A330R/P396R |
| BP460 | E233D/G237D/P238D/S267A/H268E/P271G/A330R/P396S |
| BP461 | E233D/G237D/P238D/S267A/H268E/P271G/A330R/P396T |
| BP462 | E233D/G237D/P238D/S267A/H268E/P271G/A330R/P396V |
| BP463 | E233D/G237D/P238D/S267A/H268E/P271G/A330R/P396W |
| BP464 | E233D/G237D/P238D/S267A/H268E/P271G/A330R/P396Y |
| BP465 | E233D/G237D/P238D/H268D/P271G/Y296D/A330R/E333K |
| BP466 | E233D/G237D/P238D/H268D/P271G/Y296D/A330R/E333R |
| BP467 | E233D/G237D/P238D/H268D/P271G/Y296D/A330R/E334S |
| BP468 | E233D/G237D/P238D/H268D/P271G/Y296D/A330R/E334T |
| BP469 | E233D/G237D/P238D/H268D/P271G/Y296D/A330R/E333S |
| BP470 | E233D/G237D/P238D/H268D/P271G/Y296D/A330R/E333T |
| BP471 | E233D/G237D/P238D/H268D/P271G/Y296D/A330R/P331S |
| BP472 | E233D/G237D/P238D/H268D/P271G/Y296D/A330S |
| BP473 | E233D/G237D/P238D/H268D/P271G/Y296D/A327G/A330R |
| BP474 | E233D/G237D/P238D/H268D/P271G/Y296D/A330R/P331S |
| BP475 | E233D/G237D/P238D/H268D/P271G/Y296D/A327G/A330S |
| BP476 | E233D/G237D/P238D/H268D/P271G/Y296D/A327G/A330S/P331S |
| BP477 | E233D/G237D/P238D/H268D/P271G/Y296D/A327G/A330R/P331S |
| BP478 | E233D/G237D/P238D/H268D/P271G/Y296D/A330R + S131C/K133R/G137E/G138S/Q196K/I199T/N203D/K214R/P217S + 219-221 DELETION + K222Y/T223G/H224P/T225P |
| BP479 | E233D/G237D/P238D/V264I/V266L/S267A/H268E/P271G |
| BP480 | E233D/G237D/P238D/V266L/H268E/E269D/P271G |
| BP481 | E233D/G237D/P238D/V264I/S267A/H268E/P271G |

(Table 6-5 is a continuation table of Table 6-4.)

[Table 6-5]

| ALTERED Fc REGION | ALTERED AMINO ACID (EU NUMBERING) |
|---|---|
| BP482 | E233D/G237D/P238D/S267A/H268E/E269N/P271G |
| BP483 | E233D/G237D/P238D/V266L/S267A/H268E/P271G |
| BP484 | E233D/G237D/P238D/S267A/H268E/E269D/P271G |
| BP485 | E233D/G237D/P238D/S267A/H268E/E269Y/P271G |
| BP487 | E233D/G237D/P238D/V264I/S267A/H268E/P271G/A330R/P396M |
| BP488 | E233D/G237D/P238D/V264I/S267A/H268E/P271G/Y296D/A330R |
| BP489 | E233D/G237D/P238D/V264I/S267A/H268E/P271G/Y296D/A330R/P396M |
| BP490 | G237D/P238D/V264I/S267A/H268E/P271G/A330R |

(continued)

| ALTERED Fc REGION | ALTERED AMINO ACID (EU NUMBERING) |
|---|---|
| BP491 | G237D/P238D/V264I/S267A/H268E/P271G/Y296D/A330R |
| BP492 | P238D/V264I/S267A/H268E/P271G |
| BP493 | P238D/V264I/S267A/H268E/P271G/Y296D |
| BP494 | G237D/P238D/S267A/H268E/P271G/Y296D/A330R |
| BP495 | G237D/P238D/S267G/H268E/P271G/Y296D/A330R |
| BP496 | E233D/G237D/P238D/V264I/S267A/H268E/P271G/Y296D |
| BP497 | E233D/G237D/P238D/V264I/S267A/H268E/P271G/A327G/A330R |
| BP498 | E233D/G237D/P238D/V264I/S267A/H268E/P271G/A330R/P396L |
| BP499 | E233D/G237D/P238D/V264I/S267A/H268E/P271G/Y296D/A330R/P396L |
| BP500 | G237D/P238D/V264I/S267A/H268E/P271G/Y296D |
| BP501 | G237D/P238D/V264I/S267A/H268E/P271G |
| BP502 | E233D/G237D/P238D/V264I/S267A/H268E/P271G/Y296D/A327G/A330R |
| BP503 | E233D/G237D/P238D/V264I/S267A/H268E/P271G/Y296D/A327G/A330R/P396M |
| BP504 | E233D/G237D/P238D/V264I/S267A/H268E/P271G/E272P |
| BP505 | E233D/G237D/P238D/V264I/S267A/H268E/P271G/E272D |
| BP506 | E233D/G237D/P238D/V264I/S267A/H268E/P271G/E272P/Y296D/A330R |
| BP507 | E233D/G237D/P238D/V264I/S267A/H268E/P271G/E272P/A330R |
| BP508 | E233D/G237D/P238D/V264I/S267A/H268E/P271G/E272P/Y296D |
| BP509 | E233D/G237D/P238D/V264I/S267A/H268E/P271G/E272D/Y296D |
| BP510 | G237D/P238D/V264I/S267A/H268E/P271G/E272P/A330R |
| BP511 | G237D/P238D/V264I/S267A/H268E/P271G/E272P/Y296D/A330R |
| BP513 | E233D/G237D/P238D/H268E/E272D/P271G |

(Table 6-6 is a continuation table of Table 6-5.)

[Table 6-6]

| ALTERED Fc REGION | ALTERED AMINO ACID (EU NUMBERING) |
|---|---|
| BP514 | H233D/G237D/P238D/H268E/E272F/P271G |
| BP517 | E233D/G237D/P238D/H268E/E272I/P271G |
| BP521 | E233D/G237D/P238D/H268H/E272N/P271G |
| BP521 | E233D/G237D/P238D/H268F/E272Q/P271G |
| BP531 | E233D/G237D/P238D/V2641/S267G/H268E/P271G/Y296D/A330R/P396M |
| BP532 | E233D/G237D/P238D/V264I/H268E/P271G/Y296D/A330R/P396M |
| BP533 | E233D/G237D/P238D/V264I/S267G/H268E/P271G/Y296D/A330R/P396L |
| BP534 | E233D/G237D/P238D/V264I/H268E/P271G/Y296D/A330R/P396L |
| BP535 | E233D/G237D/P238D/V264I/S267G/H268E/P271G/Y296D/A327G/A330R/P396M |
| BP536 | E233D/G237D/P238D/V264I/H268E/P271G/Y296D/A327G/A330R/P396M |
| BP537 | G237D/P238D/V264I/S267G/H268E/P271G/A330R |
| BP538 | G237D/P238D/V264I/H268E/P271G/A330R |

(continued)

| ALTERED Fc REGION | ALTERED AMINO ACID (EU NUMBERING) |
|---|---|
| BP539 | G237D/P238D/V264I/S267G/H268E/P271G/E272P/Y296D/A330R |
| BP540 | G237D/P238D/V264I/H268E/P271G/E272P/Y296D/A330R |
| BP541 | E233D/G237D/P238D/H268D/P271G/K274Q/Y296D/A330R |
| BP542 | E233D/G237D/P238D/H268D/P271G/Y296F/A330R |
| BP543 | E233D/G237D/P238D/H268Q/P271G/Y296D/A330R |
| BP544 | E233D/G237D/P238D/H268D/P271G/Y296D/A330R/R355Q |
| BP545 | E233D/G237D/P238D/H268D/P271G/Y296D/A330R/D356E |
| BP546 | E233D/G237D/P239D/H268D/P271G/Y296D/A330R/L358M |
| BP547 | E233D/G237D/P238D/H268D/P271G/Y296D/A330R/K409R |
| BP548 | E233D/G237D/P238D/H268D/P271G/Y296D/A330R/Q419E |
| BP549 | C237D/P238D/S267G/H268R/P271G/A330R |
| BP550 | G237D/P238D/V264I/S267G/H268E/P271G/E272D/Y296D/A330R |
| BP551 | G237D/P238D/V264I/H268E/P271G/E272D/Y296D/A330R |
| BP552 | E233D/G237D/P238D/V264I/S267A/H268E/P271G/E272D/Y296D/A330R |
| BP553 | E233D/G237D/P238D/V264I/S267A/H268E/P271G/E272D/A330R |
| BP554 | G237D/P238D/V264I/S267A/H268E/P271G/E272D/A330R |
| BP555 | G237D/P238D/V264I/S267A/H268E/P271G/E272D/Y296D/A330R |
| BP556 | G237D/P238D/V264I/S267G/H268E/P271G/Y296D/A330R |
| ALTERED Fc REGION | ALTERED AMINO ACID (EU NUMBERING) |
| BP514 | E233D/G237D/P238D/H268E/E272F/P271G |
| BP517 | E233D/G237D/P238D/H268E/E272I/P271G |
| BP521 | E233D/G237D/P238D/H268E/E272N/P271G |
| BP521 | E233D/G237D/P238D/H268E/E272Q/P271G |
| BP531 | E233D/G237D/P238D/V264I/S267G/H268E/P271G/Y296D/A330R/P396M |
| BP532 | E233D/G237D/P238D/V264I/H268E/P271G/Y296D/A330R/P396M |
| BP533 | E233D/G237D/P238D/V264I/S267G/H268E/P271G/Y296D/A330R/P396L |
| BP534 | E233D/G237D/P238D/V264I/H268E/P271G/Y296D/A330R/P396L |
| BP535 | E233D/G237D/P238D/V264I/S267G/H268E/P271G/Y296D/A327G/A330R/P396M |
| BP536 | E233D/G237D/P238D/V264I/H268E/P271G/Y296D/A327G/A330R/P396M |
| BP537 | G237D/P238D/V264I/S267G/H268E/P271G/A330R |
| BP538 | G237D/P238D/V264I/H268E/P271G/A330R |
| BP539 | G237D/P238D/V264I/S267G/H268E/P271G/E272P/Y296D/A330R |
| BP540 | G237D/P238D/V264I/H268E/P271G/E272P/Y296D/A330R |
| BP541 | E233D/G237D/P238D/H268D/P271G/K274Q/Y296D/A330R |
| BP542 | E233D/G237D/P238D/H268D/P271G/Y296F/A330R |
| BP543 | E233D/G237D/P238D/H268Q/P271G/Y296D/A330R |
| BP544 | E233D/G237D/P238D/H268D/P271G/Y296D/A330R/R355Q |
| BP545 | E233D/G237D/P238D/H268D/P271G/Y296D/A330R/D356E |

(continued)

| ALTERED Fc REGION | ALTERED AMINO ACID (EU NUMBERING) |
|---|---|
| BP546 | E233D/G237D/P238D/H268D/P271G/Y296D/A330R/L358M |
| BP547 | E233D/G237D/P238D/H268D/P271G/Y296D/A330R/K409R |
| BP548 | E233D/G237D/P238D/H268D/P271G/Y296D/A330R/Q419E |
| BP549 | G237D/P238D/S267G/H268E/P271G/A330R |
| BP550 | G237D/P238D/V264I/S267G/H268E/P271G/E272D/Y296D/A330R |
| BP551 | G237D/P238D/V264I/H268E/P271G/E272D/Y296D/A330R |
| BP552 | E233D/G237D/P238D/V264I/S267A/H268E/P271G/E272D/Y296D/A330R |
| BP553 | E233D/G237D/P238D/V264I/S267A/H268E/P271G/E272D/A330R |
| BP554 | G237D/P238D/V264I/S267A/H268E/P271G/E272D/A330R |
| BP555 | G237D/P238D/V264I/S267A/H268E/P271G/E272D/Y296D/A330R |
| BP556 | G237D/P238D/V264I/S267G/H268E/P271G/Y296D/A330R |

(Table 6-7 is a continuation table of Table 6-6.)

[Table 6-7]

| ALTERED Fc REGION | ALTERED AMINO ACID (EU NUMBERING) |
|---|---|
| BP558 | G237D/P238D/V264I/S267G/H268E/P271G/E272D/A330R |
| BP559 | P238D/V264I/S267A/H268E/P271G/E272D/Y296D |
| BP560 | P238D/S267G/H268E/P271G/Y296D/A330R |
| BP561 | E233D/G237D/P238D/H268D/P271G/E272D/Y296D/A330R |
| BP562 | G237D/P238D/H268D/P271G/E272D/Y296D/ A330R |
| BP563 | E233D/G237D/P238D/H268E/P271G/E272D/Y296D/A330R |
| BP564 | G237D/P238D/H268E/P271G/E272D/Y296D/A330R |
| BP565 | E233D/G237D/P238D/S267A/H268E/P271G/Y296D/A330R |

Four types of FcγRs, FcγRI, FcγRIIb, FcγRIII, and FcγRIV, have been identified in mice. In humans as well, as corresponding FcγRs, FcγRI, FcγRIIa, FcγRIIb, FcγRIIIa, FcγRIIIa, and FcγRIIIb have been identified. FcγRIIb which is considered to be the only inhibitory type among these FcγRs is conserved in both humans and mice. The other FcγRs, except for FcγRIIIb, transmit activation signals *via* the immunoreceptor tyrosine-based activating motif (ITAM), whereas FcγRIIb transmits inhibitory signals *via* the immunoreceptor tyrosine-based inhibitory motif (ITIM) present inside the cells (Nat. Rev. Immunol. (2008) 8, 34-47).

FcγRIIb1 and FcγRIIb2 have been reported as splicing variants of FcγRIIb. In both humans and mice, FcγRIIb1 has a longer intracellular domain than FcγRIIb2. FcγRIIb1 has been confirmed to be expressed in B cells, and FcγRIIb2 has been confirmed to be expressed in macrophages, mast cells, dendritic cells, basophils, neutrophils, and eosinophils (J. Clin. Immunol. (2005) 25 (1), 1-18).

So far, in humans, dysfunction and decreased expression of FcγRIIb have been reported to be correlated with onset of autoimmune diseases. For example, it has been reported that in some SLE patients, binding of transcriptional activators is attenuated due to polymorphism in an expression promoter region of FcγRIIb, which results in the decreased FcγRIIb expression (Hum. Genet. (2005) 117, 220-227; J. Immunol. (2004) 172, 7192-7199; and J. Immunol. (2004) 172, 7186-7191). Furthermore, among SLE patients, two types of allotypes have been reported, where the amino acid at position 233 is Ile or Thr in FcγRIIb. This position exists in the transmembrane region of FcγRIIb, and it is reported that FcγRIIb is less likely to exist in the lipid raft when the amino acid at position 233 is Thr compared to when this amino acid is Ile, and as a result, signal transduction function of FcγRIIb decreases (Nat. Med. (2005) 11, 1056-1058; Hum. Mol. Genet., (2005) 14, 2881-2892). In mice as well, knockout mice produced by disrupting the FcγRIIb gene in C57BL/6 mice has been reported to present SLE-like symptoms such as autoantibody production and glomerulonephritis (Immunity 13 (2000) 277-285; J. Exp. Med. (2002) 195, 1167-1174). Furthermore, so far, reduced expression level of FcγRIIb and such have been reported in mice considered to be models with natural onset of SLE (Immunogenetics (2000) 51, 429-435; Int. Immunol. (1999) 11, 1685-1691; Curr. Biol. (2000) 10, 227-230; J. Immunol. (2002) 169, 4340-4346). From these reports, FcyRIIb is considered to regulate humoral immunity in mice as in humans.

When an antibody carrying an Fc of the present invention eliminates antigens *via* FcγRIIb, the endocytosis function of FcγRIIb is considered to be making the most important contribution among the functions of FcγRIIb. As described above, FcγRIIb1 and FcγRIIb2 exist as splicing variants of FcγRIIb, but it is reported that the latter is mainly involved in the endocytosis of an immunocomplex of an antibody and antigen (J. Immunol. (1994), 152 574-585; Science (1992) 256, 1808-1812; Cell (1989) 58, 317-327). So far, mouse FcyRIIb2 has been reported to be incorporated into a clathrin-coated pit and endocytosed (Cell (1989) 58, 317-327). Furthermore, it has been reported that a dileucine motif is necessary for FcyRIIb2-mediated endocytosis, and the dileucine motif is conserved in both humans and mice (EMBO J. (1994) 13 (13), 2963-2969). From these, FcyRIIb2 may have an endocytotic ability in humans as in mice.

On the other hand, unlike FcγRIIb2, it has been reported that FcγRIIb1 does not cause endocytosis. FcγRIIb1 has an inserted sequence in its intracellular domain that is not found in FcγRIIb2. It is considered that this sequence inhibits the uptake of FcγRIIb1 into a clathrin-coated pit, and as a result endocytosis is inhibited (J. Cell. Biol. (1992) 116, 875-888; J. Cell. Biol. (1989) 109, 3291-3302). In humans as well, FcγRIIb1 has an insertion sequence at a site similar to that of FcγRIIb2 as in mice; therefore, difference in the endocytotic ability between FcγRIIb1 and FcγRIIb2 is presumed to be caused by a similar mechanism. Furthermore, in both humans and mice, approximately 40% of immunocomplexes on the cell surface is reported to be taken up into the cell in 20 minutes (Mol. Immunol. (2011) 49, 329-337; Science (1992) 256, 1808-1812). Therefore, in humans as well, FcγRIIb2 is presumed to uptake immunocomplexes into cells at rates similar to those in mice.

Since FcγRIIb is the only one that has ITIM inside the cell in both humans and mice among the FcγR family and the distribution of expressing cells are the same, it is presumed that its function in immune control is similar. Furthermore, considering the fact that immunocomplexes are taken up into cells at similar rates in humans and mice, antigen elimination effects of antibodies mediated by FcγRIIb in humans may be predictable using mice. Antigen-binding molecules mF44 and mF46 have properties of binding to soluble antigens in a pH-dependent manner, and have enhanced affinity to mouse FcγRIIb and FcγRIII compared to mIgG1 which is an antigen-binding molecule having the property of binding to a soluble antigen in a pH-dependent manner. Indeed, it is shown in Example 5 that antigen clearance increased when mF44 or mF46 was administered to normal mice compared to when mIgG1 was administered.

Furthermore, in the later-described Example 6, a similar experiment was carried out using Fc receptor γ chain-deficient mice. It has been reported that FcγRs other than FcγRIIb are expressed only in the co-presence of a gamma chain in mice. Thus, only FcγRIIb is expressed in the Fc receptor γ chain-deficient mice. Administration of mF44 or mF46, which are antigen-binding molecules having the property of binding to soluble antigens in a pH-dependent manner, to Fc receptor γ chain-deficient mice enables assessment of antigen elimination-acceleration effects when FcγRIIb-binding is selectively enhanced. From the results of Example 6, when mF44 or mF46 (which are antigen-binding molecules having the property of binding to soluble antigens in a pH-dependent manner) was administered to Fc receptor γ chain-deficient mice, antigen clearance was shown to increase compared to when mIgG1 (which is an antigen-binding molecule having the property of binding to soluble antigens in a pH-dependent manner) was administered to the mice. Furthermore, the results of Example 6 shows that when administered to Fc receptor γ chain-deficient mice, mF44 or mF46 cause similar degrees of antigen elimination as when administered to normal mice.

In Example 6, a similar experiment was performed using FcγRIII-deficient mice. Since mIgG1, mF44, and mF46 bind only to FcγRIIb and FcγRIII among the mFcγRs, administration of the antibodies to FcγRIII-deficient mice enables assessment of antigen elimination-accelerating effects when FcγRIIb-binding is selectively enhanced. The results of Example 6 indicate that when mF44 or mF46 was administered to FcγRIII-deficient mice, antigen clearance was increased compared to when mIgG1 was administered to the mice antigen clearance. Furthermore, the results of Example 6 showed that when administered to FcγRIII-deficient mice, mF44 and mF46 cause similar degrees of antigen elimination as when administered to Fc receptor γ chain-deficient mice and when administered to normal mice.

These results revealed that antigen elimination can be accelerated by enhancing selective binding to FcγRIIb alone without enhancing binding to active FcγRs.

In addition to the reported documents discussed so far, based on the above-mentioned assessment results using mice, it is considered that uptake of immunocomplexes into cells *via* FcγRIIb takes place *in vivo* in humans as in mice, and as a result, antibodies that have Fc with selectively enhanced binding to human FcγRIIb can accelerate elimination of its antigens. Furthermore, as discussed above, since uptake of immunocomplexes into cells *via* FcγRIIb is considered to take place at similar rates in mice and humans, effects of accelerating antigen elimination comparable to those of antibodies having Fc with enhanced affinity to mouse FcγRIIb may be achieved *in vivo* in humans by using Fc in which affinity to human FcγRIIb is enhanced to a similar extent.

As described in WO2009/125825, Fv4-IgG1 is an antibody that results from conferring to a humanized anti-IL-6 receptor antibody H54/L28-IgG1 the activity to bind to the antigen in a pH-dependent manner, *i.e.,* altering the variable region to confer the property to bind to an antigen at pH 7.4 and dissociate from the antigen at pH 5.8. WO2009/125825 showed that the elimination of soluble human IL-6 receptor is greatly accelerated in mice co-administered with Fv4 IgG1 and soluble human IL-6 receptor as the antigen as compared to mice co-administered with H54/L28-IgG1 and the antigen. Herein, heavy chain H54-IgG1 and light-chain L28-CK included in H54/L28-IgG1 are shown in SEQ ID NO: 36 and SEQ ID NO: 37, respectively; and heavy chain VH3-IgG1 and light-chain VL3-CK included in Fv4-IgG1 are shown in SEQ ID NO: 38 and SEQ ID NO: 39, respectively.

Soluble human IL-6 receptor bound to an antibody H54/L28-IgG1, which binds to soluble human IL-6 receptor, is recycled to the plasma along with the antibody *via* FcRn. Meanwhile, antibody Fv4-IgG1 which binds to soluble human IL-6 receptor in a pH-dependent manner dissociates from the soluble human IL-6 receptor that has been bound to the antibody under an acidic condition in the endosome. Since the dissociated soluble human IL-6 receptor is degraded in the lysosome, elimination of the soluble human IL-6 receptor can be greatly accelerated, and the antibody Fv4-IgG1 which binds to the soluble human IL-6 receptor in a pH-dependent manner is recycled to the plasma after binding to FcRn in the endosome. Since the recycled antibody can bind to a soluble human IL-6 receptor again, binding to the antigen (soluble human IL-6 receptor) and recycling to the plasma *via* FcRn are repeated. As a result, a single antibody molecule can repeatedly bind to the soluble human IL-6 receptor multiple times (WO2009/125825).

On the other hand, as disclosed in the present invention, it was found that plasma concentration of the soluble antigen can be reduced greatly by administration of an antigen-binding molecule with enhanced FcγR-binding activity of the Fcγ receptor-binding domain included in the antigen-binding molecule which comprises an antigen-binding domain in which antigen-binding activity changes depending on the ion concentration condition such as pH, an FcRn-binding domain having FcRn-binding activity under an acidic pH range condition, and an Fcγ receptor-binding domain.

While not being restricted to a particular theory, the unexpected decrease in soluble antigen concentration in plasma observed by administration of an antigen-binding molecule with enhanced binding to FcγRs, which comprises an antigen-binding domain in which antigen-binding activity changes depending on the ion-concentration condition such as pH and an FcRn-binding domain having FcRn-binding activity under an acidic pH range condition can be explained as follows.

As described above, an antigen-binding molecule such as Fv4-IgG1 comprising an antigen-binding domain in which antigen-binding activity changes depending on the ion-concentration condition may be able to bind repeatedly to the antigen multiple times, but the effect of dissociating the soluble antigen in the endosome to accelerate the antigen elimination from plasma may be dependent on the rate of uptake of the complex of the antigen and antigen-binding molecule into the endosome. The antigen-binding molecules with enhanced binding activities to various FcγRs, which comprise an antigen-binding domain in which antigen-binding activity changes depending on the ion-concentration condition, are actively taken up into cells by binding to various FcγRs expressed on the cell membrane, and can circulate in the plasma again by recycling *via* binding between FcRn and the FcRn-binding domain in the molecule having binding activity to FcRn under an acidic pH range condition. More specifically, since the aforementioned antigen-binding molecules that formed complexes with soluble antigens in plasma are taken up actively into cells *via* FcyRs expressed on the cell membrane, the effect of accelerating elimination of soluble antigens in plasma may be more pronounced than that of antigen-binding molecules whose binding activities to various FcyRs are not enhanced.

FcγR-binding activities of antibodies that bind to membrane antigens have an important role in cytotoxic activity of the antibodies. Therefore, when cytotoxic activity is necessary for an antibody to be used as a pharmaceutical, a human IgG1 isotype which has high FcγR-binding activity is used, and the technique of enhancing the FcγR-binding activities of the antibody to enhance the cytotoxic activity of the antibody is widely utilized. On the other hand, the role of FcγR-binding activities of antibodies that bind to soluble antigens and are used as pharmaceuticals had not been known, and differences in physiological effects on organisms administered with human IgG1 with high FcγR-binding activities and human IgG2 and human IgG4 with low FcγR-binding activities, due to their differences in FcyR-binding activities, had not been fully examined so far. As described later in the Examples, it was actually confirmed that in the plasma of individuals administered with antibodies whose FcγR-binding activities have been lost, changes in soluble-antigen concentration were not affected. On the other hand, in the present invention, the concentration of soluble antigens in the plasma was found to be greatly reduced in individuals administered with antigen-binding molecules with enhanced FcγR-binding activities and comprising an antigen-binding domain whose binding activity to soluble antigens changes depending on the ion concentration condition. More specifically, by combining an FcRn-binding domain having an FcRn-binding activity under an acidic pH range condition and an antigen-binding domain whose binding to soluble antigens changes depending on the ion concentration condition, which are domains included in antigen-binding molecules targeting soluble antigens, an advantage of enhancing binding to FcγR was found for the first time.

Antigen-binding molecule

In the present invention, "an antigen-binding molecule" is used in the broadest sense to refer to a molecule having human FcRn-binding activity at an acidic pH range and containing an antigen-binding domain and an Fcγ receptor-binding domain. Specifically, the antigen-binding molecules include various types of molecules as long as they exhibit the antigen-binding activity. Molecules in which an antigen-binding domain is linked to an Fc region include, for example, antibodies. Antibodies may include single monoclonal antibodies (including agonistic antibodies and antagonistic antibodies), human antibodies, humanized antibodies, chimeric antibodies, and such. Alternatively, when used as antibody fragments, they preferably include antigen-binding domains and antigen-binding fragments (for example, Fab, F(ab')2, scFv, and Fv). Scaffold molecules where three dimensional structures, such as already-known stable $\alpha/\beta$ barrel protein structure, are used as a scaffold (base) and only some portions of the structures are made into libraries to construct antigen-binding domains are also included in antigen-binding molecules of the present invention.

An antigen-binding molecule of the present invention may contain at least some portions of an Fc region that mediates the binding to FcRn and Fcγ receptor. In a non-limiting embodiment, the antigen-binding molecule includes, for example, antibodies and Fc fusion proteins. A fusion protein refers to a chimeric polypeptide comprising a polypeptide having a first amino acid sequence that is linked to a polypeptide having a second amino acid sequence that would not naturally link in nature. For example, a fusion protein may comprise the amino acid sequence of at least a portion of an Fc region (for example, a portion of an Fc region responsible for the binding to FcRn or a portion of an Fc region responsible for the binding to Fcγ receptor) and a non-immunoglobulin polypeptide containing, for example, the amino acid sequence of the ligand-binding domain of a receptor or a receptor-binding domain of a ligand. The amino acid sequences may be present in separate proteins that are transported together to a fusion protein, or generally may be present in a single protein; however, they are included in a new rearrangement in a fusion polypeptide. Fusion proteins can be produced, for example, by chemical synthesis, or by genetic recombination techniques to express a polynucleotide encoding peptide regions in a desired arrangement.

Respective domains of the present invention can be linked together via linkers or directly via polypeptide binding.

The linkers comprise arbitrary peptide linkers that can be introduced by genetic engineering, synthetic linkers, and linkers disclosed in, for example, Protein Engineering (1996) 9(3), 299-305. However, peptide linkers are preferred in the present invention. The length of the peptide linkers is not particularly limited, and can be suitably selected by those skilled in the art according to the purpose. The length is preferably five amino acids or more (without particular limitation, the upper limit is generally 30 amino acids or less, preferably 20 amino acids or less), and particularly preferably 15 amino acids.

For example, such peptide linkers preferably include:

Ser
Gly·Ser
Gly·Gly·Ser
Ser·Gly·Gly
Gly·Gly·Gly·Ser (SEQ ID NO: 26)
Ser·Gly·Gly·Gly (SEQ ID NO: 27)
Gly·Gly·Gly·Gly·Ser (SEQ ID NO: 28)
Ser·Gly·Gly·Gly·Gly (SEQ ID NO: 29)
Gly·Gly·Gly·Gly·Gly·Ser (SEQ ID NO: 30)
Ser·Gly·Gly·Gly·Gly·Gly (SEQ ID NO: 31)
Gly·Gly·Gly·Gly·Gly·Gly·Ser (SEQ ID NO: 32)
Ser·Gly·Gly·Gly·Gly·Gly·Gly (SEQ ID NO: 33)
(Gly·Gly·Gly·Gly·Ser (SEQ ID NO: 28))n
(Ser·Gly·Gly·Gly·Gly (SEQ ID NO: 29))n

where n is an integer of 1 or larger. The length or sequences of peptide linkers can be selected accordingly by those skilled in the art depending on the purpose.

Synthetic linkers (chemical crosslinking agents) is routinely used to crosslink peptides, and for example:

N-hydroxy succinimide (NHS),
disuccinimidyl suberate (DSS),
bis(sulfosuccinimidyl) suberate (BS3),
dithiobis(succinimidyl propionate) (DSP),
dithiobis(sulfosuccinimidyl propionate) (DTSSP),
ethylene glycol bis(succinimidyl succinate) (EGS),
ethylene glycol bis(sulfosuccinimidyl succinate) (sulfo-EGS),
disuccinimidyl tartrate (DST), disulfosuccinimidyl tartrate (sulfo-DST),
bis[2-(succinimidoxycarbonyloxy)ethyl] sulfone (BSOCOES),
and bis[2-(sulfosuccinimidoxycarbonyloxy)ethyl] sulfone (sulfo-BSOCOES). These crosslinking agents are commercially available.

When multiple linkers for linking the respective domains are used, they may all be of the same type, or may be of different types.

In addition to the linkers exemplified above, linkers with peptide tags such as His tag, HA tag, myc tag, and FLAG tag may also be suitably used. Furthermore, hydrogen bonding, disulfide bonding, covalent bonding, ionic interaction, and properties of binding with each other as a result of combination thereof may be suitably used. For example, the affinity between CH1 and CL of antibody may be used, and Fc regions originating from the above-described bispecific antibodies may also be used for hetero Fc region association. Moreover, disulfide bonds formed between domains may also be suitably used.

In order to link respective domains via peptide linkage, polynucleotides encoding the domains are linked together in frame. Known methods for linking polynucleotides in frame include techniques such as ligation of restriction fragments, fusion PCR, and overlapping PCR. Such methods can be appropriately used alone or in combination to construct antigen-binding molecules of the present invention. In the present invention, the terms "linked" and "fused", or "linkage" and "fusion" are used interchangeably. These terms mean that two or more elements or components such as polypeptides are linked together to form a single structure by any means including the above-described chemical linking means and genetic recombination techniques. Fusing in frame means, when two or more elements or components are polypeptides, linking two or more units of reading frames to form a continuous longer reading frame while maintaining the correct reading frames of the polypeptides. When two molecules of Fab are used as an antigen-binding domain, an antibody, which is an antigen-binding molecule of the present invention where the antigen-binding domain is linked in frame to an Fc region via peptide bond without linker, can be used as a preferred antigen-binding molecule of the present invention.

FcRn

Unlike Fcγ receptor belonging to the immunoglobulin superfamily, FcRn, human FcRn in particular, is structurally similar to polypeptides of major histocompatibility complex (MHC) class I, exhibiting 22% to 29% sequence identity to class I MHC molecules (Ghetie el al., Immunol. Today (1997) 18 (12): 592-598). FcRn is expressed as a heterodimer consisting of soluble β or light chain (β2 microglobulin) complexed with transmembrane α or heavy chain. Like MHC, FcRn α chain comprises three extracellular domains (α1, α2, and α3) and its short cytoplasmic domain anchors the protein onto the cell surface, α1 and α2 domains interact with the FcRn-binding domain of the antibody Fc region (Raghavan et al., Immunity (1994) 1: 303-315).

FcRn is expressed in maternal placenta and york sac of mammals, and is involved in mother-to-fetus IgG transfer. In addition, in neonatal small intestine of rodents, where FcRn is expressed, FcRn is involved in transfer of maternal IgG across brush border epithelium from ingested colostrum or milk. FcRn is expressed in a variety of other tissues and endothelial cell systems of various species. FcRn is also expressed in adult human endothelia, muscular blood vessels, and hepatic sinusoidal capillaries. FcRn is believed to play a role in maintaining the plasma IgG concentration by mediating recycling of IgG to serum upon binding to IgG. Typically, binding of FcRn to IgG molecules is strictly pH dependent. The optimal binding is observed in an acidic pH range below 7.0.

Human FcRn whose precursor is a polypeptide having the signal sequence of SEQ ID NO: 34 (the polypeptide with the signal sequence is shown in SEQ ID NO: 35) forms a complex with human β2-microglobulin *in vivo.* As shown in the Reference Examples described below, soluble human FcRn complexed with β2-microglobulin is produced by using conventional recombinant expression techniques. FcRn-binding domain of the present invention can be assessed for their binding activity to such a soluble human FcRn complexed with β2-microglobulin. In the present invention, unless otherwise specified, human FcRn refers to a form capable of binding to an FcRn-binding domain of the present invention. Examples include a complex between human FcRn and human β2-microglobulin.

Antigen-binding molecules of the present invention have an FcRn-binding domain. The FcRn-binding domain is not particularly limited as long as the antigen-binding molecules have an FcRn-binding activity in an acidic pH range, and it may be a domain that has direct or indirect binding activity to FcRn. Preferred examples of such a domain include the Fc region of IgG immunoglobulin, albumin, albumin domain 3, anti-FcRn antibody, anti-FcRn peptide, anti-FcRn scaffold molecule, and such which have an activity to directly bind to FcRn, or molecules that bind to IgG or albumin, and such that have an activity to indirectly bind to FcRn. In the present invention, a domain that has FcRn-binding activity in an acidic pH range and in a neutral pH range is preferred. If the domain already has FcRn-binding activity in an acidic pH range, it may preferably be used as it is. If the domain does not have or has weak FcRn-binding activity in an acidic pH range, amino acids in the antigen-binding molecule may be altered to confer FcRn-binding activity. Alternatively, amino acids may be altered in a domain already having FcRn-binding activity in an acidic pH range to increase the FcRn-binding activity. For amino acid alteration of the FcRn-binding domain, the alteration of interest can be identified by comparing the FcRn-binding activities in an acidic pH range before and after the amino acid alteration.

The preferred human FcRn-binding domain is a region that directly binds to FcRn. Such preferred FcRn-binding domains include, for example, antibody Fc regions. Meanwhile, regions capable of binding to a polypeptide such as albumin or IgG, which has FcRn-binding activity, can indirectly bind to FcRn *via* albumin, IgG, or such. Therefore, for the FcRn-binding region of the present invention, a region that binds to a polypeptide having FcRn-binding activity may be preferably used. An Fc region contains an amino acid sequence derived from the constant region of an antibody heavy chain. An Fc region is a portion of the antibody heavy chain constant region beginning from the N terminus of the hinge region at the papain cleavage site, which is on the amino acid at approximately position 216 according to EU numbering, and including the hinge, CH2 and CH3 domains.

The binding activity of an FcRn binding domain for FcRn, in particular human FcRn, or an antigen-binding molecule comprising the domain

The binding activity of an FcRn binding domain of the present invention to FcRn, human FcRn in particular, can be measured by methods known to those skilled in the art, as described in the section "Binding Activity" above. Those skilled in the art can appropriately determine the conditions other than pH. The antigen-binding activity and human FcRn-binding activity of an antigen-binding molecule can be assessed based on the dissociation constant (KD), apparent dissociation constant (KD), dissociation rate (kd), apparent dissociation rate (kd), and such. They can be measured by methods known to those skilled in the art. For example, Biacore (GE healthcare), Scatchard plot, or flow cytometer may be used.

When the FcRn-binding activity of an FcRn-binding domain is measured, conditions other than the pH are not particularly limited, and can be appropriately selected by those skilled in the art. Measurements can be carried out, for example, at 37°C using MES buffer, as described in WO 2009/125825. Alternatively, the FcRn-binding activity of an FcRn-binding domain can be measured by methods known to those skilled in the art, and may be measured by using, for example, Biacore (GE Healthcare) or such. The binding activity of an FcRn-binding domain to FcRn can be assessed by pouring, as an analyte, FcRn, an FcRn-binding domain, or an antigen-binding molecule of the present invention containing the FcRn-binding domain into a chip immobilized with an FcRn-binding domain, an antigen-binding molecule of the present invention containing the FcRn-binding domain, or FcRn.

The acidic pH range presented as the condition for having binding activity between FcRn and an FcRn-binding domain in an antigen-binding molecule of the present invention generally refers to pH 4.0 to pH 6.5. Preferably it refers to pH 5.5 to pH 6.5, and particularly preferably it refers to pH 5.8 to pH 6.0 which is close to the pH in an early endosome *in vivo.* The neutral pH range presented as the condition for having binding activity between FcRn and an antigen-binding molecule of the present invention or an FcRn-binding domain included in such a molecule generally refers to pH 6.7 to pH 10.0. Neutral pH range is preferably a range indicated by any pH value from pH 7.0 to pH 8.0, and is preferably selected from pH 7.0, 7.1, 7.2, 7.3, 7.4, 7.5, 7.6, 7.7, 7.8, 7.9, and 8.0, and is particularly preferably pH 7.4 which is close to the pH in plasma (in blood) *in vivo*. When evaluating the binding affinity between human FcRn and a human FcRn-binding domain or an antigen-binding molecule containing that domain at pH 7.4 is difficult due to low binding affinity, pH 7.0 can be used instead of pH 7.4. As temperature to be used in assay conditions, binding affinity between an FcRn-binding domain and FcRn may be assessed at any temperature from 10°C to 50°C. Preferably, a temperature from 15°C to 40°C is used to determine the binding affinity between an FcRn-binding domain and human FcRn. More preferably, any temperature from 20°C to 35°C such as any one of 20, 21, 22, 23, 24, 25, 26, 27, 28, 29, 30, 31, 32, 33, 34, and 35°C is also equally used to determine the binding affinity between an FcRn-binding domain and FcRn. A temperature of 25°C is a non-limiting example of an embodiment of the present invention.

According to Yeung et al. (J. Immunol. (2009) 182, 7663-7671), the human FcRn-binding activity of native human IgG1 in an acidic pH range (pH 6.0) is KD 1.7 μM, but the activity can be hardly detected in a neutral pH range. Therefore, in a preferred embodiment, an antigen-binding molecule of the present invention having human FcRn-binding activity under an acidic pH range condition, which includes antigen-binding molecules whose human FcRn-binding activity under an acidic pH range condition is KD 20 μM or stronger and human FcRn-binding activity under a neutral pH range condition is equivalent to that of a native human IgG may be used. In a more preferred embodiment, an antigen-binding molecule of the present invention including antigen-binding molecules whose human FcRn-binding activity under an acidic pH range condition is KD 2.0 μM or stronger may be used. In an even more preferred embodiment, antigen-binding molecules whose human FcRn-binding activity under an acidic pH range condition is KD 0.5 μM or stronger may be used. The above-mentioned KD values are determined by the method described in The Journal of Immunology (2009) 182: 7663-7671 (by immobilizing antigen-binding molecule onto a chip and loading human FcRn as the analyte).

In the present invention, an Fc region that has FcRn-binding activity under an acidic pH range condition is preferred. If the domain is an Fc region already having FcRn-binding activity under an acidic pH range condition, it can be used as it is. If the domain does not have or has weak FcRn-binding activity under an acidic pH range condition, amino acids in the antigen-binding molecule may be altered to obtain an Fc region having the desired FcRn-binding activity. An Fc region having the desired FcRn-binding activity or enhanced FcRn-binding activity under an acidic pH range condition can also be preferably obtained by altering amino acids in the Fc region. Amino acid alteration of an Fc region that confers such a desired binding activity can be identified by comparing the FcRn-binding activity under an acidic pH range condition before and after the amino acid alteration. Those skilled in the art can make appropriate amino acid alterations using a well-known method similar to the aforementioned method used to alter the Fcγ receptor-binding activity.

An Fc region having an FcRn-binding activity under an acidic pH range condition, which is included in an antigen-binding molecule of the present invention, may be obtained by any method; but specifically, an FcRn-binding domain having FcRn-binding activity or having enhanced FcRn-binding activity under an acidic pH range condition can be obtained by altering amino acids of a human IgG immunoglobulin used as the starting Fc region. Preferred Fc regions of IgG immunoglobulins for the alteration include Fc regions of human IgG (IgG1, IgG2, IgG3, IgG4, and variants thereof). For alterations to other amino acids, amino acids at any position may be altered as long as the Fc region has FcRn-binding activity under an acidic pH range condition, or the binding activity to human FcRn under an acidic range condition can be increased. When an antigen-binding molecule includes an Fc region of a human IgG1 as the Fc region, it preferably includes an alteration that has the effect of enhancing binding to FcRn under an acidic pH range condition compared to the binding activity of the starting Fc region of human IgG1. Preferred examples of such amino acids that can be altered include amino acids at positions 238, 252, 253, 254, 255, 256, 265, 272, 286, 288, 303, 305, 307, 309, 311, 312, 317, 340, 356, 360, 362, 376, 378, 380, 382, 386, 388, 400, 413, 415, 424, 433, 434, 435, 436, 439, and/or 447 (EU numbering) as described in WO2000/042072. Similarly, preferred examples of amino acids that can be altered also include amino acids at positions 251, 252, 254, 255, 256, 308, 309, 311, 312, 385, 386, 387, 389, 428, 433, 434, and/or 436 (EU numbering) as described in WO2002/060919. Furthermore, such amino acids that can be altered also include amino acids at positions 250, 314, and 428 (EU numbering) as described in WO2004/092219. Such amino acids that can be altered further include amino acids at positions 251, 252, 307, 308, 378, 428, 430, 434, and/or 436 (EU numbering) as described in WO2010/045193. These amino acid alterations enhance the FcRn-binding under an acidic pH range condition of an Fc region of an IgG immunoglobulin.

In the present invention, an Fc region that has an FcRn-binding activity under an acidic pH range condition is preferred. If the domain is an Fc region already having FcRn-binding activity under an acidic pH range condition, it may be used as it is. If the domain does not have or has weak FcRn-binding activity under an acidic pH range condition, amino acids in the antigen-binding molecule may be altered to obtain an Fc region having the desired FcRn-binding activity. An Fc region having the desired FcRn-binding activity or enhanced FcRn-binding activity under an acidic pH range condition can preferably be obtained by altering amino acids in the Fc region. Amino acid alteration of an Fc region that confers such a desired binding activity can be identified by comparing the FcRn-binding activity under an acidic pH range condition before and after the amino acid alteration. Those skilled in the art can make appropriate amino acid alterations using a well-known method similar to the aforementioned method used to alter the Fcγ receptor-binding activity.

An Fc region having FcRn-binding activity under an acidic pH range condition, which is included in an antigen-binding molecule of the present invention may be obtained by any method; but specifically, an FcRn-binding domain having binding activity or having enhanced binding activity to FcRn under an acidic pH range condition can be obtained by altering amino acids of a human IgG immunoglobulin used as the starting Fc region. Preferred examples of Fc regions of IgG immunoglobulins for the alteration include Fc regions of human IgG (IgG1, IgG2, IgG3, IgG4, and variants thereof). For alterations to other amino acids, amino acids at any position may be altered as long as an Fc region has FcRn-binding activity under an acidic pH range condition, or the binding activity to human FcRn under an acidic range condition can be increased. When an antigen-binding molecule comprises an Fc region of a human IgG1 as the Fc region, it preferably includes an alteration that has effects of enhancing binding to FcRn under an acidic pH range condition compared to the binding activity of the starting Fc region of human IgG1. Examples of such amino acids that can be altered include amino acids at positions 252, 254, 256, 309, 311, 315, 433, and/or 434, as well as amino acids that may be combined with them, which are amino acids at positions 253, 310, 435, and/or 426 (EU numbering) as described in WO1997/034631. Preferred amino acids are amino acids at positions 238, 252, 253, 254, 255, 256, 265, 272, 286, 288, 303, 305, 307, 309, 311, 312, 317, 340, 356, 360, 362, 376, 378, 380, 382, 386, 388, 400, 413, 415, 424, 433, 434, 435, 436, 439, and/or 447 (EU numbering) as described in WO2000/042072. Similarly, preferred examples of such amino acids that can be altered also include amino acids at positions 251, 252, 254, 255, 256, 308, 309, 311, 312, 385, 386, 387, 389, 428, 433, 434, and/or 436 (EU numbering) as described in WO2002/060919. Furthermore, such amino acids that can be altered include amino acids at positions 250, 314, and 428 (EU numbering) as described in WO2004/092219. In addition, preferred examples of such amino acids that can be altered include amino acids at positions 238, 244, 245, 249, 252, 256, 257, 258, 260, 262, 270, 272, 279, 283, 285, 286, 288, 293, 307, 311, 312, 316, 317, 318, 332, 339, 341, 343, 375, 376, 377, 378, 380, 382, 423, 427, 430, 431, 434, 436, 438, 440, and/or 442 as described in WO2006/020114. Other preferred examples of such amino acids that can be altered also include amino acids at positions 251, 252, 307, 308, 378, 428, 430, 434, and/or 436 (EU numbering) as described in WO2010/045193. These amino acid alterations enhance the FcRn-binding under an acidic pH range condition of an Fc region of an IgG immunoglobulin.

In a non-limiting embodiment of alterations that produce the effect of enhancing binding to FcRn under an acidic pH range condition compared to the binding activity of the starting Fc region of human IgG1 when an Fc region of human IgG1 is included as the Fc region, examples include at least one or more amino acid alterations selected from the group consisting of:

either Arg or Leu at amino acid position 251;
any of Phe, Ser, Thr, and Tyr at amino acid position 252;
either Ser or Thr at amino acid position 254;
any of Arg, Gly, Ile, and Leu at amino acid position 255;
any of Ala, Arg, Asn, Asp, Gln, Glu, and Thr at amino acid position 256;
either Ile or Thr at amino acid position 308;
Pro at amino acid position 309;
any of Glu, Leu, and Ser at amino acid position 311;
either Ala or Asp at amino acid position 312;
either Ala or Leu at amino acid position 314;
any of Ala, Arg, Asp, Gly, His, Lys, Ser, and Thr at amino acid position 385;
any of Arg, Asp, Ile, Lys, Met, Pro, Ser, and Thr at amino acid position 386;
any of Ala, Arg, His, Pro, Ser, and Thr at amino acid position 387;
any of Asn, Pro, and Ser at amino acid position 389;
any of Leu, Met, Phe, Ser, and Thr at amino acid position 428;
any of Arg, Gln, His, Ile, Lys, Pro, and Ser at amino acid position 433;
any of His, Phe, and Tyr at amino acid position 434; and
any of Arg, Asn, His, Lys, Met, and Thr at amino acid position 436
according to EU numbering. The number of amino acids to be altered is not particularly limited, and the amino acid may be altered at just one position or at two or more positions.

When an Fc region of human IgG1 is included as the Fc region, a non-limiting embodiment of alterations that produce effects of enhancing binding to FcRn under an acidic pH range condition compared to the binding activity of the starting Fc region of human IgG1 can be an alteration(s) comprising Ile at amino acid position 308, Pro at amino acid position 309, and/or Glu at amino acid position 311 according to EU numbering. In another non-limiting embodiment of the alterations, the alterations may be Thr at amino acid position 308, Pro at amino acid position 309, Leu at amino acid position 311, Ala at amino acid position 312, and/or Ala at amino acid position 314. In yet another non-limiting embodiment of the alterations, the alterations may be Ile or Thr at amino acid position 308; Pro at amino acid position 309; Glu, Leu, or Ser at amino acid position 311; Ala at amino acid position 312; and/or Ala or Leu at amino acid position 314. In a different non-limiting embodiment of the alterations, the alterations can be Thr at amino acid position 308, Pro at amino acid position 309, Ser at amino acid position 311, Asp at amino acid position 312, and/or Leu at amino acid position 314.

When an Fc region of human IgG1 is included as the Fc region, a non-limiting embodiment of alterations that produce effects of enhancing binding to FcRn under an acidic pH range condition compared to the binding activity of the starting Fc region of human IgG1 can be an alteration(s) comprising Leu at amino acid position 251, Tyr at amino acid position 252, Ser or Thr at amino acid position 254, Arg at amino acid position 255, and/or Glu at amino acid position 256 according to EU numbering.

When an Fc region of human IgG1 is included as the Fc region, a non-limiting embodiment of alterations that produce effects of enhancing binding to FcRn under an acidic pH range condition compared to the binding activity of the starting Fc region of human IgG1 can be an alteration(s) comprising any one of Leu, Met, Phe, Ser, and Thr at amino acid position 428; any one of Arg, Gln, His, Ile, Lys, Pro, and Ser at amino acid position 433; any one of His, Phe, and Tyr at amino acid position 434; and/or any one of Arg, Asn, His, Lys, Met, and Thr at amino acid position 436 indicated by EU numbering. In another non-limiting embodiment of the alteration(s), the alteration(s) may include His or Met at amino acid position 428 and/or His or Met at amino acid position 434.

When an Fc region of human IgG1 is included as the Fc region, a non-limiting embodiment of alterations that produce effects of enhancing binding to FcRn under an acidic pH range condition compared to the binding activity of the starting Fc region of human IgG1 can be an alteration(s) comprising Arg at amino acid position 385, Thr at amino acid position 386, Arg at amino acid position 387, and/or Pro at amino acid position 389 according to EU numbering. In another non-limiting embodiment of the alterations, the alteration(s) can be Asp at amino acid position 385, Pro at amino acid position 386, and/or Ser at amino acid position 389.

Furthermore, when an Fc region of human IgG1 is included as the Fc region, a non-limiting embodiment of alterations that produce effects of enhancing binding to FcRn under an acidic pH range condition compared to the binding activity of the starting Fc region of human IgG1, alterations include those of at least one or more amino acids selected from the group consisting of:

either Gln or Glu at amino acid position 250; and
either Leu or Phe at amino acid position 428
according to EU numbering. The number of amino acids to be altered is not particularly limited, and the amino acid may be altered at one position alone or at two or more positions.

When an Fc region of human IgG1 is included as the Fc region, a non-limiting embodiment of alterations that produce effects of enhancing binding to FcRn under an acidic pH range condition compared to the binding activity of the starting Fc region of human IgG1 can be an alteration(s) comprising Gln at amino acid position 250, and/or either Leu or Phe at amino acid position 428 according to EU numbering. In another non-limiting embodiment of the alterations, the alterations can be those comprising Glu at amino acid position 250 and/or either Leu or Phe at amino acid position 428.

When an Fc region of human IgG1 is included as the Fc region, a non-limiting embodiment of alterations that produce effects of enhancing binding to FcRn under an acidic pH range condition compared to the binding activity of the starting Fc region of human IgG1, examples include alterations of at least two or more amino acids selected from the group consisting of:

either Asp or Glu at amino acid position 251;
Tyr at amino acid position 252;
Gln at amino acid position 307;
Pro at amino acid position 308;
Val at amino acid position 378;
Ala at amino acid position 380;
Leu at amino acid position 428;
either Ala or Lys at amino acid position 430;
any one of Ala, His, Ser, and Tyr at amino acid position 434; and
Ile at amino acid position 436
according to EU numbering. The number of amino acids to be altered is not particularly limited, and the amino acid may be altered at only two positions or at three or more positions.

When an Fc region of human IgG1 is included as the Fc region, a non-limiting embodiment of alterations that produce effects of enhancing binding to FcRn under an acidic pH range condition compared to the binding activity of the starting Fc region of human IgG1 can be alteration(s) comprising Gln at amino acid position 307, and either Ala or Ser at amino acid position 434 according to EU numbering. In another non-limiting embodiment of the alterations, the alterations may include Pro at amino acid position 308 and Ala at amino acid position 434. In yet another non-limiting embodiment of the alterations, the alterations may include Tyr at amino acid position 252 and Ala at amino acid position 434. In a different non-limiting embodiment of the alterations, the alterations may include Val at amino acid position 378 and Ala at amino acid position 434. In another non-limiting embodiment of the alterations, the alterations may include Leu at amino acid position 428 and Ala at amino acid position 434. In yet another non-limiting embodiment of the alterations, the alterations may include Ala at amino acid position 434 and lie at amino acid position 436. Furthermore, in another non-limiting embodiment of the alterations, the alterations may include Pro at amino acid position 308 and Tyr at amino acid position 434. Furthermore, in another different non-limiting embodiment of the alterations, the alterations may include Gln at amino acid position 307 and Ile at amino acid position 436.

When an Fc region of human IgG1 is included as the Fc region, a non-limiting embodiment of alterations that produce effects of enhancing binding to FcRn under an acidic pH range condition compared to the binding activity of the starting Fc region of human IgG1 can be an alteration(s) comprising Gln at amino acid position 307, Ala at amino acid position 380, and Ser at amino acid position 434 as according to EU numbering. In another non-limiting embodiment of the alterations, the alterations may include Gln at amino acid position 307, Ala at amino acid position 380, and Ala at amino acid position 434. In yet another non-limiting embodiment of the alterations, the alterations may include Tyr at amino acid position 252, Pro at amino acid position 308, and Tyr at amino acid position 434. In a different non-limiting embodiment of the alterations, the alterations may include Asp at amino acid position 251, Gln at amino acid position 307, and His at amino acid position 434.

When an Fc region of human IgG1 is included as the Fc region, in a non-limiting embodiment of alterations that produce effects of enhancing binding to FcRn under an acidic pH range condition compared to the binding activity of the starting Fc region of human IgG1, examples include alterations of at least two or more amino acids selected from the group consisting of:

Leu at amino acid position 238;
Leu at amino acid position 244;
Arg at amino acid position 245;

Pro at amino acid position 249;
Tyr at amino acid position 252;
Pro at amino acid position 256;
any one of Ala, Ile, Met, Asn, Ser, and Val at amino acid position 257;
Asp at amino acid position 258;
Ser at amino acid position 260;
Leu at amino acid position 262;
Lys at amino acid position 270;
either Leu or Arg at amino acid position 272;
any one of Ala, Asp, Gly, His, Met, Asn, Gln, Arg, Ser, Thr, Trp, and Tyr at amino acid position 279;
any one of Ala, Asp, Phe, Gly, His, Ile, Lys, Leu, Asn, Pro, Gln, Arg, Ser, Thr, Trp, and Tyr at amino acid position 283;
Asn at amino acid position 285;
Phe at amino acid position 286;
either Asn or Pro at amino acid position 288;
Val at amino acid position 293;
any one of Ala, Glu, and Met at amino acid position 307;
any one of Ala, Ile, Lys, Leu, Met, Val, and Trp at amino acid position 311;
Pro at amino acid position 312;
Lys at amino acid position 316;
Pro at amino acid position 317;
either Asn or Thr at amino acid position 318;
any one of Phe, His, Lys, Leu, Met, Arg, Ser, and Trp at amino acid position 332;
any one of Asn, Thr, and Trp at amino acid position 339;
Pro at amino acid position 341;
any one of Glu, His, Lys, Gln, Arg, Thr, and Tyr at amino acid position 343;
Arg at amino acid position 375;
any one of Gly, Ile, Met, Pro, Thr, and Val at amino acid position 376;
Lys at amino acid position 377;
either Asp or Asn at amino acid position 378;
any one of Asn, Ser, and Thr at amino acid position 380;
any one of Phe, His, Ile, Lys, Leu, Met, Asn, Gln, Arg, Ser, Thr, Val, Trp, and Tyr at amino acid position 382;
Asn at amino acid position 423;
Asn at amino acid position 427;
any one of Ala, Phe, Gly, His, Ile, Lys, Leu, Met, Asn, Gln, Arg, Ser, Thr, Val, and Tyr at amino acid position 430;
either His or Asn at amino acid position 431;
any one of Phe, Gly, His, Trp, and Tyr at amino acid position 434;
any one of Ile, Leu, and Thr at amino acid position 436;
any one of Lys, Leu, Thr, and Trp at amino acid position 438;
Lys at amino acid position 440; and
Lys at amino acid position 442;
according to EU numbering. The number of amino acids to be altered is not particularly limited, and the amino acid may be altered only at two positions or at three or more positions.

When an Fc region of human IgG1 is included as the Fc region, a non-limiting embodiment of alterations that produce effects of enhancing binding to FcRn under an acidic pH range condition compared to the binding activity of the starting Fc region of human IgG1 can be alterations comprising Ile at amino acid position 257 and Ile at amino acid position 311 according to EU numbering. In another non-limiting embodiment of the alterations, the alterations may include Ile at amino acid position 257 and His at amino acid position 434. In yet another non-limiting embodiment of the alterations, the alterations may include Val at amino acid position 376 and His at amino acid position 434.

Furthermore, as described later, for the FcRn-binding domain included in an antigen-binding molecule of the present invention, an Fc region having FcRn-binding activity under a neutral pH range condition may also be preferably used. Such Fc region can be obtained by any method according to the aforementioned method of obtaining Fc regions having FcRn-binding activity under an acidic pH range condition, but specifically, an FcRn-binding domain having a binding activity or having enhanced binding activity to FcRn under a neutral pH range condition can be obtained by altering amino acids of a human IgG immunoglobulin used as the starting Fc region. Preferred Fc regions of IgG immunoglobulins for the alteration include Fc regions of human IgG (IgG1, IgG2, IgG3, IgG4, and variants thereof). For alterations to other amino acids, amino acid at any position may be altered as long as Fc region has FcRn-binding activity under a neutral pH range condition or the binding activity to human FcRn under an acidic pH range condition can be increased. When an antigen-binding molecule includes an Fc region of a human IgG1 as the Fc region, it preferably comprises an alteration that has effects of enhancing binding to FcRn under a neutral pH range condition compared to the binding activity of the starting Fc region of human IgG1. Preferred examples of such altered Fc regions include human FcRn-binding domains in which at least one or more amino acids selected from the group consisting of amino acids at positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 in the starting Fc region site according to EU numbering are different from the corresponding amino acids in the native Fc region.

Preferred examples of such altered Fc regions include Fc regions comprising at least one or more amino acids selected from the group consisting of:

Met at amino acid position 237;
Ala at amino acid position 238;
Lys at amino acid position 239;
Ile at amino acid position 248;
any one of Ala, Phe, Ile, Met, Gln, Ser, Val, Trp, and Tyr at amino acid position 250;
any one of Phe, Trp, and Tyr at amino acid position 252;
Thr at amino acid position 254;
Glu at amino acid position 255;
any one of Asp, Glu, and Gln at amino acid position 256;
any one of Ala, Gly, Ile, Leu, Met, Asn, Ser, Thr, and Val at amino acid position 257;
His at amino acid position 258;
Ala at amino acid position 265;
Phe at amino acid position 270;
either Ala or Glu at amino acid position 286;
His at amino acid position 289;
Ala at amino acid position 297;
Gly at amino acid position 298;
Ala at amino acid position 303;
Ala at amino acid position 305;
any one of Ala, Asp, Phe, Gly, His, Ile, Lys, Leu, Met, Asn, Pro, Gln, Arg, Ser, Val, Trp, and Tyr at amino acid position 307;
any one of Ala, Phe, Ile, Leu, Met, Pro, Gln, and Thr at amino acid position 308;
any one of Ala, Asp, Glu, Pro, and Arg at amino acid position 309;
any one of Ala, His, and Ile at amino acid position 311;
either Ala or His at amino acid position 312;
either Lys or Arg at amino acid position 314;
either Ala or His at amino acid position 315;
Ala at amino acid position 317;
Gly at amino acid position 325;
Val at amino acid position 332;
Leu at amino acid position 334;
His at amino acid position 360;
Ala at amino acid position 376;
Ala at amino acid position 380;
Ala at amino acid position 382;
Ala at amino acid position 384;
either Asp or His at amino acid position 385;
Pro at amino acid position 386;
Glu at amino acid position 387;
either Ala or Ser at amino acid position 389;
Ala at amino acid position 424;
any one of Ala, Asp, Phe, Gly, His, Ile, Lys, Leu, Asn, Pro, Gln, Ser, Thr, Val, Trp, and Tyr at amino acid position 428;
Lys at amino acid position 433;
any one of Ala, Phe, His, Ser, Trp, and Tyr at amino acid position 434; and
His at amino acid position 436;
in the Fc region according to EU numbering.

For example, by using these amino acid alterations alone or multiple alterations in combination, binding of an IgG Fc region to FcRn in an acidic pH range and/or neutral pH range can be enhanced; however, the amino acid alterations to be introduced are not particularly limited, and as long as the effect of improving plasma retention is conferred, any amino acid alteration may be introduced.

Pharmaceutical compositions

When a conventional neutralizing antibody against a soluble antigen is administered, the plasma retention of the antigen is expected to be prolonged by binding to the antibody. In general, antibodies have a long half-life (one week to three weeks) while the half-life of antigen is generally short (one day or less). Meanwhile, antibody-bound antigens have a significantly longer half-life in plasma as compared to when the antigens are present alone. For this reason, administration of existing neutralizing antibody results in an increased antigen concentration in plasma. Such cases have been reported with various neutralizing antibodies that target soluble antigens including, for example, IL-6 (J. Immunotoxicol. (2005) 3, 131-139), amyloid beta (mAbs (2010) 2 (5), 1-13), MCP-1 (ARTHRITIS & RHEUMATISM (2006) 54, 2387-2392), hepcidin (AAPS J. (2010) 4, 646-657), and sIL-6 receptor (Blood (2008) 112 (10), 3959-64). Administration of existing neutralizing antibodies has been reported to increase the total plasma antigen concentration to about 10 to 1,000 times (the level of increase varies depending on antigen) the base line. Herein, the total plasma antigen concentration refers to a concentration as a total amount of antigen in plasma, *i.e.,* the sum of concentrations of antibody-bound and antibody-unbound antigens. An increase in the total plasma antigen concentration is undesirable for such antibody pharmaceuticals that target a soluble antigen. The reason is that the antibody concentration has to be higher than at least the total plasma antigen concentration to neutralize the soluble antigen. Specifically, "the total plasma antigen concentration is increased to 10 to 1,000 times" means that, in order to neutralize the antigen, the plasma antibody concentration (*i.e.,* antibody dose) has to be 10 to 1,000 times higher as compared to when increase in the total plasma antigen concentration does not occur. Conversely, if the total plasma antigen concentration can be reduced by 10 to 1,000 times as compared to the existing neutralizing antibody, the antibody dose can also be reduced to similar extent. Thus, antibodies capable of decreasing the total plasma antigen concentration by eliminating the soluble antigen from plasma are highly useful as compared to existing neutralizing antibodies.

While the present invention is not restricted to a particular theory, the reason for increase in the number of antigens that can bind to a single antigen-binding molecule and the reason for enhanced dissipation of plasma antigen concentration when an antigen-binding molecule is administered to a living organism which leads to increase of uptake of the antigen-binding molecule into cells *in vivo,* wherein the antigen-binding molecule comprises an antigen-binding domain whose antigen-binding activity changes depending on the ion-concentration condition so that the antigen-binding activity is lower under an acidic pH range condition than under a neutral pH range condition, and an FcRn-binding domain such as an antibody constant region having human Fcγ receptor-binding activity under a neutral pH range condition, can be explained as follows.

For example, when an antibody that binds to a membrane antigen is administered *in vivo,* after binding to an antigen, the antibody is, in a state bound to the antigen, incorporated into the endosome *via* intracellular internalization. Then, the antibody is transferred to the lysosome while remaining bound to the antigen, and is degraded together with the antigen there. The internalization-mediated elimination from plasma is referred to as antigen-dependent elimination, and has been reported for many antibody molecules (Drug Discov Today (2006) 11(1-2), 81-88). When a single IgG antibody molecule binds to antigens in a divalent manner, the single antibody molecule is internalized while remaining bound to the two antigens, and is degraded in the lysosome. In the case of typical antibodies, thus, a single IgG antibody molecule cannot bind to three antigen molecules or more. For example, a single IgG antibody molecule having a neutralizing activity cannot neutralize three antigen molecules or more.

The plasma retention of IgG molecule is relatively long (the elimination is slow) since FcRn, which is known as a salvage receptor for IgG molecule, functions. IgG molecules incorporated into endosomes by pinocytosis bind under the endosomal acidic condition to FcRn expressed in endosomes. IgG molecules that cannot bind to human FcRn are transferred to lysosomes and degraded there. Meanwhile, IgG molecules bound to FcRn are transferred to cell surface. The IgG molecules are dissociated from FcRn under the neutral condition in plasma, and thus they are recycled back to plasma.

Alternatively, when antigen-binding molecules are antibodies that bind to a soluble antigen, the *in vivo* administered antibodies bind to antigens, and then the antibodies are incorporated into cells while remaining bound to the antigens. Most of antibodies incorporated into cells bind to FcRn in the endosome and then are transferred to cell surface. The antibodies are dissociated from FcRn under the neutral condition in plasma and released to the outside of cells. However, antibodies having typical antigen-binding domains whose antigen-binding activity does not vary depending on ion concentration condition such as pH are released to the outside of cells while remaining bound to the antigens, and thus cannot bind to an antigen again. Thus, like antibodies that bind to membrane antigens, single typical IgG antibody molecule whose antigen-binding activity does not vary depending on ion concentration condition such as pH cannot bind to three antigen molecules or more.

Antibodies that bind to antigens in a pH-dependent manner, which strongly bind to antigens under the neutral pH range condition in plasma and are dissociated from antigens under the endosomal acidic pH range condition (antibodies that bind to antigens under the neutral pH range condition and are dissociated under an acidic pH range condition), and antibodies that bind to antigens in a calcium ion concentration-dependent manner, which strongly bind to antigens under a high calcium ion concentration condition in plasma and are dissociated from antigens under a low calcium ion concentration condition in the endosome (antibodies that bind to antigens under a high calcium ion concentration condition and are dissociated under a low calcium ion concentration condition) can be dissociated from antigen in the endosome. Antibodies that bind to antigens in a pH-dependent manner or in a calcium ion concentration-dependent manner, when recycled to plasma by FcRn after dissociation from antigens, can again bind to an antigen. Thus, such single antibody molecule can repeatedly bind to several antigen molecules. Meanwhile, antigens bound to antigen-binding molecules are dissociated from antibody in the endosome and degraded in the lysosome without recycling to plasma. By administering such antigen-binding molecules *in vivo,* antigen uptake into cells is accelerated, and it is possible to decrease plasma antigen concentration.

Uptake of antigens bound by antigen-binding molecules into cells are further enhanced by conferring or enhancing the Fcγ receptor-binding activity under the neutral pH range condition (pH 7.4) to antibodies that bind to antigens in a pH-dependent manner, which strongly bind to antigens under the neutral pH range condition in plasma and are dissociated from antigens under the endosomal acidic pH range condition (antibodies that bind to antigens under the neutral pH range condition and are dissociated under an acidic pH range condition), and antibodies that bind to antigens in a calcium ion concentration-dependent manner, which strongly bind to antigens under a high calcium ion concentration condition in plasma and are dissociated from antigens under a low calcium ion concentration condition in the endosome (antibodies that bind to antigens under a high calcium ion concentration condition and are dissociated under a low calcium ion concentration condition). Specifically, by administering such antigen-binding molecules *in vivo,* the antigen elimination is accelerated, and it is possible to reduce plasma antigen concentration. Typical antibodies that do not have the ability to bind to antigens in a pH-dependent manner or in a calcium ion concentration-dependent manner, and antigen-antibody complexes of such antibodies are incorporated into cells by non-specific endocytosis, and transported onto cell surface by binding to FcRn under the endosomal acidic condition. They are dissociated from FcRn under the neutral condition on cell surface and recycled to plasma. Thus, when an antibody that binds to an antigen in a fully pH-dependent manner (that binds under the neutral pH range condition and is dissociated under an acidic pH range condition) or in a fully calcium ion concentration-dependent manner (that binds under a high calcium ion concentration condition and is dissociated under a low calcium ion concentration condition) binds to an antigen in plasma and is dissociated from the antigen in the endosome, the rate of antigen elimination is considered to be equal to the rate of uptake into cells of the antibody or antigen/antibody complex by non-specific endocytosis. When the pH or calcium ion concentration dependency of antigen/antibody binding is insufficient, antigens that are not dissociated from antibodies in the endosome are, along with the antibodies, recycled to plasma. On the other hand, when the pH dependency is sufficiently strong, the rate limiting step of antigen elimination is the cellular uptake by non-specific endocytosis. Meanwhile, FcRn transports antibodies from the endosome to the cell surface, and a fraction of FcRn is expected to be also distributed on the cell surface.

The present inventors considered that IgG immunoglobulins having binding activity or having enhanced binding activity to Fcγ receptors under a neutral pH range condition can bind to Fcγ receptors present on the cell surface, and that the IgG immunoglobulins are taken up into cells in an Fcγ receptor-dependent manner by binding to Fcγ receptors present on the cell surface. The rate of uptake into cells *via* Fcγ receptors is faster than the rate of uptake into cells by non-specific endocytosis. Therefore, it is thought that the rate of antigen elimination by antigen-binding molecules can further be accelerated by enhancing the ability to bind to Fcγ receptors under a neutral pH range condition. That is, antigen-binding molecules having the ability to bind to Fcγ receptors under a neutral pH range condition uptake antigens into cells more quickly than common (native human) IgG immunoglobulins, and after binding to FcRn in the endosome and dissociating the antigens, they are recycled again into plasma, and they bind again to antigens and are taken up into cells *via* Fcγ receptors. Since turnover of this cycle can be increased by increasing the ability to bind to Fcγ receptors under a neutral pH range condition, the rate of antigen elimination from plasma is accelerated. Furthermore, by making antigen-binding molecules that have decreased antigen-binding activity under an acidic pH range condition compared to antigen-binding activity under a neutral pH range condition, the rate of antigen elimination from plasma can further be accelerated. By increasing the number of cycles resulting from accelerating the rate of turnover of this cycle, the number of antigen molecules that can be bound by a single antigen-binding molecule may increase. Antigen-binding molecules of the present invention comprise an antigen-binding domain and an Fcγ receptor-binding domain, and since the Fcγ receptor-binding domain does not affect antigen binding, and based on the above-mentioned mechanism, it is considered that regardless of the type of antigens, antigen uptake into cells by antigen-binding molecules can be enhanced and the rate of antigen elimination can be accelerated by reducing binding activity (binding ability) of the antigen-binding molecule to antigens under an ion concentration condition such as acidic pH range or low calcium ion concentration condition compared to binding activity (binding ability) to antigens under an ion concentration condition such as neutral pH range or high calcium ion concentration condition, and/or enhancing binding activity to Fcγ receptors at pH in plasma. Therefore, antigen-binding molecules of the present invention may exhibit better effects than conventional therapeutic antibodies in terms of reduction of side effects caused by antigens, increase in antibody dose, and improvement of *in vivo* kinetics of antibodies.

Fig. 1 shows an embodiment of the mechanism for eliminating soluble antigens from plasma by administering ion concentration-dependent antigen-binding antibodies with enhanced binding to Fcγ receptors at neutral pH compared to conventional neutralizing antibodies. Herein below, hydrogen ion concentration (that is, pH)-dependent antigen-binding antibodies will be described as an example of the ion concentration-dependent antigen-binding antibodies, but the mechanisms are not limited to hydrogen ion concentration. Existing neutralizing antibodies which do not have pH-dependent antigen-binding ability may be gradually taken up mainly by non-specific interactions with cells after binding to soluble antigens in plasma. The complexes of neutralizing antibodies and soluble antigens, which are taken up into cells, translocate to acidic endosomes and are recycled into plasma by FcRn. On the other hand, a pH-dependent antigen-binding antibody with enhanced binding to Fcγ receptors under a neutral condition binds to a soluble antigen in plasma, and thereafter, it may be quickly taken up into cells expressing Fcγ receptors on the cell surface *via* interaction with an Fcγ receptor besides non-specific interaction. Here, the soluble antigens bound to pH-dependent antigen-binding antibody dissociate from the antibody due to pH-dependent binding ability in the acidic endosomes. Soluble antigens that have dissociated from the antibody then translocate to lysosomes, and they are degraded by proteolysis. Meanwhile, antibodies that released the soluble antigens bind to FcRn in the acidic endosome, and are then recycled onto the cell membrane by FcRn, and are released again into plasma. In this way, the free antibodies that are recycled can bind again to other soluble antigens. Such pH-dependent antigen-binding antibodies whose binding to Fcγ receptors under a neutral condition can translocate a large amount of soluble antigens to lysosomes to reduce the total antigen concentration in plasma by repeating a cycle of: uptake into cells *via* Fcγ receptors; dissociation and degradation of soluble antigens; and antibody recycling.

Specifically, the present invention also relates to pharmaceutical compositions comprising antigen-binding molecules of the present invention, antigen-binding molecules produced by alteration methods of the present invention, or antigen-binding molecules produced by production methods of the present invention. Antigen-binding molecules of the present invention or antigen-binding molecules produced by production methods of the present invention are useful as pharmaceutical compositions since they, when administered, have the strong effect to reduce the plasma antigen concentration as compared to typical antigen-binding molecules, and exhibit the improved *in vivo* immune response, pharmacokinetics, and others in animals administered with the molecules. The pharmaceutical compositions of the present invention may comprise pharmaceutically acceptable carriers.

In the present invention, pharmaceutical compositions generally refer to agents for treating or preventing, or testing and diagnosing diseases.

The pharmaceutical compositions of the present invention can be formulated by methods known to those skilled in the art. For example, they can be used parenterally, in the form of injections of sterile solutions or suspensions including water or other pharmaceutically acceptable liquid. For example, such compositions can be formulated by mixing in the form of unit dose required in the generally approved medicine manufacturing practice, by appropriately combining with pharmacologically acceptable carriers or media, specifically with sterile water, physiological saline, vegetable oil, emulsifier, suspension, surfactant, stabilizer, flavoring agent, excipient, vehicle, preservative, binder, or such. In such formulations, the amount of active ingredient is adjusted to obtain an appropriate amount in a pre-determined range.

Sterile compositions for injection can be formulated using vehicles such as distilled water for injection, according to standard formulation practice.

Aqueous solutions for injection include, for example, physiological saline and isotonic solutions containing dextrose or other adjuvants (for example, D-sorbitol, D-mannose, D-mannitol, and sodium chloride). It is also possible to use in combination appropriate solubilizers, for example, alcohols (ethanol and such), polyalcohols (propylene glycol, polyethylene glycol, and such), non-ionic surfactants (polysorbate 80(TM), HCO-50, and such).

Oils include sesame oil and soybean oils. Benzyl benzoate and/or benzyl alcohol can be used in combination as solubilizers. It is also possible to combine buffers (for example, phosphate buffer and sodium acetate buffer), soothing agents (for example, procaine hydrochloride), stabilizers (for example, benzyl alcohol and phenol), and/or antioxidants. Appropriate ampules are filled with the prepared injections.

The pharmaceutical compositions of the present invention are preferably administered parenterally. For example, the compositions in the dosage form for injections, transnasal administration, transpulmonary administration, or transdermal administration are administered. For example, they can be administered systemically or locally by intravenous injection, intramuscular injection, intraperitoneal injection, subcutaneous injection, or such.

Administration methods can be appropriately selected in consideration of the patient's age and symptoms. The dose of a pharmaceutical composition containing an antigen-binding molecule can be, for example, from 0.0001 to 1,000 mg/kg for each administration. Alternatively, the dose can be, for example, from 0.001 to 100,000 mg per patient.

However, the present invention is not limited by the numeric values described above. The doses and administration methods vary depending on the patient's weight, age, symptoms, and such. Those skilled in the art can set appropriate doses and administration methods in consideration of the factors described above.

Amino acids contained in the amino acid sequences of the present invention may be post-translationally modified (for example, the modification of an N-terminal glutamine into a pyroglutamic acid by pyroglutamylation is well-known to those skilled in the art). Naturally, such post-translationally modified amino acids are included in the amino acid sequences in the present invention.

Methods that use antigen-binding molecules of the present invention

The present invention also provides a method comprising contacting an antigen-binding molecule with an Fcγ receptor-expressing cell *in vivo* or *ex vivo,* wherein the antigen-binding molecule has human FcRn-binding activity under an acidic pH range condition and comprises an antigen-binding domain and an Fcγ receptor-binding domain, in which antigen-binding activity of the antigen-binding domain changes depending on the ion concentration condition and the Fcγ receptor-binding domain has higher binding activity to the Fcγ receptor under a neutral pH range condition than a native Fcγ receptor-binding domain to which the sugar chain linked at position 297 (EU numbering) is a fucose-containing sugar chain, wherein the method is any one of the following:

(i) a method of increasing the number of antigens to which a single antigen-binding molecule can bind;
(ii) a method of eliminating plasma antigens;
(iii) a method of improving pharmacokinetics of the antigen-binding molecule;
(iv) a method of promoting intracellular dissociation of an antigen from the antigen-binding molecule, wherein the antigen was extracellularly bound to the antigen-binding molecule;
(v) a method of promoting extracellular release of an antigen-binding molecule in an antigen-unbound form; and
(vi) a method of decreasing the concentration of free antigen or the total antigen concentration in plasma.

Furthermore, the present invention provides a method comprising enhancing Fcγ receptor-binding activity under a neutral pH range condition of the Fcγ receptor-binding domain in an antigen-binding molecule compared to that of a native Fcγ receptor-binding domain to which the sugar chain linked at position 297 (EU numbering) is a fucose-containing sugar chain, wherein the antigen-binding molecule has human FcRn-binding activity under an acidic pH range condition and comprises an Fcγ receptor-binding domain and an antigen-binding domain whose antigen-binding activity changes depending on the ion concentration condition, wherein the method is any one of the following:

(i) a method of altering an antigen-binding molecule in which intracellular uptake of the antigen to be bound is enhanced;
(ii) a method of increasing the number of antigens to which a single antigen-binding molecule can bind;
(iii) a method of increasing the ability of the antigen-binding molecule to eliminate plasma antigens;
(iv) a method of improving pharmacokinetics of antigen-binding molecule;
(v) a method of promoting intracellular dissociation of an antigen from an antigen-binding molecule, wherein the antigen has been extracellularly bound to the antigen-binding molecule;
(vi) a method of promoting extracellular release of an antigen-binding molecule in an antigen-unbound form, wherein the antigen-binding molecule had been taken up into a cell in an antigen-bound form; and
(vii) a method of altering an antigen-binding molecule, which can decrease total antigen concentration or free antigen concentration in plasma.

Examples of methods of contacting an antigen-binding molecule with an Fcγ receptor-expressing cell *in vivo* or *ex vivo* include (1) the so-called *ex vivo* method where plasma containing the antigen-binding molecules and antigens that bind to the antigen-binding molecules is temporarily taken out from a living organism; contacted with cells expressing Fcγ receptors; and after a certain period of time, the plasma containing the extracellularly recycled (or also referred to as re-secreted or recirculated) antigen-binding molecules without bounded antigens is then placed back into the living organism, and (2) the method of administering the antigen-binding molecules to a living organism. In the method of (1), one may also utilize a method of: temporarily taking out from a living organism plasma containing the antigens to which the antigen-binding molecules bind; contacting with cells expressing the Fcγ receptors and antigen-binding molecules; and after a certain period of time, returning the plasma into the living organism.

In the present invention, Fcγ receptor-expressing cells are not limited to particular cells and any kind of cells may be used as long as they are cells that express the desired Fcγ receptor(s). To identify cells that express the desired Fcγ receptor(s), publicly known databases such as Human Protein Atlas (http://www.proteinatlas.org/) may be used. Furthermore, whether the receptors are expressed in cells used for contacting antigen-binding molecules of the present invention can be confirmed by a method that confirms expression of a gene encoding the desired Fcγ receptor(s), or by an immunological method that uses antibodies that bind to the desired Fcγ receptor(s). These methods are also publicly known. Since contact of Fcγ receptor-expressing cells with antigen-binding molecules and antigens that bind to the antigen-binding molecules is carried out *in vivo* as well, contacting Fcγ receptor-expressing cells with antigen-binding molecules in the present invention include administering the antigen-binding molecules to living organisms. The duration of contact is, for example, a suitable duration from among one minute to several weeks, 30 minutes to one week, one hour to three days, and two hours to one day. More specifically, the duration necessary for uptake of the antigen-binding molecules or antigens bound to the antigen-binding molecules into cells by endocytosis mediated by Fcγ receptors is appropriately employed for the duration of contact. For example, various immune cells may be used for the Fcγ receptor-expressing cells.

A method of enhancing binding activity of the Fcγ receptor-binding domain to an Fcγ receptor under a neutral pH range condition than that of a native Fcγ receptor-binding domain to which the sugar chain linked at position 297 (EU numbering) is a fucose-containing sugar chain is described in the later-described section on method for producing antigen-binding molecules of the present invention.

## Method of altering an antigen-binding molecule with enhanced intracellular uptake of antigens that bind to the antigen-binding molecule

The present invention provides methods of altering an antigen-binding molecule with enhanced intracellular uptake of antigens that bind to the antigen-binding molecule, wherein the method comprises enhancing, under a neutral pH range condition, Fcγ receptor-binding activity of the Fcγ receptor-binding domain in an antigen-binding molecule compared to that of a native Fcγ receptor-binding domain to which the sugar chain linked at position 297 (EU numbering) is a fucose-containing sugar chain, wherein the antigen-binding molecule has human FcRn-binding activity under an acidic pH range condition and comprises an Fcγ receptor-binding domain and an antigen-binding domain whose antigen-binding activity changes depending on the ion concentration condition.

In the present invention, "intracellular uptake of antigens" by an antigen-binding molecule means that antigens are taken up into cells by Fcγ receptor-mediated endocytosis and internalization. In the present invention, "enhance uptake into cells" means that rate of uptake into cells of antigen-binding molecules bound to antigens in plasma is increased and/or the amount of antigens that were taken up which are recycled in plasma is reduced, and it is only necessary that the rate of uptake into cells is increased compared to the antigen-binding molecule prior to reduction of antigen-binding activity (binding ability) of the antigen-binding molecule under an ion concentration condition such as an acidic pH range or low calcium ion concentration compared to the antigen-binding activity under an ion concentration condition such as a neutral pH range or high calcium ion concentration in addition to enhancing binding activity of the antigen-binding molecule to a Fcγ receptor in a neutral pH range, and it is preferred that the rate is increased compared to a native human IgG to which the sugar chain linked at position 297 (EU numbering) is a fucose-containing sugar chain, and it is particularly preferred that the rate is increased compared to any of a native human IgG1, IgG2, IgG3, or IgG4. Accordingly, in the present invention, whether intracellular uptake of antigens by antigen-binding molecules is enhanced can be determined by observing whether the rate of uptake of antigens into cells increased. The rate of uptake of antigens into cells can be calculated, for example, by adding the antigen-binding molecule and antigen to a culture medium containing Fcγ receptor-expressing cells and measuring the decrease in antigen concentration in the culture medium over time or measuring the amount of antigens taken up into the Fcγ receptor-expressing cells over time. Using the method of increasing the rate by which antigens are taken up into cells by the antigen-binding molecule of the present invention, for example, the rate of antigen elimination in plasma can be increased by administering the antigen-binding molecule. Therefore, whether uptake of antigen into cells by the antigen-binding molecule is enhanced can also be confirmed, for example, by assessing whether the rate of antigen elimination in plasma is accelerated, or whether administration of antigen-binding molecule reduces the total antigen concentration in plasma.

In the present invention, "total antigen concentration in plasma" means the sum of the concentrations of antigens bound to the antigen-binding molecules and unbound antigens, or "free antigen concentrations in plasma" which is the concentration of antigens not bound to antigen-binding molecules. Various methods for measuring "total antigen concentration in plasma" or " free antigen concentrations in plasma" are well-known in the art as herein described below.

In the present invention, "native human IgG" means unmodified human IgG, and is not limited to a particular class of IgG. Furthermore, in "native human IgG", it is desirable that the sugar chain linked at position 297 (EU numbering) is a fucose-containing sugar chain. This means that as long as human IgG1, IgG2, IgG3, or IgG4 can bind to human FcRn in an acidic pH range, it can be used as a "native human IgG". Preferably, "native human IgG" may be human IgG1.

## Method of increasing the number of antigens that can be bound by a single antigen-binding molecule

The present invention provides a method of increasing the number of antigens to which a single antigen-binding molecule can bind, wherein the method comprises contacting an antigen-binding molecule with an Fcγ receptor-expressing cell *in vivo* or *ex vivo,* wherein the antigen-binding molecule has human FcRn-binding activity under an acidic pH range condition and comprises an antigen-binding domain and an Fcγ receptor-binding domain, in which an antigen-binding activity of the antigen-binding domain changes depending on the ion concentration condition and the Fcγ receptor-binding domain has higher binding activity to the Fcγ receptor under a neutral pH range condition compared to a native Fcγ receptor-binding domain to which the sugar chain linked at position 297 (EU numbering) is a fucose-containing sugar chain.

Furthermore, the present invention provides a method of increasing the number of antigens to which a single antigen-binding molecule can bind, wherein the method comprises enhancing Fcγ receptor-binding activity under a neutral pH range condition of the Fcγ receptor-binding domain in an antigen-binding molecule compared to that of a native Fcγ receptor-binding domain to which the sugar chain linked at position 297 (EU numbering) is a fucose-containing sugar chain, wherein the antigen-binding molecule has human FcRn-binding activity under an acidic pH range condition and comprises an Fcγ receptor-binding domain and an antigen-binding domain whose antigen-binding activity changes depending on the ion concentration condition.

The phrase "number of antigens to which a single antigen-binding molecule can bind" in the present invention means the number of antigens to which an antigen-binding molecule can bind until the molecule is degraded and eliminated. The phrase "increasing the number of antigens to which a single antigen-binding molecule can bind" in the present invention refers to increasing the number of times an antigen molecule bound to the antigen-binding molecule dissociates and the antigen-binding molecule binds again to an antigen molecule. Antigen molecules that bind to the antigen-binding molecule may be the same antigen molecule or different molecules existing in the reaction system where both molecules are present. In other words, it refers to the total number of times the antigen-binding molecule binds to an antigen in the reaction system. In another expression, when one cycle is assumed to be intracellular uptake of an antigen-binding molecule bound to an antigen, dissociation of the antigen in an endosome, and then return of the antigen-binding molecule to the outside of the cell, the phrase refers to increase in the number of times this cycle can be repeated until the antigen-binding molecule is degraded and eliminated. After binding to an Fcγ receptor, antigen-binding molecules of the present invention having Fcγ receptor-binding activity in a neutral pH range is taken up into a cell expressing this Fcγ receptor by endocytosis. The antigen-binding molecule of the present invention that dissociated from the Fcγ receptor under an ion concentration condition such as an acidic pH range or low calcium ion concentration is recycled to the outside of the cell again by binding to FcRn under an acidic pH range condition. The antigen-binding molecule of the present invention which is recycled to outside the cell after dissociating antigen from the antigen-binding molecule under an ion concentration condition such as an acidic pH range or low calcium ion concentration can bind again to an antigen. Therefore, whether the number of cycles increased may be assessed by determining whether the aforementioned " intracellular uptake is enhanced", or whether the later described " pharmacokinetics is improved".

Method of eliminating plasma antigens or method of increasing the ability of the antigen-binding molecule to eliminate plasma antigens

The present invention provides a method of eliminating plasma antigens, which comprises contacting an antigen-binding molecule with an Fcγ receptor-expressing cell *in vivo* or *ex vivo,* wherein the antigen-binding molecule has human FcRn-binding activity under an acidic pH range condition and comprises an antigen-binding domain and an Fcγ receptor-binding domain, in which an antigen binding activity of the antigen-binding domain changes depending on the ion concentration condition, and the Fcγ receptor-binding domain has higher binding activity to the Fcγ receptor under a neutral pH range condition compared to a native Fcγ receptor-binding domain to which the sugar chain linked at position 297 (EU numbering) is a fucose-containing sugar chain.

Furthermore, the present invention provides a method of increasing the ability of the antigen-binding molecule to eliminate plasma antigens, wherein the method comprises enhancing Fcγ receptor-binding activity under a neutral pH range condition of the Fcγ receptor-binding domain in an antigen-binding molecule compared to that of a native Fcγ receptor-binding domain to which the sugar chain linked at position 297 (EU numbering) is a fucose-containing sugar chain, wherein the antigen-binding molecule has human FcRn-binding activity under an acidic pH range condition and comprises an Fcγ receptor-binding domain and an antigen-binding domain whose antigen-binding activity changes depending on the ion concentration condition.

In the present invention, the phrase "method of increasing the ability to eliminate plasma antigens" has the same meaning as "method of increasing the ability of an antigen-binding molecule to eliminate antigens from plasma".

In the present invention, "ability to eliminate plasma antigens" refers to the ability to eliminate from plasma the antigens present in plasma when antigen-binding molecules are administered *in vivo* or antigen-binding molecules are secreted *in vivo.* Therefore, in the present invention, all that the phrase "an ability of an antigen-binding molecule to eliminate plasma antigens increases" has to mean is that when an antigen-binding molecule is administered, rate of antigen elimination from the plasma is accelerated compared to before reducing the antigen-binding activity of the antigen-binding molecule under an ion concentration condition such as acidic pH range or low calcium ion concentration compared to the antigen-binding activity under a neutral pH range or high calcium ion concentration in addition to enhancing binding activity of the antigen-binding molecule to a Fcγ receptor in a neutral pH range. Whether the ability of antigen-binding molecules to eliminate plasma antigens increases can be determined, for example, by administering soluble antigens and antigen-binding molecules to a living organism and measuring the plasma concentration of the soluble antigens after the administration. When the concentration of soluble antigens in plasma is decreased after administration of soluble antigens and antigen-binding molecules by enhancing the binding activity of the antigen-binding molecules to Fcγ receptors under a neutral pH range condition, or by reducing the antigen-binding activity of the antigen-binding molecule under an ion concentration condition such as acidic pH range or low calcium ion concentration compared to the antigen-binding activity under an ion concentration condition such as a neutral pH range or high calcium ion concentration in addition to enhancing Fcγ receptor-binding activity, it can be determined that the ability of antigen-binding molecules to eliminate plasma antigens is enhanced. Soluble antigens may be antigens to which antigen-binding molecules are actually bounded (antigens in the form of an antigen/antigen-binding molecule complex), or antigens to which antigen-binding molecules are not bound, and their concentrations may be determined as "concentration of antigen-binding molecule-bound antigens in plasma" and "concentration of antigen-binding molecule-unbound antigens in plasma", respectively (the latter has the same meaning as " free antigen concentrations in plasma"). Since "total antigen concentration in plasma" means the sum of the concentrations of antigen-binding molecule-bound antigens and antigen-binding molecule-unbound antigens, or "concentration of free antigens in plasma" which is the concentration of antigen-binding molecule-unbound antigens, soluble antigen concentration can be determined as "total antigen concentration in plasma". Various methods of measuring "total antigen concentration in plasma" or "free antigen concentrations in plasma" are well-known in the art as herein described below.

Method of improving the pharmacokinetics of antigen-binding molecules

The present invention provides a method of improving pharmacokinetics of an antigen-binding molecule, which comprises contacting an antigen-binding molecule with an Fcγ receptor-expressing cell *in vivo* or *ex vivo,* wherein the antigen-binding molecule has human FcRn-binding activity under an acidic pH range condition and comprises an antigen-binding domain and an Fcγ receptor-binding domain, in which an antigen-binding activity of the antigen-binding domain changes depending on the ion concentration condition, and the Fcγ receptor-binding domain has higher binding activity to the Fcγ receptor under a neutral pH range condition compared to a native Fcγ receptor-binding domain to which the sugar chain linked at position 297 (EU numbering) is a fucose-containing sugar chain.

Furthermore, the present invention provides a method of improving the pharmacokinetics of an antigen-binding molecule, wherein the method comprises enhancing Fcγ receptor-binding activity under a neutral pH range condition of the Fcγ receptor-binding domain in an antigen-binding molecule compared to that of a native Fcγ receptor-binding domain to which the sugar chain linked at position 297 (EU numbering) is a fucose-containing sugar chain, wherein the antigen-binding molecule having human FcRn-binding activity under an acidic pH range condition comprises an Fcγ receptor-binding domain and an antigen-binding domain whose antigen-binding activity changes depending on the ion concentration condition.

Herein, "enhancement of pharmacokinetics", "improvement of pharmacokinetics", and "superior pharmacokinetics" can be restated as "enhancement of plasma (blood) retention", "improvement of plasma (blood) retention", "superior plasma (blood) retention", and "prolonged plasma (blood) retention". These terms are synonymous.

Herein, "improvement of pharmacokinetics" means not only prolongation of the period until elimination from the plasma (for example, until the antigen-binding molecule is degraded intracellularly or the like and cannot return to the plasma) after administration of the antigen-binding molecule to humans, or non-human animals such as mice, rats, monkeys, rabbits, and dogs, but also prolongation of the plasma retention of the antigen-binding molecule in a form that allows antigen binding (for example, in an antigen-free form of the antigen-binding molecule) during the period of administration to elimination due to degradation. Native IgG can bind to FcRn from non-human animals. For example, mouse can be preferably used to be administered in order to confirm the property of the antigen-binding molecule of the invention since native human IgG can bind to mouse FcRn stronger than to human FcRn (Int Immunol. (2001) 13(12): 1551-1559). As another example, mouse in which its native FcRn genes are deficient and a transgene for human FcRn gene is harbored to be expressed (Methods Mol Biol. 2010; 602: 93-104) can also be preferably used as a subject to be administered in order to confirm the property of the antigen-binding molecule of the invention described hereinafter. Specifically, "improvement of pharmacokinetics" also includes prolongation of the period until elimination due to degradation of the antigen-binding molecule not bound to antigens (the antigen-free form of antigen-binding molecule). The antigen-binding molecule in plasma cannot bind to a new antigen if the antigen-binding molecule has already bound to an antigen. Thus, the longer the period that the antigen-binding molecule is not bound to an antigen, the longer the period that it can bind to a new antigen (the higher the chance of binding to another antigen). This enables reduction of the time period that an antigen is free of the antigen-binding molecule *in vivo* and prolongation of the period that an antigen is bound to the antigen-binding molecule. The plasma concentration of the antigen-free form of antigen-binding molecule can be increased and the period that the antigen is bound to the antigen-binding molecule can be prolonged by accelerating the antigen elimination from the plasma by administration of the antigen-binding molecule. Specifically, herein "improvement of the pharmacokinetics of antigen-binding molecule" includes the improvement of a pharmacokinetic parameter of the antigen-free form of the antigen-binding molecule (any of prolongation of the half-life in plasma, prolongation of mean retention time in plasma, and impairment of plasma clearance), prolongation of the period that the antigen is bound to the antigen-binding molecule after administration of the antigen-binding molecule, and acceleration of antigen-binding molecule-mediated antigen elimination from the plasma. The improvement of pharmacokinetics of antigen-binding molecule can be assessed by determining any one of the parameters, half-life in plasma, mean plasma retention time, and plasma clearance for the antigen-binding molecule or the antigen-free form thereof ("Pharmacokinetics: Enshu-niyoru Rikai (Understanding through practice)" Nanzando). For example, the plasma concentration of the antigen-binding molecule or antigen-free form thereof is determined after administration of the antigen-binding molecule to mice, rats, monkeys, rabbits, dogs, or humans. Then, each parameter is determined. When the plasma half-life or mean plasma retention time is prolonged, the pharmacokinetics of the antigen-binding molecule can be judged to be improved. The parameters can be determined by methods known to those skilled in the art. The parameters can be appropriately assessed, for example, by noncompartmental analysis using the pharmacokinetics analysis software WinNonlin (Pharsight) according to the appended instruction manual. The plasma concentration of antigen-free antigen-binding molecule can be determined by methods known to those skilled in the art, for example, using the assay method measured in a known method (Clin Pharmacol. 2008 Apr; 48(4): 406-417).

Herein, "improvement of pharmacokinetics" also includes prolongation of the period that an antigen is bound to an antigen-binding molecule after administration of the antigen-binding molecule. Whether the period that an antigen is bound to the antigen-binding molecule after administration of the antigen-binding molecule is prolonged can be assessed by determining the plasma concentration of free antigen. The prolongation can be judged based on the determined plasma concentration of free antigen or the time period required for an increase in the ratio of free antigen concentration to the total antigen concentration.

The plasma concentration of free antigen not bound to the antigen-binding molecule or the ratio of free antigen concentration to the total concentration can be determined by methods known to those skilled in the art, for example, the method measured in Pharm Res. 2006 Jan; 23 (1): 95-103 can be used. Alternatively, when an antigen exhibits a particular function in vivo, whether the antigen is bound to an antigen-binding molecule that neutralizes the antigen function (antagonistic molecule) can be deteremined by testing whether the antigen function is neutralized. Whether the antigen function is neutralized can be assessed by assaying an *in vivo* marker that reflects the antigen function. Whether the antigen is bound to an antigen-binding molecule that activates the antigen function (agonistic molecule) can be assessed by assaying an in vivo marker that reflects the antigen function.

Determination of the plasma concentration of free antigen and ratio of the amount of free antigen in plasma to the amount of total antigen in plasma, *in vivo* marker assay, and such measurements are not particularly limited; however, the assays are preferably carried out after a certain period of time has passed after administration of the antigen-binding molecule. In the present invention, the period after administration of the antigen-binding molecule is not particularly limited; those skilled in the art can determine the appropriate period depending on the properties and the like of the administered antigen-binding molecule. Such periods include, for example, one day after administration of the antigen-binding molecule, three days after administration of the antigen-binding molecule, seven days after administration of the antigen-binding molecule, 14 days after administration of the antigen-binding molecule, and 28 days after administration of the antigen-binding molecule. Herein, "plasma antigen concentration" refers to either "total antigen concentration in plasma" which is the sum of antigen-binding molecule bound antigen and non-bound antigen concentration or "free antigen concentration in plasma" which is antigen-binding molecule non-bound antigen concentration.

Total antigen concentration in plasma can be lowered by administration of antigen-binding molecule of the present invention by 2-fold, 5-fold, 10-fold, 20-fold, 50-fold, 100-fold, 200-fold, 500-fold, 1,000-fold, or even higher compared to the administration of a reference antigen-binding molecule comprising the native human IgG Fc region in which a sugar chain linked to at position 297 (EU numbering) is a sugar chain having fucose as an Fcγ receptor-binding domain or compared to when antigen-binding domain molecule of the present invention comprising the antigen-binding domain is not administered.

Molar antigen/antigen-binding molecule ratio can be calculated as shown below;

value A: Molar antigen concentration at each time point
value B: Molar antigen-binding molecule concentration at each time point
value C: Molar antigen concentration per molar antigen-binding molecule concentration (molar antigen/antigen-binding molecule ratio) at each time point

$$C=A/B.$$

Smaller value C indicates higher efficiency of antigen elimination per antigen-binding molecule whereas higher value C indicates lower efficiency of antigen elimination per antigen-binding molecule.

Molar antigen/antigen-binding molecule ratio can be calculated as described above.

Molar antigen/antigen-binding molecule ratio can be lowered by administration of antigen-binding molecule of present invention by 2-fold, 5-fold, 10-fold, 20-fold, 50-fold, 100-fold, 200-fold, 500-fold, 1,000-fold, or even higher as compared to the administration of a reference antigen-binding molecule comprising the wild-type human IgG Fc region as a human Fcγ receptor-binding domain.

In the present invention, a native human IgG I, IgG2, IgG3 or IgG4 is preferably used as the native human IgG which is used as a reference native human IgG to be compared with the antigen-binding molecules for their Fcγ receptor-binding activity or *in vivo* activity. Preferably, a reference antigen-binding molecule comprising the same antigen-binding domain as the antigen-binding molecule of interest and a native human IgG Fc region as the Fcγ receptor-binding domain can be appropriately used. More preferably, a native human IgG1 is used as a reference native human IgG to be compared with the antigen-binding molecules for their Fcγ receptor-binding activity or *in vivo* activity. Furthermore, in the present invention, as a reference antigen-binding molecule to be compared with the antigen-binding molecules of the present invention, an antigen-binding molecule comprising an antigen-binding domain whose antigen-binding activity does not change depending on the ion concentration, an antigen-binding molecule comprising an FcRn-binding domain whose FcRn-binding activity under an acidic pH range condition has not been enhanced, an antigen-binding molecule comprising an Fcγ receptor-binding domain that does not have selective binding activity to Fcγ receptors, or such may also be appropriately used depending on the purpose.

Reduction of total antigen concentration in plasma or molar antigen/antibody ratio can be assessed as described in Examples 6, 8, and 13. More specifically, using human FcRn transgenic mouse line 32 or line 276 (Jackson Laboratories, Methods Mol Biol. 2010; 602: 93-104), they can be assessed by either antigen-antibody co-administration model or steady-state antigen infusion model when antigen-binding molecule do not cross-react to the mouse counterpart antigen. When an antigen-binding molecule cross-react with mouse counterpart, they can be assessed by simply administering antigen-binding molecule to human FcRn transgenic mouse line 32 or line 276 (Jackson Laboratories). In co-administration model, mixture of antigen-binding molecule and antigen is administered to the mouse. In steady-state antigen infusion model, infusion pump containing antigen solution is implanted to the mouse to achieve constant plasma antigen concentration, and then antigen-binding molecule is administered to the mouse. Test antigen-binding molecule is administered at same dosage. Total antigen concentration in plasma, free antigen concentration in plasma and plasma antigen-binding molecule concentration is measured at appropriate time point using method known to those skilled in the art.

For assesing the effects of an Fcγ receptor-binding domain having selective binding activity to Fcγ receptors, when an antigen-binding molecule does not cross-react with a mouse counterpart antigen, total antigen concentration in plasma or decrease in antigen/antibody mole ratio can be assessed by either the antigen-antibody simultaneous injection model or the steady-state antigen injection model using the conventionally used C57BL/6J mice (Charles River Japan). When an antigen-binding molecule cross-reacts with the mouse counterpart, the antigen-binding molecule can simply be injected to conventionally used C57BL/6J mice (Charles River Japan) to carry out the assessment.

Total or free antigen concentration in plasma and molar antigen/antigen-binding molecule ratio can be measured at 2, 4, 7, 14, 28, 56, or 84 days after administration to evaluate the long-term effect of the present invention. In other words, a long term plasma antigen concentration is determined by measuring total or free antigen concentration in plasma and molar antigen/ antigen-binding molecule ratio at 2, 4, 7, 14, 28, 56, or 84 days after administration of an antigen-binding molecule in order to evaluate the property of the antigen-binding molecule of the present invention. Whether the reduction of plasma antigen concentration or molar antigen/antigen-binding molecule ratio is achieved by antigen-binding molecule described in the present invention can be determined by the evaluation of the reduction at any one or more of the time points described above.

Total or free antigen concentration in plasma and molar antigen/antigen-binding molecule ratio can be measured at 15 min, 1, 2, 4, 8, 12, or 24 hours after administration to evaluate the short-term effect of the present invention. In other words, a short term plasma antigen concentration is determined by measuring total or free antigen concentration in plasma and molar antigen/antigen-binding molecule ratio at 15 min, 1, 2, 4, 8, 12, or 24 hours after administration of an antigen-binding molecule in order to evaluate the property of the antigen-binding molecule of the present invention.

Route of administration of an antigen-binding molecule of the present invention can be selected from intradermal, intravenous, intravitreal, subcutaneous, intraperitoneal, parenteral and intramuscular injection.

In the present invention, improvement of pharmacokinetics in human is preferred. When the plasma retention in human is difficult to determine, it may be predicted based on the plasma retention in mice (for example, normal mice, human antigen-expressing transgenic mice, human FcRn-expressing transgenic mice) or monkeys (for example, cynomolgus monkeys).

Method of promoting intracellular dissociation of an antigen from an antigen-binding molecule, wherein the antigen has been extracellularly bound to the antigen-binding molecule

The present invention provides a method of promoting intracellular dissociation of an antigen from an antigen-binding molecule, wherein the antigen has been extracellularly bound to the antigen-binding molecule, wherein the method comprises contacting the antigen-binding molecule with an Fcγ receptor-expressing cell *in vivo* or *ex vivo,* wherein the antigen-binding molecule has human FcRn-binding activity under an acidic pH range condition and comprises an antigen-binding domain and an Fcγ receptor-binding domain, in which an antigen-binding activity of the antigen-binding domain changes depending on the ion concentration condition, and the Fcγ receptor-binding domain has higher binding activity to the Fcγ receptor under a neutral pH range condition compared to a native Fcγ receptor-binding domain to which the sugar chain linked at position 297 (EU numbering) is a fucose-containing sugar chain.

The present invention provides a method of promoting intracellular dissociation of an antigen from an antigen-binding molecule, wherein the antigen has been extracellularly bound to the antigen-binding molecule, wherein the method comprises enhancing Fcγ receptor-binding activity under a neutral pH range condition of the Fcγ receptor-binding domain in an antigen-binding molecule compared to that of a native Fcγ receptor-binding domain to which the sugar chain linked at position 297 (EU numbering) is a fucose-containing sugar chain, wherein the antigen-binding molecule has human FcRn-binding activity under an acidic pH range condition and comprises an Fcγ receptor-binding domain and an antigen-binding domain whose antigen-binding activity changes depending on the ion concentration condition.

In the present invention, the place where an antigen dissociates from an antigen-binding molecule may be any as long as it is in a cell, but preferably it is in an early endosome. In the present invention, "intracellular dissociation of an antigen from an antigen-binding molecule, wherein the antigen has been extracellularly bound to the antigen-binding molecule" does not have to mean that all antigens taken up into cells after being bound to antigen-binding molecules are intracellularly dissociated from the antigen-binding molecules, and the intracellular dissociation of antigens from antigen-binding molecules only needs to be higher in ratio when compared to before lowering the antigen-binding activity of the antigen-binding molecules under an ion concentration condition such as an acidic pH range or low calcium ion concentration than under an ion concentration condition such as a neutral pH range or high calcium ion concentration and increasing the Fcγ receptor-binding activity in a neutral pH range. Furthermore, the method of promoting intracellular dissociation of an antigen from an antigen-binding molecule may be referred to as a method that enhances intracellular uptake of antigen-bound antigen-binding molecules and confers to the antigen-binding molecules the property of easing promotion of intracellular dissociation of antigens from the antigen-binding molecules.

Method of promoting extracellular release of an antigen-binding molecule in an antigen-unbound form

The present invention provides a method of promoting extracellular release of an antigen-binding molecule in an antigen-unbound form, wherein the method comprises contacting an antigen-binding molecule with an Fcγ receptor-expressing cell *in vivo* or *ex vivo,* wherein the antigen-binding molecule has human FcRn-binding activity under an acidic pH range condition and comprises an antigen-binding domain and an Fcγ receptor-binding domain, in which an antigen-binding activity of the antigen-binding domain changes depending on the ion concentration condition, and the Fcγ receptor-binding domain has higher binding activity to the Fcγ receptor under a neutral pH range condition compared to a native Fcγ receptor-binding domain to which the sugar chain linked at position 297 (EU numbering) is a fucose-containing sugar chain.

Furthermore, the present invention provides a method of promoting extracellular release of an antigen-binding molecule in an antigen-unbound form, wherein the antigen-binding molecule has been taken up into a cell in an antigen-bound form, wherein the method comprises enhancing Fcγ receptor-binding activity under a neutral pH range condition of the Fcγ receptor-binding domain in the antigen-binding molecule compared to that of a native Fcγ receptor-binding domain to which the sugar chain linked at position 297 (EU numbering) is a fucose-containing sugar chain, wherein the antigen-binding molecule has human-FcRn-binding activity under an acidic pH range condition and comprises an Fcγ receptor-binding domain and an antigen-binding domain whose antigen-binding activity changes depending on the ion concentration condition.

In the present invention, the phrase "extracellular release of an antigen-binding molecule in an antigen-unbound form, wherein the antigen-binding molecule has been taken up into a cell in an antigen-bound form" does not have to mean that all antigen-binding molecules that has been taken up into a cell in an antigen-bound form are extracellularly released in an antigen-unbound form, and the ratio of antigen-binding molecules extracellularly released in an antigen-unbound form only needs to be higher when compared to before lowering the antigen-binding activity of the antigen-binding molecules under an ion concentration condition such as an acidic pH range or low calcium ion concentration than under an ion concentration condition such as a neutral pH range or high calcium ion concentration and increasing the Fcγ receptor-binding activity in a neutral pH range. The extracellularly released antigen-binding molecules preferably maintain antigen-binding activity. Furthermore, the method of promoting extracellular release of an antigen-binding molecule in an antigen-unbound form, wherein the antigen-binding molecule has been taken up into a cell in an antigen-bound form may be referred to as a method that enhances uptake of antigen-bound antigen-binding molecules into cells and confers to the antigen-binding molecules the property of easing promotion of extracellular release of an antigen-binding molecule in an antigen-unbound form.

Method of decreasing total antigen concentration or free antigen concentration in plasma, or a method of altering an antigen-binding molecule which can decrease total antigen concentration or free antigen concentration in plasma

The present invention provides a method of decreasing total antigen concentration or free antigen concentration in plasma, wherein the method comprises contacting the antigen-binding molecule with an Fcγ receptor-expressing cell *in vivo* or *ex vivo,* wherein the antigen-binding molecule has human FcRn-binding activity under an acidic pH range condition and comprises an antigen-binding domain and an Fcγ receptor-binding domain, in which an antigen-binding activity of the antigen-binding domain changes depending on the ion concentration condition, and the Fcγ receptor-binding domain has higher binding activity to the Fcγ receptor under a neutral pH range condition compared to a native Fcγ receptor-binding domain to which the sugar chain linked at position 297 (EU numbering) is a fucose-containing sugar chain.

Furthermore, the present invention provides method of altering an antigen-binding molecule which can decrease total antigen concentration or free antigen concentration in plasma, wherein the method comprises enhancing Fcγ receptor-binding activity under a neutral pH range condition of the Fcγ receptor-binding domain in the antigen-binding molecule compared to that of a native Fcγ receptor-binding domain to which the sugar chain linked at position 297 (EU numbering) is a fucose-containing sugar chain, wherein the antigen-binding molecule has human FcRn-binding activity under an acidic pH range condition and comprises an Fcγ receptor-binding domain and an antigen-binding domain whose antigen-binding activity changes depending on the ion concentration condition.

The method of assessing decrease of total antigen concentration or free antigen concentration in plasma is described in the aforementioned section on the method of improving pharmacokinetics of antigen-binding-molecules.

*Ex vivo* method of eliminating the antigens from plasma

An example of a non-limiting embodiment of the use of an antigen-binding molecule for the method of eliminating the antigens from plasma, which is provided by the present invention, includes use of the antigen-binding molecule for a so-called *ex vivo* method of eliminating the antigens from plasma, which comprises contacting the antigen-binding molecule of the present invention with plasma isolated from subjects to allow forming immunocomplexes, and allowing the immunocomplexes to contact cells expressing Fcγ receptors and FcRn. The rate of elimination of plasma antigens can be promoted by replacing/combining a method of administering antigen-binding molecules *in vivo* with a so-called *ex vivo* method where plasma containing antigen-binding molecules and antigens that bind to the antigen-binding molecules is temporarily taken out from a living organism, and then contacted with cells expressing Fcγ receptors and FcRn, and the plasma containing extracellularly recycled (or also referred to as re-secreted or recirculated) antigen-binding molecules without bound antigen after a certain period of time are returned into the living organism.

Furthermore, an example of a non-limiting embodiment of the use of an antigen-binding molecule for the method of eliminating the antigens from plasma, which is provided by the present invention, includes use of the antigen-binding molecule for a so-called *ex vivo* method of eliminating the antigens from plasma, which comprises contacting immunocomplexes present in plasma isolated from subjects who have been administered with the antigen-binding molecules of the present invention with cells expressing FcRn and Fcγ receptors.

Whether or not the antigens are eliminated from plasma can be confirmed, for example, by evaluating whether or not the rate of elimination of plasma antigens mentioned above is promoted when, instead of the antigen-binding molecule of the present invention, an antigen-binding molecule comprising an antigen-binding domain in which the antigen-binding activity does not change depending on the ion concentration, an antigen-binding molecule comprising an FcRn-binding domain whose FcRn-binding activity under an acidic pH range condition has not been enhanced, or an antigen-binding molecule comprising an Fcγ receptor-binding domain that does not have selective binding activity to Fcγ receptors is used as a control for comparison.

Method of producing antigen-binding molecules

The present invention also provides a method of producing an antigen-binding molecule comprising an antigen-binding domain and an Fcγ receptor-binding domain, and human FcRn-binding activity under an acidic pH range condition, wherein an antigen-binding activity of the antigen-binding domain changes depending on the ion concentration condition

and the Fcγ receptor-binding domain has higher binding activity to the Fcγ receptor under a neutral pH range condition than a native Fcγ receptor-binding domain to which the sugar chain linked at position 297 (EU numbering) is a fucose-containing sugar chain.

Specifically, the present invention provides a method of producing an antigen-binding molecule, which comprises the steps of the following (a) to (f):

(a) determining an antigen-binding activity of an antigen-binding domain under a condition of high calcium ion concentration;
(b) determining an antigen-binding activity of the antigen-binding domain under a condition of low calcium ion concentration;
(c) selecting an antigen-binding domain for which the antigen-binding activity determined in (a) is higher than the antigen-binding activity determined in (b);
(d) linking a polynucleotide encoding the antigen-binding domain selected in (c) to a polynucleotide encoding an Fcγ receptor-binding domain having a human FcRn-binding activity in an acidic pH range, which has higher binding activity to the Fcγ receptor under a neutral pH range condition than a native Fcγ receptor-binding domain to which the sugar chain linked at position 297 (EU numbering) is a fucose-containing sugar chain;
(e) culturing cells introduced with a vector to which the polynucleotide obtained in (d) is operably linked; and
(f) collecting antigen-binding molecules from the cell culture of (e).

The present invention also provides a method of producing an antigen-binding molecule, which comprises the steps of the following (a) to (f):

(a) determining an antigen-binding activity of an antibody under a high calcium ion concentration condition;
(b) determining an antigen-binding activity of the antibody under a low calcium ion concentration condition;
(c) selecting an antibody for which the antigen-binding activity determined in (a) is higher than the antigen-binding activity determined in (b);
(d) linking a polynucleotide encoding an antigen-binding domain of the antibody selected in (c) to a polynucleotide encoding an Fcγ receptor-binding domain having human FcRn-binding activity in an acidic pH range and having higher binding activity to the Fcγ receptor under a neutral pH range condition than a native Fcγ receptor-binding domain to which the sugar chain linked at position 297 (EU numbering) is a fucose-containing sugar chain;
(e) culturing cells introduced with a vector to which the polynucleotide obtained in (d) is operably linked; and
(f) collecting antigen-binding molecules from the cell culture of (e).

In addition, the present invention provides a method of producing an antigen-binding molecule, which comprises the steps of the following (a) to (f):

(a) determining an antigen-binding activity of an antigen-binding domain under a neutral pH range condition;
(b) determining an antigen-binding activity of the antigen-binding domain under an acidic pH range condition;
(c) selecting the antigen-binding domain for which the antigen-binding activity determined in (a) is higher than the antigen-binding activity determined in (b);
(d) linking a polynucleotide encoding the antigen-binding domain selected in (c) to a polynucleotide encoding an Fcγ receptor-binding domain having a human FcRn-binding activity under an acidic pH range condition, which has higher binding activity to the Fcγ receptor under a neutral pH range condition than a native Fcγ receptor-binding domain to which the sugar chain linked at position 297 (EU numbering) is a fucose-containing sugar chain;
(e) culturing cells introduced with a vector to which the polynucleotide obtained in (d) is operably linked; and
(f) collecting antigen-binding molecules from the cell culture of (e).

In addition, the present invention provides a method of producing an antigen-binding molecule, which comprises the steps of the following (a) to (f):

(a) determining an antigen-binding activity of an antibody under a neutral pH range condition;
(b) determining an antigen-binding activity of the antibody under an acidic pH range condition;
(c) selecting the antibody for which the antigen-binding activity determined in (a) is higher than the antigen-binding activity determined in (b);
(d) linking a polynucleotide encoding the antigen-binding domain of the antibody selected in (c) to a polynucleotide encoding an Fcγ receptor-binding domain having human-FcRn-binding activity in an acidic pH range and having higher binding activity to the Fcγ receptor under a neutral pH range condition than a native Fcγ receptor-binding domain to which the sugar chain linked at position 297 (EU numbering) is a fucose-containing sugar chain;

(e) culturing cells introduced with a vector to which the polynucleotide obtained in (d) is operably linked; and
(f) collecting antigen-binding molecules from the cell culture of (e).

The terms "cell", "cell line", and "cell culture" are used synonymously herein, and such designations may include all progeny of a cell or cell line. Thus, for example, the terms "transformants" and "transformed cells" include the primary subject cell and cultures derived therefrom without regard for the number of transfers. It is also understood that all progeny may not be precisely identical in DNA content due to deliberate or inadvertent mutations. Mutant progeny that have substantially the same function or biological activity as screened for in the originally transformed cell may also be included. Where distinct designations are intended, such intention will be clear from the context of the description.
When referring to the expression of a coding sequence, the term "control sequences" refers to DNA nucleotide sequences that are necessary for the expression of an operably linked coding sequence in a particular host organism. The control sequences that are suitable for prokaryotes include, for example, a promoter, optionally an operator sequence, a ribosome binding site, and possibly, other sequences as yet poorly understood. Eukaryotic cells are known to utilize promoters, polyadenylation signals, and enhancers for the expression of a coding sequence.
For a nucleic acid, the term "operably linked" means that the nucleic acid is placed into a functional relationship with another nucleic acid sequence. For example, DNA for a presequence or secretory leader is operably linked to DNA for a polypeptide if it is expressed as a precursor protein that participates in the secretion of the polypeptide. A promoter or enhancer is operably linked to a coding sequence if it affects the transcription of the sequence. A ribosome binding site is operably linked to a coding sequence if it is positioned so as to facilitate translation. Generally, "operably linked" means that the DNA sequences being linked are contiguous and, in the case of a secretory leader, contiguous and being in the reading frame. However, enhancers do not have to be contiguous. Linking is accomplished by ligation at suitable restriction sites. If such sites do not exist, the synthetic oligonucleotide adaptors or linkers are used in accordance with conventional practice. Furthermore, linked nucleic acids may be produced by the above-mentioned overlap extension PCR technique.
"Ligation" refers to the process of forming phosphodiester bonds between two nucleic acid fragments. For ligation of the two fragments, the ends of the fragments must be compatible with each other. In some cases, the ends will be directly compatible after endonuclease digestion. However, it may be necessary first to convert the cohesive ends commonly produced after endonuclease digestion to blunt ends to make them compatible for ligation. For blunting the ends, the DNA is treated in a suitable buffer for at least 15 minutes at 15°C with about 10 units of the Klenow fragment of DNA polymerase I or T4 DNA polymerase in the presence of the four deoxyribonucleotide triphosphates. The DNA is then purified by phenol-chloroform extraction and ethanol precipitation, or by silica purification. The DNA fragments that are to be ligated together are put in solution in equimolar amounts. The solution will contain ATP, ligase buffer, and a ligase such as T4 DNA ligase at about 10 units per 0.5 μg of DNA. If the DNA is to be ligated to a vector, the vector is first linearized by digestion with appropriate restriction endonucleases. The linearized fragment is then treated with bacterial alkaline phosphatase or calf intestinal phosphatase to prevent self-ligation of the fragment during the ligation step.
In the production methods of the present invention, antigen-binding domains or antibodies having higher antigen-binding activity under a high-calcium-ion-concentration condition than under a low-calcium-ion-concentration condition, which have been selected by the method described in the above-mentioned section on "ion concentration conditions" are isolated. Furthermore, antigen-binding domains or antibodies having higher antigen-binding activity in a neutral pH range condition than in an acidic pH range condition, which have been selected by the method described in the above-mentioned section on "ion concentration conditions" are isolated. For example, when antigen-binding domains isolated in this manner are selected from a library, as described later in the Examples, polynucleotides encoding the antigen-binding domains are isolated by conventional gene amplification from viruses such as phages. Alternatively, when antigen-binding domains or antibodies isolated in this manner are those selected from cultures of cells such as hybridomas, as indicated in the aforementioned section on antibodies, antibody genes and such are isolated by conventional gene amplification from those cells.
Next, a polynucleotide encoding an antigen-binding domain isolated as described above is linked in frame to a polynucleotide encoding an Fcγ-receptor-binding domain having human-FcRn-binding activity in an acidic pH range, and has higher binding activity to the Fcγ receptor in a neutral pH range condition than a native Fcγ-receptor-binding domain in which the sugar chain bound at position 297 (EU numbering) is a fucose-containing sugar chain. Suitable example of the Fcγ-receptor-binding domain includes an antibody Fc region as described in the above-mentioned section on Fcγ-receptor-binding domain. Furthermore, examples of the Fcγ receptor include FcγRIa, FcγRIIa(R), FcγRIIa(H), FcγRIIb, FcγRIIIa(V), or FcγRIIIa(F).
Suitable examples of the antibody Fc region include Fc regions having at least one or more amino acids, selected from the group consisting of amino acids at positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, and 440 in the Fc region site according to EU numbering, that are different from the amino acids at corresponding sites in the native Fc region. A suitable example of the native Fc region is an Fc region of any one of IgG1, IgG2, IgG3, and IgG4.

In a non-limiting embodiment, a suitable example of the antibody Fc region is an Fc region comprising at least one or more amino acids selected from the group consisting of:

either Lys or Tyr at amino acid position 221;
any one of Phe, Trp, Glu, and Tyr at amino acid position 222;
any one of Phe, Trp, Glu, and Lys at amino acid position 223;
any one of Phe, Trp, Glu, and Tyr at amino acid position 224;
any one of Glu, Lys, and Trp at amino acid position 225;
any one of Glu, Gly, Lys, and Tyr at amino acid position 227;
any one of Glu, Gly, Lys, and Tyr at amino acid position 228;
any one of Ala, Glu, Gly, and Tyr at amino acid position 230;
any one of Glu, Gly, Lys, Pro, and Tyr at amino acid position 231;
any one of Glu, Gly, Lys, and Tyr at amino acid position 232;
any one of Ala, Asp, Phe, Gly, His, Ile, Lys, Leu, Met, Asn, Gln, Arg, Ser, Thr, Val, Trp, and Tyr at amino acid position 233;
any one of Ala, Asp, Glu, Phe, Gly, His, Ile, Lys, Met, Asn, Pro, Gln, Arg, Ser, Thr, Val, Trp, and Tyr at amino acid position 234;
any one of Ala, Asp, Glu, Phe, Gly, His, Ile, Lys, Met, Asn, Pro, Gln, Arg, Ser, Thr, Val, Trp, and Tyr at amino acid position 235;
any one of Ala, Asp, Glu, Phe, His, Ile, Lys, Leu, Met, Asn, Pro, Gln, Arg, Ser, Thr, Val, Trp, and Tyr at amino acid position 236;
any one of Asp, Glu, Phe, His, Ile, Lys, Leu, Met, Asn, Pro, Gln, Arg, Ser, Thr, Val, Trp, and Tyr at amino acid position 237;
any one of Asp, Glu, Phe, Gly, His, Ile, Lys, Leu, Met, Asn, Gln, Arg, Ser, Thr, Val, Trp, and Tyr at amino acid position 238;
any one of Asp, Glu, Phe, Gly, His, Ile, Lys, Leu, Met, Asn, Pro, Gln, Arg, Thr, Val, Trp, and Tyr at amino acid position 239;
any one of Ala, Ile, Met, and Thr at amino acid position 240;
any one of Asp, Glu, Leu, Arg, Trp, and Tyr at amino acid position 241;
any one of Leu, Glu, Leu, Gln, Arg, Trp, and Tyr at amino acid position 243;
His at amino acid position 244;
Ala at amino acid position 245;
any one of Asp, Glu, His, and Tyr at amino acid position 246;
any one of Ala, Phe, Gly, His, Ile, Leu, Met, Thr, Val, and Tyr at amino acid position 247;
any one of Glu, His, Gln, and Tyr at amino acid position 249;
either Glu or Gln at amino acid position 250;
Phe at amino acid position 251;
any one of Phe, Met, and Tyr at amino acid position 254;
any one of Glu, Leu, and Tyr at amino acid position 255;
any one of Ala, Met, and Pro at amino acid position 256;
any one of Asp, Glu, His, Ser, and Tyr at amino acid position 258;
any one of Asp, Glu, His, and Tyr at amino acid position 260;
any one of Ala, Glu, Phe, Ile, and Thr at amino acid position 262;
any one of Ala, Ile, Met, and Thr at amino acid position 263;
any one of Asp, Glu, Phe, Gly, His, Ile, Lys, Leu, Met, Asn, Pro, Gln, Arg, Ser, Thr, Trp, and Tyr at amino acid position 264;
any one of Ala, Leu, Phe, Gly, His, Ile, Lys, Leu, Met, Asn, Pro, Gln, Arg, Ser, Thr, Val, Trp, and Tyr at amino acid position 265;
any one of Ala, Ile, Met, and Thr at amino acid position 266;
any one of Asp, Glu, Phe, His, Ile, Lys, Leu, Met, Asn, Pro, Gln, Arg, Thr, Val, Trp, and Tyr at amino acid position 267;
any one of Asp, Glu, Phe, Gly, Ile, Lys, Leu, Met, Pro, Gln, Arg, Thr, Val, and Trp at amino acid position 268;
any one of Phe, Gly, His, Ile, Lys, Leu, Met, Asn, Pro, Arg, Ser, Thr, Val, Trp, and Tyr at amino acid position 269;
any one of Glu, Phe, Gly, His, Ile, Leu, Met, Pro, Gln, Arg, Ser, Thr, Trp, and Tyr at amino acid position 270;

any one of Ala, Asp, Glu, Phe, Gly, His, Ile, Lys, Leu, Met, Asn, Gln, Arg, Ser, Thr, Val, Trp, and Tyr at amino acid position 271;
any one of Asp, Phe, Gly, His, Ile, Lys, Leu, Met, Pro, Arg, Ser, Thr, Val, Trp, and Tyr at amino acid position 272;
either Phe or Ile at amino acid position 273;
any one of Asp, Glu, Phe, Gly, His, Ile, Leu, Met, Asn, Pro, Arg, Ser, Thr, Val, Trp, and Tyr at amino acid position 274;
either Leu or Trp at amino acid position 275;
any one of Asp, Glu, Phe, Gly, His, Ile, Leu, Met, Pro, Arg, Ser, Thr, Val, Trp, and Tyr at amino acid position 276;
any one of Asp, Glu, Gly, His, Ile, Lys, Leu, Met, Asn, Pro, Gln, Arg, Ser, Thr, Val, and Trp at amino acid position 278;
Ala at amino acid position 279;
any one of Ala, Gly, His, Lys, Leu, Pro, Gln, Trp, and Tyr at amino acid position 280;
any one of Asp, Lys, Pro, and Tyr at amino acid position 281;
any one of Glu, Gly, Lys, Pro, and Tyr at amino acid position 282;
any one of Ala, Gly, His, Ile, Lys, Leu, Met, Pro, Arg, and Tyr at amino acid position 283;
any one of Asp, Glu, Leu, Asn, Thr, and Tyr at amino acid position 284;
any one of Asp, Glu, Lys, Gln, Trp, and Tyr at amino acid position 285;
any one of Glu, Gly, Pro, and Tyr at amino acid position 286;
any one of Asn, Asp, Glu, and Tyr at amino acid position 288;
any one of Asp, Gly, His, Leu, Asn, Ser, Thr, Trp, and Tyr at amino acid position 290;
any one of Asp, Glu, Gly, His, Ile, Gln, and Thr at amino acid position 291;
any one of Ala, Asp, Glu, Pro, Thr, and Tyr at amino acid position 292;
any one of Phe, Gly, His, Ile, Leu, Met, Asn, Pro, Arg, Ser, Thr, Val, Trp, and Tyr at amino acid position 293;
any one of Phe, Gly, His, Ile, Lys, Leu, Met, Asn, Pro, Arg, Ser, Thr, Val, Trp, and Tyr at amino acid position 294;
any one of Asp, Glu, Phe, Gly, His, Ile, Lys, Met, Asn, Pro, Arg, Ser, Thr, Val, Trp, and Tyr at amino acid position 295;
any one of Ala, Asp, Glu, Gly, His, Ile, Lys, Leu, Met, Asn, Gln, Arg, Ser, Thr, and Val at amino acid position 296;
any one of Asp, Glu, Phe, Gly, His, Ile, Lys, Leu, Met, Pro, Gln, Arg, Ser, Thr, Val, Trp, and Tyr at amino acid position 297;
any one of Ala, Asp, Glu, Phe, His, Ile, Lys, Met, Asn, Gln, Arg, Thr, Val, Trp, and Tyr at amino acid position 298;
any one of Ala, Asp, Glu, Phe, Gly, His, Ile, Lys, Leu, Met, Asn, Pro, Gln, Arg, Ser, Val, Trp, and Tyr at amino acid position 299;
any one of Ala, Asp, Glu, Gly, His, Ile, Lys, Leu, Met, Asn, Pro, Gln, Arg, Ser, Thr, Val, and Trp at amino acid position 300;
any one of Asp, Glu, His, and Tyr at amino acid position 301;
Ile at amino acid position 302;
any one of Asp, Gly, and Tyr at amino acid position 303;
any one of Asp, His, Leu, Asn, and Thr at amino acid position 304;
any one of Glu, Ile, Thr, and Tyr at amino acid position 305;
any one of Ala, Asp, Asn, Thr, Val, and Tyr at amino acid position 311;
Phe at amino acid position 313;
Leu at amino acid position 315;
either Glu or Gln at amino acid position 317;
any one of His, Leu, Asn, Pro, Gln, Arg, Thr, Val, and Tyr at amino acid position 318;
any one of Asp, Phe, Gly, His, Ile, Leu, Asn, Pro, Ser, Thr, Val, Trp, and Tyr at amino acid position 320;
any one of Ala, Asp, Phe, Gly, His, Ile, Pro, Ser, Thr, Val, Trp, and Tyr at amino acid position 322;
Ile at amino acid position 323;
any one of Asp, Phe, Gly, His, Ile, Leu, Met, Pro, Arg, Thr, Val, Trp, and Tyr at amino acid position 324;
any one of Ala, Asp, Glu, Phe, Gly, His, Ile, Lys, Leu, Met, Pro, Gln, Arg, Ser, Thr, Val, Trp, and Tyr at amino acid position 325;
any one of Ala, Asp, Glu, Gly, Ile, Leu, Met, Asn, Pro, Gln, Ser, Thr, Val, Trp, and Tyr at amino acid position 326;
any one of Ala, Asp, Glu, Phe, Gly, His, Ile, Lys, Leu, Met, Asn, Pro, Arg, Thr, Val, Trp, and Tyr at amino acid position 327;
any one of Ala, Asp, Glu, Phe, Gly, His, Ile, Lys, Met, Asn, Pro, Gln, Arg, Ser, Thr, Val, Trp, and Tyr at amino acid position 328;
any one of Asp, Glu, Phe, Gly, His, Ile, Lys, Leu, Met, Asn, Gln, Arg, Ser, Thr, Val, Trp, and Tyr at amino acid position 329;
any one of Cys, Glu, Phe, Gly, His, Ile, Lys, Leu, Met, Asn, Pro, Arg, Ser, Thr, Val, Trp, and Tyr at amino acid position 330;
any one of Asp, Phe, His, Ile, Leu, Met, Gln, Arg, Thr, Val, Trp, and Tyr at amino acid position 331;
any one of Ala, Asp, Glu, Phe, Gly, His, Lys, Leu, Met, Asn, Pro, Gln, Arg, Ser, Thr, Val, Trp, and Tyr at amino acid position 332;
any one of Ala, Asp, Glu, Phe, Gly, His, Ile, Leu, Met, Pro, Ser, Thr, Val, and Tyr at amino acid position 333;
any one of Ala, Glu, Phe, Ile, Leu, Pro, and Thr at amino acid position 334;
any one of Asp, Phe, Gly, His, Ile, Leu, Met, Asn, Pro, Arg, Ser, Val, Trp, and Tyr at amino acid position 335;
any one of Glu, Lys, and Tyr at amino acid position 336;
any one of Glu, His, and Asn at amino acid position 337;
any one of Asp, Phe, Gly, Ile, Lys, Met, Asn, Gln, Arg, Ser, and Thr at amino acid position 339; either Ala or Val at amino acid position 376;
either Gly or Lys at amino acid position 377;
Asp at amino acid position 378;
Asn at amino acid position 379;
any one of Ala, Asn, and Ser at amino acid position 380;
either Ala or Ile at amino acid position 382;
Glu at amino acid position 385;
Thr at amino acid position 392;
Leu at amino acid position 396;
Lys at amino acid position 421;
Asn at amino acid position 427;
either Phe or Leu at amino acid position 428;
Met at amino acid position 429;
Trp at amino acid position 434;
Ile at amino acid position 436; and
any one of Gly, His, Ile, Leu, and Tyr at amino acid position 440;
in the Fc region site according to EU numbering.

Furthermore, for Fc regions of the present invention, Fc regions which have binding activity or enhanced binding activity to FcRn in an acidic pH range condition may be suitably used. Examples of such Fc regions include Fc regions of IgG-type immunoglobulins such as Fc regions of human IgG (IgG1, IgG2, IgG3, IgG4, and variants thereof). For variants having alterations to other amino acids, Fc regions with amino acid alterations at any position may be used as long as there is FcRn-binding activity in an acidic pH range or the binding activity to human FcRn in an acidic pH range condition can be increased, and when an antigen-binding molecule includes an Fc region of a human IgG1 as the Fc region, it preferably includes an alteration that enhances binding to FcRn in an acidic pH range condition compared to the binding activity of the starting-material Fc region of human IgG1. Suitable examples of amino acids that can be altered include the amino acids at positions 238, 252, 253, 254, 255, 256, 265, 272, 286, 288, 303, 305, 307, 309, 311, 312, 317, 340, 356, 360, 362, 376, 378, 380, 382, 386, 388, 400, 413, 415, 424, 433, 434, 435, 436, 439, and/or 447 (EU numbering) as described in WO2000/042072. Similarly, suitable examples of amino acids that can be altered also include the amino acids at positions 251, 252, 254, 255, 256, 308, 309, 311, 312, 385, 386, 387, 389, 428, 433, 434, and/or 436 (EU numbering) as described in WO2002/060919. Furthermore, examples of amino acids that can be altered also include the amino acids at positions 250, 314, and 428 (EU numbering) as described in WO2004/092219. Other suitable examples of amino acids that can be altered also include the amino acids at positions 251, 252, 307, 308, 378, 428, 430, 434, and/or 436 (EU numbering) as described in WO2010/045193. Fc regions produced by enhancing the FcRn-binding in an acidic pH range of an IgG immunoglobulin Fc region by the amino acid alterations may be used in the production methods of the present invention.

Furthermore, as described later, for the Fc region included in an antigen-binding molecule of the present invention, an Fc region having FcRn-binding activity in a neutral pH range may also be suitably used. Such an Fc region may be obtained by any method according to the aforementioned method for obtaining Fc regions having FcRn-binding activity in an acidic pH range. Specifically, an Fc region containing an FcRn-binding domain having binding activity or having enhanced binding activity to FcRn in a neutral pH range due to alteration of amino acids of the Fc region of a human IgG immunoglobulin used as the starting-material Fc region may be obtained. Favorable Fc regions of IgG immunoglobulins for the alteration include Fc regions of human IgG (IgG1, IgG2, IgG3, IgG4, and variants thereof). For variants having alterations to other amino acids, Fc regions with amino acid alterations at any position may be used as long as there is FcRn-binding activity in a neutral pH range or the binding activity to human FcRn in a neutral pH range can be increased. When an antigen-binding molecule includes an Fc region of a human IgG1 as the Fc region, it preferably includes an alteration that enhances binding to FcRn in a neutral pH range compared to the binding activity of the starting-material Fc region of human IgG1. Suitable examples of such altered Fc regions include human Fc regions having at least one or more amino acids, selected from the group consisting of amino acids at positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434, and 436 in the starting-material Fc region site according to EU numbering, that are different from the corresponding amino acids in the native Fc region.

Suitable examples of such altered Fc regions include Fc regions containing at least one amino acid selected from the group consisting of:

Met at amino acid position 237;
Ala at amino acid position 238;
Lys at amino acid position 239;
Ile at amino acid position 248;
any one of Ala, Phe, Ile, Met, Gln, Ser, Val, Trp, and Tyr at amino acid position 250;
any one of Phe, Trp, and Tyr at amino acid position 252;
Thr at amino acid position 254;
Glu at amino acid position 255;
any one of Asp, Glu, and Gln at amino acid position 256;
any one of Ala, Gly, Ile, Leu, Met, Asn, Ser, Thr, and Val at amino acid position 257;
His at amino acid position 258;
Ala at amino acid position 265;
Phe at amino acid position 270;
either Ala or Glu at amino acid position 286;
His at amino acid position 289;
Ala at amino acid position 297;
Gly at amino acid position 298;
Ala at amino acid position 303;
Ala at amino acid position 305;
any one of Ala, Asp, Phe, Gly, His, Ile, Lys, Leu, Met, Asn, Pro, Gln, Arg, Ser, Val, Trp, and Tyr at amino acid position 307;
any one of Ala, Phe, Ile, Leu, Met, Pro, Gln, and Thr at amino acid position 308;
any one of Ala, Asp, Glu, Pro, and Arg at amino acid position 309;
any one of Ala, His, and Ile at amino acid position 311;
either Ala or His at amino acid position 312;
either Lys or Arg at amino acid position 314;
either Ala or His at amino acid position 315;
Ala at amino acid position 317;
Gly at amino acid position 325;
Val at amino acid position 332;
Leu at amino acid position 334;
His at amino acid position 360;
Ala at amino acid position 376;
Ala at amino acid position 380;
Ala at amino acid position 382;
Ala at amino acid position 384;
either Asp or His at amino acid position 385;
Pro at amino acid position 386;
Glu at amino acid position 387;
either Ala or Ser at amino acid position 389;
Ala at amino acid position 424;
any one of Ala, Asp, Phe, Gly, His, Ile, Lys, Leu, Asn, Pro, Gln, Ser, Thr, Val, Trp, and Tyr at amino acid position 428;
Lys at amino acid position 433;
any one of Ala, Phe, His, Ser, Trp, and Tyr at amino acid position 434; and
His at amino acid position 436;
in the Fc region according to EU numbering.

For example, by using the amino acid alterations individually, or by using more than one of them in combination, FcRn-binding of an IgG Fc region in an acidic and/or neutral pH range can be enhanced, and the amino acid alterations that are introduced are not particularly limited, and as long as the retentivity in plasma is improved, any amino acid alteration may be introduced.

An antigen-binding molecule of the present invention is isolated from the culture of cells transformed by a desired expression vector carrying an operably linked polynucleotide obtained by linking, as described above, a polynucleotide encoding the antigen-binding domain to a polynucleotide encoding an Fcγ receptor-binding domain having human-FcRn-binding activity in an acidic pH range and having higher binding activity to the Fcγ receptor in a neutral pH range condition than a native Fcγ receptor-binding domain in which the sugar chain bound at position 297 (EU numbering) is a fucose-containing sugar chain. Antigen-binding molecules of the present invention are produced using a method according to the method for producing antibodies described in the above-mentioned section on antibodies.

All prior art documents cited in the specification are incorporated herein by reference.

Herein below, the present invention will be specifically described with reference to the Examples, but it is not to be construed as being limited thereto.

[Examples]

[Example 1] Preparation of antigen-binding molecules whose mouse FcyR-binding activity under a neutral pH range condition is higher than the binding activity of native human IgG Fc region

(1-1) pH-dependent human IL-6 receptor-binding antibodies

H54/L28-IgG1 which comprises H54-IgG1 (SEQ ID NO: 36) and L28-CK (SEQ ID NO: 37) described in WO2009/125825 is a humanized anti-IL-6 receptor antibody. Meanwhile, Fv4-IgG1 which comprises VH3-IgG1 (SEQ ID NO: 38) and VL3-CK (SEQ ID NO: 39) is a humanized anti-IL-6 receptor antibody resulting from conferring, to H54/L28-IgG1, the property of binding to soluble human IL-6 receptor in a pH-dependent manner (which binds at pH 7.4 and dissociates at pH 5.8). The *in vivo* mouse test described in WO2009/125825 demonstrated that, in the group administered with a mixture of Fv4-IgG1 and soluble human IL-6 receptor as the antigen, the elimination of soluble human IL-6 receptor from plasma was significantly accelerated as compared to the group administered with a mixture of H54/L28-IgG1 and soluble human IL-6 receptor as the antigen.

The soluble human IL-6 receptor bound to H54/L28-IgG1, which is an antibody that binds to a soluble human IL-6 receptor, is, together with the antibody, recycled to plasma by FcRn. Meanwhile, Fv4-IgG1, which is an antibody that binds to a soluble human IL-6 receptor in a pH dependent manner, dissociates soluble human IL-6 receptor under the acidic condition in the endosome. The dissociated soluble human IL-6 receptor is degraded in the lysosomes, thus this enables considerable acceleration of the elimination of soluble human IL-6 receptor. Furthermore, after binding to FcRn in the endosome, Fv4-IgG1, which is an antibody that binds to a soluble human IL-6 receptor in a pH dependent manner, is recycled to the plasma. Since the recycled antibody can bind to soluble human IL-6 receptor again, the antibody repeatedly binds to the antigen (soluble human IL-6 receptor) and is recycled by FcRn to the plasma. It is thought that, as a result, a single antibody molecule can bind repeatedly several times to soluble human IL-6 receptor (WO 2009/125825).

(1-2) Preparation of an anti-human IL-6 receptor antibody with enhanced mouse FcyR binding and anti-human IL-6 receptor antibody without mouse FcγR binding

VH3-IgG1-F1022 (SEQ ID NO: 40), an antigen-binding molecule with enhanced mouse FcyR binding, was prepared by substituting Asp for Lys at position 326 (EU numbering) and Tyr for Leu at position 328 (EU numbering) in VH3-IgG1. Fv4-IgG1-F1022 containing VH3-IgG1-F1022 as the heavy chain and VL3-CK as the light chain was produced using the method described in Reference Example 2.

Meanwhile, VH3-IgG1-F760 (SEQ ID NO: 41), an antigen-binding molecule without mouse FcyR binding, was prepared by substituting Arg for Leu at position 235 and Lys for Ser at position 239 (EU numbering) in VH3-IgG1. Fv4-IgG1-F760 containing VH3-IgG1-F760 as the heavy chain and VL3-CK as the light chain was produced using the method described in Reference Example 2.

(1-3) Assessment of mouse FcyR-binding activity

VH3/L(WT)-IgG1, VH3/L(WT)-IgG1-F1022, and VH3/L(WT)-IgG1-F760, which contain VH3-IgG1, VH3-IgG1-F1022, and VH3-IgG1-F760 as the heavy chain, and L(WT)-CK (SEQ ID NO: 42) as the light chain, were produced using the method described in Reference Example 2. These antibodies were kinetically analyzed for their mouse FcyR binding as described below.

(1-4) Kinetic analysis of mouse FcγR binding

The binding of antibodies to mouse FcγRI, FcγRIIb, FcγRIII, and FcγRIV (hereinafter, referred to as mouse FcγRs) (prepared by Reference Example 26) was kinetically analyzed using Biacore T100 and T200 (GE Healthcare). An appropriate amount of protein L (ACTIGEN) was immobilized onto a Sensor chip CM4 (GE Healthcare) by an amino coupling method, and antibodies of interest were captured thereto. Then, diluted solutions of mouse FcyRs and a running buffer as a blank were injected, and the mouse FcγRs were allowed to interact with antibodies captured onto the sensor chip. The running buffer used was 20 mmol/l ACES, 150 mmol/l NaCl, 0.05% (w/v) Tween20, pH 7.4. This buffer was also used to dilute the mouse FcyRs. The sensor chip was regenerated using 10 mmol/l glycine-HCl, pH 1.5. All measurements were carried out at 25°C. The binding rate constant ka (1/Ms) and dissociation rate constant kd (1/s), which are kinetic parameters, were calculated from the sensorgrams obtained by the measurement. $K_D$ (M) of each antibody for human FcγR was calculated based on the values. Each parameter was calculated using Biacore T100 or T200 Evaluation Software (GE Healthcare).

The result shown in Table 7 was obtained by the measurement. VH3/L (WT)-IgG1-F1022 was demonstrated to have increased binding activity to mFcyRI, mFcyRIIb, and mFcγRIII as compared to VH3/L (WT)-IgG1. Regarding VH3/L (WT)-IgG1-F760, the binding to the various mouse FcyRs was undetectable, demonstrating that VH3/L (WT)-IgG1-F760 lacks the binding activity to the various mouse FcyRs.

[Table 7]

| VARIANT NAME | KD (M) | | | |
|---|---|---|---|---|
| | mFc γR I | mFc γR IIb | mFc γR III | mFc γR IV |
| IgG1 | 6.0E-08 | 5.0E-07 | 2.2E-07 | 2.4E-08 |
| F1022 | 9.1E-09 | 8.5E-09 | 8.1E-09 | 3.8E-08 |
| F760 | NOT DETECTED | NOT DETECTED | NOT DETECTED | NOT DETECTED |

(1-5) Preparation of antibodies with low fucose content

Known methods for increasing the FcyR-binding activity of antibodies include methods for making sugar chains linked to an antibody be sugar chains with low fucose content (J. Biol. Chem. (2003) 278, 3466-3473) in addition to methods for introducing an amino acid alteration into the Fc region of an antibody. An Fv4-IgG1 with low fucose content (hereinafter, abbreviated as Fv4-IgG1-Fuc) was produced by expressing Fv4-IgG1 using fucose transporter gene-deficient CHO cells (WO2006067913) as host cells according to the method described in Reference Example 4. It has been reported that, of the mFcγRs (mouse Fcγ receptors), antibodies with low fucose content have selectively increased FcγRIV-binding activity (Science, 2005, 310 (5753) 1510-1512).

[Example 2] Effect of eliminating antigens from plasma by antigen-binding molecules whose FcyR-binding activity is higher than the binding activity of native human IgG Fc region

(2-1) Effect of H54/L28-IgG1 and Fv4-IgG1 to eliminate antigens from plasma

H54/L28-IgG1, which is an anti-human IL-6 receptor antibody, and Fv4-IgG1 having the property of binding to human IL-6 receptor in a pH-dependent manner were produced by the method described in Reference Example 1. *In vivo* infusion tests were carried out using the produced H54/L28-IgG1 and Fv4-IgG1 by the method described below.

(2-1-1) *In vivo* infusion tests using human FcRn transgenic mice

An animal model in which the soluble human IL-6 receptor concentration is maintained constant in plasma was created by implanting an infusion pump (MINI-OSMOTIC PUMP MODEL2004, alzet) containing soluble human IL-6 receptor under the skin on the back of human FcRn transgenic mice (B6.mFcRn-/-.hFcRn Tg line 32 +/+ mouse, Jackson Laboratories, Methods Mol Biol. (2010) 602, 93-104). The *in vivo* dynamics after administration of an anti-human IL-6 receptor antibody was assessed in the animal model. To suppress the production of neutralizing antibodies against soluble human IL-6 receptor, an anti-mouse CD4 monoclonal antibody (prepared by a known method) was administered once at 20 mg/kg into the caudal vein. Then, an infusion pump containing 92.8 μg/ml soluble human IL-6 receptor was subcutaneously implanted on the back of the mice. Three days after implantation of the infusion pump, an anti-human IL-6 receptor antibody was administered once at 1 mg/kg into the caudal vein. The blood was collected from the mice 15 minutes, seven hours, one day, two days, four days, and seven days after administration of the anti-human IL-6 receptor antibody. Immediately, the collected blood was centrifuged at 15,000 rpm and 4°C for 15 minutes to prepare plasma. The isolated plasma was stored in a freezer set at -20°C or below until use.

(2-1-2) Determination of the human IL-6 receptor (hsIL-6R) concentration in plasma by an electrochemilumine scent method

The human IL-6 receptor concentrations in mouse plasma were determined by an electrochemiluminescent method. hsIL-6R standard curve samples prepared at 2000, 1000, 500, 250, 125, 62.5, and 31.25 pg/ml and assay samples of mouse plasma diluted 50 times or more were mixed with Monoclonal Anti-human IL-6R Antibody (R&D), Biotinylated Anti-human IL-6 R Antibody (R&D), Tocilizumab, which had been ruthenated with SULFO-TAG NHS Ester (Meso Scale Discovery). The mixtures were incubated at 37°C overnight. Tocilizumab was prepared at a final concentration of 333 μg/ml. Then, the reaction mixtures were aliquoted in an MA400 PR Streptavidin Plate (Meso Scale Discovery). The solution reacted at room temperature for one hour was washed out, and then Read Buffer T (x4) (Meso Scale Discovery) was aliquoted. Immediately thereafter, the measurement was carried out using SECTOR PR 400 Reader (Meso Scale Discovery). The concentration of human IL-6 receptor was determined based on the response of the standard curve using analysis software SOFTmax PRO (Molecular Devices).

A time course of the monitored human IL-6 receptor concentration is shown in Fig. 2. As compared to H54/L28-IgG1, Fv4-IgG1 that binds to human IL-6 receptor in a pH-dependent manner could reduce the human IL-6 receptor concentration, but could not reduce it below the baseline without antibody administration. That is, the administered antibody which binds to an antigen in a pH-dependent manner could not reduce the antigen concentration in plasma below the level prior to antibody administration.

(2-2) The effect of eliminating an antigen from plasma by an antibody with increased or reduced FcγR-binding activity

Whether the time course of human IL-6 receptor concentration is influenced by increasing or reducing the FcyR-binding activity of Fv4-IgG1, which is a pH-dependent human IL-6 receptor-binding antibody, was assessed by the method described below. Using Fv4-IgG1, Fv4-IgG1-F760, Fv4-IgG1-F1022, and Fv4-IgG1-Fuc prepared as described in Example 1, *in vivo* infusion tests were performed by the method described below.

(2-2-1) *In vivo* infusion tests using human FcRn transgenic mice

A animal model in which the soluble human IL-6 receptor concentration is maintained constant in plasma was created by implanting an infusion pump (MINI-OSMOTIC PUMP MODEL2004, alzet) containing soluble human IL-6 receptor under the skin on the back of human FcRn transgenic mice (B6.mFcRn-/-.hFcRn Tg line 32 +/+ mouse, Jackson Laboratories, Methods Mol Biol. (2010) 602, 93-104). In the animal model, an anti-human IL-6 receptor antibody was administered simultaneously with Sanglopor (CSL Behring) which is a human immunoglobulin preparation, to assess the *in vivo* dynamics of the soluble human IL-6 receptor after antibody administration. To suppress the production of neutralizing antibodies against soluble human IL-6 receptor, an anti-mouse CD4 monoclonal antibody (prepared by a known method) was administered once at 20 mg/kg into the caudal vein. Then, an infusion pump containing 92.8 μg/ml soluble human IL-6 receptor was subcutaneously implanted on the back of the mice. Three days after implantation of the infusion pump, an anti-human IL-6 receptor antibody and Sanglopor were administered once at 1 mg/kg and 1000 mg/kg, respectively, into the caudal vein. The blood was collected from the mice 15 minutes, seven hours, one day, two days, four days, and seven days after administration of the anti-human IL-6 receptor antibody. The blood was collected from the mice 15 minutes, seven hours, one day, two days, three days, and seven days after administration of the anti-human IL-6 receptor antibody. Immediately, the collected blood was centrifuged at 15,000 rpm and 4°C for 15 minutes to prepare the plasma. The isolated plasma was stored in a freezer set at -20°C or below until use.

(2-2-2) Determination of the soluble human IL-6 receptor (hsIL-6R) concentration in plasma by an electrochemiluminescent method

The hsIL-6R concentrations in mouse plasma were determined by the same electrochemiluminescent method as described in (2-1-2).

The result is shown in Fig. 3. The time course of human IL-6 receptor concentration in plasma of mice administered with Fv4-IgG1-F760, from which the mouse FcγR binding of Fv4-IgG1 is deleted, was demonstrated to be comparable to that in mice administered with Fv4-IgG1. The cytotoxic activity to a membrane antigen depends on the FcγR binding, and thus the cytotoxic activity is lost when eliminating the FcγR binding. On the other hand, even when administering an antibody, from which mouse FcγR binding is deleted, against human IL-6 receptor which is a soluble antigen, there was no effect on the time course of human IL-6 receptor concentration in the plasma of the administered mice. Thus, it would be thought that the FcγR binding of an antibody against the soluble antigen has no contribution to the time course of antigen concentration in the plasma of mice administered with the antibody.

Surprisingly, however, the human IL-6 receptor concentration in the plasma of mice administered with Fv4-IgG1-F1022 with enhanced mouse FcγR binding was considerably reduced as compared to the human IL-6 receptor concentration in the plasma of mice administered with Fv4-IgG1. As to the degree of reduction, the concentration was confirmed to be decreased below the baseline human IL-6 receptor concentration without antibody administration. In particular, the human IL-6 receptor concentration in the plasma of mice administered with Fv4-IgG1-F1022 was reduced down to about 1/100 three days after administration as compared to the case of Fv4-IgG1 administration. This finding demonstrates that, by administering to mice an antibody that binds to human IL-6 receptor in a pH-dependent manner and whose FcγR binding has been enhanced, the human IL-6 receptor concentration in the plasma of the mice can be significantly reduced, and as to the degree of reduction, the antigen concentration in plasma can be reduced below the level before antibody administration.

Furthermore, it was also demonstrated that, as compared to mice administered with Fv4-IgG1, the human IL-6 receptor concentration in plasma was reduced in mice administered with Fv4-IgG1-Fuc which has sugar chains with low fucose content and with increased mouse FcγR IV-binding activity. In particular, the human IL-6 receptor concentration in the plasma of mice administered with Fv4-IgG1-Fuc was reduced down to about 1/2 seven days after administration as compared to the case of Fv4-IgG1 administration. The above finding demonstrates that, by administering to mice a pH-dependent antigen-binding molecule that binds to human IL-6 receptor in a pH-dependent manner and whose FcyR binding has been enhanced, the soluble antigen concentration in the plasma of the mice can be reduced. In this case, methods for enhancing the FcγR binding are not particularly limited to introduction of amino acid alterations. It was demonstrated that such enhancement can be achieved, for example, by using a human IgG Fc region to which a sugar chain with low fucose content is linked at position 297 (EU numbering); however, the effect of Fv4-IgG1-Fuc to reduce antigen concentration was smaller than Fv4-F1022. Based on this result, it would be thought that, of several FcyRs (FcγRI, II, III, and IV for mouse), mFcγIV, to which the binding of Fv4-IgG1-Fuc is enhanced, does not have a large contribution to the reduction of antigen concentration as an FcγR.

Thus, it was revealed that, by administering to an individual an antibody that binds to a soluble antigen in a pH-dependent manner and whose FcγR binding has been enhanced, the soluble antigen concentration in the plasma of the individual can be markedly reduced.

Without being bound by a particular theory, the unexpected reduction of soluble antigen concentration in plasma, which was observed when administering an antigen-binding molecule that comprises an antigen-binding domain whose FcγR binding has been enhanced and whose antigen-binding activity is altered depending on the ion concentration condition such as pH and an FcRn-binding domain that has FcRn-binding activity under an acidic pH range condition, can be explained as follows.

IgG antibodies that are non-specifically incorporated into cells return to the cell surface by binding to FcRn under the acidic condition in the endosome, and then dissociate from FcRn under the neutral condition in plasma. In such a case, when an antibody that neutralizes the function of a soluble antigen by binding to the antigen is administered to mice in which the concentration of the soluble antigen is maintained constant in plasma, the soluble antigen in plasma forms a complex with the antibody administered. The soluble antigen incorporated into cells while remaining as the complex is thought to be recycled, in a state bound to the antibody, to the plasma together with the antibody, because the Fc region of the antibody binds to FcRn under the acidic condition in the endosome.

Meanwhile, when the antibody against the soluble antigen is an antibody that binds to the antigen in a pH-dependent manner (*i.e.,* an antibody that dissociates the soluble antigen under the acidic condition in the endosome), the soluble antigen that is non-specifically incorporated into cells while remaining as a complex with the antibody, is dissociated from the antibody in the endosome and degraded in the lysosome; thus, the soluble antigen is not recycled to the plasma. That is, it is thought that Fv4-IgG1 incorporated as a complex with the soluble antigen into cells can dissociate the soluble antigen in the endosome and thus accelerate the elimination of the soluble antigen.

As described above, antigen-binding molecules such as Fv4-IgG1, which contain an antigen-binding domain whose antigen-binding activity is altered depending on the ion concentration, are thought to be capable of binding to antigens repeatedly several times. The effect to accelerate the elimination of soluble antigens from the plasma by dissociating them in the endosome is thought to depend on the rate of incorporation of the antigen/antigen-binding molecule complex into the endosome. An antigen-binding molecule that contains an antigen-binding domain whose binding activity to various FcyRs has been increased and whose antigen-binding activity is altered depending on the condition of ion concentration, is actively incorporated into cells by binding to various FcyRs expressed on the cell membrane, and can be shuttled back to plasma by recycling *via* the binding between FcRn and the FcRn-binding domain comprised in the molecule, which has FcRn-binding activity under an acidic pH range condition. That is, it is thought that, since the above antigen-binding molecule which forms a complex with a soluble antigen in plasma is actively incorporated into cells *via* FcγR expressed on the cell membrane, its effect to accelerate the elimination of the soluble antigen from plasma is more markedly shown than antigen-binding molecules whose binding activity to various FcγRs has not been increased.

In the living organism, various FcγRs are expressed on the cell membrane of immune cells, and play a variety of functions. Any FcγRs may be used to incorporate antibodies into cells. Specifically, in human, the presence of inhibitory FcγRIIb, and activating FcγRs including FcγRI, FcγRIIa, and FcγRIIIa is known, and antibodies may be incorporated by any of them. Antibodies may be incorporated by all or any one of the FcγRs. Alternatively, antibodies may be incorporated in such a manner mediated by various activating FcγRs alone, or by inhibitory FcγRIIb alone.

On the other hand, to achieve the above purposes, it is possible to employ any methods for increasing the FcγR-binding activity of the FcγR-binding domain of antigen-binding molecules. For example, as shown in Example 1, amino acid mutations for increasing the FcγR-binding activity may be introduced into the FcγR-binding domain of antigen-binding molecules, or one can use low-fucose-type antibodies. Meanwhile, the effect to increase the FcγR-binding activity, which is achieved by such methods, may be an effect of augmenting the binding to any FcγR. Specifically, it is possible to increase the binding activity to any one, some, or all of the FcγRs. Furthermore, it is possible to only increase the binding activity to various activating FcγRs, or inhibitory FcγRIIb.

The FcγR-binding activity of an antibody that binds to a membrane antigen plays an important role in the cytotoxic activity of the antibody. Thus, when it is necessary for an antibody used as a pharmaceutical agent to have cytotoxic activity, a human IgG1 isotype with strong FcγR-binding activity is used. In addition, techniques to enhance the cytotoxic activity of such antibodies by increasing the FcγR-binding activity of the antibodies are used commonly in the art.

Meanwhile, the role of the FcγR-binding activity of antibodies that bind to soluble antigens and which are used as pharmaceutical agents has not been known in the art. There has been no sufficient assessment on what difference in the effect on the living organism administered with the antibodies is caused by the difference in the FcγR-binding activity between human IgG1 with high FcγR-binding activity and human IgG2 and human IgG4 with low FcγR-binding activity. Actually, it was demonstrated in the present Example that there was no influence on the time course of soluble antigen concentration in the plasma of the individuals administered with an antibody that lacks FcγR-binding activity. Meanwhile, in the present invention, it was revealed that the soluble antigen concentration was significantly reduced in the plasma of the individuals administered with an antigen-binding molecule whose FcγR-binding activity has been increased and which contains an antigen-binding domain whose soluble antigen-binding activity is altered depending on the ion concentration condition. Specifically, it can be said that the present inventors revealed for the first time the benefit of the enhancement of FcγR binding by combining an FcRn-binding domain that has FcRn-binding activity under an acidic pH range condition with an antigen-binding domain whose soluble antigen binding is altered depending on the ion concentration condition, comprised in an antigen-binding molecule targeted to a soluble antigen.

[Example 3] Effect of eliminating antigens from plasma by antigen-binding molecules whose FcγR-binding activity is greater than that of native human IgG Fc region and whose human FcRn-binding activity has been increased under an acidic pH range condition

(3-1) Preparation of antigen-binding molecules whose FcγR-binding activity is greater than the binding activity of native human IgG Fc region and whose human FcRn-binding activity has been increased under an acidic pH range condition

A reported method for improving the retention of IgG antibody in plasma is to improve the FcRn binding under an acidic pH range condition. It is thought that, when the FcRn binding under an acidic pH range condition is improved by introducing an amino acid substitution into the Fc region of an IgG antibody, this increases the recycling efficiency from the endosome to plasma, resulting in an improvement of the plasma retention of the IgG antibody.

There are many reports on amino acid alterations to improve the plasma retention by improving the human FcRn-binding activity under an acidic pH range condition. Such alterations include, for example:
the method for substituting Leu for Met at position 428 and Ser for Asn at position 434 (EU numbering) in an IgG antibody (Nat. Biotechnol, (2010) 28, 157-159); the method for substituting Ala for Asn at position 434 (Drug. Metab. Dispos. (2010) 38 (4), 600-605); the method for substituting Tyr for Met at position 252, Thr for Ser at position 254, and Glu for Thr at position 256 (J. Biol. Chem. (2006) 281, 23514-23524); the method for substituting Gln for Thr at position 250 and Leu for Met at position 428 (J. Immunol. (2006) 176 (1) 346-356); the method for substituting His for Asn at position 434 (Clin. Pharm. & Ther. (2011) 89 (2) 283-290.); and WO2010/106180; WO2010/045193; WO2009/086320; WO2009/058492; WO2008/022152; WO2006/050166, WO2006/053301, WO2006/031370; WO2005/123780; WO2005/047327; WO2005/037867; WO2004/035752; and WO2002/060919.

VH3-IgG1-F1093 (SEQ ID NO: 43) with a substitution of Leu for Met at position 428 and Ser for Asn at position 434 (EU numbering) in VH3-IgG1-F1022 was prepared to improve the pharmacodynamics of Fv4-IgG1-F1022 that was demonstrated to produce, when administered, the effect of significantly reducing the soluble antigen concentration in plasma, as described in Example 2. Fv4-IgG1-F1093 comprising VH3-IgG1-F1093 as the heavy chain and VL3-CK as the light chain was constructed using the method described in Reference Example 2.

(3-2) Effect of eliminating antigens from plasma by antigen-binding molecules whose FcyR-binding activity is greater than that of native human IgG Fc region and whose human FcRn-binding activity has been increased under an acidic pH range condition

An *in vivo* infusion test was carried out for Fv4-IgG1-F1093 by the same method as described in Example (2-1-1) using human FcRn transgenic mice in which the soluble human IL-6 receptor concentration is maintained constant in plasma. Soluble human IL-6 receptor concentrations in the plasma of the mice were determined by the method described in Example (2-1-2). The result is shown in Fig. 4.

(3-2-1) Determination of the anti-human IL-6 receptor antibody concentration in plasma by the ELISA method

Anti-human IL-6 receptor antibody concentrations in mouse plasma were determined by the ELISA method. First, an anti-Fv4 MABTECH ideotype antibody was aliquoted in a Nunc-Immuno Plate, MaxiSoup (Nalge nunc International). The plate was allowed to stand at 4°C overnight to prepare a plate immobilized with the anti-Fv4 ideotype antibody. The ideotype antibody was obtained by immunizing a rabbit with Fv4-M73 (WO2009/125825). After purifying the serum using an ion-exchange resin, the antibody was affinity-purified by a column immobilized with Fv4-M73, followed by adsorption using an immobilized column for human. Standard curve samples containing an anti-human IL-6 receptor antibody (concentration in plasma: 0.8, 0.4, 0.2, 0.1, 0.05, 0.025, and 0.0125 μg/ml) and assay samples of mouse plasma diluted 100 times or more were prepared. 100 μl each of the standard curve and assay samples were combined with 200 μl of 20 ng/ml soluble human IL-6 receptor. The resulting mixtures were allowed to stand at room temperature for one hour, and aliquoted to each well of the plate immobilized with the anti-Fv4 ideotype antibody. The plate was allowed to stand at room temperature for another one hour. Then, Biotinylated Anti-human IL-6 R Antibody (R&D) was reacted thereto at room temperature for one hour. Next, Streptavidin-PolyHRP80 (Stereospecific Detection Technologies) was reacted thereto at room temperature for one hour. The chromogenic reaction of the reaction solution was performed using as a substrate TMB One Component HRP Microwell Substrate (BioFX Laboratories). After terminating the reaction with IN sulfuric acid (Showa Chemical), the absorbance at 450 nm of the reaction solution of each well was measured with a microplate reader. Antibody concentrations in mouse plasma were determined based on the absorbance of the standard curve using the analysis software SOFTmax PRO (Molecular Devices).

The result is shown in Fig. 5.

(3-3) Improvement of pharmacodynamics by increasing the human FcRn-binding activity under an acidic pH range condition

As shown in Fig. 5, in the group administered with Fv4-IgG1-F1022 resulting from the enhancement of the FcγR-binding activity of Fv4-IgG1 under a neutral pH range condition, the plasma retention of the administered antibody was demonstrated to be reduced as compared to the group administered with Fv4-IgG1. Meanwhile, in the group administered with Fv4-IgG1-F1093 resulting from the enhancement of the human FcRn-binding activity of Fv4-IgG1-F1022 under an acidic pH range condition, the plasma retention of the administered antibody was demonstrated to be significantly improved as compared to the group administered with Fv4-IgG1-F1022.

Furthermore, as shown in Fig. 4, the time course of the soluble human IL-6 receptor concentration in the plasma of the Fv4-IgG1-F1022-administered group was equivalent to that of the Fv4-IgG1-F1093-administered group, up to three days after antibody administration. On day three after administration, as compared to the Fv4-IgG1-administered group, the soluble human IL-6 receptor concentration in plasma was reduced as much as about 100 times in both of the Fv4-IgG1-F1022 and Fv4-IgG1-F1093-administered groups. However, on day seven after antibody administration, the soluble human IL-6 receptor concentration in plasma was observed to be elevated in the Fv4-IgG1-F1022-administered group as compared to on day three after administration. On the other hand, in the Fv4-IgG1-F1093-administered group, an increase in the plasma concentration of soluble human IL-6 receptor was not observed, showing that the effect to reduce the soluble human IL-6 receptor concentration was sustained in this administration group.

Specifically, Fv4-IgG1-F1093, when administered, reduced the soluble human IL-6 receptor concentration in the plasma of the administered individual down to about 1/100 as compared to Fv4-IgG1, and in addition, it sustained this condition for a long period. Thus, Fv4-IgG1-F1093 was demonstrated to be a highly excellent antigen-binding molecule. Without being bound by a particular theory, the phenomenon observed herein can be explained as follows. Fv4-IgG1-F1022 in which the FcyR-binding activity of Fv4-IgG1 has been increased under a neutral pH range condition is thought to be incorporated in a large amount mainly into immune cells expressing FcγR on the cell membrane. The incorporated antibody is transferred into the endosome, and by binding to FcRn in the endosome, the antibody is recycled to the plasma. When the FcRn-binding activity of the antibody is not high enough under the condition at acidic pH in the endosome, the antibody incorporated into the endosome is thought to be incapable of sufficient recycling. Specifically, a possible reason for the reduced plasma retention of Fv4-IgG1-F1022 relative to Fv4-IgG1 would be that the FcRn-binding activity under an acidic pH range condition is insufficient for sufficient recycling of the endosome-incorporated antibody to the plasma by FcRn binding, and the antibody that was not recycled was degraded in the lysosome.

On the other hand, as with Fv4-IgG1-F1022, Fv4-IgG1-F1093 resulting from the enhancement of the human FcRn-binding activity of Fv4-IgG1-F1022 under an acidic pH range condition is thought to be incorporated in a large amount mainly into immune cells expressing FcγR on the cell membrane. An antibody incorporated and transferred into the endosome is recycled to the plasma by binding to FcRn in the endosome. Since its human FcRn-binding activity under an acidic pH range condition is enhanced, Fv4-IgG1-F1093 is thought to have sufficient FcRn-binding activity in the endosome. Thus, after incorporation into cells, most of Fv4-IgG1-F1093 is recycled to the plasma. Thus, it would be thought that the plasma retention of Fv4-IgG1-F1093 was improved in administered individuals as compared to Fv4-IgG1-F1022.

On the other hand, it has been known that the plasma retention of ordinary antibodies is improved when their FcRn-binding activity is improved under an acidic pH range condition. However, it is thought that, when the antibody retention in plasma is improved, the plasma retention of antibody-bound antigens is also improved, and this results in an increase of the antigen concentration in plasma. In actual, as described in WO2010/088444, Antibody 18E introduced with the alteration YTE into Antibody 18, which is a human IgG1 antibody against IL-6, to increase the FcRn-binding activity under an acidic pH range condition, showed improved antibody retention in the plasma of cynomolgus monkeys, and at the same time, the concentration of the IL-6 antigen was also elevated in the plasma.

Surprisingly, however, when administering Fv4-IgG1-F1093 introduced with an alteration similar to YTE for increasing the FcRn-binding activity under an acidic pH range condition into Fv4-F1022 that binds to the antigen in a pH-dependent manner and has increased FcyR-binding activity, the plasma retention of the antibody was significantly improved in the administered individuals without increasing the concentration of soluble human IL-6 receptor which is the antigen. Rather, on day seven after antibody administration, the soluble human IL-6 receptor concentration remained low in the individuals administered with Fv4-IgG1-F1093 as compared to those administered with Fv4-F1022.

Without being bound by a particular theory, the phenomenon observed herein can be explained as follows. When administered to a living organism, an antibody without pH-dependent antigen binding is non-specifically incorporated into cells. Antigens that remain to be bound to the antibody are recycled to the plasma in the same extent as the antibody. Meanwhile, for an antibody with increased FcRn-binding activity under an acidic pH range condition, the extent of recycling to the plasma in a living organism administered with the antibody is higher than that of an antibody without increased FcRn-binding activity, and this results in an increased extent of recycling of antigens bound to the antigen to the plasma in the living organism. Thus, due to the improved plasma retention of the antibody administered in the living organism, the plasma concentration of the antigen to which the antibody binds is thought to be also increased in the living organism.

Meanwhile, when administered to a living organism, an antibody that binds to an antigen in a pH-dependent manner and which has increased FcyR-binding activity is mainly incorporated into immune cells expressing FcyR on the cell membrane, and this reduces the plasma retention. Furthermore, after being incorporated into the cells while bound to the antibody, the antigen is dissociated from the antibody in the endosome and then degraded in the lysosome, resulting in a decrease of the antigen concentration in plasma in the living organism. When the FcRn-binding activity is increased under an acidic pH range condition, the antibody retention in plasma, even if worsened due to increased FcγR-binding activity, is improved by an increase in the rate of recycling by FcRn. In this case, since the antigen bound to the antibody that binds to the antigen in a pH-dependent manner is dissociated from the antibody in the endosome and directly degraded in the lysosome, it is not thought that the antigen concentration is increased in the plasma. Furthermore, the improved plasma retention of the antibody administered to the living organism is thought to allow the antigen elimination effect of the antibody to be sustained, and the antigen concentration to be maintained low for a longer period.

The above findings demonstrate that the plasma retention of an administered antibody is improved in a living organism administered with the antibody in which the human FcRn-binding activity under an acidic pH range condition is enhanced in an antigen-binding molecule whose FcyR-binding activity is higher than that of native human IgG Fc region. Furthermore, it was revealed that, in this case, the antibody retention in plasma is improved without deteriorating the antigen-elimination effect.

[Example 4] Further assessment of the effect of eliminating antigens from plasma antigen-binding molecules whose FcyR-binding activity is greater than that of native human IgG Fc region and whose human FcRn-binding activity has been increased under an acidic pH range condition

(4-1) The antigen elimination effect in the living organism administered with an antibody whose FcyR-binding activity is higher than that of the Fc region of native human IgG and which has human FcRn-binding activity increased under conditions of acidic pH range

As described in Example 2, the antigen concentration in plasma was significantly reduced in the group administered with Fv4-IgG1-F1022 with enhanced mouse FcγR binding. Meanwhile, as shown in Example 3, the reduced plasma retention observed in the Fv4-IgG1-F1022-administered group was markedly improved by increasing the human FcRn-binding activity of Fv4-IgG1-F1022 under an acidic pH range condition. Next, the effect of eliminating soluble antigens from plasma by enhancing mouse FcyR binding and the effect of improving the plasma retention of an antibody in the living organism administered with it by enhancing the human FcRn binding activity under an acidic pH range condition, were further assessed as described below.

(4-2) Preparation of an anti-human IL-6 receptor antibody with enhanced mouse FcyR binding

VH3-IgG1-F1087 (SEQ ID NO: 123) resulting from substituting Asp for Lys at position 326 (EU numbering) in VH3-IgG1, and VH3-IgG1-F1182 (SEQ ID NO: 124) resulting from substituting Asp for Ser at position 239 and Glu for Ile at position 332 (EU numbering) in VH3-IgG1, were prepared as antigen-binding molecules with enhanced mouse FcγR binding. Fv4-IgG1-F1087 that contains VH3-IgG1-F1087 as the heavy chain and VL3-CK as the light chain, and Fv4-IgG1-F1182 that contains VH3-IgGl-F1182 as the heavy chain and VL3-CK as the light chain, were produced using the method described in Reference Example 2.

(4-3) Assessment of mouse FcyR-binding activity

VH3/L (WT)-IgG1-F1087 and VH3/L (WT)-IgG1-F1182 which contain VH3-IgG1-F1087 and VH3-IgG1-F1182 as the heavy chain, respectively, and L (WT)-CK (SEQ ID NO: 42) as the light chain, were prepared by the method described in Reference Example 2. These antibodies and VH3/L (WT)-IgG1-F1022 were assessed for their mouse FcyR-binding activity by the method described in Reference Example 2. The result is shown in Table 8. In addition, the ratio of the increase in the mouse FcyR-binding activity of each variant relative to the IgG1 before alteration is shown in Table 9.

[Table 8]

| VARIANT NAME | KD (M) | | | |
|---|---|---|---|---|
| | mFc γ RI | mFc γ RIIb | mFc γ RIII | mFcγ RIV |
| IgGI | 5.3E-08 | 9.8E-07 | 2.4E-06 | 8.6E-08 |
| F1022 | 7.6E-09 | 1.0E-08 | 5.5E-09 | 1.4E-07 |
| F1087 | 2.9E-08 | 5.6E-08 | 5.2E-08 | 3.3E-07 |
| F1182 | 2.4E-09 | 1.1E-07 | 4.8E-07 | 5.3E-10 |

[Table 9]

| VARIANT NAME | RATIO OF BINDING TO IgG 1 | | | |
|---|---|---|---|---|
| | mFc γ RI | mFcγRIIb | mFc γ RIII | mFc γ RIV |
| IgG1 | 1.0 | 1.0 | 1.0 | 1.0 |
| F1022 | 7.0 | 93.6 | 440.5 | 0.6 |
| F1087 | 1.8 | 17.5 | 46.2 | 0.3 |
| F1182 | 22.1 | 9.1 | 5.0 | 162.3 |

As shown in Table 9, it was demonstrated that F1087 and F1022 had increased binding activity to mouse FcγRI, mouse FcγRIIb, and mouse FcγRIII as compared to IgG1, whereas their mouse FcγRIV-binding activity was not increased. Regarding the binding activity of F1087 to mouse FcγRI, mouse FcγRIIb, mouse FcγRIII, and mouse FcγRIV, the extent of its increase was revealed to be smaller than that of F1022. Meanwhile, it was shown that the binding activity of F1182 to mouse FcγRI and mouse FcγRIV was considerably increased, whereas the extent of increase in its binding activity to FcγRIIb and FcγRIII was smaller than those of F1022 and F1087. As mentioned above, these three types of variants showed enhanced binding to some mouse FcγRs; however, it was shown that the FcγR to which the binding activity is selectively increased and the extent of the increase vary depending on the variant.

(4-4) The effect of eliminating antigens from the plasma in an individual administered with Fv4-IgG1-F1087 and Fv4-IgG1-F1182

By the same method as described in Example 2, *in vivo* infusion tests using human FcRn transgenic mice were carried out to determine the soluble IL-6 receptor concentrations in the plasma of the mice. The result is shown in Fig. 6.

In both of the groups administered with Fv4-IgG1-F1087 and Fv4-IgG1-F1182 *in vivo,* which have increased mouse FcγR-binding activity as compared to Fv4-IgG1, the *in vivo* plasma concentration of soluble human IL-6 receptor reduced as compared to the group administered with Fv4-IgG1. The effect to reduce the above plasma concentration of soluble human IL-6 receptor was high especially in the group administered with Fv4-IgG1-F1087 which has enhanced binding to mouse FcγRII and mouse FcγRIII. Meanwhile, the effect of F1182 administration to reduce the plasma concentration of soluble human IL-6 receptor was small in the group administered with F1182 *in vivo* which has considerably increased binding activity to mouse FcγRI and mouse FcγRIV (as well as several-fold enhanced binding to mouse FcγRII and mouse FcγRIII). It was thought from these results that the mouse FcγRs that more significantly contribute by an effect that efficiently decreases the antigen concentration in the plasma of mice administered with a pH-dependent antigen-binding antibody, are mouse FcγRII and/or mouse FcγRIII. Specifically, it is thought that the plasma antigen concentration can be more efficiently reduced *in vivo* by administering into a living organism a pH-dependent antigen-binding antibody with enhanced binding to mouse FcγRII and/or mouse FcγRIII.

(4-5) Preparation of antigen-binding molecules whose FcγR-binding activity is greater than the binding activity of native human IgG Fc region and which have increased human FcRn-binding activity under an acidic pH range condition

As described in Example 3, when compared to human FcRn transgenic mice administered with Fv4-IgG1-F1022, the plasma retention of an administered antibody is markedly improved in human FcRn transgenic mice administered with Fv4-IgG1-F1093 resulting from increasing the human FcRn-binding activity under an acidic pH range condition of Fv4-IgG1-F1022 in which the mouse FcγR-binding activity has been increased. Whether this effect is also observed in human FcRn transgenic mice administered with Fv4-IgG1-F1087 and Fv4-IgG1-F1182, and whether the same effect is observed in mice administered with variants whose human FcRn-binding activity has been increased under an acidic pH range condition by addition of an alteration distinct from the alteration assessed in Example 3 were assessed as follows.

VH3-IgG1-F1180 (SEQ ID NO: 125) and VH3-IgG1-F1181 (SEQ ID NO: 126) were prepared by substituting Leu for Met at position 428 and Ser for Asn at position 434 (EU numbering) in the heavy chains VH3-IgG1-F1087 and VH3-IgG1-F1182, in order to increase their human FcRn-binding activity of Fv4-IgG1-F1087 and Fv4-IgG1-F1182 under an acidic pH range condition. Furthermore, VH3-IgG1-F1412 (SEQ ID NO: 127) was prepared by substituting Ala for Asn at position 434 (EU numbering) in the heavy chain VH3-IgG1-F1087, in order to increase the human FcRn-binding activity of Fv4-IgG1-F1087 under an acidic pH range condition. Fv4-IgG1-F1180, Fv4-IgG1-F1181, and Fv4-IgG1-F1412, which contain the above heavy chains and VL3-CK as the light chain, were prepared using the method described in Reference Example 2.

(4-6) Improvement of pharmacodynamics of antibodies with increased human FcRn-binding activity under an acidic pH range condition

*In vivo* infusion tests were carried out by administering Fv4-IgG1-F1180, Fv4-IgG1-F1181, and Fv4-IgG1-F1412 to human FcRn transgenic mice according to the same method as described in Example 2 to determine the soluble IL-6 receptor concentrations in the plasma of the mice. The results on the antibody concentrations in the plasma of the mouse groups administered with Fv4-IgG1-F1087, Fv4-IgG1-F1180, Fv4-IgG1-F1412, and Fv4-IgG1 are shown in Fig. 7. The results on the antibody concentrations in the plasma of the mouse groups administered with Fv4-IgG1-F1182, Fv4-IgG1-F1181, and Fv4-IgG1 are shown in Fig. 8. Meanwhile, the plasma antibody concentrations in the mouse groups were measured by the method described in Example 3. The results on the plasma soluble IL-6 receptor concentrations of Fv4-IgG1-F1087, Fv4-IgG1-F1180, Fv4-IgG1-F1412, and Fv4-IgG1 in the mouse groups are shown in Fig. 9; and the results on the plasma soluble IL-6 receptor concentrations of Fv4-IgG1-F1182, Fv4-IgG1-F1181, and Fv4-IgG1 are shown in Fig. 10.

It was confirmed that, as compared to the group of mice administered with Fv4-IgG1-F1182, the plasma retention of administered antibodies was improved in the group of mice administered with Fv4-IgG1-F1181 resulting from increasing the human FcRn-binding activity of Fv4-IgG1-F1182 in an acidic pH range. Meanwhile, the soluble IL-6 receptor concentration in the plasma of the mouse groups administered with Fv4-IgG1-F1181 was comparable to that in the group of mice administered with Fv4-IgG1-F1182. When compared to the mouse groups administered with Fv4-IgG1, the soluble IL-6 receptor concentration in the plasma was decreased in both groups.

On the other hand, as compared to the group of mice administered with Fv4-IgG1-F1087, the plasma retention of administered antibodies was improved in both groups of mice administered with Fv4-IgG1-F1180 and Fv4-IgG1-F1412 resulting from increasing the human FcRn-binding activity of Fv4-IgG1-F1087 in an acidic pH range, and surprisingly, the plasma retention was improved up to a level comparable to that of the mouse groups administered with Fv4-IgG1. Furthermore, the sustainability of the effect of reducing the soluble IL-6 receptor concentration in plasma was improved by the improvement of the plasma antibody retention in the groups of administered mice. Specifically, in the groups of administered mice, the soluble IL-6 receptor concentrations in plasma 14 days and 21 days after administration of Fv4-IgG1-F1180 and Fv4-IgG1-F1412 were significantly reduced as compared to the concentrations 14 days and 21 days after administration of Fv4-IgG1-F1087.

In view of the above, as for the groups of mice administered with the four examples of antibodies, Fv4-IgG1-F1093, Fv4-IgG1-F1181, Fv4-IgG1-F1180, and Fv4-IgG1-F1412, it was demonstrated that the plasma retention can be improved in a living organism administered with an antibody in which the human FcRn-binding activity under an acidic pH range condition has been enhanced in an antigen-binding molecule whose FcγR-binding activity is higher than the binding activity of native human IgG Fc region. It was also demonstrated that, in the living organism administered with the antigen-binding molecule, the plasma retention is improved without deteriorating the effect of eliminating antigens from the living organism, and rather, the antigen elimination effect can be sustained.

(4-7) Preparation of antigen-binding molecules with increased human FcRn-binding activity under an acidic pH range condition and suppressed binding to a rheumatoid factor

In recent years, an antibody molecule resulting from substituting His for Asn at position 434 (EU numbering) in a humanized anti-CD4 antibody to improve the plasma retention by increasing its human FcRn-binding activity under an acidic pH range condition, has been reported to bind to the rheumatoid factor (RF) (Clin. Pharmacol. Ther. (2011) 89 (2), 283-290). This antibody has a human IgG1 Fc region and a substitution of His for Asn at position 434 (EU numbering) in the FcRn-binding site. The rheumatoid factor has been demonstrated to recognize and bind to the substituted portion.

As described in Example (4-6), as compared to the case where Fv4-IgG1-F1087 was administered to human FcRn transgenic mice, Fv4-IgG1-F1180 resulting from increasing under conditions of acidic pH range the human FcRn-binding activity of Fv4-IgG1-F1087 with increased mouse FcγR-binding activity, showed improved retention in plasma. Various alterations have been reported to increase the human FcRn-binding activity under conditions of acidic pH range. Of such modifications, a variant with a substitution of Leu for Met at position 428 and Ser for Asn at position 434 (EU numbering) in the heavy chain has been reported to show augmented binding to rheumatoid factors.

However, a variant that has a substitution of Thr for Tyr at position 436 (EU numbering) in addition to the above substitutions at positions 428 and 434 (EU numbering) shows significantly reduced binding to rheumatoid factors while retaining increased human FcRn-binding activity under conditions of acidic pH range.

That is, antigen-binding molecules that have increased human FcRn-binding activity under an acidic pH range condition but do not have the binding to the rheumatoid factor can be produced by introducing into the site of the Fc region an alteration that reduces the rheumatoid factor-binding activity alone without reducing the FcRn-binding activity under an acidic pH range condition.

Such alterations used for reducing the rheumatoid factor-binding activity include alterations at positions 248-257, 305-314, 342-352, 380-386, 388, 414-421, 423, 425-437, 439, and 441-444 (EU numbering), preferably those at positions 387, 422, 424, 426, 433, 436, 438, and 440 (EU numbering), and particularly preferably, an alteration that substitutes Glu or Ser for Val at position 422, an alteration that substitutes Arg for Ser at position 424, an alteration that substitutes Asp for His at position 433, an alteration that substitutes Thr for Tyr at position 436, an alteration that substitutes Arg or Lys for Gln at position 438, and an alteration that substitutes Glu or Asp for Ser at position 440 (EU numbering). These alterations may be used alone or in combination.

Alternatively, it is possible to introduce N-type glycosylation sequences to reduce the rheumatoid factor-binding activity. Specifically, known N-type glycosylation sequences include Asn-Xxx-Ser/Thr (Xxx represents an arbitrary amino acid other than Pro). This sequence can be introduced into the Fc region to add an N-type sugar chain, and the binding to RF can be inhibited by the steric hindrance of the N-type sugar chain. Alterations used for adding an N-type sugar chain preferably include an alteration that substitutes Asn for Lys at position 248, an alteration that substitutes Asn for Ser at position 424, an alteration that substitutes Asn for Tyr at position 436 and Thr for Gln at position 438, and an alteration that substitutes of Asn for Qln at position 438, according to EU numbering, particularly preferably an alteration that substitutes Asn for Ser at position 424 (EU numbering).

(4-8) Assessment of the pharmacodynamics-improving effect of antigen-binding molecules with increased human FcRn-binding activity under conditions of acidic pH range and reduced rheumatoid factor binding

In order to assess the effect of antibodies with an alteration that reduces the above-mentioned rheumatoid factor-binding activity, Fv4-IgG1-F1782 with a substitution of Leu for Met at position 428, Ser for Asn at position 434, and Thr for Tyr at position 436 (EU numbering) in the heavy chain of Fv4-IgG1-F1087, was produced using the method described in Reference Example 2. As described in Example (4-7), Fv4-IgG1-F1782 is an antibody whose human FcRn-binding activity under conditions of acidic pH and mouse FcγR-binding activity have been both increased, but its rheumatoid factor-binding activity has not been increased as compared to native human IgG1. To test whether the plasma retention of antibody Fv4-IgG1-F1782 has been improved as compared to Fv4-IgG1-F1087, the pharmacodynamics of the antibodies in the plasma of human FcRn transgenic mice administered with the antibodies was assessed by the same method as described in Example 2. The plasma concentration of soluble human IL-6 receptor was determined by the method described in Example (2-1-2), while plasma antibody concentrations were determined by the method described in Example (3-2-1).

A time course of antibody concentrations in plasma is shown in Fig. 11, and a time course of plasma concentration of soluble human IL-6 receptor is shown in Fig. 12. Fv4-IgG1-F1782 showed improved plasma antibody retention as compared to Fv4-IgG1-F1087. Meanwhile, the plasma concentration of soluble human IL-6 receptor was significantly reduced in the groups administered with the above antibodies as compared to the Fv4-IgG1 administration group.

Without being bound by a particular theory, the above results can be interpreted as follows. The results described in Examples 3 and 4 show that the plasma retention can be prolonged in the living organism administered with an antibody resulting from increasing under conditions of acidic pH range the human FcRn-binding activity of an antigen-binding molecule whose FcγR-binding activity is higher than the binding activity of the Fc region of natural human IgG. It was also demonstrated that, in the living organism administered with such an antigen-binding molecule, the plasma retention is prolonged without deteriorating the effect of eliminating antigens from the living organism, and rather the antigen elimination effect can be sustained.

However, it is concerned that antigen-binding molecules introduced with an alteration for increasing the human FcRn-binding activity under conditions of acidic pH range would have a risk of increased rheumatoid factor-binding activity. Thus, the plasma retention can be improved without increasing the rheumatoid factor-binding activity by introducing into the binding molecules a mutation that reduces the rheumatoid factor-binding activity while retaining the human FcRn-binding activity under conditions of acidic pH range.

In other words, it was revealed that when administered into a living organism, antigen-binding molecules that have the property of binding to antigens in a pH-dependent manner, and have increased human FcRn-binding activity under conditions of acidic pH range, and whose FcγR-binding activity is greater than that of the Fc region of native human IgG, and that have reduced rheumatoid factor-binding activity, have an excellent property in that the antigen-binding molecules show prolonged plasma retention without increasing their rheumatoid factor-binding activity, and effectively reduce the soluble antigen concentration in the living organism.

[Example 5] Effect of eliminating antigens from the plasma of a living organism administered with an antigen-binding molecule whose FcγR-binding activity is higher than the binding activity of native mouse IgG Fc region

(5-1) The antigen elimination effect from the plasma of a living organism administered with a mouse antibody with increased FcγR-binding activity

As described in Examples 1 to 4, it was demonstrated that the elimination of soluble human IL-6 receptor from mouse plasma is accelerated in the groups of human FcRn transgenic mice administered with antigen-binding molecules resulting from increasing the mouse FcγR-binding activity of antigen-binding molecules that have a human antibody Fc region and the property of binding to human IL-6 receptor in a pH-dependent manner. Whether this effect is also achieved in normal mice having mouse FcRn that was administered with antigen-binding molecules that have a mouse antibody Fc region and the property of binding to human IL-6 receptor in a pH-dependent manner, was assessed in normal mice having mouse FcRn as follows.

(5-2) Preparation of mouse antibodies with increased FcγR-binding activity

For a mouse IgG1 antibody having the property of binding to human IL-6 receptor in a pH-dependent manner, the heavy chain VH3-mIgG1 (SEQ ID NO: 128) and the light chain VL3-mk1 (SEQ ID NO: 129) were constructed using the method described in Reference Example 2. Meanwhile, to increase the mouse FcγR-binding activity of VH3-mIgG1, VH3-mIgG1-mF44 (SEQ ID NO: 130) was produced by substituting Asp for Ala at position 327 (EU numbering). Likewise, VH3-mIgG1-mF46 (SEQ ID NO: 131) was produced by substituting Asp for Ser at position 239 and Asp for Ala at position 327, according to EU numbering, in VH3-mIgG1. Fv4-mIgG1, Fv4-mIgG1-mF44, and Fv4-mIgG1-mF46, which contain VH3-mIgG1, VH3-mIgG1-mF44, and VH3-mIgG1-mF46, respectively, as the heavy chain, and VL3-mk1 as the light chain, were prepared using the method described in Reference Example 2.

(5-3) Assessment of mouse FcyR-binding activity of mouse antibodies with enhanced FcyR-binding activity

VH3/L (WT)-mIgG1, VH3/L (WT)-mIgG1-mF44, and VH3/L (WT)-mIgG1-mF46, which contain VH3-mIgG1, VH3-mIgG1-mF44, and VH3-mIgG1-mF46, respectively, as the heavy chain, and L (WT)-CK (SEQ ID NO: 42) as the light chain, were prepared by the method described in Reference Example 2. These antibodies were assessed for their mouse FcγR-binding activity by the method described in Reference Example 25. The result is shown in Table 10. In addition, the ratio of the increase in the mouse FcγR-binding activity of each variant relative to the mIgG1 before alteration is shown in Table 11.

[Table 10]

| VARIANT NAME | KD (M) | | | |
|---|---|---|---|---|
| | mFc γ RI | mFc γ RIIb | mFc γ RIII | mFc γ RIV |
| mIgG1 | NOT DETECTED | 1.1E-07 | 2.IE-07 | NOT DETECTED |
| mF44 | NOT DETECTED | 8.9E-09 | 6.7E-09 | NOT DETECTED |
| mF46 | NOT DETECTED | 1.2E-09 | 3.6E-09 | NOT DETECTED |

[Table 11]

| VARIANT NAME | RATIO OF BINDING TO IgG 1 | | | |
|---|---|---|---|---|
| | mFc γ RI | mFcγRIIb | mFc γ RIII | mFc γ RIV |
| mIgG1 | NOT DETECTED | 1.0 | 1.0 | NOT DETECTED |
| mF44 | NOT DETECTED | 11.9 | 31.0 | NOT DETECTED |
| mF46 | NOT DETECTED | 91.4 | 57.5 | NOT DETECTED |

The assessment result of Example 4 showing that VH3/L (WT)-mIgG1 having the Fc region of native mouse IgG1 antibody only binds to mouse FcγRIIb and mouse FcγRIII but not to mouse FcγRI and mouse FcγRIV, suggests that mouse FcγRs important for the reduction of antigen concentration are mouse FcγRII and/or mouse FcγRIII. VH3/L (WT)-mIgG-mF44 and VH3/L (WT)-mIgG1-mF46 introduced with an alteration that is thought to increase the FcγR-binding activity of VH3/L (WT)-mIgG1 was demonstrated to have increased binding activity to both of mouse FcγRIIb and mouse FcγRIII.

(5-4) Assessment of the effect to reduce the soluble IL-6 receptor concentration in the plasma of normal mice

The effect to eliminate soluble IL-6 receptor from the plasma of normal mice administered with the anti-human IL-6 receptor antibody Fv4-mIgG1, Fv4-mIgG1-mF44, or Fv4-mIgG1mF46 was assessed as follows.

An animal model where the soluble human IL-6 receptor concentration is maintained in a steady state in plasma was created by implanting an infusion pump (MINI-OSMOTIC PUMP MODEL2004, alzet) containing soluble human IL-6 receptor under the skin on the back of normal mice (C57BL/6J mouse, Charles River Japan). The *in vivo* dynamics of soluble human IL-6 receptor after administration of the anti-human IL-6 receptor antibody was assessed in the animal model. To suppress the production of antibodies against soluble human IL-6 receptor, an anti-mouse CD4 monoclonal antibody was administered once at 20 mg/kg into the caudal vein. Then, an infusion pump containing 92.8 μg/ml soluble human IL-6 receptor was subcutaneously implanted on the back of the mice. Three days after implantation of the infusion pump, the anti-human IL-6 receptor antibody was administered once at 1 mg/kg into the caudal vein. The blood was collected from the mice 15 minutes, seven hours, one day, two days, four days, seven days, 14 days (or 15 days), and 21 days (or 22 days) after administration of the anti-human IL-6 receptor antibody. Immediately thereafter, the collected blood was centrifuged at 15,000 rpm and 4°C for 15 minutes to prepare the plasma. The isolated plasma was stored in a freezer set at -20°C or below until use.

The soluble human IL-6 receptor concentrations in plasma were determined by the method described in (2-1-2). The result is shown in Fig. 13.

Surprisingly, it was demonstrated that, in mice administered with mF44 and mF46 introduced with an alteration to increase the binding activity of mIgG1 (native mouse IgG1) to mouse FcγRIIb and mouse FcyRIII, the plasma IL-6 receptor concentration was markedly reduced as compared to mice administered with mIgG1. In particular, even on day 21 after administration of mF44, the plasma IL-6 receptor concentration in the mF44-administered group was reduced by about 6 times as compared to the plasma IL-6 receptor concentration in the group without antibody administration, and about 10 times as compared to the mIgG1-administered group. On the other hand, on day seven after administration of mF46, the plasma IL-6 receptor concentration in the mF46-administered group was markedly reduced by about 30 times as compared to the plasma IL-6 receptor concentration in the group without antibody administration, and about 50 times as compared to the mIgG1-administered group.

The above findings demonstrate that the elimination of soluble IL-6 receptor from plasma was also accelerated in mice administered with antibodies in which the mouse FcyR-binding activity of an antigen-binding molecule having the Fc regions of mouse IgG1 antibody is increased, as with antibodies in which the mouse FcyR-binding activity of an antigen-binding molecule having the Fc region of human IgG1 antibody is increased. Without being bound by a particular theory, the phenomenon observed as described above can be explained as follows.

When administered to mice, antibodies that bind to a soluble antigen in a pH-dependent manner and have increased FcγR-binding activity are actively incorporated mainly into cells expressing FcγR on the cell membrane. The incorporated antibodies dissociate the soluble antigen under an acidic pH condition in the endosome, and then recycled to plasma *via* FcRn. Thus, a factor that achieves the effect of eliminating the plasma soluble antigen of such an antibody is the FcγR-binding activity level of the antibody. Specifically, as the FcγR-binding activity is greater, the incorporation into FcγR-expressing cells occurs more actively, and this makes the elimination of soluble antigens from plasma more rapid. Furthermore, as long as the FcγR-binding activity has been increased, the effect can be assessed in the same manner regardless of whether the Fc region contained in an antibody originates from human or mouse IgG1. Specifically, the assessment can be achieved for an Fc region of any animal species, such as any of human IgG1, human IgG2, human IgG3, human IgG4, mouse IgG1, mouse IgG2a, mouse IgG2b, mouse IgG3, rat IgG, monkey IgG, and rabbit IgG, as long as the binding activity to the FcγR of the animal species to be administered has been increased.

[Example 6] The antigen elimination effect by antibodies with the binding activity increased in a FcyRIIb-selective manner

(6-1) The antigen elimination effect of antibodies in which the FcγRIIb-binding activity has been selectively increased

FcγRIII-deficient mice (B6.129P2-FcgrFcγR3tm1Sjv/J mouse, Jackson Laboratories) express mouse FcγRI, mouse FcγRIIb, and mouse FcγRIV, but not mouse FcγRIII. Meanwhile, Fc receptor γ chain-deficient mice (Fcerlg mouse, Taconic, Cell (1994) 76, 519-529) express mouse FcγRIIb alone, but not mouse FcγRI, mouse FcγRIII, and mouse FcγRIV.

As described in Example 5, it was demonstrated that mF44 and mF46 with increased FcγR-binding activity of native mouse IgG1 show selectively enhanced binding to mouse FcγRIIb and mouse FcγRIII. It was conceived that, using the selectively increased binding activity of the antibodies, the condition under which an antibody with selectively enhanced mouse FcγRIIb binding is administered can be mimicked by administering mF44 and mF46 to mouse FcγRIII-deficient mice or Fc receptor γ chain-deficient mice which do not express mouse FcγRIII.

(6-2) Assessment of the antigen elimination effect in a living organism administered with an antibody with -selective enhancement of binding to mouse FcγRIIb using FcγRIII-deficient mice

The effect to eliminate soluble IL-6 receptor from plasma in FcγRIII-deficient mice administered with the anti-human IL-6 receptor antibody Fv4-mIgG1, Fv4-mIgG1-mF44, or Fv4-mIgG1-mF46 was assessed by the same method described in Example 5. The soluble human IL-6 receptor concentrations in the plasma of the mice were determined by the method described in Example (2-1-2). The result is shown in Fig. 14.

Surprisingly, it was demonstrated that, the plasma IL-6 receptor concentrations in FcγRIII-deficient mice administered with mF44 and mF46, which mimic the condition under which the mouse FcγRIIb-binding activity of mIgG1 (native mouse IgG1) is selectively increased, were markedly reduced as compared to the plasma IL-6 receptor concentration in mice administered with mIgG1. In particular, the plasma IL-6 receptor concentration of the mF44-administered group was reduced by about three times as compared to that of the mIgG1-administered group and the accumulation of antibody concentration due to antibody administration was suppressed. Meanwhile, on day three after administration, the plasma IL-6 receptor concentration of the mF46-administered group was markedly reduced by about six times as compared to the plasma IL-6 receptor concentration of the group without antibody administration, and about 25 times as compared to the plasma IL-6 receptor concentration of the mIgG1-administered group. This result shows that, as the mouse FcyRIIb-binding activity of an anti-human IL-6 receptor antibody that binds to the antigen in a pH-dependent manner is greater, the IL-6 receptor concentration can be reduced more in the plasma of mice administered with the antibody.

(6-3) Assessment of the antigen elimination effect in the plasma of a living organism administered with an antibody with selective enhancement of mouse FcγRIIb binding using Fc receptor γ chain-deficient mice

The effect to eliminate soluble IL-6 receptor from the plasma of Fc receptor γ chain-deficient mice administered with the anti-human IL-6 receptor antibody Fv4-mIgG1, Fv4-mIgG1-mF44, or Fv4-mIgG1mF46, was assessed by the same method as described in Example 5. The soluble human IL-6 receptor concentrations in the plasma of the mice were determined by the method described in Example (2-1-2). The result is shown in Fig. 15.

As with the case where mF44 and mF46 were administered to FcγRIII-deficient mice, the plasma IL-6 receptor concentration in Fc receptor γ chain-deficient mice administered with mF44 and mF46, which mimic the condition resulting from the selective increase in the mouse FcyRIIb-binding activity of mIgG1 (native mouse IgG1), was demonstrated to be markedly reduced as compared to the plasma IL-6 receptor concentration in Fc receptor γ chain-deficient mice administered with mIgG1. In particular, the plasma IL-6 receptor concentration in the mF44-administered group was reduced to about three times that in the mIgG1-administered group, and the accumulation of antigen concentration due to antibody administration was suppressed. Meanwhile, on day three after administration, the plasma IL-6 receptor concentration in the mF46-administered group was markedly reduced by about five times as compared to that in the group without antibody administration, and about 15 times as compared to that in the mIgG1-administered group.

The results described in Examples (6-2) and (6-3) show that the soluble antigen concentration in the plasma is markedly reduced in the group administered with an antibody that binds to a soluble antigen in a pH-dependent manner and has selectively increased mouse FcyRIIb-binding activity.

[Example 7] The antigen elimination effect of antibodies with selective enhancement of the binding to FcγRIII

(7-1) The antigen elimination effect in the plasma of a living organism administered with antibodies with selectively enhanced FcγRIII binding

FcγRIIb-deficient mice (FcgrFcγR2b (FcyRII) mouse, Taconic) (Nature (1996) 379 (6563), 346-349) express mouse FcγRI, mouse FcγRIII, and mouse FcγRIV, but not mouse FcγRIIb. As described in Example 5, it was demonstrated that mF44 and mF46 resulting from increasing the FcγR-binding activity of native mouse IgG1 show selectively enhanced binding to mouse FcγRIIb and mouse FcγRIII. It was conceived that, based on the use of the selectively increased binding activity of the antibodies, the condition of administration of an antibody with selectively enhanced binding to mouse FcγRIII can be mimicked by administering mF44 or mF46 to mouse FcγRIIb-deficient mice which do not express mouse FcγRIIb.

As described in Example 6, the soluble antigen concentration was reduced in the plasma of FcγRIII-deficient mice, which mimic the condition of administration of an antibody with selectively increased mouse FcγRIIb-binding activity. Meanwhile, whether the soluble antigen concentration is reduced in the plasma of FcγRIIb-deficient mice, which mimic the condition of administration of an antibody with selectively increased mouse FcγRIII-binding activity, was assessed by the test described below.

(7-2) Assessment of the antigen elimination effect by selective enhancement of mouse FcγRIII binding using FcγRIIb-deficient mice

The effect to eliminate soluble IL-6 receptor from the plasma of FcγRIIb-deficient mice administered with the anti-human IL-6 receptor antibody Fv4-mIgG1, Fv4-mIgG1-mF44, or Fv4-mIgG1mF46, was assessed by the same method as described in Example 5. The soluble human IL-6 receptor concentrations in plasma were determined by the method described in Example (2-1-2). The result is shown in Fig. 16.

Surprisingly, in the groups administered with mF44 and mF46, which mimic selective increase of the mouse FcγRIII-binding activity of mIgG1 (native mouse IgG1), the plasma IL-6 receptor concentration was reduced, but the reduction was not confirmed compared to that shown in Example 6.

Without being bound by a particular theory, based on the results described in Examples 5, 6, and 7, the following discussion is possible. The elimination of soluble IL-6 receptor from plasma was found to be markedly accelerated in normal mice expressing both mouse FcγRIIb and mouse FcγRIII that were administered with mF44 and mF46 with selectively increased binding activity of mIgG1 (native mouse IgG1) to mouse FcγRIIb and mouse FcγRIII. Furthermore, it was revealed that, when mF44 and mF46 were administered to mice that express mouse FcγRIIb but not mouse FcγRIII (*i.e.,* FcγRIII-deficient mice and Fc receptor γ chain-deficient mice), the elimination of soluble IL-6 receptor from plasma was also accelerated markedly in the mice. Meanwhile, when mF44 and mF46 were administered to mice that express mouse FcγRIII but not mouse FcγRIIb (*i.e.,* FcγRII-deficient mice), the elimination of soluble IL-6 receptor from plasma was not accelerated in the mice to the extent that they were administered in FcγRIII-deficient mice or Fc receptor γ chain-deficient mice.

From the above findings, it is thought that, the antibodies mF44 and mF46 in which the binding activity of mIgG1 (native mouse IgG1) to mouse FcγRIIb and mouse FcγRIII is increased, are incorporated into FcγR-expressing cells mainly by mouse FcγRIIb, and thus the soluble antigen in the plasma that binds to the antibodies is eliminated. Meanwhile, the FcγRIII-mediated incorporation of antibody/antigen complexes into FcγR-expressing cells is thought not to significantly contribute to the elimination of the soluble antigen from plasma.

Furthermore, as shown in Example 4, the plasma concentration of soluble IL-6 receptor was markedly reduced in mice administered with Fv4-IgG1-F1087 having increased binding activity to mouse FcγRIIb and mouse FcγRIII, in particular. Meanwhile, the effect to eliminate soluble IL-6 receptor from the plasma of mice administered with Fv4-IgG1-F1182 with increased binding activity to mouse FcγRI and mouse FcγRIV, in particular, was smaller than that of Fv4-IgG1-F1087.

Furthermore, as shown in Example 2, in mice administered with Fv4-IgG1-Fuc whose mouse FcγRIV-binding activity has been considerably increased by having sugar chains with low fucose content (Science (2005) 310 (5753) 1510-1512), the plasma concentration of soluble IL-6 receptor was reduced as compared to that in mice administered with Fv4-IgG1; however, the reduction effect was as small as about twice. Thus, mouse FcγRIV-mediated incorporation of antibodies into FcγR-expressing cells is thought not to significantly contribute to the elimination of soluble antigens from plasma.

In view of the above, it was demonstrate that, of several mouse FcγRs, mouse FcγRIIb and mouse FcγIII, in particular mouse FcγRIIb, plays a major role in antibody incorporation into FcγR-expressing cells in mice. Thus, it would be thought that mutations to be introduced into the mouse FcγR-binding domain preferably include, but are not limited to, mutations that augment the binding to mouse FcγRIIb and mouse FcγIII, in particular, the binding to mouse FcγRIIb.

The above findings demonstrate that, in mice, the binding activity to mouse FcγRIIb and mouse FcγIII, in particular, the FcγRIIb-binding activity of the antibodies to be administered is more preferably increased to accelerate the elimination of soluble antigens from the plasma of a living organism by administering to it antigen-binding molecules that bind to soluble antigens in a pH-dependent manner and have increased FcγR binding activity. Specifically, when administered to a living organism, antigen-binding molecules that bind to soluble antigens in a pH-dependent manner and have increased binding activity to mouse FcγRIIb and mouse FcγIII, in particular increased FcγRIIb-binding activity, can accelerate the elimination of the soluble antigens from plasma and effectively reduce the plasma concentration of soluble antigens, and thus, the antigen-binding molecules were revealed to show a very effective action.

[Example 8] Assessment of the platelet aggregatory ability of antibodies containing an Fc region introduced with an existing alteration that enhances the FcγRIIb binding

<u>(8-1) Preparation of antibodies containing an Fc region introduced with an existing alteration that enhances the FcγRIIb binding</u>

As described in Reference Example 7, antigens can be efficiently eliminated from the plasma of the living organism by administering antibodies with selectively increased FcγRIIb-binding activity to the living organism. Furthermore, the administration of antibodies containing an Fc region with selectively increased FcγRIIb-binding activity is thought to be preferred from the viewpoint of safety and side effects in the living organism administered with such antibodies.

However, the mouse FcγRIIb binding and mouse FcγRIII binding are both enhanced in mF44 and mF46, and thus the binding enhancement is not selective for mouse FcγRIIb. Since the homology between mouse FcγRIIb and mouse FcγRIII is high, it would be difficult to find an alteration that enhances the mouse FcγRIIb-selective binding while distinguishing the two. Moreover, there is no previous report on Fc regions with selectively enhanced mouse FcγRIIb binding. Also, the homology between human FcγRIIb and human FcγRIIa (the two allotypes, 131Arg and 131His) is also known to be high. Moreover, there is no report on Fc regions that contain an alteration that augments the human FcγRIIb-selective binding while distinguising the two (Seung et al., (Mol. Immunol. (2008) 45, 3926-3933); Greenwood et al., (Eur. J. Immunol. (1993) 23 (5), 1098-1104)). Furthermore, the FcγRIIa binding has been reported to play an important role in the platelet aggregatory activity of an antibody (Meyer et al., (J. Thromb. Haemost. (2009), 7 (1), 171-181); Robles-Carrillo et al., (J. Immunol. (2010), 185 (3), 1577-1583)). In view of these reports, it is possible that an antibody with augmented FcγRIIa binding can increase the risk of developing thrombosis in a living organism administered with it. Thus, whether antibodies with augmented FcγRIIa binding have an increased platelet aggregatory activity was assessed as follows.

<u>(8-2) Assessment of the human FcγR-binding activity of antibodies containing an Fc region introduced with an existing alteration that enhances the FcγRIIb binding</u>

Antibodies containing an Fc region introduced with an existing alteration that enhances the human FcγRIIb binding were analyzed for their affinity for human FcγRIa, R-type and H-type FcγRIIa, FcγRIIb, and FcγRIIIa by the following procedure. An H chain was constructed to have, as the antibody H chain variable region, the antibody variable region IL6R-H (SEQ ID NO: 132) against human interleukin 6 receptor which is disclosed in WO2009/125825, and as the antibody H chain constant region, IL6R-G1d (SEQ ID NO: 133) that has G1d resulting from removing the C-terminal Gly and Lys from human IgG1. Then, IL6R-G1d-v3 (SEQ ID NO: 138) was constructed by altering the Fc region of IL6R-G1d by the substitution of Glu for Ser at position 267 (EU numbering) and Phe for Leu at position 328 (EU numbering), as described in Seung et al., (Mol. Immunol. (2008) 45, 3926-3933). IL6R-L (SEQ ID NO: 135) which is the L chain of anti-human interleukin 6 receptor antibody was used as a common antibody L chain, and expressed in combination with respective H chains according to the method described in Reference Example 1, and the resulting antibodies were purified. Hereinafter, antibodies containing IL6R-G1d and IL6R-G1d-v3 as the heavy chain are referred to as IgG1 and IgG1-v3, respectively.

Then, the interaction between FcyR and the above antibodies was kinetically analyzed using Biacore T100 (GE Healthcare). The assay for the interaction was carried out at 25°C using HBS-EP+ (GE Healthcare) as a running buffer. The chip used was a Series S Sencor Chip CM5 (GE Healthcare) immobilized with Protein A by an amino coupling method. Each FcγR diluted with the running buffer was allowed to interact with the antibodies of interest captured onto the chip to measure the binding of the antibodies to each FcγR. After measurement, 10 mM glycine-HCl (pH 1.5) was reacted to the chip to wash off the captured antibodies to repeatedly use the regenerated chip. A sensorgram obtained as a result of the measurement was analyzed using 1:1 Langmuir binding model with Biacore Evaluation Software, and binding rate constant ka (L/mol/s) and dissociation rate constant kd (1/s) were calculated, and the dissociation constant KD (mol/l) was calculated from these values. The KD values of IgG1 and IgG1-v3 to each FcγR are shown in Table 12 (the KD values of each antibody to each FcyR), while the relative KD values of IgG1-v3, which are obtained by dividing KD of IgG1 to each FcγR by KD of IgG1-v3 to each FcγR, are shown in Table 13.

[Table 12]

| ANTIBODY | KD (M) | | | | |
|---|---|---|---|---|---|
| | Fc γ RIa | Fc γ RIIaR | Fc γ RIIaH | Fc γ RIIb | Fc γ RIIIa |
| IgG1 | 3.4E-10 | 1.2E-06 | 7.7E-07 | 5.3E-06 | 3.1E-06 |
| IgG1-v3 | 1.9E-10 | 2.3E-09 | 1.5E-06 | 1.3E-08 | 8.8E-06 |

[Table 13]

| | Fc γ RIa | Fc γ RIIaR | Fc γ RIIaH | Fc γ RIIb | Fc γ RIIIa |
|---|---|---|---|---|---|
| KD VALUE RATIO | 1.8 | 522 | 0.51 | 408 | 0.35 |

<u>(8-3) Assessment of the ability to aggregate platelets</u>

Next, whether the increased/reduced FcγRIIa affinity of the antibody containing the Fc region with the substitution of Glu for Ser at position 267 and Phe for Leu at position 328 (EU numbering) in the Fc region of IgG1 changes the platelet aggregatory ability, was assessed using platelets derived from donors with H-type or R-type FcγRIIa. The antibody comprising as the light chain omalizumab_VL-CK (SEQ ID NO: 137) and omalizumab_VH-G1d (SEQ ID NO: 136) that contains the heavy chain variable region of hIgG1 antibody (human IgG1 constant region) that binds to IgE and the G1d heavy chain constant region, was constructed using the method described in Reference Example 2. Furthermore, omalizumab_VH-G1d-v3 (SEQ ID NO: 138) was constructed by substituting Glu for Ser at position 267 and Phe for Leu at position 328 (EU numbering) in omalizumab_VH-G1d. Omalizumab-G1d-v3, which contains omalizumab_VH-G1d-v3 as the heavy chain and omalizumab_VL-CK as the light chain, was prepared using the method described in Reference Example 2. This antibody was assessed for the platelet aggregatory ability.

Platelet aggregation was assayed using the platelet aggregometer HEMA TRACER 712 (LMS Co.). First, about 50 ml of whole blood was collected at a fixed amount into 4.5-ml evacuated blood collection tubes containing 0.5 ml of 3.8% sodium citrate, and this was centrifuged at 200 g for 15 minutes. The resultant supernatant was collected and used as platelet-rich plasma (PRP). After PRP was washed with buffer A (137 mM NaCl, 2.7 mM KCl, 12 mM $NaHCO_3$, 0.42 mM $NaH_2PO_4$, 2 mM $MgCl_2$, 5 mM HEPES, 5.55 mM dextrose, 1.5 U/ml apyrase, 0.35 % BSA), the buffer was replaced with buffer B (137 mM NaCl, 2.7 mM KCl, 12 mM $NaHCO_3$, 0.42 mM $NaH_2PO_4$, 2 mM $MgCl_2$, 5 mM HEPES, 5.55 mM dextrose, 2 mM $CaCl_2$, 0.35 % BSA). This yielded washed platelets at a density of about 300,000/μl. 156 μl of the washed platelets was aliquoted into assay cuvettes containing a stir bar in the platelet aggregometer. The platelets were stirred at 1000 rpm with the stir bar in the cuvettes maintained at 37.0°C in the platelet aggregometer. 44 μl of the immune complex of omalizumab-G1d-v3 and IgE at a molar ratio of 1:1, prepared at final concentrations of 600 μg/ml and 686 μg/ml, respectively, was added to the cuvettes. The platelets were reacted with the immune complex for five minutes. Then, at a concentration that does not allow secondary platelet aggregation, adenosine diphosphate (ADP, SIGMA) was added to the reaction mixture to test whether the aggregation is enhanced.

The result of platelet aggregation for each donor with an FcγRIIa polymorphic form (R/H or H/H) obtained from the above assay is shown in Figs. 17 and 18, respectively. From the result in Fig. 17, platelet aggregation is observed when the immune complex is added to the platelets of a donor with the FcγRIIa polymorphic form (R/H). Meanwhile, as shown in Fig. 18, platelet aggregation was not observed when the immune complex is added to the platelets of a donor with the FcγRIIa polymorphic form (H/H).

Next, platelet activation was assessed using activation markers. Platelet activation can be measured based on the increased expression of an activation marker such as CD62p (p-selectin) or active integrin on the platelet membrane surface. 2.3 μl of the immune complex was added to 7.7 μl of the washed platelets prepared by the method described above. After five minutes of reaction at room temperature, activation was induced by adding ADP at a final concentration of 30 μM, and whether the immune complex enhances the ADP-dependent activation was assessed. A sample added with phosphate buffer (pH 7.4) (Gibco), instead of the immune complex, was used as a negative control. Staining was performed by adding, to each post-reaction sample, PE-labeled anti-CD62 antibody (BECTON DICKINSON), PerCP-labeled anti-CD61 antibody, and FITC-labeled PAC-1 antibody (BD bioscience). Fluorescence intensity for each stain was measured using a flow cytometer (FACS CantoII, BD bioscience).

The result on CD62p expression, obtained by the above assay method, is shown in Fig. 19. The result on the activated integrin expression is shown in Fig. 20. The washed platelets used were obtained from a healthy person with the FcγRIIa polymorphic form R/H. The expression amount of both CD62p and active integrin expressed on platelet membrane surface, which is induced by ADP stimulation, was enhanced in the presence of the immune complex.

The above results demonstrate that the antibody having the Fc region introduced with an existing alteration that enhances the human FcγRIIb binding, which is the substitution of Glu for Ser at position 267 and Phe for Leu at position 328 (EU numbering) in the Fc region of IgG1, promotes the aggregation of platelets expressing FcγRIIa with the FcγRIIa allotype in which the amino acid at position 131 is R, as compared to platelets expressing FcγRIIa with the FcγRIIa polymorphic form in which the amino acid at position 131 is H. That is, it was suggested that the risk of developing thrombosis due to platelet aggregation can be increased when an antibody containing an Fc region introduced with an existing alteration that enhances the binding to existing human FcγRIIb is administered to humans having R-type FcγRIIa in at least one allele. It was shown that the antigen-binding molecules containing an Fc region of the present invention that enhances the FcγRIIb binding more selectively not only improves the antigen retention in plasma, but also possibly solves the above problems. Thus, the usefulness of the antigen-binding molecules of the present invention is obvious.

(8-4) Comparison of the platelet aggregatory ability between an antibody (BP230) comprising Fc with selectively augmented FcγRIIb binding and an antibody comprising an existing Fc with selectively augmented FcγRIIb binding

(8-4-1) Preparation of antibodies with selectively augmented FcγRIIb binding

As described in Example (8-3), it was shown that antibodies with augmented FcγRIIa binding have increased platelet aggregatory ability and increased platelet activation ability and, they can increase the risk of developing thrombosis when administered to humans. Meanwhile, considering that of the FcγRs, FcγRIIa alone is expressed on platelets, it is thought that antibodies with selectively augmented FcγRIIb binding do not show increased platelet aggregatory ability or activation ability, or do not increase the risk of developing thrombosis when administered to humans. To confirm this, antibodies with selectively augmented FcyRIIb binding were actually tested for their platelet aggregatory ability and platelet activation ability.

Specifically, omalizumab_VH-G1d (SEQ ID NO: 136) and omalizumab_VL-CK (SEQ ID NO: 137), as the heavy chain and light chain of the hIgG1 antibody (human IgG1 constant region) that binds to IgE, respectively, were produced by the method described in Reference Example 2. Then, to selectively increase the human FcγRIIb-binding activity of omalizumab_VH-G1d, omalizumab_VH-BP230 (SEQ ID NO: 140) was produced by substituting Asp for Glu at position 233, Asp for Gly at position 237, Asp for Pro at position 238, Asp for His at position 268, Gly for Pro at position 271, Asp for Tyr at position 296, Arg for Ala at position 330, and Glu for Lys at position 439 (EU numbering). Likewise, to increase the human FcγRIIb- and FcγRIIa R-binding activity of omalizumab_VH-G1d, omalizumab_VH-G1d-v3 (SEQ ID NO: 138) was produced by substituting Glu for Ser at position 267 and Phe for Leu at position 328 (EU numbering).

Omalizumab-BP230 that comprises omalizumab_VH-BP230 (SEQ ID NO: 140) as the heavy chain and omalizumab_VL-CK (SEQ ID NO: 137) as the light chain, and omalizumab-G1d-v3 that comprises omalizumab_VH-G1d-v3 (SEQ ID NO: 138) as the heavy chain and omalizumab_VL-CK (SEQ ID NO: 137) as the light chain, were produced using the method described in Reference Example 2. These antibodies were assessed for their ability to aggregate and activate platelets.

(8-4-2) Assessment of omalizumab-BP230 and omalizumab-G1d-v3 for their human FcγR-binding activity

The analysis results on the affinity between each human FcγR and the Fc region of omalizumab-G1d-v3 resulting from augmenting the human FcγRIIb binding of omalizumab, which is a known antibody, are shown in Example (8-2). The affinity of the Fc region of omalizumab-BP230 for each human FcγR was also analyzed in the same manner,and the results are shown in Table 22.

(8-4-3) Assessment of the ability to aggregate platelets

Then, omalizumab-BP230 and omalizumab-G1d-v3 produced as described in Example (8-4-1) were assayed for their binding using platelets from a donor with the FcγRIIa polymorphic form (R/H). The assay result was used to assess whether the platelet aggregatory ability is altered depending on the level of the affinity for FcγRIIa.

Platelet aggregation assay was carried out using the platelet aggregometer HEMA TRACER 712 (LMS Co.). First, about 50 ml of whole blood was prepared by collecting fixed amounts of blood into 4.5-ml evacuated blood collection tubes containing 0.5 ml of 3.8% sodium citrate, and centrifuged at 200 g for 15 minutes. The resultant supernatant was collected and used as platelet-rich plasma (PRP). After washing PRP with buffer A (137 mM NaCl, 2.7 mM KCl, 12 mM $NaHCO_3$, 0.42 mM $NaH_2PO_4$, 2 mM $MgCl_2$, 5 mM HEPES, 5.55 mM dextrose, 1.5 U/mL apyrase, 0.35 % BSA), the buffer was changed with buffer B (137 mM NaCl, 2.7 mM KCl, 12 mM $NaHCO_3$, 0.42 mM $NaH_2PO_4$, 2 mM $MgCl_2$, 5 mM HEPES, 5.55 mM dextrose, 2 mM $CaCl_2$, 0.35 % BSA). This yielded washed platelets at a density of about 300,000/μl. 150.2 μl of washed platelets was aliquoted to assay cuvettes containing a stir bar in the platelet aggregometer. The platelets were stirred at 1000 rpm with the stir bars in the cuvettes maintained at 37.0°C in the platelet aggregometer. 24.3 μl of omalizumab-G1d-v3 or omalizumab-BP230 prepared at a final concentration of 600 μg/ml was added to the cuvettes. After one minute of reaction, 25.4 μl of IgE, which was prepared so that its molar ratio to the antibody is 1:1, was added thereto. The mixtures were incubated for five minutes. Then, at a concentration that does not induce secondary platelet aggregation, adenosine diphosphate (ADP, SIGMA) was added to the reaction mixture to test whether the aggregation is augmentated. The washed platelets used were obtained from one healthy person with the FcγRIIa polymorphic form R/H.

The result obtained by the above-described assay method is shown in Fig. 21. The result shown in Fig. 21 revealed that platelets were strongly aggregated when adding the immune complex containing omalizumab-G1d-v3. On the other hand, there was no difference in platelet aggregation between when PBS was added and when the immune complex containing omalizumab-BP230 was added.

Next, the platelet activation by omalizumab-G1d-v3 and omalizumab-BP230 was assessed. Platelet activation can be measured based on the increased expression of an activation marker such as CD62p (p-selectin) and active integrin, on the platelet membrane surface. 1.22 μl of omalizumab-G1d-v3 or omalizumab-BP230 prepared at a final concentration of 600 μg/ml was added to 7.51 μl of washed platelets prepared by the method described above. After five minutes of reaction at room temperature, 1.27 μl of IgE, which was prepared so that its molar ratio to the antibody is 1:1, was added thereto. Then, after five minutes of reaction at room temperature, whether platelet activation is induced was assessed. The negative control used was a sample added with phosphate buffer (pH 7.4, Gibco), instead of immune complexes. After reaction, each sample was fluorescently stained using PE-labeled anti-CD62 antibody (BECTON DICKINSON), PerCP-labeled anti-CD61 antibody, and FITC-labeled PAC-1 antibody (BD bioscience), and the fluorescence intensity was determined using a flow cytometer (FACS CantoII, BD bioscience). The washed platelets used were obtained from one healthy person with the FcγRIIa polymorphic form R/H.

The results obtained by the above-described assay method are shown in Figs. 22 and 23. The expression of both CD62p and active integrin on the platelet membrane surface was increased by addition of the immune complex containing omalizumab-G1d-v3. Meanwhile, the expression of the molecules on the platelet membrane surface by addition of the immune complex with omalizumab-BP230 was comparable to the expression on the platelet membrane surface by addition of the phosphate buffer.

The above results demonstrate that the antibody that comprising the existing modified Fc region with substitutions of Glu for Ser at position 267 and Phe for Leu at position 328 (EU numbering) in the Fc region of IgG1, and which has augmented binding to both human FcyRIIb and human FcγRIIa R, promote the aggregation and activation of platelets in which the amino acid of position 131 is Arg in at least either of the alleles for the polymorphic FcγRIIa gene, as compared to the antibody that comprises an Fc region with substitutions of Asp for Glu at position 233, Asp for Gly at position 237, Asp for Pro at position 238, Asp for His at position 268, Gly for Pro at position 271, Asp for Tyr at position 296, Arg for Ala at position 330, and Glu for Lys at position 439 (EU numbering), and which has selectively augmented binding to human FcγRIIb. Specifically, the antibody that comprises the existing Fc region variant with augmented human FcγRIIb binding was suggested to have a problem that it can increase the risk of developing thrombosis due to platelet aggregation in humans with the FcγRIIa R allele; on the other hand, the Fc region variant with selectively augmented FcγRIIb binding was demonstrated to overcome this problem.

[Example 9] Comprehensive analysis of FcγRIIb binding of variants introduced with an alteration at the hinge portion in addition to the P238D alteration

In an Fc produced by substituting Pro at position 238 (EU numbering) with Asp in a naturally-occurring human IgG1, an anticipated combinatorial effect could not be obtained even by combining it with another alteration predicted to further increase FcγRIIb binding from the analysis of naturally-occurring antibodies. Therefore, in order to find variants that further enhance FcγRIIb binding, alterations were comprehensively introduced into the altered Fc produced by substituting Pro at position 238 (EU numbering) with Asp. IL6R-F11 (SEQ ID NO: 141) was produced by introducing an alteration of substituting Met at position 252 (EU numbering) with Tyr and an alteration of substituting Asn at position 434 (EU numbering) with Tyr in IL6R-G1d (SEQ ID NO: 133) which was used as the antibody H chain. Furthermore, IL6R-F652 (SEQ ID NO: 142) was prepared by introducing an alteration of substituting Pro at position 238 (EU numbering) with Asp into IL6R-F11. Expression plasmids containing an antibody H chain sequence were prepared for each of the antibody H chain sequences produced by substituting the region near the residue at position 238 (EU numbering) (positions 234 to 237, and 239 (EU numbering)) in L6R-F652 each with 18 amino acids excluding the original amino acids and Cysteine. IL6R-L (SEQ ID NO: 135) was utilized as an antibody L chain. These variants were expressed and purified by the method of Reference Example 2. These Fc variants are called PD variants. Interactions of each PD variant with FcγRIIa type R and FcγRIIb were comprehensively evaluated by the method of Reference Example 25.

A figure that shows the results of analyzing the interaction with the respective FcyRs was produced according to the following method. The value obtained by dividing the value for the amount of binding of each PD variant to each FcγR by the value for the amount of FcγR binding of the pre-altered antibody which is used as the control (IL6R-F652/IL6R-L, which has an alteration of substituting Pro at position 238 (EU numbering) with Asp and then multiplying the result by 100, was shown as the relative binding activity value of each PD variant to each FcγR. The horizontal axis shows relative values of the FcγRIIb-binding activity of each PD variant, and the vertical axis shows relative values of the FcγRIIa type R-binding activity values of each PD variant (Fig. 24).

As a result, it was found that the FcγRIIb binding of eleven types of alterations were enhanced compared with the antibody before introducing alterations, and they have the effects of maintaining or enhancing FcγRIIa type R-binding. The activities of these eleven variants to bind FcγRIIb and FcγRIIa R are summarized in Table 14. In the table, the sequence ID numbers refer to those of the H chains of the variants, and alteration refers to the alteration introduced into IL6R-F11 (SEQ ID NO: 141).

[Table 14]

| VARIANT NAME | ALTERATION | RELATIVE BINDING ACTIVITY TO FcγRIIb | RELATIVE BINDING ACTIVITY TO FcγRIIaR |
|---|---|---|---|
| IL6R-F652/IL6R-L | P238D | 100 | 100 |
| IL6R-PD042/IL6R-L | P238D/L234W | 106 | 240 |
| IL6R-PD043/IL6R-L | P238D/L234Y | 112 | 175 |
| IL6R-PD079/IL6R-L | P238D/G237A | 101 | 138 |
| IL6R-PD080/IL6R-L | P238D/G237D | 127 | 222 |
| IL6R-PD081/IL6R-L | P238D/G237E | 101 | 117 |
| IL6R-PD082/IL6R-L | P238D/G237F | 108 | 380 |
| IL6R-PD086/IL6R-L | P238D/G237L | 112 | 268 |
| IL6R-PD087/IL6R-L | P238D/G237M | 109 | 196 |

(continued)

| VARIANT NAME | ALTERATION | RELATIVE BINDING ACTIVITY TO FcγRIIb | RELATIVE BINDING ACTIVITY TO FcγRIIaR |
|---|---|---|---|
| IL6R-PD094/IL6R-L | P238D/G237W | 122 | 593 |
| IL6R-PD095/IL6R-L | P238D/G237Y | 124 | 543 |
| IL6R-PD097/IL6R-L | P238D/S239D | 139 | 844 |

Fig. 25 shows relative values for the FcγRIIb-binding activity obtained by additionally introducing the above eleven alterations into a variant carrying the P238D alteration, and relative values for the FcγRIIb-binding activity of a variant obtained by introducing the alterations into an Fc that does not contain the P238D. These eleven alterations enhanced the amount of FcγRIIb binding compared with before introduction when they were further introduced into the P238D variant. On the contrary, the effect of lowering FcγRIIb binding was observed for eight of those alterations except G237F, G237W, and S239D, when they were introduced into the variant that does not contain P238D (data not shown).

These results showed that, based on the effects of introducing alterations into a naturally-occurring IgG1, it is difficult to predict the effects of combining and introducing the same alterations into the variant containing the P238D alteration. In other words, it would not have been possible to discover these eight alterations identified this time without this investigation that introduces the same alterations are combined and introduced into the variant containing the P238D alteration.

The results of measuring KD values of the variants indicated in Table 14 for FcγRIa, FcγRIIaR, FcγRIIaH, FcγRIIb, and FcγRIIIaV by the method of Reference Example 25 are summarized in Table 15. In the table, alteration refers to the alteration introduced into IL6R-F11 (SEQ ID NO: 141). The template used for producing IL6R-F11, IL6R-G1d/IL6R-L, is indicated with an asterisk (*). Furthermore, KD (IIaR)/KD (IIb) and KD (IIaH)/KD (IIb) in the table respectively show the value obtained by dividing the KD value of each variant for FcγRIIaR by the KD value of each variant for FcγRIIb, and the value obtained by dividing the KD value of each variant for FcγRIIaH by the KD value of each variant for FcγRIIb. KD (IIb) of the parent polypeptide / KD (IIb) of the variant refers to a value obtained by dividing the KD value of the parent polypeptide for FcγRIIb by the KD value of each variant for FcγRIIb.

In addition, Table 15 shows KD values for the stronger of the FcγRIIaR- and FcγRIIaH-binding activities of each variant / KD values for the stronger of the FcγRIIaR- and FcγRIIaH-binding activities of the parent polypeptide. Here, parent polypeptide refers to a variant which has IL6R-F11 (SEQ ID NO: 141) as the H chain. It was determined that due to weak binding of FcγR to IgG, it was impossible to accurately analyze by kinetic analysis, and thus the gray-filled cells in Table 15 show values calculated by using Equation 2 of Reference Example 25.

[Equation 2]

$$KD = C \bullet R_{max} / (R_{eq} - RI) - C$$

Table 15 shows that all variants improved their affinity for FcγRIIb in comparison with IL6R-F11, and the range of improvement was 1.9 fold to 5.0 fold. The ratio of KD value of each variant for FcγRIIaR / KD value of each variant for FcγRIIb, and the ratio of KD value of each variant for FcγRIIaH / KD value of each variant for FcγRIIb represent an FcγRIIb-binding activity relative to the FcγRIIaR-binding activity and FcγRIIaH-binding activity, respectively. That is, these values show the degree of binding selectivity of each variant for FcγRIIb, and a larger value indicates a higher binding selectivity for FcγRIIb. For the parent polypeptide IL6R-F11/IL6R-L, the ratio of KD value for FcγRIIaR / KD value for FcγRIIb and the ratio of KD value for FcγRIIaH / KD value for FcγRIIb are both 0.7, and accordingly all variants in Table 15 showed improvement of binding selectivity for FcγRIIb in comparison with the parent polypeptide. When the KD value for the stronger of the FcγRIIaR- and FcγRIIaH-binding activities of a variant / KD value for the stronger of the FcγRIIaR- and FcγRIIaH-binding activities of the parent polypeptide is 1 or more, this means that the stronger of the FcγRIIaR-and FcγRIIaH-binding activities of a variant has equivalent or reduced binding compared with the binding by the stronger of the FcγRIIaR- and FcγRIIaH-binding activities of the parent polypeptide. Since this value was 0.7 to 5.0 for the variants obtained this time, one may say that binding by the stronger of the FcγRIIaR- and FcγRIIaH-binding activities of the variants obtained this time was nearly the same or decreased in comparison with the parent polypeptide. These results showed that compared with the parent polypeptide, the variants obtained this time have maintained or decreased binding activities to FcγRIIa type R and type H and enhanced binding activity to FcγRIIb, and thus have improved selectivity for FcγRIIb. Furthermore, compared with IL6R-F11, all variants had lower affinity to FcγRIa and FcγRIIIaV.

[Table 15]

| ALTERATION | KD [mol/L] | | | | | KD(IIaR)/KD(IIb) | KD(IIaH)/KD(IIb) | KD (IIb) OF PARENT POLYPEPTIDE/ KD (IIb) OF ALTERED POLYPEPTIDE | KD VALUE FOR THE STRONGER OF THE BINDING ACTIVITIES OF A VARIANT TO FcγRIIaR AND FcγRIIaH/ KD VALUE FOR THE STRONGER OF THE BINDING ACTIVITIES OF THE PARENT POLYPEPTIDE TO FcγRIIaR AND FcγRIIaH |
|---|---|---|---|---|---|---|---|---|---|
| | FcγRIa | FcγRIIaR | FcγRIIaH | FcγRIIb | FcγRIIIaV | | | | |
| * | 3.2E-10 | 1.0E-06 | 6.7E-07 | 2.6E-06 | 3.5E-07 | 0.4 | 0.3 | 2.6 | 0.1 |
| | 9.0E-10 | 5.0E-06 | 5.0E-06 | 6.8E-06 | 1.5E-06 | 0.7 | 0.7 | 1.0 | 1.0 |
| L234W/P238D | 6.3E-08 | 1.6E-05 | 1.9E-05 | 2.0E-06 | 3.7E-05 | 8.1 | 9.5 | 3.4 | 3.2 |
| L234Y/P238D | 7.5E-08 | 2.6E-05 | 2.3E-05 | 1.6E-06 | 4.5E-05 | 15.9 | 14.4 | 4.2 | 4.6 |
| G237A/P238D | 1.4E-07 | 3.2E-05 | 2.1E-05 | 3.0E-06 | 3.7E-05 | 10.5 | 7.0 | 2.3 | 4.2 |
| G237D/P238D | 1.4E-07 | 2.1E-05 | 2.5E-05 | 2.0E-06 | 4.3E-05 | 10.7 | 12.8 | 3.5 | 4.2 |
| G237E/P238D | 3.4E-07 | 3.8E-05 | 2.5E-05 | 3.6E-06 | 4.1E-05 | 10.6 | 7.0 | 1.9 | 5.0 |
| G237F/P238D | 5.2E-08 | 1.4E-05 | 1.6E-05 | 3.4E-06 | 4.3E-05 | 4.1 | 4.7 | 2.0 | 2.8 |
| G237L/P238D | 1.2E-07 | 1.8E-05 | 1.8E-05 | 2.6E-06 | 4.1E-05 | 6.9 | 7.1 | 2.7 | 3.5 |
| G237M/P238D | 5.2E-08 | 2.2E-05 | 2.0E-05 | 2.9E-06 | 3.7E-05 | 7.7 | 7.0 | 2.4 | 4.0 |
| G237W/P238D | 3.6E-08 | 7.2E-06 | 1.2E-05 | 2.3E-06 | 3.8E-05 | 3.1 | 5.2 | 2.9 | 1.4 |
| G237Y/P238D | 9.3E-08 | 7.9E-06 | 1.5E-05 | 2.3E-06 | 4.2E-05 | 3.4 | 6.4 | 2.9 | 1.6 |
| P238D/S239D | 4.9E-09 | 3.5E-06 | 1.9E-05 | 1.4E-06 | 1.7E-05 | 2.6 | 14.0 | 5.0 | 0.7 |

[Example 10] X-ray crystal structure analysis of a complex formed between an Fc containing P238D and an extracellular region of FcγRIIb

As indicated earlier in Example 9, even though an alteration that is predicted from the analysis of naturally-occurring IgG1 antibodies to improve FcγRIIb-binding activity or selectivity for FcγRIIb is introduced into an Fc containing P238D, the FcγRIIb-binding activity was found to decrease, and the reason for this may be that the structure at the interacting interface between Fc and FcγRIIb is changed due to introduction of P238D. Therefore, to pursue the reason for this phenomena, the three-dimensional structure of the complex formed between an IgG1 Fc containing the P238D mutation (hereinafter, referred to as Fc (P238D)) and the extracellular region of FcγRIIb was elucidated by X-ray crystal structure analysis, and this was compared to the three-dimensional structure of the complex formed between the Fc of a naturally-occurring IgG1 (hereinafter, referred to as Fc (WT)) and the extracellular region of FcγRIIb, and the binding modes were compared. Multiple reports have been made on the three-dimensional structure of a complex formed between an Fc and an FcγR extracellular region; and the three-dimensional structures of the Fc (WT) / FcγRIIIb extracellular region complex (Nature (2000) 400, 267-273; J. Biol. Chem. (2011) 276, 16469-16477), the Fc (WT) / FcγRIIIa extracellular region complex (Proc. Natl. Acad. Sci. USA (2011) 108, 12669-126674), and the Fc (WT)/FcγRIIa extracellular region complex (J. Immunol. (2011) 187, 3208-3217) have been analyzed. While the three-dimensional structure of the Fc (WT) / FcγRIIb extracellular region complex has not been analyzed, the three-dimensional structure of a complex formed with Fc (WT) is known for FcγRIIa and FcγRIIb, and their extracellular regions match 93% in amino acid sequence and have very high homology. Thus, the three-dimensional structure of the Fc (WT) / FcγRIIb extracellular region complex was predicted by modeling using the crystal structure of the Fc (WT) / FcγRIIa extracellular region complex.

The three-dimensional structure of the Fc (P238D) / FcγRIIb extracellular region complex was determined by X-ray crystal structure analysis at 2.6 Å resolution. The structure obtained as a result of this analysis is shown in Fig. 26. The FcγRIIb extracellular region is bound between two Fc CH2 domains, and this was similar to the three-dimensional structures of complexes formed between Fc (WT) and the respective extracellular region of FcγRIIIa, FcγRIIIb, or FcγRIIa analyzed so far. Next, for detailed comparison, the crystal structure of the Fc (P238D) / FcγRIIb extracellular region complex and the model structure of the Fc (WT) / FcγRIIb extracellular region complex were superimposed by the least squares fitting based on the Cα atom pair distances with respect to the FcγRIIb extracellular region and the Fc CH2 domain A (Fig. 27). In that case, the degree of overlap between Fc CH2 domains B was not satisfactory, and conformational differences were found in this portion. Furthermore, using the crystal structure of the Fc (P238D) / FcγRIIb extracellular region complex and the model structure of the Fc (WT) / FcγRIIb extracellular region complex, pairs of atoms that have a distance of 3.7 Å or less between the extracted FcγRIIb extracellular region and Fc CH2 domain B were extracted and compared in order to compare the interatomic interaction between FcγRIIb and Fc (WT) CH2 domain B with the interatomic interaction between FcγRIIb and Fc (P238D). As shown in Table 16, the interatomic interactions between Fc CH2 domain B and FcγRIIb in Fc (P238D) and Fc (WT) did not match.

[Table 16]

| FcγRIIb **ATOM** | **Fc (P238D) CH2 DOMAIN B INTERACTION PARTNER (DISTANCE BETWEEN ATOMS, Å)** | Fc (WT) CH2 **DOMAIN B INTERACTION PARTNER (DISTANCE BETWEEN ATOMS, Å)** |
|---|---|---|
| Val 116 CG2 | | Asp 265 OD2 (3.47)<br>Gly 237 O (3.65) |
| Ser 126 OG | Ser 298 N (3.31)<br>Ser 298 CB (3.32)<br>Tyr 296 O (3.05) | |
| Lys 128 CA | Ser 298 OG (3.50) | |
| Phe 129 CB | Ser 298 O (3.36) | |
| Phe 129 CD2 | | Asn 297 CB (3.50)<br>Asn 297 CG (3.43) |
| Lys 128 C | Ser 298 OG (3.47) | |
| Phe 129 N | Ser 298 OG (3.30) | |
| Phe 129 O | Ser 267 OG (3.54) | |
| Arg 131 CB | Val | Val 266 O (3.02) |

(continued)

| FcγRIIb ATOM | Fc (P238D) CH2 DOMAIN B INTERACTION PARTNER (DISTANCE BETWEEN ATOMS, Å) | Fc (WT) CH2 DOMAIN B INTERACTION PARTNER (DISTANCE BETWEEN ATOMS, Å) |
|---|---|---|
| Arg 131 CG | | Val 266 O (3.22) |
| Arg 131 CD | | Val 266 CG1 (3.45)<br>Val 266 C (3.55)<br>Val 266 O (3.10) |
| Arg 131 NE | Ala 327 O (3.60) | Val 266 C (3.66)<br>Val 266 O (3.01)<br>Val 266 N (3.49) |
| Arg 131 CZ | Asp 270 CG (3.64)<br>Asp 270 OD2 (3.22)<br>Asp 270 OD1 (3.27)<br>Ala 327 CB (3.63) | Val 266 N (3.13) |
| Arg 131 NH1 | Asp 270 CG (3.19)<br>Asp 270 OD2 (2.83)<br>Asp 270 OD1 (2.99)<br>Ser 267 CB (3.56) | Val 266 CG1 (3.47)<br>Val 266 N (3.43)<br>Thr 299 OG1 (3.66)<br>Ser 298 O (3.11) |
| Arg 131 NH2 | Asp 270 CG (3.20)<br>Asp 270 OD2 (2.80)<br>Asp 270 OD1 (2.87)<br>Ala 327 CB (3.66) | Asp 265 CA (3.16)<br>Val 266 N (3.37) |
| Tyr 157 CE1 | | Leu 234 CG (3.64)<br>Leu 234 CD1 (3.61) |
| Tyr 157 OH | | Gly 236 O (3.62)<br>Leu 234 CA (3.48)<br>Leu 234 CG (3.45) |

Furthermore, the detailed structures around P238D were compared by superposing the X-ray crystal structure of Fc (P238D)/FcγRIIb extracellular region complex on the model structure of the Fc (WT)/FcγRIIb extracellular region complex using the least squares method based on the Cα atomic distance between Fc CH2 domains A and B alone. As the position of the amino acid residue at position 238 (EU numbering), *i.e.*, a mutagenesis position of Fc (P238D), is altered from Fc (WT), the loop structure around the amino acid residue at position 238 following the hinge region is found to be different between Fc (P238D) and Fc (WT) (Fig. 28). Pro at position 238 (EU numbering) is originally located inside Fc (WT), forming a hydrophobic core with residues around position 238. However, if Pro at position 238 (EU numbering) is altered to highly hydrophilic and charged Asp, the presence of the altered Asp residue in a hydrophobic core is energetically disadvantageous in terms of desolvation. Therefore, in Fc (P238D), to cancel this energetically disadvantageous situation, the amino acid residue at position 238 (EU numbering) changes its orientation to face the solvent side, and this may have caused this change in the loop structure near the amino acid residue at position 238. Furthermore, since this loop is not far from the hinge region crosslinked by an S-S bond, its structural change will not be limited to a local change, and will affect the relative positioning of the FcCH2 domain A and domain B. As a result, the interatomic interactions between FcγRIIb and Fc CH2 domain B have been changed. Therefore, predicted effects could not be observed when alterations that improve selectivity and binding activity towards FcγRIIb in a naturally-occurring IgG were combined with an Fc containing the P238D alteration.

Furthermore, as a result of structural changes due to introduction of P238D in Fc CH2 domain A, a hydrogen bond has been found between the main chain of Gly at position 237 (EU numbering), which is adjacent to P238D mutated, and Tyr at position 160 in FcγRIIb (Fig. 29). The residue in FcγRIIa that corresponds to this Tyr 160 is Phe; and when the binding is to FcγRIIa, this hydrogen bond is not formed. Considering that the amino acid at position 160 is one of the few differences between FcγRIIa and FcγRIIb at the interface of interaction with Fc, the presence of this hydrogen bond which is specific to FcγRIIb is presumed to have led to improvement of FcγRIIb-binding activity and decrease of FcγRIIa-binding activity in Fc (P238D), and improvement of its selectivity. Furthermore, in Fc CH2 domain B, an electrostatic interaction is observed between Asp at position 270 (EU numbering) and Arg at position 131 in FcγRIIb (Fig. 30). In FcγRIIa type H, which is one of the allotypes of FcγRIIa, the residue corresponding to Arg at position 131 of FcγRIIb is His, and therefore cannot form this electrostatic interaction. This can explain why the Fc (P238D)-binding activity is lowered in FcγRIIa type H compared with FcγRIIa type R. Observations based on such results of X-ray crystal structure analysis showed that the change of the loop structure near P238D due to P238D introduction and the accompanying change in the relative domain positioning causes formation of new interactions which is not found in the binding of the naturally-occurring IgG and FcγR, and this could lead to a selective binding profile of P238D variants for FcγRIIb.

[Expression and Purification of Fc (P238D)]

An Fc containing the P238D alteration was prepared as follows. First, Cys at position 220 (EU numbering) of hIL6R-IgG1-v1 (SEQ ID NO: 143) was substituted with Ser. Then, genetic sequence of Fc (P238D) from Glu at position 236 (EU numbering) to its C terminal was cloned by PCR. Using this cloned genetic sequence, production of expression vectors, and expression and purification of Fc (P238D) were carried out according to the method of Reference Examples 1 and 2. Cys at position 220 (EU numbering) forms a disulfide bond with Cys of the L chain in general IgG1. The L chain is not co-expressed when Fc alone is prepared, and therefore, the Cys residue was substituted with Ser to avoid formation of unnecessary disulfide bonds.

[Expression and purification of the FcγRIIb extracellular region]

The FcγRIIb extracellular region was prepared according to the method of Reference Example 25.

[Purification of the Fc (P238D) / FcγRIIb extracellular region complex]

To 2 mg of the FcγRIIb extracellular region sample obtained for use in crystallization, 0.29 mg of Endo F1 (Protein Science (1996) 5: 2617-2622) expressed and purified from *Escherichia coli* as a glutathione S-transferase fusion protein was added. This was allowed to remain at room temperature for three days in 0.1 M Bis-Tris buffer at pH 6.5, and the N-linked oligosaccharide was cleaved, except for *N*-acetylglucosamine directly bound to Asn of the FcγRIIb extracellular region. Next, the FcγRIIb extracellular region sample subjected to carbohydrate cleavage treatment, which was concentrated by ultrafiltration with 5000 MWCO, was purified by gel filtration chromatography (Superdex200 10/300) using a column equilibrated in 20 mM HEPS at pH 7.5 containing 0.05 M NaCl. Furthermore, to the obtained carbohydrate-cleaved FcγRIIb extracellular region fraction, Fc (P238D) was added so that the molar ratio of the FcγRIIb extracellular region would be present in slight excess. The mixture concentrated by ultrafiltration with 10,000 MWCO was subjected to purification by gel filtration chromatography (Superdex200 10/300) using a column equilibrated in 20 mM HEPS at pH 7.5 containing 0.05 M NaCl. Thus, a sample of the Fc (P238D) / FcγRIIb extracellular region complex was obtained.

[Crystallization of the Fc (P238D) / FcγRIIb extracellular region complex]

Using the sample of the Fc (P238D) / FcyRIIb extracellular region complex which was concentrated to approximately 10 mg/mL by ultrafiltration with 10,000 MWCO, crystallization of the complex was carried out by the sitting drop vapor diffusion method. Hydra II Plus One (MATRIX) was used for crystallization; and for a reservoir solution containing 100 mM Bis-Tris pH 6.5, 17% PEG3350, 0.2 M ammonium acetate, and 2.7% (w/v) D-Galactose, a crystallization drop was produced by mixing at a ratio of reservoir solution : crystallization sample = 0.2 μl : 0.2 μl. The crystallization drop after sealing was allowed to remain at 20°C, and thus thin plate-like crystals were obtained.

[Measurement of X-ray diffraction data from an Fc (P238D) / FcγRIIb extracellular region complex crystal]

One of the obtained single crystals of the Fc (P238D) / FcγRIIb extracellular region complex was soaked into a solution of 100 mM Bis-Tris pH 6.5, 20% PEG3350, ammonium acetate, 2.7% (w/v) D-Galactose, 22.5% (v/v) ethylene glycol. The single crystal was fished out of the solution using a pin with attached tiny nylon loop, and frozen in liquid nitrogen. Then, the X-ray diffraction data of the crystal was measured at synchrotron radiation facility Photon Factory BL-1A in High Energy Accelerator Research Organization. During the measurement, the crystal was constantly placed in a nitrogen stream at -178°C to maintain in a frozen state, and a total of 225 X ray diffraction images were collected using Quantum 270 CCD detector (ADSC) attached to a beam line with rotating the crystal 0.8° at a time. Determination of cell parameters, indexing of diffraction spots, and diffraction data processing from the obtained diffraction images were performed using the Xia2 program (CCP4 Software Suite), XDS Package (Walfgang Kabsch) and Scala (CCP4 Software Suite); and finally, diffraction intensity data of the crystal up to 2.46 Å resolution was obtained. The crystal belongs to the space group P21, and has the following cell parameters; a = 48.85 Å, b = 76.01 Å, c = 115.09 Å, $\alpha$ = 90°, $\beta$ = 100.70°, $\gamma$ = 90°.

[X ray crystal structure analysis of the Fc (P238D) / FcyRIIb extracellular region complex]

Crystal structure of the Fc (P238D) / Fc$\gamma$RIIb extracellular region complex was determined by the molecular replacement method using the program Phaser (CCP4 Software Suite). From the size of the obtained crystal lattice and the molecular weight of the Fc (P238D) / Fc$\gamma$RIIb extracellular region complex, the number of complexes in the asymmetric unit was predicted to be one. From the structural coordinates of PDB code: 3SGJ which is the crystal structure of the Fc (WT) / Fc$\gamma$RIIIa extracellular region complex, the amino acid residue portions of the A chain positions 239-340 and the B chain positions 239-340 were taken out as separate coordinates, and they were set respectively as models for searching the Fc CH2 domains. The amino acid residue portions of the A chain positions 341-444 and the B chain positions 341-443 were taken out as a single set of coordinates from the same structural coordinates of PDB code: 3SGJ; and this was set as a model for searching the Fc CH3 domains. Finally, from the structural coordinates of PDB code: 2FCB which is a crystal structure of the Fc$\gamma$RIIb extracellular region, the amino acid residue portions of the A chain positions 6-178 was taken out and set as a model for searching the Fc$\gamma$RIIb extracellular region. The orientation and position of each search model in the crystal lattice were determined in the order of Fc CH3 domain, Fc$\gamma$RIIb extracellular region, and Fc CH2 domain, based on the rotation function and translation function to obtain the initial model for the crystal structure of the Fc (P238D) / Fc$\gamma$RIIb extracellular region complex. When rigid body refinement which moves the two Fc CH2 domains, the two Fc CH3 domains, and the FcyRIIb extracellular region was performed on the obtained initial model, the crystallographic reliability factor, R value became 40.4%, and the Free R value became 41.9% to diffraction intensity data from 25 Å to 3.0 Å at this point. Furthermore, structural refinement using the program Refmac5 (CCP4 Software Suite), and revision of the model to observe the electron density maps whose coefficient have 2Fo-Fc or Fo-Fc, which are calculated based on the experimentally determined structural factor Fo, the calculated structural factor Fc and the calculated phase using the model, was carried out by the Coot program (Paul Emsley). Model refinement was carried out by repeating these steps. Finally, as a result of incorporation of water molecules into the model based on the electron density maps which use 2Fo-Fc or Fo-Fc as the coefficient, and the following refinement, the crystallographic reliability factor, R values and the Free R value of the model containing 4846 non-hydrogen atoms became 23.7% and 27.6% to 24291 diffraction intensity data from 25 Å to 2.6 Å resolution, respectively.

[Production of a model structure of the Fc (WT) / Fc$\gamma$RIIb extracellular region complex]

Based on the structural coordinates of PDB code: 3RY6 which is a crystal structure of the Fc (WT) / Fc$\gamma$RIIa extracellular region complex, the Build Mutants function of the Discovery Studio 3.1 program (Accelrys) was used to introduce mutations to match the amino acid sequence of Fc$\gamma$RIIb into Fc$\gamma$RIIa in this structural coordinates. In that case, the Optimization Level was set to High, Cut Radius was set to 4.5, five models were generated, and the one with the best energy score from among them was set as the model structure for the Fc (WT)/ Fc$\gamma$RIIb extracellular region complex.

[Example 11] Analysis of Fc$\gamma$R binding of Fc variants whose alteration sites were determined based on crystal structures

Based on the results of X-ray crystal structure analysis on the complex formed between Fc (P238D) and the Fc$\gamma$RIIb extracellular region obtained in Example 10, variants were constructed by comprehensively introducing alterations into sites on the altered Fc having substitution of Pro at position 238 (EU numbering) with Asp that were predicted to affect interaction with Fc$\gamma$RIIb (residues of positions 233, 240, 241, 263, 265, 266, 267, 268, 271, 273, 295, 296, 298, 300, 323, 325, 326, 327, 328, 330, 332, and 334 (EU numbering)), and whether combinations of alterations that further enhance Fc$\gamma$RIIb binding in addition to the P238D alteration can be obtained, was examined.

IL6R-B3 (SEQ ID NO: 144) was produced by introducing into IL6R-G1d (SEQ ID NO: 134), the alteration produced by substituting Lys at position 439 (EU numbering) with Glu. Next, IL6R-BF648 was produced by introducing into IL6R-B3, the alteration produced by substituting Pro at position 238 (EU numbering) with Asp. IL6R-L (SEQ ID NO: 135) was utilized as the common antibody L chain. These antibody variants expressed were purified according to the method of Reference Example 2. The binding of these antibody variants to each of the Fc$\gamma$Rs (Fc$\gamma$RIa, Fc$\gamma$RIIa type H, Fc$\gamma$RIIa type R, Fc$\gamma$RIIb, and Fc$\gamma$RIIIa type V) was comprehensively evaluated by the method of Reference Example 25.

A figure was produced according to the following method to show the results of analyzing the interactions with the respective Fc$\gamma$Rs. The value for the amount of binding of each variant to each Fc$\gamma$R was divided by the value for the amount of binding of the pre-altered control antibody (IL6R-BF648/IL6R-L, alteration by substituting Pro at position 238 (EU numbering) with Asp) to each Fc$\gamma$R, and the obtained was then multiplied by 100 and shown as the relative binding activity value of each variant to each Fc$\gamma$R. The horizontal axis shows the relative binding activity value of each variant to Fc$\gamma$RIIb, and the vertical axis shows the relative binding activity value of each variant to Fc$\gamma$RIIa type R (Fig. 31).

As shown in Fig. 31, the results show that of all the alterations, 24 types of alterations were found to maintain or enhance FcγRIIb binding in comparison with the pre-altered antibody. The binding of these variants to each of the FcγRs are shown in Table 17. In the table, alteration refers to the alteration introduced into IL6R-B3 (SEQ ID NO: 144). The template used for producing IL6R-B3, IL6R-G1d/IL6R-L, is indicated with an asterisk (*).

[Table 17]

| VARIANT NAME | ALTERATION | RELATIVE BINDING | | | | |
|---|---|---|---|---|---|---|
| | | FcyRIa | FcγRIIaR | FcγRIIaH | FcγRIIb | FcγRIIIa |
| IL6R-G1d/IL6R-L | * | 140 | 650 | 1670 | 62 | 3348 |
| IL6R-B3/IL6R-L | | 145 | 625 | 1601 | 58 | 3264 |
| IL6R-BF648/IL6R-L | P238D | 100 | 100 | 100 | 100 | 100 |
| IL6R-2B002/IL6R-L | P238D/E233D | 118 | 103 | 147 | 116 | 147 |
| IL6R-BP100/IL6R-L | P238D/S267A | 121 | 197 | 128 | 110 | 138 |
| IL6R-BP102/IL6R-L | P238D/S267Q | 104 | 165 | 66 | 106 | 86 |
| IL6R-BP103/IL6R-L | P238D/S267V | 56 | 163 | 69 | 107 | 77 |
| IL6R-BP106/IL6R-L | P238D/H268D | 127 | 150 | 110 | 116 | 127 |
| IL6R-BP107/IL6R-L | P238D/H268E | 123 | 147 | 114 | 118 | 129 |
| IL6R-BP110/IL6R-L | P238D/H268N | 105 | 128 | 127 | 101 | 127 |
| IL6R-BP112/IL6R-L | P238D/P271G | 119 | 340 | 113 | 157 | 102 |
| IL6R-2B128/IL6R-L | P238D/Y296D | 95 | 87 | 37 | 103 | 96 |
| IL6R-2B169/IL6R-L | P238D/V323I | 73 | 92 | 83 | 104 | 94 |
| IL6R-2B171/IL6R-L | P238D/V323L | 116 | 117 | 115 | 113 | 122 |
| IL6R-2B172/IL6R-L | P238D /V323M | 140 | 244 | 179 | 132 | 144 |
| IL6R-BP136/IL6R-L | P238D/ K326A | 117 | 159 | 103 | 119 | 102 |
| IL6R-BP117/IL6R-L | P238D/K326D | 124 | 166 | 96 | 118 | 105 |
| IL6R-BP120/IL6R-L | P238D/K326E | 125 | 175 | 92 | 114 | 103 |
| IL6R-BP126/IL6R-L | P238D/K326L | 113 | 167 | 132 | 103 | 146 |
| IL6R-BP119/IL6R-L | P238D/K326M | 117 | 181 | 133 | 110 | 145 |
| IL6R-BP142/IL6R-L | P238D/K326N | 98 | 103 | 97 | 106 | 102 |
| IL6R-BP121/IL6R-L | P238D/K326Q | 118 | 155 | 135 | 113 | 157 |
| IL6R-BP118/IL6R-L | P238D/K326S | 101 | 132 | 128 | 104 | 144 |
| IL6R-BP116/IL6R-L | P238D/K326T | 110 | 126 | 110 | 108 | 114 |
| IL6R-BP911/IL6R-L | P238D/A330K | 52 | 101 | 108 | 119 | 120 |
| IL6R-BP078/IL6R-L | P238D/A330M | 106 | 101 | 89 | 105 | 91 |
| IL6R-BP912/IL6R-L | P238D/A330R | 60 | 81 | 93 | 103 | 97 |

The results of measuring KD values of the variants shown in Table 17 for FcγRIa, FcγRIIaR, FcγRIIaH, FcγRIIb, and FcγRIIIa V types by the method of Reference Example 25 are summarized in Table 18. In the table, alteration refers to the alteration introduced into IL6R-B3 (SEQ ID NO: 144). The template used for producing IL6R-B3, IL6R-G1d/IL6R-L, is indicated with an asterisk (*). Furthermore, KD (IIaR)/KD (IIb) and KD (IIaH)/KD (IIb) in the table respectively represent the value obtained by dividing the KD value of each variant for FcγRIIaR by the KD value of each variant for FcγRIIb, and the value obtained by dividing the KD value of each variant for FcγRIIaH by the KD value of each variant for FcγRIIb. KD (IIb) of the parent polypeptide / KD (IIb) of the altered polypeptide refers to the value obtained by dividing the KD value of the parent polypeptide for FcγRIIb by the KD value of each variant for FcγRIIb. In addition, the KD value for the stronger of the FcγRIIaR- and FcγRIIaH-binding activities of each variant / KD value for the stronger of the FcγRIIaR- and FcγRIIaH-binding activities of the parent polypeptide are shown in Table 18. Here, parent polypeptide refers to the variant which has IL6R-B3 (SEQ ID NO: 144) as the H chain. It was determined that due to weak binding of FcγR to IgG, it was impossible to accurately analyze by kinetic analysis, and thus the gray-filled cells in Table 18 show values calculated by using Equation 2 of Reference Example 25.

[Equation 2]

$$KD = C \bullet R_{max} / (R_{eq} - RI) - C$$

[Table 18]

| VARIANT NAME | ALTERATION | KD(mol/L) | | | | | | | | |
|---|---|---|---|---|---|---|---|---|---|---|
| | | FcγRIa | FcγRIIaR | FcγRIIaH | FcγRIIb | FcγRIIIaV | KD(IIaR)/KD(IIb) | KD(IIaH)/KD(IIb) | KD(IIb) OF THE PARENT POLYPEPTIDE / KD(IIb) OF THE ALTERED POLYPEPTIDE | KD VALUE FOR THE STRONGER OF THE FcγRIIaR- AND FcγRIIaH-BINDING ACTIVITIES OF THE VARIANT / KD VALUE FOR THE STRONGER OF THE FcγRIIaR- AND FcγRIIaH-BINDING ACTIVITIES OF THE PARENT POLYPEPTIDE |
| IL6R-G1d/IL6R-L | * | 3.2E-10 | 1.0E-06 | 6.7E-07 | 2.6E-06 | 3.5E-07 | 0.4 | 0.3 | 1.2 | 0.9 |
| IL6R-B3/IL6R-L | | 4.2E-10 | 1.1E-06 | 7.7E-07 | 3.1E-06 | 3.3E-07 | 0.3 | 0.2 | 1.0 | 1.0 |
| IL6R-BF648/IL6R-L | P238D | 1.1E-08 | 1.5E-05 | 4.0E-05 | 1.2E-06 | 7.1E-05 | 13.0 | 33.9 | 2.6 | 19.9 |
| IL6R-2B002/IL6R-L | P238D/E233D | 6.4E-09 | 1.9E-05 | 8.6E-05 | 9.3E-07 | 5.3E-05 | 20.4 | 92.3 | 3.3 | 24.7 |
| IL6R-BP100/IL6R-L | P238D/S267A | 1.1E-09 | 7.8E-06 | 4.6E-05 | 1.1E-06 | 5.9E-05 | 7.3 | 42.6 | 2.9 | 10.2 |
| IL6R-BP102/IL6R-L | P238D/S267Q | 8.2E-09 | 8.4E-06 | 6.1E-05 | 9.0E-07 | 8.2E-05 | 9.4 | 67.6 | 3.4 | 11.0 |
| IL6R-BP103/IL6R-L | P238D/S267V | 3.5E-08 | 1.1E-05 | 8.8E-05 | 1.2E-06 | 1.1E-04 | 9.0 | 71.5 | 2.5 | 14.4 |
| IL6R-BP106/IL6R-L | P238D/H268D | 4.0E-09 | 1.1E-05 | 3.6E-05 | 9.3E-07 | 5.5E-05 | 11.6 | 38.7 | 3.3 | 14.0 |
| IL6R-BP107/IL6R-L | P238D/H268E | 1.5E-09 | 1.2E-05 | 5.2E-05 | 9.3E-07 | 6.3E-05 | 12.7 | 56.1 | 3.3 | 15.3 |
| IL6R-BP110/IL6R-L | P238D/H268N | 7.3E-09 | 1.7E-05 | 4.7E-05 | 1.5E-06 | 6.4E-05 | 11.7 | 31.5 | 2.1 | 22.6 |
| IL6R-BP112/IL6R-L | P238D/P271G | 6.5E-09 | 3.5E-06 | 3.5E-05 | 3.2E-07 | 6.9E-05 | 11.0 | 109.4 | 9.7 | 4.6 |
| IL6R-2B128/IL6R-L | P238D/Y296D | 1.3E-08 | 2.6E-05 | 3.4E-05 | 1.4E-06 | 7.2E-05 | 17.7 | 23.6 | 2.1 | 33.1 |
| IL6R-2B169/IL6R-L | P238D/V323I | 2.5E-08 | 1.9E-05 | 4.8E-05 | 1.2E-06 | 7.5E-05 | 15.8 | 40.7 | 2.6 | 24.3 |
| IL6R-2B171/IL6R-L | P238D/V323L | 9.1E-09 | 1.6E-05 | 3.4E-05 | 1.1E-06 | 5.7E-05 | 15.0 | 31.8 | 2.9 | 20.8 |
| IL6R-2B172/IL6R-L | P238D/V323M | 3.0E-09 | 6.1E-06 | 2.1E-05 | 7.7E-07 | 4.8E-05 | 8.0 | 27.3 | 4.0 | 8.0 |
| IL6R-BP136/IL6R-L | P238D/K326A | 6.6E-09 | 9.1E-06 | 3.8E-05 | 8.0E-07 | 6.9E-05 | 11.4 | 47.6 | 3.9 | 11.8 |
| IL6R-BP117/IL6R-L | P238D/K326D | 4.1E-09 | 9.2E-06 | 4.1E-05 | 8.0E-07 | 6.7E-05 | 11.6 | 51.4 | 3.9 | 12.0 |
| IL6R-BP120/IL6R-L | P238D/K326E | 6.6E-09 | 9.6E-06 | 6.5E-05 | 1.0E-06 | 7.9E-05 | 9.3 | 63.1 | 3.0 | 12.5 |
| IL6R-BP126/IL6R-L | P238D/K326L | 7.4E-09 | 1.1E-05 | 4.5E-05 | 1.4E-06 | 5.6E-05 | 7.8 | 31.7 | 2.2 | 14.4 |
| IL6R-BP119/IL6R-L | P238D/K326M | 7.0E-09 | 9.9E-06 | 4.5E-05 | 1.1E-06 | 5.6E-05 | 8.7 | 39.5 | 2.7 | 12.8 |
| IL6R-BP142/IL6R-L | P238D/K326N | 5.3E-09 | 1.8E-05 | 9.3E-05 | 1.2E-06 | 1.1E-04 | 15.5 | 79.5 | 2.6 | 23.5 |
| IL6R-BP121/IL6R-L | P238D/K326Q | 1.1E-08 | 1.3E-05 | 4.4E-05 | 1.1E-06 | 5.2E-05 | 11.7 | 40.4 | 2.8 | 16.6 |
| IL6R-BP118/IL6R-L | P238D/K326S | 1.2E-08 | 1.5E-05 | 4.6E-05 | 1.2E-06 | 5.6E-05 | 13.2 | 40.0 | 2.7 | 19.7 |
| IL6R-BP116/IL6R-L | P238D/K326T | 2.6E-09 | 1.5E-05 | 5.4E-05 | 1.1E-06 | 7.2E-05 | 13.3 | 48.2 | 2.8 | 19.4 |
| IL6R-BP911/IL6R-L | P238D/A330K | 4.9E-08 | 1.6E-05 | 3.7E-05 | 8.9E-07 | 5.8E-05 | 18.5 | 41.7 | 3.5 | 21.3 |
| IL6R-BP078/IL6R-L | P238D/A330M | 8.2E-09 | 1.5E-05 | 4.5E-05 | 1.1E-06 | 7.8E-05 | 13.4 | 41.3 | 2.8 | 19.0 |
| IL6R-BP912/IL6R-L | P238D/A330R | 3.8E-08 | 2.6E-05 | 3.8E-05 | 1.5E-06 | 7.8E-05 | 17.8 | 25.9 | 2.1 | 34.0 |

Table 18 shows that in comparison with IL6R-B3, all variants showed improvement of affinity for FcγRIIb, and the range of improvement was 2.1 fold to 9.7 fold. The ratio of KD value of each variant for FcγRIIaR / KD value of each variant for FcγRIIb, and the ratio of KD value of each variant for FcγRIIaH / KD value of each variant for FcγRIIb represent an FcγRIIb-binding activity relative to the FcγRIIaR-binding activity and FcγRIIaH-binding activity, respectively. That is, these values show the degree of binding selectivity of each variant for FcγRIIb, and a greater value indicates a higher binding selectivity for FcγRIIb. Since the ratio of KD value for FcγRIIaR / KD value for FcγRIIb, and the ratio of KD value for FcγRIIaH / KD value for FcγRIIb in the parent polypeptide IL6R-B3/IL6R-L were 0.3 and 0.2, respectively, all variants in Table 18 showed improvement of binding selectivity for FcγRIIb in comparison with the parent polypeptide. When the KD value for the stronger of the FcγRIIaR- and FcγRIIaH-binding activities of a variant / KD value for the stronger of the FcγRIIaR- and FcγRIIaH-binding activities of the parent polypeptide is 1 or more, this means that the stronger of the FcγRIIaR- and FcγRIIaH-binding activities of a variant has equivalent or decreased binding compared with the binding by the stronger of the FcγRIIaR- and FcγRIIaH-binding activities of the parent polypeptide. Since this value was 4.6 to 34.0 for the variants obtained this time, one may say that in comparison with the parent polypeptide, the variants obtained this time had reduced binding by the stronger of the FcγRIIaR- and FcγRIIaH-binding activities. These results showed that compared with the parent polypeptide, the variants obtained this time have maintained or decreased FcγRIIa type R- and type H-binding activities, enhanced FcyRIIb-binding activity, and improved selectivity for FcγRIIb. Furthermore, compared with IL6R-B3, all variants had lower affinity to FcγRIa and FcγRIIIaV.

With regard to the promising variants among the obtained combination variants, the factors leading to their effects were studied using the crystal structure. Fig. 32 shows the crystal structure of the Fc (P238D) / FcγRIIb extracellular region complex. In this figure, the H chain positioned on the left side is Fc Chain A, and the H chain positioned on the right side is Fc Chain B. Here, one can see that the site at position 233 (EU numbering) in Fc Chain A is located near Lys at position 113 of FcγRIIb. However, in this crystal structure, the E233 side chain is in a condition of considerably high mobility, and its electron density is not well observed. Therefore, the alteration produced by substituting Glu at position 233 (EU numbering) with Asp leads to decrease in the degree of freedom of the side chain since the side chain becomes one carbon shorter. As a result, the entropy loss when forming an interaction with Lys at position 113 of FcγRIIb may be decreased, and consequently this is speculated to contribute to improvement of binding free energy.

Similarly, Fig. 33 shows the environment near the site at position 330 (EU numbering) in the structure of the Fc (P238D) / FcγRIIb extracellular region complex. This figure shows that the environment around the site at position 330 (EU numbering) of Fc Chain A of Fc (P238D) is a hydrophilic environment composed of Ser at position 85, Glu at position 86, Lys at position 163, and such of FcγRIIb. Therefore, the alteration produced by substituting Ala at position 330 (EU numbering) with Lys or Arg is speculated to contribute to strengthening the interaction with Ser at position 85 or Glu at position 86 in FcγRIIb.

Fig. 34 depicts the structures of Pro at position 271 (EU numbering) of Fc Chain B after superimposing the crystal structures of the Fc (P238D) / FcγRIIb extracellular region complex and the Fc (WT) / FcγRIIIa extracellular region complex by the least squares fitting based on the Cα atom pair distances with respect to Fc Chain B. These two structures match well, but have different three-dimensional structures of Pro at position 271 (EU numbering). When the weak electron density around this area in the crystal structure of the Fc (P238D)/FcyRIIb extracellular region complex is also taken into consideration, it is suggested that there is possibility that Pro at position 271 (EU numbering) in Fc (P238D) / FcγRIIb causes a large strain on the structure, thus disturbing the loop structure to attain an optimal structure. Therefore, the alteration produced by substituting Pro at position 271 (EU numbering) with Gly gives flexibility to this loop structure, and is speculated to contribute to enhancement of binding by reducing the energetic barrier when allowing an optimum structure to form during interaction with FcγRIIb.

[Example 12] Examination of the combinatorial effect of alterations that enhance FcγRIIb binding when combined with P238D

Of the alterations obtained in Examples 9 and 11, those that enhanced FcγRIIb binding or maintained FcγRIIb binding and showed effects of suppressing binding to other FcγRs were combined with each other, and its effect was examined.

Particularly good alterations selected from Tables 14 and 18 were introduced into the antibody H chain IL6R-BF648 in a similar manner to the method of Example 11. IL6R-L was utilized as the antibody L chain, the expressed antibodies were purified according to the method of Reference Example 1. The binding to each of the FcγRs (FcγRIa, FcγRIIa H type, FcγRIIa R type, FcγRIIb, and FcγRIIIa V type) was comprehensively evaluated by the method of Reference Example 25.

According to the following method, relative binding activities were calculated for the results of analyzing interactions with the respective FcγRs. The value for the amount of binding of each variant to each FcγR was divided by the value for the amount of binding of the pre-altered control antibody (IL6R-BF648/IL6R-L with substitution of Pro at position 238 (EU numbering) with Asp to each FcγR, and multiplied by 100; and then the value was shown as the relative binding activity value of each variant to each FcγR (Table 19).

In the table, alteration refers to the alteration introduced into IL6R-B3 (SEQ ID NO: 144). The template used for producing IL6R-B3, IL6R-G1d/IL6R-L, is indicated with an asterisk (*).

[Table 19-1]

| VARIANT NAME | ALTERATION | RELATIVE BINDING ACTIVITY | | | | |
|---|---|---|---|---|---|---|
| | | FcgRIa | FcgRIIaR | FcgRIIaH | FcgRIIb | FcgRIIIaV |
| IL6R-G1d/IL6R-L | | 140 | 650 | 1670 | 62 | 3348 |
| IL6R-B3/IL6R-L | | 145 | 625 | 1601 | 58 | 3264 |
| IL6R-BF648/IL6R-L | P238D | 100 | 100 | 100 | 100 | 100 |
| IL6R-2B253/IL6R-L | E233D/P238D/V323M | 155 | 288 | 207 | 156 | 126 |
| IL6R-2B261/IL6R-L | E233D/P238D/Y296D | 100 | 94 | 91 | 115 | 87 |
| IL6R-BP082/IL6R-L | E233D/P238D/A330K | 74 | 126 | 106 | 136 | 87 |
| IL6R-BP083/IL6R-L | P238D/Y296D/A330K | 50 | 87 | 91 | 122 | 107 |
| IL6R-BP084/IL6R-L | P238D/V323M/A330K | 109 | 203 | 162 | 141 | 106 |
| IL6R-BP085/IL6R-L | G237D/P238D/A330K | 19 | 279 | 158 | 152 | 104 |
| IL6R-BP086/IL6R-L | P238D/K326A/A330K | 72 | 155 | 116 | 137 | 123 |
| IL6R-BP087/IL6R-L | L234Y/P238D/A330K | 33 | 163 | 179 | 137 | 158 |
| IL6R-BP088/IL6R-L | G237D/P238D/K326A/A330K | 25 | 377 | 166 | 161 | 122 |
| IL6R-BP089/IL6R-L | L234Y/P238D/K326A/A330K | 43 | 222 | 186 | 147 | 136 |
| IL6R-BP129/IL6R-L | E233D/P238D/Y296D/A330K | 68 | 111 | 98 | 138 | 95 |
| IL6R-BP130/IL6R-L | E233D/P238D/V323M/A330K | 104 | 272 | 224 | 160 | 115 |
| IL6R-BP131/IL6R-L | E233D/G237D/P238D/A330K | 33 | 364 | 253 | 160 | 118 |
| IL6R-BP132/IL6R-L | E233D/P238D/K326A/A330K | 91 | 191 | 130 | 150 | 120 |
| IL6R-BP133/IL6R-L | E233D/L234Y/P238D/A330K | 41 | 174 | 151 | 137 | 114 |
| IL6R-BP143/IL6R-L | L234Y/P238D/K326A | 86 | 238 | 143 | 133 | 114 |
| IL6R-BP144/IL6R-L | G237D/P238D/K326A | 64 | 204 | 108 | 121 | 128 |
| IL6R-BP145/IL6R-L | L234Y/G237D/P238D | 41 | 350 | 224 | 152 | 153 |
| IL6R-BP146/IL6R-L | L234Y/G237D/P238D/K326A | 50 | 445 | 203 | 156 | 180 |
| IL6R-BP147/IL6R-L | L234Y/G237D/P238D/K326A/A330K | 24 | 650 | 582 | 177 | 209 |
| IL6R-BP148/IL6R-L | E233D/L234Y/G237D/P238D/K326A/A330K | 33 | 603 | 462 | 176 | 227 |
| IL6R-BP149/IL6R-L | E233D/L234Y/G237D/P238D/Y296D/K326A/A330K | 29 | 539 | 401 | 173 | 166 |
| IL6R-BP150/IL6R-L | L234Y/G237D/P238D/K326A/A330R | 30 | 757 | 770 | 183 | 204 |
| IL6R-BP151/IL6R-L | E233D/L234Y/G237D/P238D/K326A/A330R | 39 | 705 | 621 | 180 | 221 |
| IL6R-BP152/IL6R-L | E233D/L234Y/G237D/P238D/Y296D/K326A/A330R | 34 | 638 | 548 | 178 | 146 |
| IL6R-BP176/IL6R-L | E233D/P238D/K326D/A330K | 102 | 201 | 128 | 147 | 131 |
| IL6R-BP177/IL6R-L | E233D/L234Y/G237D/P238D/P271G/K326D/A330K | 57 | 691 | 409 | 177 | 186 |
| IL6R-BP178/IL6R-L | E233D/G237D/P238D/P271G/A330K | 51 | 653 | 259 | 179 | 110 |
| IL6R-BP179/IL6R-L | G237D/P238D/P271G/K326A/A330K | 39 | 570 | 226 | 177 | 125 |
| IL6R-BP180/IL6R-L | G237D/P238D/P271G/A330K | 29 | 602 | 203 | 179 | 100 |

Table 19-2 is a continuation table of Table 19-1.

[Table 19-2]

| | | | | | | |
|---|---|---|---|---|---|---|
| IL6R-BP181/IL6R-L | E233D/P238D/P271G/K326A/A330K | 108 | 362 | 150 | 170 | 122 |
| IL6R-BP182/IL6R-L | E233D/P238D/P271G/Y296D/A330K | 95 | 413 | 139 | 173 | 120 |
| IL6R-BP183/IL6R-L | E233D/L234Y/P238D/P271G/K326A/A330K | 83 | 423 | 191 | 164 | 113 |
| IL6R-BP184/IL6R-L | E233D/P238D/P271G/A330K | 96 | 436 | 131 | 171 | 106 |
| IL6R-BP185/IL6R-L | E233D/L234Y/G237D/P238D/P271G/K326A/A330K | 47 | 670 | 446 | 179 | 191 |
| IL6R-BP186/IL6R-L | E233D/L234Y/G237D/P238D/P271G/Y296D/K326A/A330K | 43 | 614 | 368 | 175 | 143 |
| IL6R-BP187/IL6R-L | L234Y/P238D/P271G/K326A/A330K | 68 | 387 | 205 | 157 | 124 |
| IL6R-BP188/IL6R-L | E233D/G237D/P238D/H268D/P271G/A330K | 74 | 636 | 234 | 179 | 121 |
| IL6R-BP189/IL6R-L | G237D/P238D/H268D/P271G/K326A/A330K | 56 | 557 | 183 | 177 | 141 |
| IL6R-BP190/IL6R-L | G237D/P238D/H268D/P271G/A330K | 50 | 615 | 224 | 181 | 155 |
| IL6R-BP191/IL6R-L | E233D/P238D/H268D/P271G/K326A/A330K | 125 | 382 | 145 | 170 | 142 |
| IL6R-BP192/IL6R-L | E233D/P238D/H268D/P271G/Y296D/A330K | 109 | 406 | 122 | 172 | 118 |
| IL6R-BP193/IL6R-L | E233D/P238D/H268D/P271G/A330K | 113 | 449 | 154 | 173 | 135 |
| IL6R-BP194/IL6R-L | E233D/L234Y/G237D/P238D/H268D/P271G/K326A/A330K | 69 | 672 | 395 | 178 | 249 |
| IL6R-BP195/IL6R-L | E233D/L234Y/G237D/P238D/H268D/P271G/Y296D/K326A/A330K | 68 | 661 | 344 | 181 | 221 |
| IL6R-BP196/IL6R-L | L234Y/P238D/H268D/P271G/K326A/A330K | 89 | 402 | 195 | 157 | 137 |
| IL6R-BP197/IL6R-L | E233D/L234Y/G237D/P238D/H268D/P271G/Y296D/K326D/A330K | 71 | 642 | 294 | 179 | 206 |
| IL6R-BP198/IL6R-L | E233D/L234Y/P238D/H268D/P271G/K326A/A330K | 104 | 449 | 188 | 164 | 157 |
| IL6R-BP199/IL6R-L | E233D/P238D/K326D/A330R | 112 | 172 | 116 | 144 | 103 |
| IL6R-BP200/IL6R-L | E233D/L234Y/G237D/P238D/P271G/K326D/A330R | 60 | 754 | 517 | 188 | 164 |
| IL6R-BP201/IL6R-L | E233D/G237D/P238D/P271G/A330R | 57 | 696 | 359 | 186 | 121 |
| IL6R-BP202/IL6R-L | G237D/P238D/P271G/K326A/A330R | 43 | 615 | 285 | 185 | 108 |
| IL6R-BP203/IL6R-L | G237D/P238D/P271G/A330R | 35 | 637 | 255 | 185 | 88 |
| IL6R-BP204/IL6R-L | E233D/P238D/P271G/K326A/A330R | 110 | 301 | 137 | 165 | 121 |
| IL6R-BP205/IL6R-L | E233D/P238D/P271G/Y296D/A330R | 97 | 335 | 108 | 167 | 93 |
| IL6R-BP206/IL6R-L | E233D/P238D/P271G/A330R | 101 | 362 | 123 | 168 | 92 |
| IL6R-BP207/IL6R-L | E233D/P238D/A330R | 74 | 103 | 103 | 124 | 97 |
| IL6R-BP208/IL6R-L | E233D/G237D/P238D/H268D/P271G/A330R | 81 | 690 | 310 | 188 | 118 |
| IL6R-BP209/IL6R-L | G237D/P238D/H268D/P271G/K326A/A330R | 68 | 625 | 267 | 186 | 153 |
| IL6R-BP210/IL6R-L | G237D/P238D/H268D/P271G/A330R | 57 | 661 | 279 | 187 | 135 |
| IL6R-BP211/IL6R-L | E233D/P238D/H268D/P271G/K326A/A330R | 128 | 312 | 111 | 165 | 87 |
| IL6R-BP212/IL6R-L | E233D/P238D/H268D/P271G/Y296D/A330R | 117 | 363 | 135 | 173 | 122 |
| IL6R-BP213/IL6R-L | E233D/P238D/H268D/P271G/A330R | 118 | 382 | 123 | 169 | 100 |
| IL6R-BP214/IL6R-L | E233D/L234Y/G237D/P238D/Y296D/K326D/A330K | 36 | 498 | 285 | 174 | 165 |

The results of measuring KD values of the variants shown in Table 19 for FcγRIa, FcγRIIaR, FcγRIIaH, FcγRIIb,

and FcγRIIIaV types by the method of Reference Example 25 are summarized in Tables 20-1 and 20-2. In the table, alteration refers to the alteration introduced into IL6R-B3 (SEQ ID NO: 144). The template used for producing IL6R-B3, IL6R-G1d/IL6R-L, is indicated with an asterisk (*). Furthermore, KD (IIaR)/KD (IIb) and KD (IIaH)/KD (IIb) in the table respectively represent the value obtained by dividing the KD value of the variant for FcγRIIaR by the KD value of the variant for FcγRIIb, and the value obtained by dividing the KD value of the variant for FcγRIIaH by the KD value of each variant for FcγRIIb. KD (IIb) of the parent polypeptide / KD (IIb) of the altered polypeptide refers to the value obtained by dividing the KD value of the parent polypeptide for FcγRIIb by the KD value of each variant for FcγRIIb. In addition, the KD value for the stronger of the FcγRIIaR- and FcγRIIaH-binding activities of each variant / KD value for the stronger of the FcγRIIaR- and FcγRIIaH-binding activities of the parent polypeptide are shown in Tables 20-1 and 20-2. Here, parent polypeptide refers to the variant which has IL6R-B3 (SEQ ID NO: 144) as the H chain. It was determined that due to weak binding of FcγR to IgG, it was impossible to accurately analyze by kinetic analysis, and thus the values of gray-filled cells in Tables 20-1 and 20-2 show values calculated by using Equation 2 of Reference Example 25.

[Equation 2]

$$KD = C \bullet R_{max} / (R_{eq} - RI) - C$$

Tables 20-1 and 20-2 show that in comparison with IL6R-B3, all variants showed improvement of affinity for FcγRIIb, and the range of improvement was 3.0 fold to 99.0 fold. The ratio of KD value of each variant for FcγRIIaR / KD value of each variant for FcγRIIb, and the ratio of KD value of each variant for FcγRIIaH / KD value of each variant for FcγRIIb represent an FcγRIIb-binding activity relative to the FcγRIIaR-binding activity and FcγRIIaH-binding activity, respectively. That is, those values show the degree of binding selectivity of each variant for FcγRIIb, and a greater value indicates a higher binding selectivity for FcγRIIb. Since the ratio of KD value for FcγRIIaR / KD value for FcγRIIb, and the ratio of KD value for FcγRIIaH / KD value for FcγRIIb of the parent polypeptide IL6R-B3/IL6R-L were 0.3 and 0.2, respectively, all variants in Tables 20-1 and 20-2 showed improvement of binding selectivity for FcγRIIb in comparison with the parent polypeptide. When the KD value for the stronger of the FcγRIIaR- and FcγRIIaH-binding activities of a variant / KD value for the stronger of the FcγRIIaR- and FcγRIIaH-binding activities of the parent polypeptide is 1 or more, this means that the stronger of the FcγRIIaR- and FcγRIIaH-binding activities of a variant has equivalent or decreased binding compared with the binding by the stronger of the FcγRIIaR- and FcγRIIaH-binding activities of the parent polypeptide. Since this value was 0.7 to 29.9 for the variants obtained this time, one may say that binding by the stronger of the FcγRIIaR- and FcγRIIaH-binding activities of the variants obtained this time was nearly equivalent or decreased compared with that of the parent polypeptide. These results showed that compared with the parent polypeptide, the variants obtained this time have maintained or decreased FcγRIIa type Rand type H-binding activities, enhanced FcyRIIb-binding activity, and improved selectivity for FcyRIIb. Furthermore, compared with IL6R-B3, all variants had lower affinity for FcyRIa and FcγRIIIaV.

[Table 20-1]

| VARIANT NAME | ALTERATION | KD(mol/L) | | | | | KD(IIaR)/ KD(IIb) | KD(IIaH)/ KD(IIb) | KD(IIb) OF THE PARENT POLYPEPTIDE / KD(IIb) OF THE ALTERED POLYPEPTIDE | KD VALUE FOR THE STRONGER OF THE FcγRIIaR- AND FcγRIIaH-BINDING ACTIVITIES OF THE VARIANT / KD VALUE FOR THE STRONGER OF THE FcγRIIaR- AND FcγRIIaH-BINDING ACTIVITIES OF THE PARENT POLYPEPTIDE |
|---|---|---|---|---|---|---|---|---|---|---|
| | | FcγRIa | FcγRIIaR | FcγRIIaH | FcγRIIb | FcγRIIIaV | | | | |
| IL6R-G1d/IL6R-L | | 3.2E-10 | 1.0E-06 | 6.7E-07 | 2.6E-06 | 3.5E-07 | 0.4 | 0.3 | 1.2 | 0.9 |
| IL6R-B3/IL6R-L | | 4.2E-10 | 1.1E-06 | 7.7E-07 | 3.1E-06 | 3.3E-07 | 0.3 | 0.2 | 1.0 | 1.0 |
| IL6R-BF648/IL6R-L | P238D | 1.1E-08 | 1.5E-05 | 4.0E-05 | 1.2E-06 | 7.1E-05 | 13.0 | 33.9 | 2.6 | 19.9 |
| IL6R-2B253/IL6R-L | E233D/P238D/V323M | 1.4E-09 | 5.0E-06 | 1.3E-05 | 4.3E-07 | 5.0E-05 | 11.5 | 30.1 | 7.2 | 6.5 |
| IL6R-2B261/IL6R-L | E233D/P238D/Y296D | 9.0E-09 | 2.2E-05 | 3.3E-05 | 1.0E-06 | 7.3E-05 | 21.8 | 32.4 | 3.0 | 28.8 |
| IL6R-BP062/IL6R-L | E233D/P238D/A330K | 1.8E-08 | 1.2E-05 | 3.7E-05 | 5.4E-07 | 8.1E-05 | 22.8 | 69.0 | 5.8 | 15.8 |
| IL6R-BP063/IL6R-L | P238D/Y296D/A330K | 3.8E-08 | 2.3E-05 | 4.4E-05 | 7.9E-07 | 6.6E-05 | 29.0 | 55.5 | 3.9 | 29.9 |
| IL6R-BP084/IL6R-L | P238D/V323M/A330K | 7.0E-09 | 7.2E-06 | 2.4E-05 | 5.0E-07 | 6.7E-05 | 14.3 | 47.6 | 6.1 | 9.4 |
| IL6R-BP085/IL6R-L | G237D/P238D/A330K | 2.9E-07 | 4.2E-06 | 2.4E-05 | 3.2E-07 | 6.8E-05 | 13.1 | 74.5 | 9.6 | 5.5 |
| IL6R-BP086/IL6R-L | P238D/K326A/A330K | 2.7E-08 | 9.7E-06 | 3.4E-05 | 5.7E-07 | 5.7E-05 | 17.1 | 59.9 | 5.4 | 12.6 |
| IL6R-BP087/IL6R-L | L234Y/P238D/A330K | 3.8E-08 | 9.7E-06 | 2.1E-05 | 6.1E-07 | 4.4E-05 | 16.0 | 34.7 | 5.1 | 12.6 |
| IL6R-BP088/IL6R-L | G237D/P238D/K326A/A330K | 3.9E-07 | 2.9E-06 | 2.3E-05 | 2.2E-07 | 5.7E-05 | 13.3 | 106.5 | 14.3 | 3.7 |
| IL6R-BP089/IL6R-L | L234Y/P238D/K326A/A330K | 6.3E-08 | 6.4E-06 | 2.0E-05 | 3.9E-07 | 5.1E-05 | 16.6 | 51.9 | 8.0 | 8.3 |
| IL6R-BP129/IL6R-L | E233D/P238D/Y296D/A330K | 2.5E-08 | 1.5E-05 | 4.0E-05 | 5.2E-07 | 7.5E-05 | 29.3 | 77.5 | 6.0 | 19.6 |
| IL6R-BP130/IL6R-L | E233D/P238D/V323M/A330K | 1.8E-09 | 5.3E-06 | 2.6E-05 | 3.0E-07 | 7.1E-05 | 17.5 | 85.5 | 10.2 | 6.9 |
| IL6R-BP131/IL6R-L | E233D/G237D/P238D/A330K | 1.2E-07 | 3.1E-06 | 1.4E-05 | 2.5E-07 | 5.9E-05 | 12.5 | 56.9 | 12.6 | 4.0 |
| IL6R-BP132/IL6R-L | E233D/P238D/K326A/A330K | 1.5E-08 | 8.0E-06 | 3.0E-05 | 3.7E-07 | 5.8E-05 | 21.5 | 81.1 | 8.4 | 10.3 |
| IL6R-BP133/IL6R-L | E233D/L234Y/P238D/A330K | 1.3E-07 | 8.6E-06 | 2.6E-05 | 5.6E-07 | 6.2E-05 | 15.5 | 46.8 | 5.6 | 11.2 |
| IL6R-BP143/IL6R-L | L234Y/P238D/K326A | 1.6E-08 | 5.7E-06 | 2.7E-05 | 5.7E-07 | 6.2E-05 | 10.0 | 47.1 | 5.4 | 7.5 |
| IL6R-BP144/IL6R-L | G237D/P238D/K326A | 3.7E-08 | 6.9E-06 | 3.6E-05 | 7.9E-07 | 5.5E-05 | 8.7 | 45.8 | 3.9 | 8.9 |
| IL6R-BP145/IL6R-L | L234Y/G237D/P238D | 1.2E-07 | 3.4E-06 | 1.7E-05 | 3.4E-07 | 4.5E-05 | 9.9 | 49.9 | 9.1 | 4.4 |
| IL6R-BP146/IL6R-L | L234Y/G237D/P238D/K326A | 7.4E-08 | 2.1E-06 | 1.8E-05 | 2.3E-07 | 3.8E-05 | 9.3 | 80.0 | 13.7 | 2.7 |
| IL6R-BP147/IL6R-L | L234Y/G237D/P238D/K326A/A330K | 1.4E-07 | 8.9E-07 | 5.1E-06 | 6.6E-08 | 3.3E-05 | 13.6 | 77.7 | 47.1 | 1.2 |
| IL6R-BP148/IL6R-L | E233D/L234Y/G237D/P238D/K326A/A330K | 8.9E-08 | 1.1E-06 | 7.0E-06 | 7.5E-08 | 3.0E-05 | 14.5 | 93.8 | 41.4 | 1.4 |
| IL6R-BP149/IL6R-L | E233D/L234Y/G237D/P238D/Y296D/K326A/A330K | 1.2E-07 | 1.4E-06 | 8.4E-06 | 9.3E-08 | 3.7E-05 | 15.0 | 89.9 | 33.1 | 1.8 |
| IL6R-BP150/IL6R-L | L234Y/G237D/P238D/K326A/A330R | 3.2E-07 | 5.5E-07 | 3.4E-06 | 3.1E-08 | 3.4E-05 | 17.7 | 109.0 | 99.0 | 0.7 |
| IL6R-BP151/IL6R-L | E233D/L234Y/G237D/P238D/K326A/A330R | 8.4E-08 | 6.7E-07 | 4.7E-06 | 4.0E-08 | 3.1E-05 | 16.9 | 117.8 | 77.4 | 0.9 |
| IL6R-BP152/IL6R-L | E233D/L234Y/G237D/P238D/Y296D/K326A/A330R | 7.3E-08 | 8.1E-07 | 5.6E-06 | 4.1E-08 | 4.8E-05 | 19.5 | 135.9 | 75.0 | 1.0 |
| IL6R-BP176/IL6R-L | E233D/P238D/K326D/A330K | 7.3E-09 | 6.9E-06 | 3.0E-05 | 3.6E-07 | 5.4E-05 | 19.1 | 83.1 | 8.6 | 8.9 |
| IL6R-BP177/IL6R-L | E233D/L234Y/G237D/P238D/P271G/K326D/A330K | 3.3E-08 | 7.1E-07 | 8.2E-06 | 5.2E-08 | 3.7E-05 | 13.8 | 159.2 | 60.0 | 0.9 |
| IL6R-BP178/IL6R-L | E233D/G237D/P238D/P271G/A330K | 4.3E-08 | 9.3E-07 | 1.4E-05 | 5.1E-08 | 6.4E-05 | 18.1 | 272.4 | 60.1 | 1.2 |
| IL6R-BP179/IL6R-L | G237D/P238D/P271G/K326A/A330K | 6.4E-08 | 1.4E-06 | 1.6E-05 | 8.4E-08 | 5.6E-05 | 16.7 | 190.9 | 36.9 | 1.8 |
| IL6R-BP180/IL6R-L | G237D/P238D/P271G/A330K | 9.8E-08 | 1.2E-06 | 1.8E-05 | 6.2E-08 | 7.0E-05 | 18.6 | 290.8 | 49.9 | 1.5 |
| IL6R-BP181/IL6R-L | E233D/P238D/P271G/K326A/A330K | 7.5E-09 | 3.2E-06 | 2.6E-05 | 1.6E-07 | 5.7E-05 | 20.3 | 162.5 | 19.3 | 4.2 |
| IL6R-BP182/IL6R-L | E233D/P238D/P271G/Y296D/A330K | 1.0E-08 | 2.6E-06 | 2.8E-05 | 1.1E-07 | 5.8E-05 | 23.5 | 256.9 | 28.3 | 3.3 |

Table 20-2 is a continuation table of Table 20-1.

[Table 20-2]

| | | | | | | | | | | |
|---|---|---|---|---|---|---|---|---|---|---|
| IL6R-BP183/IL6R-L | E233D/L234Y/P238D/P271G/K326A/A330K | 1.7E-08 | 2.6E-06 | 1.5E-05 | 2.4E-07 | 5.6E-05 | 10.7 | 62.5 | 12.9 | 3.3 |
| IL6R-BP184/IL6R-L | E233D/P238D/P271G/A330K | 1.1E-08 | 2.3E-06 | 3.0E-05 | 1.3E-07 | 6.6E-05 | 18.2 | 238.1 | 24.5 | 3.0 |
| IL6R-BP185/IL6R-L | E233D/L234Y/G237D/P238D/P271G/K326A/A330K | 6.3E-08 | 8.8E-07 | 7.3E-06 | 6.9E-08 | 3.6E-05 | 12.6 | 105.2 | 44.5 | 1.1 |
| IL6R-BP186/IL6R-L | E233D/L234Y/G237D/P238D/P271G/Y296D/K326A/A330K | 4.5E-08 | 9.6E-07 | 9.3E-06 | 6.1E-08 | 4.9E-05 | 15.8 | 152.5 | 50.7 | 1.3 |
| IL6R-BP187/IL6R-L | L234Y/P238D/P271G/K326A/A330K | 2.5E-08 | 2.8E-06 | 1.8E-05 | 2.9E-07 | 5.6E-05 | 9.7 | 62.3 | 10.7 | 3.6 |
| IL6R-BP188/IL6R-L | E233D/G237D/P238D/H268D/P271G/A330K | 2.1E-08 | 1.0E-06 | 1.6E-05 | 4.6E-08 | 5.8E-05 | 21.9 | 350.1 | 67.6 | 1.3 |
| IL6R-BP189/IL6R-L | G237D/P238D/H268D/P271G/K326A/A330K | 4.2E-08 | 1.4E-06 | 2.1E-05 | 7.4E-08 | 4.9E-05 | 18.5 | 263.8 | 41.8 | 1.8 |
| IL6R-BP190/IL6R-L | G237D/P238D/H268D/P271G/A330K | 6.3E-08 | 1.1E-06 | 1.7E-05 | 5.8E-08 | 4.5E-05 | 19.3 | 292.6 | 53.2 | 1.5 |
| IL6R-BP191/IL6R-L | E233D/P238D/H268D/P271G/K326A/A330K | 4.0E-09 | 3.0E-06 | 2.7E-05 | 1.5E-07 | 4.9E-05 | 20.3 | 184.9 | 21.2 | 3.8 |
| IL6R-BP192/IL6R-L | E233D/P238D/H268D/P271G/Y296D/A330K | 6.6E-09 | 2.6E-06 | 3.2E-05 | 1.1E-07 | 5.9E-05 | 23.1 | 283.2 | 27.3 | 3.4 |
| IL6R-BP193/IL6R-L | E233D/P238D/H268D/P271G/A330K | 6.3E-09 | 2.2E-06 | 2.5E-05 | 1.2E-07 | 5.2E-05 | 18.3 | 206.6 | 25.5 | 2.9 |
| IL6R-BP194/IL6R-L | E233D/L234Y/G237D/P238D/H268D/P271G/K326A/A330K | 2.4E-08 | 8.2E-07 | 8.5E-06 | 5.2E-08 | 2.7E-05 | 15.8 | 163.5 | 59.4 | 1.1 |
| IL6R-BP195/IL6R-L | E233D/L234Y/G237D/P238D/H268D/P271G/Y296D/K326A/A330K | 2.3E-08 | 9.1E-07 | 1.0E-05 | 5.0E-08 | 3.1E-05 | 18.2 | 200.8 | 62.0 | 1.2 |
| IL6R-BP196/IL6R-L | L234Y/P238D/H268D/P271G/K326A/A330K | 1.4E-08 | 3.0E-06 | 1.9E-05 | 2.2E-07 | 5.1E-05 | 13.4 | 85.2 | 13.9 | 3.9 |
| IL6R-BP197/IL6R-L | E233D/L234Y/G237D/P238D/H268D/P271G/Y296D/K326D/A330K | 1.9E-08 | 9.8E-07 | 1.2E-05 | 5.8E-08 | 3.3E-05 | 17.1 | 208.7 | 53.7 | 1.3 |
| IL6R-BP198/IL6R-L | E233D/L234Y/P238D/H268D/P271G/K326A/A330K | 1.1E-08 | 2.2E-06 | 2.0E-05 | 2.0E-07 | 4.4E-05 | 11.0 | 101.5 | 15.7 | 2.8 |
| IL6R-BP199/IL6R-L | E233D/P238D/K326D/A330R | 6.4E-09 | 8.6E-06 | 2.6E-05 | 4.9E-07 | 6.1E-05 | 17.5 | 53.0 | 6.3 | 11.1 |
| IL6R-BP200/IL6R-L | E233D/L234Y/G237D/P238D/P271G/K326D/A330R | 3.3E-08 | 6.3E-07 | 4.2E-06 | 3.4E-08 | 3.8E-05 | 18.6 | 123.9 | 91.2 | 0.8 |
| IL6R-BP201/IL6R-L | E233D/G237D/P238D/P271G/A330R | 5.1E-08 | 8.4E-07 | 6.9E-06 | 4.0E-08 | 5.2E-05 | 21.0 | 172.1 | 77.1 | 1.1 |
| IL6R-BP202/IL6R-L | G237D/P238D/P271G/K326A/A330R | 9.5E-08 | 1.2E-06 | 9.2E-06 | 6.4E-08 | 5.9E-05 | 19.2 | 144.0 | 48.4 | 1.6 |
| IL6R-BP203/IL6R-L | G237D/P238D/P271G/A330R | 1.8E-07 | 9.9E-07 | 1.1E-05 | 4.9E-08 | 7.2E-05 | 20.5 | 226.8 | 63.7 | 1.3 |
| IL6R-BP204/IL6R-L | E233D/P238D/P271G/K326A/A330R | 7.6E-09 | 4.5E-06 | 2.1E-05 | 2.5E-07 | 5.2E-05 | 17.6 | 82.7 | 12.2 | 5.8 |
| IL6R-BP205/IL6R-L | E233D/P238D/P271G/Y296D/A330R | 7.7E-09 | 3.5E-06 | 2.8E-05 | 1.6E-07 | 6.8E-05 | 21.8 | 176.1 | 19.4 | 4.5 |
| IL6R-BP206/IL6R-L | E233D/P238D/P271G/A330R | 8.2E-09 | 3.1E-06 | 2.4E-05 | 2.0E-07 | 6.9E-05 | 16.1 | 123.1 | 15.8 | 4.1 |
| IL6R-BP207/IL6R-L | E233D/P238D/A330R | 2.2E-08 | 1.9E-05 | 2.9E-05 | 8.4E-07 | 6.5E-05 | 23.0 | 34.5 | 3.7 | 25.1 |
| IL6R-BP208/IL6R-L | E233D/G237D/P238D/H268D/P271G/A330R | 1.9E-08 | 8.5E-07 | 8.3E-06 | 3.2E-08 | 5.3E-05 | 26.3 | 256.2 | 95.4 | 1.1 |
| IL6R-BP209/IL6R-L | G237D/P238D/H268D/P271G/K326A/A330R | 3.9E-08 | 1.2E-06 | 1.0E-05 | 5.1E-08 | 4.1E-05 | 22.7 | 195.3 | 60.4 | 1.5 |
| IL6R-BP210/IL6R-L | G237D/P238D/H268D/P271G/A330R | 6.5E-08 | 1.0E-06 | 9.5E-06 | 3.9E-08 | 4.6E-05 | 25.4 | 241.1 | 78.4 | 1.3 |
| IL6R-BP211/IL6R-L | E233D/P238D/H268D/P271G/K326A/A330R | 4.2E-09 | 4.1E-06 | 2.7E-05 | 2.2E-07 | 7.3E-05 | 18.5 | 120.5 | 13.8 | 5.4 |
| IL6R-BP212/IL6R-L | E233D/P238D/H268D/P271G/Y296D/A330R | 5.2E-09 | 3.5E-06 | 2.2E-05 | 1.7E-07 | 5.2E-05 | 21.1 | 133.3 | 18.7 | 4.5 |
| IL6R-BP213/IL6R-L | E233D/P238D/H268D/P271G/A330R | 4.1E-09 | 3.1E-06 | 2.4E-05 | 1.8E-07 | 6.3E-05 | 17.7 | 136.4 | 17.6 | 4.0 |
| IL6R-BP214/IL6R-L | E233D/L234Y/G237D/P238D/Y296D/K326D/A330K | 5.9E-08 | 1.7E-06 | 9.2E-06 | 1.2E-07 | 3.8E-05 | 14.5 | 78.0 | 26.2 | 2.2 |

[Example 13] Preparation of variants with enhanced FcγRIIb binding

As shown in Example 8, when enhancing the FcγRIIb binding, it is preferable that the FcγRIIb binding is enhanced while maximally suppressing the binding to other activating FcγRs. Thus, the present inventors additionally produced variants with enhanced FcγRIIb binding or improved selectivity to FcγRIIb by combining alterations that enhance the FcγRIIb binding or improving the selectivity to FcγRIIb. Specifically, the alterations described in Examples 9, 11, and 12 which were found to be effective when combined with alteration P238D, were combined with one another, on the basis of the P238D alteration which showed the excellent effect to enhance the FcγRIIb binding and to improve the selectivity to FcγRIIb.

Variants were produced by combining the Fc regions of IL6R-G1d (SEQ ID NO: 133) and IL6R-B3 (SEQ ID NO: 144) with alterations E233D, L234Y, G237D, S267Q, H268D, P271G, Y296D, K326D, K326A, A330R, and A330K described in Examples 9, 11, and 12 which were found to be effective when combined with alteration P238D. Using IL6R-L (SEQ ID NO: 135) as the antibody L chain, antibodies comprising the above-described variants in the heavy chain were expressed and purified according to the method described in Reference Examples 1 and 2. The resulting variants were respectively assessed for the binding to each FcγR (FcγRIa, FcγRIIaH, FcγRIIaR, FcγRIIb, or FcγRIIIaV) by the method described in Reference Example 25.

The KD of each variant to each FcγR is shown in Table 21. "Alteration" refers to an alteration introduced into IL6R-B3 (SEQ ID NO: 144). IL6R-B3/IL6R-L which is used as the template to produce each variant is indicated by asterisk (*). "KD (IIaR)/KD (IIb)" in the table shows the value obtained by dividing the KD of each variant for FcγRIIaR by the KD of each variant for FcγRIIb. The greater the value, the higher the selectivity to FcγRIIb. "KD (IIb) of parent polypeptide/KD (IIb) of altered polypeptide" shows the value obtained by dividing the KD value of IL6R-B3/IL6R-L for FcγRIIb by the KD value of each variant for FcγRIIb. Meanwhile, KD (IIaR) of parent polypeptide/KD (IIaR) of altered polypeptide shows the value obtained by dividing the KD value of IL6R-B3/IL6R-L for FcγRIIaR by the KD value of each variant for FcγRIIaR. In Table 21, the numeral in the gray-filled cells indicates that the binding of FcγR to IgG was concluded to be too weak to analyze correctly by kinetic analysis and thus was calculated using:

[Equation 2]

$$KD = C \bullet R_{max} / (R_{eq} - RI) - C$$

described in Reference Example 25.

[Table 21]

| VARIANT NAME | ALTERATION | KD AGAINST FcγRIa (mol/L) | KD AGAINST FcγRIIaR (mol/L) | KD AGAINST FcγRIIaH (mol/L) | KD AGAINST FcγRIIb (mol/L) | KD AGAINST FcγRIIIaV (mol/L) | KD (IIaR)/KD (IIb) | KD (IIb) OF PARENT POLYPEPTIDE/ KD (IIb) OF ALTERED POLYPEPTIDE | KD (IIaR) OF PARENT POLYPEPTIDE/ KD (IIaR) OF ALTERED POLYPEPTIDE |
|---|---|---|---|---|---|---|---|---|---|
| IL6R-G1d/IL6R-L | | 3.20E-10 | 1.00E-06 | 6.70E-07 | 2.60E-06 | 3.50E-07 | 0.4 | 1.2 | 1.1 |
| IL6R-B3/IL6R-L | * | 4.20E-10 | 1.10E-06 | 7.70E-07 | 3.10E-06 | 3.30E-07 | 0.3 | 1 | 1 |
| IL6R-BP648/IL6R-L | P238D | 1.10E-08 | 1.50E-05 | 4.00E-05 | 1.20E-06 | 7.10E-05 | 12.5 | 2.6 | 0.1 |
| IL6R-BP215/IL6R-L | G237D/P238D/H268D/P271G/Y296D/A330K | 4.30E-08 | 1.30E-06 | 1.40E-05 | 4.10E-08 | 6.70E-05 | 31.7 | 75.6 | 0.8 |
| IL6R-BP216/IL6R-L | G237D/P238D/S267Q/H268D/P271G/A330K | 6.20E-07 | 2.90E-06 | 2.60E-05 | 1.40E-07 | 5.30E-05 | 20.7 | 22.1 | 0.4 |
| IL6R-BP217/IL6R-L | G237D/P238D/S267Q/H268D/P271G/Y296D/A330K | 2.80E-06 | 3.60E-06 | 2.80E-05 | 1.50E-07 | 6.00E-05 | 24 | 20.7 | 0.3 |
| IL6R-BP218/IL6R-L | G237D/P238D/H268D/P271G/K326D/A330K | 3.70E-08 | 1.50E-06 | 1.20E-05 | 7.60E-08 | 3.80E-05 | 19.7 | 40.8 | 0.7 |
| IL6R-BP219/IL6R-L | L234Y/G237D/P238D/H268D/P271G/A330K | 4.60E-08 | 6.10E-07 | 2.50E-06 | 3.40E-08 | 2.90E-05 | 17.9 | 91.2 | 1.8 |
| IL6R-BP220/IL6R-L | E233D/G237D/P238D/H268D/P271G/Y296D/A330K | 2.00E-08 | 1.10E-06 | 1.20E-05 | 3.60E-08 | 5.80E-05 | 30.6 | 86.1 | 1 |
| IL6R-BP221/IL6R-L | L234Y/G237D/P238D/Y296D/K326A/A330R | 1.30E-07 | 7.10E-07 | 2.50E-06 | 2.80E-08 | 4.60E-05 | 25.4 | 110.7 | 1.5 |
| IL6R-BP222/IL6R-L | L234Y/G237D/P238D/P271G/K326A/A330R | 5.10E-08 | 7.10E-07 | 2.60E-06 | 3.40E-08 | 4.70E-05 | 20.9 | 91.2 | 1.5 |
| IL6R-BP223/IL6R-L | L234Y/G237D/P238D/H268D/P271G/K326A/A330R | 2.70E-08 | 6.00E-07 | 2.80E-06 | 2.50E-08 | 3.20E-05 | 24 | 124 | 1.8 |
| IL6R-BP224/IL6R-L | L234Y/G237D/P238D/S267Q/H268D/P271G/K326A/A330R | 6.20E-09 | 4.50E-07 | 9.50E-06 | 3.50E-08 | 4.10E-05 | 12.9 | 88.6 | 2.4 |
| IL6R-BP225/IL6R-L | L234Y/G237D/P238D/K326D/A330R | 9.50E-08 | 6.90E-07 | 2.80E-06 | 3.50E-08 | 3.20E-05 | 19.7 | 88.6 | 1.6 |
| IL6R-BP226/IL6R-L | L234Y/G237D/P238D/P271G/K326D/A330R | 5.20E-08 | 5.70E-07 | 2.40E-06 | 3.30E-08 | 3.60E-05 | 17.3 | 93.9 | 1.9 |
| IL6R-BP227/IL6R-L | L234Y/G237D/P238D/H268D/P271G/K326D/A330R | 2.70E-08 | 6.20E-07 | 2.90E-06 | 3.20E-08 | 2.60E-05 | 19.4 | 96.9 | 1.8 |
| IL6R-BP228/IL6R-L | L234Y/G237D/P238D/S267Q/H268D/P271G/K326D/A330R | 5.50E-09 | 4.20E-07 | 1.10E-05 | 4.00E-08 | 3.20E-05 | 10.5 | 77.5 | 2.6 |
| IL6R-BP229/IL6R-L | E233D/L234Y/G237D/P238D/P271G/K326A/A330R | 5.60E-08 | 8.10E-07 | 3.30E-06 | 4.20E-08 | 3.70E-05 | 19.3 | 73.8 | 1.4 |
| IL6R-BP230/IL6R-L | E233D/G237D/P238D/H268D/P271G/Y296D/A330R | 1.40E-08 | 5.70E-07 | 9.60E-06 | 2.10E-08 | 6.70E-05 | 27.1 | 147.6 | 1.9 |
| IL6R-BP231/IL6R-L | G237D/P238D/H268D/P271G/Y296D/A330R | 9.40E-09 | 7.40E-07 | 1.10E-05 | 2.30E-08 | 4.00E-05 | 32.2 | 134.8 | 1.5 |
| IL6R-BP232/IL6R-L | L234Y/G237D/P238D/P271G/K326A/A330K | 7.60E-08 | 8.40E-07 | 3.30E-06 | 5.60E-08 | 4.50E-05 | 15 | 55.4 | 1.3 |
| IL6R-BP233/IL6R-L | L234Y/G237D/P238D/P271G/A330K | 7.00E-08 | 6.90E-07 | 2.80E-06 | 3.70E-08 | 5.10E-05 | 18.6 | 83.8 | 1.6 |
| IL6R-BP234/IL6R-L | E233D/L234Y/G237D/P238D/S267Q/H268D/P271G/Y296D/K326D/A330K | 6.50E-09 | 1.20E-06 | 2.00E-05 | 1.20E-07 | 3.10E-05 | 10 | 25.8 | 0.9 |
| IL6R-BP235/IL6R-L | E233D/L234Y/G237D/P238D/H268D/P271G/Y296D/K326D/A330R | 3.50E-09 | 6.80E-07 | 7.50E-06 | 4.40E-08 | 2.50E-05 | 15.5 | 70.5 | 1.6 |
| IL6R-BP236/IL6R-L | E233D/L234Y/G237D/P238D/S267Q/H268D/P271G/Y296D/K326D/A330R | 7.70E-09 | 8.40E-07 | 1.90E-05 | 6.50E-08 | 3.90E-05 | 12.9 | 47.7 | 1.3 |
| IL6R-BP237/IL6R-L | E233D/L234Y/G237D/P238D/S267Q/H268D/P271G/Y296D/K326A/A330K | 4.10E-09 | 1.10E-06 | 1.90E-05 | 1.00E-07 | 3.50E-05 | 11 | 31 | 1 |
| IL6R-BP238/IL6R-L | E233D/L234Y/G237D/P238D/H268D/P271G/Y296D/K326A/A330R | 2.40E-09 | 6.40E-07 | 7.00E-06 | 3.60E-08 | 2.70E-05 | 17.8 | 86.1 | 1.7 |
| IL6R-BP239/IL6R-L | E233D/L234Y/G237D/P238D/S267Q/H268D/P271G/Y296D/K326A/A330R | 7.60E-09 | 8.10E-07 | 1.70E-05 | 6.00E-08 | 4.80E-05 | 13.5 | 51.7 | 1.4 |
| IL6R-BP240/IL6R-L | E233D/G237D/P238D/S267Q/H268D/P271G/A330R | 7.60E-09 | 1.50E-06 | 2.60E-05 | 9.50E-08 | 5.20E-05 | 15.8 | 32.6 | 0.7 |
| IL6R-BP241/IL6R-L | E233D/G237D/P238D/H268D/P271G/K326D/A330R | 1.10E-09 | 6.80E-07 | 9.00E-06 | 4.50E-08 | 3.10E-05 | 15.1 | 68.9 | 1.6 |
| IL6R-BP242/IL6R-L | E233D/G237D/P238D/H268D/P271G/K326A/A330R | 1.90E-09 | 7.50E-07 | 8.60E-06 | 5.10E-08 | 2.90E-05 | 14.7 | 60.8 | 1.5 |
| IL6R-BP243/IL6R-L | E233D/L234Y/G237D/P238D/H268D/P271G/A330R | 3.00E-09 | 5.40E-07 | 6.00E-06 | 3.60E-08 | 2.50E-05 | 15 | 86.1 | 2 |
| IL6R-BP244/IL6R-L | E233D/G237D/P238D/S267Q/H268D/P271G/Y296D/A330R | 7.80E-09 | 1.80E-06 | 2.60E-05 | 1.10E-07 | 4.60E-05 | 16.4 | 28.2 | 0.6 |
| IL6R-BP245/IL6R-L | E233D/G237D/P238D/S267Q/H268D/P271G/Y296D/K326D/A330R | 6.30E-09 | 1.40E-06 | 2.50E-05 | 8.30E-08 | 3.90E-05 | 16.9 | 37.3 | 0.8 |
| IL6R-BP246/IL6R-L | E233D/G237D/P238D/S267Q/H268D/P271G/Y296D/K326A/A330R | 8.00E-09 | 1.60E-06 | 2.30E-05 | 9.20E-08 | 4.40E-05 | 17.4 | 33.7 | 0.7 |
| IL6R-BP247/IL6R-L | E233D/G237D/P238D/H268D/P271G/Y296D/K326D/A330R | 7.50E-09 | 8.10E-07 | 1.20E-05 | 3.70E-08 | 4.40E-05 | 21.9 | 83.8 | 1.4 |
| IL6R-BP248/IL6R-L | E233D/G237D/P238D/H268D/P271G/Y296D/K326A/A330R | 1.70E-09 | 8.20E-07 | 1.10E-05 | 3.50E-08 | 4.40E-05 | 23.4 | 88.6 | 1.3 |
| IL6R-BP249/IL6R-L | E233D/L234Y/G237D/P238D/H268D/P271G/Y296D/A330R | 7.00E-09 | 6.20E-07 | 7.20E-06 | 3.70E-08 | 2.80E-05 | 16.8 | 83.8 | 1.8 |

When taking the binding to each FcγR by IL6R-B3/IL6R-L resulting from introducing the K439E alteration into the H chain of IL6R-G1d/IL6R-L containing the sequence of native human IgG1 as 1, the binding of IL6R-G1d/IL6R-L to FcγRIa as 1.3 times; the binding of IL6R-G1d/IL6R-L to FcγRIIaR was 1.1 times; the binding of IL6R-G1d/IL6R-L to FcγRIIaH was 1.1 times, the binding of IL6R-G1d/IL6R-L to FcγRIIb binding was 1.2 times, and the binding of IL6R-G1d/IL6R-L to FcγRIIIaV was 0.9 times. Thus, for any given FcγR type, the binding of IL6R-B3/IL6R-L to FcγR was comparable to the binding of IL6R-G1d/IL6R-L to FcγR. Thus, the comparison of the binding of each variant to each FcγR with that of IL6R-B3/IL6R-L prior to introduction of the alteration is assumed to be equivalent to the comparison of the binding of each variant to each FcγR with the binding to each FcγR by IL6R-G1d/IL6R-L containing the sequence of native human IgG1. For this reason, in the subsequent Examples below, the binding activity of each variant to each FcγR will be compared to that to each FcγR by IL6R-B3/IL6R-L prior to introduction of the alteration. Table 21 shows that all the variants have increased FcγRIIb binding activity as compared to IL6R-B3 prior to introduction of the alteration. The binding activity of IL6R-BF648/IL6R-L, which was the lowest, was increased by 2.6 times, while the binding activity of IL6R-BP230/IL6R-L, which is the highest, was increased by 147.6 times. Regarding the value of KD (IIaR)/KD (IIb) that represents the selectivity, the value for IL6R-BP234/IL6R-L, which was the lowest, was 10.0, while the value for IL6R-BP231/IL6R-L, which was the highest, was 32.2. Compared to 0.3 for IL6R-B3/IL6R-L prior to introduction of the alteration, these values imply that all the variants have improved selectivity. All the variants showed lower binding activity to FcγRIa, FcγRIIaH, and FcγRIIIaV than that of IL6R-B3/IL6R-L prior to introduction of the alteration.

[Example 14] X-ray crystal structure analysis of the complexes of FcγRIIb extracellular region or FcγRIIaR extracellular region and Fc region with enhanced FcγRIIb binding

As shown in Example 13, the FcγRIIb binding of variant IL6R-BP230/IL6R-L, whose FcγRIIb binding was enhanced most, was enhanced to about 150 times as compared to IL6R-B3/IL6R-L prior to introduction of the alteration, while the enhancement of its FcγRIIaR binding was suppressed to an extent of about 1.9 times. Thus, IL6R-BP230/IL6R-L is a variant excellent in both FcγRIIb binding and selectivity. However, the present inventors sought a possibility to create more preferable variants with further enhanced FcγRIIb binding while suppressing the FcγRIIaR binding as possible.

As shown in Fig. 30 described in Example 10, in the Fc region with alteration P238D, Asp at position 270 (EU numbering) in its CH2 domain B forms a tight electrostatic interaction with Arg at position 131 in FcγRIIb. This amino acid residue at position 131 is His in FcγRIIIa and FcγRIIaH, while it is Arg in FcγRIIaR like in FcγRIIb. Thus, there is no difference between FcγRIIaR and FcγRIIb in terms of the interaction of the amino acid residue at position 131 with Asp at position 270 (EU numbering) in the CH2 domain B. This is assumed to be a major factor for the poor selectivity between the FcγRIIb binding and FcγRIIaR binding of the Fc region.

On the other hand, the extracellular regions of FcγRIIa and FcγRIIb are 93% identical in amino acid sequence, and thus they share very high homology. Based on the crystal structure analysis of the complex of the Fc region of native IgG1 (hereinafter abbreviated as Fc (WT)) and the extracellular region of FcγRIIaR (J. Imunol. (2011) 187, 3208-3217), a difference found around the interface between the two interacting with each other was only three amino acids (Gln127, Leu132, Phe160) between FcγRIIaR and FcγRIIb. Thus, the present inventors predicted that it was extremely difficult to improve the selectivity of the Fc region between the FcγRIIb binding and FcγRIIaR binding.

In this context, the present inventors conceived that, in order to further enhance the FcγRIIb-binding activity of the Fc region, and to improve the selectivity of the Fc regions for the binding to FcγRIIb and FcγRIIaR binding, it was important to clarify subtle differences between Fc region-FcγRIIb interaction and Fc region-FcγRIIaR interaction by analyzing not only the three-dimensional structure of the complex of the Fc region with enhanced FcγRIIb binding and the extracellular region of FcγRIIb but also the three-dimensional structure of the complex of the Fc region with enhanced FcγRIIb binding and the extracellular region of FcγRIIaR. First, the present inventors analyzed the X-ray crystal structure of the complex of the extracellular region of FcγRIIb or FcγRIIaR and Fc (P208) resulting from eliminating the K439E alteration from the Fc region of IL6R-BP208/IL6R-L created as described in Example 12, which was the variant used as the basis in producing IL6R-BP230/IL6R-L.

(14-1) X-ray crystal structure analysis of the complex of Fc (P208) and the extracellular region of FcγRIIb

[Expression and purification of Fc (P208)]

Fc (P208) was prepared as described below. First, IL6R-P208 was produced by substituting Lys for Glu at position 439 (EU numbering) in IL6R-BP208, as is in the case of the sequence of native human IgG1. Then, the gene sequence of Fc (P208), which was cloned by PCR from Glu at position 216 (EU numbering) to the C terminus using as a template a DNA encoding a variant with a substitution of Ser for Cys at position 220 (EU numbering), was cloned. Expression vector construction, expression, and purification were achieved according to the method described in Reference Examples 1 and 2. Meanwhile, Cys at position 220 (EU numbering) in ordinary IgG1 forms a disulfide bond to a Cys in the L chain. When preparing the Fc region alone, the L chain is not expressed in combination. Thus, Cys at position 220 was substituted by Ser to avoid unnecessary disulfide bond formation.

[Expression and purification of the extracellular region of FcγRIIb]

The extracellular region of FcγRIIb was prepared according to the method described in Reference Example 25.

[Purification of the Fc (P208)/FcγRIIb extracellular region complex]

0.15 mg of the purified product of Endo F1 (Protein Science (1996) 5, 2617-2622) expressed in *E. coli* as a fusion protein with glutathione S-transferase was added 1.5 mg of a crystallization sample of the extracellular region of FcγRIIb. This added sample in 0.1 M Bis-Tris buffer (pH 6.5) was allowed to stand at room temperature for three days to cleave off N-type sugar chains except N-acetylglucosamine directly linked to the Asn in the sample of the extracellular region of FcγRIIb. Then, the sample of the extracellular region of FcγRIIb subjected to the sugar chain cleavage treatment was concentrated with a 5000MWCO ultrafiltration filter, and purified by chromatography with a gel filtration column (Superdex200 10/300) equilibrated with 20 mM HEPES (pH7.5)/0.1 M NaCl. Next, Fc (P208) was added in such a way that the extracellular region of FcγRIIb is present in a slightly excessive molar ratio. After concentrating with a 10000MWCO ultrafiltration filter, the purified fraction of the extracellular region of FcγRIIb subjected to the sugar chain cleavage was purified by chromatography with a gel filtration column (Superdex200 10/300) equilibrated with 25 mM HEPES (pH 7.5)/0.1 M NaCl. The purified fraction prepared as described above was used as a sample of Fc (P208)/FcγRIIb extracellular region complex in the subsequent assessment.

[Crystallization of the complex of Fc (P208)/FcγRIIb extracellular region]

A sample of Fc (P208)/FcγRIIb extracellular region complex concentrated to about 10 mg/ml with a 10000MWCO ultrafiltration filter was crystallized using the hanging drop vapor diffusion method in combination with the seeding method. VDXm plate (Hampton Research) was used for crystallization. Using a reservoir solution of 0.1 M Bis-Tris (pH 6.5)/19%(w/v) PEG3350/0.2 M potassium phosphate dibasic, crystallization drops were prepared at a mixing ratio of reservoir solution : crystallization sample = 0.85 μl : 0.85 μl. Crystals of the complex obtained under the same condition were crushed with Seed Bead (Hampton Research) to prepare a seed crystal solution. The crystallization drops were added with 0.15 μl of a diluted solution prepared from the seed solution and allowed to stand at 20°C in sealed reservoir wells. This yielded plate-like crystals.

[X-ray diffraction data measurements from an Fc (P208)/FcγRIIb extracellular region complex crystal]

A single crystal of Fc (P208)/FcγRIIb extracellular region complex prepared as described above was soaked into a solution of 0.1 M Bis-Tris (pH 6.5)/24% (w/v) PEG3350/0.2 M potassium phosphate dibasic/20% (v/v) ethylene glycol. Then, the single crystal was fished out of the solution using a pin with attached tiny nylon loop, and frozen in liquid nitrogen. X-ray diffraction data of the single crystal was collected with Spring-8 BL32XU. During the measurement, the crystal was constantly placed in a nitrogen stream at -178°C to maintain in a frozen state. A total of 300 X-ray diffraction images of the single crystal were collected using CCD detector MX-225HE (RAYONIX) attached to a beam line with rotating the single crystal 0.6° at a time. Based on the obtained diffraction images, lattice constant determination, diffraction spot indexing, and diffraction data processing were performed using programs Xia2 (J. Appl. Cryst. (2010) 43, 186-190), XDS Package (Acta Cryst. (2010) D66, 125-132) and Scala (Acta Cryst. (2006) D62, 72-82). Finally, the diffraction intensity data of the single crystal up to 2.81 Å resolution was obtained. The crystal belongs to the space group $C222_1$ with lattice constant a = 156.69 Å, b = 260.17 Å, c = 56.85 Å, α = 90°, β = 90°, and γ = 90°.

[X-ray crystal structure analysis of Fc (P208)/FcyRIIb extracellular region complex]

The structure of Fc (P208)/FcγRIIb extracellular region complex was determined by a molecular replacement method using program Phaser (J. Appl. Cryst. (2007) 40, 658-674). The number of complexes in an asymmetrical unit was estimated to be one from the size of the obtained crystal lattice and the molecular weight of Fc (P208)/FcγRIIb extracellular region complex. The segments spanning the amino acid residues at positions 239-340 of the A chain and at positions 239-340 of the B chain, which were retrieved as an independent coordinate from the structural coordinate of PDB code: 3SGJ for the crystal structure of Fc (WT)/FcγRIIIa extracellular region complex, were used as a model for searching the CH2 domain of the Fc region. Likewise, the segments spanning the amino acid residues at positions 341-444 of the A chain and at positions 341-443 of the B chain, which were retrieved as a coordinate from the structural coordinate of PDB code: 3SGJ, were used as a model for searching the CH3 domain of the Fc region. Finally, the segment spanning the amino acid residues at positions 6-178 of the A chain, which was retrieved from the structural coordinate of PDB code: 2FCB for the crystal structure of the extracellular region of FcγRIIb, was used as a model for searching Fc (P208). The present inventors tried to determine the orientations and positions of the respective search models of the CH3 domain of the Fc region, the extracellular region of FcγRIIb, and the CH2 domain of the Fc region in the crystal lattices based on the rotation function and translation function, but failed to determine the position of one of the CH2 domains. Then, with reference to the crystal structure of the complex of Fc (WT)/FcγRIIIa extracellular region, the position of the last CH2 domain A was determined from an electron density map that was calculated based on the phase determined for the remaining three parts. Thus, the present inventors obtained an initial model for the crystal structure of the complex of Fc (P208)/FcγRIIb extracellular region. The crystallographic reliability factor R value of the structural model for the data of diffracted intensity at 25 to 3.0 Å was 42.6% and Free R value was 43.7% after rigid body refinement where the two CH2 domains and two CH3 domains of the Fc region, and the extracellular region of FcγRIIb were allowed to deviate from the obtained initial structural model. Then, structural model refinement was achieved by repeating structural refinement using program REFMAC5 (Acta Cryst. (2011) D67, 355-367) followed by revision of the structural model performed using program Coot (Acta Cryst. (2010) D66, 486-501) with reference to the electron density maps where the coefficients 2Fo-Fc and Fo-Fc were calculated using experimentally determined structural factor Fo, structural factor Fc region calculated according to the structural model, and the phases calculated according to the structural model. Then, further refinement was carried out based on the electron density maps with coefficients of 2Fo-Fc and Fo-Fc by integrating water molecules into the structural model. With 27259 diffracted intensity data at 25 to 2.81 Å resolution, ultimately the crystallographic reliability factor R value was 24.5% and free R value was 28.2% for the structural model comprising 4786 non-hydrogen atoms.

The three-dimensional structure of the complex of Fc (P208)/FcγRIIb extracellular region was determined at a resolution of 2.81 Å by structure analysis. The structure obtained by the analysis is shown in Fig. 35. FcγRIIb extracellular region was revealed to be bound and sandwiched between the two CH2 domains of the Fc region, which resembles the three-dimensional structures of the previously analyzed complexes of Fc (WT), which is the Fc of native IgG, and each of the extracellular regions of FcγRIIIa (Proc. Natl. Acad. Sci. USA (2011) 108, 12669-126674), FcγRIIIb (Nature (2000) 400, 267-273; J. Biol. Chem. (2011) 276, 16469-16477), and FcγRIIa (J. Immunol. (2011) 187 (6), 3208-3217).

A close observation of the complex of Fc (P208)/FcγRIIb extracellular region revealed a change in the loop structure at positions 233 to 239 (EU numbering) following the hinge region in the CH2 domain A of the Fc region due to an influence of the introduced the G237D and P238D alterations as compared to the complex of Fc (WT)/FcγRIIaR extracellular region (Fig. 36). This leads to that the main chain of Asp at position 237 (EU numbering) in Fc (P208) formed a tight hydrogen bond to the side chain of Tyr at position 160 in FcγRIIb (Fig. 37). In both FcγRIIaH and FcγRIIaR, the amino acid residue at position 160 is Phe, which is incapable of forming such a hydrogen bond. This suggests that the above described hydrogen bond has important contribution to the enhancement of the FcγRIIb binding and the acquisition of the FcγRIIa binding selectivity of Fc (P208), *i.e.,* improvement of the FcγRIIb-binding activity and reduction of FcγRIIa-binding activity of Fc (P208).

On the other hand, the side chain of Asp at position 237 (EU numbering) in Fc (P208) forms neither particularly significant interaction in the FcγRIIb binding nor interaction with other residues within the Fc region. Ile at position 332, Glu at position 333, and Lys at position 334 (EU numbering) in the Fc region are located close to Asp at position 237 (EU numbering) (Fig. 38). When the amino acid residues of these positions are substituted by hydrophilic residues to form an interaction with the side chain of Asp at position 237 (EU numbering) in Fc (P208) and the loop structure can be stabilized by the interaction, this can lead to reduction of the entropic energy loss due to the hydrogen bonding between the Fc region and Tyr at position 160 in FcγRIIb and thereby to an increase in the binding free energy, *i.e.,* an increase in the binding activity.

When the X-ray crystal structure of the complex of Fc (P238D) with the P238D alteration and FcγRIIb extracellular region described in Example 10 is compared to the X-ray crystal structure of the complex of Fc (P208) and FcγRIIb extracellular region, deviations are observed at five portions in Fc (P208) as compared to Fc (P238D) and most of the changes are seen only at the side chain level. Meanwhile, a positional deviation at the main chain level due to the Pro-to-Gly alteration at position 271 (EU numbering) is also observed in the CH2 domain B of the Fc region, and in addition there is a structural change in the loop at positions 266 to 270 (EU numbering) (Fig. 39). As described in Example 11, it is suggested that, when Asp at position 270 (EU numbering) in Fc (P238D) forms a tight electrostatic interaction with Arg at position 131 in FcγRIIb, the interaction can induce stereochemical stress at Pro at position 271 (EU numbering). The experiment described herein suggests that the structural change observed with the alteration to Gly for the amino acid at position 271 (EU numbering) is assumed to be a result of elimination of the structural distortion accumulated at Pro prior to the alteration and the elimination results in an increase in the free energy for the FcγRIIb binding, *i.e.,* an increase in the binding activity.

Furthermore, it was demonstrated that, due to the change of the loop structure at positions 266 to 271 (EU numbering), Arg at position 292 (EU numbering) underwent a structural change while being in two states. In this case, the electrostatic interaction (Fig. 39) formed between Arg at position 292 (EU numbering) and Asp at position 268 (EU numbering) which is an altered residue in Fc (P208) can contribute to the stabilization of the loop structure. Since the electrostatic interaction formed between Asp at position 270 (EU numbering) in the loop and Arg at position 131 in FcγRIIb largely contribute to the binding activity of Fc (P208) to FcγRIIb, the stabilization of the loop structure in the binding conformation was likely to reduce the entropic energy loss upon binding. Thus, the alteration is expected to result in an increase in the binding free energy, *i.e.,* an increase in the binding activity.

Moreover, the possibility of alteration to increase the activity was scrutinized based on the result of structural analysis. Ser at position 239 (EU numbering) was found as a candidate for the site to introduce alteration. As shown in Fig. 40, Ser at position 239 (EU numbering) in the CH2 domain B is present at the position toward which Lys at position 117 in FcγRIIb extends most naturally in structure. However, since the electron density was not observed for Lys at position 117 in FcγRIIb by the analysis described above, the Lys has no definite structure. In this situation, Lys117 is likely to have only a limited effect on the interaction with Fc (P208). When Ser at position 239 (EU numbering) in the CH2 domain B is substituted with negatively charged Asp or Glu, such an alteration is expected to cause an electrostatic interaction with the positively charged Lys at position 117 in FcyRIIb, thereby resulting in improved FcyRIIb-binding activity.

On the other hand, an observation of the structure of Ser at position 239 (EU numbering) in the CH2 domain A revealed that, by forming a hydrogen bond to the main chain of Gly at position 236 (EU numbering), the side chain of this Ser stabilized the loop structure at positions 233 to 239, including Asp at position 237 (EU numbering) that forms a hydrogen bond to the side chain of Tyr at position 160 in FcγRIIb, following the hinge region (Fig. 37). The stabilization of the loop structure in the binding conformation can reduce the entropic energy loss upon binding, and result in an increase in the binding free energy, *i.e.,* an improvement of the binding activity. Meanwhile, when Ser at position 239 (EU numbering) in the CH2 domain A is substituted with Asp or Glu, the loop structure can become unstable due to loss of the hydrogen bond to the main chain of Gly at position 236 (EU numbering). In addition, the alteration can result in electrostatic repulsion to Asp at position 265 (EU numbering) in close proximity, leading to further destabilization of the loop structure. The energy for the destabilization corresponds to loss of free energy for the FcγRIIb binding, which can result in reduction in the binding activity.

(14-2) X-ray crystal structure analysis of the complex of Fc (P208) and FcγRIIaR extracellular region

[Expression and purification of the extracellular region of FcγRIIaR]

The extracellular region of FcγRIIaR was prepared according to the method described in Reference Example 2.

[Purification of the complex of Fc (P208)/FcγRIIaR type extracellular region]

1.5 mg of purified sample of the extracellular region of FcγRIIaR was added with 0.15 mg of the purified product of Endo F1 (Protein Science (1996) 5, 2617-2622) expressed in *E. coli* as a fusion protein with S-transferase, 20 μl of 5 U/ml Endo F2 (QA-bio), and 20 μl of 5 U/ml Endo F3 (QA-bio). After 9 days of incubation at room temperature in 0.1 M Na acetate buffer (pH 4.5), the sample was further added with 0.07 mg of the above-described Endo F1, 7.5 μl of the above-described Endo F2, and 7.5 μl of the above-described Endo F3, and was incubated for three days to cleave off N-type sugar chains except N-acetylglucosamine directly linked to the Asn in the sample of the extracellular region of FcγRIIa R. Then, the sample of the extracellular region of FcγRIIaR concentrated with a 10000MWCO ultrafiltration filter and subjected to the above-described sugar chain cleavage treatment was purified by chromatography with a gel filtration column (Superdex200 10/300) equilibrated with 25 mM HEPES (pH 7)/0.1M NaCl. Next, Fc (P208) was added in such a way that the extracellular region of FcγRIIaR is present in a slightly excessive molar ratio. After concentrating with a 10000MWCO ultrafiltration filter, the purified fraction of the extracellular region of FcγRIIaR subjected to the above-described sugar chain cleavage treatment was purified by chromatography with a gel filtration column (Superdex200 10/300) equilibrated with 25 mM HEPES (pH 7)/0.1 M NaCl. The purified fraction prepared as described above was used as a sample of Fc (P208)/FcγRIIaR type extracellular region complex in the subsequent assessment.

[Crystallization of the complex of Fc (P208)/FcγRIIaR type extracellular region]

A sample of Fc (P208)/FcγRIIa R type extracellular region complex concentrated to about 10 mg/ml with a 10000MWCO ultrafiltration filter was crystallized using the sitting drop vapor diffusion method. Using a reservoir solution of 0.1 M Bis-Tris (pH 7.5)/26% (w/v) PEG3350/ 0.2 M ammonium sulfate, crystallization drops were prepared at a mixing ratio of reservoir solution : crystallization sample = 0.8 μl : 1.0 μl. The drops were tight sealed and allowed to stand at 20°C. This yielded plate-like crystals.

[X-ray diffraction data measurement from Fc (P208)/FcγRIIaR extracellular region complex crystal]

A single crystal of Fc (P208)/FcγRIIaR extracellular region complex prepared as described above was soaked into a solution of 0.1 M Bis-Tris (pH 7.5)/27.5% (w/v) PEG3350/0.2 M ammonium sulfate/20% (v/v) glycerol. Then, the crystal was fished out of the solution using a pin with attached tiny nylon loop, and frozen in liquid nitrogen. X-ray diffraction data of the single crystal was collected from Photon Factory BL-17A of the synchrotron radiation institution in the High Energy Accelerator Research Organization. The crystal was constantly placed in a nitrogen stream at -178°C to maintain in a frozen state during the measurement. A total of 225 X-ray diffraction images of the single crystal were collected using CCD detector Quantum 315r (ADSC) equipped to the beam line with rotating the single crystal at 0.6° at a time. Based on the obtained diffraction images, lattice constant determination, diffraction spot indexing, and diffraction data processing were performed using programs Xia2 (J. Appl. Cryst. (2010) 43, 186-190), XDS Package (Acta Cryst. (2010) D66, 125-132), and Scala (Acta Cryst. (2006) D62, 72-82). Finally, diffraction intensity data up to 2.87 Å resolution was obtained. The crystal belongs to the space group $C222_1$ with lattice constant a = 154.31 Å, b = 257.61 Å, c = 56.19 Å, $\alpha$ = 90°, $\beta$ = 90°, and $\gamma$ = 90°.

[X-ray crystal structure analysis of Fc (P208)/FcγRIIaR type extracellular region complex]

The structure of Fc (P208)/FcγRIIaR type extracellular region complex was determined by a molecular replacement method using program Phaser (J. Appl. Cryst. (2007) 40, 658-674). The number of complexes in an asymmetrical unit was estimated to be one from the size of the obtained crystal lattice and the molecular weight of Fc (P208)/FcγRIIaR extracellular region complex. Using, as a search model, the crystallographic structure of Fc (P208)/FcγRIIb extracellular region complex obtained as described in Example (14-1), the orientation and position of Fc (P208)/FcγRIIaR extracellular region complex in the crystal lattices were determined based on the rotation function and translation function. The crystallographic reliability factor R value of the structural model for the data of diffracted intensity at 25 to 3.0 Å was 38.4% and Free R value was 30.0% after rigid body refinement where the two CH2 domains and two CH3 domains of the Fc region, and the extracellular region of FcγRIIaR were allowed to independently deviate from the obtained initial structural model. Then, structural model refinement was achieved by repeating structural refinement using program REFMAC5 (Acta Cryst. (2011) D67, 355-367) followed by revision of the structural model performed using program Coot (Acta Cryst. (2010) D66, 486-501) with reference to the electron density maps where the coefficients Fo-Fc and 2Fo-Fc were calculated using experimentally determined structural factor Fo, structural factor Fc calculated according to the model, and the phases calculated according to the model. Finally, further refinement was carried out based on the electron density maps with coefficients Fo-Fc and 2Fo-Fc by integrating water molecules into the structural model. With 24838 diffracted intensity data at 25 to 2.87 Å resolution, ultimately the crystallographic reliability factor R value was 26.3% and free R value was 38.0% for the structural model comprising 4758 non-hydrogen atoms.

The three-dimensional structure of the complex of Fc (P208)/FcγRIIaR extracellular region was determined at a resolution of 2.87 Å by structure analysis. A comparison of the crystal structure between the complex of Fc (P208)/FcγRIIaR type extracellular region and the complex of Fc (P208)/FcγRIIb extracellular region described in Example (14-1) detected almost no difference at the level of overall structure (Fig. 41), reflecting the very high amino acid identity between the two Fcγ receptors.

However, a precise observation of the structures at the electron density level detected some differences that can lead to improvement of the selectivity between the FcyRIIb binding and the FcγRIIaR binding of the Fc region. The amino acid residue at position 160 in FcγRIIaR is not Tyr but Phe. As shown in Fig. 42, the hydrogen bond between the main chain of the amino acid residue at position 237 (EU numbering) in the CH2 domain A of the Fc region and Tyr at position 160 in FcγRIIb, though formed upon binding between FcγRIIb and the Fc region with alteration P238D, is expected not to be formed upon binding between FcγRIIb and the Fc region with alteration P238D. The absence of the hydrogen bond formation can be a major factor for improving the selectivity between the FcγRIIb binding and the FcγRIIaR binding of the Fc region introduced with alteration P238D. Further comparison at the electron density level showed that, in the Fc region/FcγRIIb complex, electron density was clearly observable for the side chains of Leu at positions 235 (EU numbering) and 234 (EU numbering), whereas the electron density of the side chains was unclear in the Fc region/FcγRIIaR complex. This suggests that the loop near position 237 (EU numbering) fluctuates due to the reduced FcγRIIaR interaction around this position. Meanwhile, a structural comparison of the CH2 domain B of the Fc region (Fig. 43) in same region revealed that, in the complex of the Fc region and FcγRIIb, electron density was observable up to Asp at position 237 (EU numbering), whereas, in the complex structure of the Fc region and FcγRIIaR, electron density was observable up to three residues prior to Asp at position 237 (EU numbering), *i.e.,* up to around Leu at position 234 (EU numbering), suggesting that FcγRIIaR binding forms an interaction over a larger region as compared to the FcγRIIb binding. The finding described above suggests the possibility that, in the CH2 domain A of the Fc region, the region from position 234 to 238 (EU numbering) has a large contribution to the binding between the Fc region and FcγRIIb, while in the CH2 domain B of the Fc region the region from position 234 to 238 (EU numbering) has a large contribution to the binding between the Fc region and FcγRIIaR.

[Example 15] Fc variants for which alteration sites were determined based on crystal structure

As described in Example 14, Asp at position 268 (EU numbering) was suggested to electrostatically interact with Arg at position 292 (EU numbering) (Fig. 39) as a result of the local structural change due to introduction of the alteration P271G in domain B of the variant with enhanced FcγRIIb binding (P208). There is a possibility that the loop structure at positions 266 to 271 (EU numbering) is stabilized by the formation of the interaction, resulting in enhancement of the FcγRIIb binding. Thus, the present inventors assessed whether the FcγRIIb binding of the variant could be enhanced by additional stabilization of its loop structure due to enhancement of the electrostatic interaction by substituting Glu for Asp at position 268 (EU numbering) in the variant. On the other hand, as shown in Fig. 38, Tyr at position 160 (EU numbering) in FcγRIIb interacts with the main chain of Asp at position 237 (EU numbering) in domain A of P208. Meanwhile, the side chain of Asp at position 237 (EU numbering) is located close to Ile at position 332, Glu at position 333, and Lys at position 334 (EU numbering) in the molecule without forming any particularly significant interaction. Thus, the present inventors also assessed whether the interaction with Tyr at position 160 in FcγRIIb can be enhanced through stabilization of the loop structure at positions 266 to 271 (EU numbering) due to increased interaction with the side chain of Asp at position 237 (EU numbering) by substituting hydrophilic amino acid residues at the positions described above.

Variants introduced with each of the alterations H268E, I332T, I332S, I332E, I332K, E333K, E333R, E333S, E333T, K334S, K334T, and K334E that were found based on the structural information, were produced using as a template IL6R-BP230/IL6R-L prepared as described in Example 13, which showed excellent selectivity to FcγRIIb. IL6R-L (SEQ ID NO: 135) was used as the antibody L chain. Antibodies containing the light chain of IL6R-L and the above-described heavy chain variants were expressed and purified according to the method described in Reference Examples 1 and 2. The purified antibodies were assessed for their binding to each FcγR (FcγRIa, FcγRIIaH, FcγRIIaR, FcγRIIb, or FcγRIIIaV) by the method described in Reference Example 25.

The KD of each variant to each FcγR is shown in Table 22. In the table, "alteration" refers to an alteration introduced into IL6R-BP3 (SEQ ID NO: 144). IL6R-B3/IL6R-L which is used as the template to produce IL6R-BP230 is indicated by asterisk (*). KD (IIb) of parent polypeptide/KD (IIb) of altered polypeptide in the table shows the value obtained by dividing the KD value of IL6R-B3/IL6R-L for FcγRIIb by the KD value of each variant for FcγRIIb. Meanwhile, KD (IIaR) of parent polypeptide/KD (IIaR) of altered polypeptide shows the value obtained by dividing the KD value of IL6R-B3/IL6R-L for FcγR IIaR by the KD value of each variant for FcγR IIaR. KD (IIaR)/KD (IIb) shows the value obtained by dividing the KD of each variant for FcγRIIaR by the KD of the variant for FcγRIIb. The greater the value, the higher the selectivity to FcγRIIb. In Table 22, the numeral in the gray-filled cells indicates that the binding of FcγR to IgG was concluded to be too weak to analyze correctly by kinetic analysis and thus was calculated using:

[Equation 2]

$$KD = C \bullet R_{max} / (R_{eq} - RI) - C$$

described in Reference Example 25.

[Table 22]

| VARIANT NAME | ALTERATION INTRODUCED INTO IL6R-BP230 | KD AGAINST FcγRIa (mol/L) | KD AGAINST FcγRIIaR (mol/L) | KD AGAINST FcγRIIaH (mol/L) | KD AGAINST FcγRIIb (mol/L) | KD AGAINST FcγRIIIaV (mol/L) | KD (IIaR)/KD (IIb) | KD (IIaR) OF PARENT POLYPEPTIDE/ KD (IIaR) OF ALTERED POLYPEPTIDE | KD (IIb) OF PARENT POLYPEPTIDE/ KD (IIb) OF ALTERED POLYPEPTIDE |
|---|---|---|---|---|---|---|---|---|---|
| IL6R-G1d/IL6R-L | | 3.20E-10 | 1.00E-06 | 6.70E-07 | 2.60E-06 | 3.50E-07 | 0.4 | 1.1 | 1.2 |
| IL6R-B3/IL6R-L | * | 4.20E-10 | 1.10E-06 | 7.70E-07 | 3.10E-06 | 3.30E-07 | 0.3 | 1 | 1 |
| IL6R-BP230/IL6R-L | | 1.40E-08 | 5.70E-07 | 9.60E-06 | 2.10E-08 | 6.70E-05 | 27.5 | 1.9 | 149 |
| IL6R-BP264/IL6R-L | H268E | 6.50E-09 | 4.80E-07 | 9.20E-06 | 1.20E-08 | 5.20E-05 | 40.6 | 2.3 | 265 |
| IL6R-BP384/IL6R-L | K334R | 3.00E-09 | 1.30E-06 | 1.80E-05 | 7.10E-08 | 4.50E-05 | 17.7 | 0.9 | 43.5 |
| IL6R-BP390/IL6R-L | I332S | 1.60E-09 | 4.90E-07 | 7.30E-06 | 2.10E-08 | 2.90E-05 | 22.9 | 2.2 | 144.9 |
| IL6R-BP391/IL6R-L | I332T | 9.60E-10 | 3.40E-07 | 4.40E-06 | 1.30E-08 | 1.90E-05 | 26.6 | 3.2 | 242.2 |
| IL6R-BP392/IL6R-L | I332K | 7.90E-09 | 7.30E-06 | 2.80E-05 | 9.90E-07 | 2.90E-05 | 7.3 | 0.2 | 3.1 |
| IL6R-BP393/IL6R-L | I332R | 1.10E-08 | 3.90E-06 | 4.60E-05 | 2.70E-06 | 4.80E-05 | 1.4 | 0.3 | 1.2 |
| IL6R-BP465/IL6R-L | E333K | 1.60E-08 | 6.10E-07 | 1.50E-05 | 2.10E-08 | 6.70E-05 | 29.8 | 1.8 | 151.2 |
| IL6R-BP466/IL6R-L | E333R | 1.50E-08 | 5.20E-07 | 1.10E-05 | 1.70E-08 | 2.90E-05 | 30.4 | 2.1 | 181.3 |
| IL6R-BP467/IL6R-L | K334S | 8.90E-10 | 1.10E-06 | 1.20E-05 | 4.10E-08 | 3.20E-05 | 25.8 | 1 | 75.4 |
| IL6R-BP468/IL6R-L | K334T | 9.70E-10 | 1.10E-06 | 9.70E-06 | 4.00E-08 | 2.70E-05 | 26.3 | 1 | 77.7 |
| IL6R-BP469/IL6R-L | E333S | 1.30E-08 | 6.00E-07 | 1.20E-05 | 2.30E-08 | 3.70E-05 | 26.4 | 1.8 | 136.6 |
| IL6R-BP470/IL6R-L | E333T | 1.50E-08 | 4.90E-07 | 1.00E-05 | 1.60E-08 | 3.70E-05 | 30.6 | 2.2 | 192.5 |

Both FcγRIIb-binding activity and FcγRIIb selectivity of IL6R-BP264/IL6R-L, IL6R-BP465/IL6R-L, IL6R-BP466/IL6R-L, and IL6R-BP470, resulting from introducing alterations H268E, E333K, E333R, and E333T, respectively, into IL6R-BP230/IL6R-L were increased as compared to those of IL6R-BP230/IL6R-L. The FcγRIIb selectivity of IL6R-BP391/IL6R-L introduced with the I332T alteration was reduced while its FcγRIIb-binding activity was increased as compared to IL6R-BP230/IL6R-L.

[Example 16] Exhaustive introduction of alterations at amino acid residues around position 271 (EU numbering)

In the structural comparison between Fc (P208) and FcγRIIb and Fc (P238D)/FcγRIIb, the most significant difference is found in the structure around position 271 (EU numbering) in the CH2 domain B of the Fc region (Fig. 39). As described in Example 11, it is suggested that, when, in Fc (P238D), Asp at position 270 (EU numbering) forms a tight electrostatic interaction with Arg at position 131 in FcγRIIb, the interaction can induce stereochemical stress at Pro at position 271 (EU numbering). In the structure of Fc (P208)/FcγRIIb, due to the substitution of Gly for Pro at position 271 (EU numbering), a positional deviation occurred at the main chain level so as to eliminate the structural distortion, resulting in a large structural change around position 271. There is a possibility that additional stabilization of the structure changed around position 271 further reduces the entropic energy loss caused by the binding upon formation of an electrostatic interaction with Arg at position 131 in FcγRIIb. Thus, alterations that enhance the FcγRIIb binding or increase the FcγRIIb selectivity of the Fc region were sought by exhaustive introduction of alterations at amino acid residues around position 271 (EU numbering).

IL6R-BP267 was constructed as a template in exhaustive introduction of alterations by introducing alterations E233D, G237D, P238D, H268E, and P271G into IL6R-B3 (SEQ ID NO: 144). IL6R-L (SEQ ID NO: 135) was used as the antibody L chain. Antibodies containing the light chain of IL6R-L and the above-described heavy chain variants were expressed and purified according to the method described in Reference Examples 1 and 2. The purified antibodies were assessed for their binding to each FcγR (FcγRIa, FcγRIIaH, FcγRIIaR, FcγRIIb, or FcγRIIIaV) by the method described in Reference Example 25. The amino acids at positions 264, 265, 266, 267, 269, and 272 (EU numbering) in IL6R-BP267 were substituted with each of 18 types of amino acids, except Cys and the amino acid prior to substitution. IL6R-L (SEQ ID NO: 135) was used as the antibody L chain. Antibodies containing the light chain of IL6R-L and the above-described heavy chain variants were expressed and purified according to the method described in Reference Examples 1 and 2. The antibodies, purified by the method of Reference Example 25, were assessed for their binding to each FcγR (FcγRIa, FcγRIIaH, FcγRIIaR, FcγRIIb, or FcγRIIIaV) by the method described in Reference Example 25. Variants whose FcγRIIb binding has been enhanced or FcγRIIb selectivity has been increased as compared to the FcγRIIb binding or FcγIIb selectivity of IL6R-BP267/IL6R-L prior to introduction of the alterations are shown in Table 23.

[Table 23]

| VARIANT NAME | ALTERATION INTRODUCED INTO IL6R-BP267 | KD AGAINST FcγRIa (mol/L) | KD AGAINST FcγRIIaR (mol/L) | KD AGAINST FcγRIIaH (mol/L) | KD AGAINST FcγRIIb (mol/L) | KD AGAINST FcγRIIIaV (mol/L) | KD (IIaR)/KD (IIb) | KD (IIaR) OF PARENT POLYPEPTIDE/ KD (IIaR) OF ALTERED POLYPEPTIDE | KD (IIb) OF PARENT POLYPEPTIDE/ KD (IIb) OF ALTERED POLYPEPTIDE |
|---|---|---|---|---|---|---|---|---|---|
| IL6R-B3/IL6R-L | * | 3.20E-10 | 1.00E-06 | 6.70E-07 | 2.60E-06 | 3.50E-07 | 0.4 | 1 | 1 |
| IL6R-BP267/IL6R-L | | 4.00E-09 | 1.70E-06 | 1.90E-05 | 1.30E-07 | 5.30E-05 | 13 | 0.6 | 20.5 |
| IL6R-BP348/IL6R-L | S267A | 5.50E-10 | 7.00E-07 | 2.20E-05 | 4.60E-08 | 2.70E-05 | 15.3 | 1.4 | 56.9 |
| IL6R-BP300/IL6R-L | V264I | 9.60E-09 | 6.90E-07 | 2.20E-05 | 5.80E-08 | 5.60E-05 | 11.9 | 1.4 | 44.8 |
| IL6R-BP367/IL6R-L | E269D | 3.10E-09 | 1.20E-06 | 4.60E-05 | 1.00E-07 | 5.30E-05 | 11.7 | 0.8 | 25.7 |
| IL6R-BP350/IL6R-L | S267E | 8.90E-10 | 1.50E-07 | 8.30E-05 | 1.00E-07 | 8.90E-05 | 1.5 | 6.5 | 25.2 |
| IL6R-BP333/IL6R-L | V266F | 9.10E-09 | 1.50E-06 | 3.40E-05 | 1.20E-07 | 5.90E-05 | 12.5 | 0.7 | 22.2 |
| IL6R-BP352/IL6R-L | S267G | 1.80E-09 | 1.90E-06 | 2.80E-05 | 1.20E-07 | 4.30E-05 | 15.7 | 0.5 | 21.3 |
| IL6R-BP339/IL6R-L | V266M | 4.60E-09 | 1.40E-06 | 1.80E-05 | 1.30E-07 | 2.40E-05 | 11.3 | 0.7 | 20.6 |
| IL6R-BP520/IL6R-L | E272M | 3.90E-09 | 3.00E-06 | 3.10E-05 | 1.70E-07 | 4.70E-05 | 17.5 | 0.3 | 14.9 |
| IL6R-BP523/IL6R-L | E272Q | 3.70E-09 | 2.70E-06 | 2.90E-05 | 1.70E-07 | 4.10E-05 | 15.9 | 0.4 | 15.5 |
| IL6R-BP313/IL6R-L | D265E | 2.60E-08 | 1.30E-05 | 4.70E-05 | 8.40E-07 | 3.80E-05 | 15.6 | 0.1 | 3.1 |
| IL6R-BP513/IL6R-L | E272D | 3.80E-09 | 1.70E-06 | 3.90E-05 | 1.10E-07 | 7.50E-05 | 15.4 | 0.6 | 23.6 |
| IL6R-BP521/IL6R-L | E272N | 3.60E-09 | 2.90E-06 | 4.40E-05 | 1.90E-07 | 9.90E-05 | 15.2 | 0.3 | 13.5 |
| IL6R-BP338/IL6R-L | V266L | 1.50E-08 | 2.20E-06 | 2.20E-05 | 1.50E-07 | 2.50E-05 | 15 | 0.5 | 17.9 |
| IL6R-BP517/IL6R-L | E272I | 3.20E-09 | 2.10E-06 | 2.20E-05 | 1.40E-07 | 3.50E-05 | 14.7 | 0.5 | 18.1 |
| IL6R-BP514/IL6R-L | E272F | 4.30E-09 | 3.00E-06 | 6.40E-05 | 2.10E-07 | 9.10E-05 | 14 | 0.3 | 12.3 |

The KD value of each variant to each FcγR is shown in Table 23. In the table, "alteration" refers to an alteration introduced into IL6R-BP3, which was used as a template. IL6R-B3/IL6R-L which is used as the template to produce IL6R-BP267 is indicated by asterisk (*). In the table, KD (IIb) of parent polypeptide/KD (IIb) of altered polypeptide shows the value obtained by dividing the KD value of IL6R-B3/IL6R-L for FcγRIIb by the KD value of each variant for FcγRIIb. Meanwhile, KD (IIaR) of parent polypeptide/KD (IIaR) of altered polypeptide shows the value obtained by dividing the KD of IL6R-B3/IL6R-L for FcγRIIaR by the KD of each variant for FcγR IIaR. KD (IIaR)/KD (IIb) shows the value obtained by dividing the KD value of each variant for FcγRIIaR by the KD value of the variant for FcγRIIb. The greater the value, the higher the selectivity to FcγRIIb. In Table 23, the numeral in the gray-filled cells indicates that the binding of FcγR to IgG was concluded to be too weak to analyze correctly by kinetic analysis and thus was calculated using:

[Equation 2]

$$KD = C \bullet R_{max} / (R_{eq} - RI) - C$$

described in Reference Example 25.

All the binding activities of variants shown in Table 23 to FcγRIa, FcγRIIaH, and FcγRIIIaV were comparable or reduced as compared to that of IL6R-B3/IL6R-L. Meanwhile, the FcγRIIb-binding activity of variants resulting from adding alterations S267A, V264I, E269D, S267E, V266F, S267G, and V266M, respectively, to IL6R-BP267/IL6R-L were increased as compared to that of IL6R-BP267/IL6R-L prior to addition of alteration. Meanwhile, the KD (IIaR)/KD (IIb) values of variants resulting from adding the S267A, S267G, E272M, E272Q, D265E, E272D, E272N, V266L, E272I, and E272F alterations, respectively, to IL6R-BP267/IL6R-L were increased as compared to that of IL6R-BP267/IL6R-L prior to addition of alteration. This demonstrates that the S267A, S267G, E272M, E272Q, D265E, E272D, E272N, V266L, E272I, and E272F alterations produce the effect to improve the FcyRIIb selectivity.

[Example 17] Enhancement of the FcγRIIb binding by introduction of alterations into CH3 region

A substitution alteration of Leu for Pro at position 396 (EU numbering) has been reported to enhance the FcγRIIb binding (Cancer Res. (2007) 67, 8882-8890). The amino acid at position 396 (EU numbering) is present at a position which is not directly involved in the interaction with FcγR. However, the amino acid can be assumed to have an effect on the interaction with FcγR by changing the antibody structure. Thus, the present inventors assessed whether the FcγRIIb binding of the Fc region is enhanced or its FcγRIIb selectivity is increased by exhaustive introduction of amino acid alterations at position 396 (EU numbering) in the Fc region.

IL6R-BP423 was constructed as a template in exhaustive introduction of alterations by introducing alterations E233D, G237D, P238D, S267A, H268E, P271G, and A330R into IL6R-B3 (SEQ ID NO: 144). Variants, in which the amino acid at position 396 (EU numbering) in IL6R-BP423 was substituted with each of 18 types of amino acids, except cysteine and the amino acid prior to substitution, were constructed. IL6R-L (SEQ ID NO: 135) was used as the antibody L chain. Antibodies containing the light chain of IL6R-L and the above-described heavy chain variants were expressed and purified according to the method described in Reference Example 1. The purified antibodies were assessed for their binding to each FcγR (FcγRIa, FcγRIIaH, FcγRIIaR, FcγRIIb, or FcγRIIIaV) by the method described in Reference Example 25. The binding of the resulting variants to each FcγR is shown in Table 24.

[Table 24]

| VARIANT NAME | ALTERATION INTRODUCED INTO IL6R-BP423 | KD AGAINST FcγRIa (mol/L) | KD AGAINST FcγRIIaR (mol/L) | KD AGAINST FcγRIIaH (mol/L) | KD AGAINST FcγRIIb (mol/L) | KD AGAINST FcγRIIIaV (mol/L) | KD (IIaR)/ KD (IIb) | KD (IIaR) OF PARENT POLYPEPTIDE/ KD (IIaR) OF ALTERED POLYPEPTIDE | KD (IIb) OF PARENT POLYPEPTIDE/ KD (IIb) OF ALTERED POLYPEPTIDE |
|---|---|---|---|---|---|---|---|---|---|
| IL6R-G1d/IL6R-L | | 3.20E-10 | 1.00E-06 | 6.70E-07 | 2.60E-06 | 3.50E-07 | 0.4 | 1.1 | 1.2 |
| IL6R-B3/IL6R-L | * | 4.20E-10 | 1.10E-06 | 7.70E-07 | 3.10E-06 | 3.30E-07 | 0.3 | 1 | 1 |
| IL6R-BP423/IL6R-L | | 7.70E-10 | 1.80E-07 | 2.00E-06 | 5.10E-09 | 1.60E-05 | 34.2 | 6.3 | 604 |
| IL6R-BP447/IL6R-L | P396A | 9.00E-10 | 1.60E-07 | 2.00E-06 | 5.30E-09 | 2.50E-05 | 29.7 | 7 | 584 |
| IL6R-BP448/IL6R-L | P396D | 7.50E-10 | 1.30E-07 | 1.40E-06 | 4.10E-09 | 9.70E-06 | 31.7 | 8.5 | 759 |
| IL6R-BP449/IL6R-L | P396E | 9.10E-10 | 1.40E-07 | 1.50E-06 | 4.60E-09 | 1.20E-05 | 29.8 | 8 | 667 |
| IL6R-BP450/IL6R-L | P396F | 8.40E-10 | 1.20E-07 | 1.30E-06 | 4.10E-09 | 9.60E-06 | 29.4 | 9.2 | 763 |
| IL6R-BP451/IL6R-L | P396G | 9.80E-10 | 1.80E-07 | 2.00E-06 | 6.20E-09 | 1.20E-05 | 29.2 | 6.1 | 499 |
| IL6R-BP452/IL6R-L | P396H | 7.50E-10 | 1.30E-07 | 1.50E-06 | 5.10E-09 | 1.10E-05 | 25.9 | 8.3 | 602 |
| IL6R-BP453/IL6R-L | P396I | 7.50E-10 | 1.20E-07 | 9.30E-07 | 4.60E-09 | 7.40E-06 | 25.5 | 9.4 | 675 |
| IL6R-BP454/IL6R-L | P396K | 8.20E-09 | 1.30E-07 | 1.40E-06 | 4.80E-09 | 9.10E-06 | 27.5 | 8.4 | 649 |
| IL6R-BP455/IL6R-L | P396L | 7.50E-10 | 1.30E-07 | 1.60E-06 | 4.00E-09 | 8.50E-06 | 31.8 | 8.6 | 767 |
| IL6R-BP456/IL6R-L | P396M | 6.00E-10 | 1.20E-07 | 2.00E-06 | 3.50E-09 | 9.20E-06 | 35.3 | 8.9 | 888 |
| IL6R-BP457/IL6R-L | P396N | 9.10E-10 | 1.50E-07 | 2.60E-06 | 5.20E-09 | 1.30E-05 | 28.9 | 7.3 | 591 |
| IL6R-BP458/IL6R-L | P396Q | 7.80E-10 | 1.40E-07 | 1.40E-06 | 4.50E-09 | 1.10E-05 | 31.1 | 7.9 | 687 |
| IL6R-BP459/IL6R-L | P396R | 1.10E-09 | 1.50E-07 | 1.40E-06 | 5.10E-09 | 1.20E-05 | 28.9 | 7.5 | 607 |
| IL6R-BP460/IL6R-L | P396S | 8.70E-10 | 1.60E-07 | 3.20E-06 | 6.50E-09 | 1.40E-05 | 25.2 | 6.7 | 478 |
| IL6R-BP461/IL6R-L | P396T | 1.30E-09 | 1.30E-07 | 1.50E-06 | 5.10E-09 | 9.90E-06 | 24.4 | 8.8 | 602 |
| IL6R-BP462/IL6R-L | P396V | 9.70E-10 | 1.30E-07 | 1.40E-06 | 5.20E-09 | 9.00E-06 | 25 | 8.5 | 593 |
| IL6R-BP463/IL6R-L | P396W | 1.30E-09 | 1.60E-07 | 1.90E-06 | 5.60E-09 | 1.20E-05 | 28.1 | 7 | 554 |
| IL6R-BP464/IL6R-L | P396Y | 1.10E-09 | 1.30E-07 | 2.10E-06 | 4.00E-09 | 9.90E-06 | 31.5 | 8.7 | 773 |

In the table, "alteration introduced into IL6R-BP423" refers to an alteration introduced into IL6R-BP423, which was used as a template. IL6R-B3/IL6R-L which is used as the template to produce IL6R-BP423 is indicated by asterisk (*). In the table, KD (IIb) of parent polypeptide/KD (IIb) of altered polypeptide shows the value obtained by dividing the KD value of IL6R-B3/IL6R-L for FcγRIIb by the KD value of each variant for FcγRIIb. Meanwhile, KD (IIaR) of parent polypeptide/KD (IIaR) of altered polypeptide shows the value obtained by dividing the KD value of IL6R-B3/IL6R-L for FcγR IIaR by the KD value of each variant for FcγR IIaR. KD (IIaR)/KD (IIb) shows the value obtained by dividing the KD of each variant for FcγRIIaR by the KD of the variant for FcγRIIb. The greater the value, the higher the selectivity to FcγRIIb. In Table 24, the numeral in the gray-filled cells indicates that the binding of FcγR to IgG was concluded to be too weak to analyze correctly by kinetic analysis and thus was calculated using:

[Equation 2]

$$KD = C \bullet R_{max} / (R_{eq} - RI) - C$$

described in Reference Example 25.

The result shown in Table 24 demonstrates that: the FcγRIIb-binding activity of IL6R-BP456/IL6R-L resulting from introducing alteration P396M into IL6R-BP423/IL6R-L, IL6R-BP455/IL6R-L resulting from introducing alteration P396L into IL6R-BP423/IL6R-L, IL6R-BP464/IL6R-L resulting from introducing alteration P396Y into IL6R-BP423/IL6R-L, IL6R-BP450/IL6R-L resulting from introducing alteration P396F into IL6R-BP423/IL6R-L, IL6R-BP448/IL6R-L resulting from introducing alteration P396D into IL6R-BP423/IL6R-L, IL6R-BP458/IL6R-L resulting from introducing alteration P396Q into IL6R-BP423/IL6R-L, IL6R-BP453/IL6R-L resulting from introducing alteration P396I into IL6R-BP423/IL6R-L, IL6R-BP449/IL6R-L resulting from introducing alteration P396E into IL6R-BP423/IL6R-L, IL6R-BP454/IL6R-L resulting from introducing alteration P396K into IL6R-BP423/IL6R-L, and IL6R-BP459/IL6R-L resulting from introducing alteration P396R into IL6R-BP423/IL6R-L was all increased as compared to that of IL6R-BP423/IL6R-L prior to introduction of the alterations. Meanwhile, the KD (IIaR)/KD (IIb) value of IL6R-BP456/IL6R-L resulting from introducing alteration P396M into IL6R-BP423/IL6R-L was larger as compared to that of IL6R-BP423/IL6R-L prior to introduction of the alteration, demonstrating the improved FcγRIIb selectivity. As seen in Table 24, the binding activity of the prepared variants to FcγRIa, FcγRIIaH, and FcγRIIIaV was all lower than that of IL6R-B3/IL6R-L, which was the parent polypeptide.

[Example 18] Preparation of variants with enhanced FcγRIIb binding using subclass sequences

The FcγR binding profile varies depending on the subclass of human IgG. The present inventors assessed whether the difference in the binding activity to each FcγR between IgG1 and IgG4 could be utilized to increase the FcγRIIb-binding activity and/or improve the selectivity. First, IgG1 and IgG4 were analyzed for their binding activity to each FcγR. IL6R-G4d (SEQ ID NO: 164) containing G4d was constructed as the antibody H chain. G4d is an Fc region that lacks the C-terminal Gly and Lys and contains a substitution of Pro for Ser at position 228 (EU numbering) in human IgG4. IL6R-L (SEQ ID NO: 135) was used as the antibody L chain. Antibodies containing the light chain of IL6R-L and IL6R-G1d/IL6R-L or IL6R-G4d/IL6R-L were expressed and purified according to the method described in Reference Examples 1 and 2. The purified antibodies were assessed for their binding to each FcγR (FcγRIa, FcγRIIaH, FcγRIIaR, FcγRIIb, or FcγRIIIaV) by the method described in Reference Example 25. The binding of the resulting variants to each FcγR is summarized in Table 25.

[Table 25]

| VARIANT NAME | KD AGAINST FcγRIa (mol/L) | KD AGAINST FcγRIIaR (mol/L) | KD AGAINST FcγRIIaH (mol/L) | KD AGAINST FcγRIIb (mol/L) | KD AGAINST FcγRIIIaV (mol/L) |
|---|---|---|---|---|---|
| IL6R-G1d/IL6R-L | 1.20E-10 | 9.70E-07 | 6.50E-07 | 3.90E-06 | 4.20E-07 |
| IL6R-G4d/IL6R-L | 6.60E-10 | 2.10E-06 | 3.40E-06 | 2.60E-06 | 3.40E-06 |

It was demonstrated that the FcγRIIb binding of IL6R-G4d/IL6R-L was 1.5 times stronger than that of IL6R-G1d/IL6R-L whereas the FcγRIIaR binding of IL6R-G4d/IL6R-L was 2.2 times weaker than that of IL6R-G1d/IL6R-L.

Meanwhile, the binding activity of IL6R-G4d/IL6R-L to FcγRIa, FcγRIIaH, and FcγRIIIaV was lower than that of IL6R-G1d/IL6R-L. The result described above revealed that IL6R-G4d had preferable characteristics as compared to IL6R-G1d in terms of both FcγRIIb-binding activity and selectivity.

Fig. 44 is an alignment to compare the CH1 sequences of G1d and G4d up to the C terminus (positions 118 to 445 (EU numbering)). In Fig. 44, amino acid residues that are different between G1d and G4d are boxed with thick line. The present inventors assessed whether the FcγRIIb binding could be further increased and/or the FcγRIIb selectivity could be further improved by selecting, from the above-described different amino acids, some portions that are predicted to be involved in the interaction with FcγR, and grafting at least one amino acid residue or more of the G4d sequence, which confers a property preferable from the viewpoint of both FcγRIIb-binding activity and selectivity, to a variant with enhanced FcγRIIb binding.

Specifically, the present inventors produced:

IL6R-BP473 resulting from introducing alteration A327G into IL6R-BP230;
IL6R-BP472 resulting from introducing alteration A330S into IL6R-BP230;
IL6R-BP471 resulting from introducing alteration P331S into IL6R-BP230;
IL6R-BP474 resulting from introducing alterations A330S and P331S into IL6R-BP230;
IL6R-BP475 resulting from introducing alterations A327G and A330S into IL6R-BP230;
IL6R-BP476 resulting from introducing alterations A327G, A330S, and P331S into IL6R-BP230; and
IL6R-BP477 resulting from introducing alterations A327G and P331S into IL6R-BP230.

Furthermore, to construct IL6R-BP478, the amino acids from Ala at position 118 to Thr at position 225 (EU numbering) in IL6R-BP230 was substituted with the amino acids of the G4d sequence from Ala at position 118 to Pro at position 222 (EU numbering). IL6R-L (SEQ ID NO: 135) was used as the antibody L chain. Antibodies containing the light chain of IL6R-L and the heavy chain variants described above were purified according to the method described in Reference Examples 1 and 2. The purified antibodies were assessed for their binding activity to each FcγR (FcγRIa, FcγRIIaH, FcγRIIaR, FcγRIIb, or FcγRIIIaV) by the method described in Reference Example 25.

The KD value of each variant to each FcγR is shown in Table 26. KD (IIb) of parent polypeptide/KD (IIb) of altered polypeptide in the table shows the value obtained by dividing the KD value of IL6R-B3/IL6R-L for FcγRIIb by the KD value of each variant for FcγRIIb. In the table, "alteration introduced into IL6R-BP230" refers to an alteration introduced into IL6R-BP230. IL6R-B3/IL6R-L used as the template to produce IL6R-BP230 is indicated by *1. Meanwhile, IL6R-BP478, in which the G4d sequence from Ala at position 118 up to Pro at position 222 (EU numbering) has been substituted for the segment from Ala at position 118 up to Thr at position 225 (EU numbering) in IL6R-BP230, is indicated by *2. KD (IIaR) of parent polypeptide/KD (IIaR) of altered polypeptide shows the value obtained by dividing the KD value of IL6R-B3/IL6R-L for FcγR IIaR by the KD value of the variant for FcγR IIaR. KD (IIaR)/KD (IIb) shows the value obtained by dividing the KD of each variant for FcγRIIaR by the KD of the variant for FcγRIIb. The greater the value, the higher the selectivity to FcγRIIb. In Table 26, the numeral in the gray-filled cells indicates that the binding of FcγR to IgG was concluded to be too weak to analyze correctly by kinetic analysis and thus was calculated using:

[Equation 2]

$$KD = C \bullet R_{max} / (R_{eq} - RI) - C$$

described in Reference Example 25.

[Table 26]

| VARIANT NAME | ALTERATION INTRODUCED INTO IL6R-BP230 | KD AGAINST FcγRIa (mol/L) | KD AGAINST FcγRIIaR (mol/L) | KD AGAINST FcγRIIaH (mol/L) | KD AGAINST FcγRIIb (mol/L) | KD AGAINST FcγRIIIaV (mol/L) | KD (IIaR)/KD (IIb) | KD (IIaR) OF PARENT POLYPEPTIDE/ KD (IIaR) OF ALTERED POLYPEPTIDE | KD (IIb) OF PARENT POLYPEPTIDE/ KD (IIb) OF ALTERED POLYPEPTIDE |
|---|---|---|---|---|---|---|---|---|---|
| IL6R-G1d/IL6R-L | | 3.20E-10 | 1.00E-06 | 6.70E-07 | 2.60E-06 | 3.50E-07 | 0.4 | 1.1 | 1.2 |
| IL6R-B3/IL6R-L | *1 | 4.20E-10 | 1.10E-06 | 7.70E-07 | 3.10E-06 | 3.30E-07 | 0.3 | 1 | 1 |
| IL6R-BP230/IL6R-L | | 1.40E-08 | 5.70E-07 | 9.60E-06 | 2.10E-08 | 6.70E-05 | 27.5 | 1.9 | 149 |
| IL6R-BP471/IL6R-L | P331S | 7.30E-09 | 8.00E-07 | 1.20E-05 | 3.50E-08 | 7.10E-05 | 22.7 | 1.4 | 88.1 |
| IL6R-BP472/IL6R-L | A330S | 5.20E-09 | 3.30E-06 | 2.40E-05 | 1.50E-07 | 3.80E-05 | 21.5 | 0.3 | 20.3 |
| IL6R-BP473/IL6R-L | A327G | 6.20E-09 | 3.80E-07 | 4.80E-06 | 1.80E-08 | 3.60E-05 | 21.1 | 2.9 | 172.2 |
| IL6R-BP474/IL6R-L | A330S/P331S | 4.10E-09 | 3.00E-06 | 3.70E-05 | 1.80E-07 | 5.50E-05 | 16.6 | 0.4 | 16.9 |
| IL6R-BP475/IL6R-L | A327G/A330S | 4.90E-09 | 1.00E-06 | 1.50E-05 | 1.10E-07 | 4.60E-05 | 9.7 | 1.1 | 29.2 |
| IL6R-BP476/IL6R-L | A327G/A330S/P331S | 5.90E-09 | 1.30E-06 | 1.90E-05 | 1.30E-07 | 4.90E-05 | 9.7 | 0.9 | 23.7 |
| IL6R-BP477/IL6R-L | A327G/P331S | 9.20E-09 | 5.10E-07 | 7.60E-06 | 3.70E-08 | 5.80E-05 | 14 | 2.2 | 84.9 |
| IL6R-BP478/IL6R-L | *2 | 7.70E-09 | 5.40E-07 | 6.70E-06 | 1.90E-08 | 3.50E-05 | 28 | 2 | 160.6 |

Of the variants shown in Table 26, IL6R-BP473/IL6R-L introduced with the alteration A327G showed FcγRIIb binding augmented by 1.2 times compared to that of IL6R-BP230/IL6R-L. Meanwhile, IL6R-BP478/IL6R-L, in which the amino acids from Ala at position 118 to Thr at position 225 (EU numbering) in IL6R-BP230 have been substituted by the amino acids from Ala at position 118 to Pro at position 222 (EU numbering) in the amino acid sequence of G4d, exhibited FcγRIIb and FcγRIIaR binding augmented by 1.1 times compared to that of IL6R-BP230/IL6R-L. All of the variants showed lower binding activities to FcγRIa, FcγRIIaH, and FcγRIIIaV as compared to the parental polypeptide IL6R-B3/IL6R-L.

The variants with the modifications at other sites shown in Fig. 44 were also assessed. Specifically, into the antibody H chain IL6R-BP230, K274Q was introduced to prepare IL6R-BP541; ; Y296F was introduced to prepare IL6R-BP542; H268Q was introduced to prepare IL6R-BP543; R355Q was introduced to prepare IL6R-BP544; D356E was introduced to prepare IL6R-BP545; L358M was introduced to prepare IL6R-BP546; K409R was introduced to prepare IL6R-BP547; and Q419E was introduced to prepare IL6R-BP548. Meanwhile, IL6R-L was used as the common antibody L chain. Antibodies that contain the above heavy chain variant and the light chain IL6R-L were purified according to the methods described in Reference Examples 1 and 2. The purified antibodies were assessed for their binding to each FcγR (FcγRIa, FcγRIIaH, FcγRIIaR, FcγRIIb, or FcγRIIIaV) by the method of Reference Example 25.

The KD of each variant to each FcγR is shown in Table 27. In the Table, "KD (IIb) of the parent polypeptide/KD (IIb) of the altered polypeptide" represents the value obtained by dividing the KD value of IL6R-B3/IL6R-L to FcγRIIb by the KD value of each variant to FcyRIIb. In the Table, "alteration of IL6R-BP230" refers to a modification introduced into IL6R-BP230. IL6R-B3/IL6R-L used as the template to produce IL6R-BP230 is indicated with *1. "KD (IIaR) of the parent polypeptide/KD (IIaR) of the altered polypeptide" represents the value obtained by dividing the KD value of IL6R-B3/IL6R-L to FcγRIIaR by the KD value of the same variant to FcγRIIaR. KD (IIaR)/KD (IIb) represents the value obtained by dividing KD of each variant to FcγRIIaR by KD of the same variant to FcγRIIb. The greater the value, the higher the selectivity to FcγRIIb. In Table 27, the numeral in the cell filled with gray indicates that the binding of FcγR to IgG was weak, and it was determined that the analysis could not be correctly performed by kinetic analysis, and thus was calculated using:

[Equation 2]

$$KD = C \bullet R_{max} / (R_{eq} - RI) - C$$

described in Reference Example 25.

As shown in Table 27, IL6R-BP541/IL6R-L resulting from introducing K274Q into IL6R-BP230/IL6R-L, IL6R-BP544/IL6R-L resulting from introducing R355Q into IL6R-BP230/IL6R-L, IL6R-BP545/IL6R-L resulting from introducing D356E into IL6R-BP230/IL6R-L, and IL6R-BP546/IL6R-L resulting from introducing L358M into IL6R-BP230/IL6R-L, showed augmented FcγRIIb binding as compared to IL6R-BP230/IL6R-L prior to the introduction of alteration. Of them, IL6R-BP544/IL6R-L resulting from introducing R355Q into IL6R-BP230/IL6R-L, IL6R-BP545/IL6R-L resulting from introducing D356E into IL6R-BP230/IL6R-L, and IL6R-BP546/IL6R-L resulting from introducing L358M into IL6R-BP230/IL6R-L, were shown to have an increased KD(IIaR)/KD(IIb) value and improved selectivity to FcγRIIb, as compared to IL6R-BP230/IL6R-L prior to the introduction of alteration.

[Table 27]

| VARIANT NAME | ALTERATION OF IL6R-BP230 | KD AGAINST FcγRIa (mol/L) | KD AGAINST FcγRIIaR (mol/L) | KD AGAINST FcγRIIaH (mol/L) | KD AGAINST FcγRIIb (mol/L) | KD AGAINST FcγRIIIaV (mol/L) | KD (IIaR)/ KD (IIb) | KD(IIaR) OF THE PARENT POLYPEPTIDE / KD(IIaR) OF THE ALTERED POLYPEPTIDE | KD(IIb) OF THE PARENT POLYPEPTIDE / KD(IIb) OF THE ALTERED POLYPEPTIDE |
|---|---|---|---|---|---|---|---|---|---|
| IL6R-G1d/IL6R-L | | 3.2E-10 | 1.0E-06 | 6.7E-07 | 2.6E-06 | 3.5E-07 | 0.4 | 1.1 | 1.2 |
| IL6R-B3/IL6R-L | *1 | 4.2E-10 | 1.1E-06 | 7.7E-07 | 3.1E-06 | 3.3E-07 | 0.3 | 1.0 | 1.0 |
| IL6R-BP230/IL6R-L | | 1.0E-08 | 4.9E-07 | 9.7E-06 | 1.8E-08 | 3.9E-05 | 28.0 | 2.2 | 175.6 |
| IL6R-BP541/IL6R-L | K274Q | 1.1E-08 | 4.5E-07 | 9.3E-06 | 1.6E-08 | 4.1E-05 | 27.8 | 2.4 | 189.6 |
| IL6R-BP542/IL6R-L | Y296F | 1.3E-08 | 4.9E-07 | 9.7E-06 | 2.0E-08 | 4.3E-05 | 24.4 | 2.2 | 153.7 |
| IL6R-BP543/IL6R-L | H268Q | 2.3E-08 | 5.6E-07 | 7.4E-06 | 2.0E-08 | 4.6E-05 | 27.3 | 1.9 | 151.5 |
| IL6R-BP544/IL6R-L | R355Q | 9.8E-09 | 4.8E-07 | 1.2E-05 | 1.7E-08 | 4.5E-05 | 28.8 | 2.2 | 183.9 |
| IL6R-BP545/IL6R-L | D356E | 9.9E-09 | 5.7E-07 | 9.1E-06 | 1.7E-08 | 4.5E-05 | 32.7 | 1.9 | 178.6 |
| IL6R-BP546/IL6R-L | L358M | 9.0E-09 | 5.0E-07 | 1.0E-05 | 1.5E-08 | 3.7E-05 | 32.8 | 2.2 | 204.6 |
| IL6R-BP547/IL6R-L | K409R | 1.2E-08 | 4.9E-07 | 7.5E-06 | 1.9E-08 | 3.5E-05 | 25.5 | 2.2 | 162.6 |
| IL6R-BP548/IL6R-L | Q419E | 1.2E-08 | 5.0E-07 | 9.4E-06 | 1.9E-08 | 3.4E-05 | 26.2 | 2.2 | 161.8 |

[Example 19] Assessment of combinations of alterations that enhance the FcγRIIb binding or improve the FcγRIIb selectivity

Additional combinations of the alterations described herein in the sections up to and including Example 18, which alterations had been found to be effective in the aspect of enhancement of the FcγRIIb binding or the improvement of the FcγRIIb selectivity, were assessed. Specifically, the alterations that had been assessed to be effective in enhancing the FcyRIIb binding and/or improving the FcγRIIb selectivity were introduced in combination into IL6R-B3 (SEQ ID NO: 144). Furthermore, existing alterations S267E and L328F that enhance the FcyRIIb binding (Seung et al., (Mol. Immunol. (2008) 45, 3926-3933)) were introduced into IL6R-B3 to produce IL6R-BP253 as a comparison control. IL6R-L (SEQ ID NO: 135) was used as the antibody L chain. Antibodies containing the light chain of IL6R-L and the above-described heavy chain variants were expressed and purified according to the method as described in Reference Examples 1 and 2. The purified antibodies were assessed for their binding to each FcγR (FcγRIa, FcγRIIaH, FcγRIIaR, FcγRIIb, or FcγRIIIaV) by the method described in Reference Example 25.

The KD of each variant to each FcγR is shown in Table 28. In the table, "alteration" refers to an alteration introduced into IL6R-B3 (SEQ ID NO: 144). IL6R-B3/IL6R-L which is used as the template to produce each variant is indicated by asterisk (*). KD (IIb) of parent polypeptide/KD (IIb) of altered polypeptide shows the value obtained by dividing the KD value of IL6R-B3/IL6R-L for FcγRIIb by the KD value of each variant for FcγRIIb. Meanwhile, KD (IIaR) of parent polypeptide/KD (IIaR) of altered polypeptide shows the value obtained by dividing the KD value of IL6R-B3/IL6R-L for FcγR IIaR by the KD of the variant for FcγRIIaR. KD (IIaR)/KD (IIb) shows the value obtained by dividing the KD of each variant for FcγRIIaR by the KD of the variant for FcγRIIb. The greater the value, the higher the selectivity to FcγRIIb as compared to FcγRIIaR. Meanwhile, KD (IIaH)/KD (IIb) shows the value obtained by dividing the KD of each variant for FcγRIIaH by the KD of the variant for FcγRIIb. The greater the value, the higher the selectivity to FcγRIIb as compared to FcγRIIaH. In Table 28, the numeral in the gray-filled cells indicates that the binding of FcγR to IgG was concluded to be too weak to analyze correctly by kinetic analysis and thus was calculated using:

[Equation 2]

$$KD = C \bullet R_{max} / (R_{eq} - RI) - C$$

described in Reference Example 25.

[Table 28-1]

| VARIANT NAME (L chain: IL6R-L) | ALTERATION | KD (mol/L) | | | | | KD (IIaR)/ KD (IIb) | KD (IIaH)/ KD (IIb) | KD(IIaR) OF THE PARENT POLYPEPTIDE / KD(IIaR) OF THE ALTERED POLYPEPTIDE | KD(IIb) OF THE PARENT POLYPEPTIDE / KD(IIb) OF THE ALTERED POLYPEPTIDE |
|---|---|---|---|---|---|---|---|---|---|---|
| | | FcγRIa | FcγRIIaR | FcγRIIaH | FcγRIIb | FcγRIIIaV | | | | |
| IL6R-G1d | G1d | 3.2E-10 | 1.0E-06 | 6.7E-07 | 2.6E-06 | 3.5E-07 | 0.4 | 0.3 | 1.1 | 1.2 |
| IL6R-B3 | B3 | 4.2E-10 | 1.1E-06 | 7.7E-07 | 3.1E-06 | 3.3E-07 | 0.3 | 0.2 | 1 | 1 |
| IL6R-BP253 | S267E/L328F | 6.7E-11 | 2.1E-09 | 1.2E-06 | 1.1E-08 | 3.6E-06 | 0.2 | 107.1 | 528.8 | 276.8 |
| IL6R-BP262 | G237D/P238D/H268E/P271G | 1.0E-08 | 2.0E-06 | 4.5E-05 | 1.2E-07 | 5.6E-05 | 17 | 375 | 0.5 | 25.8 |
| IL6R-BP264 | E233D/G237D/P238D/H268E/P271G/Y296D/A330R | 7.4E-09 | 3.5E-07 | 2.8E-06 | 1.2E-08 | 2.6E-05 | 28.3 | 227.6 | 3.2 | 252 |
| IL6R-BP265 | G237D/P238D/H268E/P271G/Y296D/A330R | 2.3E-08 | 6.3E-07 | 1.2E-05 | 1.5E-08 | 9.6E-05 | 41.2 | 789.5 | 1.8 | 203.9 |
| IL6R-BP266 | E233D/G237D/P238D/H268E/P271G/A330R | 1.4E-08 | 3.2E-07 | 1.1E-05 | 1.8E-08 | 4.0E-05 | 18 | 621.5 | 3.4 | 175.1 |
| IL6R-BP268 | E233D/G237D/P238D/H268E/P271G/Y296D | 4.5E-09 | 1.8E-06 | 2.1E-05 | 9.2E-08 | 2.8E-05 | 19.6 | 228.3 | 0.6 | 33.7 |
| IL6R-BP269 | G237D/P238D/H268E/P271G/Y296D | 1.4E-08 | 2.2E-06 | 7.2E-05 | 1.1E-07 | 1.4E-04 | 19.6 | 637.2 | 0.5 | 27.4 |
| IL6R-BP423 | E233D/G237D/P238D/S267A/H268E/P271G/A330R | 7.7E-10 | 1.8E-07 | 2.0E-06 | 5.1E-09 | 1.6E-05 | 34.2 | 390.6 | 6.3 | 605.5 |
| IL6R-BP425 | E233D/G237D/P238D/V266L/S267A/H268E/P271G/A330R | 4.1E-09 | 2.2E-07 | 1.5E-05 | 9.1E-09 | 4.2E-05 | 23.6 | 1644.7 | 5.1 | 339.9 |
| IL6R-BP426 | E233D/G237D/P238D/S267A/H268E/E269D/P271G/A330R | 1.0E-09 | 1.6E-07 | 4.9E-05 | 5.9E-09 | 4.1E-05 | 27.6 | 8361.8 | 6.8 | 529 |
| IL6R-BP428 | E233D/G237D/P238D/S267G/H268E/P271G/A330R | 4.9E-09 | 3.9E-07 | 4.2E-05 | 1.4E-08 | 3.6E-05 | 28 | 3000 | 2.8 | 221.4 |
| IL6R-BP429 | E233D/G237D/P238D/V264I/S267G/H268E/P271G/A330R | 6.2E-09 | 1.7E-07 | 3.5E-06 | 5.4E-09 | 6.8E-05 | 31.5 | 648.1 | 6.5 | 574.1 |
| IL6R-BP430 | E233D/G237D/P238D/V266L/S267G/H268E/P271G/A330R | 1.7E-08 | 2.2E-07 | 1.1E-05 | 1.2E-08 | 3.5E-05 | 18.5 | 909.1 | 4.9 | 256.2 |
| IL6R-BP431 | E233D/G237D/P238D/S267G/H268E/E269D/P271G/A330R | 3.6E-09 | 4.1E-07 | 7.6E-06 | 1.2E-08 | 3.2E-05 | 34.6 | 649.6 | 2.7 | 265 |
| IL6R-BP433 | E233D/G237D/P238D/H268D/P271G/Y296D/A330K/I332T | 7.5E-10 | 6.8E-07 | 7.3E-06 | 3.4E-08 | 2.6E-05 | 20 | 216 | 1.6 | 91.7 |
| IL6R-BP434 | E233D/G237D/P238D/H268D/P271G/Y296D/K326D/A330R/I332T | 5.5E-10 | 3.4E-07 | 4.1E-06 | 1.2E-08 | 2.5E-05 | 27.2 | 333.3 | 3.3 | 252 |
| IL6R-BP435 | E233D/G237D/P238D/H268D/P271G/Y296D/K326A/A330R/I332T | 1.0E-09 | 4.2E-07 | 3.4E-06 | 1.6E-08 | 2.2E-05 | 27.1 | 217.9 | 2.6 | 198.7 |
| IL6R-BP436 | E233D/G237D/P238D/S267A/H268E/P271G/Y296D/A330R/I332T | 2.6E-10 | 2.2E-07 | 2.1E-06 | 5.1E-09 | 1.3E-05 | 43.8 | 411 | 4.9 | 606.7 |
| IL6R-BP437 | G237D/P238D/S267A/H268E/P271G/Y296D/A330R/I332T | 7.5E-10 | 2.2E-07 | 1.4E-06 | 5.9E-09 | 1.1E-05 | 37.7 | 236.5 | 4.9 | 523.6 |
| IL6R-BP438 | E233D/G237D/P238D/S267A/H268E/P271G/A330R/I332T | 2.1E-10 | 1.8E-07 | 1.6E-06 | 5.5E-09 | 6.8E-06 | 32.7 | 293.6 | 6.2 | 568.8 |
| IL6R-BP439 | E233D/G237D/P238D/V264I/V266L/S267A/H268E/P271G/A330R | 8.7E-09 | 1.3E-07 | 2.8E-06 | 6.1E-09 | 6.6E-05 | 20.9 | 460.5 | 8.7 | 509.9 |
| IL6R-BP440 | E233D/G237D/P238D/V264I/H268E/P271G/A330R | 8.7E-09 | 1.3E-07 | 1.6E-06 | 5.2E-09 | 2.8E-05 | 24 | 307.1 | 8.8 | 595 |
| IL6R-BP441 | E233D/G237D/P238D/V266L/H268E/P271G/A330R | 1.7E-08 | 3.6E-07 | 8.8E-06 | 1.5E-08 | 3.7E-05 | 24 | 582.8 | 3 | 205.3 |

Table 28-2 is the continuation of Table 28-1.

[Table 28-2]

| | | | | | | | | | |
|---|---|---|---|---|---|---|---|---|---|
| IL6R-BP442 | E233D/G237D/P238D/H268E/E269D/P271G/A330R | 4.5E-09 | 3.8E-07 | 4.7E-06 | 1.2E-08 | 2.5E-05 | 30.6 | 379 | 2.9 | 250 |
| IL6R-BP443 | E233D/G237D/P238D/V266L/H268E/E269D/P271G/A330R | 1.8E-08 | 5.1E-07 | 9.5E-06 | 2.3E-08 | 2.3E-05 | 21.7 | 406 | 2.2 | 132.5 |
| IL6R-BP445 | E233D/G237D/P238D/V264I/S267A/H268E/P271G/A330R | 2.0E-09 | 8.0E-08 | 1.5E-06 | 2.6E-09 | 2.4E-05 | 31 | 581.4 | 13.8 | 1201.6 |
| IL6R-BP479 | E233D/G237D/P238D/V264I/V266L/S267A/H268E/P271G | 5.3E-09 | 9.0E-07 | 1.5E-05 | 5.6E-08 | 4.0E-05 | 16.1 | 268.3 | 1.2 | 55.5 |
| IL6R-BP480 | E233D/G237D/P238D/V266L/H268E/E269D/P271G | 1.3E-08 | 6.3E-06 | 2.1E-05 | 2.0E-07 | 4.6E-05 | 32.1 | 107.7 | 0.2 | 15.9 |
| IL6R-BP481 | E233D/G237D/P238D/V264I/S267A/H268E/P271G | 1.0E-09 | 4.0E-07 | 6.8E-06 | 1.9E-08 | 2.4E-05 | 20.5 | 350.5 | 2.8 | 159.8 |
| IL6R-BP483 | E233D/G237D/P238D/V266L/S267A/H268E/P271G | 1.3E-09 | 1.3E-06 | 1.8E-05 | 7.8E-08 | 2.5E-05 | 16.8 | 230.8 | 0.8 | 39.7 |
| IL6R-BP484 | E233D/G237D/P238D/S267A/H268E/E269D/P271G | 8.2E-10 | 7.8E-07 | 1.1E-05 | 4.6E-08 | 2.5E-05 | 17.1 | 240.7 | 1.4 | 67.8 |
| IL6R-BP487 | E233D/G237D/P238D/V264I/S267A/H268E/P271G/A330R/P396M | 1.2E-09 | 3.9E-08 | 8.4E-07 | 1.2E-09 | 1.0E-05 | 33.8 | 730.4 | 28.3 | 2695.7 |
| IL6R-BP488 | E233D/G237D/P238D/V264I/S267A/H268E/P271G/Y296D/A330R | 2.2E-09 | 7.4E-08 | 1.6E-06 | 1.9E-09 | 2.0E-05 | 40.1 | 864.9 | 14.8 | 1675.7 |
| IL6R-BP489 | E233D/G237D/P238D/V264I/S267A/H268E/P271G/Y296D/A330R/P396M | 1.3E-09 | 4.3E-08 | 8.7E-07 | 1.0E-09 | 1.2E-05 | 42.8 | 870 | 25.7 | 3100 |
| IL6R-BP490 | G237D/P238D/V264I/S267A/H268E/P271G/A330R | 4.5E-09 | 1.1E-07 | 2.4E-06 | 2.4E-09 | 2.3E-05 | 46.7 | 1000 | 9.8 | 1291.7 |
| IL6R-BP491 | G237D/P238D/V264I/S267A/H268E/P271G/Y296D/A330R | 5.3E-09 | 1.2E-07 | 2.2E-06 | 3.0E-09 | 2.1E-05 | 38.8 | 723.7 | 9.3 | 1019.7 |
| IL6R-BP492 | P238D/V264I/S267A/H268E/P271G | 7.9E-10 | 9.2E-07 | 1.6E-05 | 2.4E-08 | 3.6E-05 | 38.8 | 678 | 1.2 | 131.4 |
| IL6R-BP493 | P238D/V264I/S267A/H268E/P271G/Y296D | 8.2E-10 | 1.1E-06 | 1.9E-05 | 2.1E-08 | 3.5E-05 | 52.1 | 900.5 | 1 | 146.9 |
| IL6R-BP494 | G237D/P238D/S267A/H268E/P271G/Y296D/A330R | 3.9E-09 | 2.5E-07 | 5.4E-06 | 6.6E-09 | 4.0E-05 | 38.6 | 820.7 | 4.3 | 471.1 |
| IL6R-BP495 | G237D/P238D/S267G/H268E/P271G/Y296D/A330R | 8.3E-09 | 4.9E-07 | 1.2E-05 | 9.7E-09 | 3.3E-05 | 50.9 | 1243.5 | 2.2 | 321.2 |
| IL6R-BP496 | E233D/G237D/P238D/V264I/S267A/H268E/P271G/Y296D | 1.2E-09 | 4.7E-07 | 3.7E-06 | 1.8E-08 | 3.0E-05 | 25.5 | 201.1 | 2.3 | 168.5 |
| IL6R-BP497 | E233D/G237D/P238D/V264I/S267A/H268E/P271G/A327G/A330R | 2.1E-09 | 8.5E-08 | 9.6E-07 | 4.1E-09 | 2.8E-05 | 21 | 236.5 | 12.9 | 763.5 |
| IL6R-BP498 | E233D/G237D/P238D/V264I/S267A/H268E/P271G/A330R/P396L | 1.3E-09 | 5.1E-08 | 9.3E-07 | 1.7E-09 | 1.0E-05 | 30.8 | 563.6 | 21.7 | 1878.8 |
| IL6R-BP499 | E233D/G237D/P238D/V264I/S267A/H268E/P271G/Y296D/A330R/P396L | 1.2E-09 | 4.9E-08 | 1.0E-06 | 1.5E-09 | 1.2E-05 | 33.8 | 684.9 | 22.3 | 2123.3 |
| IL6R-BP500 | G237D/P238D/V264I/S267A/H268E/P271G/Y296D | 2.3E-09 | 7.2E-07 | 2.5E-05 | 2.4E-08 | 3.9E-05 | 29.9 | 1033.1 | 1.5 | 128.1 |
| IL6R-BP501 | G237D/P238D/V264I/S267A/H268E/P271G | 2.1E-09 | 6.3E-07 | 1.4E-05 | 2.5E-08 | 1.9E-05 | 25.1 | 555.6 | 1.7 | 123 |
| IL6R-BP502 | E233D/G237D/P238D/V264I/S267A/H268E/P271G/Y296D/A327G/A330R | 2.1E-09 | 1.1E-07 | 1.3E-06 | 3.7E-09 | 2.4E-05 | 29.5 | 352.3 | 10.1 | 840.1 |
| IL6R-BP503 | E233D/G237D/P238D/V264I/S267A/H268E/P271G/Y296D/A327G/A330R/P396M | 1.2E-09 | 5.7E-08 | 8.6E-07 | 1.7E-09 | 2.1E-05 | 33.2 | 502.9 | 19.4 | 1812.9 |
| IL6R-BP504 | E233D/G237D/P238D/V264I/S267A/H268E/P271G/E272P | 1.4E-09 | 4.5E-07 | 1.6E-05 | 2.4E-08 | 3.4E-05 | 18.5 | 658.4 | 2.4 | 127.6 |
| IL6R-BP505 | E233D/G237D/P238D/V264I/S267A/H268E/P271G/E272D | 1.1E-09 | 4.3E-07 | 1.1E-05 | 2.1E-08 | 3.8E-05 | 20 | 514 | 2.6 | 144.9 |
| IL6R-BP506 | E233D/G237D/P238D/V264I/S267A/H268E/P271G/E272P/Y296D/A330R | 3.1E-09 | 1.2E-07 | 2.5E-06 | 3.4E-09 | 5.5E-05 | 35.1 | 731 | 9.2 | 906.4 |
| IL6R-BP507 | E233D/G237D/P238D/V264I/S267A/H268E/P271G/E272P/A330R | 2.6E-09 | 1.0E-07 | 1.8E-06 | 2.9E-09 | 2.6E-05 | 34.2 | 618.6 | 11.1 | 1065.3 |
| IL6R-BP508 | E233D/G237D/P238D/V264I/S267A/H268E/P271G/E272P/Y296D | 1.4E-09 | 5.4E-07 | 2.0E-05 | 2.1E-08 | 6.1E-05 | 26 | 961.5 | 2 | 149 |

Table 28-3 is the continuation of Table 28-2.

[Table 28-3]

| | | | | | | | | | | |
|---|---|---|---|---|---|---|---|---|---|---|
| IL6R-BP509 | E233D/G237D/P238D/V264I/S267A/H268E/P271G/E272D/Y296D | 1.1E-09 | 5.2E-07 | 7.9E-06 | 1.8E-08 | 2.3E-05 | 29.2 | 443.8 | 2.1 | 174.2 |
| IL6R-BP510 | G237D/P238D/V264I/S267A/H268E/P271G/E272P/A330R | 6.0E-09 | 1.7E-07 | 4.0E-06 | 3.8E-09 | 2.5E-05 | 43.5 | 1041.7 | 6.6 | 807.3 |
| IL6R-BP511 | G237D/P238D/V264I/S267A/H268E/P271G/E272P/Y296D/A330R | 6.0E-09 | 1.8E-07 | 4.3E-06 | 3.5E-09 | 7.1E-05 | 50.6 | 1235.6 | 6.3 | 890.8 |
| IL6R-BP531 | E233D/G237D/P238D/V264I/S267G/H268E/P271G/Y296D/A330R/P396M | 9.4E-09 | 1.2E-07 | 3.5E-06 | 3.8E-09 | 2.7E-05 | 33.1 | 933.3 | 8.9 | 826.7 |
| IL6R-BP532 | E233D/G237D/P238D/V264I/H268E/P271G/Y296D/A330R/P396M | 1.2E-08 | 9.4E-08 | 1.9E-06 | 3.2E-09 | 2.6E-05 | 29.3 | 593.8 | 11.7 | 968.8 |
| IL6R-BP533 | E233D/G237D/P238D/V264I/S267G/H268E/P271G/Y296D/A330R/P396L | 7.7E-09 | 1.2E-07 | 2.6E-06 | 4.1E-09 | 2.7E-05 | 29.3 | 634.1 | 9.2 | 756.1 |
| IL6R-BP534 | E233D/G237D/P238D/V264I/H268E/P271G/Y296D/A330R/P396L | 9.3E-09 | 9.1E-08 | 1.8E-06 | 3.0E-09 | 2.5E-05 | 30.7 | 606.1 | 12.1 | 1043.8 |
| IL6R-BP535 | E233D/G237D/P238D/V264I/S267G/H268E/P271G/Y296D/A327G/A330R/P396M | 1.1E-08 | 9.2E-08 | 3.2E-06 | 4.0E-09 | 3.3E-05 | 23.2 | 806 | 11.9 | 780.9 |
| IL6R-BP536 | E233D/G237D/P238D/V264I/H268E/P271G/Y296D/A327G/A330R/P396M | 8.9E-09 | 7.9E-08 | 1.3E-06 | 3.0E-09 | 2.3E-05 | 26.6 | 437.7 | 13.9 | 1043.8 |
| IL6R-BP537 | G237D/P238D/V264I/S267G/H268E/P271G/A330R | 2.9E-08 | 2.7E-07 | 3.1E-06 | 6.9E-09 | 3.6E-05 | 39.1 | 447.3 | 4.1 | 447.3 |
| IL6R-BP538 | G237D/P238D/V264I/H268E/P271G/A330R | 5.5E-08 | 2.0E-07 | 3.0E-06 | 5.3E-09 | 3.3E-05 | 38.6 | 568.2 | 5.4 | 587.1 |
| IL6R-BP539 | G237D/P238D/V264I/S267G/H268E/P271G/E272P/Y296D/A330R | 6.4E-08 | 3.3E-07 | 5.6E-06 | 8.4E-09 | 3.4E-05 | 39 | 666.7 | 3.4 | 369 |
| IL6R-BP540 | G237D/P238D/V264I/H268E/P271G/E272P/Y296D/A330R | 9.6E-08 | 2.1E-07 | 4.6E-06 | 5.7E-09 | 3.9E-05 | 36.6 | 802.8 | 5.2 | 541 |
| IL6R-BP549 | G237D/P238D/S267G/H268E/P271G/A330R | 1.8E-08 | 5.7E-07 | 1.1E-05 | 1.6E-08 | 2.4E-05 | 35.9 | 696.2 | 1.9 | 196.2 |
| IL6R-BP550 | G237D/P238D/V264I/S267G/H268E/P271G/E272D/Y296D/A330R | 2.5E-08 | 3.4E-07 | 5.0E-06 | 7.6E-09 | 4.8E-05 | 44.2 | 655.3 | 3.3 | 406.3 |
| IL6R-BP551 | G237D/P238D/V264I/H268E/P271G/E272D/Y296D/A330R | 3.2E-08 | 2.5E-07 | 2.8E-06 | 6.4E-09 | 4.8E-05 | 38.1 | 435.5 | 4.5 | 482.1 |
| IL6R-BP552 | E233D/G237D/P238D/V264I/S267A/H268E/P271G/E272D/Y296D/A330R | 3.2E-09 | 9.7E-08 | 1.9E-06 | 2.6E-09 | 3.0E-05 | 37.3 | 733.6 | 11.4 | 1196.9 |
| IL6R-BP553 | E233D/G237D/P238D/V264I/S267A/H268E/P271G/E272D/A330R | 3.4E-09 | 8.6E-08 | 1.4E-06 | 3.1E-09 | 2.0E-05 | 27.8 | 453.1 | 12.8 | 1003.2 |
| IL6R-BP554 | G237D/P238D/V264I/S267A/H268E/P271G/E272D/A330R | 8.0E-09 | 1.5E-07 | 2.3E-06 | 4.4E-09 | 2.4E-05 | 32.7 | 518 | 7.6 | 698.2 |
| IL6R-BP555 | G237D/P238D/V264I/S267A/H268E/P271G/E272D/Y296D/A330R | 9.4E-09 | 1.6E-07 | 3.2E-06 | 4.1E-09 | 3.0E-05 | 39.7 | 778.6 | 6.7 | 754.3 |
| IL6R-BP556 | G237D/P238D/V264I/S267G/H268E/P271G/Y296D/A330R | 4.3E-08 | 3.0E-07 | 5.8E-06 | 8.4E-09 | 6.0E-05 | 35.4 | 692.1 | 3.7 | 369.9 |
| IL6R-BP557 | G237D/P238D/S267G/H268D/P271G/Y296D/A330R | 1.3E-08 | 8.5E-07 | 1.5E-05 | 2.0E-08 | 2.9E-05 | 42 | 746.3 | 1.3 | 154.2 |
| IL6R-BP558 | G237D/P238D/V264I/S267G/H268E/P271G/E272D/A330R | 1.3E-08 | 3.3E-07 | 4.9E-06 | 9.0E-09 | 3.6E-05 | 36.4 | 543.2 | 3.4 | 343.7 |
| IL6R-BP559 | P238D/V264I/S267A/H268E/P271G/E272D/Y296D | 1.1E-09 | 1.6E-06 | 2.0E-05 | 2.8E-08 | 4.4E-05 | 58.4 | 711.7 | 0.7 | 110.3 |
| IL6R-BP560 | P238D/S267G/H268E/P271G/Y296D/A330R | 5.6E-09 | 4.2E-06 | 3.1E-05 | 1.8E-07 | 4.1E-05 | 22.8 | 168.5 | 0.3 | 16.8 |
| IL6R-BP561 | E233D/G237D/P238D/H268D/P271G/E272D/Y296D/A330R | 9.4E-09 | 5.1E-07 | 5.3E-06 | 1.8E-08 | 3.7E-05 | 28 | 291.2 | 2.2 | 170.3 |
| IL6R-BP562 | G237D/P238D/H268D/P271G/E272D/Y296D/A330R | 2.5E-08 | 6.8E-07 | 1.1E-05 | 2.4E-08 | 5.3E-05 | 29 | 466.1 | 1.6 | 131.4 |
| IL6R-BP563 | E233D/G237D/P238D/H268E/P271G/E272D/Y296D/A330R | 1.2E-08 | 4.6E-07 | 8.3E-06 | 1.6E-08 | 3.8E-05 | 29.1 | 525.3 | 2.4 | 196.2 |
| IL6R-BP564 | G237D/P238D/H268E/P271G/E272D/Y296D/A330R | 3.1E-08 | 5.8E-07 | 1.0E-05 | 2.2E-08 | 4.9E-05 | 26.2 | 454.5 | 1.9 | 140.9 |
| IL6R-BP565 | E233D/G237D/P238D/S267A/H268E/P271G/Y296D/A330R | 2.4E-09 | 2.3E-07 | 4.7E-06 | 5.5E-09 | 2.1E-05 | 41.5 | 856.1 | 4.8 | 564.7 |

Of the variants shown in Table 28, IL6R-BP253/IL6R-L added with the existing alterations that enhance the FcγRIIb binding exhibited FcγRIIb- and FcγRIIaR-binding activities increased to 277 times and 529 times those of IL6R-B3/IL6R-L prior to introduction of the alterations, respectively. Furthermore, the FcγRIa-binding activity of IL6R-BP253/IL6R-L was also greater than that of IL6R-B3/IL6R-L. Meanwhile, the FcγRIIaH binding and FcγRIIIaV binding of IL6R-BP253/IL6R-L were reduced as compared to those of IL6R-B3/IL6R-L. Among other variants, IL6R-BP436/IL6R-L and IL6R-BP438/IL6R-L showed an FcγRIa binding slightly enhanced as compared to that of IL6R-B3/IL6R-L prior to introduction of the alterations. All other variants showed a reduced FcγRIa binding. In addition, all the variants exhibited reduced FcγRIIaH binding and FcγRIIIaV binding as compared to those of IL6R-B3/IL6R-L.

The variants produced as described in this section were compared with IL6R-BP253/IL6R-L, which is an existing variant with augmented FcγRIIb binding. The KD(IIaH)/KD(IIb) value of IL6R-BP480/IL6R-L, which was the lowest, was 107.7, while the value of IL6R-BP426/IL6R-L, which was the highest, was 8362. Thus, the values of the two variants were higher than 107.1 of IL6R-BP253/IL6R-L. Meanwhile, the KD(IIaR)/KD(IIb) value of IL6R-BP479/IL6R-L, which was the lowest, was 16.1, while the value of IL6R-BP559/IL6R-L, which was the highest, was 58.4. Thus, the values of the two variants were higher than 0.2 of IL6R-BP253/IL6R-L. These results demonstrate that the selectivity for FcγRIIb of the variants shown in Table 28 has been improved as compared to the selectivity of the variants added with the existing modifications that augment the FcγRIIb binding. In particular, all of IL6R-BP559/IL6R-L, IL6R-BP493/IL6R-L, IL6R-BP557/IL6R-L, IL6R-BP492/IL6R-L, and IL6R-BP500/IL6R-L have FcγRIIb-binding activity increased by 100 times or more, while their FcγRIIaR binding remained at 1.5 times or less, as compared to IL6R-B3/IL6R-L. Thus, the effect of the augmented FcγRIIb binding is expected to be achieved while avoiding side effects resulting from augmentation of FcγRIIaR binding.

Regarding IL6R-BP489/IL6R-L, IL6R-BP487/IL6R-L, IL6R-BP499/IL6R-L, IL6R-BP498/IL6R-L, IL6R-BP503/IL6R-L, IL6R-BP488/IL6R-L, IL6R-BP490/IL6R-L, IL6R-BP445/IL6R-L, IL6R-BP552/IL6R-L, IL6R-BP507/IL6R-L, IL6R-BP536/IL6R-L, IL6R-BP534/IL6R-L, IL6R-491/IL6R-L, IL6R-BP553/IL6R-L, IL6R-BP532/IL6R-L, IL6R-BP506/IL6R-L, IL6R-BP511/IL6R-L, IL6R-BP502/IL6R-L, IL6R-BP531/IL6R-L, IL6R-BP510/IL6R-L, IL6R-BP535/IL6R-L, IL6R-BP497/IL6R-L, IL6R-BP533/IL6R-L, IL6R-BP555/IL6R-L, IL6R-BP554/IL6R-L, IL6R-BP436/IL6R-L, IL6R-BP423/IL6R-L, IL6R-BP440/IL6R-L, IL6R-BP538/IL6R-L, IL6R-BP429/IL6R-L, IL6R-BP438/IL6R-L, IL6R-BP565/IL6R-L, IL6R-BP540/IL6R-L, BP426/IL6R-L, IL6R-BP437/IL6R-L, IL6R-BP439/IL6R-L, IL6R-BP551/IL6R-L, IL6R-BP494/IL6R-L, IL6R-BP537/IL6R-L, IL6R-BP550/IL6R-L, IL6R-BP556/IL6R-L, IL6R-BP539/IL6R-L, IL6R-BP558/IL6R-L, IL6R-BP425/IL6R-L, and IL6R-BP495/IL6R-L, their FcγRIIb binding was higher than that of IL6R-BP253/IL6R-L added with the existing modification that augments the FcγRIIb binding. Of the above, the augmentation of the FcγRIIb binding ranges from 321 times (lowest) to 3100 times (highest), compared to the binding of IL6R-B3/IL6R-L (which is defined to be 1), from IL6R-BP495/IL6R-L to IL6R-BP489/IL6R-L, respectively. Thus, it can be said that these variants are superior in both of the level and selectivity of augmentation of the FcγRIIb binding activity compared to the prior art.

[Example 20] Preparation of antibodies that bind to human IgA in a calcium-dependent manner (20-1) Preparation of human IgA (hIgA)

Examples 2 to 4 show that molecules that have augmented mouse FcγR binding and which bind in a pH-dependent manner to human IL-6 receptor as an antigen, can significantly reduce the concentration of the antigen in plasma. Then, an additional test was carried out using antibodies to human IgA as an antigen, in order to assess the presence of a similar effect of eliminating soluble antigens from plasma in a living organism administered with antibodies that have augmented mouse FcγR binding and which bind in a pH-dependent manner to antigens other than human IL-6 receptor. The antigen, human IgA (hereinafter also referred to as hIgA) (its variable region is from an anti-human IL6R antibody) was prepared using the following recombination technique. hIgA was expressed by culturing host cells containing a recombinant vectors carrying H (WT)-IgA1 (SEQ ID NO: 145) and L (WT) (SEQ ID NO: 42), and purified by a method known to those skilled in the art using ion-exchange chromatography and gel filtration chromatography.

(20-2) Expression and purification of an antibody that binds to hIgA

GA2-IgG1 (heavy chain, SEQ ID NO: 146; light chain, SEQ ID NO: 147) is an antibody that binds to hIgA. A DNA sequence encoding GA2-IgG1 (heavy chain, SEQ ID NO: 146; light chain, SEQ ID NO: 147) was inserted into an animal cell expression plasmid by a method known to those skilled in the art. The antibody was expressed and purified by the method described below. Cells of the human fetal kidney cell-derived FreeStyle 293-F line (Invitrogen) were suspended in FreeStyle 293 Expression Medium (Invitrogen). The cell suspension was plated at a cell density of 1.33 x $10^6$ cells/ml in 3 ml to each well of a 6-well plate. Then, the prepared plasmid was introduced into cells by the lipofection method. The cells were cultured in a $CO_2$ incubator (37°C, 8%$CO_2$, 90 rpm) for 4 days. From the isolated culture supernatant, the antibody was purified by a method known to those skilled in the art using rProtein A Sepharose™ Fast Flow (Amersham Biosciences). The absorbance (wavelength: 280 nm) of the solution of the purified antibody was measured using a spectrophotometer. The antibody concentration was determined using the extinction coefficient calculated from the measured value by the PACE method (Protein Science (1995) 4, 2411-2423).

(20-3) Assessment of the isolated antibody for its calcium-dependent hIgA-binding ability

The antibody isolated as described in Example (20-2) was assessed for its hIgA-binding activity (dissociation constant, KD (M)) using Biacore T200 (GE Healthcare). The binding activity was measured using as a running buffer, 0.05% tween20, 20 mmol/l ACES, 150 mmol/l NaCl (pH 7.4 or pH 5.8) containing 3 μM or 1.2 mM $CaCl_2$. An appropriate amount of recombinant Protein A/G (Thermo Scientific) was immobilized onto a Sensor chip CM5 (GE Healthcare) by an amino coupling method, and the antibody was allowed to bind thereto. Then, an appropriate concentration of hIgA (described in (A1-1)) was injected as an analyte and allowed to interact with the antibody on the sensor chip. The measurement was carried out at 37°C. After measurement, 10 mmol/L glycine-HCl (pH 1.5) was injected to regenerate the sensor chip. From the measurement result, the dissociation constant KD (M) was calculated by curve fitting analysis and equilibrium analysis using Biacore T200 Evaluation Software (GE Healthcare). The result is shown in Table 29. GA2-IgG1 strongly bound to hIgA at a $Ca^{2+}$ concentration of 1.2 mM, and weakly bound to hIgA at a $Ca^{2+}$ concentration of 3 μM. Meanwhile, at a $Ca^{2+}$ concentration of 1.2 mM, GA2-IgG1 strongly bound to human IgA at pH 7.4, and weakly bound to human IgA at pH 5.8. In summary, GA2-IgG1 was demonstrated to bind to human IgA in a pH- and calcium-dependent manner.

[Table 29]

| Antibody name | Condition | Fit | ka | kd | KD[M] |
|---|---|---|---|---|---|
| GA2-IgG1 | pH7.4, 1.2mM Ca | 1:1binding model | 4.0E+05 | 1.6E-02 | 3.9E-08 |
| | pH7.4, 3μM Ca | Steady State Affinity | - | - | 6.7E-06 |
| | pH5.8, 1.2mM Ca | Steady State Affinity | - | - | 4.0E-06 |
| | pH5.8, 3μM Ca | Steady State Affinity | - | - | 5.0E-06 |

[Example 21] Preparation of antibody variants that bind to hIgA in a calcium-dependent manner

Next, to further accelerate antigen (hIgA) elimination from plasma, GA2-F1087 (heavy chain, SEQ ID NO: 148) was produced by substituting Asp for Lys at position 326 (EU numbering) in GA2-IgG1 for augmenting the mouse FcγR binding of GA2-IgG1 that binds to hIgA in a calcium-dependent manner. A DNA sequence encoding GA2-F1087 (heavy chain, SEQ ID NO: 148; light chain, SEQ ID NO: 147) was inserted into an animal expression plasmid by a method known to those skilled in the art. Antibody variants were expressed by the above-described method using the plasmid. The concentrations of the variants were measured after purification. Antibodies comprising the above modification exhibited significantly augmented mouse FcγR binding, as shown in Example (4-3).

[Example 22] Assessment of the effect on the plasma antigen retention in normal mice administered with Ca-dependent hIgA-binding antibodies

(22-1) *In vivo* tests using normal mice

hIgA (human IgA, prepared as described in Example (20-1)) was administered alone or in combination with an anti-hIgA antibody to normal mice (C57BL/6J mouse, Charles River Japan). After administration, the *in vivo* dynamics of hIgA and anti-hIgA antibodies was assessed. An hIgA solution (80 μg/ml) or a mixed solution of hIgA and an anti-hIgA antibody was administered once at a dose of 10 ml/kg into the caudal vein. The anti-hIgA antibodies used were GA2-IgG1 and GA2-F1087 described above.

In all of the mixed solutions, the concentration of hIgA was 80 μg/ml, and the concentration of anti-hIgA antibody was 2.69 mg/ml. In this experiment, the anti-hIgA antibodies were present significantly in excess over hIgA, and thus most of hIgA was thought to bind to the antibodies. In the group administered with GA-IgG1, from the mice, the blood was collected five minutes, seven hours, one day, two days, three days, and seven days after administration. Meanwhile, in the group administered with GA-F1087, from the mice, the blood was collected five minutes, 30 minutes, one hour, two hours, one day, three days, and seven days after administration. The collected blood was immediately centrifuged at 12,000 rpm and 4°C for 15 minutes to isolate the plasma. The isolated plasma was stored in a freezer at -20°C or below until use.

(22-2) Determination of the plasma anti-hIgA antibody concentration in normal mice by the ELISA method

Anti-hIgA antibody concentrations in mouse plasma were measured by the ELISA method. First, to prepare an anti-human IgG-immobilized plate, Anti-Human IgG (γ-chain specific) F(ab')2 Fragment of Antibody (SIGMA) was aliquoted to each well of a Nunc-Immuno Plate, MaxiSorp (Nalge nunc International), and the plate was allowed to stand at 4°C overnight. Calibration curve samples of anti-hIgA antibody prepared as standard solutions for the plasma concentration (0.5, 0.25, 0.125, 0.0625, 0.03125, 0.01563, and 0.007813 μg/ml) and assay samples of mouse plasma diluted 100 times or more, were aliquoted to the above-mentioned anti-human IgG-immobilized plate. After one hour of incubation of the plate at 25°C, Goat Anti-Human IgG (γ chain specific) Biotin (BIOT) Conjugate (Southern Biotechnology Associates Inc.) was aliquoted to each well of the plate. Then, the plate was incubated at 25°C for one hour. Next, Streptavidin-PolyHRP80 (Stereospecific Detection Technologies) was aliquoted to each well of the plate. Then, the plate was incubated at 25°C for one hour. Chromogenic reaction was performed using as a substrate TMB One Component HRP Microwell Substrate (BioFX Laboratories). After terminating the reaction with IN sulfuric acid (Showa Chemical), the absorbance of the reaction solution in each well was measured at 450 nm with a microplate reader. Anti-hIgA antibody concentrations in mouse plasma were determined based on the absorbance of the standard curve using the analysis software SOFTmax PRO (Molecular Devices). A time course of the antibody concentrations of GA2-IgG1 and GA2-F1087 in the plasma of normal mice after intravenous administration, which were measured by the method described above, is shown in Fig. 45. The results demonstrate that, with respect to the clone GA2-IgG1 that has pH-dependent, strong hIgA-binding activity, the plasma concentration of the antibody is not significantly reduced even if the FcγR binding is augmented.

(22-3) Determination of the plasma hIgA concentration by the ELISA method

hIgA concentrations in mouse plasma were measured by the ELISA method. First, to prepare an anti-human IgA-immobilized plate, Goat anti-Human IgA Antibody (BETHYL) was aliquoted to each well of a Nunc-Immuno Plate, MaxiSoup (Nalge nunc International), and the plate was allowed to stand at 4°C overnight. Calibration curve samples of hIgA were prepared as standard solutions for the plasma concentration (0.4, 0.2, 0.1, 0.05, 0.025, 0.0125, and 0.00625 μg/ml), and used. 100 μl each of the calibration curve samples and assay samples of mouse plasma diluted 100 times or more, was combined with 200 μl of 500 ng/ml hsL6R. This was mixed and incubated at room temperature for one hour. Then, 100 μl of the mixtures was aliquoted to the anti-human IgA-immobilized plate. The plate was allowed to stand at room temperature for one hour. Next, Biotinylated Anti-human IL-6 R Antibody (R&D) was aliquoted to each well of the plate. After one hour of incubation at room temperature, Streptavidin-PolyHRP80 (Stereospecific Detection Technologies) was aliquoted to each well of the plate. The plate was incubated at room temperature for one hour. Chromogenic reaction was performed using as a substrate TMB One Component HRP Microwell Substrate (BioFX Laboratories). After terminating the reaction with IN sulfuric acid (Showa Chemical), the absorbance of the reaction solution in each well was measured at 450 nm with a microplate reader. The concentrations in mouse plasma were determined based on the absorbance of the standard curve using the analysis software SOFTmax PRO (Molecular Devices). A time course of the hIgA concentration in the plasma of normal mice after intravenous administration, which was measured by the above method, is shown in Fig. 46.

The result showed that, in mice administered with hIgA in combination with GA2-IgG1 having a Ca-dependent hIgA-binding activity of 100 times or more greater, hIgA elimination was accelerated compared to the administration of hIgA alone. Meanwhile, in the plasma of mice administered with GA2-F1087 with augmented binding to hIgA and FcγR, the concentration of hIgA was reduced below the measurable range (0.006 μg/ml or more) one day after administration, and thus the hIgA elimination was significantly accelerated compared to the plasma of mice administered with GA-IgG1. The findings described in Examples 2 to 7 above demonstrate the increased antigen elimination effect of antibodies with augmented FcγR binding in mice administered with IL6R and anti-IL6R antibody. Likewise, a similar effect was also demonstrated to be achieved in mice administered with the hIgA antigen and anti-hIgA antibody.

[Example 23] Preparation of a pH-dependent anti-IgE antibody

(23-1) Preparation of an anti-human IgE antibody

In order to prepare a pH-dependent anti-human IgE antibody, human IgE (heavy chain, SEQ ID NO: 149; light chain, SEQ ID NO: 150) (its variable region is from an anti-human glypican 3 antibody) was expressed as an antigen using FreeStyle293 (Life Technologies). The expressed human IgE was prepared and purified by a general chromatographic method known to those skilled in the art. An antibody that binds to human IgE in a pH-dependent manner was selected from many antibodies isolated. The heavy chain and light chain variable regions of the selected anti-human IgE antibody were fused with a human IgG1 heavy chain constant region and a human light chain constant region, and the resulting antibody gene was inserted into a vector. Using the vector, a recombinant anti-human IgE antibody was expressed and purified. The prepared antibody was named clone 278 (hereinafter referred to as 278-IgG1; heavy chain, SEQ ID NO: 151, light chain, SEQ ID NO: 152).

(23-2) Assessment of the anti-human IgE antibody for the human IgE-binding activity and pH-dependent binding activity

Antibodies capable of dissociating antigens in the endosome can be produced in such a way that they bind to antigens not only in a pH-dependent manner but also in a Ca-dependent manner. Thus, 278-IgG1 and the control human IgG1 antibody Xolair (omalizumab, Novartis) without pH/Ca-dependent IgE-binding ability were assessed for the pH-dependent binding ability and pH/Ca-dependent binding ability to human IgE (hIgE). Specifically, 278-IgG1 and Xolair were assessed for their hIgE-binding activity (dissociation constant, KD (M)) using Biacore T200 (GE Healthcare). Measurements were carried out using the following three types of running buffers:

1.2 mmol/l $CaCl_2$, 0.05% tween20, 20 mmol/l ACES, 150 mmol/l NaCl, pH 7.4
1.2 mmol/l $CaCl_2$, 0.05% tween20, 20 mmol/l ACES, 150 mmol/l NaCl, pH 5.8
3 μmol/l $CaCl_2$, 0.05% tween20, 20 mmol/l ACES, 150 mmol/l NaCl, pH 5.8

An appropriate amount of a peptide (hereinafter referred to as "biotinylated GPC3 peptide") resulting from adding biotin to the C-terminal Lys of a chemically synthesized sequence derived from human glypican 3 protein (SEQ ID NO: 153) was loaded on a Sensor chip SA (GE Healthcare), and immobilized on the Sensor chip SA based on biotin/streptavidin affinity. An appropriate concentration of human IgE was injected and captured by the biotinylated GPC3 peptide to immobilize human IgE on the chip. An appropriate concentration of 278-IgG1 was injected as an analyte, and allowed to interact with human IgE on the sensor chip. Then, 10 mmol/L glycine-HCl (pH 1.5) was injected to regenerate the sensor chip. All of the assay for the interaction was performed at 37°C. Using Biacore T200 Evaluation Software (GE Healthcare), the assay results were analyzed by curve fitting to determine the binding rate constant ka (1/Ms) and dissociation rate constant kd (1/s). Dissociation constant KD (M) was calculated from the above constants. Then, the pH-dependent binding was assessed by calculating the KD ratio of each antibody between the conditions of pH 5.8/1.2 mM Ca and pH 7.4/1.2 mM Ca. The pH/Ca-dependent binding was assessed by calculating the KD ratio of each antibody between the conditions of pH 5.8/3 μM Ca and pH 7.4/1.2 mM Ca. The results are shown in Table 30.

[Table 30]

| **Antibody name (abbreviated)** | **Buffer condition** | **ka (1/Ms)** | **kd (1/s)** | **KD (M)** | **pH dependency** | **pH/Ca dependency** |
|---|---|---|---|---|---|---|
| | | | | | **KD(pH5.8, 1.2 mM Ca) /KD (pH7.4, 1.2 mM Ca)** | **KD(pH5.8, 3 μM Ca) /KD(pH7.4, 1.2 mM Ca)** |
| **Clone 278** | pH7.4, 1.2 mM Ca | 1.5E+06 | 3.6E-03 | 2.4E-09 | 842.5 | 1636.5 |
| | pH5.8, 1.2 mM Ca | 1.2E+05 | 2.3E-01 | 2-0E-06 | | |
| | pH5.8, 3 μM Ca | 6.2E+04 | 2.4E-01 | 3.9E-06 | | |
| **Xolair** | pH7.4, 1.2 mM Ca | 2.5E+06 | 1.1E-02 | 4.4E-09 | 2.3 | 2.9 |
| | pH5.8, 1.2 mM Ca | 2.4E+06 | 2.4E-02 | 9.9E-09 | | |
| | pH5.8, 3 μM Ca | 1.4E+06 | 17E-02 | 1-3E-08 | | |

[Example 24] Preparation of an antibody variant that binds to human IgE in a pH-dependent manner

Next, for further accelerating the elimination of antigen (human IgE) from plasma, a DNA sequence encoding 278-F1087 (heavy chain, SEQ ID NO: 154; light chain, SEQ ID NO: 152) with a substitution of Asp for Lys at position 326 (EU numbering) in 278-IgG1 was inserted into an animal expression plasmid by a method known to those skilled in the art, in order to augment the mouse FcγR binding of 278-IgG1 that binds to human IgE in a pH-dependent manner. The antibody variants were expressed by the above-mentioned method using animal cells introduced with the plasmid. The concentrations of the antibody variants were determined after purification.

[Example 25] *In vivo* assessment of 278-IgG1

(25-1) Preparation of human IgE (hIgE (Asp6)) for *in vivo* assessment

hIgE (Asp6) (its variable region is from an anti-human glypican 3 antibody), which is a human IgE for *in vivo* assessment, comprising the heavy chain (SEQ ID NO: 155) and light chain (SEQ ID NO: 150), was prepared by the same method as described in Example (23-1). hIgE (Asp6) is a molecule in which asparagine has been replaced with aspartic acid in the six N-glycosylation sites in human IgE, so that time-dependent changes in the concentration of human IgE as an antigen in the plasma does not affect the heterogeneity of N-linked sugar chains of human IgE.

(25-2) Assessment of the effect of accelerating human IgE elimination from the plasma of normal mice administered with clone 278

As described in Examples 2 to 4, and 22, the antigen concentration was demonstrated to be significantly reduced in the plasma of mice administered with the molecules that bind in a pH-dependent manner to human IL-6 receptor or human IgA as an antigen, and whose binding to mouse FcγR has been augmented. An additional test was carried out using antibodies to human IgE as an antigen to assess whether a similar effect of eliminating soluble antigens from the plasma of a living organism administered with antibodies with augmented mouse FcγR binding that bind in a pH-dependent manner to antigens other than human IL-6 receptor and human IgA, when the binding to mouse FcγR is augmented.

hIgE (Asp6) and anti-human IgE antibodies were assessed for their *in vivo* dynamics after administration of hIgE (Asp6) alone, or hIgE (Asp6) in combination with the anti-hIgE antibodies (278-IgG1 and 278-F1087) to C57BL/6J mice (Charles river Japan). hIgE (Asp6) (20 μg/ml) or a mixture of hIgE (Asp6) and an anti-human IgE antibody was administered once at 10 mL/kg into the caudal vein (as described in Table 31, all antibodies were prepared at the same concentration). In this case, each antibody was present significantly in excess over hIgE (Asp6), and thus almost all of hIgE (Asp6) was thought to bind to the antibody. In the group administered with clone 278 (278-IgG1), from the mice, the blood was collected five minutes, two hours, seven hours, one day, two days, four days, five days, seven days, 14 days, and 21 days after administration. In the group administered with 278-F1087, from the mice, the blood was collected five minutes, 30 minutes, one hour, two hours, one day, three days, seven days, 14 days, and 21 days after administration. The collected blood was immediately centrifuged at 15,000 rpm and 4°C for 5 minutes to isolate the plasma. The isolated plasma was stored in a freezer at -20°C or below until use.

[Table 31]

| Anti-hIgE antibody | hIgE (Asp6) concentration in administered solution (μg/mL) | Anti-hIgE antibody concentration in administered solution (μg/mL) |
|---|---|---|
| 278-IgG1 | 20 | 100 |
| 278-F1087 | 20 | 100 |

(25-3) Determination of the plasma anti-human IgE antibody concentration in normal mice

Anti-hIgE antibody concentrations in mouse plasma were measured by the ELISA method. Standard curve samples were prepared at 0.4, 0.2, 0.1, 0.05, 0.025, 0.0125, and 0.00625 μg/ml for plasma concentrations. To secure the homogeneity of the immune complex between hIgE (Asp6) and anti-hIgE antibody, hIgE (Asp6) was added at 1 μg/ml to the standard curve samples and assay samples of mouse plasma. The samples of the 278-hIgG1 administration group and the corresponding standard curve samples were allowed to stand at room temperature for 30 minutes. Meanwhile, the samples of the 278-F1087 administration group and the corresponding standard curve samples were stirred at 37°C overnight. After incubation or stirring, the standard curve samples and assay samples of mouse plasma were aliquoted to an immunoplate (Nunc-Immuno Plate, MaxiSorp (Nalge nunc International)) immobilized with Anti-Human Kappa Light Chain Antibody (Bethyl Laboratories), and this was allowed to stand/stirred at room temperature for two hours (the samples of the 278-F1087 administration group and the standard curve samples of 278-F1087), or allowed to stand at 4°C overnight (the samples of the 278-hIgG1 administration group and the standard curve samples of 278-hIgG1). Then, Rabbit anti-Human IgG (Fc) Secondary antibody, Biotin conjugate (Pierce Biotechnology) and Streptavidin-Poly HRP80 (Stereospecific Detection Technologies) were each reacted in succession for one hour. Chromogenic reaction was performed using as a substrate TMB One Component HRP Microwell Substrate (BioFX Laboratories). After terminating the reaction with IN sulfuric acid (Showa Chemical), the concentrations in mouse plasma were determined based on the color development by a method for measuring the absorbance at 450 nm with a microplate reader. The concentrations in mouse plasma were determined based on the absorbance of the standard curve using the analysis software SOFTmax PRO (Molecular Devices). A time course of antibody concentrations in plasma after intravenous administration, which were determined by the above method, is shown in Fig. 47. The result demonstrates that, in mice administered with the variants resulting from augmenting the FcyR binding of 278-IgG1 with pH-dependent, strong human IgE-binding activity, the antibody concentration in the plasma of the mice was not significantly reduced as compared to that of 278-IgG1.

(25-4) Determination of the plasma hIgE (Asp6) concentration in normal mice

hIgE (Asp6) concentrations in mouse plasma were measured by the ELISA method. Calibration curve samples were prepared at 192, 96, 48, 24, 12, 6, and 3 ng/ml for plasma concentrations. To secure the homogeneity of the immune complex between hIgE (Asp6) and anti-hIgE antibody, in the group administrated with 278-hIgG1, Xolair (Novartis) was added at 10 μg/ml to the standard curve and assay samples of mouse plasma, and the mixtures were allowed to stand at room temperature for 30 munities. In the group administrated with 278-F1087, 278-F1022 (heavy chain, SEQ ID NO: 156; light chain, SEQ ID NO: 152; prepared in the same manner as Example 24) or 278-F760 (heavy chain, SEQ ID NO: 157; light chain, SEQ ID NO: 152; prepared in the same manner as Example 24) was added at 20 μg/ml, and the mixtures were stirred at 37°C for 60 hours. The assay samples of mouse plasma were aliquoted to an immunoplate (MABTECH) immobilized with anti-human IgE, or an immunoplate (Nunc F96 MicroWell Plate (Nalge nunc International)) immobilized with anti-human IgE (clone 107, MABTECH), and this was allowed to stand or stirred at room temperature for two hours, or allowed to stand at 4°C overnight. Then, the human GPC3 core protein (SEQ ID NO: 158), anti-GPC3 antibody (in-house preparation) biotinylated with NHS-PEG4-Biotin (Thermo Fisher Scientific), and Sterptavidin-PolyHRP80 (Stereospecific Detection Technologies) were each reacted in succession for one hour. Chromogenic reaction was performed using as a substrate TMB One Component HRP Microwell Substrate (BioFX Laboratories). After terminating the reaction with IN sulfuric acid (Showa Chemical), the concentrations in mouse plasma were determined based on the color development by a method for measuring the absorbance at 450 nm with a microplate reader. Alternatively, chromogenic reaction was performed using as a substrate SuperSignal(r) ELISA Pico Chemiluminescent Substrate (Thermo Fisher Scientific), and the concentrations in mouse plasma were determined by a method for measuring the luminescence intensity with a microplate reader. The concentrations in mouse plasma were determined based on the absorbance or luminescence intensity of the standard curve using the analysis software SOFTmax PRO (Molecular Devices). A time course of hIgE (Asp6) concentrations in plasma after intravenous administration, which were determined by the above method, is shown in Fig. 48.

Regarding the elimination of human IgE alone, the result demonstrates that, in mice administered with human IgE in combination with 278-IgG1 having the pH-dependent, strong binding activity, the elimination of human IgE was accelerated as compared to the administration of human IgE alone. Furthermore, in mice administered with human IgE in combination with 278-F1087 resulting from augmenting FcγR binding of 278-IgG1, the elimination of human IgE was demonstrated to be significantly accelerated as compared to the mice administered with human IgE alone, or human IgE in combination with 278-IgG1. That is, it was shown that the antigen elimination was accelerated not only in mice administered with the above-mentioned anti-IL6R antibody and anti-IgA antibody with augmented FcγR binding, but also in mice administered with the anti-IgE antibody with augmented FcγR binding.

[Reference Example 1] Construction of expression vectors of amino acid-substituted IgG antibodies

Mutants were prepared using the QuikChange Site-Directed Mutagenesis Kit (Stratagene) by the method described in the appended instruction manual. Plasmid fragments containing the mutants were inserted into animal cell expression vectors to construct desired H-chain and L-chain expression vectors. The nucleotide sequences of the resulting expression vectors were determined by the methods known to those skilled in the art.

[Reference Example 2] Expression and purification of IgG antibodies

Antibodies were expressed using the following method. Human embryonic kidney cancer-derived HEK293H cell line (Invitrogen) was suspended in DMEM (Invitrogen) supplemented with 10% Fetal Bovine Serum (Invitrogen). The cells were plated at 10 ml per dish in dishes for adherent cells (10 cm in diameter; CORNING) at a cell density of 5 to 6 x $10^5$ cells/ml and cultured in a $CO_2$ incubator (37°C, 5% $CO_2$) for one whole day and night. Then, the medium was removed by aspiration, and 6.9 ml of CHO-S-SFM-II medium (Invitrogen) was added. The prepared plasmid was introduced into the cells by the lipofection method. The resulting culture supernatants were collected, centrifuged (approximately 2,000 x g, 5 min, room temperature) to remove cells, and sterilized by filtering through 0.22-μm filter MILLEX (registered trademark)-GV (Millipore) to obtain the supernatants. Antibodies were purified from the obtained culture supernatants by a method known to those skilled in the art using rProtein A Sepharose™ Fast Flow (Amersham Biosciences). To determine the concentration of the purified antibody, absorbance was measured at 280 nm using a spectrophotometer. Antibody concentrations were calculated from the determined values using an absorbance coefficient calculated by the method described in Protein Science (1995) 4: 2411-2423.

[Reference Example 3] Preparation of soluble human IL-6 receptor (hsIL-6R)

Recombinant human IL-6 receptor of human IL-6 receptor which is an antigen was prepared in the manner described below. A CHO line that constantly expresses soluble human IL-6 receptor composed of an amino acid sequence consisting of the 1st to 357th amino acid from the N terminus as reported in J. Immunol. (1994) 152, 4958-4968 (hereinafter, hsIL-6R) was constructed using a method known among persons with ordinary skill in the art. hsIL-6R was expressed by culturing this CHO line. hsIL-6R was purified from culture supernatant of the resulting CHO line by the two steps of Blue Sepharose 6 FF column chromatography and gel filtration column chromatography. The fraction that eluted as the main peak in the final step was used as the final purified product.

[Reference Example 4] Preparation of antibodies with a low fucose content

Antibodies with a low fucose content were expressed by the following method. Cells of a fucose transporter-deficient CHO line (Patent Document WO2006/067913) suspended in α-MEM medium (Invitrogen) supplemented with 10 % Fetal Bovine Serum (CCB) were plated at a concentration of 2E+6 cells/10 ml in an adherent cell dish (diameter: 10 cm; CORNING). After culture in a $CO_2$ incubator (37°C, 5% $CO_2$) for one day and night, the medium was removed by aspiration, and then 7 ml of CHO-S-SFM-II (Invitrogen) medium was added thereto. 7 ml of CHO-S-SFM-II (Invitrogen) medium was added to cells separately prepared and introduced with plasmids by a lipofection method. After 72 hours, the culture supernatants were collected by centrifugation (about 2000 g, 5 minutes, room temperature) and sterilized by filtration through 0.22-μm filter MILLEX(R)-GV (Millipore). From the resulting culture supernatants, antibodies were purified by a method known to those skilled in the art using rProtein A SepharoseTM Fast Flow (Amersham Biosciences). The concentrations of purified antibodies were calculated using the extinction coefficient calculated by the method described in Protein Science (1995) 4, 2411-2423, based on the absorbance at 280 nm measured by a spectrophotometer.

[Reference Example 5] Acquisition of antibodies that bind to IL-6 receptor in Ca-dependent manner from a human antibody library using phage display technology

<u>(5-1) Preparation of a phage display library for naive human antibodies</u>

A phage display library for human antibodies, consisting of multiple phages presenting the Fab domains of mutually different human antibody sequences, was constructed according to a method known to those skilled in the art using a poly A RNA prepared from human PBMC, and commercial human poly A RNA as a template.

<u>(5-2) Acquisition of antibody fragments that bind to antigen in Ca-dependent manner from the library by bead panning</u>

The constructed phage display library for naive human antibodies was subjected to initial selection through concentration of only antibody fragments having an antigen (IL-6 receptor)-binding ability or concentration of antibody fragments using a Ca concentration-dependent antigen (IL-6 receptor)-binding ability as an indicator. Concentration of antibody fragments using a Ca concentration-dependent antigen (IL-6 receptor)-binding ability as an indicator were conducted through elution of the phage library phages bound to IL-6 receptor in the presence of Ca ions with EDTA that chelates the Ca ions Biotinylated IL-6 receptor was used as an antigen.

Phages were produced from *Escherichia coli* carrying the constructed phage display phagemid. A phage library solution was obtained by diluting with TBS a phage population precipitated by adding 2.5 M NaCl/10% PEG to the E. coli culture solution in which the phages were produced. Subsequently, BSA and $CaCl_2$ were added to the phage library solution at a final concentration of 4% BSA and 1.2 mM of calcium ion concentration. A common panning method using an antigen immobilized on magnetic beads was referred to as a panning method (J. Immunol. Methods. (2008) 332 (1-2), 2-9; J. Immunol. Methods. (2001) 247 (1-2), 191-203; Biotechnol. Prog. (2002) 18(2) 212-20; Mol. Cell Proteomics (2003) 2 (2), 61-9). NeutrAvidin coated beads (Sera-Mag SpeedBeads NeutrAvidin-coated) or Streptavidin coated beads (Dynabeads M-280 Streptavidin) were used as magnetic beads.

Specifically, 250 pmol of the biotin-labeled antigen was added to the prepared phage library solution to allow the contact of said phage library solution with the antigen at room temperature for 60 minutes. Magnetic beads, blocked with BSA, were added to be bound to antigen-phage complexes at room temperature for 15 minutes. The beads were washed once with 1 mL of 1.2 mM $CaCl_2$/TBS (TBS containing 1.2 mM $CaCl_2$). Subsequently, a phage solution was recovered by a general elution method to concentrate an antibody fragment having an IL-6 receptor-binding ability, or by elution from beads suspended in 2 mM EDTA/TBS (TBS containing 2 mM EDTA) to concentrate an antibody fragment using an IL-6 receptor-binding ability in a Ca concentration-dependent manner as an indicator. The recovered phage solution was added to 10 mL of the *E. coli* strain TG1 in a logarithmic growth phase (OD600 of 0.4-0.7). The *E. coli* was cultured with gentle stirring at 37°C for 1 hour to allow the phages to infect the *E. coli*. The infected *E. coli* was inoculated into a 225 mm × 225 mm plate. Subsequently, the phages were recovered from the culture medium of the *E. coli* after inoculation to prepare a phage library solution.

In the second and subsequent panning, the phages were enriched using the Ca-dependent binding ability as an indicator. Specifically, 40 pmol of the biotin-labeled antigen was added to the prepared phage library solution to allow the contact of the phage library with the antigen at room temperature for 60 minutes. Magnetic beads, blocked with BSA, were added to be bound to antigen-phage complexes at room temperature for 15 minutes. The beads were washed with 1 mL of 1.2 mM $CaCl_2$/TBST and 1.2 mM $CaCl_2$/TBS. Subsequently, the beads, to which 0.1 mL of 2 mM EDTA/TBS was added, were suspended at room temperature. Immediately after that, the beads were separated using a magnetic stand to collect a phage solution. The recovered phage solution was added to 10 mL of the *E. coli* strain TG1 in a logarithmic growth phase (OD600 of 0.4-0.7). The *E. coli* was cultured with gentle stirring at 37°C for 1 hour to allow the phages to infect the *E. coli*. The infected *E. coli* was inoculated into a 225 mm x 225 mm plate. Subsequently, the phages were recovered from the culture medium of the *E. coli* after inoculation to collect a phage library solution. The panning using the Ca-dependent binding ability as an indicator was repeated several times.

(5-3) Examination by phage ELISA

A phage-containing culture supernatant was collected according to a routine method (Methods Mol. Biol. (2002) 178, 133-145) from a single colony of *E. coli,* obtained as described above.

A culture supernatant containing phages, to which BSA and $CaCl_2$ were added at a final concentration of 4% BSA and 1.2 mM of calcium ion concentration was subjected to ELISA as described below. A StreptaWell 96 microtiter plate (Roche) was coated overnight with 100 μL of PBS containing the biotin-labeled antigen. Each well of said plate was washed with PBST to remove the antigen, and then the wells were blocked with 250 μL of 4% BSA-TBS for 1 hour or longer. Said plate with the prepared culture supernatant added to each well, from which the 4% BSA-TBS was removed, was allowed to stand undisturbed at 37°C for 1 hour, allowing the binding of phage-presenting antibody to the antigen present in each well. To each well washed with 1.2 mM $CaCl_2$/TBST, 1.2 mM $CaCl_2$/TBS or 1 mM EDTA/TBS was added. The plate was allowed to stand undisturbed for 30 minutes at 37°C for incubation. After washing with 1.2 mM $CaCl_2$/TBST, an HRP-conjugated anti-M13 antibody (Amersham Pharmacia Biotech) diluted with TBS at a final concentration of 4% BSA and 1.2 mM of ionized calcium concentration was added to each well, and the plate was incubated for 1 hour. After washing with 1.2 mM $CaCl_2$/TBST, the chromogenic reaction of the solution in each well with a TMB single solution (ZYMED) added was stopped by adding sulfuric acid. Subsequently, said color was measured by measuring absorbance at 450 nm.

As a result of the above phage ELISA, the base sequence of a gene amplified with specific primers and an antibody fragment identified as having a Ca-dependent antigen-binding ability as a template was analyzed.

(5-4) Antibody expression and purification

As a result of the above phage ELISA, a clone identified as having a Ca-dependent antigen-binding ability was introduced into an expression plasmid for animal cells. Antibodies were expressed as described below. FreeStyle 293-F strain (Invitrogen) derived from human fetal kidney cells was suspended in FreeStyle 293 Expression Medium (Invitrogen), followed by inoculation of 3 mL into each well of a 6-well plate at a cell density of 1.33 x $10^6$ cell /mL. The prepared plasmid was introduced into the cells by lipofection. The cells were cultured for 4 days in a $CO_2$ incubator (37°C, 8% $CO_2$, 90 rpm). Antibodies were purified from the culture supernatant obtained above by a method known in the art using rProtein A Sepharose™ Fast Flow (Amersham Biosciences). Absorbance of the purified antibody solution was measured at 280 nm using a spectrophotometer. Antibody concentration was calculated from the measurements obtained using an extinction coefficient calculated by the PACE method (Protein Science (1995) 4, 2411-2423).

[Reference Example 6] Examination of Ca-dependent binding ability of the obtained antibodies to human IL-6 receptor

To examine whether or not the binding activities of antibodies 6RL#9-IgG1 [heavy chain (SEQ ID NO: 44; a sequence in which a IgG1 -derived constant region has been linked to SEQ ID NO: 9); light chain SEQ ID NO: 45] and FH4-IgG1 [heavy chain, SEQ ID NO: 46; light chain SEQ ID NO: 47], obtained in Reference Example 5, to human IL-6 receptor are Ca-dependent, the kinetic analysis of the antigen-antibody reactions of these antibodies with human IL-6 receptor was conducted using Biacore T100 (GE Healthcare). H54/L28-IgG1 [heavy chain SEQ ID NO: 36; light chain SEQ ID NO: 37], described in WO 2009125825, was used as a control antibody that has no Ca-dependent binding activity to human IL-6 receptor. The kinetic analysis of the antigen-antibody reactions was conducted in solutions with 2 mM and 3 μM calcium ion concentrations, set as high and low calcium ion concentration conditions, respectively. The antibody of interest was captured on Sensor chip CM4 (GE Healthcare) on which an appropriate amount of Protein A (Invitrogen) was immobilized by an amine coupling method. Two buffers [10 mM ACES, 150 mM NaCl, 0.05% (w/v) Tween 20, and 2 mM $CaCl_2$ (pH 7.4) or 10 mM ACES, 150 mM NaCl, 0.05% (w/v) Tween 20, and 3 μmol/L $CaCl_2$ (pH 7.4)] were used as running buffers. These buffers were used for diluting human IL-6 receptor. All the measurements were conducted at 37°C.

In the kinetic analysis of antigen-antibody reaction using H54L28-IgG1 antibody, the H54L28-IgG1 antibody captured on the sensor chip was allowed to interact with IL-6 receptor by injecting a diluent of IL-6 receptor and running buffer (blank) at a flow rate of 20 μL/min for 3 minutes. Subsequently, after the dissociation of IL-6 receptor was observed using running buffer at a flow rate of 20 μL/min for 10 minutes, the sensor chip was regenerated by injecting 10 mM glycine-HCl (pH 1.5) at a flow rate 30 μL/min for 30 seconds. Kinetics parameters, binding constant (ka) (1/Ms) and dissociation rate constant (kd) (1/s), were calculated from the sensorgrams obtained in the measurement. These values were used to calculate the dissociation constant (KD) (M) of the H54L28-IgG1 antibody for human IL-6 receptor. Each parameter was calculated using the Biacore T100 Evaluation Software (GE Healthcare).

In the kinetic analysis of antigen-antibody reaction using FH4-IgG1 and 6RL#9-IgG1 antibodies, the FH4-IgG1 or 6RL#9-IgG1 antibody captured on the sensor chip was allowed to interact with IL-6 receptor by injecting a diluent of IL-6 receptor and running buffer (blank) at a flow rate of 5 μL/min for 15 minutes. Subsequently, the sensor chip was regenerated by injecting 10 mM glycine-HCl (pH 1.5) at a flow rate 30 μL/min for 30 seconds. Dissociation constants (KD) (M) were calculated from the sensorgrams obtained in the measurement, using a steady-state affinity model. Each parameter was calculated using the Biacore T100 Evaluation Software (GE Healthcare).

The dissociation constants (KD) between each antibody and IL-6 receptor in the presence of 2 mM $CaCl_2$, determined by the above method, are shown in Table 32.

[Table 32]

| ANTIBODY | H54/L28-IgG1 | FH4-IgG1 | 6RL#9-IgG1 |
|---|---|---|---|
| kD(M) | 1.9E-9 | 5.9E-7 | 2.6E-7 |

The KD value of the H54/L28-IgG1 antibody under the condition of 3 μM Ca concentration can be calculated in the same manner as in the presence of 2 mM Ca concentration. Under the condition of 3 μM Ca concentration, FH4-IgG1 and 6RL#9-IgG1 antibodies were barely observed to be bound to IL-6 receptor, thus the calculation of KD values by the method described above is difficult. However, the KD values of these antibodies under the condition of 3 μM Ca concentration can be estimated using Equation 3 (Biacore T100 Software Handbook, BR-1006-48, AE 01/2007) described below.

[Equation 3]

$$Req = C \times Rmax / (KD+C) + RI$$

The meaning of each parameter in the aforementioned [Equation 3] is as follows:

Req (RU): Steady state binding levels
Rmax (RU): Analyte binding capacity of the surface
RI (RU): Bulk refractive index contribution in the sample
C (M): Analyte concentration
KD (M): Equilibrium dissociation constant

The approximate results of dissociation constant KD values for the antibodies and IL-6 receptor at a Ca concentration of 3 μmol/L, estimated using the above-described [Equation 3], are shown in Table 33. In Table 33, the Req, Rmax, RI, and C values are estimated based on the assay result.

[Table 33]

| ANTIBODY | H54/L28-IgG1 | FH4-IgG1 | 6RL#9-IgG1 |
|---|---|---|---|
| Req(RU) | | 5 | 10 |
| Rmax(RU) | | 39 | 72 |
| RI(RU) | | 0 | 0 |
| C(M) | | 5E-06 | 5E-06 |
| KD(M) | 2.2E-9 | 3.4E-05 | 3.1E-05 |

Based on the findings described above, it was predicted that the KD between IL-6 receptor and FH4-IgG1 antibody or 6RL#9-IgG1 antibody was increased by about 60 or 120 times (the affinity was reduced by 60 or 120 times or more) when the concentration of $CaCl_2$ in the buffer was decreased from 2 mM to 3μM.

Table 34 summarizes the KD values at $CaCl_2$ concentrations of 2 mM and 3 μM, and the Ca dependency of the the KD values for the three types of antibodies H54/L28-IgG1, FH4-IgG1, and 6RL#9-IgG1.

[Table 34]

| ANTIBODY | H54/L28-IgG1 | FH4-IgG1 | 6RL#9-IgG1 |
|---|---|---|---|
| KD (M) (2 mM $CaCl_2$) | 1.9E-9 | 5.9E-7 | 2.6E-7 |
| KD (M) (3 μM $CaCl_2$) | 2.2E-9 | 3.4E-5 OR MORE | 3.1E-5 OR MORE |
| Ca DEPENDENCY | ABOUT THE SAME | ABOUT 60 TIMES OR MORE | ABOUT 120 TIMES OR MORE |

No difference in the binding of the H54/L28-IgG1 antibody to IL-6 receptor due to the difference in Ca concentration was observed. On the other hand, the binding of FH4-IgG1 and 6RL#9-IgG1 antibodies to IL-6 receptor was observed to be significantly attenuated under the condition of the low Ca concentration (Table 34).

[Reference Example 7] Examination of calcium ion binding to the antibody obtained

Subsequently, the intermediate temperature of thermal denaturation (Tm value) was measured by differential scanning calorimetry (DSC) as an indicator for examining calcium ion binding to the antibody (MicroCal VP-Capillary DSC, MicroCal). The intermediate temperature of thermal denaturation (Tm value) is an indicator of stability. The intermediate temperature of thermal denaturation (Tm value) becomes higher when a protein is stabilized through calcium ion binding, as compared with no calcium ion binding (J. Biol. Chem. (2008) 283, 37, 25140-25149). The binding activity of calcium ion to antibody was examined by examining changes in the Tm value of the antibody depending on the changes in the calcium ion concentration of the antibody solution. The purified antibody was subjected to dialysis (EasySEP, TOMY) using an external solution of 20 mM Tris-HCl, 150 mM NaCl, and 2 mM $CaCl_2$ (pH 7.4), or 20 mM Tris-HCl, 150 mM NaCl, and 3 μM $CaCl_2$ (pH 7.4). DSC measurement was conducted at a heating rate of 240°C/hr from 20 to 115°C using an antibody solution prepared at about 0.1 mg/mL with the dialysate as a test substance. The intermediate temperatures of thermal denaturation (Tm values) of the Fab domains of each antibody, calculated based on the denaturation curve obtained by DSC, are shown in Table 35.

[Table 35]

| ANTIBODY | CALCIUM ION CONCENTRATION | | ΔTm(°C) |
|---|---|---|---|
| | 3 μM | 2 mM | 2 mM-3 μM |
| H54/L28-IgG1 | 92.87 | 92.87 | 0.00 |
| FH4-IgG1 | 74.71 | 78.97 | 4.26 |
| 6RL#9-IgG1 | 77.77 | 78.98 | 1.21 |

From the results shown in Table 35, it is indicated that the Tm values of the Fab of the FH4-IgG1 and 6RL#9-IgG1 antibodies, which show a calcium-dependent binding ability, varied with changes in the calcium ion concentration, while the Tm values of the Fab of the H54/L28-IgG1 antibody which shows no calcium-dependent binding ability do not vary with changes in the calcium ion concentration. The variation in the Tm values of the Fab of the FH4-IgG1 and 6RL#9-IgG1 antibodies demonstrates that calcium ions bound to these antibodies to stabilize the Fab portions. The above results show that calcium ions bound to the FH4-IgG1 and 6RL#9-IgG1 antibodies, while no calcium ion bound to the H54/L28-IgG1 antibody.

[Reference Example 8] Identification of calcium ion-binding site in antibody 6RL#9 by X-ray crystal structure analysis

(8-1) X-ray crystal structure analysis

As described in Reference Example 7, the measurements of thermal denaturation temperature Tm suggested that antibody 6RL#9 binds to calcium ion. However, it was unpredictable which portion of antibody 6RL#9 binds to calcium ion. Then, by using the technique of X-ray crystal structure, residues of antibody 6RL#9 that interact with calcium ion were identified.

(8-2) Expression and purification of antibody 6RL#9

Antibody 6RL#9 was expressed and purified for X-ray crystal structure analysis. Specifically, animal expression plasmids constructed to be capable of expressing the heavy chain (SEQ ID NO: 44) and light chain (SEQ ID NO: 45) of antibody 6RL#9 were introduced transiently into animal cells. The constructed plasmids were introduced by the lipofection method into cells of human fetal kidney cell-derived FreeStyle 293-F (Invitrogen) suspended in 800 ml of the FreeStyle 293 Expression Medium (Invitrogen) (final cell density: 1 x $10^6$ cells/mL). The plasmid-introduced cells were cultured in a $CO_2$ incubator (37°C, 8% $CO_2$, 90 rpm) for five days. From the culture supernatant obtained as described above, antibodies were purified by a method known to those skilled in the art using the rProtein A Sepharose™ Fast Flow (Amersham Biosciences). Absorbance at 280 nm of purified antibody solutions was measured using a spectrophotometer. Antibody concentrations were calculated from the measured values using an extinction coefficient calculated by the PACE method (Protein Science (1995) 4, 2411-2423).

(8-3) Purification of antibody 6RL#9 Fab fragment

Antibody 6RL#9 was concentrated to 21 mg/ml using an ultrafilter with a molecular weight cutoff of 10,000 MWCO. A 5 mg/mL antibody sample (2.5 mL) was prepared by diluting the antibody solution using 4 mM L-cysteine/5 mM EDTA/20 mM sodium phosphate buffer (pH 6.5). 0.125 mg of papain (Roche Applied Science) was added to the sample. After stirring, the sample was incubated at 35°C for two hours. After incubation, a tablet of Protease Inhibitor Cocktail Mini, EDTA-free (Roche Applied Science) was dissolved in 10 ml of 25 mM MES buffer, pH 6, and added to the sample. The sample was incubated on ice to stop the papain proteolytic reaction. Then, the sample was loaded onto a 1-ml cation-exchange column HiTrap SP HP (GE Healthcare) equilibrated with 25 mM MES buffer (pH 6), downstream of which a 1-ml HiTrap MabSelect Sure Protein A column (GE Healthcare) was connected in tandem. A purified fraction of the Fab fragment of antibody 6RL#9 was obtained by performing elution with a linear NaCl concentration gradient up to 300 mM in the above-described buffer. Then, the resulting purified fraction was concentrated to about 0.8 ml using a 5000 MWCO ultrafilter. The concentrate was loaded onto a gel filtration column Superdex 200 10/300 GL (GE Healthcare) equilibrated with 100 mM HEPES buffer (pH 8) containing 50 mM NaCl. The purified Fab fragment of antibody 6RL#9 for crystallization was eluted from the column using the same buffer. All the column treatments described above were carried out at a low temperature of 6 to 7.5°C.

(8-4) Crystallization of the antibody 6RL#9 Fab fragment in the presence of Ca

Seed crystals of the 6RL#9 Fab fragment were prepared in advance under general conditions. Then, the purified Fab fragment of antibody 6RL#9 in 5 mM $CaCl_2$ was concentrated to 12 mg/ml with a 5000 MWCO ultrafilter. Next, the sample concentrated as described above was crystallized by the hanging drop vapor diffusion method using 100 mM HEPES buffer (pH 7.5) containing 20% to 29% PEG4000 as a reservoir solution. The above-described seed crystals were crushed in 100 mM HEPES buffer (pH 7.5) containing 29% PEG4000 and 5 mM $CaCl_2$, and serially diluted to 100 to 10,000 folds. Then, 0.2 μl of diluted solutions were combined with a mixture of 0.8 μl of the reservoir solution and 0.8 μl of the concentrated sample to prepare crystallization drops on a glass cover slide. The crystal drops were allowed to stand at 20°C for two to three days to prepare thin plate-like crystals. X-ray diffraction data were collected using the crystals.

(8-5) Crystallization of the antibody 6RL#9 Fab fragment in the absence of Ca

The purified Fab fragment of antibody 6RL#9 was concentrated to 15 mg/ml using a 5000 MWCO ultrafilter.

Then, the sample concentrated as described above was crystallized by the hanging drop vapor diffusion method using 100 mM HEPES buffer (pH 7.5) containing 18% to 25% PEG4000 as a reservoir solution. Crystals of the antibody 6RL#9 Fab fragment obtained in the presence of Ca were crushed in 100 mM HEPES buffer (pH 7.5) containing 25% PEG4000, and serially diluted to 100 to 10,000 folds. Then, 0.2 μl of diluted solutions were combined with a mixture of 0.8 μl of the reservoir solution and 0.8 μl of the concentrated sample to prepare crystallization drops on a glass cover slide. The crystal drops were allowed to stand at 20°C for two to three days to prepare thin plate-like crystals. X-ray diffraction data were collected using the crystals.

(8-6) X-ray diffraction data measurement of Fab fragment crystal from antibody 6RL#9 in the presence of Ca

A single crystal of the Fab fragment of antibody 6RL#9 prepared in the presence of Ca was soaked into 100 mM HEPES buffer (pH 7.5) solution containing 35% PEG4000 and 5 mM $CaCl_2$. The single crystal was fished out of the exterior solution using a pin with attached tiny nylon loop, and frozen in liquid nitrogen. X-ray diffraction data of the frozen crystal was collected from beam line BL-17A of the Photon Factory in the High Energy Accelerator Research Organization. The frozen crystal was constantly placed in a nitrogen stream at -178°C to maintain in a frozen state during the measurement. A total of 180 diffraction images were collected using the CCD detector Quantum315r (ADSC) attached to the beam line with rotating the crystal 1° at a time. Lattice constant determination, diffraction spot indexing, and diffraction data analysis were performed using programs Xia2 (CCP4 Software Suite), XDS Package (Walfgang Kabsch), and Scala (CCP4 Software Suite). Finally, diffraction intensity data up to 2.2 Å resolution was obtained. The crystal belongs to the space group $P2_12_12_1$ with lattice constant a = 45.47 Å, b = 79.86 Å, c = 116.25 Å, α = 90°, β = 90°, and γ = 90°.

(8-7) X-ray diffraction data measurement of the Fab fragment crystal from antibody 6RL#9 in the absence of Ca

Crystals of the Fab fragment of antibody 6RL#9 prepared in the absence of Ca were soaked in 100 mM HEPES buffer (pH 7.5) solution containing 35% PEG4000. By removing the exterior solution from the surface of a single crystal using a pin with attached tiny nylon loop, the single crystal was frozen in liquid nitrogen. X-ray diffraction data of the frozen crystal was collected from beam line BL-5A of the Photon Factory in the High Energy Accelerator Research Organization. The frozen crystal was constantly placed in a nitrogen stream at -178°C to maintain in a frozen state during the measurement. A total of 180 diffraction images were collected using the CCD detector Quantum210r (ADSC) attached to the beam line with rotating the crystal 1° at a time. Lattice constant determination, diffraction spot indexing, and diffraction data analysis were performed using programs Xia2 (CCP4 Software Suite), XDS Package (Walfgang Kabsch), and Scala (CCP4 Software Suite). Finally, diffraction intensity data up to 2.3 Å resolution was obtained. The crystal belongs to the space group $P2_12_12_1$ with lattice constant a = 45.40 Å, b = 79.63 Å, c = 116.07 Å, α = 90°, β = 90°, γ = 90°, and thus is structurally identical to the crystal prepared in the presence of Ca.

(8-8) Structure analysis of the Fab fragment crystal from antibody 6RL#9 in the presence of Ca

The crystal structure of the antibody 6RL#9 Fab fragment in the presence of Ca was determined by a molecular replacement method using the Phaser program (CCP4 Software Suite). The number of molecules in an asymmetrical unit was estimated to be one from the size of crystal lattice and molecular weight of the antibody 6RL#9 Fab fragment. Based on the primary sequence homology, a portion of amino acid positions 112 to 220 from A chain and a portion of amino acid positions 116 to 218 from B chain in the conformational coordinate of PDB code 1ZA6 were used as model molecules for analyzing the CL and CH1 regions. Then, a portion of amino acid positions 1 to 115 from B chain in the conformational coordinate of PDB code 1ZA6 was used as a model molecule for analyzing the VH region. Finally, a portion of amino acid positions 3 to 147 of the light chain in the conformational coordinate of PDB code 2A9M was used as a model molecule for analyzing the VL region. Based on this order, an initial structure model for the antibody 6RL#9 Fab fragment was obtained by determining from translation and rotation functions the positions and orientations of the model molecules for analysis in the crystal lattice. The crystallographic reliability factor R for the reflection data at 25 to 3.0 Å resolution was 46.9% and Free R was 48.6% after rigid body refinement where the VH, VL, CH1, and CL domains were each allowed to deviate from the initial structure model. Then, model refinement was achieved by repeating structural refinement using program Refmac5 (CCP4 Software Suite) followed by model revision performed using program Coot (Paul Emsley) with reference to the Fo-Fc and 2Fo-Fc electron density maps where the coefficients Fo-Fc and 2Fo-Fc were calculated using experimentally determined structural factor Fo, structural factor Fc calculated based on the model, and the phases. The final refinement was carried out using program Refmac5 (CCP4 Software Suite) based on the Fo-Fc and 2Fo-Fc electron density maps by adding water molecule and Ca ion into the model. With 21,020 reflection data at 25 to 2.2 Å resolution, eventually the crystallographic reliability factor R became 20.0% and free R became 27.9% for the model consisting of 3440 atoms.

(8-9) Measurement of X-ray diffraction data of the Fab fragment crystal from antibody 6RL#9 in the absence of Ca

The crystal structure of the antibody 6RL#9 Fab fragment in the absence of Ca was determined based on the structure of the crystal prepared in the presence of Ca. Water and Ca ion molecules were omitted from the conformational coordinate of the crystal of the antibody 6RL#9 Fab fragment prepared in the presence of Ca. The crystallographic reliability factor R for the data of reflection at 25 to 3.0 Å resolution was 30.3% and Free R was 31.7% after the rigid body refinement where the VH, VL, CHI, and CL domains were each allowed to deviate. Then, model refinement was achieved by repeating structural refinement using program Refmac5 (CCP4 Software Suite) followed by model revision performed using program Coot (Paul Emsley) with reference to the Fo-Fc and 2Fo-Fc electron density maps where the coefficients Fo-Fc and 2Fo-Fc were calculated using experimentally determined structural factor Fo, structural factor Fc calculated based on the model, and the phases. The final refinement was carried out using program Refmac5 (CCP4 Software Suite) based on the Fo-Fc and 2Fo-Fc electron density maps by adding water molecule to the model. With 18,357 reflection data at 25 to 2.3 Å resolution, eventually the crystallographic reliability factor R became 20.9% and free R became 27.7% for the model consisting of 3351 atoms.

(8-10) Comparison of X-ray crystallographic diffraction data of the Fab fragments of antibody 6RL#9 between in the presence and absence of Ca

When the crystal structures of the Fab fragments of antibody 6RL#9 are compared between in the presence and absence of Ca, significant changes are seen in the heavy chain CDR3. The structure of the heavy chain CDR3 of the antibody 6RL#9 Fab fragment determined by X-ray crystal structure analysis is shown in Fig. 49. Specifically, a calcium ion resided at the center of the heavy chain CDR3 loop region of the antibody 6RL#9 Fab fragment prepared in the presence of Ca. The calcium ion was assumed to interact with positions 95, 96, and 100a (Kabat numbering) of the heavy chain CDR3. It was believed that the heavy chain CDR3 loop which is important for the antigen binding was stabilized by calcium binding in the presence of Ca, and became an optimum structure for antigen binding. There is no report demonstrating that calcium binds to the antibody heavy chain CDR3. Thus, the calcium-bound structure of the antibody heavy chain CDR3 is a novel structure.

The calcium-binding motif present in the heavy chain CDR3, revealed in the structure of the Fab fragment of the 6RL#9 antibody may also become a new design element for the Ca library for obtaining an antigen-binding domain included in the antigen-binding molecule of the present invention that is against an antigen depending on the ion concentration. The calcium-binding motif was introduced into a light chain variable region in later-described Reference Examples 18 and 19, and for example, a library containing the heavy chain CDR3 of the 6RL#9 antibody and flexible residues in other CDRs including the light chain is thought to be possible.

[Reference Example 9] Preparation of antibodies that bind to IL-6 in a Ca-dependent manner from a human antibody library using phage display techniques

(9-1) Construction of a phage display library of naive human antibodies

A human antibody phage display library containing multiple phages that display various human antibody Fab domain sequences was constructed by a method known to those skilled in the art using, as a template, polyA RNA prepared from human PBMC, commercially available human polyA RNA, and such.

(9-2) Preparation of antibody fragments that bind to the antigen in a Ca-dependent manner from library by bead panning

Primary selection from the constructed phage display library of naive human antibodies was carried out by enriching antibody fragments that have antigen (IL-6)-binding activity. The antigen used was biotin-labeled IL-6.

Phages were produced from *E. coli* carrying the constructed phagemid for phage display. To precipitate the phages produced by *E. coli,* 2.5 M NaCl/10% PEG was added to the *E. coli* culture medium. The phage fraction was diluted with TBS to prepare a phage library solution. Then, BSA and $CaCl_2$ were added the phage library solution at final concentrations of 4% BSA and 1.2 mM calcium ion concentration, respectively. The panning method used was a conventional panning method using antigen-immobilized magnetic beads (J. Immunol. Methods. (2008) 332(1-2): 2-9; J. Immunol. Methods. (2001) 247(1-2): 191-203; Biotechnol. Prog. (2002) 18(2): 212-20; Mol. Cell Proteomics (2003) 2(2): 61-9). The magnetic beads used were NeutrAvidin-coated beads (Sera-Mag SpeedBeads NeutrAvidin-coated) and Streptavidin-coated beads (Dynabeads M-280 Streptavidin).

Specifically, 250 pmol of the biotin-labeled antigen was added to the prepared phage library solution. Thus, the solution was contacted with the antigen at room temperature for 60 minutes. Magnetic beads blocked with BSA were added, and the antigen-phage complex was allowed to bind to the magnetic beads at room temperature for 15 minutes.

The beads were washed three times with 1.2 mM $CaCl_2$/TBST (TBST containing 1.2 mM $CaCl_2$), and then twice with 1 ml of 1.2 mM $CaCl_2$/TBS (TBS containing 1.2 mM $CaCl_2$). Thereafter, 0.5 ml of 1 mg/ml trypsin was added to the beads. After 15 minutes of dispersion at room temperature, the beads were immediately separated using a magnetic stand to collect a phage suspension. The prepared phage suspension was added to 10 ml of *E. coli* of stain TG1 at the logarithmic growth phase (OD600 = 0.4 to 0.7). The *E. coli* was incubated with gentle stirring at 37°C for one hour to infect the phages. The infected *E. coli* was seeded in a plate (225 mm x 225 mm). Then, phages were collected from the culture medium of the seeded *E. coli* to prepare a phage library solution.

In the second round and subsequent panning, phages were enriched using the Ca-dependent binding activity as an indicator. Specifically, 40 pmol of the biotin-labeled antigen was added to the prepared phage library solution. Thus, the phage library was contacted with the antigen at room temperature for 60 minutes. Magnetic beads blocked with BSA were added, and the antigen-phage complex was allowed to bind to the magnetic beads at room temperature for 15 minutes. The beads were washed with 1 ml of 1.2 mM $CaCl_2$/TBST and 1.2 mM $CaCl_2$/TBS. Next, 0.1 ml of 2 mM EDTA/TBS was added to the beads. After dispersion at room temperature, the beads were immediately separated using a magnetic stand to collect a phage suspension. The pIII protein (helper phage-derived protein pIII) was cleaved from phages that did not display Fab by adding 5 $\mu$l of 100 mg/ml trypsin to the collected phage suspension to eliminate the ability of phages displaying no Fab to infect *E. coli*. Phages collected from the trypsinized liquid phage stock was added to 10 ml of *E. coli* cells of the TG1 strain at the logarithmic growth phase (OD600 = 0.4 to 0.7). The *E. coli* was incubated while gently stirring at 37°C for one hour to infect phage. The infected *E. coli* was seeded in a plate (225 mm x 225 mm). Then, phages were collected from the culture medium of the seeded *E. coli* to prepare a liquid stock of phage library. Panning was performed three times using the Ca-dependent binding activity as an indicator.

(9-3) Assessment by phage ELISA

Culture supernatants containing phages were collected from single colonies of *E. coli* obtained by the method described above according to a conventional method (Methods Mol. Biol. (2002) 178, 133-145). BSA and $CaCl_2$ were added at final concentrations of 4% BSA and 1.2 mM calcium ion concentration, respectively, to the phage-containing culture supernatants.

The supernatants were subjected to ELISA by the following procedure. A StreptaWell 96-well microtiter plate (Roche) was coated overnight with 100 $\mu$l of PBS containing the biotin-labeled antigen. The antigen was removed by washing each well of the plate with PBST. Then, the wells were blocked with 250 $\mu$l of 4% BSA-TBS for one hour or more. After removal of 4% BSA-TBS, the prepared culture supernatants were added to the each well. The plate was incubated at 37°C for one hour so that the antibody-displaying phages were allowed to bind to the antigen on each well. After each well was washed with 1.2 mM $CaCl_2$/TBST, 1.2 mM $CaCl_2$/TBS or 1 mM EDTA/TBS was added. The plate was left for incubation at 37°C for 30 minutes. After washing with 1.2 mM $CaCl_2$/TBST, an HRP-conjugated anti-M13 antibody (Amersham Pharmacia Biotech) diluted with TBS containing BSA and calcium ion at final concentrations of 4% and 1.2 mM calcium ion concentration was added to each well, and the plate was incubated for one hour. After washing with 1.2 mM $CaCl_2$/TBST, the TMB single solution (ZYMED) was added to each well. The chromogenic reaction in the solution of each well was stopped by adding sulfuric acid. Then, the developed color was assessed by measuring absorbance at 450 nm.

From the isolated 96 clones, antibody 6KC4-1#85 having Ca-dependent IL-6-binding activity was obtained by phage ELISA. Using antibody fragments that were predicted to have a Ca-dependent antigen-binding activity based on the result of the phage ELISA described above as a template, genes were amplified with specific primers and their sequences were analyzed. The heavy-chain and light-chain variable region sequences of antibody 6KC4-1#85 are shown in SEQ ID NOs: 10 and 48, respectively. The polynucleotide encoding the heavy-chain variable region of antibody 6KC4-1#85 (SEQ ID NO: 10) was linked to a polynucleotide encoding an IgG1-derived sequence by PCR method. The resulting DNA fragment was inserted into an animal cell expression vector to construct an expression vector for the heavy chain of SEQ ID NO: 49. A polynucleotide encoding the light-chain variable region of antibody 6KC4-1#85 (SEQ ID NO: 48) was linked to a polynucleotide encoding the constant region of the natural Kappa chain (SEQ ID NO: 50) by PCR. The linked DNA fragment was inserted into an animal cell expression vector. Sequences of the constructed variants were confirmed by a method known to those skilled in the art. Sequences of the constructed variants were confirmed by a method known to those skilled in the art.

(9-4) Expression and purification of antibodies

Clone 6KC4-1#85 that was predicted to have a Ca-dependent antigen-binding activity based on the result of phage ELISA was inserted into animal cell expression plasmids. Antibody expression was carried out by the following method. Cells of human fetal kidney cell-derived FreeStyle 293-F (Invitrogen) were suspended in the FreeStyle 293 Expression Medium (Invitrogen), and plated at a cell density of 1.33 x $10^6$ cells/ml (3 ml) into each well of a 6-well plate.

The prepared plasmids were introduced into cells by a lipofection method. The cells are cultured for four days in a $CO_2$ incubator (37°C, 8% $CO_2$, 90 rpm). From the culture supernatants, antibodies were purified using the rProtein A Sepharose™ Fast Flow (Amersham Biosciences) by a method known to those skilled in the art. Absorbance at 280 nm of the purified antibody solutions was measured using a spectrophotometer. Antibody concentrations were calculated from the determined values using an extinction coefficient calculated by the PACE method (Protein Science (1995) 4: 2411-2423).

[Reference Example 10] Assessment of antibody 6KC4-1#85 for calcium ion binding

Calcium-dependent antigen-binding antibody 6KC4-1#85 which was isolated from a human antibody library was assessed for its calcium binding. Whether the measured Tm value varies depending on the ionized calcium concentration condition was assessed according to the method described in Reference Example 7.

Tm values for the Fab domain of antibody 6KC4-1#85 are shown in Table 36. As shown in Table 36, the Tm value of the 6KC4-1#85 antibody Fab domain varied depending on the calcium ion concentration. This demonstrates that antibody 6KC4-1#85 binds to calcium.

[Table 36]

| ANTIBODY | CALCIUM ION CONCENTRATION | | Δ Tm (°C) |
|---|---|---|---|
| | 3 $\mu$M | 2 mM | 2 mM-3 $\mu$M |
| 6KC4-1#85 | 71.49 | 75.39 | 3.9 |

[Reference Example 11] Identification of calcium ion-binding site in antibody 6KC4-1#85

As demonstrated in Reference Example 10, antibody 6KC4-1#85 binds to calcium ion. However, 6KC4-1#85 does not have a calcium-binding motif such as the hVk5-2 sequence which was revealed from assessment to have a calcium-binding motif. Then, whether calcium ion binds to either or both of the heavy chain and the light chain of antibody 6KC4-1#85 was confirmed by assessing the calcium ion binding of altered antibodies resulting from exchanging the heavy chain and light chain of 6KC4-1#85 respectively with those of an anti-glypican 3 antibody (heavy chain sequence GC_H (SEQ ID NO: 51), light chain sequence GC_L (SEQ ID NO: 52)) which does not bind calcium ion. The Tm values of altered antibodies measured according to the method described in Reference Example 7 are shown in Table 37. The result suggests that the heavy chain of antibody 6KC4-1#85 binds to calcium, because the Tm values of the altered antibody having the heavy chain of antibody 6KC4-1#85 changed depending on calcium ion concentration.

[Table 37]

| HEAVY CHAIN | LIGHT CHAIN | CALCIUM ION CONCENTRATION | | ΔTm (°C) 2 |
|---|---|---|---|---|
| | | 3$\mu$M | 2 mM | mM-3$\mu$M |
| 6KC4-1#85 | 6KC4-1#85 | 71.46 | 75.18 | 3.72 |
| 6KC4-1#85 | GC_L | 78.87 | 80.01 | 1.14 |
| GC_H | 6KC4-1#85 | 75.69 | 75.94 | 0.25 |
| GC_H | GC_L | 79.94 | 80.01 | 0.07 |

Thus, to further identify residues responsible for the calcium ion binding of antibody 6KC4-1#85, altered heavy chains (6_H1-11 (SEQ ID NO: 53), 6_H1-12 (SEQ ID NO: 54), 6_H1-13 (SEQ ID NO: 55), 6_H1-14 (SEQ ID NO: 56), 6_H1-15 (SEQ ID NO: 57)) or altered light chains (6_L1-5 (SEQ ID NO: 58) and 6_L1-6 (SEQ ID NO: 59)) were constructed by substituting an Asp (D) residue in the CDR of antibody 6KC4-1#85 with an Ala (A) residue which does not participate in the binding or chelation of calcium ion. By the method described in Reference Example 9, altered antibodies were purified from the culture supernatants of animal cells introduced with expression vectors carrying the altered antibody genes. The purified altered antibodies were assessed for their calcium binding according to the method described in Reference Example 7. The measurement result is shown in Table 38. As shown in Table 38, substitution of an Ala residue for the residue at position 95 or 101 (Kabat numbering) in the heavy chain CDR3 of antibody 6KC4-1#85 resulted in loss of the calcium-binding activity of antibody 6KC4-1#85. This suggests that these residues are responsible for calcium binding. The calcium-binding motif located at the base of the CDR3 loop in the heavy chain of antibody 6KC4-1#85, which was found based on the calcium binding capacity of the antibody altered from antibody 6KC4-1#85, can be a new factor for designing Ca libraries which are used to obtain antigen-binding domains against an antigen depending on the ion concentration which are to be contained in antigen-binding molecules of the present invention. In Reference Examples 18 and 19 below, calcium-binding motifs were introduced into the light chain variable region. Meanwhile, such libraries include, for example, those containing the heavy chain CDR3 from antibody 6KC4-1#85 and flexible residues in the CDRs other than the heavy chain CDR3 but including the light chain CDRs.

[Table 38]

| HEAVY CHAIN | LIGHT CHAIN | ALTERED RESIDUE | CALCIUM ION CONCENTRATION | | ΔTm (°C) |
|---|---|---|---|---|---|
| | | | 3 μM | 2 mM | 2 mM-3 μM |
| 6KC4-1#85 | 6KC4-1#85 | WILD-TYPE | 71.49 | 75.39 | 3.9 |
| 6H1-11 | 6KC4-1 #85 | H CHAIN POSITION 61 (Kabat NUMBERING) | 71.73 | 75.56 | 3.83 |
| 6H1-12 | 6KC4-1#85 | H CHAIN POSITION 95 (Kabat NUMBERING) | 72.9 | 73.43 | 0.53 |
| 6H1-13 | 6KC4-1#85 | H CHAIN POSITION 100a (Kabat NUMBERING) | 70.94 | 76.25 | 5.31 |
| 6H1-14 | 6KC4-1 #85 | H CHAIN POSITION 100g (Kabat NUMBERING) | 73.95 | 75.14 | 1.19 |
| 6H1-15 | 6KC4-1#85 | H CHAIN POSITION 101 (Kabat NUMBERING) | 65.37 | 66.25 | 0.87 |
| 6KC4-1#85 | 6L1-5 | L CHAIN POSITION 50 (Kabat NUMBERING) | 71.92 | 76.08 | 4.16 |
| 6KC4-1#85 | 6L1-6 | L CHAIN POSITION 92 (Kabat NUMBERING) | 72.13 | 78.74 | 6.61 |

[Reference Example 12] Examination of effects of Ca-dependent binding antibody on plasma retention of antigen using normal mice

(12-1) *In vivo* test using normal mice

To a normal mouse (C57BL/6J mouse, Charles River Japan), hsIL-6R (soluble human IL-6 receptor prepared in Reference Example 3) alone was administered, or hsIL-6R and anti-human IL-6 receptor antibody were administered simultaneously to examine the kinetics of the hsIL-6R and anti-human IL-6 receptor antibody *in vivo.* A single dose (10 mL/kg) of the hsIL-6R solution (5 μg/mL) or a mixture of hsIL-6R and anti-human IL-6 receptor antibody was administered into the caudal vein. The above H54/L28-IgG1, 6RL#9-IgG1, and FH4-IgG1 were used as anti-human IL-6 receptor antibodies.

The hsIL-6R concentration in all the mixtures is 5 μg/mL. The concentrations of anti-human IL-6 receptor antibody vary with the antibodies: 0.1 mg/mL for H54/L28-IgG1 and 10 mg/mL for 6RL#9-IgG1 and FH4-IgG1. At this time, it was thought that most of the hsIL-6Rs bind to the antibody because the anti-human IL-6 receptor antibody against hsIL-6R exists in a sufficient or excessive amount. Blood samples were collected at 15 minutes, 7 hours and 1, 2, 4, 7, 14, 21, and 28 days after the administration. The blood samples obtained were immediately centrifuged for 15 minutes at 4°C and 12,000 rpm to separate plasma. The separated plasma was stored in a freezer set to -20°C or lower until the time of measurement.

(12-2) Determination of plasma anti-human IL-6 receptor antibody concentration in normal mice by ELISA

The plasma concentration of anti-human IL-6 receptor antibody in a mouse was determined by ELISA. First, Anti-Human IgG (γ-chain specific) F(ab')2 Fragment of Antibody (SIGMA) was dispensed into a Nunc-Immuno Plate, MaxiSorp (Nalge Nunc International), and was allowed to stand undisturbed overnight at 4°C to prepare an anti-human IgG-solid phase plate. Calibration curve samples at a plasma concentration of 0.64, 0.32, 0.16, 0.08, 0.04, 0.02, or 0.01 μg/mL, and mouse plasma measurement samples diluted by 100-fold or above were each dispensed into the anti-human IgG-solid phase plate, followed by incubation for 1 hour at 25°C. Subsequently, the plate was allowed to react with a biotinylated anti-human IL-6 R antibody (R&D) for 1 hour at 25°C, followed by reaction with Streptavidin-PolyHRP80 (Stereospecific Detection Technologies) for 0.5 hours at 25°C. The chromogenic reaction was conducted using TMB One Component HRP Microwell Substrate (BioFX Laboratories) as a substrate. After the chromogenic reaction was stopped by adding 1N-sulfuric acid (Showa Chemical), absorbance at 450 nm of the color solution was measured using a microplate reader. The plasma concentration in the mouse was calculated from the absorbance of the calibration curve using the SOFTmax PRO analysis software (Molecular Devices). Changes in the plasma concentrations of antibodies, H54/L28-IgG1, 6RL#9-IgG1, and FH4-IgG1, in the normal mice after intravenous administration, measured as described above, are shown in Fig. 50.

(12-3) Determination of plasma hsIL-6R concentration by an electrochemiluminescence method

The plasma concentration of hsIL-6R in a mouse was determined by an electrochemiluminescence method. A hsIL-6R calibration curve sample prepared at 2,000, 1,000, 500, 250, 125, 62.5, or 31.25 pg/mL, and a mouse plasma measurement sample diluted by 50-fold or above, were mixed with a monoclonal anti-human IL-6R antibody (R&D) ruthenated with SULFO-TAG NHS Ester (Meso Scale Discovery), and a biotinylated anti-human IL-6 R antibody (R&D), and tocilizumab (heavy chain SEQ ID NO: 60, light chain SEQ ID NO: 61), followed by overnight reaction at 4°C. At that time, the assay buffer contained 10 mM EDTA to reduce the free Ca concentration in the sample and dissociate almost all the hsIL-6Rs in the sample from 6RL#9-IgG1 or FH4-IgG1 to be bound to the added tocilizumab. Subsequently, said reaction liquid was dispensed into an MA400 PR Streptavidin Plate (Meso Scale Discovery). In addition, after washing each well of the plate that was allowed to react for 1 hour at 25°C, Read Buffer T (×4) (Meso Scale Discovery) was dispensed into each well. Immediately, the reaction liquid was subjected to measurement using a SECTOR PR 400 reader (Meso Scale Discovery). The concentration of hsIL-6R was calculated from the response of the calibration curve using the SOFTmax PRO analysis software (Molecular Devices). Changes in the plasma concentration of hsIL-6R in the normal mouse after intravenous administration, determined as described above, are shown in Fig. 51.

As a result, the disappearance of hsIL-6R was very rapid when hsIL-6R was administered alone, while the disappearance of hsIL-6R was significantly delayed when hsIL-6R was administered simultaneously with H54/L28-IgG1, a conventional antibody having no Ca-dependent binding ability to hsIL-6R. In contrast, the disappearance of hsIL-6R was significantly accelerated when hsIL-6R was administered simultaneously with 6RL#9-IgG1 or FH4-IgG1 having 100-fold or higher Ca-dependent binding ability to hsIL-6R. The plasma concentrations of hsIL-6R one day after hsIL-6R was administered simultaneously with 6RL#9-IgG1 and FH4-IgG1 were reduced 39-fold and 2-fold, respectively, as compared with simultaneous administration with H54/L28-IgG1. Thus, the calcium-dependent binding antibodies were confirmed to be able to accelerate antigen disappearance from the plasma.

[Reference Example 13] Exploration of human germline sequences that bind to calcium ion

(13-1) Antibody that binds to antigen in a calcium-dependent manner

Antibodies that bind to an antigen in a calcium-dependent manner (calcium-dependent antigen-binding antibodies) are those whose interactions with antigen change with calcium concentration. A calcium-dependent antigen-binding antibody is thought to bind to an antigen through calcium ion. Thus, amino acids that form an epitope on the antigen side are negatively charged amino acids that can chelate calcium ions or amino acids that can be a hydrogen-bond acceptor. These properties of amino acids that form an epitope allows targeting of an epitope other than binding molecules, which are generated by introducing histidines and bind to an antigen in a pH-dependent manner. Furthermore, the use of antigen-binding molecules having calcium- and pH-dependent antigen-binding properties is thought to allow the formation of antigen-binding molecules that can individually target various epitopes having broad properties. Thus, if a population of molecules containing a calcium-binding motif (Ca library) is constructed, from which antigen-binding molecules are obtained, calcium-dependent antigen-binding antibodies are thought to be effectively obtained.

(13-2) Acquisition of human germline sequences

An example of the population of molecules containing a calcium-binding motif is an example in which said molecules are antibodies. In other words, an antibody library containing a calcium-binding motif may be a Ca library.

Calcium ion-binding antibodies containing human germline sequences have not been reported. Thus, the germline sequences of antibodies having human germline sequences were cloned using as a template cDNA prepared from Human Fetal Spleen Poly RNA (Clontech) to assess whether antibodies having human germline sequences bind to calcium ion. Cloned DNA fragments were inserted into animal cell expression vectors. The nucleotide sequences of the constructed expression vectors were determined by a method known to those skilled in the art. The SEQ IDs are shown in Table 39. By PCR, polynucleotides encoding SEQ ID NO: 5 (Vk1), SEQ ID NO: 6 (Vk2), SEQ ID NO: 7 (Vk3), SEQ ID NO: 8 (Vk4), and SEQ ID NO: 62 (Vk5-2) were linked to a polynucleotide encoding the natural Kappa chain constant region (SEQ ID NO: 50). The linked DNA fragments were inserted into animal cell expression vectors. Furthermore, heavy chain variable region polynucleotides encoding SEQ ID NO: 63 (Vk1), SEQ ID NO: 64 (Vk2), SEQ ID NO: 65 (Vk3), and SEQ ID NO: 66 (Vk4) were linked by PCR to a polynucleotide encoding an IgG1 of SEQ ID NO: 163 (having a deletion of two amino acids at the C terminus of natural sequence). The resulting DNA fragments were inserted into animal cell expression vectors. The sequences of the constructed variants were confirmed by a method known to those skilled in the art.

[Table 39]

| LIGHT CHAIN GERMLINE SEQUENCE | HEAVY CHAIN (VARIABLE REGION) SEQ ID NO | LIGHT CHAIN VARIABLE REGION SEQ ID NO |
|---|---|---|
| Vk1 | 63 | 5 |
| Vk2 | 64 | 6 |
| Vk3 | 65 | 7 |
| Vk4 | 66 | 8 |
| Vk5 | 67 | 62 |

(13-3) Expression and purification of antibodies

The constructed animal cell expression vectors inserted with the DNA fragments having the five types of human germ-line sequences were introduced into animal cells. Antibody expression was carried out by the following method. Cells of human fetal kidney cell-derived FreeStyle 293-F (Invitrogen) were suspended in the FreeStyle 293 Expression Medium (Invitrogen), and plated at a cell density of 1.33 x $10^6$ cells/ml (3 ml) into each well of a 6-well plate. The prepared plasmids are introduced into cells by a lipofection method. The cells were cultured for four days in a $CO_2$ incubator (37°C, 8% $CO_2$, 90 rpm). From the culture supernatants prepared as described above, antibodies were purified using the rProtein A Sepharose™ Fast Flow (Amersham Biosciences) by a method known to those skilled in the art. Absorbance at 280 nm of the purified antibody solutions was measured using a spectrophotometer. Antibody concentrations were calculated from the determined values using an extinction coefficient calculated by the PACE method (Protein Science (1995) 4: 2411-2423).

(13-4) Assessment of antibodies having human germ-line sequences for their calcium ion-binding activity

The purified antibodies were assessed for their calcium ion-binding activity. The intermediate temperature of thermal denaturation (Tm value) was measured by differential scanning calorimetry (DSC) as an indicator for examining calcium ion binding to the antibody (MicroCal VP-Capillary DSC, MicroCal). The intermediate temperature of thermal denaturation (Tm value) is an indicator of stability. The Tm value becomes higher when a protein is stabilized through calcium ion binding, as compared with the case where no calcium ion is bound (J. Biol. Chem. (2008) 283, 37, 25140-25149). The binding activity of calcium ion to antibody was evaluated by examining changes in the Tm value of the antibody depending on the changes in the calcium ion concentration in the antibody solution. The purified antibody was subjected to dialysis (EasySEP, TOMY) using an external solution of 20 mM Tris-HCl, 150 mM NaCl, and 2 mM $CaCl_2$ (pH 7.4) or 20 mM Tris-HCl, 150 mM NaCl, and 3 $\mu$M $CaCl_2$ (pH 7.4). DSC measurement was conducted at a heating rate of 240°C/hr from 20 to 115°C using as a test substance an antibody solution prepared at about 0.1 mg/mL with the dialysate. The intermediate temperatures of thermal denaturation (Tm values) of the Fab domains of each antibody, calculated from the denaturation curve obtained by DSC, are shown in Table 40.

[Table 40]

| LIGHT CHAIN GERMLINE SEQUENCE | CALCIUM ION CONCENTRATION | | ΔTm (°C) |
|---|---|---|---|
| | 3 μM | 2 mM | 2 mM-3 μM |
| hVk1 | 80.32 | 80.78 | 0.46 |
| hVk2 | 80.67 | 80.61 | -0.06 |
| hVk3 | 81.64 | 81.36 | -0.28 |
| hVk4 | 70.74 | 70.74 | 0 |
| hVk5 | 71.52 | 74.17 | 2.65 |

The result showed that the Tm values of the Fab domains of antibodies having the hVk1, hVk2, hVk3, or hVk4 sequence did not vary depending on the calcium ion concentration in the Fab domain-containing solutions. Meanwhile, the Tm value for the antibody Fab domain having the hVk5 sequence varied depending on the calcium ion concentration in the Fab domain-containing solution. This demonstrates that the hVk5 sequence binds to calcium ion.

(13-5) Assessment of hVk5-2 sequence for calcium binding

In (5-2) of Reference Example 5, Vk5-2 variant 1 (SEQ ID NO: 68) and Vk5-2 variant 2 (SEQ ID NO: 69) were obtained in addition to Vk5-2 (SEQ ID NO: 4), all of which are classified as Vk5-2. These variants were assessed for their calcium binding. DNA fragments for Vk5-2, Vk5-2 variant 1, and Vk5-2 variant 2 were each inserted into animal cell expression vectors. The nucleotide sequences of the constructed expression vectors were determined by a method known to those skilled in the art. By the method described in (12-3) of Reference Example 12, the animal cell expression vectors inserted with DNA fragments for Vk5-2, Vk5-2 variant 1, and Vk5-2 variant 2 were introduced, in combination with animal expression vector carrying an insert to express CIM_H (SEQ ID NO: 67) as a heavy chain, into animal cells and antibodies were purified. The purified antibodies were assessed for their calcium ion-binding activity. The purified antibodies were dialyzed (EasySEP, TOMY) against 20 mM Tris-HCl/150 mM NaCl (pH 7.5) (in Table 41, indicated as 0 mM calcium ion concentration) or 20 mM Tris-HCl/150 mM NaCl/2 mM $CaCl_2$ (pH 7.5). DSC measurement was carried out at a rate of temperature increase of 240°C/hr from 20 to 115°C using antibody solutions prepared at a concentration of about 0.1 mg/mL by the same solution as used for dialysis. Based on the obtained DSC denaturation curves, the intermediate temperature of thermal denaturation (Tm value) was calculated for the Fab domain of each antibody. The Tm values are shown in Table 41.

[Table 41]

| LIGHT CHAIN | CALCIUM ION CONCENTRATION | | Δ Tm (°C) |
|---|---|---|---|
| | 0 mM | 2 mM | 2 mM-0mM |
| Vk5-2 | 71.65 | 74.38 | 2.73 |
| Vk5-2 VARIANT 1 | 65.75 | 72.24 | 6.49 |
| Vk5-2 VARIANT 2 | 66.46 | 72.24 | 5.78 |

The result showed that the Tm value for the Fab domains of antibodies having the sequence of Vk5-2, Vk5-2 variant 1, or Vk5-2 variant 2 varied depending on the calcium ion concentration in solutions containing antibodies having the Fab domains. This demonstrates that antibodies having a sequence classified as Vk5-2 bind to calcium ion.

[Reference Example 14] Assessment of the human Vk5 (hVk5) sequence

(14-1) hVk5 sequence

The only hVk5 sequence registered in Kabat database is hVk5-2 sequence. Herein, hVk5 and hVk5-2 are used synonymously. WO2010/136598 discloses that the abundance ratio of the hVk5-2 sequence in the germline sequence is 0.4%. Other reports have been also made in which the abundance ratio of the hVk5-2 sequence in the germline sequence is 0-0.06% (J. Mol. Biol. (2000) 296, 57-86; Proc. Natl. Acad. Sci. (2009) 106, 48, 20216-20221). As described above, since the hVk5-2 sequence is a sequence of low appearance frequency in the germline sequence, it was thought to be inefficient to obtain a calcium-binding antibody from an antibody library consisting of human germline sequences or B cells obtained by immunizing a mouse expressing human antibodies. Thus, it is possible to design Ca libraries containing the sequence of human hVk5-2. Meanwhile, reported synthetic antibody libraries (WO2010/105256 and WO2010/136598) did not contain the sequence of hVk5. In addition, the possibility of realization is unknown because no report has been published on the physical properties of the hVk5-2 sequence.

(14-2) Construction, expression, and purification of a non-glycosylated form of the hVk5-2 sequence

The hVk5-2 sequence has a sequence for N-type glycosylation at position 20 amino acid (Kabat numbering). Sugar chains attached to proteins exhibit heterogeneity. Thus, it is desirable to lose the glycosylation from the viewpoint of substance homogeneity. In this context, variant hVk5-2_L65 (SEQ ID NO: 70) in which the Asn (N) residue at position 20 (Kabat numbering) is substituted with Thr (T) was constructed. Amino acid substitution was carried out by a method known to those skilled in the art using the QuikChange Site-Directed Mutagenesis Kit (Stratagene). A DNA encoding the variant hVk5-2_L65 was inserted into an animal expression vector. The animal expression vector inserted with the constructed DNA encoding variant hVk5-2_L65, in combination with an animal expression vector having an insert to express CIM_H (SEQ ID NO: 67) as a heavy chain, was introduced into animal cells by the method described in Reference Example 5. The antibody comprising hVk5-2_L65 and CIM_H, which was expressed in animal cells introduced with the vectors, was purified by the method described in Reference Example 13.

(14-3) Assessment of the antibody having the non-glycosylated hVk5-2 sequence for physical properties

The isolated antibody having the modified sequence hVk5-2_L65 was analyzed by ion-exchange chromatography to test whether it is less heterogeneous than the antibody having the original sequence hVk5-2 before alteration. The procedure of ion-exchange chromatography is shown in Table 42. The analysis result showed that hVk5-2_L65 modified at the glycosylation site was less heterogeneous than the original sequence hVk5-2, as shown in Fig. 52.

[Table 42]

| | CONDITION |
|---|---|
| COLUMN | TOSOH TSKgel DEAE-NPR |
| MOBILE PHASE | A; 10 mM Tris-HCl, 3 $\mu$M $CaCl_2$(pH8.0) |
| | B; 10 mM Tris-HCl, 500 mM NaCl, 3 $\mu$M $CaCl_2$ (pH8.0) |
| GRADIENT SCHEDULE | %B = 0 - (5min) - 0 - 2%/1min |
| COLUMN TEMPERATURE | 40°C |
| DETECTION | 280 nm |
| INJECTION VOLUME | 100 $\mu$L (5 $\mu$g) |

Next, whether the less-heterogeneous hVk5-2_L65 sequence-comprising antibody binds to calcium ion was assessed by the method described in Reference Example 13. The result showed that the Tm value for the Fab domain of the antibody having hVk5-2_L65 with altered glycosylation site also varied depending on the calcium ion concentration in the antibody solutions, as shown in Table 43. Specifically, it was demonstrated that the Fab domain of the antibody having hVk5-2_L65 with altered glycosylation site binds to calcium ion.

[Table 43]

| LIGHT CHAIN | GLYCOSYLATED SEQUENCE | CALCIUM ION CONCENTRATION | | $\Delta$ Tm(°C) |
|---|---|---|---|---|
| | | 3 $\mu$M | 2 mM | 2 mM-3 $\mu$M |
| hVk5-2 | YES | 71.52 | 74.17 | 2.65 |
| hVk5-2_L65 | NO | 71.51 | 73.66 | 2.15 |

[Reference Example 15] Assessment of the calcium ion-binding activity of antibody molecules having CDR sequence of the hVk5-2 sequence

(15-1) Construction, expression, and purification of modified antibodies having a CDR sequence from the hVk5-2 sequence

The hVk5-2_L65 sequence is a sequence with altered amino acids at a glycosylation site in the framework of human Vk5-2 sequence. As described in Reference Example 14, it was demonstrated that calcium ion bound even after alteration of the glycosylation site. Meanwhile, from the viewpoint of immunogenicity, it is generally desirable that the framework sequence is a germ-line sequence. Thus, the present inventors assessed whether an antibody framework sequence could be substituted with the framework sequence of a non-glycosylated germline sequence while maintaining the calcium ion-binding activity of the antibody.

Polynucleotides encoding chemically synthesized sequences which comprise an altered framework sequence of the hVk5-2 sequence, hVk1, hVk2, hVk3, or hVk4 (CaVk1 (SEQ ID NO: 71), CaVk2 (SEQ ID NO: 72), CaVk3 (SEQ ID NO: 73), or CaVk4 (SEQ ID NO: 74), respectively) were linked by PCR to a polynucleotide encoding the constant region (SEQ ID NO: 50) of the natural Kappa chain. The linked DNA fragments were inserted into animal cell expression vectors. Sequences of the constructed variants were confirmed by a method known to those skilled in the art. Each plasmid constructed as described above was introduced into animal cells in combination with a plasmid inserted with a polynucleotide encoding CIM_H (SEQ ID NO: 67) by the method described in Reference Example 13. The expressed antibody molecules of interest were purified from culture media of the animal cells introduced with the plasmids.

(15-2) Assessment of altered antibodies having the CDR sequence of the hVk5-2 sequence for their calcium ion-binding activity

Whether calcium ion binds to altered antibodies having the CDR sequence of the hVK5-2 sequence and the framework sequences of germline sequences other than hVk5-2 (hVk1, hVk2, hVk3, and hVk4) was assessed by the method described in Reference Example 5. The assessment result is shown in Table 44. The Tm value of the Fab domain of each altered antibody was revealed to vary depending on the calcium ion concentration in the antibody solutions. This demonstrates that antibodies having a framework sequence other than the framework sequence of hVk5-2 sequence also bind to calcium ion.

[Table 44]

| GERMLINE (LIGHT CHAIN FRAMEWORK SEQUENCE) | CALCIUM ION CONCENTRATION | | Δ Tm (°C) |
|---|---|---|---|
| | 3 μM | 2 mM | 2 mM-3 μM |
| hVk1 | 77.51 | 79.79 | 2.28 |
| hVk2 | 78.46 | 80.37 | 1.91 |
| hVk3 | 77.27 | 79.54 | 2.27 |
| hVk4 | 80.35 | 81.38 | 1.03 |
| hVk5-2 | 71.52 | 74.17 | 2.65 |

The thermal denaturation temperature (Tm value), as an indicator of thermal stability, of the Fab domain of each antibody altered to have the CDR sequence of the hVK5-2 sequence and the framework sequence of a germ-line sequence other than the hVk5-2 sequence (hVk1, hVk2, hVk3, or hVk4) was demonstrated to be greater than that of the Fab domain of the original antibody having the hVk5-2 sequence. This result shows that antibodies having the CDR sequence of the hVk5-2 sequence and the framework sequence of hVk1, hVk2, hVk3, or hVk4 not only have calcium ion-binding activity but also are excellent molecules from the viewpoint of thermal stability.

[Reference Example 16] Identification of the calcium ion-binding site in human germline hVk5-2 sequence

(16-1) Design of mutation site in the CDR sequence of the hVk5-2 sequence

As described in Reference Example 15, antibodies having the light chain resulting from introduction of the CDR portion of the hVk5-2 sequence into the framework sequence of a different germline sequence were also demonstrated to bind to calcium ion. This result suggests that in hVk5-2 a calcium ion-binding site is localized within its CDR. Amino acids that bind to calcium ion, *i.e.,* chelate calcium ion, include negatively charged amino acids and amino acids that can be a hydrogen bond acceptor. Thus, it was tested whether antibodies having a mutant hVk5-2 sequence with a substitution of an Ala (A) residue for an Asp (D) or Glu (E) residue in the CDR sequence of the hVk5-2 sequence bind to calcium ion.

(16-2) Construction of variant hVk5-2 sequences with Ala substitution, and expression and purification of antibodies

Antibody molecules were prepared to comprise a light chain with substitution of an Ala residue for Asp and/or Glu residue in the CDR sequence of the hVk5-2 sequence. As described in Reference Example 14, non-glycosylated variant hVk5-2_L65 exhibited calcium ion binding and was assumed to be equivalent to the hVk5-2 sequence in terms of calcium ion binding. In this Example, amino acid substitutions were introduced into hVk5-2_L65 as a template sequence. Constructed variants are shown in Table 45. Amino acid substitutions were carried out by methods known to those skilled in the art such as using the QuikChange Site-Directed Mutagenesis Kit (Stratagene), PCR, or the In fusion Advantage PCR Cloning Kit (TAKARA) to construct expression vectors for altered light chains having an amino acid substitution.

[Table 45]

| LIGHT CHAIN VARIANT NAME | ALTERED POSITION (Kabat NUMBERING) | SEQ ID NO |
|---|---|---|
| hVk5-2_L65 | **WILD TYPE** | 70 |
| hVk5-2_L66 | 30 | 75 |
| hVk5-2_L67 | 31 | 76 |
| hVk5-2_L68 | 32 | 77 |
| hVk5-2_L69 | 50 | 78 |
| hVk5-2_L70 | 30, 32 | 79 |
| hVk5-2_L71 | 30, 50 | 80 |
| hVk5-2_L72 | 30, 32, 50 | 81 |
| hVk5-2_L73 | 92 | 82 |

Nucleotide sequences of the constructed expression vectors were confirmed by a method known to those skilled in the art. The expression vectors constructed for the altered light chains were transiently introduced, in combination with an expression vector for the heavy chain CIM_H (SEQ ID NO: 67), into cells of the human fetal kidney cell-derived HEK293H line (Invitrogen) or FreeStyle293 (Invitrogen) to express antibodies. From the obtained culture supernatants, antibodies were purified using the rProtein A SepharoseTM Fast Flow (GE Healthcare) by a method known to those skilled in the art. Absorbance at 280 nm of the purified antibody solutions was measured using a spectrophotometer. Antibody concentrations were calculated from the determined values using an extinction coefficient calculated by the PACE method (Protein Science (1995) 4: 2411-2423).

(16-3) Assessment of the calcium ion-binding activity of antibodies having an Ala substitution in the hVk5-2 sequence

Whether the obtained purified antibodies bind to calcium ion was tested by the method described in Reference Example 13. The result is shown in Table 46. Some antibodies having substitution of an Asp or Glu residue in the CDR sequence of the hVk5-2 sequence with an Ala residue which cannot be involved in calcium ion binding or chelation were revealed to have an Fab domain whose Tm did not vary by the calcium ion concentration in the antibody solutions. The substitution sites at which Ala substitution did not alter the Tm (positions 32 and 92 (Kabat numbering)) were demonstrated to be greatly important for the calcium ion-antibody binding.

[Table 46]

| LIGHT CHAIN VARIANT NAME | ALTERED POSITION (Kabat's NUMBERING) | CALCIUM ION CONCENTRATION | | Δ Tm (°C) |
|---|---|---|---|---|
| | | 0 μM | 2 mM | 2 mM-0 μM |
| hVk5-2_L65 | WILDTYPE | 71.71 | 73.69 | 1.98 |
| hVk5-2_L66 | 30 | 71.65 | 72.83 | 1.18 |
| hVk5-2_L67 | 31 | 71.52 | 73.30 | 1.78 |
| hVk5-2_L68 | 32 | 73.25 | 74.03 | 0.78 |
| hVk5-2_L69 | 50 | 72.00 | 73.97 | 1.97 |
| hVk5-2_L70 | 30, 32 | 73.42 | 73.60 | 0.18 |
| hVk5-2_L71 | 30, 50 | 71.84 | 72.57 | 0.73 |
| hVk5-2_L72 | 30, 32, 50 | 75.04 | 75.17 | 0.13 |
| hVk5-2_L73 | 92 | 75.23 | 75.04 | -0.19 |

[Reference Example 17] Assessment of the antibodies having hVk1 sequence with calcium ion-binding motif

(17-1) Construction of an hVk1 sequence with calcium ion-binding motif, and expression and purification of antibodies

The result described in Reference Example 16 on the calcium-binding activity of the Ala substitute demonstrates that Asp or Glu residues in the CDR sequence of the hVk5-2 sequence were important for calcium binding. Thus, the present inventors assessed whether an antibody can bind to calcium ion when the residues at positions 30, 31, 32, 50, and 92 (Kabat numbering) alone were introduced into a different germline variable region sequence. Specifically, variant LfVk1_Ca (SEQ ID NO: 83) was constructed by substituting the residues at positions 30, 31, 32, 50, and 92 (Kabat numbering) in the hVk5-2 sequence for the residues at positions 30, 31, 32, 50, and 92 (Kabat numbering) in the hVk1 sequence (a human germline sequence). Specifically, it was tested whether antibodies having an hVk1 sequence introduced with only 5 residues from the hVk5-2 sequence can bind to calcium. The variants were produced by the same method as described in Reference Example 16. The resulting light chain variant LfVk1_Ca and LfVk1 having the light-chain hVk1 sequence (SEQ ID NO: 84) were co-expressed with the heavy chain CIM_H (SEQ ID NO: 67). Antibodies were expressed and purified by the same method as described in Reference Example 16.

(17-2) Assessment of the calcium ion-binding activity of antibodies having a human hVk1 sequence with calcium ion-binding motif

Whether the purified antibody prepared as described above binds to calcium ion was assessed by the method described in Reference Example 13. The result is shown in Table 47. The Tm value of the Fab domain of the antibody having LfVk1 with an hVk1 sequence did not vary depending on the calcium concentration in the antibody solutions. Meanwhile, Tm of the antibody having the LfVk1_Ca sequence was shifted by 1°C or more upon change in the calcium concentration in the antibody solutions. Thus, it was shown that the antibody having LfVk1_Ca binds to calcium. The result described above demonstrates that the entire CDR sequence of hVk5-2 is not required, while the residues introduced for construction of the LfVk1_Ca sequence alone are sufficient for calcium ion binding.

[Table 47]

| LIGHT CHAIN VARIANT | CALCIUM ION CONCENTRATION | | Δ Tm(°C) |
|---|---|---|---|
| | 3 μM | 2 mM | 2 mM-3 μM |
| LfVk1 | 83.18 | 83.81 | 0.63 |
| LfVk1_Ca | 79.83 | 82.24 | 2.41 |

(17-3) Construction, expression, and purification of degradation-resistant LfVkl Ca sequence

As described in (17-1) of Reference Example 17, variant LfVk1_Ca (SEQ ID NO: 66) was constructed to have substitution of residues at positions 30, 31, 32, 50, and 92 (Kabat numbering) in the hVk5-2 sequence for residues at positions 30, 31, 32, 50, and 92 (Kabat numbering) in the hVk1 sequence (a human germline sequence). The variant was demonstrated to bind to calcium ion. Thus, it is possible to design Ca libraries containing LfVk1_Ca sequence. Meanwhile, there is no report on the properties of LfVk1_Ca sequence, and thus its feasibility was unknown. LfVk1_Ca sequence has Asp at positions 30, 31, and 32 (Kabat numbering). Thus, the Asp-Asp sequence which has been reported to be degraded under acidic condition is contained in the CDR1 sequence (J. Pharm. Biomed. Anal. (2008) 47(1), 23-30). It is desirable to avoid the degradation at acidic conditions from the viewpoint of the storage stability. Then, variants LfVk1_Ca1 (SEQ ID NO: 85), LfVk1_Ca2 (SEQ ID NO: 86), and LfVk1_Ca3 (SEQ ID NO: 87) were constructed to have substitution of Ala (A) residues for Asp (D) residues that are possibly sensitive to degradation. Amino acid substitution was carried out by a method known to those skilled in the art using the QuikChange Site-Directed Mutagenesis Kit (Stratagene). DNAs encoding the variants were inserted into animal expression vectors. In combination with an animal expression vector having an insert to express GC_H (SEQ ID NO: 51) as the heavy chain, the constructed animal expression vectors carrying DNA inserts for the variants were introduced into animal cells by the method described in Reference Example 13. The antibodies expressed in the animal cells introduced with the vectors were purified by the method described in Reference Example 13.

(17-4) Stability assessment of antibodies having the degradation-resistant LfVkl Ca sequence

Whether the antibodies prepared as described in (17-3) of Reference Example 17 were more resistant to degradation in solutions at pH 6.0 than the original antibodies having the LfVk1_Ca sequence provided for alteration was assessed by comparing the heterogeneity between respective antibodies after thermal acceleration. Each antibody was dialyzed against a solution of 20 mM Histidine-HCl, 150 mM NaCl (pH 6.0) under a condition of 4°C overnight. Dialyzed antibodies were adjusted to 0.5 mg/mL and stored at 5°C or 50°C for three days. Each antibody after storage was subjected to ion-exchange chromatography using the method described in Reference Example 14. As shown in Fig. 53, the analysis result demonstrates that LfVk1_Ca1 with an alteration at degradation site was less heterogeneous and much more resistant to degradation from thermal acceleration than the original LfVk1_Ca sequence. Specifically, it was demonstrated that degradation occurred at the Asp (D) residue of position 30 in the LfVk1_Ca sequence but it could be prevented by amino acid alteration.

(17-5) Construction of a light chain LVk1_Ca sequence resistant to degradation at the Asp residue of position 30, and expression and purification of antibodies

The result described in (17-4) of Reference Example 17 on the degradation resistance of the Ala-substituted form demonstrates that under acidic conditions the LfVk1_Ca sequence was degraded at the Asp (D) residue of position 30 (Kabat numbering) in its CDR sequence and the degradation could be prevented in the case substitution of a different amino acid (in (17-4), substituted with an Ala (A) residue) for the residue at position 30 (Kabat numbering). Then, the present inventors tested whether even a sequence with a substitution of Ser (S), a main residue capable of chelating calcium ion, for the residue at position 30 (Kabat numbering) (referred to as LfVk1_Ca6; SEQ ID NO: 88) was resistant to degradation while maintaining the calcium-binding activity. Variants were prepared by the same method as described in Reference Example 9. The altered light chains LfVk1_Ca6 and LfVk1_Ca sequences were expressed in combination with a heavy chain GC_H (SEQ ID NO: 51). Antibodies were expressed and purified by the same method as described in Reference Example 16.

(17-6) Assessment of a light chain LVk1_Ca sequence resistant to degradation at Asp residue at position 30

Purified antibodies prepared as described above were assessed for their storage stability under acidic conditions by the method described in (17-4) of Reference Example 17. The result demonstrates that antibodies having the LfVk1_Ca6 sequence are more resistant to degradation than those having the original LfVk1_Ca sequence, as shown in Fig. 54.

Then, whether antibodies having the LfVk1_Ca sequence and antibodies having the LfVk1_Ca6 sequence bind to calcium ion was tested by the method described in Reference Example 13. The result is shown in Table 48. The Tm values of the Fab domains of antibodies having LfVk1_Ca sequence and antibodies having the degradation-resistant LfVk1_Ca6 sequence were shifted by 1°C or more upon change in the calcium concentration in antibody solutions.

[Table 48]

| LIGHT CHAIN VARIANT | CALCIUM ION CONCENTRATION | | Δ Tm (°C) |
|---|---|---|---|
| | 3 μM | 2 mM | 2 mM-3 μM |
| LfVk1_Ca | 78.45 | 80.06 | 1.61 |
| LfVk1_Ca6 | 78.44 | 79.74 | 1.30 |

[Reference Example 18] Design of a population of antibody molecules (Ca library) with a calcium ion-binding motif introduced into the variable region to effectively obtain antibodies that bind to antigen in a Ca concentration-dependent manner

Preferred calcium-binding motifs include, for example, the hVk5-2 sequence and the CDR sequence, as well as residues at positions 30, 31, 32, 50, and 92 (Kabat numbering). Other calcium binding motifs include the EF-hand motif possessed by calcium-binding proteins (*e.g.,* calmodulin) and C-type lectin (*e.g.,* ASGPR).

The Ca library consists of heavy and light chain variable regions. A human antibody sequence was used for the heavy chain variable region, and a calcium-binding motif was introduced into the light chain variable region. The hVk1 sequence was selected as a template sequence of the light chain variable region for introducing a calcium-binding motif. An antibody containing an LfVk1_Ca sequence obtained by introducing the CDR sequence of hVk5-2 (one of calcium-binding motifs) into the hVk1 sequence was shown to bind to calcium ions, as shown in Reference Example 16. Multiple amino acids were allowed to appear in the template sequence to diversify antigen-binding molecules that constitute the library. Positions exposed on the surface of a variable region which is likely to interact with the antigen were selected as those where multiple amino acids are allowed to appear. Specifically, positions 30, 31, 32, 34, 50, 53, 91, 92, 93, 94, and 96 (Kabat numbering) were selected as flexible residues.

The type and appearance frequency of amino acid residues that were subsequently allowed to appear were determined. The appearance frequency of amino acids in the flexible residues of the hVk1 and hVk3 sequences registered in the Kabat database (KABAT, E.A. ET AL.: 'Sequences of proteins of immunological interest', vol. 91, 1991, NIH PUBLICATION) was analyzed. Based on the analysis results, the type of amino acids that were allowed to appear in the Ca library were selected from those with higher appearance frequency at each position. At this time, amino acids whose appearance frequency was determined to be low based on the analysis results were also selected to avoid the bias of amino acid properties. The appearance frequency of the selected amino acids was determined in reference to the analysis results of the Kabat database.

A Ca library containing a calcium-binding motif with emphasis on the sequence diversity as to contain multiple amino acids at each residue other than the motif were designed as a Ca library in consideration of the amino acids and appearance frequency set as described above. The detailed designs of the Ca library are shown in Tables 1 and 2 (with the positions in each table representing the Kabat numbering). In addition, regarding the frequency of appearance of amino acids described in Tables 1 and 2, if position 92 represented by the Kabat numbering is Asn (N), position 94 may be Leu (L) instead of Ser (S).

[Reference Example 19] Ca library preparation

A gene library of antibody heavy-chain variable regions was amplified by PCR using a poly A RNA prepared from human PBMC, and commercial human poly A RNA, etc. as a template. As described in Reference Example 18, for the light chain variable region portion of antibody, light chain portions of variable regions of antibody that increase appearance frequency of antibodies which maintain a calcium-binding motif and can bind to an antigen in a calcium concentration-dependent manner were designed. In addition, of flexible residues, for amino acid residues other than those with a calcium-binding motif introduced, a library of antibody light chain variable regions with evenly distributed amino acids of high appearance frequency in natural human antibodies was designed with reference to the information of amino acid appearance frequency in natural human antibodies (KABAT, E.A. ET AL.: 'Sequences of proteins of immunological interest', vol. 91, 1991, NIH PUBLICATION). A combination of the gene libraries of antibody heavy-chain and light-chain variable regions generated as described above, was inserted into a phagemid vector to construct a human antibody phage display library that presents Fab domains consisting of human antibody sequences (Methods Mol Biol. (2002) 178, 87-100).

The sequences of antibody gene portions isolated from *E. coli* introduced with an antibody gene library were determined according to the method described in Reference Example 23 below. The amino acid distribution in the sequences of isolated 290 types of clones and a designed amino acid distribution are shown in Fig. 55.

[Reference Example 20] Examination of the calcium ion-binding activity of molecules contained in the Ca Library

(20-1) Calcium ion-binding activity of molecules contained in the Ca library

As described in Reference Example 14, the hVk5-2 sequence that was demonstrated to bind to calcium ions is a sequence of low appearance frequency in the germline sequence. Thus, it was thought to be inefficient to obtain a calcium-binding antibody from an antibody library consisting of human germline sequences or from B cells obtained by immunizing a mouse expressing human antibodies. As a result, a Ca library was constructed. The presence or absence of a clone showing calcium binding to the constructed Ca library was examined.

(20-2) Expression and purification of antibodies

Clones of the Ca library were introduced into animal cell expression plasmids. Antibodies were expressed using the method described below. Cells of human fetal kidney cell-derived FreeStyle 293-F line (Invitrogen) were suspended in FreeStyle 293 Expression Medium (Invitrogen), and plated at a cell density of 1.33 x $10^6$ cells/ml (3ml) to each well of a 6-well plate. The prepared plasmids were introduced into the cells by a lipofection method. The cells were cultured in a $CO_2$ incubator (37°C, 8%$CO_2$, 90 rpm) for four days. By a method known to those skilled in the art, antibodies were purified using rProtein A SepharoseTM Fast Flow (Amersham Biosciences) from culture supernatants obtained as described above. The absorbance of solutions of purified antibodies was measured at 280 nm using a spectrophotometer. Antibody concentrations were calculated from the measured values by using the absorption coefficient determined by PACE method (Protein Science (1995) 4, 2411-2423).

(20-3) Assessment of prepared antibodies for their calcium ion binding

Antibodies purified as described above were assessed for their calcium ion binding by the method described in Reference Example 6. The result is shown in Table 49. The Tm of the Fab domains of multiple antibodies in the Ca library changed depending on calcium ion concentration, suggesting that the library contains molecules that bind to calcium ion.

[Table 49]

| ANTIBODY | SEQ ID NO | | CALCIUM ION CONCENTRATION | | ΔTm (°C) |
|---|---|---|---|---|---|
| | HEAVY CHAIN | LIGHT CHAIN | 3 μM | 2 mM | 2 mM-3 μM |
| Ca_B01 | 89 | 100 | 70.88 | 71.45 | 0.57 |
| Ca_E01 | 90 | 101 | 84.31 | 84.95 | 0.64 |
| Ca_H01 | 91 | 102 | 77.87 | 79.49 | 1.62 |
| Ca_D02 | 92 | 103 | 78.94 | 81.1 | 2.16 |
| Ca_E02 | 93 | 104 | 81.41 | 83.18 | 1.77 |
| Ca_H02 | 94 | 105 | 72.84 | 75.13 | 2.29 |
| Ca_D03 | 95 | 106 | 87.39 | 86.78 | -0.61 |
| Ca_C01 | 96 | 107 | 74.74 | 74.92 | 0.18 |
| Ca_G01 | 97 | 108 | 65.21 | 65.87 | 0.66 |
| Ca_A03 | 98 | 109 | 80.64 | 81.89 | 1.25 |
| Ca_B03 | 99 | 110 | 93.02 | 93.75 | 0.73 |

[Reference Example 21] Isolation of antibodies that bind to IL-6 receptor in a Ca-dependent manner

(21-1) Isolation of antibody fragments, which bind to antigens in a Ca-dependent manner, from library by bead panning

The first selection from the constructed library of antibodies that bind to the IL-6 receptor in a Ca-dependent manner was performed by enriching only antibody fragments having the ability to bind to the antigen (IL-6 receptor).

Phages were produced by *E. coli* containing the constructed phagemids for phage display. To precipitate the phages, 2.5 M NaCl/10% PEG was added to the E. coli culture media of phage production. The precipitated phage population was diluted with TBS to prepare a phage library solution. Then, BSA and $CaCl_2$ were added to the phage library solution to adjust the final BSA concentration to 4% and the final calcium ion concentration to 1.2 mM. Regarding the panning method, the present inventors referred to general panning methods using antigens immobilized onto magnetic beads (J. Immunol. Methods. (2008) 332 (1-2), 2-9; J. Immunol. Methods. (2001) 247 (1-2), 191-203; Biotechnol. Prog. (2002) 18(2) 212-20; Mol. Cell Proteomics (2003) 2 (2), 61-9). The magnetic beads used were NeutrAvidin coated beads (Sera-Mag SpeedBeads NeutrAvidin-coated) or Streptavidin coated beads (Dynabeads M-280 Streptavidin).

Specifically, 250 pmol of biotin-labeled antigen was added to the prepared phage library solution to allow the contact of the phage library solution with the antigen at room temperature for 60 minutes. BSA-blocked magnetic beads were added and allowed to bind to antigen/phage complexes at room temperature for 15 minutes. The beads were washed three times with 1 ml of 1.2 mM $CaCl_2$/TBST (TBST containing 1.2 mM $CaCl_2$) and then twice with 1 ml of 1.2 mM $CaCl_2$/TBS (TBST containing 1.2 mM $CaCl_2$). Then, the beads combined with 0.5 ml of 1mg/ml trypsin were suspended at room temperature for 15 minutes, and immediately followed by separation of beads using a magnetic stand to collect a phage solution. The collected phage solution was added to 10 ml of *E. coli* strain ER2738 in a logarithmic growth phase (OD600 of 0.4-0.7). The *E. coli* was infected with the phages by culturing them while gently stirring at 37°C for one hour. The infected *E. coli* was plated in a 225 mm x 225 mm plate. Then, the phages were collected from the culture medium of the plated *E. coli* to prepare a phage library solution.

In the second-round panning, phages were enriched using the antigen-binding ability or the Ca-dependent binding ability as an indicator.

Specifically, when the enrichment was carried out using the antigen-binding ability as an indicator, 40 pmol of biotin-labeled antigen was added to the prepared phage library solution to allow the contact of the phage library solution with the antigen at room temperature for 60 minutes. BSA-blocked magnetic beads were added and allowed to bind to antigen/phage complexes at room temperature for 15 minutes. The beads were washed three times with 1 ml of 1.2 mM $CaCl_2$/TBST and then twice with 1.2 mM $CaCl_2$/TBS. Then, the beads added with 0.5 ml of 1 mg/ml trypsin were suspended at room temperature for 15 minutes. Then immediately, the beads were separated using a magnetic stand to collect a phage solution. To eliminate the ability from phages displaying on Fab to infect *E. coli,* the pIII protein (helper phage-derived pIII protein) of phages displaying no Fab was cleaved by adding 5 μl of 100 mg/ml trypsin to the collected phage solution. The recovered phage solution was added to 10 mL of the *E. coli* strain ER2738 in a logarithmic growth phase (OD600 of 0.4-0.7). The *E. coli* was cultured with gentle stirring at 37°C for 1 hour to allow the phages to infect the *E. coli.* The infected *E. coli* was inoculated into a 225 mm x 225 mm plate. Subsequently, the phages were recovered from the culture medium of the *E. coli* after inoculation to collect a phage library solution.

When the enrichment was carried out using the Ca-dependent binding ability as an indicator, 40 pmol of biotin-labeled antigen was added to the prepared phage library solution to allow the contact of the phage library solution with the antigen at room temperature for 60 minutes. BSA-blocked magnetic beads were added and allowed to bind to antigen/phage complexes at room temperature for 15 minutes. The beads were washed with 1 ml of 1.2 mM $CaCl_2$/TBST and with 1.2 mM $CaCl_2$/TBS. Then, the beads added with 0.1 ml of 2 mM EDTA/TBS (TBS containing 2 mM EDTA) were suspended at room temperature. Then immediately, the beads were separated using a magnetic stand to collect a phage solution. To eliminate the ability from phages displaying on Fab to infect *E. coli,* the pIII protein (helper phage-derived pIII protein) of phages displaying no Fab was cleaved by adding 5 μl of 100 mg/ml trypsin to the collected phage solution. The recovered phage solution was added to 10 mL of the *E. coli* strain ER2738 in a logarithmic growth phase (OD600 of 0.4-0.7). The *E. coli* was cultured with gentle stirring at 37°C for 1 hour to allow the phages to infect the *E. coli.* The infected *E. coli* was inoculated into a 225 mm x 225 mm plate. Subsequently, the phages were recovered from the culture medium of the *E. coli* after inoculation to collect a phage library solution.

(21-2) Examination by phage ELISA

A phage-containing culture supernatant was collected according to a routine method (Methods Mol. Biol. (2002) 178, 133-145) from a single colony of *E. coli,* obtained as described above.

A culture supernatant containing phages, to which BSA and $CaCl_2$ were added was subjected to ELISA as described below. A StreptaWell 96 microtiter plate (Roche) was coated overnight with 100 μL of PBS containing the biotin-labeled antigen. Each well of said plate was washed with PBST to remove the antigen, and then the wells were

blocked with 250 μL of 4% BSA-TBS for 1 hour or longer. Said plate with the prepared culture supernatant added to each well, from which the 4% BSA-TBS was removed, was allowed to stand undisturbed at 37°C for 1 hour, allowing the binding of phage-presenting antibody to the antigen present in each well. To each well washed with 1.2 mM $CaCl_2$/TBST, 1.2 mM $CaCl_2$/TBS or 1 mM EDTA/TBS was added. The plate was allowed to stand undisturbed for 30 minutes at 37°C for incubation. After washing with 1.2 mM $CaCl_2$/TBST, an HRP-conjugated anti-M13 antibody (Amersham Pharmacia Biotech) diluted with TBS at a concentration of 1.2 mM of ionized calcium concentration was added to each well, and the plate was incubated for 1 hour. After washing with 1.2 mM $CaCl_2$/TBST, the chromogenic reaction of the solution in each well with a TMB single solution (ZYMED) added was stopped by adding sulfuric acid. Subsequently, said color was measured by measuring absorbance at 450 nm.

The base sequences of genes amplified with specific primers were analyzed for the clones subjected to phage ELISA.

The result of phage ELISA and sequence analysis is shown in Table 50.

[Table 50]

| LIBRARY | Ca LIBRARY | Ca LIBRARY |
|---|---|---|
| ENRICHMENT INDEX | ANTIGEN-BINDING ABILITY | DEPENDENT ANTIGEN-BINDING ABILITY |
| NUMBER OF PANNING | 2 | 2 |
| NUMBER OF EXAMINED CLONES | 85 | 86 |
| ELISA-POSITIVE | 77 | 75 |
| TYPES OF ELISA-POSITIVE CLONE SEQUENCES | 74 | 72 |
| TYPES OF Ca-DEPENDENT BINDING CLONE SEQUENCES | 13 | 47 |

(21-3) Expression and purification of antibodies

Clones that are determined to have Ca-dependent antigen binding ability as a result of phage ELISA were inserted into animal cell expression plasmids. Antibodies were expressed by the following method. Cells of human fetal kidney cell-derived FreeStyle 293-F (Invitrogen) were suspended in FreeStyle 293 Expression Medium (Invitrogen), and plated at a cell density of $1.33 \times 10^6$ cells/ml (3 ml) into each well of a 6-well plate. The prepared plasmids were introduced into cells by a lipofection method. The cells were cultured for four days in a $CO_2$ incubator (37°C, 8% $CO_2$, 90 rpm). From the culture supernatants prepared as described above, antibodies were purified using the rProtein A Sepharose™ Fast Flow (Amersham Biosciences) by a method known to those skilled in the art. Absorbance at 280 nm of purified antibody solutions was measured using a spectrophotometer. Antibody concentrations were calculated from the determined values using an extinction coefficient calculated by the PACE method (Protein Science (1995) 4: 2411-2423).

(21-4) Assessment of isolated antibodies for their Ca-dependent binding ability to human IL-6 receptor

Antibodies 6RC1IgG_010 (heavy chain SEQ ID NO: 111; light chain SEQ ID NO: 112), 6RC1IgG_012 (heavy chain SEQ ID NO: 113; light chain SEQ ID NO: 114), and 6RC1IgG_019 (heavy chain, SEQ ID NO: 115; light chain, SEQ ID NO: 116) isolated as described above were assessed for the Ca dependency of their human IL-6 receptor-binding activity by analyzing the interaction between the antibodies and human IL-6 receptor using Biacore T100 (GE Healthcare). Tocilizumab (heavy chain SEQ ID NO: 60; light chain SEQ ID NO: 61) was used as a control antibody that does not have Ca-dependent binding activity to human IL-6 receptor. The interaction was analyzed in solutions at 1.2 mM and 3 μM calcium ion concentration, corresponding to high and low calcium ion concentration conditions, respectively. An appropriate amount of Protein A/G (Invitrogen) was immobilized onto a Sensor chip CM5 (GE Healthcare) by an amino coupling method, and antibodies of interest were captured onto the chip. The two types of running buffers used were: 20 mM ACES/150 mM NaCl/0.05% (w/v) Tween20/1.2 mM $CaCl_2$ (pH 7.4); and 20 mM ACES/150 mM NaCl/0.05% (w/v) Tween20/3 μM $CaCl_2$ (pH 7.4). These buffers were each used to dilute human IL-6 receptor. All measurements were carried out at 37°C.

In the interaction analysis of the antigen-antibody reaction using antibody tocilizumab as a control antibody, and antibodies 6RC1IgG_010, and 6RC1IgG_012, and 6RC1IgG_019, a diluted IL-6 receptor solution and a running buffer as a blank were injected at a flow rate of 5 μl/min for three minutes to allow IL-6 receptor to interact with antibodies tocilizumab, 6RC1IgG_010, and 6RC1IgG_012, and 6RC1IgG_019 captured onto the sensor chip. Then, 10 mM glycine-HCl (pH 1.5) was injected at a flow rate of 30 μl/min for 30 seconds to regenerate the sensor chip.

Sensorgrams at the high calcium ion concentration obtained by the measurement using the above-described method are shown in Fig. 56.

Under the low calcium ion concentration condition, sensorgrams of antibodies tocilizumab, 6RC1IgG_010, and 6RC1IgG_012, and 6RC1IgG_019 were also obtained by the same method. Sensorgrams at the low calcium ion concentration are shown in Fig. 57.

The result described above shows that the IL6 receptor-binding ability of antibodies 6RC1IgG_010, and 6RC1IgG_012, and 6RC1IgG_019 was significantly reduced when the calcium ion concentration in the buffer was shifted from 1.2 mM to 3 μM.

[Reference Example 22] Design of pH-dependent binding antibody library

(22-1) Method for acquiring pH-dependent binding antibodies

WO2009/125825 discloses a pH-dependent antigen-binding antibody whose properties are changed in neutral pH and acidic pH regions by introducing a histidine into an antigen-binding molecule. The disclosed pH-dependent binding antibody is obtained by alteration to substitute a part of the amino acid sequence of the antigen-binding molecule of interest with a histidine. To obtain a pH-dependent binding antibody more efficiently without preliminarily obtaining the antigen-binding molecule of interest to be modified, one method may be obtaining an antigen-binding molecule that binds to a desired antigen from a population of antigen-binding molecules (referred to as His library) with a histidine introduced into the variable region (more preferably, a region potentially involved in antigen binding). It may be possible to efficiently obtain an antigen-binding molecule having desired properties from a His library, because histidine appears more frequently in antigen-binding molecules from His library than those from conventional antibody libraries.

(22-2) Design of a population of antibody molecules (His library) with histidine residue introduced into their variable region to effectively acquire binding antibodies that bind to antigens in a pH-dependent manner

First, positions for introducing a histidine were selected in a His library. WO 2009/125825 discloses generation of pH-dependent antigen-binding antibodies by substituting amino acid residues in the sequences of IL-6 receptor antibody, IL-6 antibody, and IL-31 receptor antibody with a histidine. In addition, an anti-egg white lysozyme antibody (FEBS Letter 11483, 309, 1, 85-88) and anti-hepcidin (WO2009/139822) antibody having a pH-dependent antigen-binding ability were generated by substituting the amino acid sequence of the antigen-binding molecule with histidines. Positions where histidines were introduced in the IL-6 receptor antibody, IL-6 antibody, IL-31 receptor antibody, egg white lysozyme antibody, and hepcidin antibody are shown in Table 51. Positions shown in Table 51 may be listed as candidate positions that can control the antigen-antibody binding. In addition, besides the position shown in Table 51, positions that are likely to have contact with antigen were also considered to be suitable for introduction of histidines.

[Table 51]

| ANTIBODY | CHAIN | POSITION (Kabat NUMBERING) | | | | | | | | |
|---|---|---|---|---|---|---|---|---|---|---|
| IL-6 RECEPTOR ANTIBODY | H | 27 | 31 | 32 | 35 | 50 | 58 | 62 | 100B | 102 |
| | L | 28 | 32 | 53 | 56 | 92 | | | | |
| IL-6 ANTIBODY | H | 32 | 59 | 61 | 99 | | | | | |
| | L | 53 | 54 | 90 | 94 | | | | | |
| IL-31 RECEPTOR ANTIBODY | H | 33 | | | | | | | | |
| | L | | | | | | | | | |
| EGG-WHILE LYSOZYME ANTIBODY | H | 33 | 98 | | | | | | | |
| | L | 54 | | | | | | | | |
| HEPCIDIN ANTIBODY | H | 52 | 56 | 95 | 100c | | | | | |
| | L | 28 | 90 | | | | | | | |

In the His library consisting of heavy-chain and light-chain variable regions, a human antibody sequence was used for the heavy chain variable region, and histidines were introduced into the light chain variable region. The positions listed above and positions that may be involved in antigen binding, *i.e.,* positions 30, 32, 50, 53, 91, 92, and 93 (Kabat numbering, Kabat EA et al. 1991. Sequence of Proteins of Immunological Interest. NIH) in the light chain were selected as positions for introducing histidines in the His library. In addition, the Vk1 sequence was selected as a template sequence of the light chain variable region for introducing histidines. Multiple amino acids were allowed to appear in the template sequence to diversify antigen-binding molecules that constitute the library. Positions exposed on the surface of a variable region that is likely to interact with the antigen were selected as those where multiple amino acids are allowed to appear. Specifically, positions 30, 31, 32, 34, 50, 53, 91, 92, 93, 94, and 96 of the light chain (Kabat numbering, Kabat EA et al. 1991. Sequence of Proteins of Immunological Interest. NIH) were selected as flexible residues.

The type and appearance frequency of amino acid residues that were subsequently allowed to appear were determined. The appearance frequency of amino acids in the flexible residues in the hVk1 and hVk3 sequences registered in the Kabat database (KABAT, E.A. ET AL.: 'Sequences of proteins of immunological interest', vol. 91, 1991, NIH PUBLICATION) was analyzed. Based on the analysis results, the type of amino acids that were allowed to appear in the His library were selected from those with higher appearance frequency at each position. At this time, amino acids whose appearance frequency was determined to be low based on the analysis results were also selected to avoid the bias of amino acid properties. The appearance frequency of the selected amino acids was determined in reference to the analysis results of the Kabat database.

As His libraries, His library 1 which is fixed to necessarily incorporate a single histidine into each CDR, and His library 2 which is more emphasized on sequence diversity than the His library 1 were designed by taking the amino acids and appearance frequency set as described above into consideration. The detailed designs of His libraries 1 and 2 are shown in Tables 3 and 4 (with the positions in each table representing the Kabat numbering). Regarding the frequency of appearance of amino acids described in Tables 3 and 4, Ser (S) at position 94 can be excluded if position 92 represented by the Kabat numbering is Asn (N).

[Reference Example 23] Preparation of a phage display library for human antibodies (His library 1) to obtain an antibody that binds to antigen in a pH-dependent manner

A gene library of antibody heavy-chain variable regions was amplified by PCR using a poly A RNA prepared from human PBMC, and commercial human poly A RNA as a template. A gene library of antibody light-chain variable regions designed as His library 1 as described in Reference Example 22 was amplified using PCR. A combination of the gene libraries of antibody heavy-chain and light-chain variable regions generated as described above was inserted into a phagemid vector to construct a human antibody phage display library which presents Fab domains consisting of human antibody sequences. For the construction method, Methods Mol Biol. (2002) 178, 87-100 was used as a reference. For the construction of the library, a linker region connecting the phagemid Fab to the phage pIII protein, and the sequences of a phage display library with a trypsin cleavage sequence inserted between the N2 and CT domains of the helper phage pIII protein gene were used. Sequences of the antibody gene portions isolated from *E. coli* into which the antibody gene library was introduced were identified, and sequence information was obtained for 132 clones. The designed amino acid distribution and the amino acid distribution of the identified sequences are shown in Fig. 58. A library containing various sequences corresponding to the designed amino acid distribution was constructed.

[Reference Example 24] Isolation of antibodies that bind to IL-6R in a pH-dependent manner

(24-1) Isolation of antibody fragments, which bind to antigens in a ph-dependent manner, from the library by bead panning

The first selection from the constructed His library 1 was performed by enriching only antibody fragments with antigen (IL-6R) binding ability.

Phages were produced by *E. coli* containing the constructed phagemids for phage display. To precipitate the phages, 2.5 M NaCl/10% PEG was added to the E. coli culture media of phage production. The precipitated phage population was diluted with TBS to prepare a phage library solution. BSA and $CaCl_2$ were added to the phage library solution to adjust the final BSA concentration to 4% and the final calcium ion concentration to 1.2 mM. Regarding the panning method, the present inventors referred to general panning methods using antigens immobilized onto magnetic beads (J. Immunol. Methods. (2008) 332 (1-2), 2-9; J. Immunol. Methods. (2001) 247 (1-2), 191-203; Biotechnol. Prog. (2002) 18(2) 212-20, Mol. Cell Proteomics (2003) 2 (2), 61-9). The magnetic beads used were NeutrAvidin coated beads (Sera-Mag SpeedBeads NeutrAvidin-coated) or Streptavidin coated beads (Dynabeads M-280 Streptavidin).

Specifically, 250 pmol of biotin-labeled antigen was added to the prepared phage library solution to allow the contact of the phage library solution with the antigen at room temperature for 60 minutes. BSA-blocked magnetic beads were added and allowed to bind to antigen/phage complexes at room temperature for 15 minutes. The beads were washed three times with 1 ml of 1.2 mM $CaCl_2$/TBST (TBS containing 1.2 mM $CaCl_2$ and 0.1% Tween20) and then twice with 1 ml of 1.2 mM $CaCl_2$/TBS (pH 7.6). Then, the beads added with 0.5 ml of 1mg/ml trypsin were suspended at room temperature for 15 minutes, and then immediately separated using a magnetic stand to collect a phage solution. The collected phage solution was added to 10 ml of *E. coli* strain ER2738 in a logarithmic growth phase (OD600 of 0.4-0.7). The *E. coli* was infected with the phages by culturing them while gently stirring at 37°C for one hour. The infected *E. coli* was plated in a 225 mm x 225 mm plate. Then, the phages were collected from the culture medium of the plated E. coli to prepare a phage library solution.

To enrich the phages, the second and subsequent rounds of panning were performed using the antigen-binding ability or the pH-dependent binding ability as an indicator. Specifically, 40 pmol of the biotin-labeled antigen was added to the prepared phage library solution to allow the contact of the phage library solution with the antigen at room temperature for 60 minutes. BSA-blocked magnetic beads were added and allowed to bind to antigen/phage complexes at room temperature for 15 minutes. The beads were washed multiple times with 1 ml of 1.2 mM $CaCl_2$/TBST and with 1.2 mM $CaCl_2$/TBS. Then, when the phages were enriched using the antigen-binding ability as an indicator, the beads added with 0.5 ml of 1 mg/ml trypsin were suspended at room temperature for 15 minutes, and then immediately separated using a magnetic stand to collect a phage solution. Alternatively, when the phages were enriched using the pH-dependent antigen-binding ability as an indicator, the beads added with 0.1 ml of 50 mM MES/1.2 mM $CaCl_2$ /150 mM NaCl (pH 5.5) were suspended at room temperature, and then immediately separated using a magnetic stand to collect a phage solution. To eliminate the ability from phages displaying no Fab to infect *E. coli,* the pIII protein (helper phage-derived pIII protein) of phages displaying no Fab was cleaved by adding 5 μl of 100 mg/ml trypsin to the collected phage solution. The collected phages were added to 10 ml of *E. coli* strain ER2738 in a logarithmic growth phase (OD600 of 0.4-0.7). The *E. coli* was infected with the phages by culturing them while gently stirring at 37°C for one hour. The infected E. coli was plated in a 225 mm x 225 mm plate. Then, the phages were collected from the culture medium of the plated *E. coli* to collect a phage library solution. The panning using the antigen-binding ability or the pH-dependent binding ability as an indicator was repeated twice.

(24-2) Assessment by phage ELISA

Phage-containing culture supernatants were collected according to a conventional method (Methods Mol. Biol. (2002) 178, 133-145) from single colonies of *E. coli* obtained by the method described above.

To the phage-containing culture supernatants, BSA and $CaCl_2$ were added at a final concentration of 4% BSA and at a final calcium ion concentration of 1.2 mM. These phage-containing culture supernatants were subjected to ELISA by the following procedure. A StreptaWell 96 microtiter plate (Roche) was coated overnight with 100 μl of PBS containing the biotin-labeled antigen. After washing each well of the plate with PBST (PBS containing 0.1% Tween20) to remove the antigen, the wells were blocked with 250 μl of 4% BSA/TBS for one hour or more. After removing 4% BSA/TBS, the prepared culture supernatants were added to each well. The antibodies presented on the phages were allowed to bind to the antigens on each well by incubating the plate at 37°C for one hour. Following wash with 1.2 mM $CaCl_2$/TBST, 1.2 mM $CaCl_2$/TBS (pH 7.6) or 1.2 mM $CaCl_2$/TBS (pH 5.5) was added to each well. The plate was incubated at 37°C for 30 minutes. After washing with 1.2 mM $CaCl_2$/TBST, HRP-coupled anti-M13 antibody (Amersham Pharmacia Biotech) diluted with TBS containing 4% BSA and 1.2 mM ionized calcium was added to each well. The plate was incubated for one hour. After washing with 1.2 mM $CaCl_2$/TBST, TMB single solution (ZYMED) was added to each well. The chromogenic reaction in the solution of each well was stopped by adding sulfuric acid, and then the absorbance at 450 nm was measured to assess the color development.

When enrichment was carried out using the antigen-binding ability as an indicator, phage ELISA following two rounds of panning showed that 17 of 96 clones were ELISA positive in an antigen-specific manner. Thus, clones were analyzed after three rounds of panning. Meanwhile, when enrichment was carried out using the pH-dependent antigen-binding ability as an indicator, phage ELISA following two rounds of panning showed that 70 of 94 clones were positive in ELISA. Thus, clones were analyzed after two rounds of panning.

The base sequences of genes amplified with specific primers were analyzed for the clones subjected to phage ELISA. The results of phage ELISA and sequence analysis are shown in Table 52 below.

[Table 52]

| LIBRARY | His LIBRARY 1 | His LIBRARY 1 |
|---|---|---|
| ENRICHMENT INDEX | ANTIGEN-BINDING ABILITY | pH-DEPENDENT ANTIGEN-BINDING ABILITY |
| NUMBER OF PANNING | 3 | 2 |
| NUMBER OF EXAMINED CLONES | 80 | 94 |

(continued)

| LIBRARY | His LIBRARY 1 | His LIBRARY 1 |
|---|---|---|
| ELISA-POSITIVE | 76 | 70 |
| TYPES OF ELISA-POSITIVE CLONE SEQUENCES | 30 | 67 |
| TYPES OF pH-DEPENDENT BINDING CLONE SEQUENCES | 22 | 47 |

By the same method, antibodies with pH-dependent antigen-binding ability were isolated from the naive human antibody phage display library. When enrichment was carried out using the antigen-binding ability as an indicator, 13 types of pH-dependent binding antibodies were isolated from 88 clones tested. Meanwhile, when enrichment was carried out using the pH-dependent antigen-binding ability as an indicator, 27 types of pH-dependent binding antibodies were isolated from 83 clones tested.

The result described above demonstrated that the variation of clones with pH-dependent antigen-binding ability isolated from the His library 1 was larger as compared to the naive human antibody phage display library.

(24-3) Expression and purification of antibodies

Clones assumed to have pH-dependent binding ability for antigens based on the result of phage ELISA were introduced into animal cell expression plasmids. Antibodies were expressed using the method described below. Cells of human fetal kidney cell-derived FreeStyle 293-F line (Invitrogen) were suspended in FreeStyle 293 Expression Medium (Invitrogen), and plated at a cell density of 1.33 x $10^6$ cells/ml (3 ml) to each well of a 6-well plate. The prepared plasmids were introduced into the cells by a lipofection method. The cells were cultured in a $CO_2$ incubator (37°C, 8%$CO_2$, 90 rpm) for four days. By a method known to those skilled in the art, antibodies were purified using rProtein A SepharoseTM Fast Flow (Amersham Biosciences) from culture supernatants obtained as described above. The absorbance of solutions of purified antibodies was measured at 280 nm using a spectrophotometer. Antibody concentrations were calculated from the measured values by using the absorption coefficient determined by PACE method (Protein Science (1995) 4, 2411-2423).

(24-4) Assessment of isolated antibodies for their pH-dependent binding ability to human IL-6 receptor

Antibodies 6RpH#01 (heavy chain, SEQ ID NO: 117; light chain, SEQ ID NO: 118), and 6RpH#02 (heavy chain, SEQ ID NO: 119; light chain SEQ ID NO: 120), and 6RpH#03 (heavy chain, SEQ ID NO: 121; light chain, SEQ ID NO: 122) isolated as described in (24-3) were assessed for the pH dependency of their human IL-6 receptor-binding activity by analyzing the interaction between the antibodies and human IL-6 receptor using Biacore T100 (GE Healthcare). Tocilizumab (heavy chain SEQ ID NO: 60; light chain SEQ ID NO: 61) was used as a control antibody that does not have pH-dependent binding activity to human IL-6 receptor. The interaction for the antigen-antibody reaction was analyzed in solutions at pH 7.4 and pH 6.0, corresponding to a neutral pH and acidic pH conditions, respectively. An appropriate amount of Protein A/G (Invitrogen) was immobilized onto a Sensor chip CM5 (GE Healthcare) by an amino coupling method, and about 300 RU each of antibodies of interest were captured onto the chip. The two types of running buffers used were: 20 mM ACES/150 mM NaCl/0.05% (w/v) Tween20/1.2 mM $CaCl_2$ (pH 7.4); and 20 mM ACES/150 mM NaCl/0.05% (w/v) Tween20/1.2 mM $CaCl_2$ (pH 6.0). These buffers were each used to dilute human IL-6 receptor. All measurements were carried out at 37°C.

In the interaction analysis of the antigen-antibody reaction using tocilizumab as a control antibody, and antibodies 6RpH#01, and 6RpH#02, and 6RpH#03, a diluted IL-6 receptor solution and a running buffer as a blank were injected at a flow rate of 5 μl/min for three minutes to allow IL-6 receptor to interact with antibodies tocilizumab, 6RpH#01, and 6RpH#02, and 6RpH#03 captured onto the sensor chip. Then, 10 mM glycine-HCl (pH 1.5) was injected at a flow rate of 30 μl/min for 30 seconds to regenerate the sensor chip.

Sensorgrams at pH 7.4 obtained by the measurement using the method described above are shown in Fig. 59. Sensorgrams under the condition of pH 6.0 obtained by the same method are shown in Fig. 60.

The result described above shows that the IL-6 receptor-binding ability of antibodies 6RpH#01, 6RpH#02, and 6RpH#03 was significantly reduced when the buffer pH was shifted from pH 7.4 to pH 6.0.

(Reference Example 25) Method for preparing human FcγR and method for analyzing the interaction between an altered antibody and human FcγR

Extracellular domains of human FcγRs were prepared by the following method. First, a gene of the extracellular domain of FcγR was synthesized by a method well known to those skilled in the art. At that time, the sequence of each FcγR was produced based on the information registered at NCBI. Specifically, FcγRI was produced based on the sequence of NCBI Accession No. NM_000566 (Version No. NM_000566.3), FcγRIIa was produced based on the sequence of NCBI Accession No. NM_001136219 (Version No. NM_001136219.1), FcγRIIb was produced based on the sequence of NCBI Accession No. NM_004001 (Version No. NM_004001.3), FcγRIIIa was produced based on the sequence of NCBI Accession No. NM_001127593 (Version No. NM_001127593.1), and FcγRIIIb was produced based on the sequence of NCBI Accession No. NM_000570 (Version No. NM_000570.3), and a His tag was attached to the C terminus. Furthermore, the presence of polymorphism is known for FcγRIIa, FcγRIIIa, and FcγRIIIb, and the polymorphic sites were produced by referring to Warmerdam et al. (J. Exp. Med., 1990, 172: 19-25) for FcγRIIa; Wu et al. (J. Clin. Invest., 1997, 100 (5): 1059-1070) for FcγRIIIa; and Ory et al. (J. Clin. Invest., 1989, 84, 1688-1691) for FcγRIIIb.

Expression vectors were constructed by inserting into animal cell expression vectors the obtained gene fragments. The constructed expression vectors were transiently introduced into human fetal kidney cancer cell-derived FreeStyle293 cells (Invitrogen) to express the proteins of interest. FcγRIIb for use in crystallographic analysis was prepared such that the sugar chain linked to FcγRIIb is of high mannose type by expressing the protein of interest in the presence of Kifunesine at a final concentration 10 μg/ml. The liquids prepared by filtering through a 0.22-μm filter the culture supernatants obtained from the culture media of the above cells subjected to transient introduction, were purified, in principle, by the following four steps:

the first step: cation-exchange column chromatography (SP Sepharose FF);
the second step: affinity column chromatography for His-tag (HisTrap HP);
the third step: gel filtration column chromatography (Superdex200); and
the fourth step: sterile filtration.

To purify FcγRI, anion-exchange column chromatography with Q sepharose FF was used for the first step. The absorbance of the purified protein was measured at 280 nm using a spectrophotometer. Based on the measured values, the concentrations of purified proteins were calculated using the extinction coefficient determined by a method such as PACE (Protein Science (1995) 4, 2411-2423).

The interaction between each antibody variant and the Fcγ receptor prepared as described above were analyzed using Biacore T100 (GE Healthcare), Biacore T200 (GE Healthcare), Biacore A100, and Biacore 4000. The running buffer used was HBS-EP+ (GE Healthcare), and the measurement temperature was 25°C. The immobilized chip used was: Series S Sensor Chip CM5 (GE Healthcare) or Series S sensor Chip CM4 (GE Healthcare) immobilized with an antigen peptide, ProteinA (Thermo Scientific), Protein A/G (Thermo Scientific), or Protein L (ACTIGEN or BioVision) by an amino coupling method. Alternatively, the immobilized chip used was: Series S Sensor Chip SA (certified) (GE Healthcare) immobilized with a pre-biotinylated antigen peptide by interacting the peptide with the chip.

Antibodies of interest were captured onto these sensor chips, and the Fcγ receptor diluted with the running buffer was allowed to interact with them. The binding amounts to the antibodies were measured, and compared between the antibodies. Since the binding amount of Fcγ receptor depends on the amount of the captured antibody, the comparison was carried out for normalized values obtained by dividing the binding amount of Fcγ receptor by the amount for each antibody captured. Meanwhile, 10 mM glycine-HCl (pH 1.5) was reacted to wash off the captured antibody from the sensor chip, thus, the sensor chip was regenerated for repeated use.

Meanwhile, the KD value of each variant antibody to FcγR was kinetically analyzed by the following method. First, antibodies of interest were captured onto the above sensor chip, and the Fcγ receptor diluted with the running buffer was allowed to interact with them. The measurement results from the obtained sensorgrams were processed by global fitting according to a 1:1 Langmuir binding model using Biacore Evaluation Software. From the values of binding rate constant ka (l/mol/s) and dissociation rate constant kd (1/s), the dissociation constant KD (mol/l) was determined.

When the interaction between each of the altered antibodies and FcγR was weak, and correct analysis was determined to be impossible by the above-mentioned kinetic analysis, the KD for such interactions were calculated using the following 1:1 binding model equation described in the Biacore T100 Software Handbook BR1006-48 Edition AE.

The behavior of interacting molecules according to the 1:1 binding model on Biacore can be described by Equation 4 shown below.

[Equation 4]

$$R_{eq} = C \bullet R_{max} / (KD + C) + RI$$

Req: a plot of steady state binding levels against analyte concentration
C: concentration
RI: bulk refractive index contribution in the sample
Rmax: analyte binding capacity of the surface

When this equation is rearranged, KD can be expressed as Equation 2 shown below.

[Equation 2]

$$KD = C \bullet R_{max} / (R_{eq} - RI) - C$$

KD can be calculated by assigning values to Rmax, RI, and C in this equation. RI and C are determined from the measurement result of a sensorgram and the measurement condition. Rmax was calculated according to the following method. For a compared antibody with sufficiently strong interaction which is simultaneously assessed in the measurement period, the Rmax value obtained by global fitting according to the above-mentioned 1:1 Langmuir binding model was divided by the amount of the compared antibody captured onto the sensor chip, and then this was multiplied by the captured amount of an altered antibody to be assessed. The resulting value was used as Rmax.

[Reference Example 26] Method for preparing mFcγR

The extracellular domain of mouse FcγR was prepared by the following method. First, the genes for the extracellular domains of FcγRs were synthesized by a method known to those skilled in the art. The genes were constructed based on the sequence information for each FcγR registered in NCBI. Specifically, mFcγRI was produced based on NCBI Reference Sequence: NP_034316.1; mFcγRII was produced based on NCBI Reference Sequence: NP_034317.1; mFcγRIII was produced based on NCBI Reference Sequence: NP_034318.2; and mFcγRIV was produced based on NCBI Reference Sequence: NP_653142.2; and a His tag was added to their C termini.

The culture supernatants obtained from culture media of the above cells subjected to the transient introduction were filtered through a 0.22-μm filter. The prepared liquids were purified, in principle, by the following four steps:

the first step: cation-exchange column chromatography (SP Sepharose FF);
the second step: His-tag affinity chromatography (HisTrap HP);
the third step: gel filtration column chromatography (Superdex200); and
the fourth step: sterile filtration.

Meanwhile, when purifying FcγRI, anion-exchange column chromatography with Q Sepharose FF was used in the first step. The absorbance of the purified proteins was measured at 280 nm using a spectrophotometer. Based on the measured values, the concentrations of purified proteins were calculated using the extinction coefficient determined by a method such as PACE (Protein Science (1995) 4, 2411-2423).

Expression vectors were constructed by inserting the obtained gene fragments into animal cell expression vectors. The constructed expression vectors were transiently introduced into human fetal kidney cancer cell-derived FreeStyle293 cells (Invitrogen) to express the proteins of interest. The culture supernatants obtained from culture media of the above cells subjected to the transient introduction were filtered through a 0.22-μm filter.

The prepared liquids were purified, in principle, by the following four steps:

the first step: ion-exchange column chromatography;
the second step: His-tag affinity chromatography (HisTrap HP);
the third step: gel filtration column chromatography (Superdex200); and
the fourth step: sterile filtration.

Meanwhile, for the ion-exchange column chromatography in the first step, when purifying mFcγRI, Q Sepharose HP was used; when purifying mFcγRII and mFcγRIV, SP Sepharose FF was used; and when purifying mFcγRIII, SP Sepharose HP was used. In the third and subsequent steps, D-PBS(-) was used as a solvent, and in the purification of mFcγRIII, the solvent used was D-PBS(-) containing 0.1 M arginine. The absorbance of the purified proteins was measured at 280 nm using a spectrophotometer. Based on the measured values, the concentrations of the purified proteins were calculated using the extinction coefficient determined by a method such as PACE (Protein Science (1995) 4, 2411-2423).

[Reference Example 27] Alteration to suppress the binding to rheumatoid factor

(27-1) Suppression of the binding to rheumatoid factor by the variants Fv4-YTE, Fv4-N434H, and LS

To suppress the binding to rheumatoid factor by the variants Fv4-YTE, Fv4-N434H, and LS, in which the plasma retention has been improved due to improved FcRn binding in an acidic pH range, the alteration Q438R/S440E or S424N was introduced into the variants. Specifically, the novel Fc variants shown in Table 53 were prepated. First, the variants were assessed for their FcRn binding affinity at pH 6.0. The result is shown in Table 53.

[Table 53]

| VARIANT NAME | KD (M) | ALTERATION |
|---|---|---|
| IgG1 | 2.4E-06 | NONE |
| YTE | 2.1E-07 | M252Y/S254T/T256E |
| F1166 | 2.1E-07 | M252Y/S254T/T256E/Q438R/S440E |
| F1167 | 2.5E-07 | M252Y/S254T/T256E/S424N |
| LS | 1.6E-07 | M428L/N434S |
| F1170 | 1.5E-07 | M428L/N434S/Q438R/S440E |
| F1171 | 2.4E-07 | S424N/M428L/N434S |
| N434H | 4.3E-07 | N434H |
| F1172 | 4.0E-07 | N434H/Q438R/S440B |
| F1173 | 5.3E-07 | S424N/N434H |

Then, the variants (Fv4-F1166, F1167, F1172, F1173, F1170, and F1171) were assessed for their binding to rheumatoid factor. The binding assay for rheumatoid factor was carried out at pH 7.4 by an electrochemiluminescene (ECL) method. In this assay, sera (Protogenex) from 15 or 30 rheumatoid patients were used. 50-fold-diluted serum samples were mixed with biotin-labeled test antibodies (1 μg/ml) and SULFO-TAG NHS ester-labeled test antibodies (1 μg/ml). The mixtures were incubated at room temperature for three hours. Then, the mixtures were aliquoted to each well of a streptavidin-coated MULTI-ARRAY 96-well plate (Meso Scale Discovery). This was incubated for two hours at room temperature, each well of the plate was washed, and Read Buffer (x4) was aliquoted thereto. The plate was placed in a SECTOR imager 2400 Reader (Meso Scale Discovery) to measure the chemiluminescence of each well.

The result is shown in Fig. 61. The rheumatoid factor binding of the variants F1166 (Q438R/S440E) and F1167 (S424N) was significantly suppressed as compared to the variant YTE that exhibited strong binding activity to rheumatoid factor in the sera of donors 90216S and 90214S. Regarding the variants F1173 and F1171 comprising the modification S424N, their rheumatoid factor binding, which is caused by the variants N434H and LS, respectively, was significantly suppressed. However, the alteration Q438R/S440E could not completely suppress the rheumatoid factor binding caused by the variants N434H and LS, and the binding to rheumatoid factor in the sera of one or two donors was observed.

(27-2) Suppression of the binding to rheumatoid factor by the LS variant

As shown in Table 54, Fc variants were produced by introducing a novel alteration into Fv4-LS. Of them, the variants (Fv4-F1380, F1384-F1386, F1388, and F1389) that retain the binding to FcRn at pH 6.0 were assessed for their rheumatoid factor binding according to the method described in Example (27-1). The result is shown in Fig. 62. These variants showed significantly suppressed binding to rheumatoid factor in the sera of donors. In particular, the rheumatoid factor binding of Fv4-F1389 comprising Y436T was comparable to that of native IgG1.

[Table 54]

| VARIANT NAME | KD (M) | ALTERATION |
|---|---|---|
| F22 (LS) | 7.1E-08 | M428L/N434S |
| F1380 | 7.3E-08 | S426D/M428L/N434S |
| F1381 | 8.6E-08 | S426E/M428L/N434S |
| F1382 | 1.3E-07 | S426K/M428L/N434S |
| F1383 | 1.6E-07 | S426R/M428L/N434S |
| F1384 | 8.6E-08 | S426A/M428L/N434S |
| F1385 | 7.7E-08 | S426Q/M428L/N434S |
| F1386 | 1.6E-07 | S426Y/M428L/N434S |
| F1387 | 1.5E-07 | M428L/N434S/Y436M |
| F1388 | 8.0E-08 | M428L/N434S/Y436F |
| F1389 | 6.8E-08 | M428L/N434S/Y436T |
| F1390 | 4.0E-07 | M428L/N434S/Y436H |
| F1391 | 4.2E-07 | M428L/N434S/Y436N |
| F1392 | 2.7E-07 | M428L/N434S/Y436K |

As shown above, one can produce antigen-binding molecules that have no rheumatoid factor-binding activity and whose human FcRn-binding activity has been increased under the condition of an acidic pH range, by introducing into the Fc region at the site a modification that reduces the rheumatoid factor-binding activity alone without reducing the FcRn-binding activity under the condition of an acidic pH range.

Such alterations that reduce the rheumatoid factor-binding activity include alterations at positions 248-257, 305-314, 342-352, 380-386, 388, 414-421, 423, 425-437, 439, and 441-444 (EU numbering). Preferably alterations at positions 387, 422, 424, 426, 433, 436, 438, and 440 (EU numbering) are used. Particularly preferably, an alteration by substitution of Glu or Ser for Val at position 422, an alteration by substitution of Arg for Ser at position 424, an alteration by substitution of Asp for His at position 433, an alteration by substitution of Thr for Tyr at position 436, an alteration by substitution of Arg or Lys for Gln at position 438, and an alteration by substitution of Glu or Asp for Ser at position 440 (EU numbering), are used. These alterations may be used alone, or multiple sites may be used in combination.

Alternatively, an N-linked sugar chain addition sequence may be introduced to reduce the rheumatoid factor-binding activity. Specifically, as the N-linked sugar chain addition sequence, Asn-Xxx-Ser/Thr (Xxx represents an arbitrary amino acid other than Pro) is known, and this sequence can be introduced into the Fc region for addition of N-linked sugar chain. The steric hindrance of N-linked sugar chain can inhibit the RF binding. For alterations for addition of N-linked sugar chain, an alteration by substitution of Asn for Lys at position 248, an alteration by substitution of Asn for Ser at position 424, an alteration by substitutions of Asn for Tyr at position 436 and Thr for Gln at position 438, and an alteration by substitution of Asn for Qln at position 438 (EU numbering), are used. Particularly preferably, an alteration by substitution of Asn for Ser at position 424 (EU numbering) is used.

## Industrial Applicability

The present invention provides methods for enhancing the uptake of antigens into cells by antigen-binding molecules, methods for increasing the number of antigens to which a single antigen-binding molecule can bind, and methods for reducing the antigen concentration in plasma by administering them. By enhancing the uptake of antigens into cells by antigen-binding molecules, it is possible to enhance the reduction of the antigen concentration in plasma by administration of antigen-binding molecules, and also improve the pharmacodynamics of antigen-binding molecules, and increase the number of antigens to which a single antigen-binding molecule can bind. Thus, antigen-binding molecules can exhibit superior *in vivo* effects than ordinary antigen-binding molecules.

The present invention relates to the following items:

1. A pharmaceutical composition which comprises an antigen-binding molecule comprising an antigen-binding domain and an Fcγ-receptor-binding domain, wherein the antigen-binding molecule has human-FcRn-binding activity in an acidic pH range condition, and wherein the antigen-binding domain has antigen-binding activity that changes depending on an ion-concentration condition, and the Fcγ-receptor-binding domain has higher binding activity to the Fcγ receptor in a neutral pH range condition than an Fc region of a native human IgG in which the sugar chain bound at position 297 according to EU numbering is a fucose-containing sugar chain.

2. The pharmaceutical composition of item 1, wherein the antigen is a soluble antigen.

3. The pharmaceutical composition of item 1 or 2, wherein the ion concentration is calcium ion concentration.

4. The pharmaceutical composition of item 3, wherein the antigen-binding domain is an antigen-binding domain in which binding activity to the antigen under a high-calcium-ion concentration condition is higher than that under a low-calcium-ion concentration condition.

5. The pharmaceutical composition of item 1 or 2, wherein the ion-concentration condition is a pH condition.

6. The pharmaceutical composition of item 5, wherein the antigen-binding domain is an antigen-binding domain in which binding activity to the antigen in a neutral pH range condition is higher than that in an acidic pH range condition.

7. The pharmaceutical composition of any one of items 1 to 6, wherein the antigen-binding molecule has neutralizing activity against the antigen.

8. The pharmaceutical composition of any one of items 1 to 7, wherein the Fcγ receptor-binding domain comprises an antibody Fc region.

9. The pharmaceutical composition of item 8, wherein the Fc region is an Fc region in which at least one or more amino acids selected from the group consisting of amino acids at positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, and 440 in the Fc region site according to EU numbering are different from amino acids at corresponding sites in a native Fc region.

10. The pharmaceutical composition of item 9, wherein the Fc region is an Fc region which comprises at least one or more amino acids selected from the group consisting of:

either Lys or Tyr at amino acid position 221;
any one of Phe, Trp, Glu, and Tyr at amino acid position 222;
any one of Phe, Trp, Glu, and Lys at amino acid position 223;
any one of Phe, Trp, Glu, and Tyr at amino acid position 224;
any one of Glu, Lys, and Trp at amino acid position 225;
any one of Glu, Gly, Lys, and Tyr at amino acid position 227;
any one of Glu, Gly, Lys, and Tyr at amino acid position 228;
any one of Ala, Glu, Gly, and Tyr at amino acid position 230;
any one of Glu, Gly, Lys, Pro, and Tyr at amino acid position 231;
any one of Glu, Gly, Lys, and Tyr at amino acid position 232;
any one of Ala, Asp, Phe, Gly, His, Ile, Lys, Leu, Met, Asn, Gln, Arg, Ser, Thr, Val, Trp, and Tyr at amino acid position 233;
any one of Ala, Asp, Glu, Phe, Gly, His, Ile, Lys, Met, Asn, Pro, Gln, Arg, Ser, Thr, Val, Trp, and Tyr at amino acid position 234;
any one of Ala, Asp, Glu, Phe, Gly, His, Ile, Lys, Met, Asn, Pro, Gln, Arg, Ser, Thr, Val, Trp, and Tyr at amino acid position 235;
any one of Ala, Asp, Glu, Phe, His, Ile, Lys, Leu, Met, Asn, Pro, Gln, Arg, Ser, Thr, Val, Trp, and Tyr at amino acid position 236;
any one of Asp, Glu, Phe, His, Ile, Lys, Leu, Met, Asn, Pro, Gln, Arg, Ser, Thr, Val, Trp, and Tyr at amino acid position 237;
any one of Asp, Glu, Phe, Gly, His, Ile, Lys, Leu, Met, Asn, Gln, Arg, Ser, Thr, Val, Trp, and Tyr at amino acid position 238;

any one of Asp, Glu, Phe, Gly, His, Ile, Lys, Leu, Met, Asn, Pro, Gln, Arg, Thr, Val, Trp, and Tyr at amino acid position 239;
any one of Ala, Ile, Met, and Thr at amino acid position 240;
any one of Asp, Glu, Leu, Arg, Trp, and Tyr at amino acid position 241;
any one of Leu, Glu, Leu, Gln, Arg, Trp, and Tyr at amino acid position 243;
His at amino acid position 244;
Ala at amino acid position 245;
any one of Asp, Glu, His, and Tyr at amino acid position 246;
any one of Ala, Phe, Gly, His, Ile, Leu, Met, Thr, Val, and Tyr at amino acid position 247;
any one of Glu, His, Gln, and Tyr at amino acid position 249;
either Glu or Gln at amino acid position 250;
Phe at amino acid position 251;
any one of Phe, Met, and Tyr at amino acid position 254;
any one of Glu, Leu, and Tyr at amino acid position 255;
any one of Ala, Met, and Pro at amino acid position 256;
any one of Asp, Glu, His, Ser, and Tyr at amino acid position 258;
any one of Asp, Glu, His, and Tyr at amino acid position 260;
any one of Ala, Glu, Phe, Ile, and Thr at amino acid position 262;
any one of Ala, Ile, Met, and Thr at amino acid position 263;
any one of Asp, Glu, Phe, Gly, His, Ile, Lys, Leu, Met, Asn, Pro, Gln, Arg, Ser, Thr, Trp, and Tyr at amino acid position 264;
any one of Ala, Leu, Phe, Gly, His, Ile, Lys, Leu, Met, Asn, Pro, Gln, Arg, Ser, Thr, Val, Trp, and Tyr at amino acid position 265;
any one of Ala, Ile, Met, and Thr at amino acid position 266;
any one of Asp, Glu, Phe, His, Ile, Lys, Leu, Met, Asn, Pro, Gln, Arg, Thr, Val, Trp, and Tyr at amino acid position 267;
any one of Asp, Glu, Phe, Gly, Ile, Lys, Leu, Met, Pro, Gln, Arg, Thr, Val, and Trp at amino acid position 268;
any one of Phe, Gly, His, Ile, Lys, Leu, Met, Asn, Pro, Arg, Ser, Thr, Val, Trp, and Tyr at amino acid position 269;
any one of Glu, Phe, Gly, His, Ile, Leu, Met, Pro, Gln, Arg, Ser, Thr, Trp, and Tyr at amino acid position 270;
any one of Ala, Asp, Glu, Phe, Gly, His, Ile, Lys, Leu, Met, Asn, Gln, Arg, Ser, Thr, Val, Trp, and Tyr at amino acid position 271;
any one of Asp, Phe, Gly, His, Ile, Lys, Leu, Met, Pro, Arg, Ser, Thr, Val, Trp, and Tyr at amino acid position 272;
either Phe or Ile at amino acid position 273;
any one of Asp, Glu, Phe, Gly, His, Ile, Leu, Met, Asn, Pro, Arg, Ser, Thr, Val, Trp, and Tyr at amino acid position 274;
either Leu or Trp at amino acid position 275;
any one of Asp, Glu, Phe, Gly, His, Ile, Leu, Met, Pro, Arg, Ser, Thr, Val, Trp, and Tyr at amino acid position 276;
any one of Asp, Glu, Gly, His, Ile, Lys, Leu, Met, Asn, Pro, Gln, Arg, Ser, Thr, Val, and Trp at amino acid position 278;
Ala at amino acid position 279;
any one of Ala, Gly, His, Lys, Leu, Pro, Gln, Trp, and Tyr at amino acid position 280;
any one of Asp, Lys, Pro, and Tyr at amino acid position 281;
any one of Glu, Gly, Lys, Pro, and Tyr at amino acid position 282;
any one of Ala, Gly, His, Ile, Lys, Leu, Met, Pro, Arg, and Tyr at amino acid position 283;
any one of Asp, Glu, Leu, Asn, Thr, and Tyr at amino acid position 284;
any one of Asp, Glu, Lys, Gln, Trp, and Tyr at amino acid position 285;
any one of Glu, Gly, Pro, and Tyr at amino acid position 286;
any one of Asn, Asp, Glu, and Tyr at amino acid position 288;
any one of Asp, Gly, His, Leu, Asn, Ser, Thr, Trp, and Tyr at amino acid position 290;
any one of Asp, Glu, Gly, His, Ile, Gln, and Thr at amino acid position 291;
any one of Ala, Asp, Glu, Pro, Thr, and Tyr at amino acid position 292;
any one of Phe, Gly, His, Ile, Leu, Met, Asn, Pro, Arg, Ser, Thr, Val, Trp, and Tyr at amino acid position 293;
any one of Phe, Gly, His, Ile, Lys, Leu, Met, Asn, Pro, Arg, Ser, Thr, Val, Trp, and Tyr at amino acid position 294;
any one of Asp, Glu, Phe, Gly, His, Ile, Lys, Met, Asn, Pro, Arg, Ser, Thr, Val, Trp, and Tyr at amino acid position 295;
any one of Ala, Asp, Glu, Gly, His, Ile, Lys, Leu, Met, Asn, Gln, Arg, Ser, Thr, and Val at amino acid position 296;
any one of Asp, Glu, Phe, Gly, His, Ile, Lys, Leu, Met, Pro, Gln, Arg, Ser, Thr, Val, Trp, and Tyr at amino acid position 297;

any one of Ala, Asp, Glu, Phe, His, Ile, Lys, Met, Asn, Gln, Arg, Thr, Val, Trp, and Tyr at amino acid position 298;
any one of Ala, Asp, Glu, Phe, Gly, His, Ile, Lys, Leu, Met, Asn, Pro, Gln, Arg, Ser, Val, Trp, and Tyr at amino acid position 299;
any one of Ala, Asp, Glu, Gly, His, Ile, Lys, Leu, Met, Asn, Pro, Gln, Arg, Ser, Thr, Val, and Trp at amino acid position 300;
any one of Asp, Glu, His, and Tyr at amino acid position 301;
Ile at amino acid position 302;
any one of Asp, Gly, and Tyr at amino acid position 303;
any one of Asp, His, Leu, Asn, and Thr at amino acid position 304;
any one of Glu, Ile, Thr, and Tyr at amino acid position 305;
any one of Ala, Asp, Asn, Thr, Val, and Tyr at amino acid position 311;
Phe at amino acid position 313;
Leu at amino acid position 315;
either Glu or Gln at amino acid position 317;
any one of His, Leu, Asn, Pro, Gln, Arg, Thr, Val, and Tyr at amino acid position 318;
any one of Asp, Phe, Gly, His, Ile, Leu, Asn, Pro, Ser, Thr, Val, Trp, and Tyr at amino acid position 320;
any one of Ala, Asp, Phe, Gly, His, Ile, Pro, Ser, Thr, Val, Trp, and Tyr at amino acid position 322;
Ile at amino acid position 323;
any one of Asp, Phe, Gly, His, Ile, Leu, Met, Pro, Arg, Thr, Val, Trp, and Tyr at amino acid position 324;
any one of Ala, Asp, Glu, Phe, Gly, His, Ile, Lys, Leu, Met, Pro, Gln, Arg, Ser, Thr, Val, Trp, and Tyr at amino acid position 325;
any one of Ala, Asp, Glu, Gly, Ile, Leu, Met, Asn, Pro, Gln, Ser, Thr, Val, Trp, and Tyr at amino acid position 326;
any one of Ala, Asp, Glu, Phe, Gly, His, Ile, Lys, Leu, Met, Asn, Pro, Arg, Thr, Val, Trp, and Tyr at amino acid position 327;
any one of Ala, Asp, Glu, Phe, Gly, His, Ile, Lys, Met, Asn, Pro, Gln, Arg, Ser, Thr, Val, Trp, and Tyr at amino acid position 328;
any one of Asp, Glu, Phe, Gly, His, Ile, Lys, Leu, Met, Asn, Gln, Arg, Ser, Thr, Val, Trp, and Tyr at amino acid position 329;
any one of Cys, Glu, Phe, Gly, His, Ile, Lys, Leu, Met, Asn, Pro, Arg, Ser, Thr, Val, Trp, and Tyr at amino acid position 330;
any one of Asp, Phe, His, Ile, Leu, Met, Gln, Arg, Thr, Val, Trp, and Tyr at amino acid position 331;
any one of Ala, Asp, Glu, Phe, Gly, His, Lys, Leu, Met, Asn, Pro, Gln, Arg, Ser, Thr, Val, Trp, and Tyr at amino acid position 332;
any one of Ala, Asp, Glu, Phe, Gly, His, Ile, Leu, Met, Pro, Ser, Thr, Val, and Tyr at amino acid position 333;
any one of Ala, Glu, Phe, Ile, Leu, Pro, and Thr at amino acid position 334;
any one of Asp, Phe, Gly, His, Ile, Leu, Met, Asn, Pro, Arg, Ser, Val, Trp, and Tyr at amino acid position 335;
any one of Glu, Lys, and Tyr at amino acid position 336;
any one of Glu, His, and Asn at amino acid position 337;
any one of Asp, Phe, Gly, Ile, Lys, Met, Asn, Gln, Arg, Ser, and Thr at amino acid position 339;
either Ala or Val at amino acid position 376;
either Gly or Lys at amino acid position 377;
Asp at amino acid position 378;
Asn at amino acid position 379;
any one of Ala, Asn, and Ser at amino acid position 380;
either Ala or Ile at amino acid position 382;
Glu at amino acid position 385;
Thr at amino acid position 392;
Leu at amino acid position 396;
Lys at amino acid position 421;
Asn at amino acid position 427;
either Phe or Leu at amino acid position 428;
Met at amino acid position 429;
Trp at amino acid position 434;
Ile at amino acid position 436; and
any one of Gly, His, Ile, Leu, and Tyr at amino acid position 440,
in the Fc region site according to EU numbering.

11. The pharmaceutical composition of any one of items 1 to 10, wherein the Fc region of a native human IgG in which the sugar chain bound at position 297 according to EU numbering is a fucose-containing sugar chain, is an Fc region of any one of native human IgG1, native human IgG2, native human IgG3, and native human IgG4 in which the sugar chain bound at position 297 according to EU numbering is a fucose-containing sugar chain.

12. The pharmaceutical composition of any one of items 1 to 11, wherein the human Fcγ receptor is FcγRIa, FcγRIIa(R), FcγRIIa(H), FcγRIIb, FcγRIIIa(V), or FcγRIIIa(F).

13. The pharmaceutical composition of any one of items 1 to 11, wherein the human Fcγ receptor is FcγRIIb.

14. The pharmaceutical composition of any one of items 8 to 13, wherein the Fc region is an Fc region which comprises at least one or more of

Asp at amino acid position 238, and
Glu at amino acid position 328
in the Fc region site according to EU numbering.

15. A method comprising the step of contacting an antigen-binding molecule with an Fcγ-receptor-expressing cell *in vivo* or *ex vivo,* wherein the antigen-binding molecule has human-FcRn-binding activity in an acidic pH range condition and comprises an antigen-binding domain whose antigen-binding activity changes depending on the ion concentration condition and an Fcγ-receptor-binding domain that has higher binding activity to the Fcγ receptor in a neutral pH range condition compared to an Fc region of a native human IgG in which the sugar chain bound at position 297 according to EU numbering is a fucose-containing sugar chain, which is a method of any one of:

(i) a method for increasing the number of antigens that can bind to a single antigen-binding molecule;
(ii) a method for eliminating plasma antigens;
(iii) a method for improving antigen-binding molecule pharmacokinetics;
(iv) a method for promoting intracellular dissociation of an antigen from an antigen-binding molecule, wherein the antigen has been extracellularly bound to the antigen-binding molecule;
(v) a method for promoting extracellular release of an antigen-binding molecule not bound to an antigen; and
(vi) a method for decreasing a total antigen concentration or free antigen concentration in plasma.

16. The method of item 15, wherein the antigen is a soluble antigen.

17. The method of item 15 or 16, wherein the ion concentration is a calcium ion concentration.

18. The method of item 17, wherein the antigen-binding domain is an antigen-binding domain in which binding activity to the antigen under a high-calcium-ion concentration condition is higher than that under a low-calcium-ion concentration condition.

19. The method of item 15 or 16, wherein the ion concentration condition is a pH condition.

20. The method of item 19, wherein the antigen-binding domain is an antigen-binding domain in which binding activity to the antigen in a neutral pH range condition is higher than that in an acidic pH range condition.

21. The method of any one of items 15 to 20, wherein the antigen-binding molecule has neutralizing activity against the antigen.

22. The method of any one of items 15 to 21, wherein the Fcγ receptor-binding domain comprises an antibody Fc region.

23. The method of item 22, wherein the Fc region is an Fc region in which at least one or more amino acids selected from the group consisting of amino acids at positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, and 440 in the Fc region site according to EU numbering are different from the amino acids at corresponding sites in the native Fc region.

24. The method of item 23, wherein the Fc region is an Fc region which comprises at least one or more amino acids selected from the group consisting of:

either Lys or Tyr at amino acid position 221;
any one of Phe, Trp, Glu, and Tyr at amino acid position 222;
any one of Phe, Trp, Glu, and Lys at amino acid position 223;
any one of Phe, Trp, Glu, and Tyr at amino acid position 224;
any one of Glu, Lys, and Trp at amino acid position 225;
any one of Glu, Gly, Lys, and Tyr at amino acid position 227;
any one of Glu, Gly, Lys, and Tyr at amino acid position 228;
any one of Ala, Glu, Gly, and Tyr at amino acid position 230;
any one of Glu, Gly, Lys, Pro, and Tyr at amino acid position 231;
any one of Glu, Gly, Lys, and Tyr at amino acid position 232;
any one of Ala, Asp, Phe, Gly, His, Ile, Lys, Leu, Met, Asn, Gln, Arg, Ser, Thr, Val, Trp, and Tyr at amino acid position 233;
any one of Ala, Asp, Glu, Phe, Gly, His, Ile, Lys, Met, Asn, Pro, Gln, Arg, Ser, Thr, Val, Trp, and Tyr at amino acid position 234;
any one of Ala, Asp, Glu, Phe, Gly, His, Ile, Lys, Met, Asn, Pro, Gln, Arg, Ser, Thr, Val, Trp, and Tyr at amino acid position 235;
any one of Ala, Asp, Glu, Phe, His, Ile, Lys, Leu, Met, Asn, Pro, Gln, Arg, Ser, Thr, Val, Trp, and Tyr at amino acid position 236;
any one of Asp, Glu, Phe, His, Ile, Lys, Leu, Met, Asn, Pro, Gln, Arg, Ser, Thr, Val, Trp, and Tyr at amino acid position 237;
any one of Asp, Glu, Phe, Gly, His, Ile, Lys, Leu, Met, Asn, Gln, Arg, Ser, Thr, Val, Trp, and Tyr at amino acid position 238;
any one of Asp, Glu, Phe, Gly, His, Ile, Lys, Leu, Met, Asn, Pro, Gln, Arg, Thr, Val, Trp, and Tyr at amino acid position 239;
any one of Ala, Ile, Met, and Thr at amino acid position 240;
any one of Asp, Glu, Leu, Arg, Trp, and Tyr at amino acid position 241;
any one of Leu, Glu, Leu, Gln, Arg, Trp, and Tyr at amino acid position 243;
His at amino acid position 244;
Ala at amino acid position 245;
any one of Asp, Glu, His, and Tyr at amino acid position 246;
any one of Ala, Phe, Gly, His, Ile, Leu, Met, Thr, Val, and Tyr at amino acid position 247;
any one of Glu, His, Gln, and Tyr at amino acid position 249;
either Glu or Gln at amino acid position 250;
Phe at amino acid position 251;
any one of Phe, Met, and Tyr at amino acid position 254;
any one of Glu, Leu, and Tyr at amino acid position 255;
any one of Ala, Met, and Pro at amino acid position 256;
any one of Asp, Glu, His, Ser, and Tyr at amino acid position 258;
any one of Asp, Glu, His, and Tyr at amino acid position 260;
any one of Ala, Glu, Phe, Ile, and Thr at amino acid position 262;
any one of Ala, Ile, Met, and Thr at amino acid position 263;
any one of Asp, Glu, Phe, Gly, His, Ile, Lys, Leu, Met, Asn, Pro, Gln, Arg, Ser, Thr, Trp, and Tyr at amino acid position 264;
any one of Ala, Leu, Phe, Gly, His, Ile, Lys, Leu, Met, Asn, Pro, Gln, Arg, Ser, Thr, Val, Trp, and Tyr at amino acid position 265;
any one of Ala, Ile, Met, and Thr at amino acid position 266;
any one of Asp, Glu, Phe, His, Ile, Lys, Leu, Met, Asn, Pro, Gln, Arg, Thr, Val, Trp, and Tyr at amino acid position 267;
any one of Asp, Glu, Phe, Gly, Ile, Lys, Leu, Met, Pro, Gln, Arg, Thr, Val, and Trp at amino acid position 268;
any one of Phe, Gly, His, Ile, Lys, Leu, Met, Asn, Pro, Arg, Ser, Thr, Val, Trp, and Tyr at amino acid position 269;
any one of Glu, Phe, Gly, His, Ile, Leu, Met, Pro, Gln, Arg, Ser, Thr, Trp, and Tyr at amino acid position 270;
any one of Ala, Asp, Glu, Phe, Gly, His, Ile, Lys, Leu, Met, Asn, Gln, Arg, Ser, Thr, Val, Trp, and Tyr at amino acid position 271;
any one of Asp, Phe, Gly, His, Ile, Lys, Leu, Met, Pro, Arg, Ser, Thr, Val, Trp, and Tyr at amino acid position 272;
either Phe or Ile at amino acid position 273;

any one of Asp, Glu, Phe, Gly, His, Ile, Leu, Met, Asn, Pro, Arg, Ser, Thr, Val, Trp, and Tyr at amino acid position 274;
either Leu or Trp at amino acid position 275;
any one of Asp, Glu, Phe, Gly, His, Ile, Leu, Met, Pro, Arg, Ser, Thr, Val, Trp, and Tyr at amino acid position 276;
any one of Asp, Glu, Gly, His, Ile, Lys, Leu, Met, Asn, Pro, Gln, Arg, Ser, Thr, Val, and Trp at amino acid position 278;
Ala at amino acid position 279;
any one of Ala, Gly, His, Lys, Leu, Pro, Gln, Trp, and Tyr at amino acid position 280;
any one of Asp, Lys, Pro, and Tyr at amino acid position 281;
any one of Glu, Gly, Lys, Pro, and Tyr at amino acid position 282;
any one of Ala, Gly, His, Ile, Lys, Leu, Met, Pro, Arg, and Tyr at amino acid position 283;
any one of Asp, Glu, Leu, Asn, Thr, and Tyr at amino acid position 284;
any one of Asp, Glu, Lys, Gln, Trp, and Tyr at amino acid position 285;
any one of Glu, Gly, Pro, and Tyr at amino acid position 286;
any one of Asn, Asp, Glu, and Tyr at amino acid position 288;
any one of Asp, Gly, His, Leu, Asn, Ser, Thr, Trp, and Tyr at amino acid position 290;
any one of Asp, Glu, Gly, His, Ile, Gln, and Thr at amino acid position 291;
any one of Ala, Asp, Glu, Pro, Thr, and Tyr at amino acid position 292;
any one of Phe, Gly, His, Ile, Leu, Met, Asn, Pro, Arg, Ser, Thr, Val, Trp, and Tyr at amino acid position 293;
any one of Phe, Gly, His, Ile, Lys, Leu, Met, Asn, Pro, Arg, Ser, Thr, Val, Trp, and Tyr at amino acid position 294;
any one of Asp, Glu, Phe, Gly, His, Ile, Lys, Met, Asn, Pro, Arg, Ser, Thr, Val, Trp, and Tyr at amino acid position 295;
any one of Ala, Asp, Glu, Gly, His, Ile, Lys, Leu, Met, Asn, Gln, Arg, Ser, Thr, and Val at amino acid position 296;
any one of Asp, Glu, Phe, Gly, His, Ile, Lys, Leu, Met, Pro, Gln, Arg, Ser, Thr, Val, Trp, and Tyr at amino acid position 297;
any one of Ala, Asp, Glu, Phe, His, Ile, Lys, Met, Asn, Gln, Arg, Thr, Val, Trp, and Tyr at amino acid position 298;
any one of Ala, Asp, Glu, Phe, Gly, His, Ile, Lys, Leu, Met, Asn, Pro, Gln, Arg, Ser, Val, Trp, and Tyr at amino acid position 299;
any one of Ala, Asp, Glu, Gly, His, Ile, Lys, Leu, Met, Asn, Pro, Gln, Arg, Ser, Thr, Val, and Trp at amino acid position 300;
any one of Asp, Glu, His, and Tyr at amino acid position 301;
Ile at amino acid position 302;
any one of Asp, Gly, and Tyr at amino acid position 303;
any one of Asp, His, Leu, Asn, and Thr at amino acid position 304;
any one of Glu, Ile, Thr, and Tyr at amino acid position 305;
any one of Ala, Asp, Asn, Thr, Val, and Tyr at amino acid position 311;
Phe at amino acid position 313;
Leu at amino acid position 315;
either Glu or Gln at amino acid position 317;
any one of His, Leu, Asn, Pro, Gln, Arg, Thr, Val, and Tyr at amino acid position 318;
any one of Asp, Phe, Gly, His, Ile, Leu, Asn, Pro, Ser, Thr, Val, Trp, and Tyr at amino acid position 320;
any one of Ala, Asp, Phe, Gly, His, Ile, Pro, Ser, Thr, Val, Trp, and Tyr at amino acid position 322;
Ile at amino acid position 323;
any one of Asp, Phe, Gly, His, Ile, Leu, Met, Pro, Arg, Thr, Val, Trp, and Tyr at amino acid position 324;
any one of Ala, Asp, Glu, Phe, Gly, His, Ile, Lys, Leu, Met, Pro, Gln, Arg, Ser, Thr, Val, Trp, and Tyr at amino acid position 325;
any one of Ala, Asp, Glu, Gly, Ile, Leu, Met, Asn, Pro, Gln, Ser, Thr, Val, Trp, and Tyr at amino acid position 326;
any one of Ala, Asp, Glu, Phe, Gly, His, Ile, Lys, Leu, Met, Asn, Pro, Arg, Thr, Val, Trp, and Tyr at amino acid position 327;
any one of Ala, Asp, Glu, Phe, Gly, His, Ile, Lys, Met, Asn, Pro, Gln, Arg, Ser, Thr, Val, Trp, and Tyr at amino acid position 328;
any one of Asp, Glu, Phe, Gly, His, Ile, Lys, Leu, Met, Asn, Gln, Arg, Ser, Thr, Val, Trp, and Tyr at amino acid position 329;
any one of Cys, Glu, Phe, Gly, His, Ile, Lys, Leu, Met, Asn, Pro, Arg, Ser, Thr, Val, Trp, and Tyr at amino acid position 330;
any one of Asp, Phe, His, Ile, Leu, Met, Gln, Arg, Thr, Val, Trp, and Tyr at amino acid position 331;
any one of Ala, Asp, Glu, Phe, Gly, His, Lys, Leu, Met, Asn, Pro, Gln, Arg, Ser, Thr, Val, Trp, and Tyr at amino acid position 332;

any one of Ala, Asp, Glu, Phe, Gly, His, Ile, Leu, Met, Pro, Ser, Thr, Val, and Tyr at amino acid position 333;
any one of Ala, Glu, Phe, Ile, Leu, Pro, and Thr at amino acid position 334;
any one of Asp, Phe, Gly, His, Ile, Leu, Met, Asn, Pro, Arg, Ser, Val, Trp, and Tyr at amino acid position 335;
any one of Glu, Lys, and Tyr at amino acid position 336;
any one of Glu, His, and Asn at amino acid position 337;
any one of Asp, Phe, Gly, Ile, Lys, Met, Asn, Gln, Arg, Ser, and Thr at amino acid position 339; either Ala or Val at amino acid position 376;
either Gly or Lys at amino acid position 377;
Asp at amino acid position 378;
Asn at amino acid position 379;
any one of Ala, Asn, and Ser at amino acid position 380;
either Ala or Ile at amino acid position 382;
Glu at amino acid position 385;
Thr at amino acid position 392;
Leu at amino acid position 396;
Lys at amino acid position 421;
Asn at amino acid position 427;
either Phe or Leu at amino acid position 428;
Met at amino acid position 429;
Trp at amino acid position 434;
Ile at amino acid position 436; and
any one of Gly, His, Ile, Leu, and Tyr at amino acid position 440,
in the Fc region site according to EU numbering.

25. The method of any one of items 15 to 24, wherein the Fc region of a native human IgG in which the sugar chain bound at position 297 according to EU numbering is a fucose-containing sugar chain, is an Fc region of any one of native human IgG1, native human IgG2, native human IgG3, and native human IgG4 in which the sugar chain bound at position 297 according to EU numbering is a fucose-containing sugar chain.

26. The method of any one of items 15 to 25, wherein the human Fcγ receptor is FcγRIa, FcγRIIa(R), FcγRIIa(H), FcγRIIb, FcγRIIIa(V), or FcγRIIIa(F).

27. The method of any one of items 15 to 25, wherein the human Fcγ receptor is FcγRIIb.

28. The method of any one of items 22 to 27, wherein the Fc region is an Fc region which comprises at least one or more of

Asp at amino acid position 238, and
Glu at amino acid position 328
in the Fc region site according to EU numbering.

29. A method comprising the step of enhancing Fcγ-receptor-binding activity in a neutral pH range condition of the Fcγ-receptor-binding domain in an antigen-binding molecule compared to that of a native human IgG Fc region in which the sugar chain bound at position 297 according to EU numbering is a fucose-containing sugar chain, wherein the antigen-binding molecule has human-FcRn-binding activity in an acidic pH range condition and comprises an Fcγ receptor-binding domain and an antigen-binding domain whose antigen-binding activity changes depending on the ion concentration condition, which is a method of any one of:

(i) a method for altering an antigen-binding molecule, wherein the intracellular uptake of the antigen to which it binds is enhanced;
(ii) a method for increasing the number of antigens that can bind to a single molecule of antigen-binding molecule;
(iii) a method for increasing the ability of an antigen-binding molecule to eliminate plasma antigens;
(iv) a method for improving antigen-binding molecule pharmacokinetics;
(v) a method for promoting intracellular dissociation of an antigen from an antigen-binding molecule, wherein the antigen has been extracellularly bound to the antigen-binding molecule;
(vi) a method for promoting extracellular release of an antigen-binding molecule not bound to an antigen, wherein the antigen-binding molecule had been taken up into a cell in an antigen-bound form; and
(vii) a method for altering an antigen-binding molecule, which can decrease a total antigen concentration or free antigen concentration in plasma.

30. The method of item 29, wherein the antigen is a soluble antigen.

31. The method of item 29 or 30, wherein the ion concentration is a calcium ion concentration.

32. The method of item 31, wherein the antigen-binding domain is an antigen-binding domain in which binding activity to the antigen under high calcium ion concentration conditions is higher than that under low calcium ion concentration conditions.

33. The method of item 29 or 30, wherein the ion concentration condition is a pH condition.

34. The method of item 33, wherein the antigen-binding domain is an antigen-binding domain in which binding activity to the antigen in a neutral pH range condition is higher than that in an acidic pH range condition.

35. The method of any one of items 29 to 34, wherein the antigen-binding molecule has neutralizing activity against the antigen.

36. The method of any one of items 29 to 35, wherein the Fcγ receptor-binding domain comprises an antibody Fc region.

37. The method of item 36, wherein the Fc region is an Fc region in which at least one or more amino acids selected from the group consisting of amino acids at positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, and 440 in the Fc region site according to EU numbering are different from the amino acids at corresponding sites in the native Fc region.

38. The method of item 37, wherein the Fc region is an Fc region comprising at least one or more amino acids selected from the group consisting of:

either Lys or Tyr at amino acid position 221;
any one of Phe, Trp, Glu, and Tyr at amino acid position 222;
any one of Phe, Trp, Glu, and Lys at amino acid position 223;
any one of Phe, Trp, Glu, and Tyr at amino acid position 224;
any one of Glu, Lys, and Trp at amino acid position 225;
any one of Glu, Gly, Lys, and Tyr at amino acid position 227;
any one of Glu, Gly, Lys, and Tyr at amino acid position 228;
any one of Ala, Glu, Gly, and Tyr at amino acid position 230;
any one of Glu, Gly, Lys, Pro, and Tyr at amino acid position 231;
any one of Glu, Gly, Lys, and Tyr at amino acid position 232;
any one of Ala, Asp, Phe, Gly, His, Ile, Lys, Leu, Met, Asn, Gln, Arg, Ser, Thr, Val, Trp, and Tyr at amino acid position 233;
any one of Ala, Asp, Glu, Phe, Gly, His, Ile, Lys, Met, Asn, Pro, Gln, Arg, Ser, Thr, Val, Trp, and Tyr at amino acid position 234;
any one of Ala, Asp, Glu, Phe, Gly, His, Ile, Lys, Met, Asn, Pro, Gln, Arg, Ser, Thr, Val, Trp, and Tyr at amino acid position 235;
any one of Ala, Asp, Glu, Phe, His, Ile, Lys, Leu, Met, Asn, Pro, Gln, Arg, Ser, Thr, Val, Trp, and Tyr at amino acid position 236;
any one of Asp, Glu, Phe, His, Ile, Lys, Leu, Met, Asn, Pro, Gln, Arg, Ser, Thr, Val, Trp, and Tyr at amino acid position 237;
any one of Asp, Glu, Phe, Gly, His, Ile, Lys, Leu, Met, Asn, Gln, Arg, Ser, Thr, Val, Trp, and Tyr at amino acid position 238;
any one of Asp, Glu, Phe, Gly, His, Ile, Lys, Leu, Met, Asn, Pro, Gln, Arg, Thr, Val, Trp, and Tyr at amino acid position 239;
any one of Ala, Ile, Met, and Thr at amino acid position 240;

any one of Asp, Glu, Leu, Arg, Trp, and Tyr at amino acid position 241;
any one of Leu, Glu, Leu, Gln, Arg, Trp, and Tyr at amino acid position 243;
His at amino acid position 244;
Ala at amino acid position 245;
any one of Asp, Glu, His, and Tyr at amino acid position 246;
any one of Ala, Phe, Gly, His, Ile, Leu, Met, Thr, Val, and Tyr at amino acid position 247;
any one of Glu, His, Gln, and Tyr at amino acid position 249;
either Glu or Gln at amino acid position 250;
Phe at amino acid position 251;
any one of Phe, Met, and Tyr at amino acid position 254;
any one of Glu, Leu, and Tyr at amino acid position 255;
any one of Ala, Met, and Pro at amino acid position 256;
any one of Asp, Glu, His, Ser, and Tyr at amino acid position 258;
any one of Asp, Glu, His, and Tyr at amino acid position 260;
any one of Ala, Glu, Phe, Ile, and Thr at amino acid position 262;
any one of Ala, Ile, Met, and Thr at amino acid position 263;
any one of Asp, Glu, Phe, Gly, His, Ile, Lys, Leu, Met, Asn, Pro, Gln, Arg, Ser, Thr, Trp, and Tyr at amino acid position 264;
any one of Ala, Leu, Phe, Gly, His, Ile, Lys, Leu, Met, Asn, Pro, Gln, Arg, Ser, Thr, Val, Trp, and Tyr at amino acid position 265;
any one of Ala, Ile, Met, and Thr at amino acid position 266;
any one of Asp, Glu, Phe, His, Ile, Lys, Leu, Met, Asn, Pro, Gln, Arg, Thr, Val, Trp, and Tyr at amino acid position 267;
any one of Asp, Glu, Phe, Gly, Ile, Lys, Leu, Met, Pro, Gln, Arg, Thr, Val, and Trp at amino acid position 268;
any one of Phe, Gly, His, Ile, Lys, Leu, Met, Asn, Pro, Arg, Ser, Thr, Val, Trp, and Tyr at amino acid position 269;
any one of Glu, Phe, Gly, His, Ile, Leu, Met, Pro, Gln, Arg, Ser, Thr, Trp, and Tyr at amino acid position 270;
any one of Ala, Asp, Glu, Phe, Gly, His, Ile, Lys, Leu, Met, Asn, Gln, Arg, Ser, Thr, Val, Trp, and Tyr at amino acid position 271;
any one of Asp, Phe, Gly, His, Ile, Lys, Leu, Met, Pro, Arg, Ser, Thr, Val, Trp, and Tyr at amino acid position 272;
either Phe or Ile at amino acid position 273;
any one of Asp, Glu, Phe, Gly, His, Ile, Leu, Met, Asn, Pro, Arg, Ser, Thr, Val, Trp, and Tyr at amino acid position 274;
either Leu or Trp at amino acid position 275;
any one of Asp, Glu, Phe, Gly, His, Ile, Leu, Met, Pro, Arg, Ser, Thr, Val, Trp, and Tyr at amino acid position 276;
any one of Asp, Glu, Gly, His, Ile, Lys, Leu, Met, Asn, Pro, Gln, Arg, Ser, Thr, Val, and Trp at amino acid position 278;
Ala at amino acid position 279;
any one of Ala, Gly, His, Lys, Leu, Pro, Gln, Trp, and Tyr at amino acid position 280;
any one of Asp, Lys, Pro, and Tyr at amino acid position 281;
any one of Glu, Gly, Lys, Pro, and Tyr at amino acid position 282;
any one of Ala, Gly, His, Ile, Lys, Leu, Met, Pro, Arg, and Tyr at amino acid position 283;
any one of Asp, Glu, Leu, Asn, Thr, and Tyr at amino acid position 284;
any one of Asp, Glu, Lys, Gln, Trp, and Tyr at amino acid position 285;
any one of Glu, Gly, Pro, and Tyr at amino acid position 286;
any one of Asn, Asp, Glu, and Tyr at amino acid position 288;
any one of Asp, Gly, His, Leu, Asn, Ser, Thr, Trp, and Tyr at amino acid position 290;
any one of Asp, Glu, Gly, His, Ile, Gln, and Thr at amino acid position 291;
any one of Ala, Asp, Glu, Pro, Thr, and Tyr at amino acid position 292;
any one of Phe, Gly, His, Ile, Leu, Met, Asn, Pro, Arg, Ser, Thr, Val, Trp, and Tyr at amino acid position 293;
any one of Phe, Gly, His, Ile, Lys, Leu, Met, Asn, Pro, Arg, Ser, Thr, Val, Trp, and Tyr at amino acid position 294;
any one of Asp, Glu, Phe, Gly, His, Ile, Lys, Met, Asn, Pro, Arg, Ser, Thr, Val, Trp, and Tyr at amino acid position 295;
any one of Ala, Asp, Glu, Gly, His, Ile, Lys, Leu, Met, Asn, Gln, Arg, Ser, Thr, and Val at amino acid position 296;
any one of Asp, Glu, Phe, Gly, His, Ile, Lys, Leu, Met, Pro, Gln, Arg, Ser, Thr, Val, Trp, and Tyr at amino acid position 297;
any one of Ala, Asp, Glu, Phe, His, Ile, Lys, Met, Asn, Gln, Arg, Thr, Val, Trp, and Tyr at amino acid position 298;
any one of Ala, Asp, Glu, Phe, Gly, His, Ile, Lys, Leu, Met, Asn, Pro, Gln, Arg, Ser, Val, Trp, and Tyr at amino acid position 299;

any one of Ala, Asp, Glu, Gly, His, Ile, Lys, Leu, Met, Asn, Pro, Gln, Arg, Ser, Thr, Val, and Trp at amino acid position 300;
any one of Asp, Glu, His, and Tyr at amino acid position 301;
Ile at amino acid position 302;
any one of Asp, Gly, and Tyr at amino acid position 303;
any one of Asp, His, Leu, Asn, and Thr at amino acid position 304;
any one of Glu, Ile, Thr, and Tyr at amino acid position 305;
any one of Ala, Asp, Asn, Thr, Val, and Tyr at amino acid position 311;
Phe at amino acid position 313;
Leu at amino acid position 315;
either Glu or Gln at amino acid position 317;
any one of His, Leu, Asn, Pro, Gln, Arg, Thr, Val, and Tyr at amino acid position 318;
any one of Asp, Phe, Gly, His, Ile, Leu, Asn, Pro, Ser, Thr, Val, Trp, and Tyr at amino acid position 320;
any one of Ala, Asp, Phe, Gly, His, Ile, Pro, Ser, Thr, Val, Trp, and Tyr at amino acid position 322;
Ile at amino acid position 323;
any one of Asp, Phe, Gly, His, Ile, Leu, Met, Pro, Arg, Thr, Val, Trp, and Tyr at amino acid position 324;
any one of Ala, Asp, Glu, Phe, Gly, His, Ile, Lys, Leu, Met, Pro, Gln, Arg, Ser, Thr, Val, Trp, and Tyr at amino acid position 325;
any one of Ala, Asp, Glu, Gly, Ile, Leu, Met, Asn, Pro, Gln, Ser, Thr, Val, Trp, and Tyr at amino acid position 326;
any one of Ala, Asp, Glu, Phe, Gly, His, Ile, Lys, Leu, Met, Asn, Pro, Arg, Thr, Val, Trp, and Tyr at amino acid position 327;
any one of Ala, Asp, Glu, Phe, Gly, His, Ile, Lys, Met, Asn, Pro, Gln, Arg, Ser, Thr, Val, Trp, and Tyr at amino acid position 328;
any one of Asp, Glu, Phe, Gly, His, Ile, Lys, Leu, Met, Asn, Gln, Arg, Ser, Thr, Val, Trp, and Tyr at amino acid position 329;
any one of Cys, Glu, Phe, Gly, His, Ile, Lys, Leu, Met, Asn, Pro, Arg, Ser, Thr, Val, Trp, and Tyr at amino acid position 330;
any one of Asp, Phe, His, Ile, Leu, Met, Gln, Arg, Thr, Val, Trp, and Tyr at amino acid position 331;
any one of Ala, Asp, Glu, Phe, Gly, His, Lys, Leu, Met, Asn, Pro, Gln, Arg, Ser, Thr, Val, Trp, and Tyr at amino acid position 332;
any one of Ala, Asp, Glu, Phe, Gly, His, Ile, Leu, Met, Pro, Ser, Thr, Val, and Tyr at amino acid position 333;
any one of Ala, Glu, Phe, Ile, Leu, Pro, and Thr at amino acid position 334;
any one of Asp, Phe, Gly, His, Ile, Leu, Met, Asn, Pro, Arg, Ser, Val, Trp, and Tyr at amino acid position 335;
any one of Glu, Lys, and Tyr at amino acid position 336;
any one of Glu, His, and Asn at amino acid position 337;
any one of Asp, Phe, Gly, Ile, Lys, Met, Asn, Gln, Arg, Ser, and Thr at amino acid position 339; either Ala or Val at amino acid position 376;
either Gly or Lys at amino acid position 377;
Asp at amino acid position 378;
Asn at amino acid position 379;
any one of Ala, Asn, and Ser at amino acid position 380;
either Ala or Ile at amino acid position 382;
Glu at amino acid position 385;
Thr at amino acid position 392;
Leu at amino acid position 396;
Lys at amino acid position 421;
Asn at amino acid position 427;
either Phe or Leu at amino acid position 428;
Met at amino acid position 429;
Trp at amino acid position 434;
Ile at amino acid position 436; and
any one of Gly, His, Ile, Leu, and Tyr at amino acid position 440,
in the Fc region site according to EU numbering.

39. The method of any one of items 29 to 38, wherein the Fc region of a native human IgG in which the sugar chain bound at position 297 according to EU numbering is a fucose-containing sugar chain, is an Fc region of any one of native human IgG1, native human IgG2, native human IgG3, and native human IgG4 in which the sugar chain bound at position 297 according to EU numbering is a fucose-containing sugar chain.

40. The method of any one of items 29 to 39, wherein the human Fcγ receptor is FcyRIa, FcγRIIa(R), FcγRIIa(H), FcyRIIb, FcγRIIIa(V), or FcγRIIIa(F).

41. The method of any one of items 29 to 39, wherein the human Fcγ receptor is FcyRIIb.

42. The method of any one of items 36 to 41, wherein the Fc region is an Fc region which comprises at least one or more of:

Asp at amino acid position 238, and
Glu at amino acid position 328
in the Fc region site according to EU numbering.

43. A method for producing an antigen-binding molecule, which comprises the steps of:

(a) determining the antigen-binding activity of an antigen-binding domain under a high-calcium-ion concentration condition;
(b) determining the antigen-binding activity of an antigen-binding domain under a low-calcium-ion concentration condition;
(c) selecting the antigen-binding domain for which the antigen-binding activity determined in (a) is higher than the antigen-binding activity determined in (b);
(d) linking a polynucleotide encoding the antigen-binding domain selected in (c) to a polynucleotide encoding an Fcγ receptor-binding domain having human-FcRn-binding activity in an acidic pH range condition and in which binding activity to the Fcγ receptor in a neutral pH range condition is higher than that of an Fc region of a native human IgG in which the sugar chain bound at position 297 according to EU numbering is a fucose-containing sugar chain;
(e) culturing cells introduced with a vector in which the polynucleotide obtained in (d) is operably linked; and
(f) collecting antigen-binding molecules from the cell culture of (e).

44. A method for producing an antigen-binding molecule, which comprises the steps of:

(a) determining the antigen-binding activity of an antibody under a high-calcium-ion concentration condition;
(b) determining the antigen-binding activity of an antibody under a low-calcium-ion concentration condition;
(c) selecting the antibody for which the antigen-binding activity determined in (a) is higher than the antigen-binding activity determined in (b);
(d) linking a polynucleotide encoding the antigen-binding domain of the antibody selected in (c) to a polynucleotide encoding an Fcγ receptor-binding domain having human-FcRn-binding activity in an acidic pH range, and in which binding activity to the Fcγ receptor in a neutral pH range condition is higher than that of an Fc region of a native human IgG in which the sugar chain bound at position 297 according to EU numbering is a fucose-containing sugar chain;
(e) culturing cells introduced with a vector in which the polynucleotide obtained in (d) is operably linked; and
(f) collecting antigen-binding molecules from the cell culture of (e).

45. A method for producing an antigen-binding molecule, which comprises the steps of:

(a) determining the antigen-binding activity of an antigen-binding domain in a neutral pH range condition;
(b) determining the antigen-binding activity of an antigen-binding domain in an acidic pH range condition;
(c) selecting the antigen-binding domain for which the antigen-binding activity determined in (a) is higher than the antigen-binding activity determined in (b);
(d) linking a polynucleotide encoding the antigen-binding domain selected in (c) to a polynucleotide encoding an Fcγ receptor-binding domain having human-FcRn-binding activity in an acidic pH range condition, and in which binding activity to the Fcγ receptor in a neutral pH range condition is higher than that of an Fc region of a native human IgG in which the sugar chain bound at position 297 according to EU numbering is a fucose-containing sugar chain;
(e) culturing cells introduced with a vector in which the polynucleotide obtained in (d) is operably linked; and
(f) collecting antigen-binding molecules from the cell culture of (e).

46. A method for producing an antigen-binding molecule, which comprises the steps of:

(a) determining the antigen-binding activity of an antibody in a neutral pH range condition;
(b) determining the antigen-binding activity of an antibody in an acidic pH range condition;
(c) selecting the antibody for which the antigen-binding activity determined in (a) is higher than the antigen-binding activity determined in (b);
(d) linking a polynucleotide encoding the antigen-binding domain of the antibody selected in (c) to a polynucleotide encoding an Fcγ receptor-binding domain having human-FcRn-binding activity in an acidic pH range condition, in which binding activity to the Fcγ receptor in a neutral pH range condition is higher than that of an Fc region of a native human IgG in which the sugar chain bound at position 297 according to EU numbering is a fucose-containing sugar chain;
(e) culturing cells introduced with a vector in which the polynucleotide obtained in (d) is operably linked; and
(f) collecting antigen-binding molecules from the cell culture of (e).

47. The production method of any one of items 43 to 46, wherein the antigen is a soluble antigen.

48. The production method of any one of items 43 to 47, wherein the Fcγ receptor-binding domain comprises an antibody Fc region.

49. The production method of item 48, wherein the Fc region is an Fc region in which at least one or more amino acids selected from the group consisting of amino acids at positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, and 440 in the Fc region site according to EU numbering are different from the amino acids at corresponding sites in the native Fc region.

50. The production method of item 49, wherein the Fc region comprises at least one or more amino acids selected from the group consisting of:

either Lys or Tyr at amino acid position 221;
any one of Phe, Trp, Glu, and Tyr at amino acid position 222;
any one of Phe, Trp, Glu, and Lys at amino acid position 223;
any one of Phe, Trp, Glu, and Tyr at amino acid position 224;
any one of Glu, Lys, and Trp at amino acid position 225;
any one of Glu, Gly, Lys, and Tyr at amino acid position 227;
any one of Glu, Gly, Lys, and Tyr at amino acid position 228;
any one of Ala, Glu, Gly, and Tyr at amino acid position 230;
any one of Glu, Gly, Lys, Pro, and Tyr at amino acid position 231;
any one of Glu, Gly, Lys, and Tyr at amino acid position 232;
any one of Ala, Asp, Phe, Gly, His, Ile, Lys, Leu, Met, Asn, Gln, Arg, Ser, Thr, Val, Trp, and Tyr at amino acid position 233;
any one of Ala, Asp, Glu, Phe, Gly, His, Ile, Lys, Met, Asn, Pro, Gln, Arg, Ser, Thr, Val, Trp, and Tyr at amino acid position 234;
any one of Ala, Asp, Glu, Phe, Gly, His, Ile, Lys, Met, Asn, Pro, Gln, Arg, Ser, Thr, Val, Trp, and Tyr at amino acid position 235;
any one of Ala, Asp, Glu, Phe, His, Ile, Lys, Leu, Met, Asn, Pro, Gln, Arg, Ser, Thr, Val, Trp, and Tyr at amino acid position 236;
any one of Asp, Glu, Phe, His, Ile, Lys, Leu, Met, Asn, Pro, Gln, Arg, Ser, Thr, Val, Trp, and Tyr at amino acid position 237;
any one of Asp, Glu, Phe, Gly, His, Ile, Lys, Leu, Met, Asn, Gln, Arg, Ser, Thr, Val, Trp, and Tyr at amino acid position 238;
any one of Asp, Glu, Phe, Gly, His, Ile, Lys, Leu, Met, Asn, Pro, Gln, Arg, Thr, Val, Trp, and Tyr at amino acid position 239;
any one of Ala, Ile, Met, and Thr at amino acid position 240;
any one of Asp, Glu, Leu, Arg, Trp, and Tyr at amino acid position 241;
any one of Leu, Glu, Leu, Gln, Arg, Trp, and Tyr at amino acid position 243;
His at amino acid position 244;
Ala at amino acid position 245;

any one of Asp, Glu, His, and Tyr at amino acid position 246;
any one of Ala, Phe, Gly, His, Ile, Leu, Met, Thr, Val, and Tyr at amino acid position 247;
any one of Glu, His, Gln, and Tyr at amino acid position 249;
either Glu or Gln at amino acid position 250;
Phe at amino acid position 251;
any one of Phe, Met, and Tyr at amino acid position 254;
any one of Glu, Leu, and Tyr at amino acid position 255;
any one of Ala, Met, and Pro at amino acid position 256;
any one of Asp, Glu, His, Ser, and Tyr at amino acid position 258;
any one of Asp, Glu, His, and Tyr at amino acid position 260;
any one of Ala, Glu, Phe, Ile, and Thr at amino acid position 262;
any one of Ala, Ile, Met, and Thr at amino acid position 263;
any one of Asp, Glu, Phe, Gly, His, Ile, Lys, Leu, Met, Asn, Pro, Gln, Arg, Ser, Thr, Trp, and Tyr at amino acid position 264;
any one of Ala, Leu, Phe, Gly, His, Ile, Lys, Leu, Met, Asn, Pro, Gln, Arg, Ser, Thr, Val, Trp, and Tyr at amino acid position 265;
any one of Ala, Ile, Met, and Thr at amino acid position 266;
any one of Asp, Glu, Phe, His, Ile, Lys, Leu, Met, Asn, Pro, Gln, Arg, Thr, Val, Trp, and Tyr at amino acid position 267;
any one of Asp, Glu, Phe, Gly, Ile, Lys, Leu, Met, Pro, Gln, Arg, Thr, Val, and Trp at amino acid position 268;
any one of Phe, Gly, His, Ile, Lys, Leu, Met, Asn, Pro, Arg, Ser, Thr, Val, Trp, and Tyr at amino acid position 269;
any one of Glu, Phe, Gly, His, Ile, Leu, Met, Pro, Gln, Arg, Ser, Thr, Trp, and Tyr at amino acid position 270;
any one of Ala, Asp, Glu, Phe, Gly, His, Ile, Lys, Leu, Met, Asn, Gln, Arg, Ser, Thr, Val, Trp, and Tyr at amino acid position 271;
any one of Asp, Phe, Gly, His, Ile, Lys, Leu, Met, Pro, Arg, Ser, Thr, Val, Trp, and Tyr at amino acid position 272;
either Phe or Ile at amino acid position 273;
any one of Asp, Glu, Phe, Gly, His, Ile, Leu, Met, Asn, Pro, Arg, Ser, Thr, Val, Trp, and Tyr at amino acid position 274;
either Leu or Trp at amino acid position 275;
any one of Asp, Glu, Phe, Gly, His, Ile, Leu, Met, Pro, Arg, Ser, Thr, Val, Trp, and Tyr at amino acid position 276;
any one of Asp, Glu, Gly, His, Ile, Lys, Leu, Met, Asn, Pro, Gln, Arg, Ser, Thr, Val, and Trp at amino acid position 278;
Ala at amino acid position 279;
any one of Ala, Gly, His, Lys, Leu, Pro, Gln, Trp, and Tyr at amino acid position 280;
any one of Asp, Lys, Pro, and Tyr at amino acid position 281;
any one of Glu, Gly, Lys, Pro, and Tyr at amino acid position 282;
any one of Ala, Gly, His, Ile, Lys, Leu, Met, Pro, Arg, and Tyr at amino acid position 283;
any one of Asp, Glu, Leu, Asn, Thr, and Tyr at amino acid position 284;
any one of Asp, Glu, Lys, Gln, Trp, and Tyr at amino acid position 285;
any one of Glu, Gly, Pro, and Tyr at amino acid position 286;
any one of Asn, Asp, Glu, and Tyr at amino acid position 288;
any one of Asp, Gly, His, Leu, Asn, Ser, Thr, Trp, and Tyr at amino acid position 290;
any one of Asp, Glu, Gly, His, Ile, Gln, and Thr at amino acid position 291;
any one of Ala, Asp, Glu, Pro, Thr, and Tyr at amino acid position 292;
any one of Phe, Gly, His, Ile, Leu, Met, Asn, Pro, Arg, Ser, Thr, Val, Trp, and Tyr at amino acid position 293;
any one of Phe, Gly, His, Ile, Lys, Leu, Met, Asn, Pro, Arg, Ser, Thr, Val, Trp, and Tyr at amino acid position 294;
any one of Asp, Glu, Phe, Gly, His, Ile, Lys, Met, Asn, Pro, Arg, Ser, Thr, Val, Trp, and Tyr at amino acid position 295;
any one of Ala, Asp, Glu, Gly, His, Ile, Lys, Leu, Met, Asn, Gln, Arg, Ser, Thr, and Val at amino acid position 296;
any one of Asp, Glu, Phe, Gly, His, Ile, Lys, Leu, Met, Pro, Gln, Arg, Ser, Thr, Val, Trp, and Tyr at amino acid position 297;
any one of Ala, Asp, Glu, Phe, His, Ile, Lys, Met, Asn, Gln, Arg, Thr, Val, Trp, and Tyr at amino acid position 298;
any one of Ala, Asp, Glu, Phe, Gly, His, Ile, Lys, Leu, Met, Asn, Pro, Gln, Arg, Ser, Val, Trp, and Tyr at amino acid position 299;
any one of Ala, Asp, Glu, Gly, His, Ile, Lys, Leu, Met, Asn, Pro, Gln, Arg, Ser, Thr, Val, and Trp at amino acid position 300;
any one of Asp, Glu, His, and Tyr at amino acid position 301;
Ile at amino acid position 302;

any one of Asp, Gly, and Tyr at amino acid position 303;
any one of Asp, His, Leu, Asn, and Thr at amino acid position 304;
any one of Glu, Ile, Thr, and Tyr at amino acid position 305;
any one of Ala, Asp, Asn, Thr, Val, and Tyr at amino acid position 311;
Phe at amino acid position 313;
Leu at amino acid position 315;
either Glu or Gln at amino acid position 317;
any one of His, Leu, Asn, Pro, Gln, Arg, Thr, Val, and Tyr at amino acid position 318;
any one of Asp, Phe, Gly, His, Ile, Leu, Asn, Pro, Ser, Thr, Val, Trp, and Tyr at amino acid position 320;
any one of Ala, Asp, Phe, Gly, His, Ile, Pro, Ser, Thr, Val, Trp, and Tyr at amino acid position 322;
Ile at amino acid position 323;
any one of Asp, Phe, Gly, His, Ile, Leu, Met, Pro, Arg, Thr, Val, Trp, and Tyr at amino acid position 324;
any one of Ala, Asp, Glu, Phe, Gly, His, Ile, Lys, Leu, Met, Pro, Gln, Arg, Ser, Thr, Val, Trp, and Tyr at amino acid position 325;
any one of Ala, Asp, Glu, Gly, Ile, Leu, Met, Asn, Pro, Gln, Ser, Thr, Val, Trp, and Tyr at amino acid position 326;
any one of Ala, Asp, Glu, Phe, Gly, His, Ile, Lys, Leu, Met, Asn, Pro, Arg, Thr, Val, Trp, and Tyr at amino acid position 327;
any one of Ala, Asp, Glu, Phe, Gly, His, Ile, Lys, Met, Asn, Pro, Gln, Arg, Ser, Thr, Val, Trp, and Tyr at amino acid position 328;
any one of Asp, Glu, Phe, Gly, His, Ile, Lys, Leu, Met, Asn, Gln, Arg, Ser, Thr, Val, Trp, and Tyr at amino acid position 329;
any one of Cys, Glu, Phe, Gly, His, Ile, Lys, Leu, Met, Asn, Pro, Arg, Ser, Thr, Val, Trp, and Tyr at amino acid position 330;
any one of Asp, Phe, His, Ile, Leu, Met, Gln, Arg, Thr, Val, Trp, and Tyr at amino acid position 331;
any one of Ala, Asp, Glu, Phe, Gly, His, Lys, Leu, Met, Asn, Pro, Gln, Arg, Ser, Thr, Val, Trp, and Tyr at amino acid position 332;
any one of Ala, Asp, Glu, Phe, Gly, His, Ile, Leu, Met, Pro, Ser, Thr, Val, and Tyr at amino acid position 333;
any one of Ala, Glu, Phe, Ile, Leu, Pro, and Thr at amino acid position 334;
any one of Asp, Phe, Gly, His, Ile, Leu, Met, Asn, Pro, Arg, Ser, Val, Trp, and Tyr at amino acid position 335;
any one of Glu, Lys, and Tyr at amino acid position 336;
any one of Glu, His, and Asn at amino acid position 337;
any one of Asp, Phe, Gly, Ile, Lys, Met, Asn, Gln, Arg, Ser, and Thr at amino acid position 339; either Ala or Val at amino acid position 376;
either Gly or Lys at amino acid position 377;
Asp at amino acid position 378;
Asn at amino acid position 379;
any one of Ala, Asn, and Ser at amino acid position 380;
either Ala or Ile at amino acid position 382;
Glu at amino acid position 385;
Thr at amino acid position 392;
Leu at amino acid position 396;
Lys at amino acid position 421;
Asn at amino acid position 427;
either Phe or Leu at amino acid position 428;
Met at amino acid position 429;
Trp at amino acid position 434;
Ile at amino acid position 436; and
any one of Gly, His, Ile, Leu, and Tyr at amino acid position 440,
in the Fc region site according to EU numbering.

51. The production method of any one of items 43 to 50, wherein the Fc region of a native human IgG in which the sugar chain bound at position 297 according to EU numbering is a fucose-containing sugar chain, is an Fc region of any one of native human IgG1, native human IgG2, native human IgG3, and native human IgG4 in which the sugar chain bound at position 297 according to EU numbering is a fucose-containing sugar chain.

52. The production method of any one of items 43 to 51, wherein the human Fcγ receptor is FcγRIa, FcγRIIa(R), FcγRIIa(H), FcγRIIb, FcγRIIIa(V), or FcγRIIIa(F).

53. The production method of any one of items 43 to 51, wherein the human Fcγ receptor is FcγRIIb.

54. The production method of any one of items 48 to 53, wherein the Fc region comprises at least one or more amino acids of:

Asp at amino acid position 238, and
Glu at amino acid position 328
in the Fc region site according to EU numbering.

## Claims

1. A pharmaceutical composition for use in a method of treatment by eliminating antigen from plasma, wherein the pharmaceutical composition comprises an antigen-binding molecule comprising an antigen-binding domain and an Fcy-receptor-binding domain, wherein the antigen-binding molecule has human-FcRn-binding activity in an acidic pH range condition, and wherein the antigen-binding domain has antigen-binding activity that changes depending on an ion-concentration condition, and the Fcy-receptor-binding domain has higher binding activity to the Fcγ receptor in a neutral pH range condition than an Fc region of a native human IgG in which the sugar chain bound at position 297 according to EU numbering is a fucose-containing sugar chain, wherein the antigen-binding activity of an antigen-binding domain that changes depending on ion concentration condition is defined by either one or both of the following (a) and (b):

(a) the value of KD(Ca 3μM) / KD(Ca 2 mM), which is the ratio of the dissociation constant (KD) for an antigen at a low-calcium-ion concentration condition to the KD at a high-calcium-ion concentration condition, is 2 or more; and
(b) the value of KD(pH 5.8) / KD(pH 7.4), which is the ratio of the KD for the antigen in an acidic pH range condition to the KD in a neutral pH range condition, is 2 or more.

**2.** The pharmaceutical composition of claim 1, wherein the antigen is a soluble antigen.

**3.** The pharmaceutical composition of any one of claims 1 to 2, wherein the antigen-binding molecule has neutralizing activity against the antigen.

**4.** The pharmaceutical composition of any one of claims 1 to 3, wherein the Fcγ receptor-binding domain comprises an antibody Fc region.

**5.** The pharmaceutical composition of claim 4, wherein the antibody Fc region is a Fc region of IgG in which an amino acid at position 238 in the Fc region site according to EU numbering is altered from Pro at corresponding sites in a native Fc region to any one of Asp, Glu, Phe, Gly, His, Ile, Lys, Leu, Met, Asn, Gln, Arg, Ser, Thr, Val, Trp, and Tyr.

**6.** The pharmaceutical composition of claim 5, wherein the Fc region is an Fc region which comprises at least one or more amino acids selected from the group consisting of:

either Lys or Tyr at amino acid position 221;
any one of Phe, Trp, Glu, and Tyr at amino acid position 222;
any one of Phe, Trp, Glu, and Lys at amino acid position 223;
any one of Phe, Trp, Glu, and Tyr at amino acid position 224;
any one of Glu, Lys, and Trp at amino acid position 225;
any one of Glu, Gly, Lys, and Tyr at amino acid position 227;
any one of Glu, Gly, Lys, and Tyr at amino acid position 228;
any one of Ala, Glu, Gly, and Tyr at amino acid position 230;
any one of Glu, Gly, Lys, Pro, and Tyr at amino acid position 231;
any one of Glu, Gly, Lys, and Tyr at amino acid position 232;
any one of Ala, Asp, Phe, Gly, His, Ile, Lys, Leu, Met, Asn, Gln, Arg, Ser, Thr, Val, Trp, and Tyr at amino acid position 233;
any one of Ala, Asp, Glu, Phe, Gly, His, Ile, Lys, Met, Asn, Pro, Gln, Arg, Ser, Thr, Val, Trp, and Tyr at amino acid position 234;
any one of Ala, Asp, Glu, Phe, Gly, His, Ile, Lys, Met, Asn, Pro, Gln, Arg, Ser, Thr, Val, Trp, and Tyr at amino acid position 235;
any one of Ala, Asp, Glu, Phe, His, Ile, Lys, Leu, Met, Asn, Pro, Gln, Arg, Ser, Thr, Val, Trp, and Tyr at amino acid position 236;
any one of Asp, Glu, Phe, His, Ile, Lys, Leu, Met, Asn, Pro, Gln, Arg, Ser, Thr, Val, Trp, and Tyr at amino acid position 237;
any one of Asp, Glu, Phe, Gly, His, Ile, Lys, Leu, Met, Asn, Pro, Gln, Arg, Thr, Val, Trp, and Tyr at amino acid position 239;
any one of Ala, Ile, Met, and Thr at amino acid position 240;
any one of Asp, Glu, Leu, Arg, Trp, and Tyr at amino acid position 241;
any one of Leu, Glu, Leu, Gln, Arg, Trp, and Tyr at amino acid position 243;
His at amino acid position 244;
Ala at amino acid position 245;
any one of Asp, Glu, His, and Tyr at amino acid position 246;
any one of Ala, Phe, Gly, His, Ile, Leu, Met, Thr, Val, and Tyr at amino acid position 247;
any one of Glu, His, Gln, and Tyr at amino acid position 249;
either Glu or Gln at amino acid position 250;
Phe at amino acid position 251;
any one of Phe, Met, and Tyr at amino acid position 254;
any one of Glu, Leu, and Tyr at amino acid position 255;

any one of Ala, Met, and Pro at amino acid position 256;
any one of Asp, Glu, His, Ser, and Tyr at amino acid position 258;
any one of Asp, Glu, His, and Tyr at amino acid position 260;
any one of Ala, Glu, Phe, Ile, and Thr at amino acid position 262;
any one of Ala, Ile, Met, and Thr at amino acid position 263;
any one of Asp, Glu, Phe, Gly, His, Ile, Lys, Leu, Met, Asn, Pro, Gln, Arg, Ser, Thr, Trp, and Tyr at amino acid position 264;
any one of Ala, Leu, Phe, Gly, His, Ile, Lys, Leu, Met, Asn, Pro, Gln, Arg, Ser, Thr, Val, Trp, and Tyr at amino acid position 265;
any one of Ala, Ile, Met, and Thr at amino acid position 266;
any one of Asp, Glu, Phe, His, Ile, Lys, Leu, Met, Asn, Pro, Gln, Arg, Thr, Val, Trp, and Tyr at amino acid position 267;
any one of Asp, Glu, Phe, Gly, Ile, Lys, Leu, Met, Pro, Gln, Arg, Thr, Val, and Trp at amino acid position 268;
any one of Phe, Gly, His, Ile, Lys, Leu, Met, Asn, Pro, Arg, Ser, Thr, Val, Trp, and Tyr at amino acid position 269;
any one of Glu, Phe, Gly, His, Ile, Leu, Met, Pro, Gln, Arg, Ser, Thr, Trp, and Tyr at amino acid position 270;
any one of Ala, Asp, Glu, Phe, Gly, His, Ile, Lys, Leu, Met, Asn, Gln, Arg, Ser, Thr, Val, Trp, and Tyr at amino acid position 271;
any one of Asp, Phe, Gly, His, Ile, Lys, Leu, Met, Pro, Arg, Ser, Thr, Val, Trp, and Tyr at amino acid position 272;
either Phe or Ile at amino acid position 273;
any one of Asp, Glu, Phe, Gly, His, Ile, Leu, Met, Asn, Pro, Arg, Ser, Thr, Val, Trp, and Tyr at amino acid position 274;
either Leu or Trp at amino acid position 275;
any one of Asp, Glu, Phe, Gly, His, Ile, Leu, Met, Pro, Arg, Ser, Thr, Val, Trp, and Tyr at amino acid position 276;
any one of Asp, Glu, Gly, His, Ile, Lys, Leu, Met, Asn, Pro, Gln, Arg, Ser, Thr, Val, and Trp at amino acid position 278;
Ala at amino acid position 279;
any one of Ala, Gly, His, Lys, Leu, Pro, Gln, Trp, and Tyr at amino acid position 280;
any one of Asp, Lys, Pro, and Tyr at amino acid position 281;
any one of Glu, Gly, Lys, Pro, and Tyr at amino acid position 282;
any one of Ala, Gly, His, Ile, Lys, Leu, Met, Pro, Arg, and Tyr at amino acid position 283;
any one of Asp, Glu, Leu, Asn, Thr, and Tyr at amino acid position 284;
any one of Asp, Glu, Lys, Gln, Trp, and Tyr at amino acid position 285;
any one of Glu, Gly, Pro, and Tyr at amino acid position 286;
any one of Asn, Asp, Glu, and Tyr at amino acid position 288;
any one of Asp, Gly, His, Leu, Asn, Ser, Thr, Trp, and Tyr at amino acid position 290;
any one of Asp, Glu, Gly, His, Ile, Gln, and Thr at amino acid position 291;
any one of Ala, Asp, Glu, Pro, Thr, and Tyr at amino acid position 292;
any one of Phe, Gly, His, Ile, Leu, Met, Asn, Pro, Arg, Ser, Thr, Val, Trp, and Tyr at amino acid position 293;
any one of Phe, Gly, His, Ile, Lys, Leu, Met, Asn, Pro, Arg, Ser, Thr, Val, Trp, and Tyr at amino acid position 294;
any one of Asp, Glu, Phe, Gly, His, Ile, Lys, Met, Asn, Pro, Arg, Ser, Thr, Val, Trp, and Tyr at amino acid position 295;
any one of Ala, Asp, Glu, Gly, His, Ile, Lys, Leu, Met, Asn, Gln, Arg, Ser, Thr, and Val at amino acid position 296;
any one of Asp, Glu, Phe, Gly, His, Ile, Lys, Leu, Met, Pro, Gln, Arg, Ser, Thr, Val, Trp, and Tyr at amino acid position 297;
any one of Ala, Asp, Glu, Phe, His, Ile, Lys, Met, Asn, Gln, Arg, Thr, Val, Trp, and Tyr at amino acid position 298;
any one of Ala, Asp, Glu, Phe, Gly, His, Ile, Lys, Leu, Met, Asn, Pro, Gln, Arg, Ser, Val, Trp, and Tyr at amino acid position 299;
any one of Ala, Asp, Glu, Gly, His, Ile, Lys, Leu, Met, Asn, Pro, Gln, Arg, Ser, Thr, Val, and Trp at amino acid position 300;
any one of Asp, Glu, His, and Tyr at amino acid position 301;
Ile at amino acid position 302;
any one of Asp, Gly, and Tyr at amino acid position 303;
any one of Asp, His, Leu, Asn, and Thr at amino acid position 304;
any one of Glu, Ile, Thr, and Tyr at amino acid position 305;
any one of Ala, Asp, Asn, Thr, Val, and Tyr at amino acid position 311;
Phe at amino acid position 313;
Leu at amino acid position 315;
either Glu or Gln at amino acid position 317;

any one of His, Leu, Asn, Pro, Gln, Arg, Thr, Val, and Tyr at amino acid position 318;
any one of Asp, Phe, Gly, His, Ile, Leu, Asn, Pro, Ser, Thr, Val, Trp, and Tyr at amino acid position 320;
any one of Ala, Asp, Phe, Gly, His, Ile, Pro, Ser, Thr, Val, Trp, and Tyr at amino acid position 322;
Ile at amino acid position 323;
any one of Asp, Phe, Gly, His, Ile, Leu, Met, Pro, Arg, Thr, Val, Trp, and Tyr at amino acid position 324;
any one of Ala, Asp, Glu, Phe, Gly, His, Ile, Lys, Leu, Met, Pro, Gln, Arg, Ser, Thr, Val, Trp, and Tyr at amino acid position 325;
any one of Ala, Asp, Glu, Gly, Ile, Leu, Met, Asn, Pro, Gln, Ser, Thr, Val, Trp, and Tyr at amino acid position 326;
any one of Ala, Asp, Glu, Phe, Gly, His, Ile, Lys, Leu, Met, Asn, Pro, Arg, Thr, Val, Trp, and Tyr at amino acid position 327;
any one of Ala, Asp, Glu, Phe, Gly, His, Ile, Lys, Met, Asn, Pro, Gln, Arg, Ser, Thr, Val, Trp, and Tyr at amino acid position 328;
any one of Asp, Glu, Phe, Gly, His, Ile, Lys, Leu, Met, Asn, Gln, Arg, Ser, Thr, Val, Trp, and Tyr at amino acid position 329;
any one of Cys, Glu, Phe, Gly, His, Ile, Lys, Leu, Met, Asn, Pro, Arg, Ser, Thr, Val, Trp, and Tyr at amino acid position 330;
any one of Asp, Phe, His, Ile, Leu, Met, Gln, Arg, Thr, Val, Trp, and Tyr at amino acid position 331;
any one of Ala, Asp, Glu, Phe, Gly, His, Lys, Leu, Met, Asn, Pro, Gln, Arg, Ser, Thr, Val, Trp, and Tyr at amino acid position 332;
any one of Ala, Asp, Glu, Phe, Gly, His, Ile, Leu, Met, Pro, Ser, Thr, Val, and Tyr at amino acid position 333;
any one of Ala, Glu, Phe, Ile, Leu, Pro, and Thr at amino acid position 334;
any one of Asp, Phe, Gly, His, Ile, Leu, Met, Asn, Pro, Arg, Ser, Val, Trp, and Tyr at amino acid position 335;
any one of Glu, Lys, and Tyr at amino acid position 336;
any one of Glu, His, and Asn at amino acid position 337;
any one of Asp, Phe, Gly, Ile, Lys, Met, Asn, Gln, Arg, Ser, and Thr at amino acid position 339;
either Ala or Val at amino acid position 376;
either Gly or Lys at amino acid position 377;
Asp at amino acid position 378;
Asn at amino acid position 379;
any one of Ala, Asn, and Ser at amino acid position 380;
either Ala or Ile at amino acid position 382;
Glu at amino acid position 385;
Thr at amino acid position 392;
Leu at amino acid position 396;
Lys at amino acid position 421;
Asn at amino acid position 427;
either Phe or Leu at amino acid position 428;
Met at amino acid position 429;
Trp at amino acid position 434;
Ile at amino acid position 436; and
any one of Gly, His, Ile, Leu, and Tyr at amino acid position 440,
in the Fc region site according to EU numbering.

**7.** The pharmaceutical composition of any one of claims 4 to 6, wherein the antibody Fc region in which the sugar chain bound at position 297 according to EU numbering is a fucose-containing sugar chain, is an Fc region of any one of native human IgG1, native human IgG2, native human IgG3, and native human IgG4 in which the sugar chain bound at position 297 according to EU numbering is a fucose-containing sugar chain.

**8.** The pharmaceutical composition of any one of claims 1 to 7, wherein the human Fcγ receptor is FcγRIa, FcγRIIa(R), FcγRIIa(H), FcγRIIb, FcγRIIIa(V), or FcγRIIIa(F).

**9.** The pharmaceutical composition of any one of claims 1 to 8, wherein the human Fcγ receptor is FcyRIIb.

**10.** The pharmaceutical composition of any one of claims 5 to 9, wherein the Fc region is an Fc region which comprises at least one or more of

Asp at amino acid position 238, and
Glu at amino acid position 328

in the Fc region site according to EU numbering.

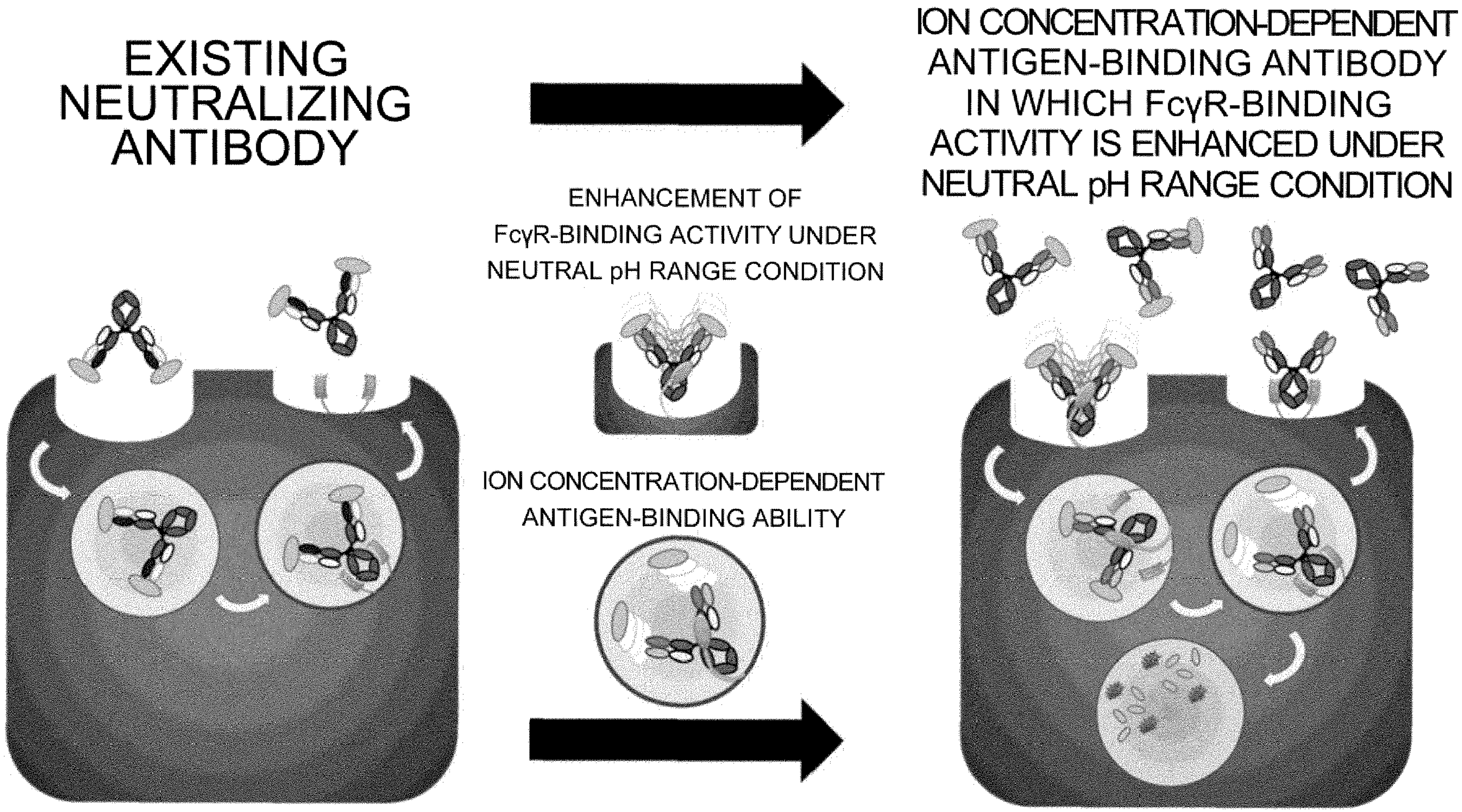
EXISTING NEUTRALIZING ANTIBODY
ENHANCEMENT OF FcγR-BINDING ACTIVITY UNDER NEUTRAL pH RANGE CONDITION
ION CONCENTRATION-DEPENDENT ANTIGEN-BINDING ABILITY
ION CONCENTRATION-DEPENDENT ANTIGEN-BINDING ANTIBODY IN WHICH FcγR-BINDING ACTIVITY IS ENHANCED UNDER NEUTRAL pH RANGE CONDITION

FIG. 1

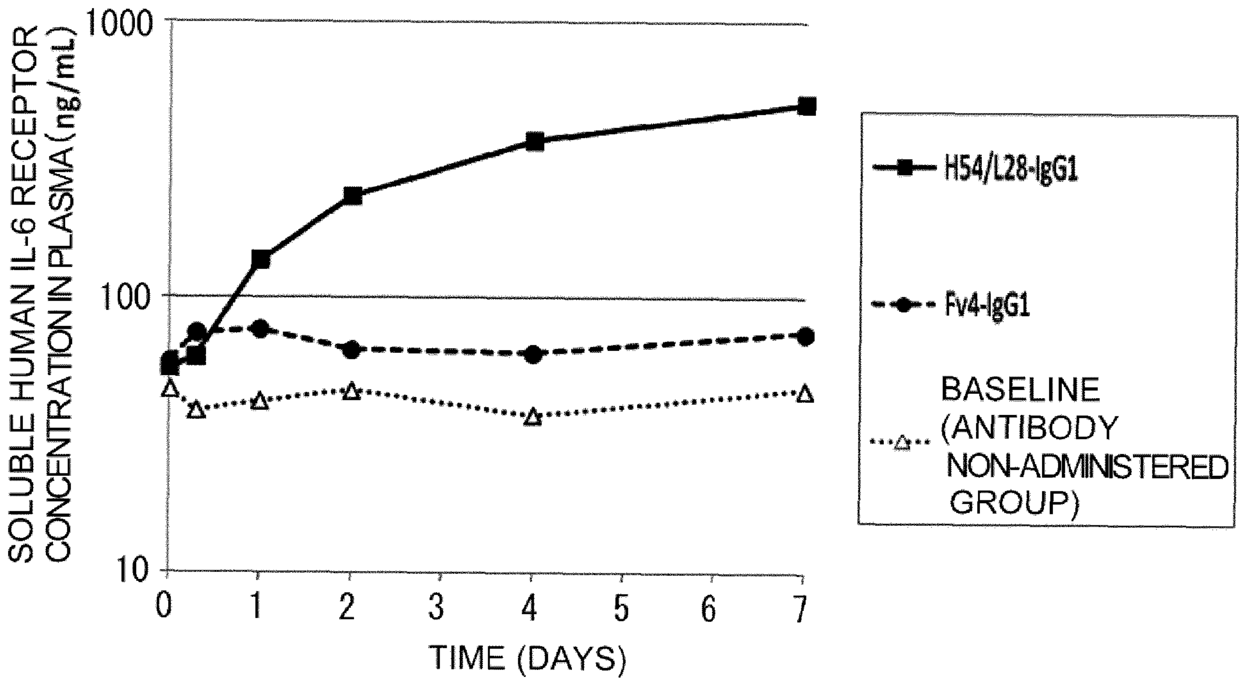
SOLUBLE HUMAN IL-6 RECEPTOR CONCENTRATION IN PLASMA (ng/mL)
1000
100
10
0
1
2
3
4
5
6
7
TIME (DAYS)
H54/L28-IgG1
Fv4-IgG1
BASELINE (ANTIBODY NON-ADMINISTERED GROUP)

FIG. 2

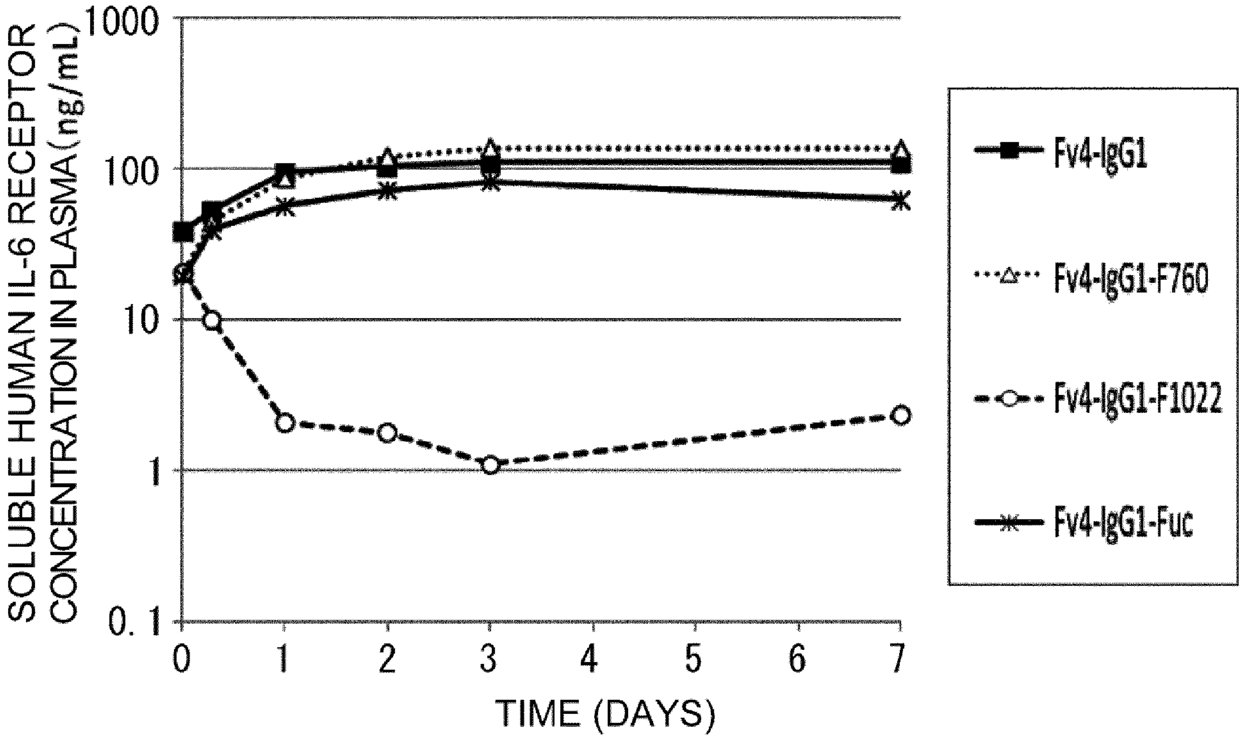
SOLUBLE HUMAN IL-6 RECEPTOR CONCENTRATION IN PLASMA (ng/mL)
1000
100
10
1
0. 1
0
1
2
3
4
5
6
7
TIME (DAYS)
Fv4-IgG1
Fv4-IgG1-F760
Fv4-IgG1-F1022
Fv4-IgG1-Fuc

FIG. 3

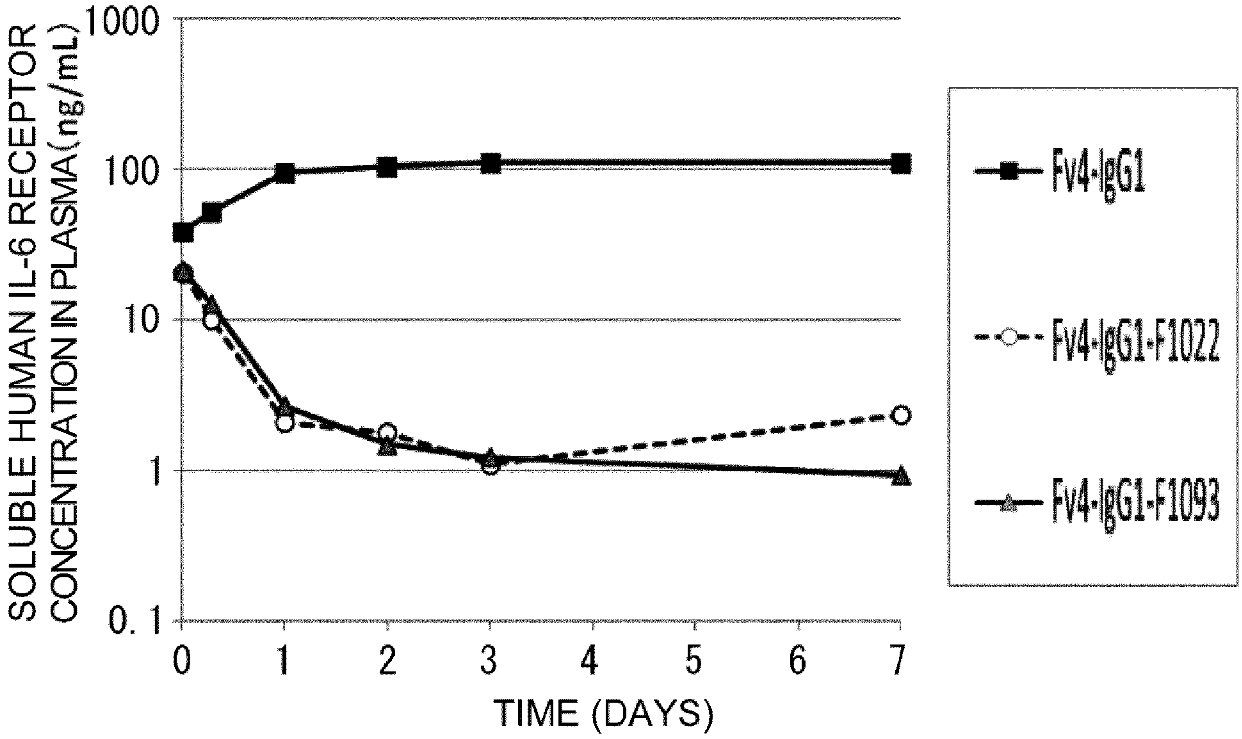
SOLUBLE HUMAN IL-6 RECEPTOR CONCENTRATION IN PLASMA (ng/mL)
1000
100
10
1
0. 1
0
1
2
3
4
5
6
7
TIME (DAYS)
Fv4-IgG1
Fv4-IgG1-F1022
Fv4-IgG1-F1093

FIG. 4

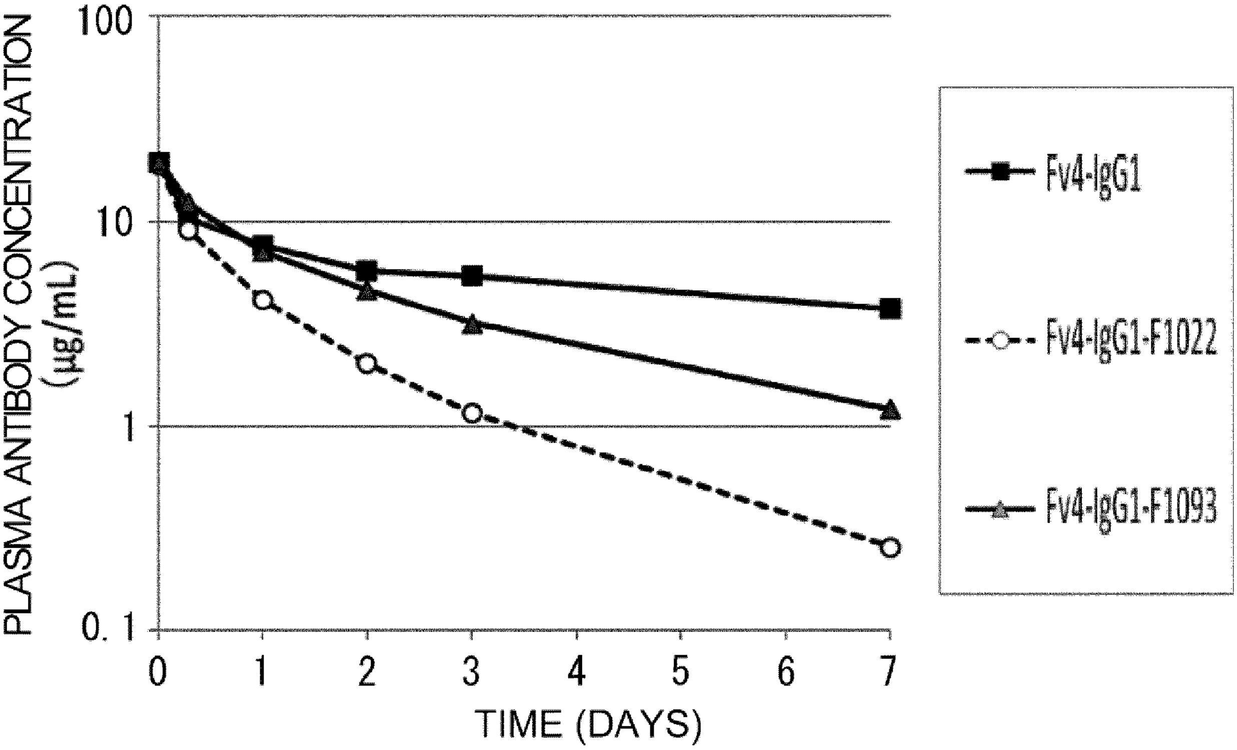
PLASMA ANTIBODY CONCENTRATION (μg/mL)
100
10
1
0. 1
0
1
2
3
4
5
6
7
TIME (DAYS)
Fv4-IgG1
Fv4-IgG1-F1022
Fv4-IgG1-F1093

FIG. 5

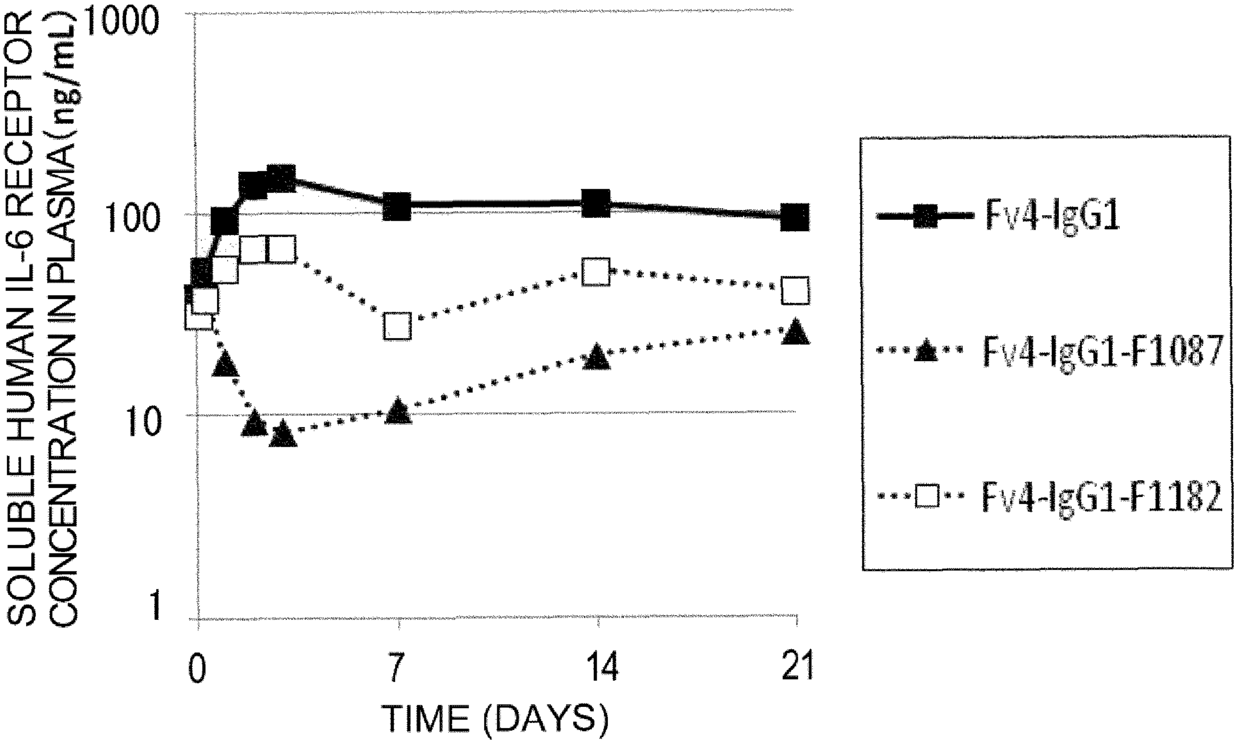
SOLUBLE HUMAN IL-6 RECEPTOR CONCENTRATION IN PLASMA(ng/mL)
1000
100
10
1
0
7
14
21
TIME (DAYS)
Fv4-IgG1
Fv4-IgG1-F1087
Fv4-IgG1-F1182

FIG. 6

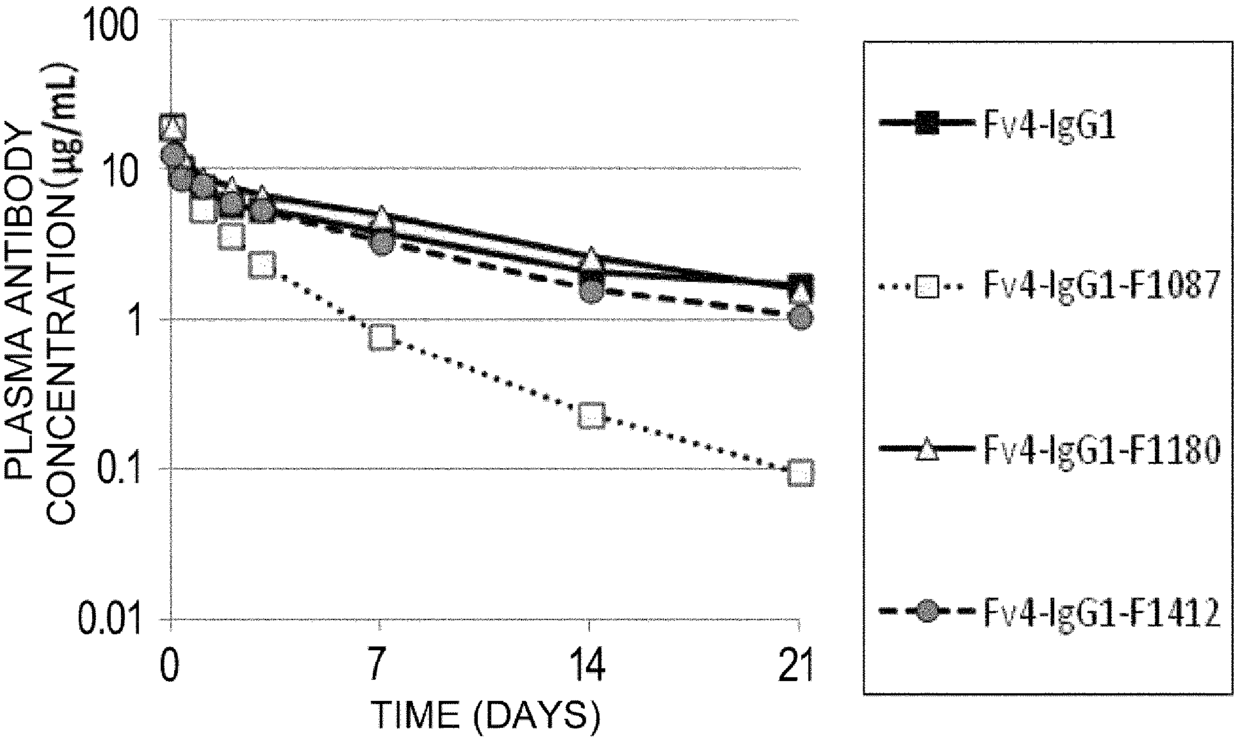
100
10
1
0.1
0.01
PLASMA ANTIBODY CONCENTRATION(μg/mL)
0
7
14
21
TIME (DAYS)
Fv4-IgG1
Fv4-IgG1-F1087
Fv4-IgG1-F1180
Fv4-IgG1-F1412

FIG. 7

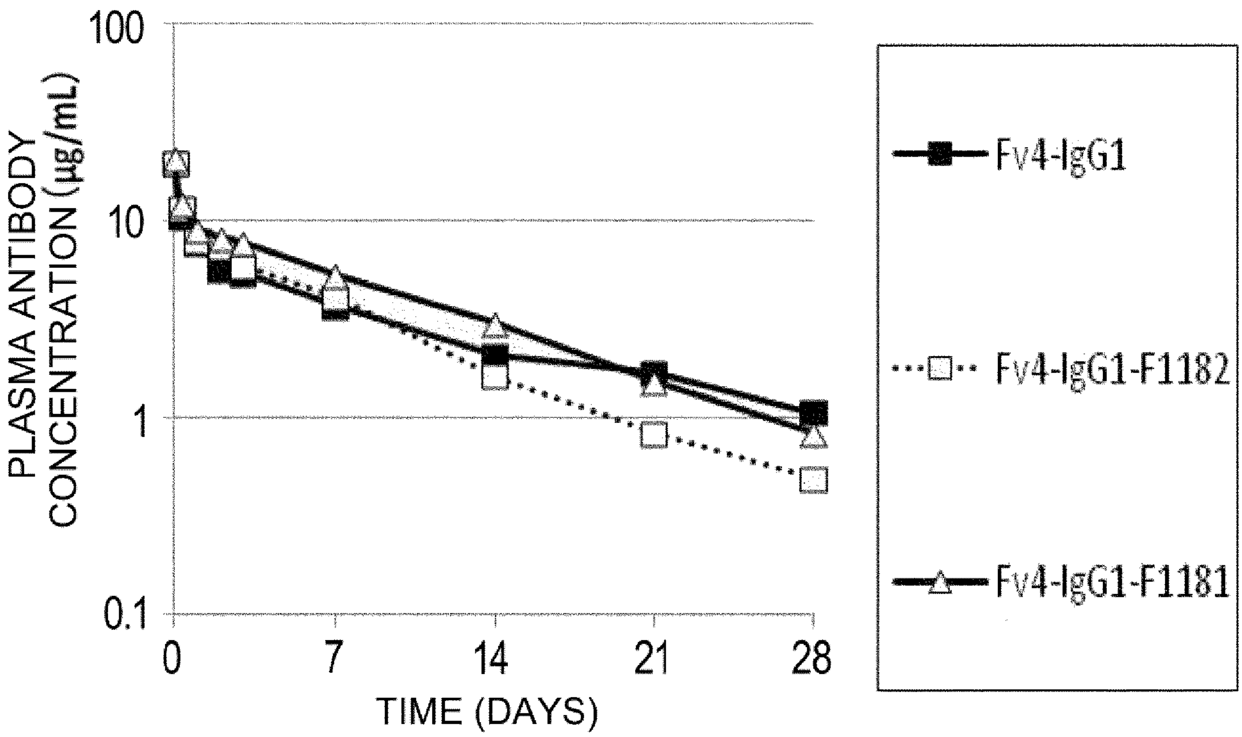
100
10
1
0.1
PLASMA ANTIBODY CONCENTRATION (μg/mL)
0
7
14
21
28
TIME (DAYS)
Fv4-IgG1
Fv4-IgG1-F1182
Fv4-IgG1-F1181

FIG. 8

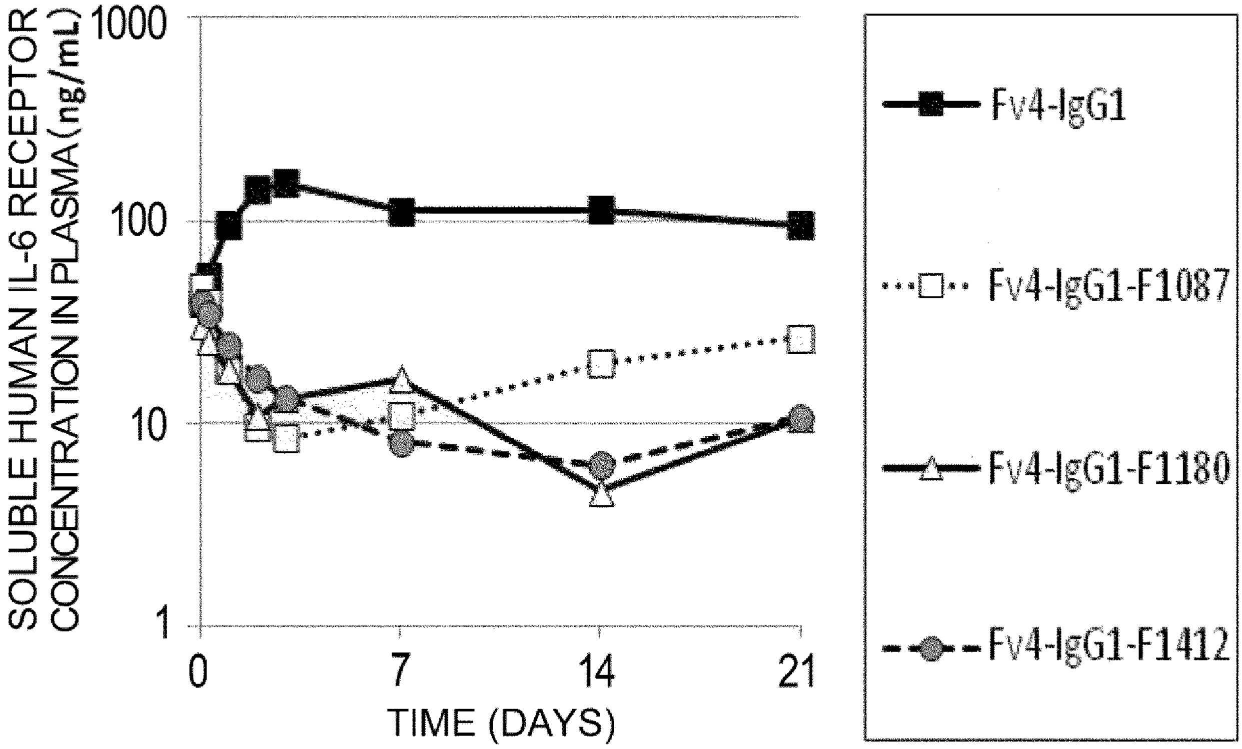
SOLUBLE HUMAN IL-6 RECEPTOR CONCENTRATION IN PLASMA (ng/mL)
1000
100
10
1
0
7
14
21
TIME (DAYS)
Fv4-IgG1
Fv4-IgG1-F1087
Fv4-IgG1-F1180
Fv4-IgG1-F1412

FIG. 9

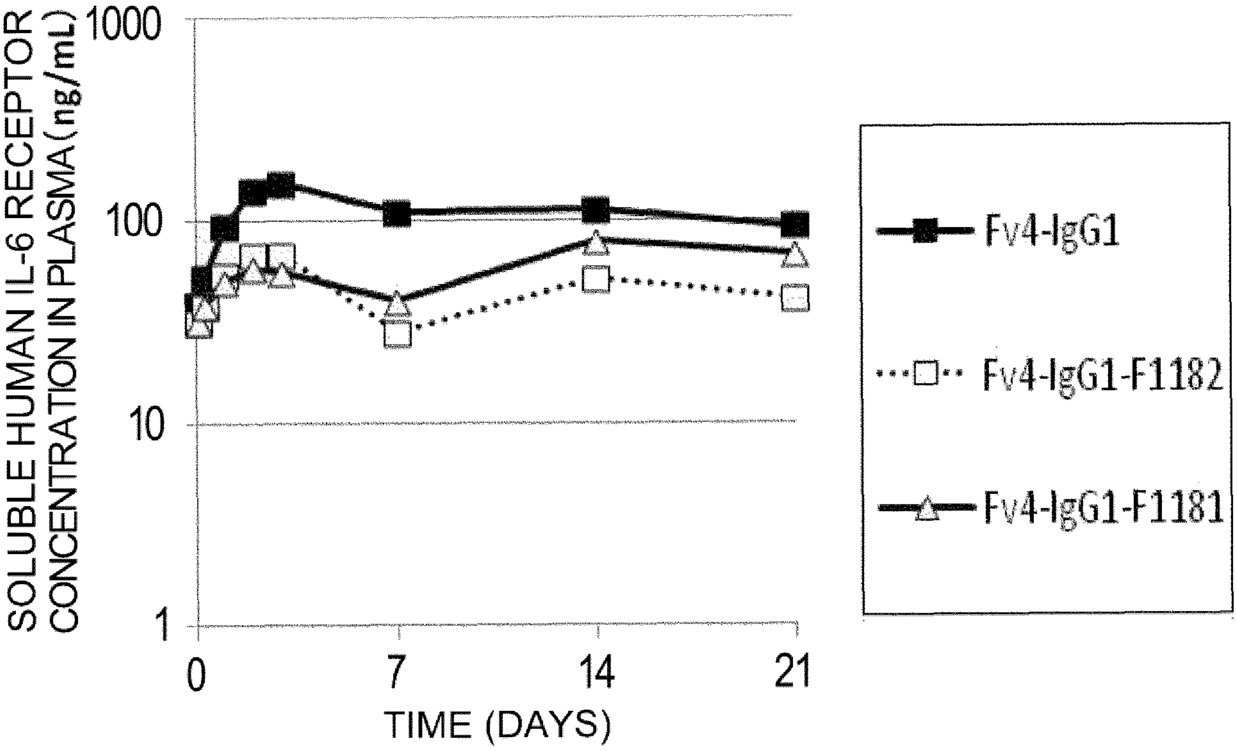
SOLUBLE HUMAN IL-6 RECEPTOR CONCENTRATION IN PLASMA (ng/mL)
1000
100
10
1
0
7
14
21
TIME (DAYS)
Fv4-IgG1
Fv4-IgG1-F1182
Fv4-IgG1-F1181

FIG. 10

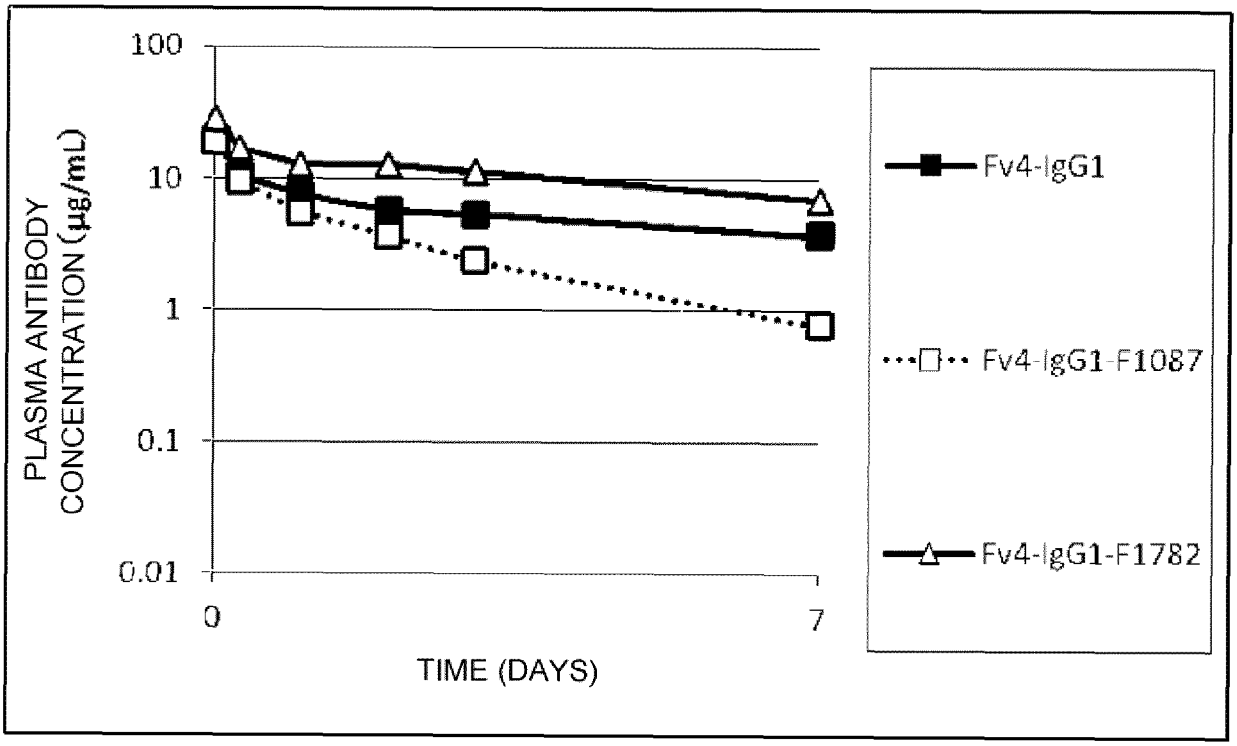
100
10
1
0.1
0.01
PLASMA ANTIBODY CONCENTRATION (μg/mL)
0
7
TIME (DAYS)
Fv4-IgG1
Fv4-IgG1-F1087
Fv4-IgG1-F1782

FIG. 11

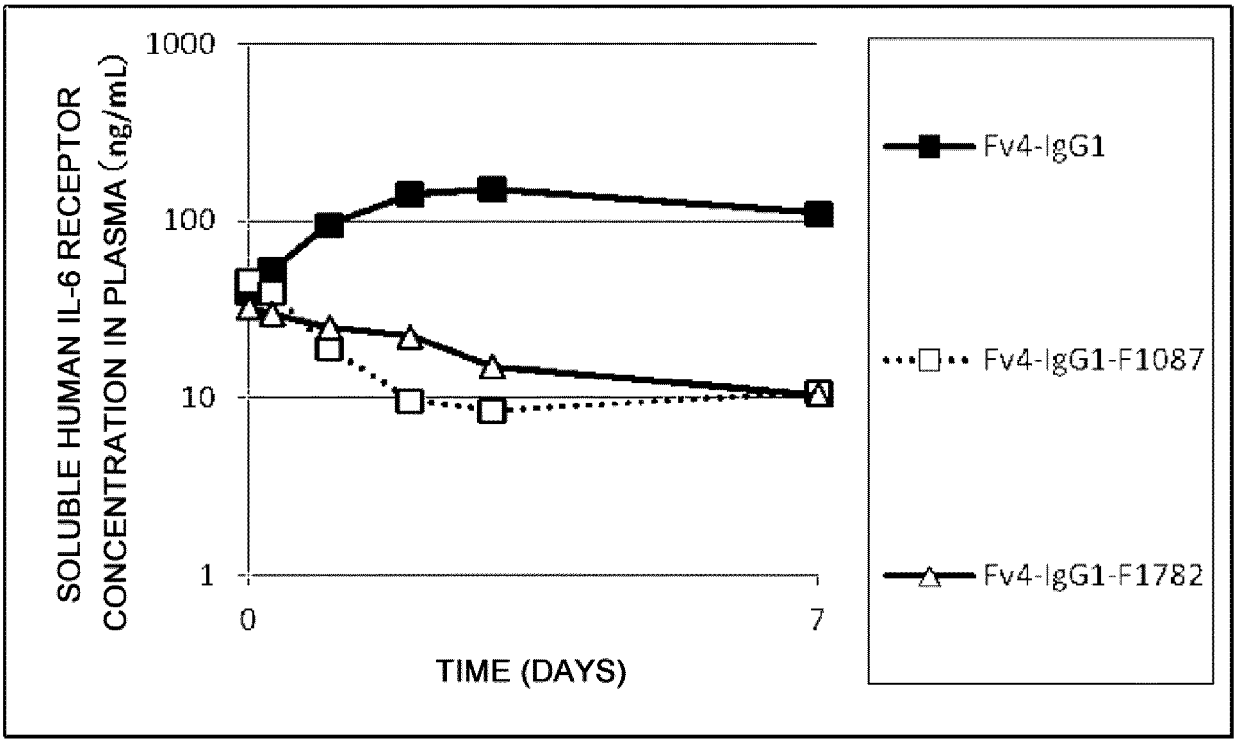
SOLUBLE HUMAN IL-6 RECEPTOR CONCENTRATION IN PLASMA (ng/mL)
1000
100
10
1
0
7
TIME (DAYS)
Fv4-IgG1
Fv4-IgG1-F1087
Fv4-IgG1-F1782

FIG. 12

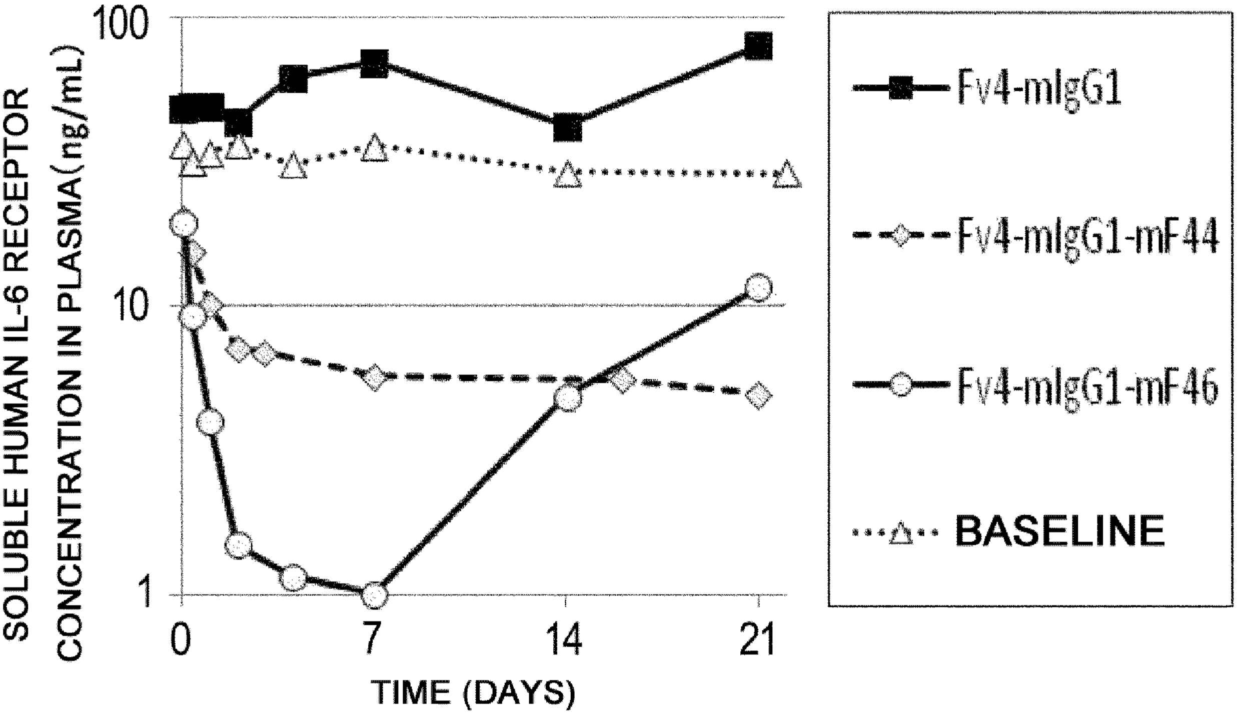
100
10
1
0
7
14
21
TIME (DAYS)
SOLUBLE HUMAN IL-6 RECEPTOR CONCENTRATION IN PLASMA (ng/mL)
Fv4-mIgG1
Fv4-mIgG1-mF44
Fv4-mIgG1-mF46
BASELINE

FIG. 13

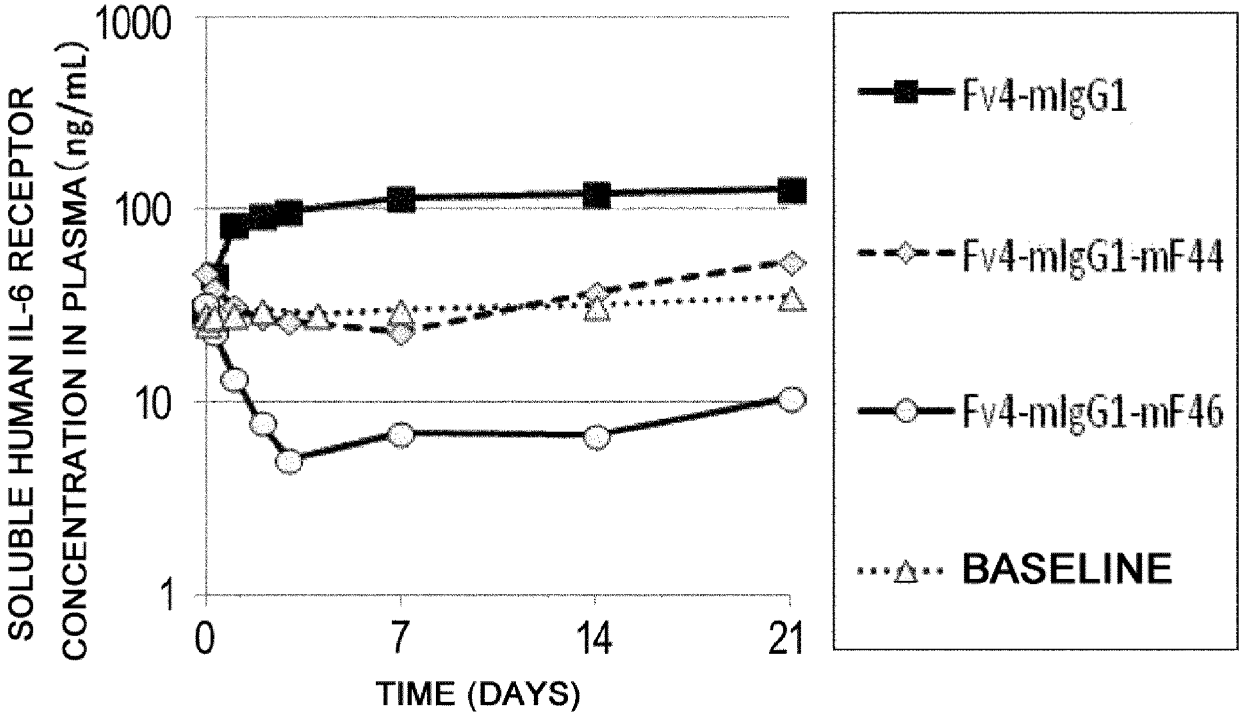
1000
100
10
1
0
7
14
21
SOLUBLE HUMAN IL-6 RECEPTOR CONCENTRATION IN PLASMA (ng/mL)
TIME (DAYS)
Fv4-mIgG1
Fv4-mIgG1-mF44
Fv4-mIgG1-mF46
BASELINE

FIG. 14

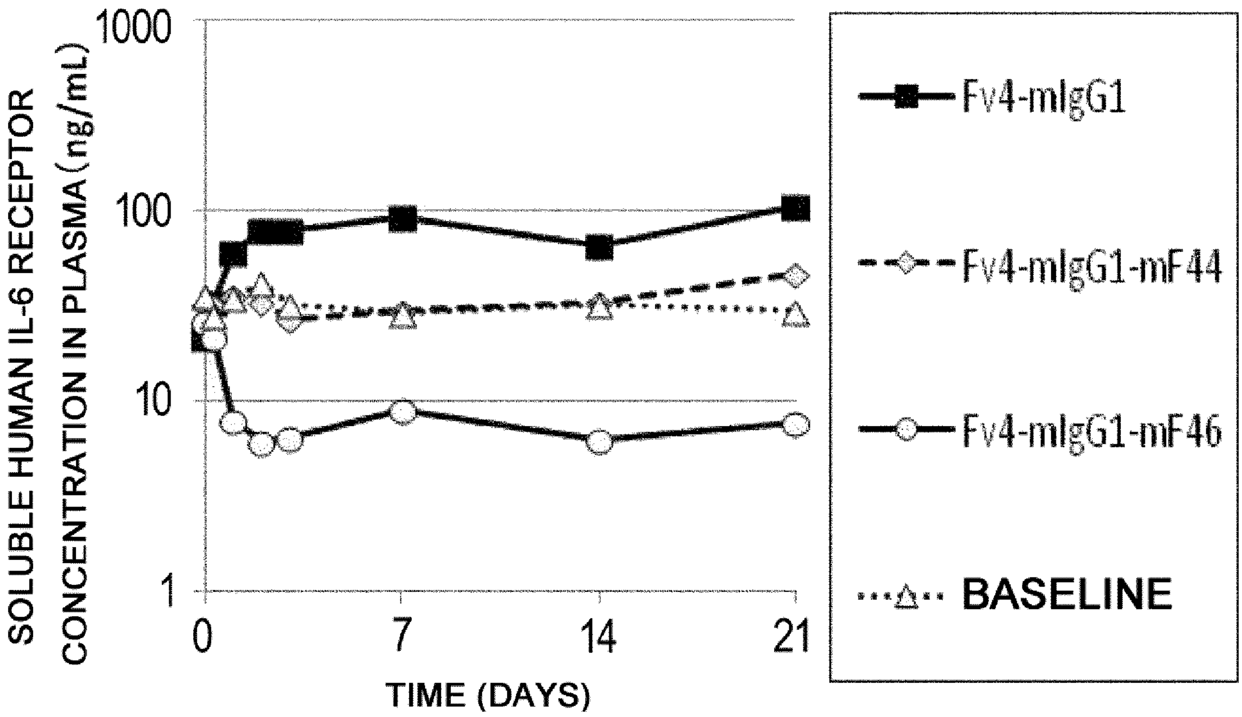
SOLUBLE HUMAN IL-6 RECEPTOR CONCENTRATION IN PLASMA (ng/mL)
1000
100
10
1
0
7
14
21
TIME (DAYS)
Fv4-mIgG1
Fv4-mIgG1-mF44
Fv4-mIgG1-mF46
BASELINE

FIG. 15

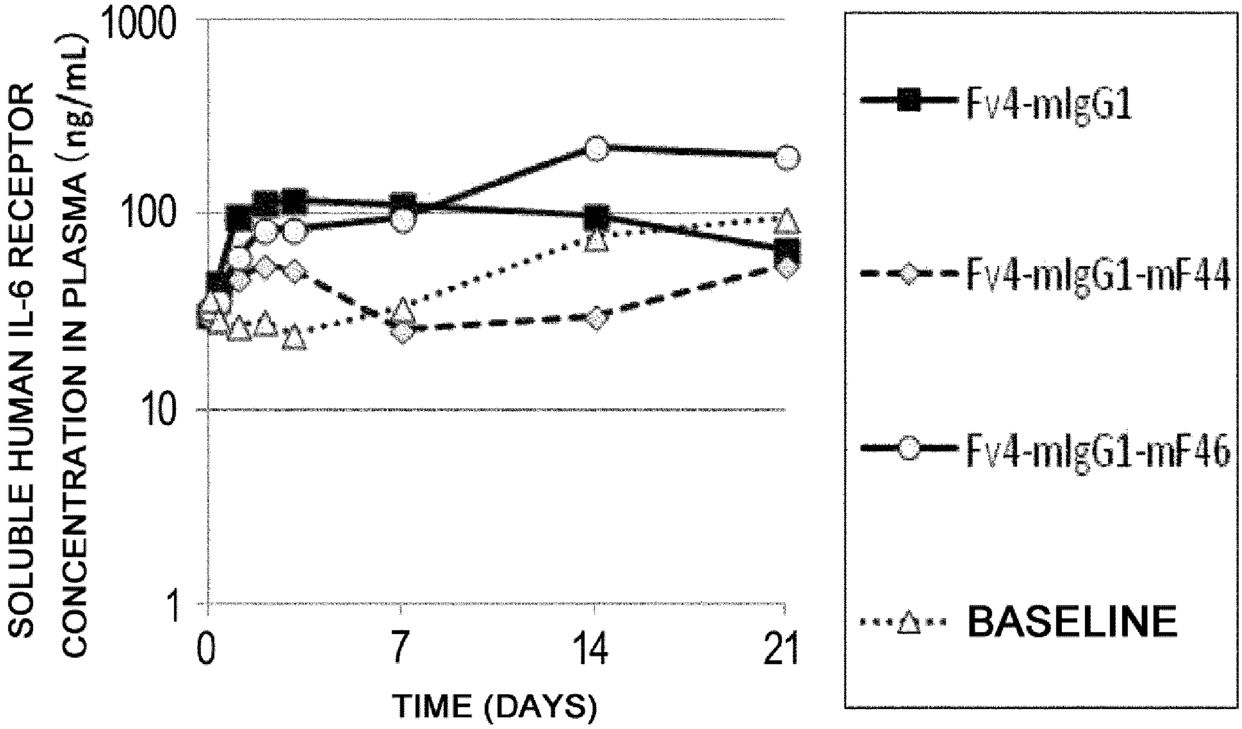
SOLUBLE HUMAN IL-6 RECEPTOR CONCENTRATION IN PLASMA (ng/mL)
1000
100
10
1
0
7
14
21
TIME (DAYS)
Fv4-mIgG1
Fv4-mIgG1-mF44
Fv4-mIgG1-mF46
BASELINE

FIG. 16

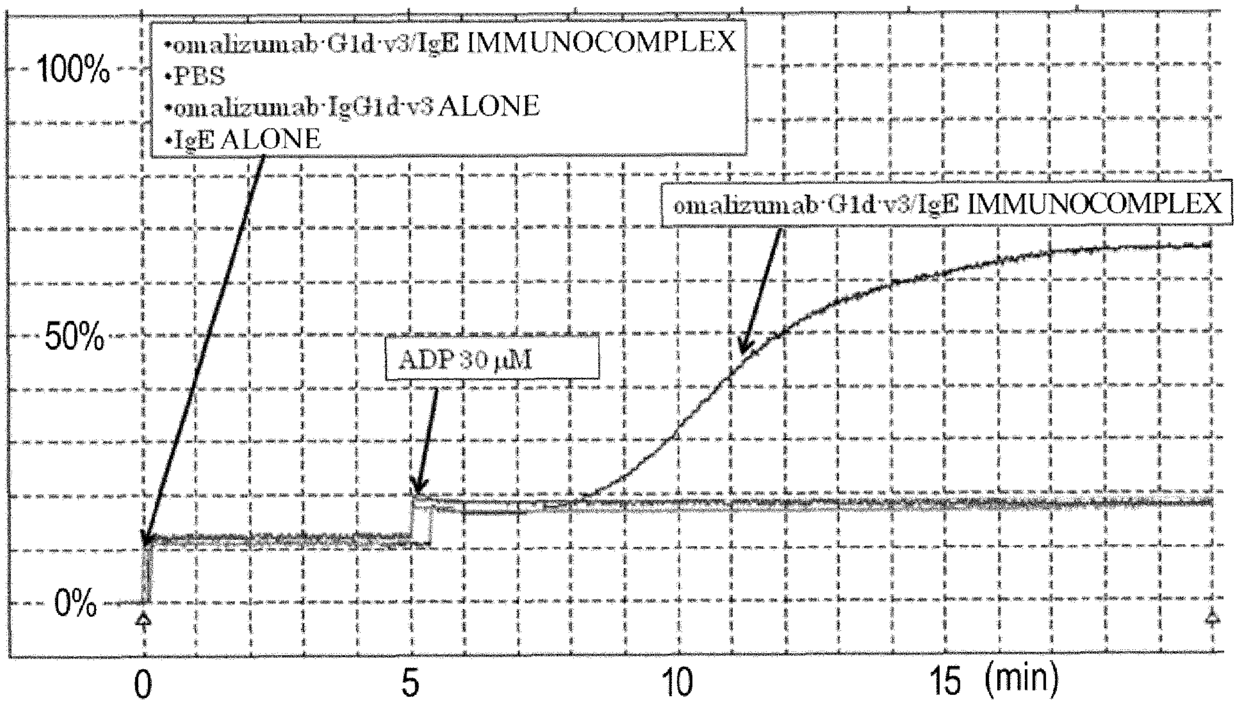
•omalizumab·G1d·v3/IgE IMMUNOCOMPLEX
•PBS
•omalizumab·IgG1d·v3 ALONE
•IgE ALONE
omalizumab·G1d·v3/IgE IMMUNOCOMPLEX
ADP 30 μM
100%
50%
0%
0
5
10
15
(min)

FIG. 17

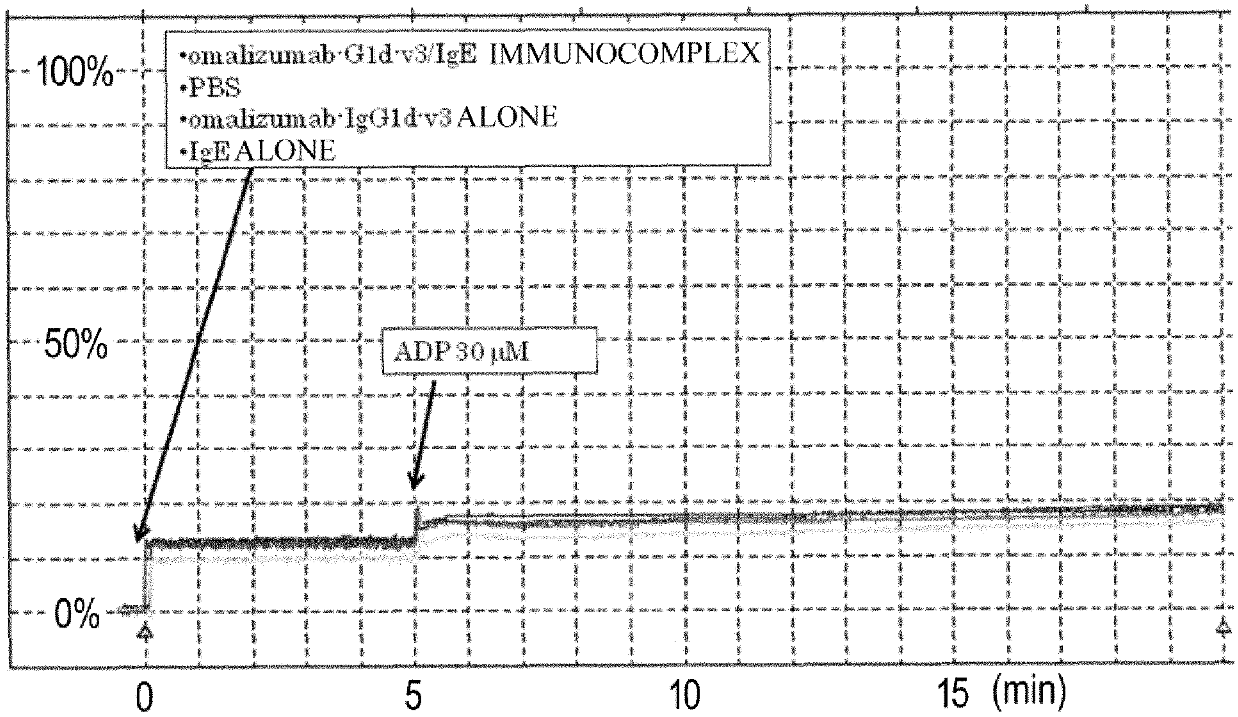

100%
50%
0%
•omalizumab-G1d-v3/IgE IMMUNOCOMPLEX
•PBS
•omalizumab-IgG1d-v3 ALONE
•IgE ALONE
ADP 30 μM
0
5
10
15
(min)


FIG. 18

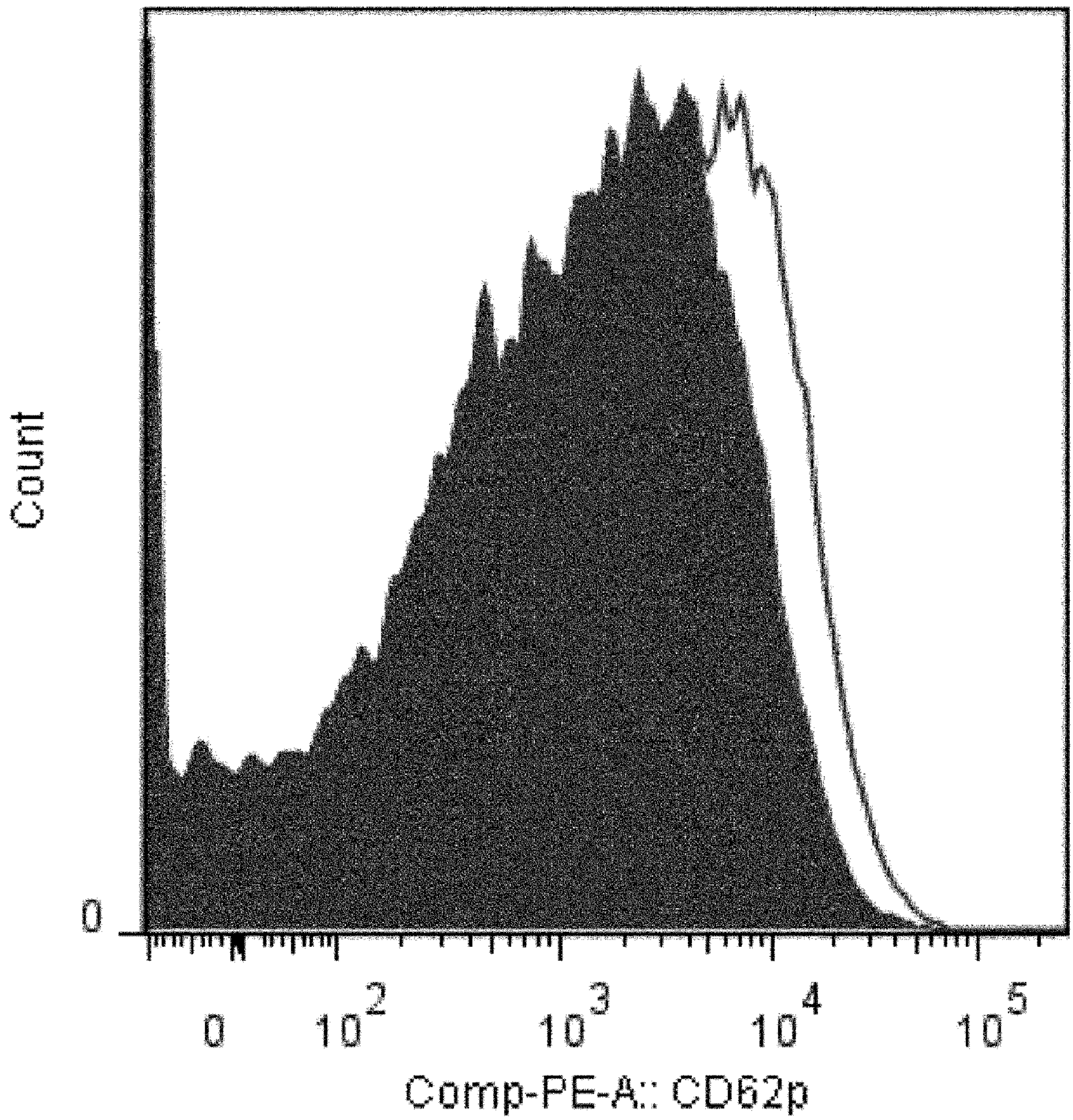


FIG. 19

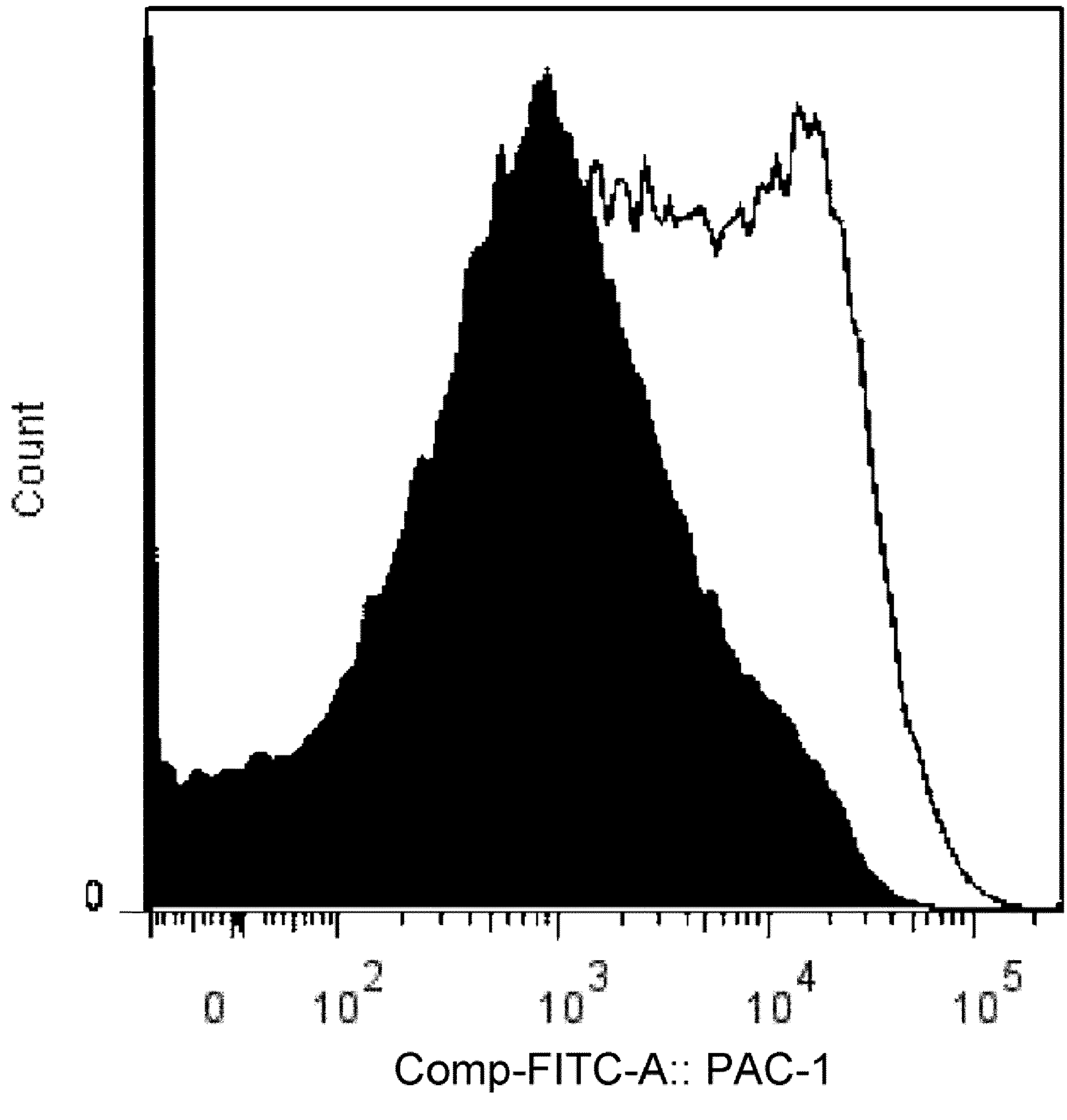


FIG. 20

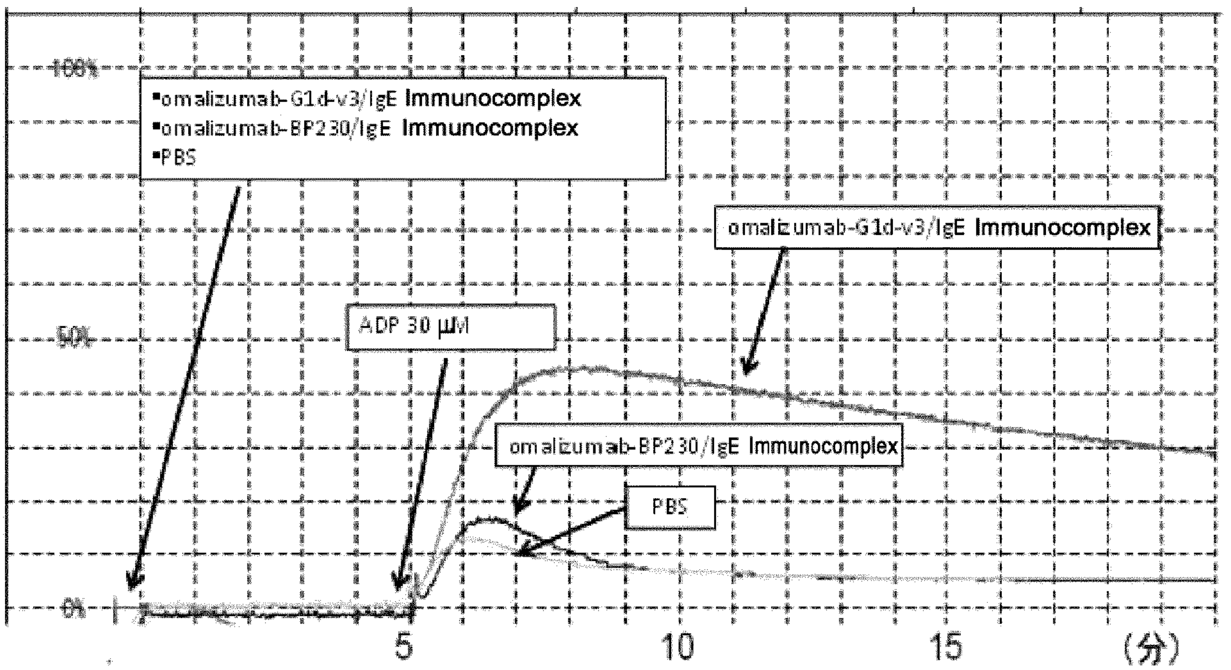
100%
50%
0%
▪omalizumab-G1d-v3/IgE Immunocomplex
▪omalizumab-BP230/IgE Immunocomplex
▪PBS
omalizumab-G1d-v3/IgE Immunocomplex
ADP 30 μM
omalizumab-BP230/IgE Immunocomplex
PBS
5
10
15
(分)

FIG. 21

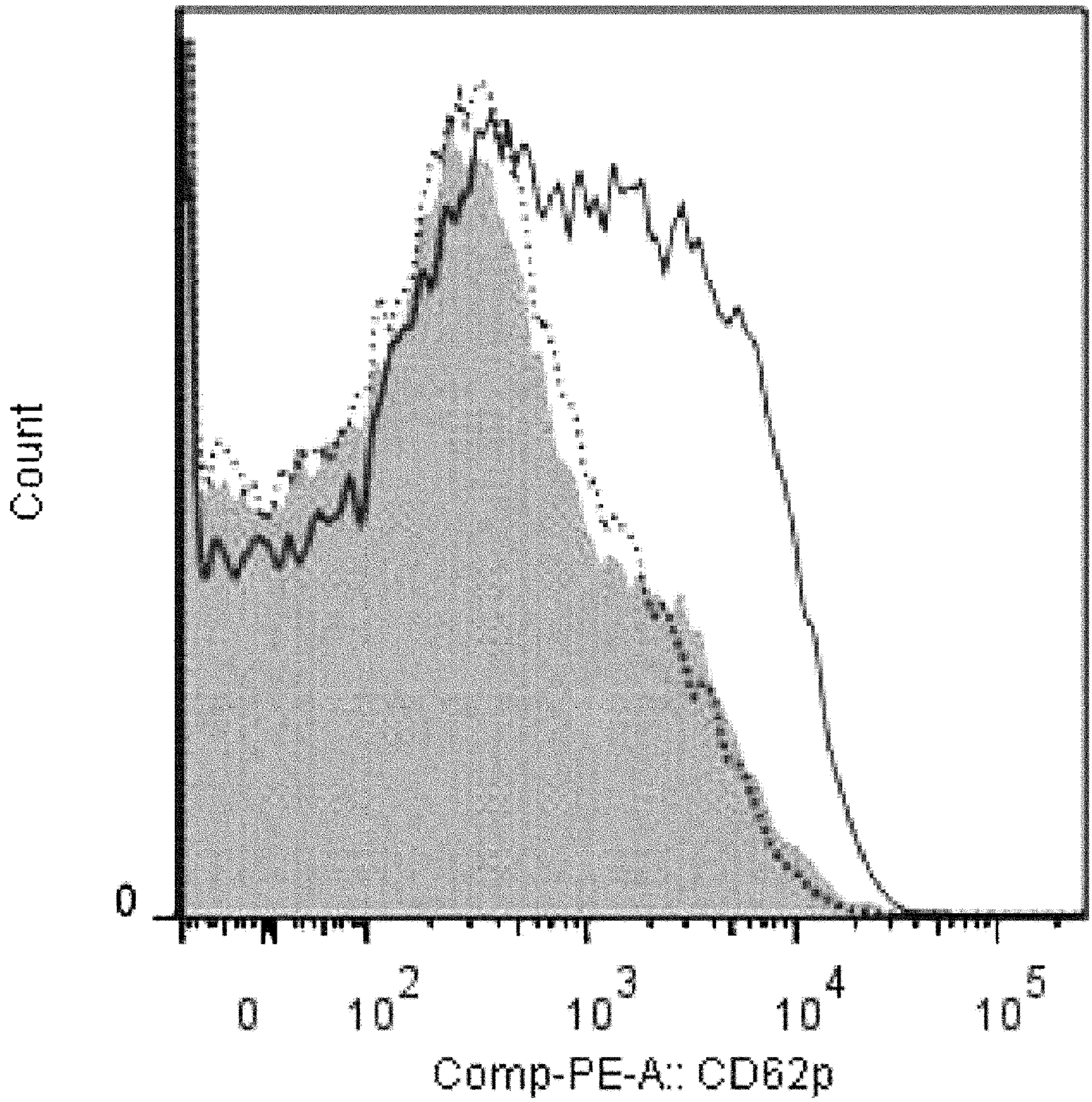


FIG. 22

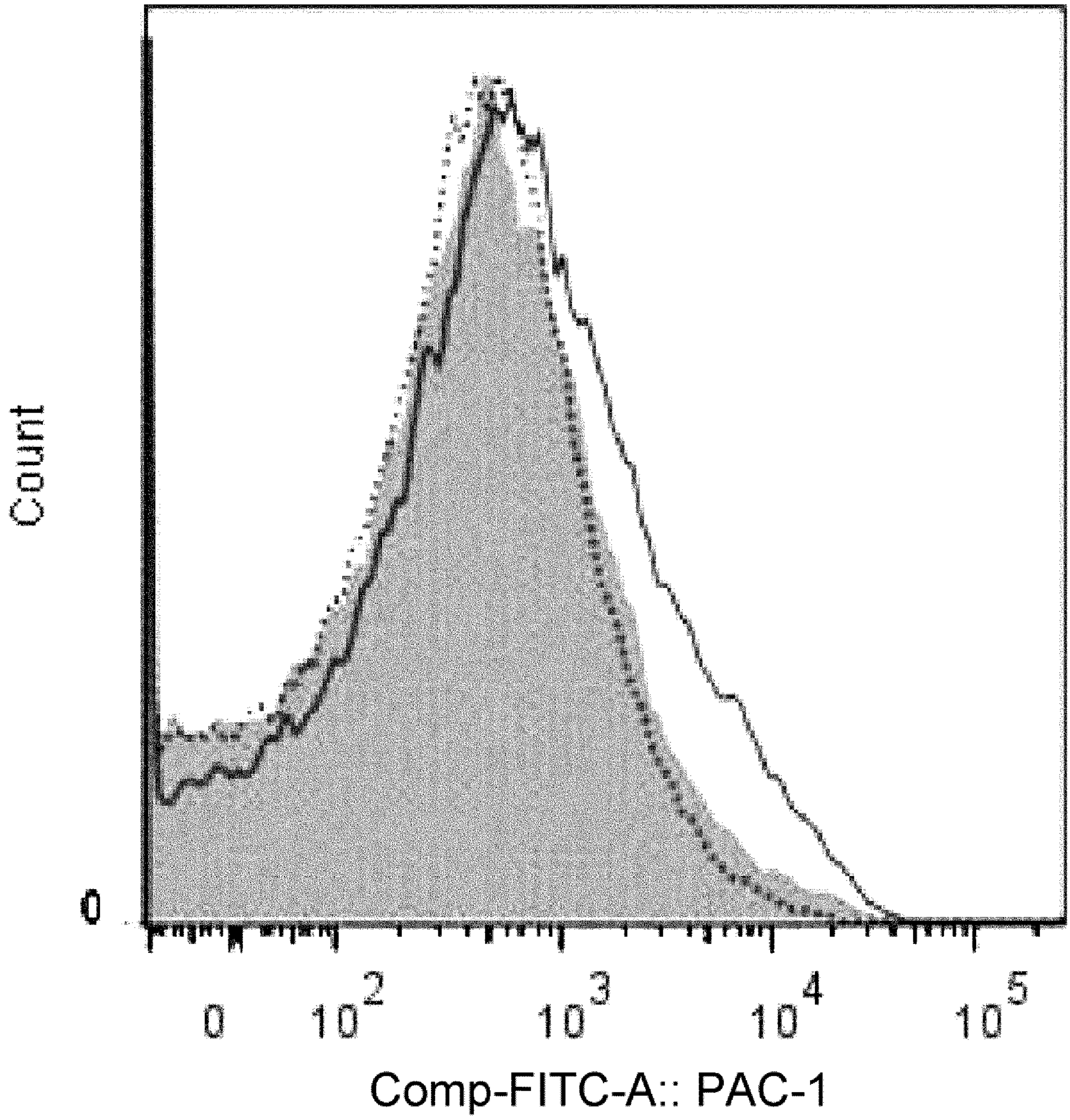


FIG. 23

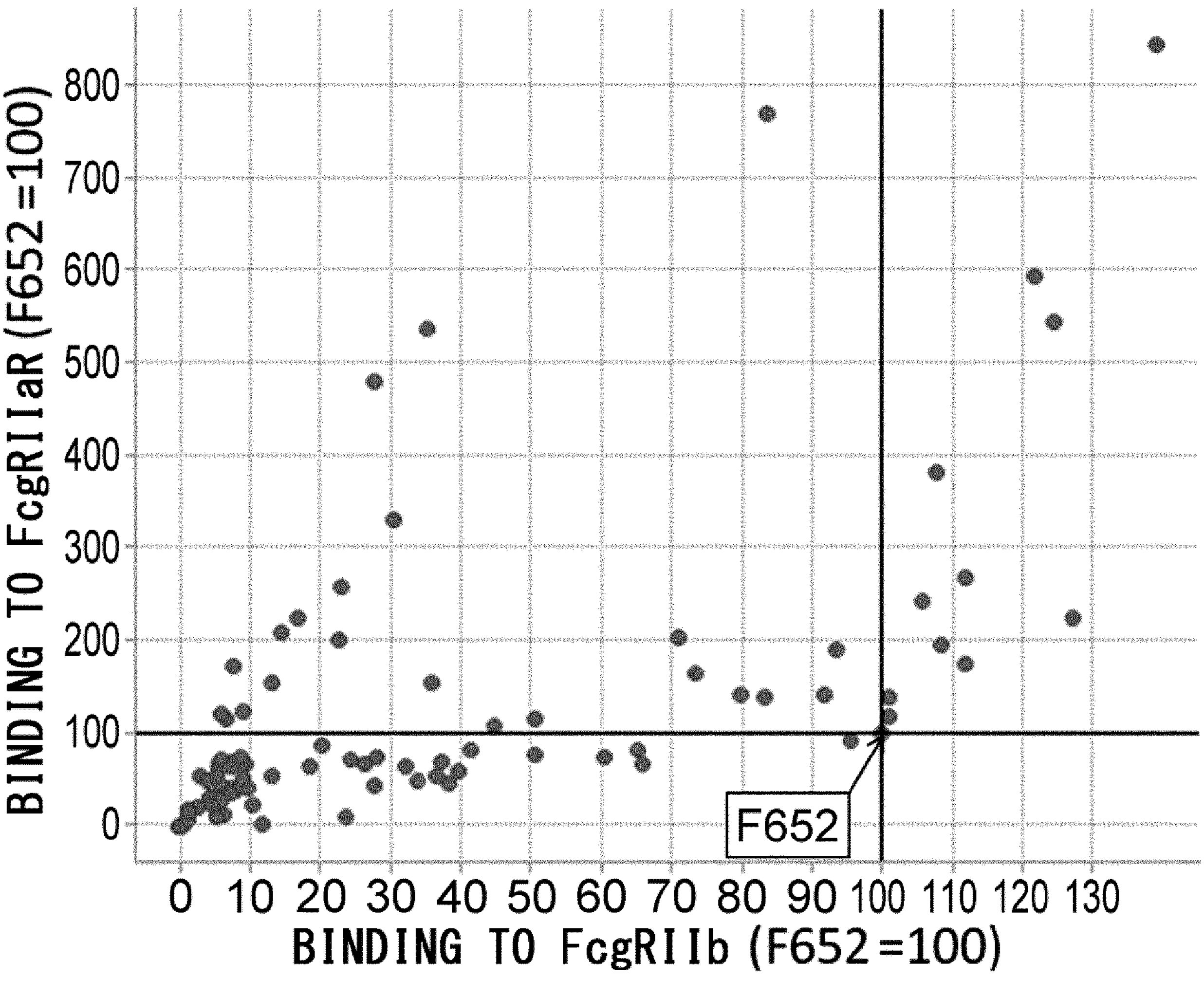
BINDING TO FcgRIIaR (F652=100)
800
700
600
500
400
300
200
100
0
F652
0 10 20 30 40 50 60 70 80 90 100 110 120 130
BINDING TO FcgRIIb (F652=100)

FIG. 24

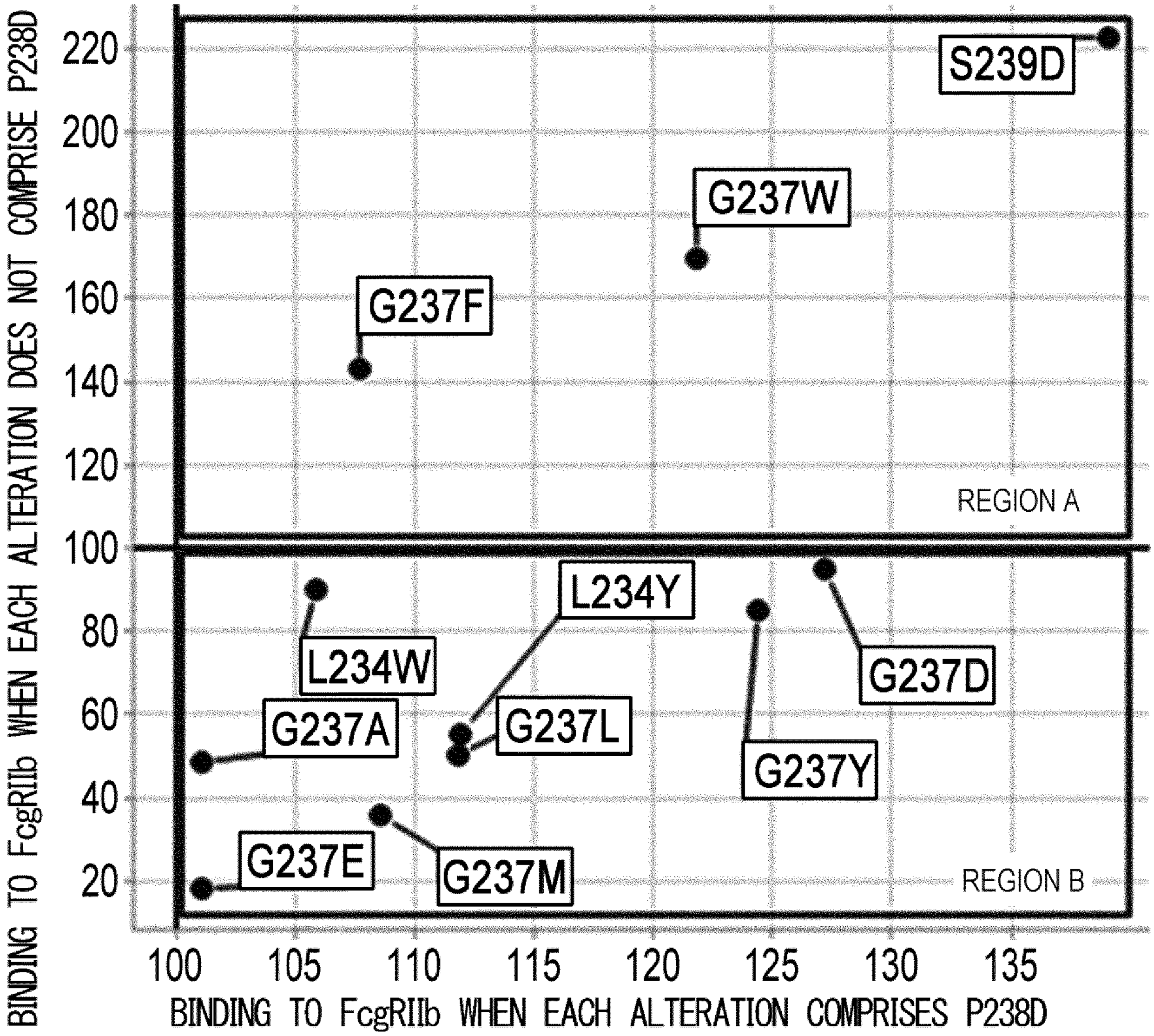
BINDING TO FcgRIIb WHEN EACH ALTERATION DOES NOT COMPRISE P238D
220
200
180
160
140
120
100
80
60
40
20
100
105
110
115
120
125
130
135
BINDING TO FcgRIIb WHEN EACH ALTERATION COMPRISES P238D
S239D
G237W
G237F
REGION A
L234Y
L234W
G237D
G237A
G237L
G237Y
G237E
G237M
REGION B

FIG. 25

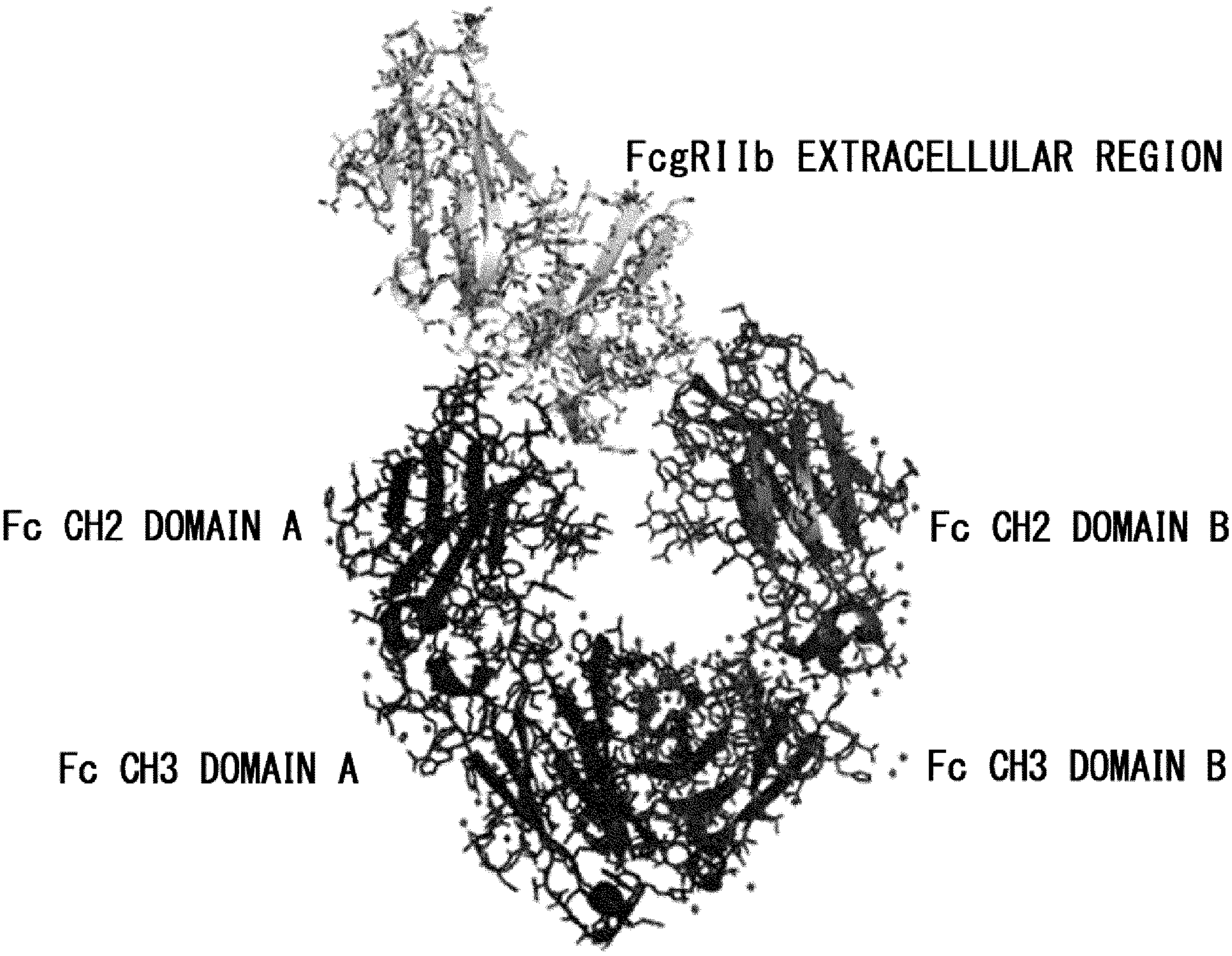
FcgRIIb EXTRACELLULAR REGION
Fc CH2 DOMAIN A
Fc CH2 DOMAIN B
Fc CH3 DOMAIN A
Fc CH3 DOMAIN B

FIG. 26

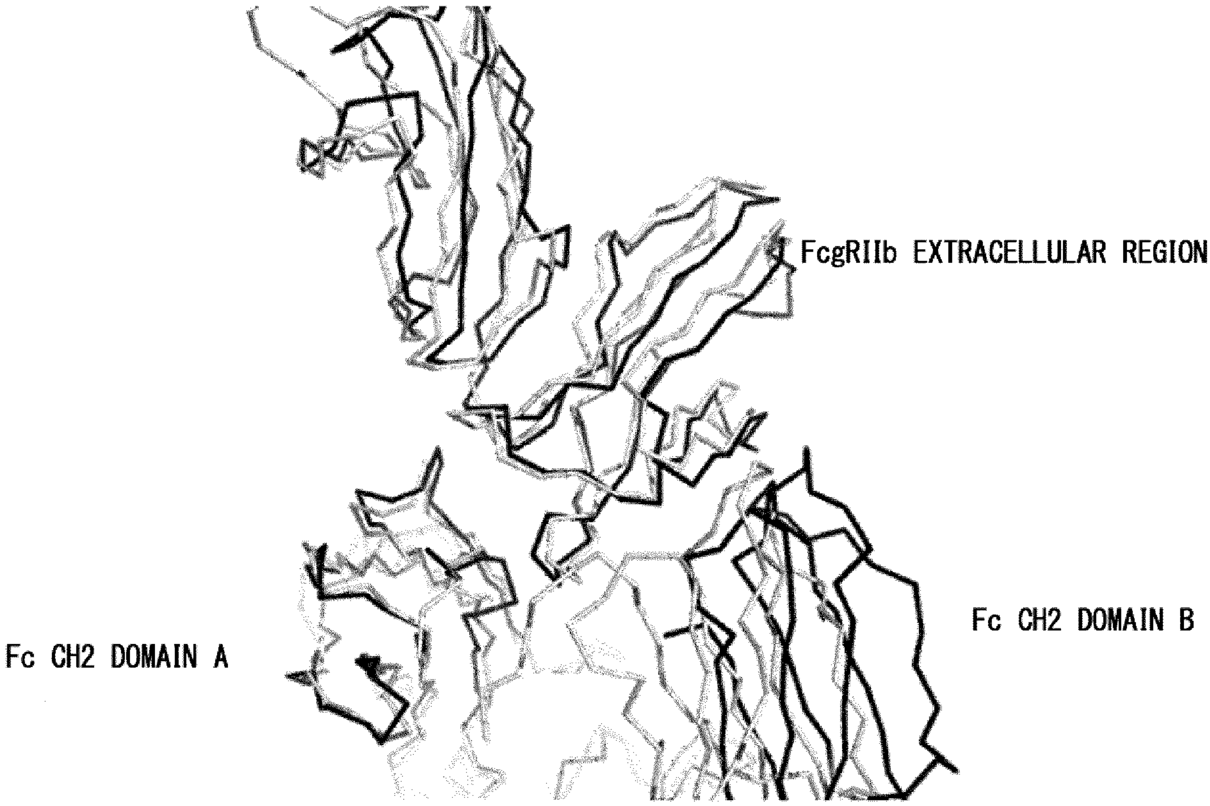


BLACK CRYSTAL STRUCTURE OF Fc(P238D)/FcγRIIb EXTRACELLULAR REGION COMPLEX
GRAY MODEL STRUCTURE OF Fc(WT)/FcγRIIb EXTRACELLULAR REGION COMPLEX

FIG. 27

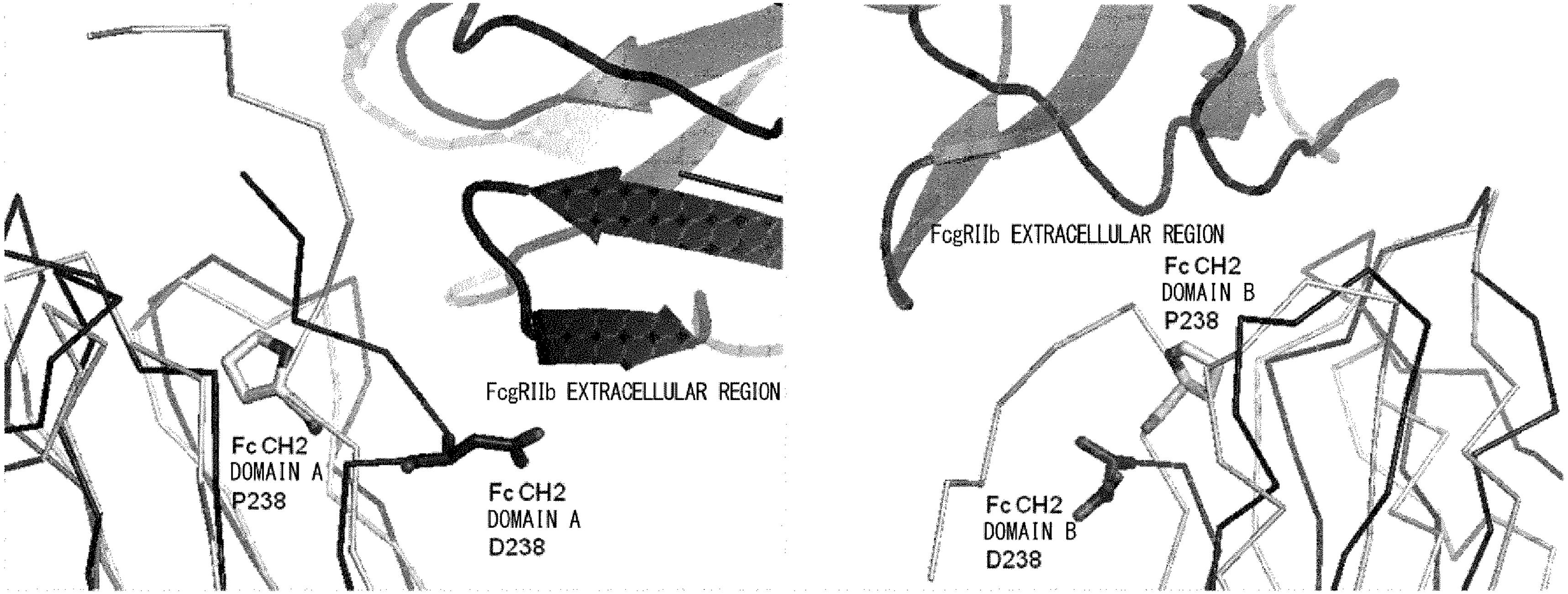


BLACK CRYSTAL STRUCTURE OF Fc(P238D)/Fcγ RIIb EXTRACELLULAR REGION COMPLEX

GRAY MODEL STRUCTURE OF Fc(WT)/Fcγ RIIb EXTRACELLULAR REGION COMPLEX

FIG. 28

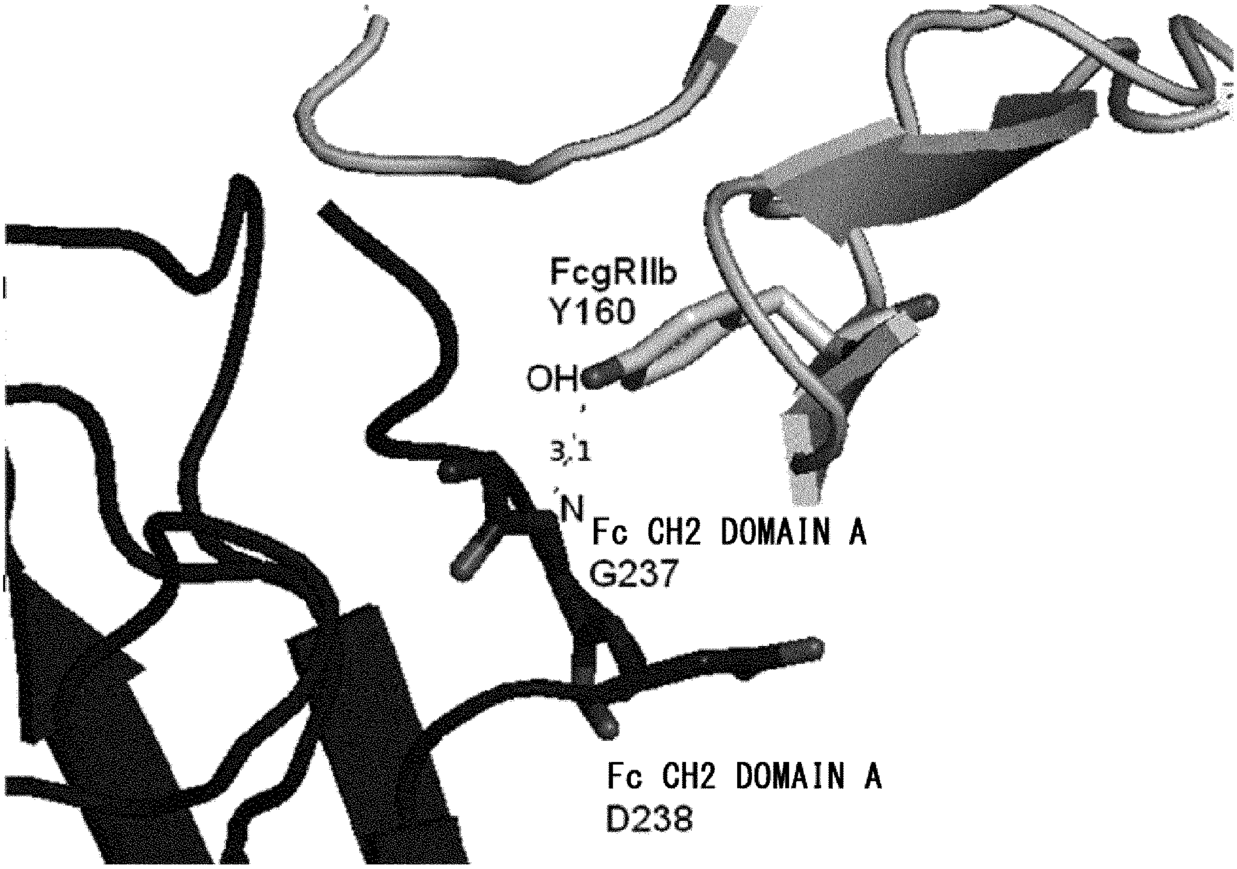


FIG. 29

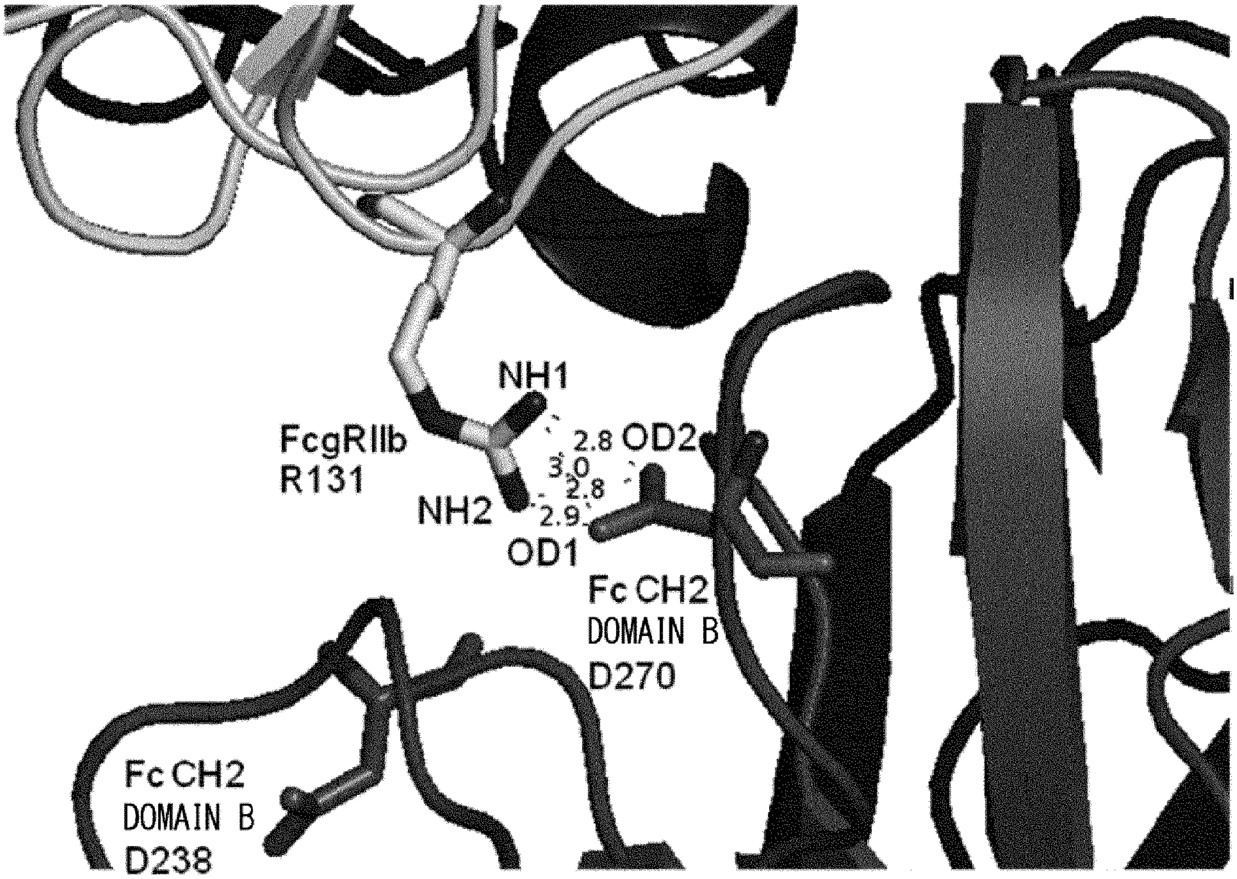
NH1
FcgRIIb
R131
2.8
OD2
3.0
2.8
NH2
2.9
OD1
Fc CH2
DOMAIN B
D270
Fc CH2
DOMAIN B
D238

FIG. 30

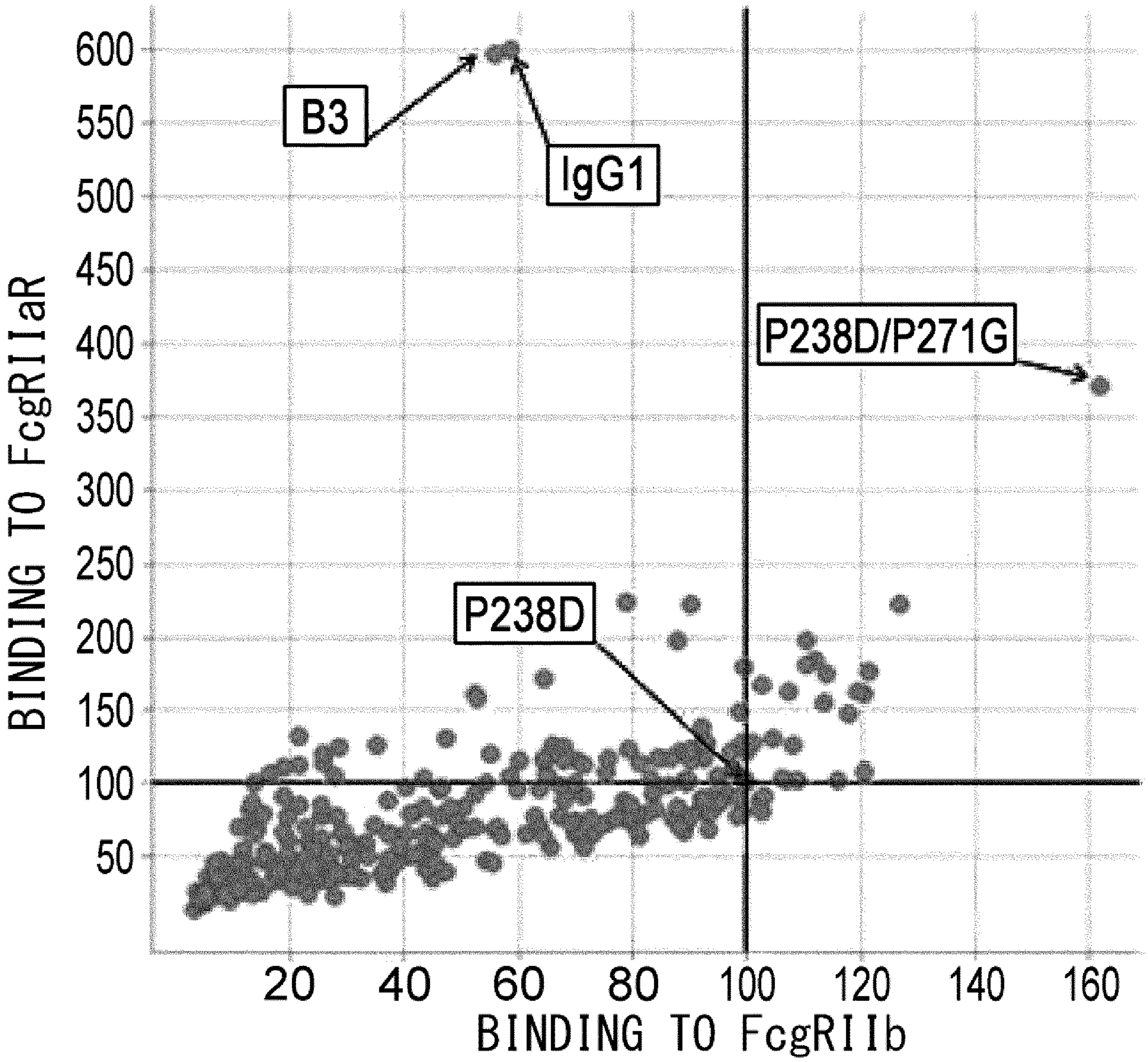
600
550
500
450
400
350
300
250
200
150
100
50
BINDING TO FcgRIIaR
B3
IgG1
P238D/P271G
P238D
20
40
60
80
100
120
140
160
BINDING TO FcgRIIb

FIG. 31

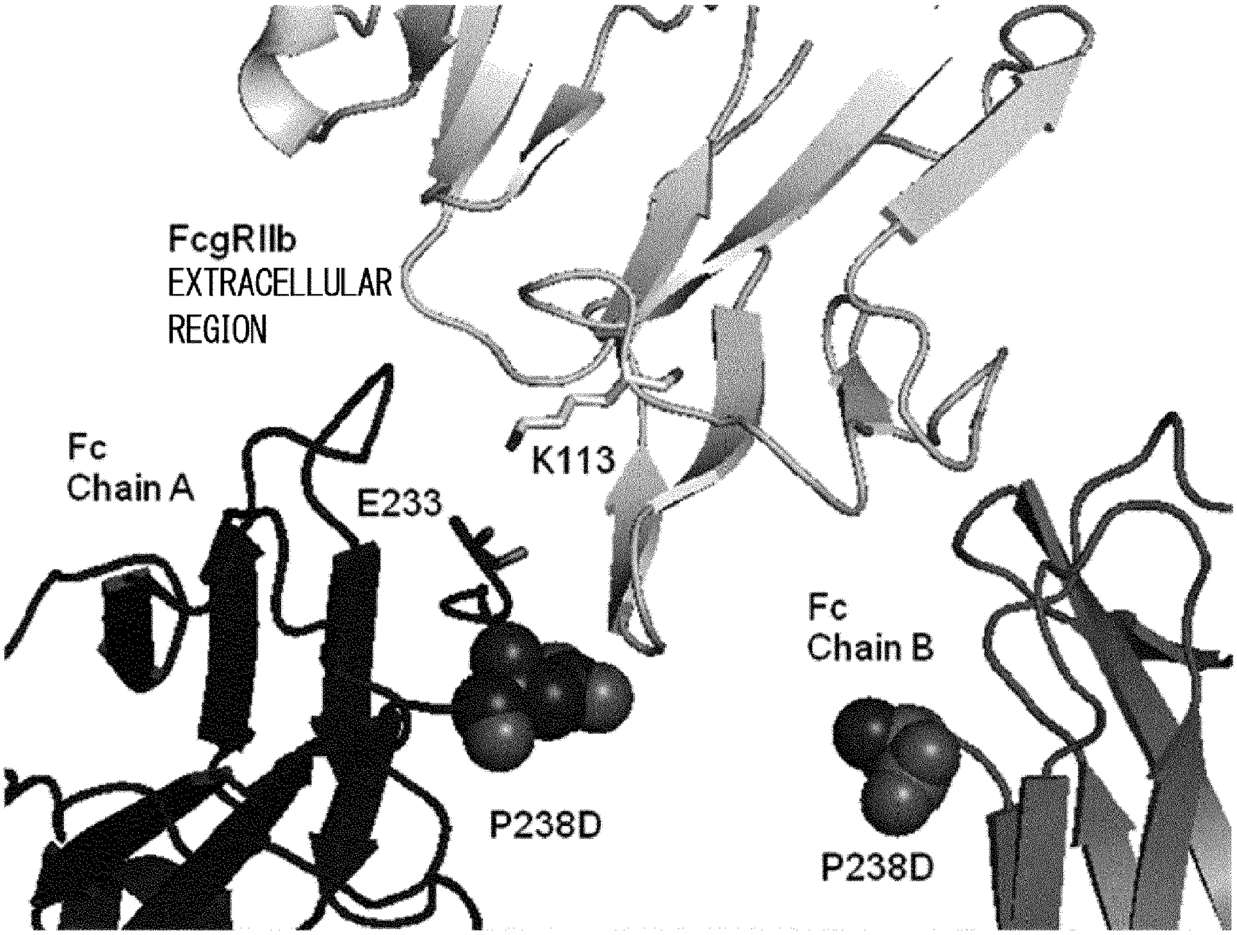
FcgRIIb
EXTRACELLULAR
REGION
Fc
Chain A
E233
K113
Fc
Chain B
P238D
P238D

FIG. 32

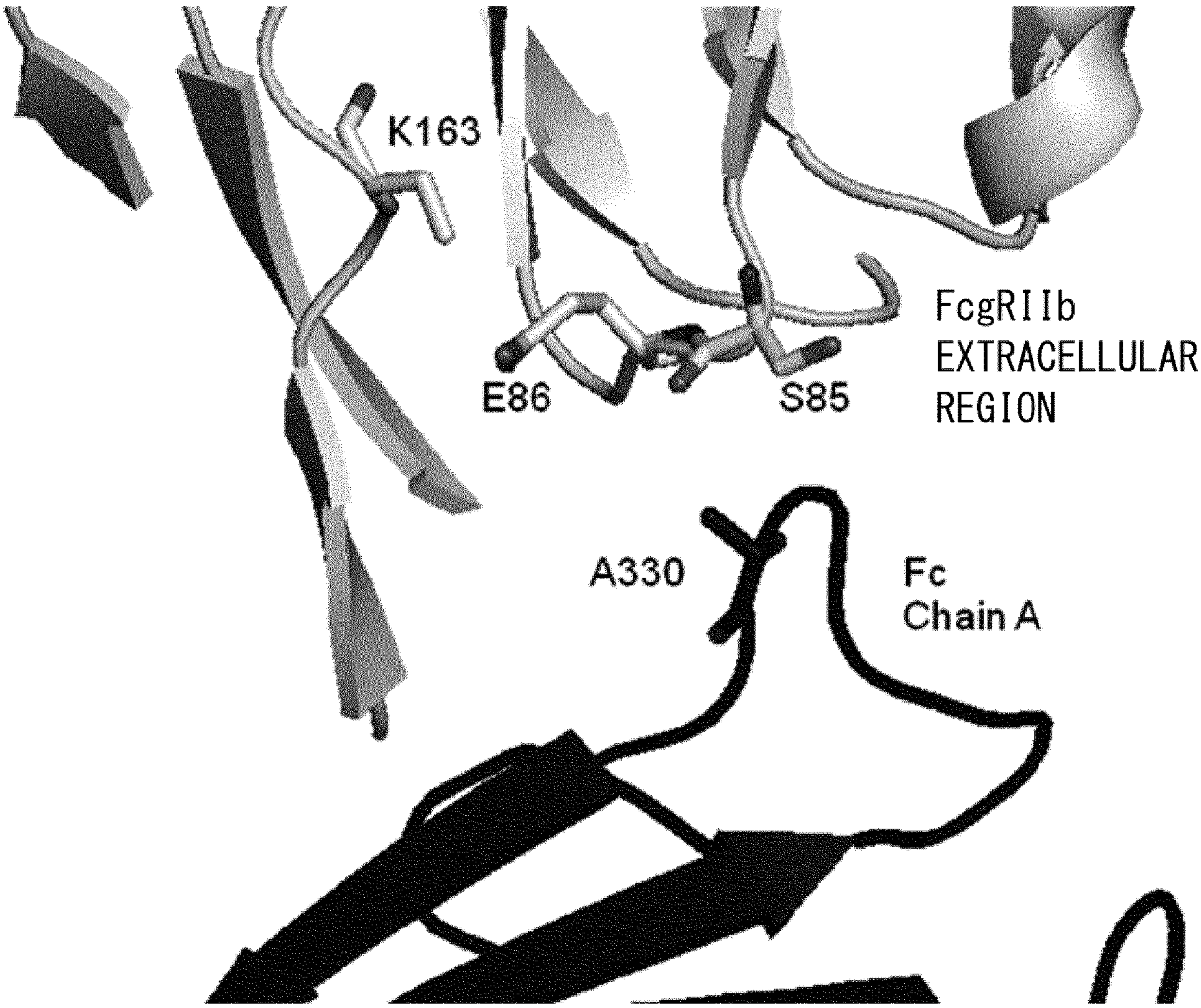


FIG. 33

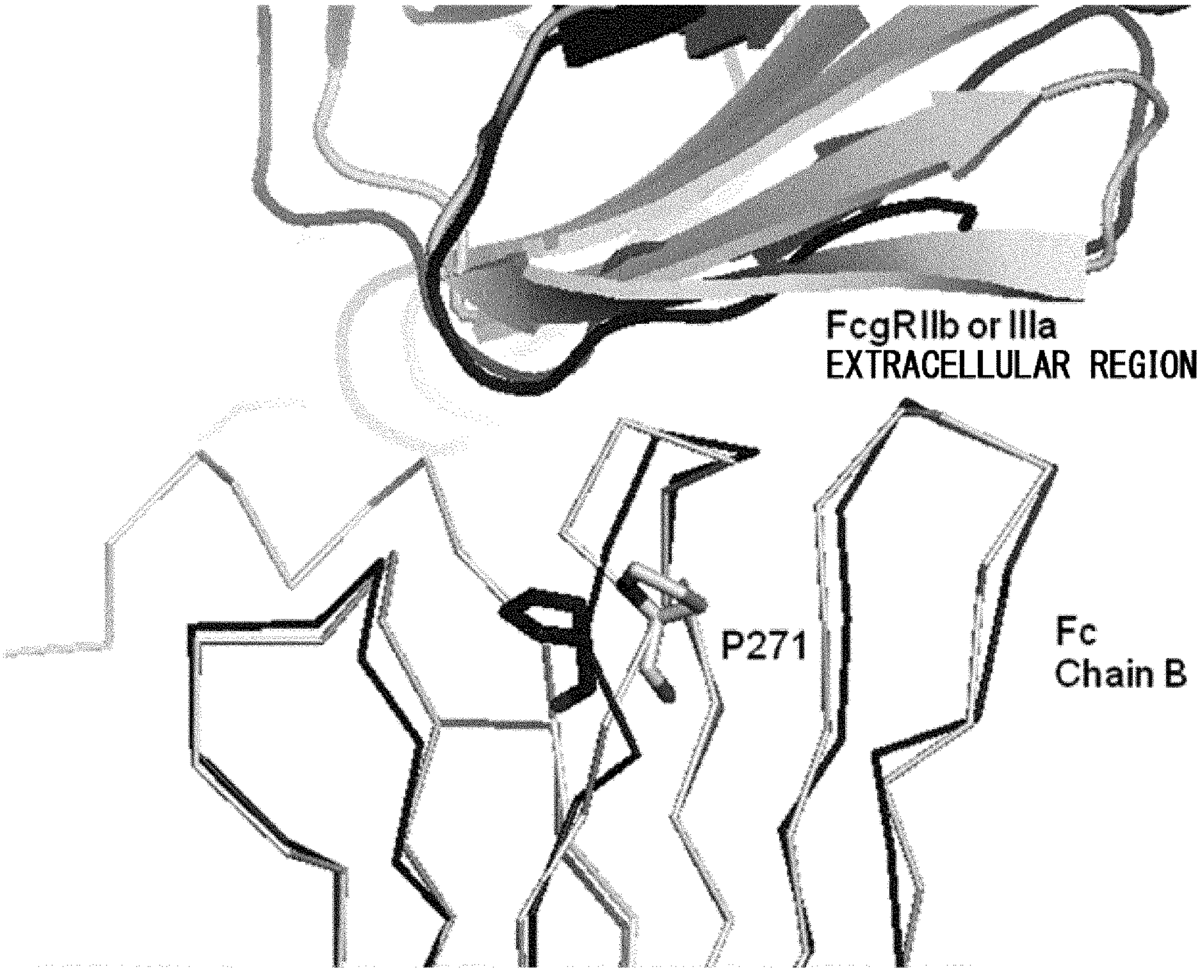


BLACK CRYSTAL STRUCTURE OF Fc(P238D)/FcγRIIb EXTRACELLULAR REGION COMPLEX
GRAY CRYSTAL STRUCTURE OF Fc(WT)/FcγRIIIa EXTRACELLULAR REGION COMPLEX (PDB code: 3SGJ)

FIG. 34

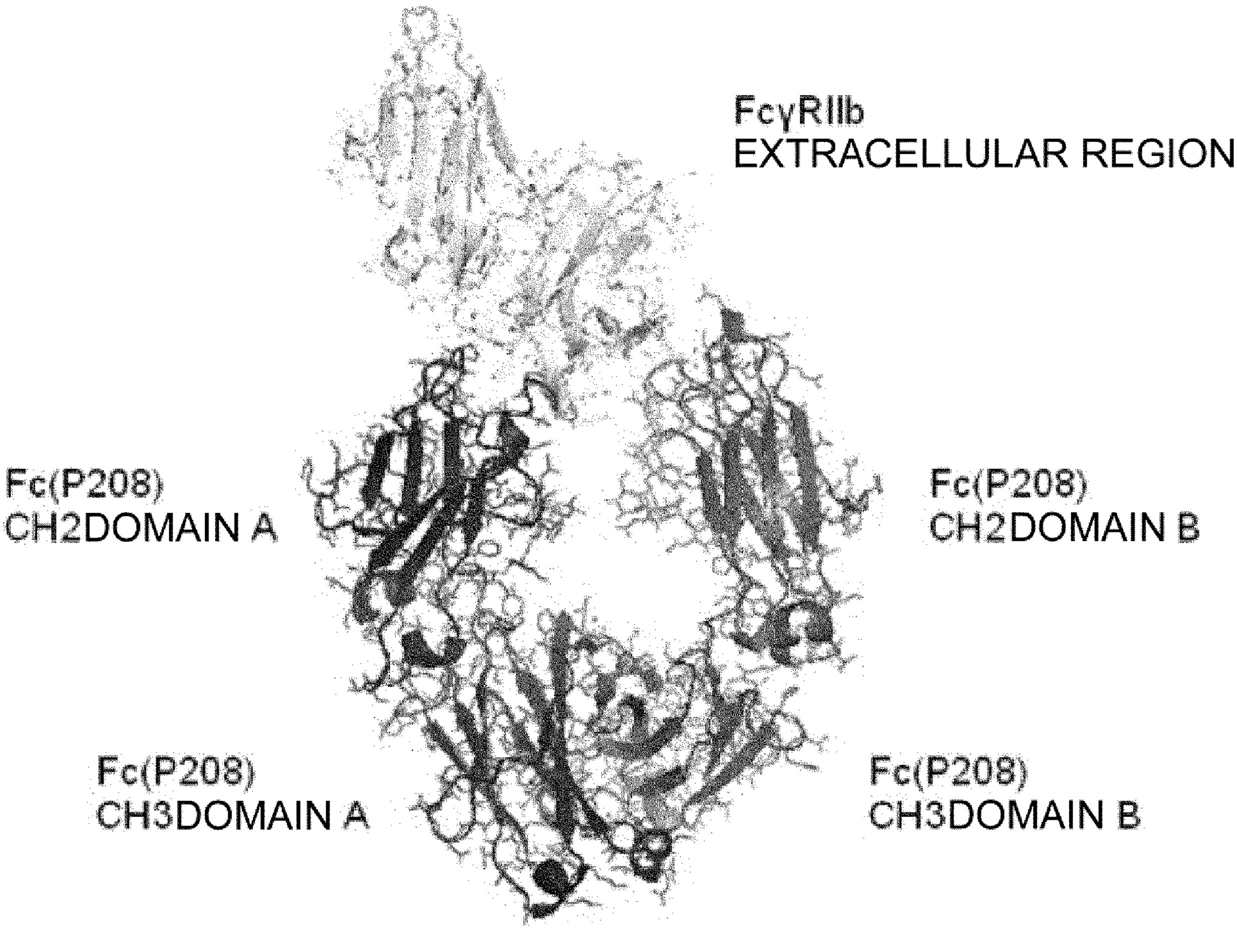
FcγRIIb
EXTRACELLULAR REGION
Fc(P208)
CH2DOMAIN A
Fc(P208)
CH2DOMAIN B
Fc(P208)
CH3DOMAIN A
Fc(P208)
CH3DOMAIN B

FIG. 35

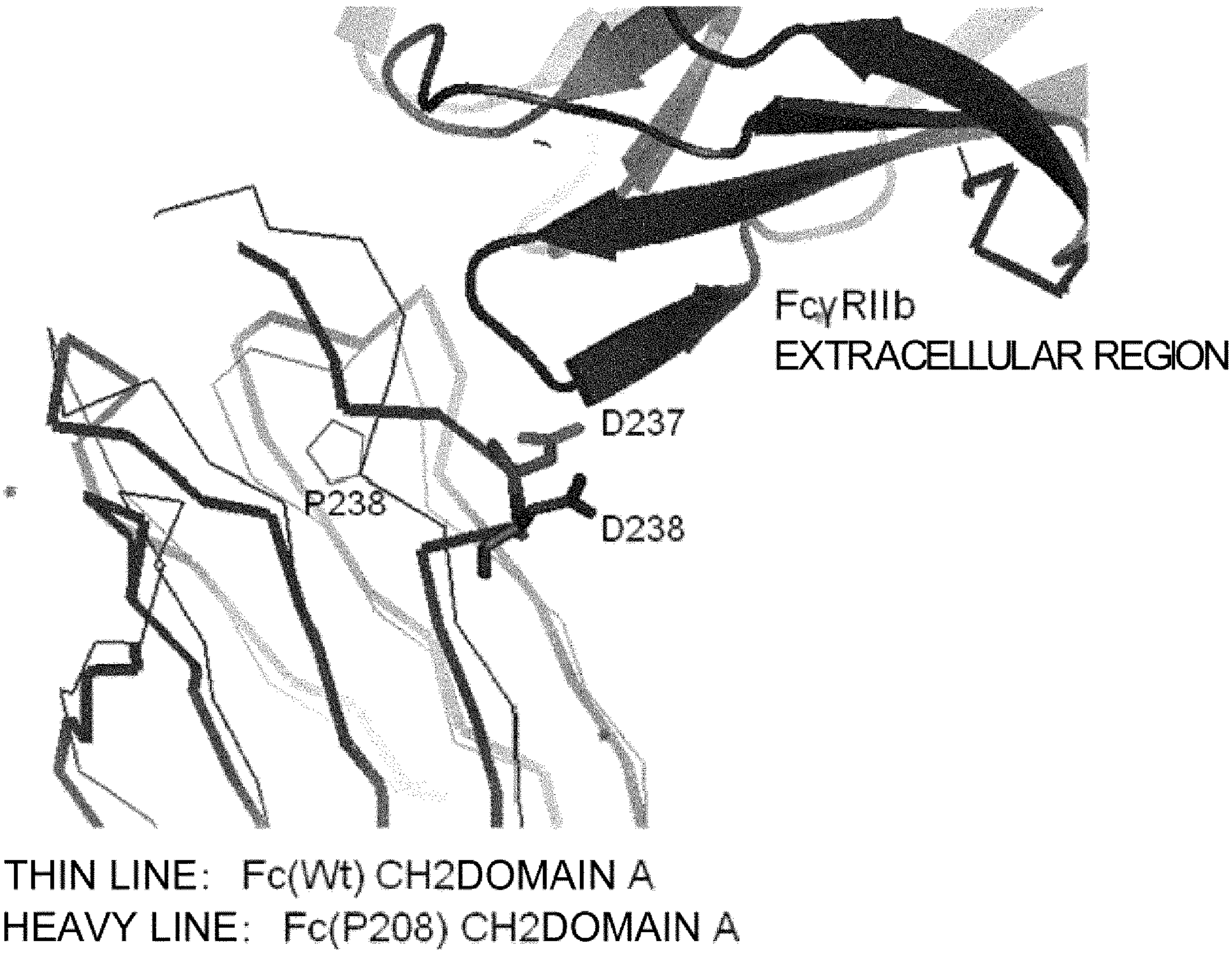
FcγRIIb
EXTRACELLULAR REGION
D237
P238
D238
THIN LINE: Fc(Wt) CH2DOMAIN A
HEAVY LINE: Fc(P208) CH2DOMAIN A

FIG. 36

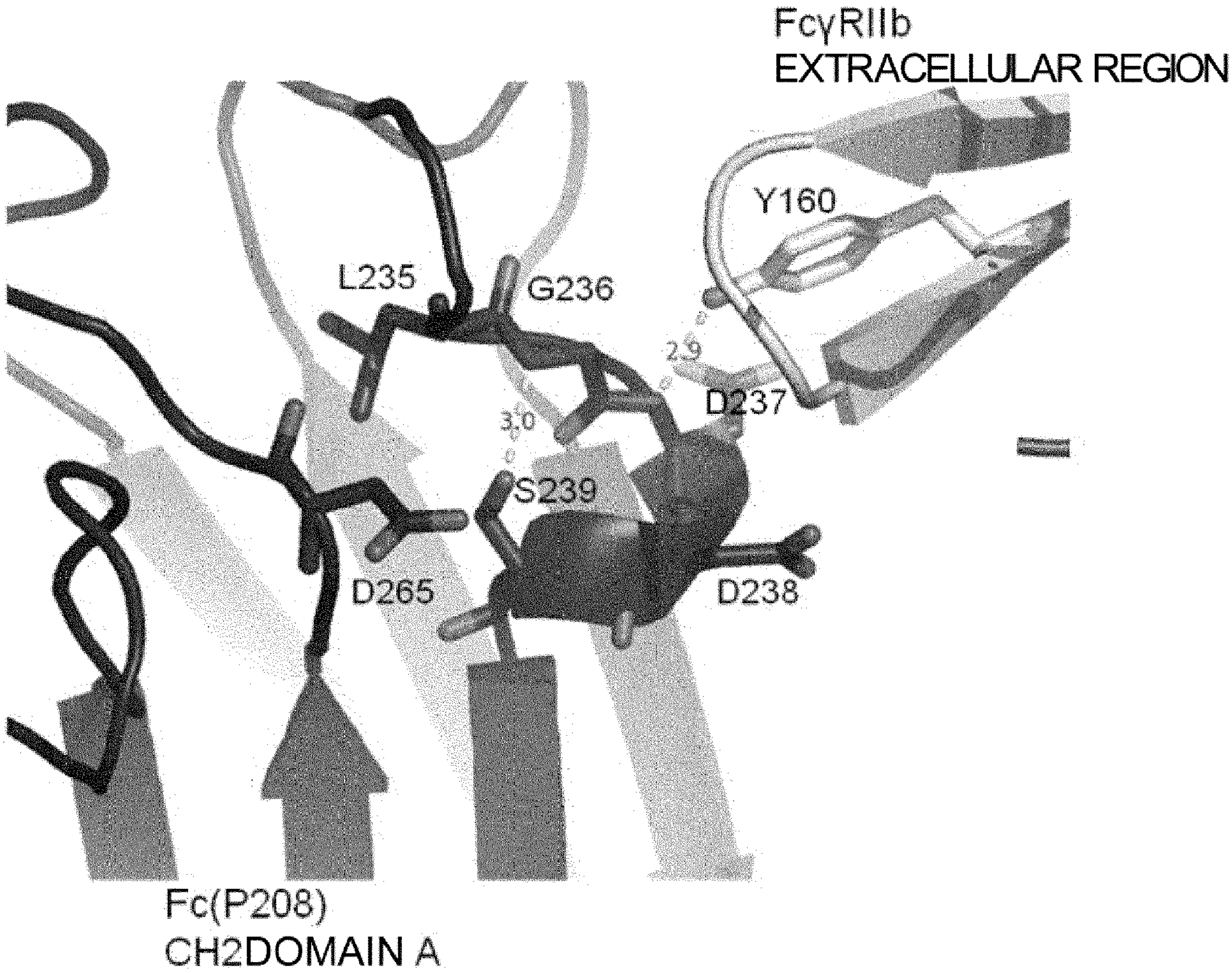
FcγRIIb
EXTRACELLULAR REGION
Y160
L235
G236
2.9
3.0
D237
S239
D265
D238
Fc(P208)
CH2DOMAIN A

FIG. 37

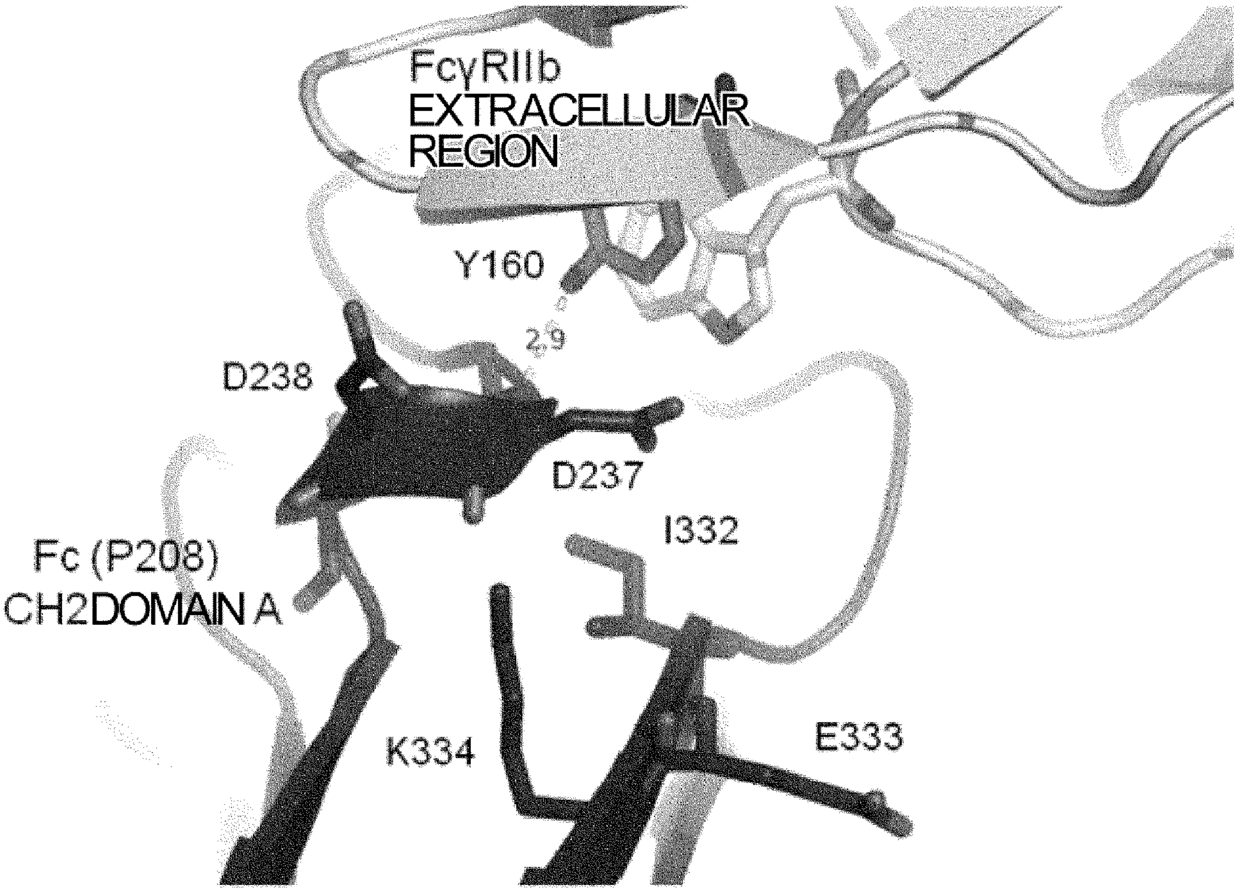
FcγRIIb
EXTRACELLULAR
REGION
Y160
2.9
D238
D237
I332
Fc (P208)
CH2DOMAIN A
K334
E333

FIG. 38

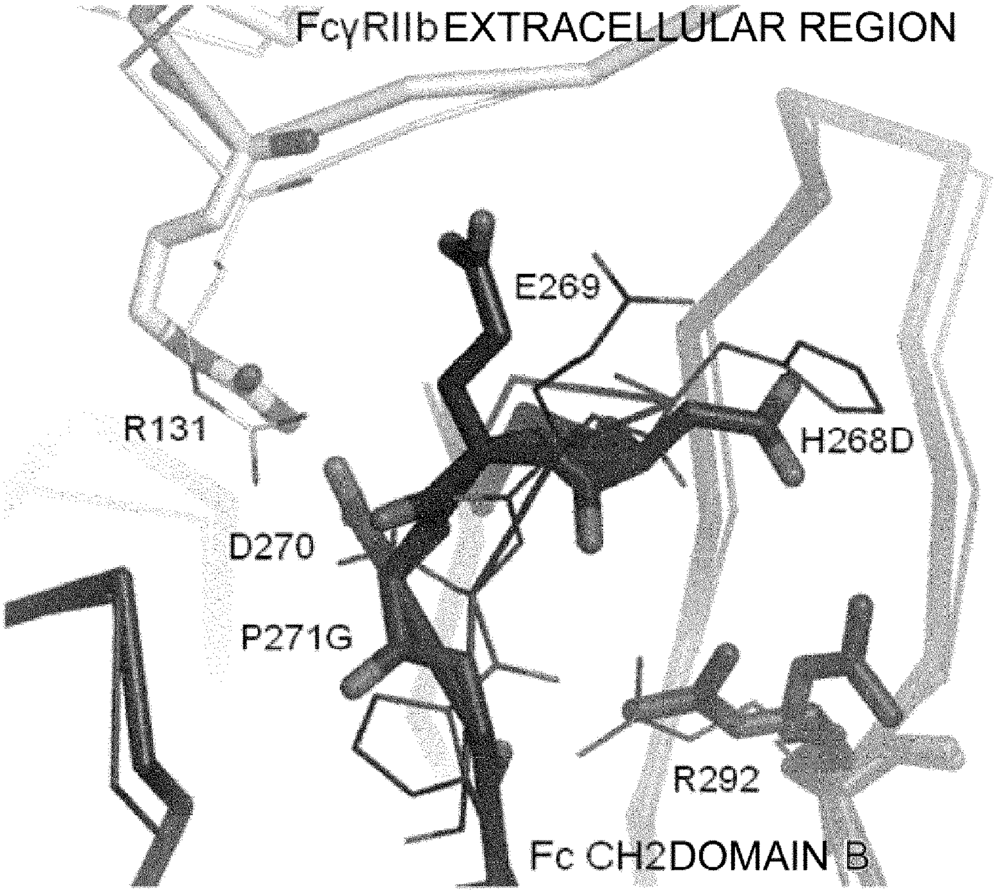


THIN LINE: X-RAY CRYSTAL STRUCTURE OF Fc(P238D)/FcγRIIb EXTRACELLULAR REGION COMPLEX
HEAVY LINE: X-RAY CRYSTAL STRUCTURE OF Fc(P208)/FcγRIIb EXTRACELLULAR REGION COMPLEX

FIG. 39

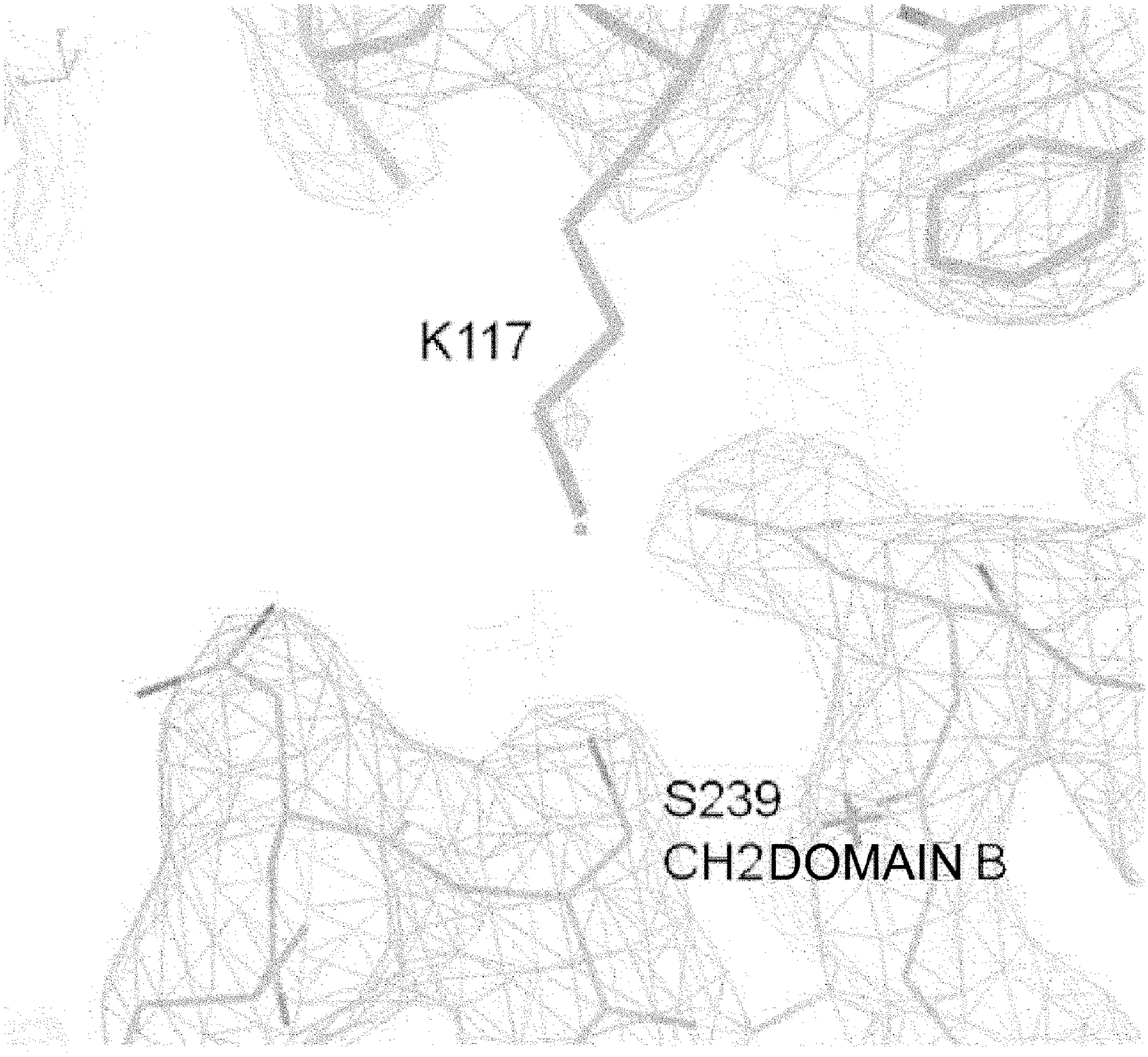


THIN LINE : Fc(P208)
HEAVY LINE : FcγRIIb EXTRACELLULAR REGION

FIG. 40

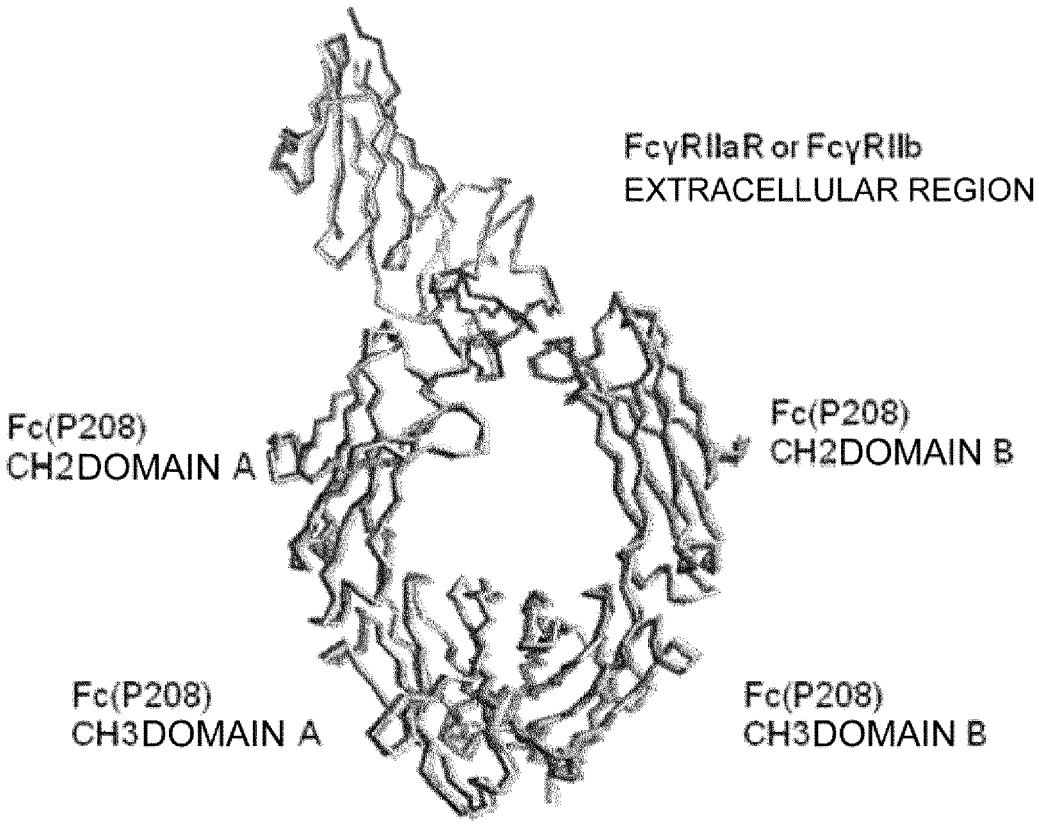


BLACK: X-RAY CRYSTAL STRUCTURE OF Fc(P208)/FcγRIIaR EXTRACELLULAR REGION COMPLEX
GRAY: X-RAY CRYSTAL STRUCTURE OF Fc(P208)/FcγRIIb EXTRACELLULAR REGION COMPLEX

FIG. 41

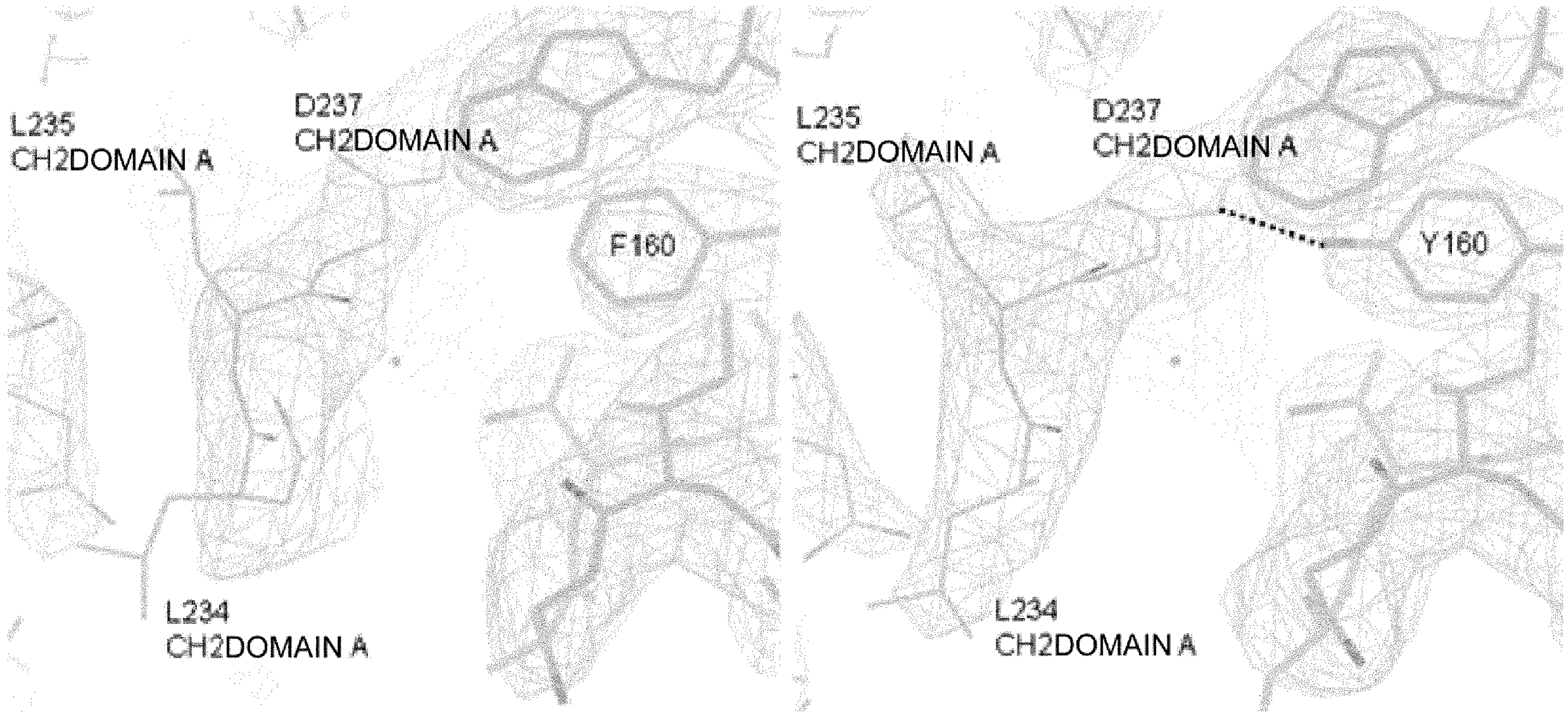


THIN LINE: Fc(P208)
HEAVY LINE: FcγRIIaR or FcγRIIb EXTRACELLULAR REGION
LEFT: X-RAY CRYSTAL STRUCTURE OF Fc(P208)/FcγRIIaR EXTRACELLULAR REGION COMPLEX
RIGHT: X-RAY CRYSTAL STRUCTURE OF Fc(P208)/FcγRIIb EXTRACELLULAR REGION COMPLEX

FIG. 42

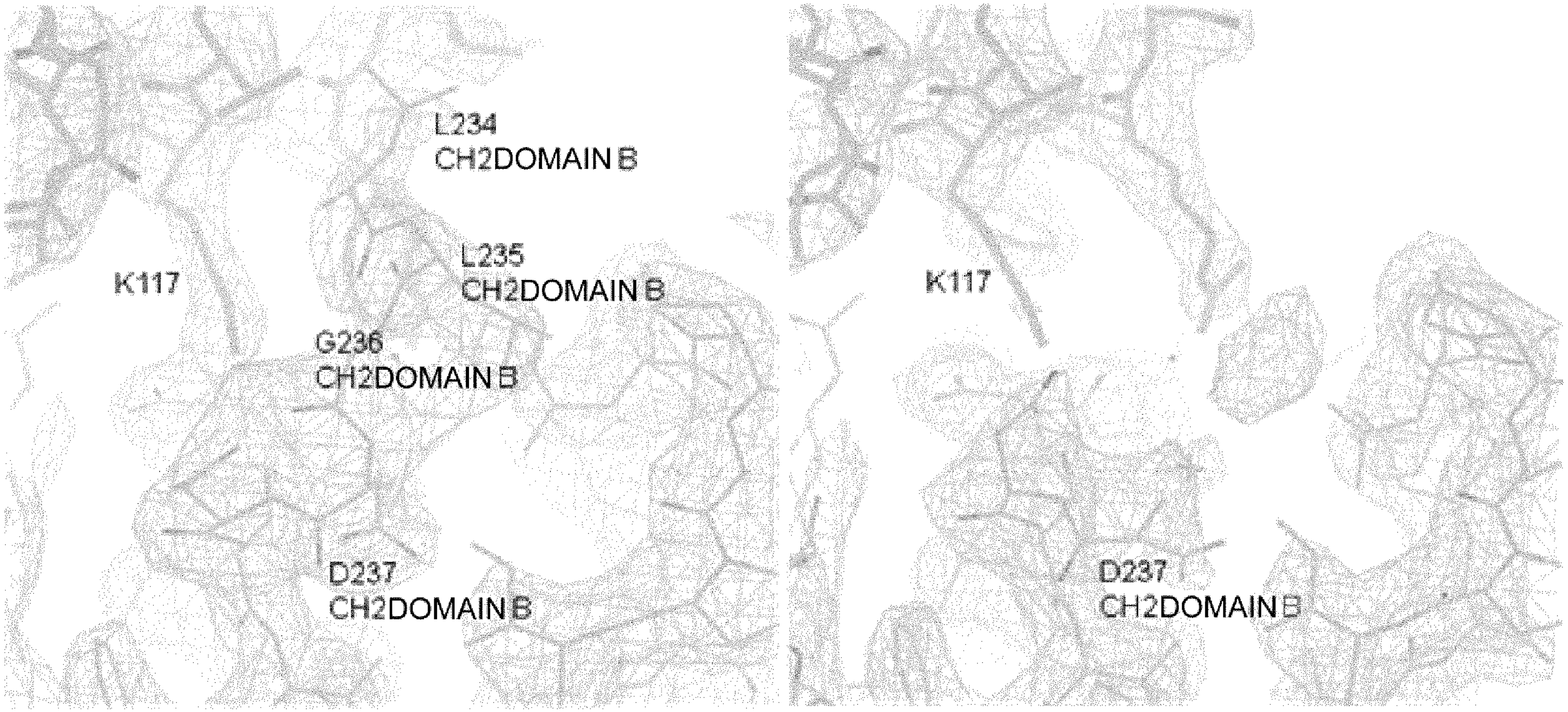


THIN LINE: Fc(P208)
HEAVY LINE: FcγRIIaR or FcγRIIb EXTRACELLULAR REGION

LEFT: X-RAY CRYSTAL STRUCTURE OF Fc(P208)/FcγRIIaR EXTRACELLULAR REGION COMPLEX

RIGHT: X-RAY CRYSTAL STRUCTURE OF Fc(P208)/FcγRIIb EXTRACELLULAR REGION COMPLEX

FIG. 43

```
G1d  118 ASTKGPSVFPLAPSSKSTSGGTAALGCLVKDYFPEPVTVSWNSGALTSGVHTFPAVLQSSGLYSLSSVVTVPSSSLGTQTYICNVNHKPS
G4d  118 ASTKGPSVFPLAPCSRSTSESTAALGCLVKDYFPEPVTVSWNSGALTSGVHTFPAVLQSSGLYSLSSVVTVPSSSLGTKTYTCNVDHKPS

G1d  208 NTKVDKKVEPKSCDKTHTCPPCPAPELLGGPSVFLFPPKPKDTLMISRTPEVTCVVVDVSHEDPEVKFNWYVDGVEVHNAKTKPREEQYN
G4d  208 NTKVDKRVESK---YGPPCPPCPAPEFLGGPSVFLFPPKPKDTLMISRTPEVTCVVVDVSQEDPEVQFNWYVDGVEVHNAKTKPREEQFN

G1d  298 STYRVVSVLTVLHQDWLNGKEYKCKVSNKALPAPIEKTISKAKGQPREPQVYTLPPSRDELTKNQVSLTCLVKGFYPSDIAVEWESNGQP
G4d  298 STYRVVSVLTVLHQDWLNGKEYKCKVSNKGLPSSIEKTISKAKGQPREPQVYTLPPSQEEMTKNQVSLTCLVKGFYPSDIAVEWESNGQP

G1d  388 ENNYKTTPPVLDSDGSFFLYSKLTVDKSRWQQGNVFSCSVMHEALHNHYTQKSLSLSP (SEQ ID NO : 161)
G4d  388 ENNYKTTPPVLDSDGSFFLYSRLTVDKSRWQEGNVFSCSVMHEALHNHYTQKSLSLSL (SEQ ID NO : 162)
```

FIG. 44

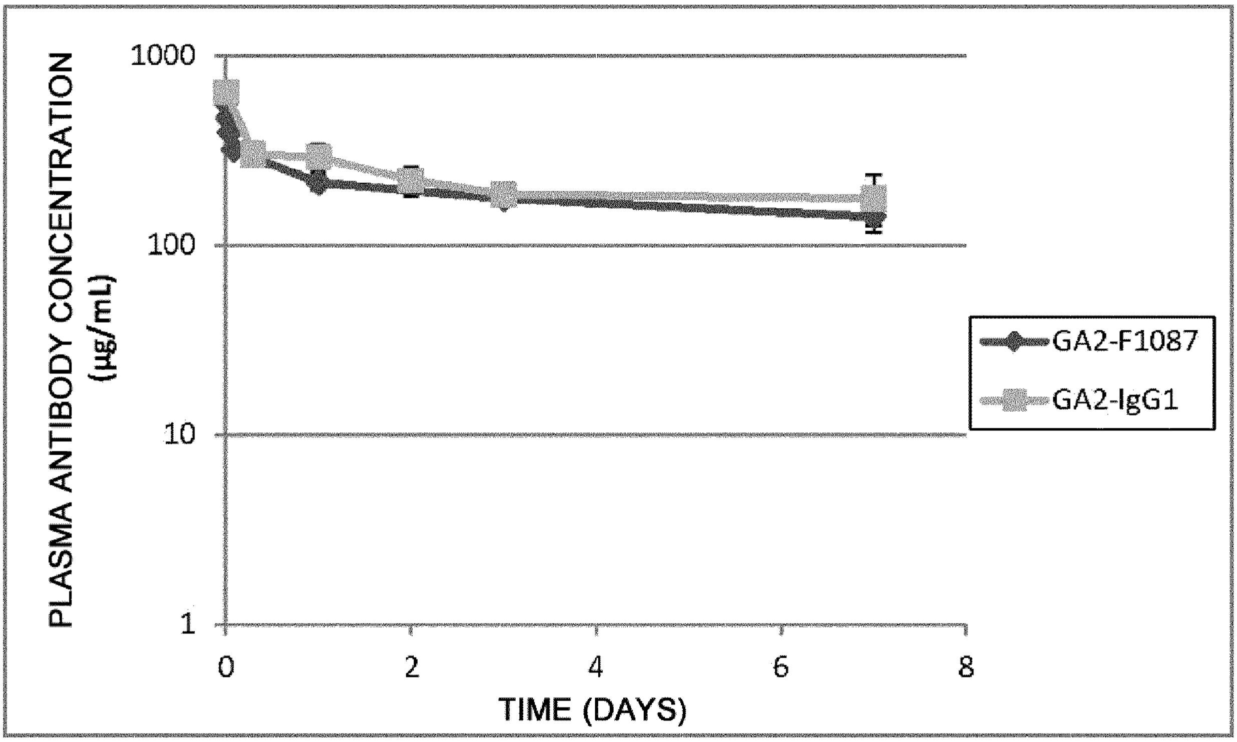
PLASMA ANTIBODY CONCENTRATION (µg/mL)
1000
100
10
1
0
2
4
6
8
TIME (DAYS)
GA2-F1087
GA2-IgG1

FIG. 45

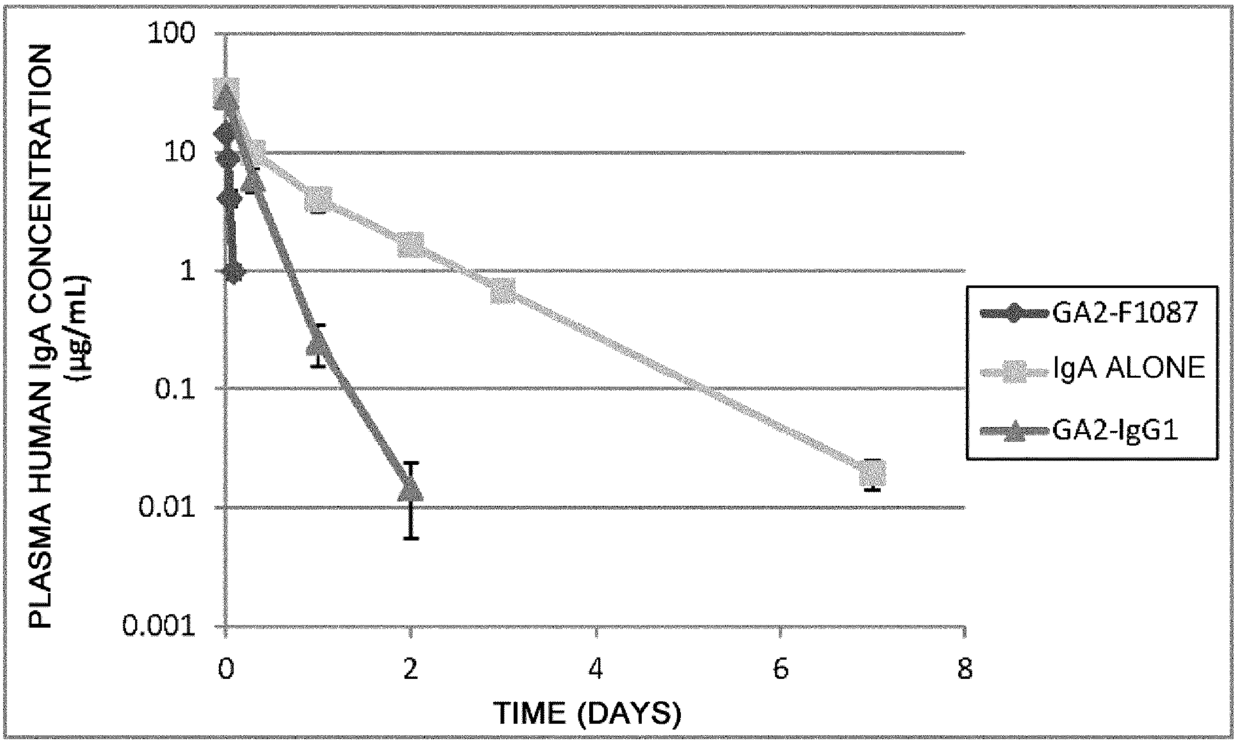
PLASMA HUMAN IgA CONCENTRATION (µg/mL)
100
10
1
0.1
0.01
0.001
0
2
4
6
8
TIME (DAYS)
GA2-F1087
IgA ALONE
GA2-IgG1

FIG. 46

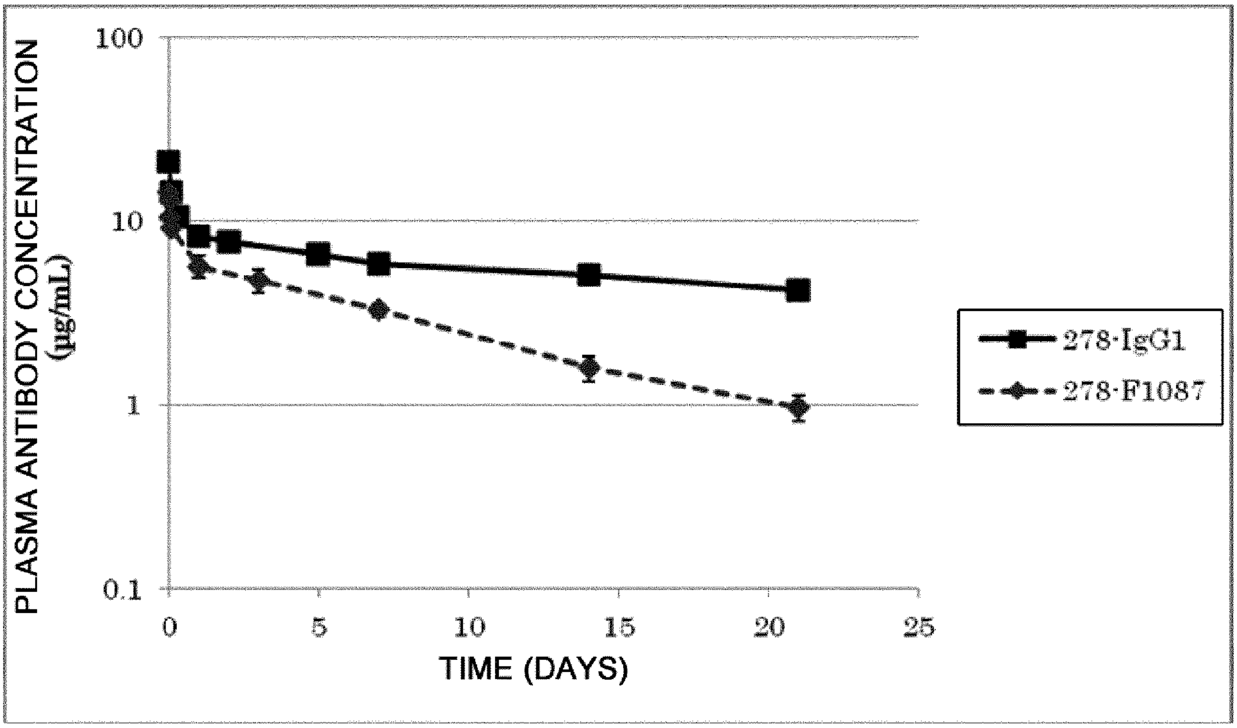

100
10
1
0.1
PLASMA ANTIBODY CONCENTRATION (μg/mL)
0
5
10
15
20
25
TIME (DAYS)
278-IgG1
278-F1087


FIG. 47

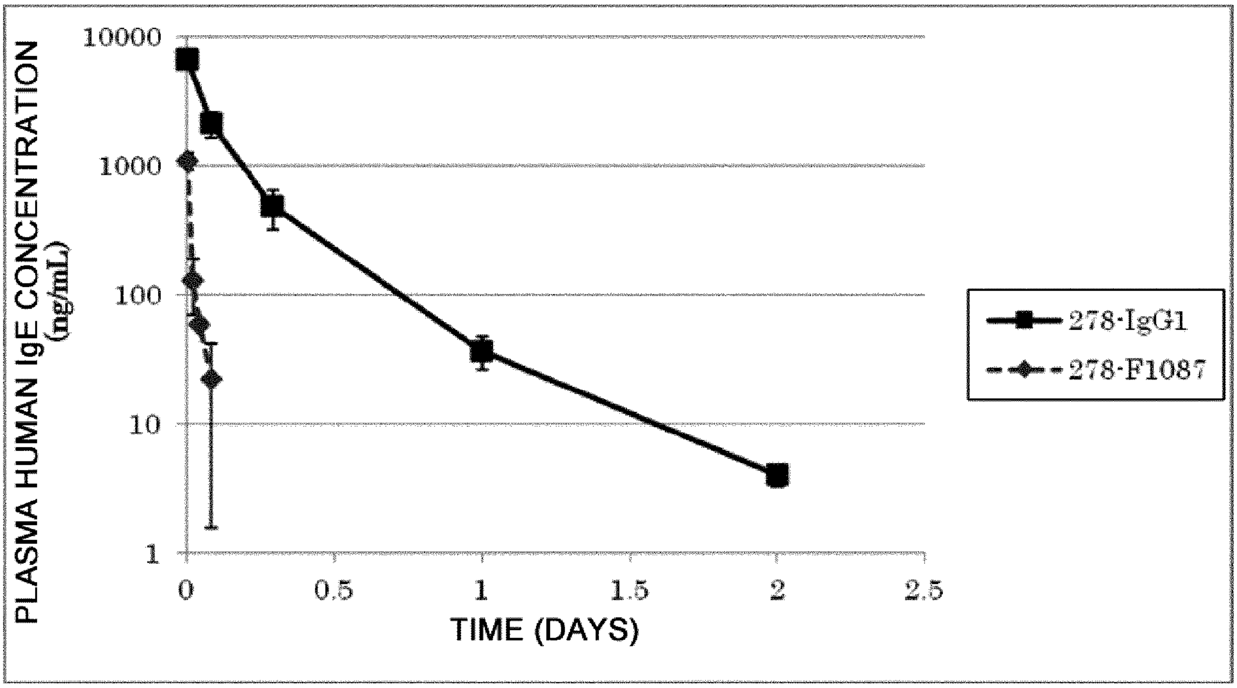
PLASMA HUMAN IgE CONCENTRATION (ng/mL)
10000
1000
100
10
1
0
0.5
1
1.5
2
2.5
TIME (DAYS)
278-IgG1
278-F1087

FIG. 48

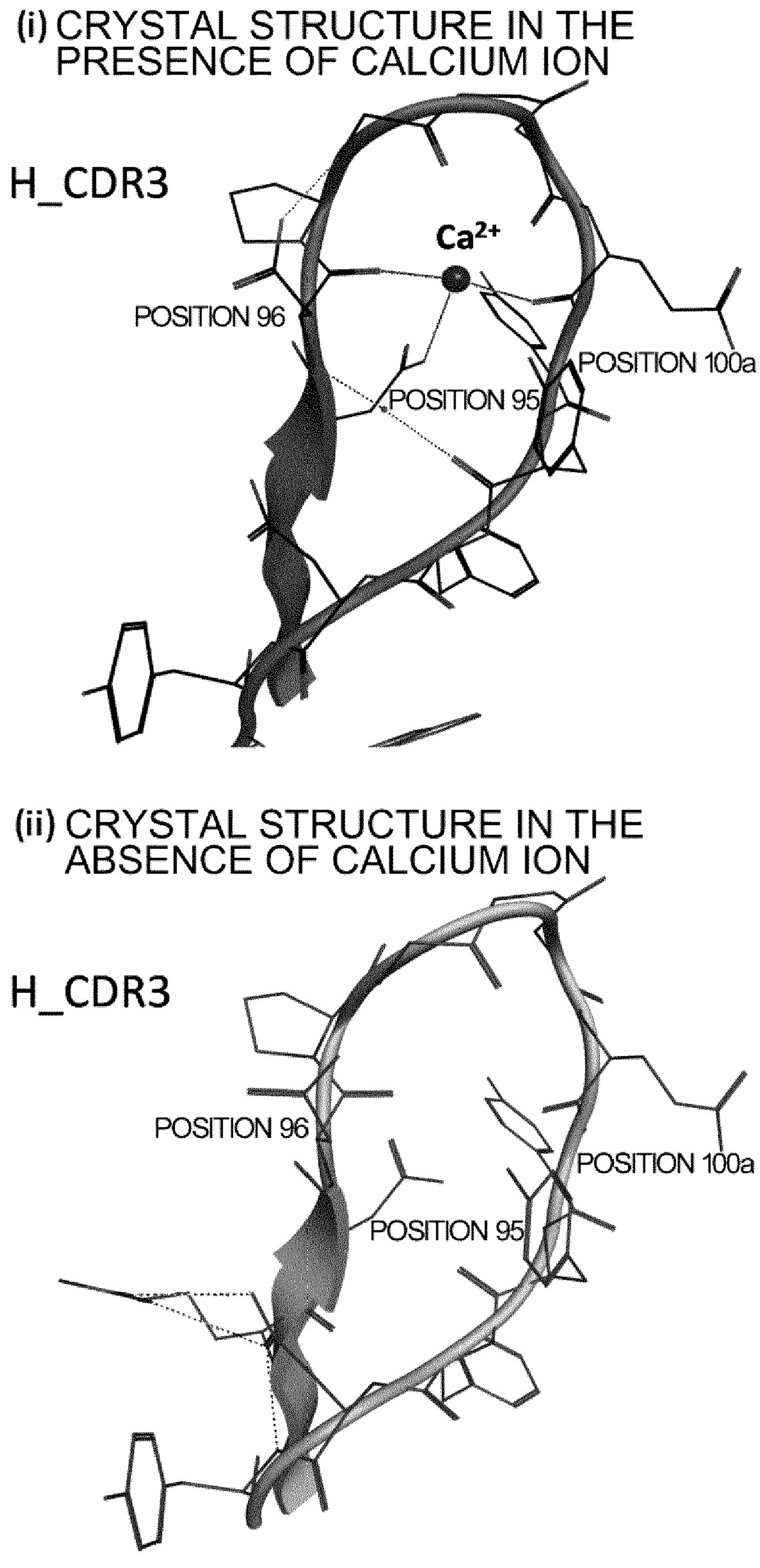
(i) CRYSTAL STRUCTURE IN THE PRESENCE OF CALCIUM ION
H_CDR3
Ca2+
POSITION 96
POSITION 100a
POSITION 95
(ii) CRYSTAL STRUCTURE IN THE ABSENCE OF CALCIUM ION
H_CDR3
POSITION 96
POSITION 100a
POSITION 95

FIG. 49

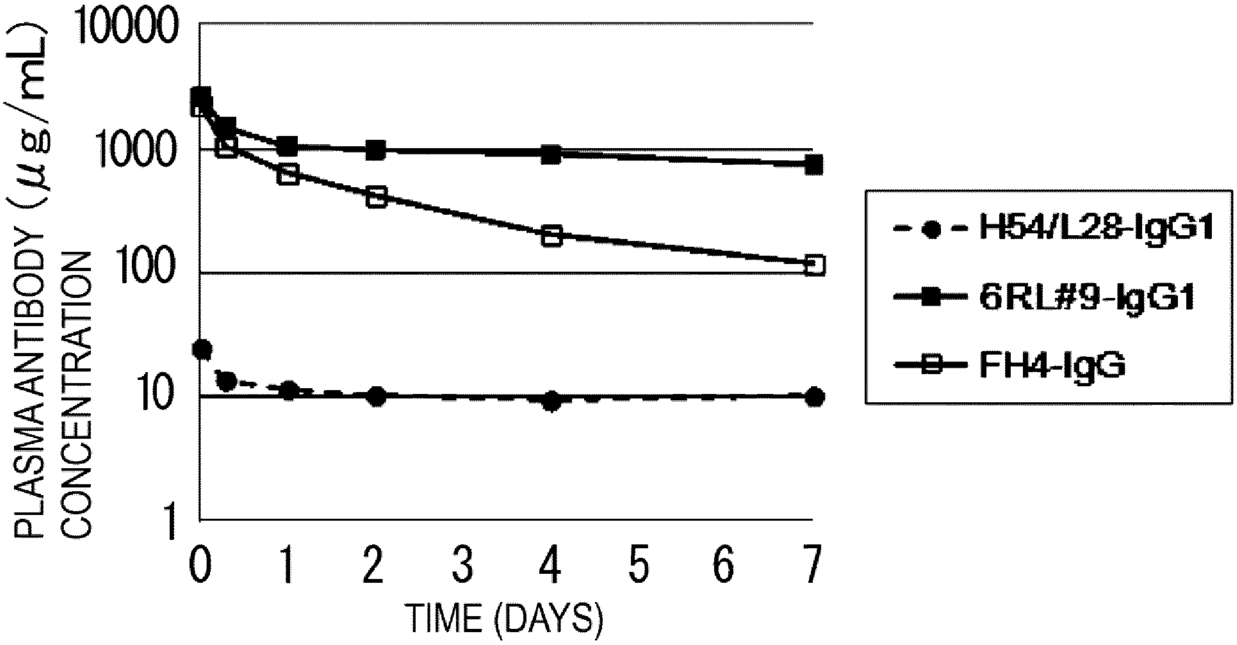
10000
1000
100
10
1
PLASMA ANTIBODY CONCENTRATION (μg/mL)
0
1
2
3
4
5
6
7
TIME (DAYS)
H54/L28-IgG1
6RL#9-IgG1
FH4-IgG

FIG. 50

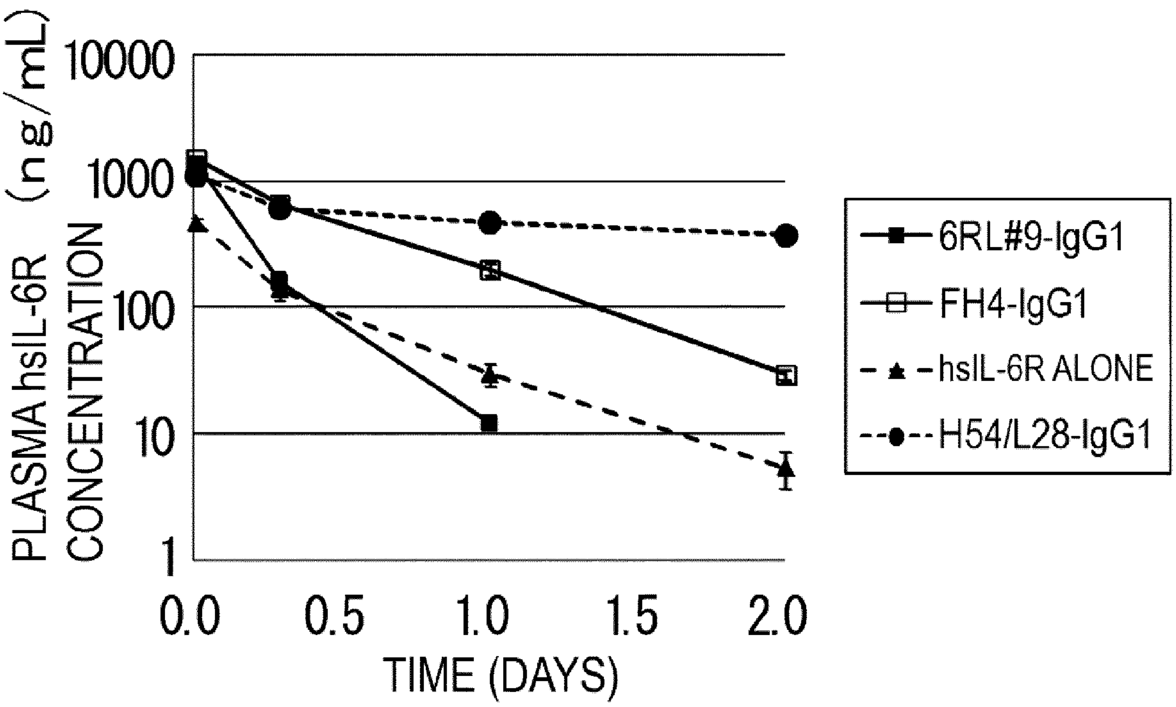
PLASMA hsIL-6R CONCENTRATION (ng/mL)
10000
1000
100
10
1
0.0
0.5
1.0
1.5
2.0
TIME (DAYS)
6RL#9-IgG1
FH4-IgG1
hsIL-6R ALONE
H54/L28-IgG1

FIG. 51

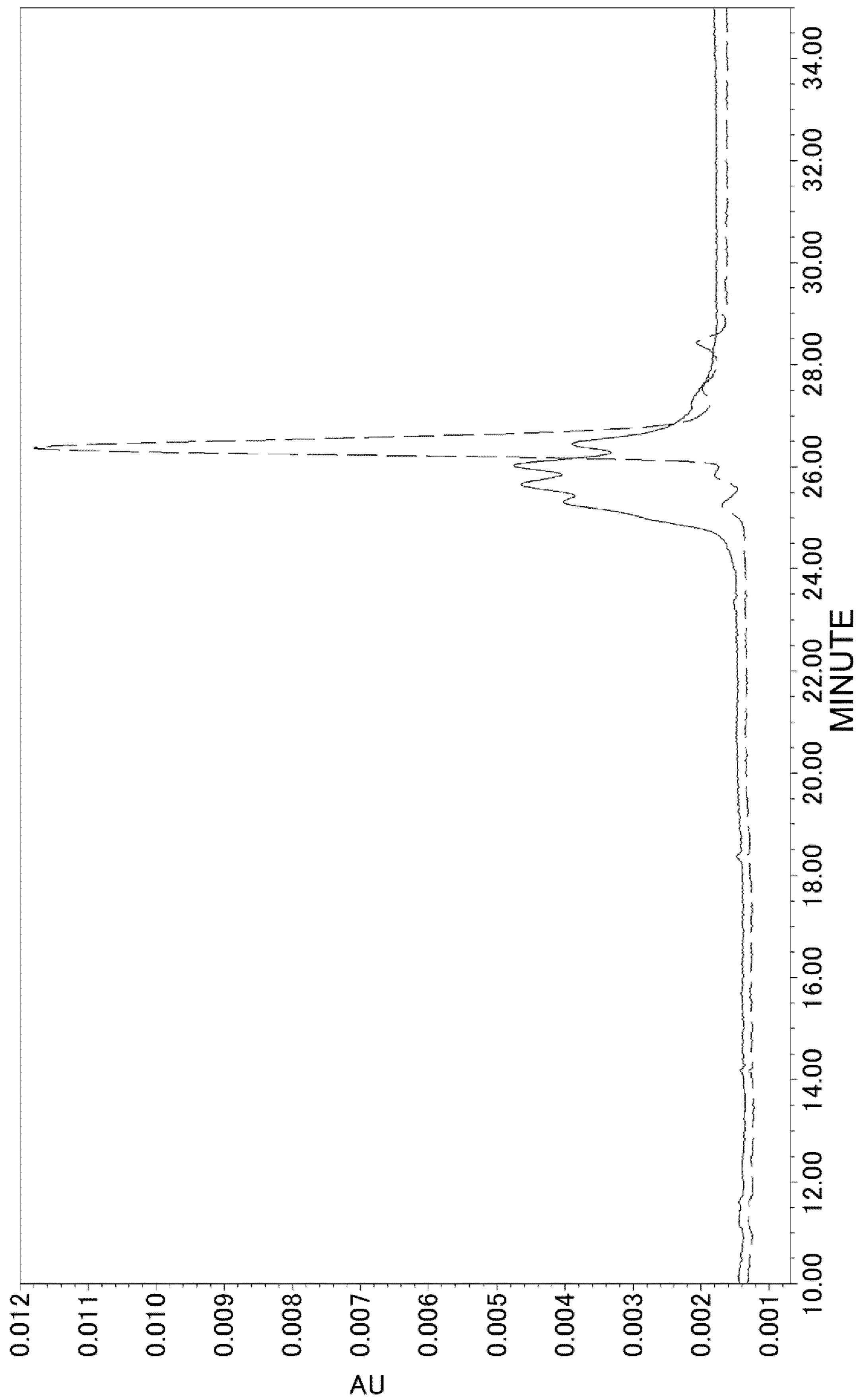
AU
0.012
0.011
0.010
0.009
0.008
0.007
0.006
0.005
0.004
0.003
0.002
0.001
MINUTE
10.00
12.00
14.00
16.00
18.00
20.00
22.00
24.00
26.00
28.00
30.00
32.00
34.00

FIG. 52

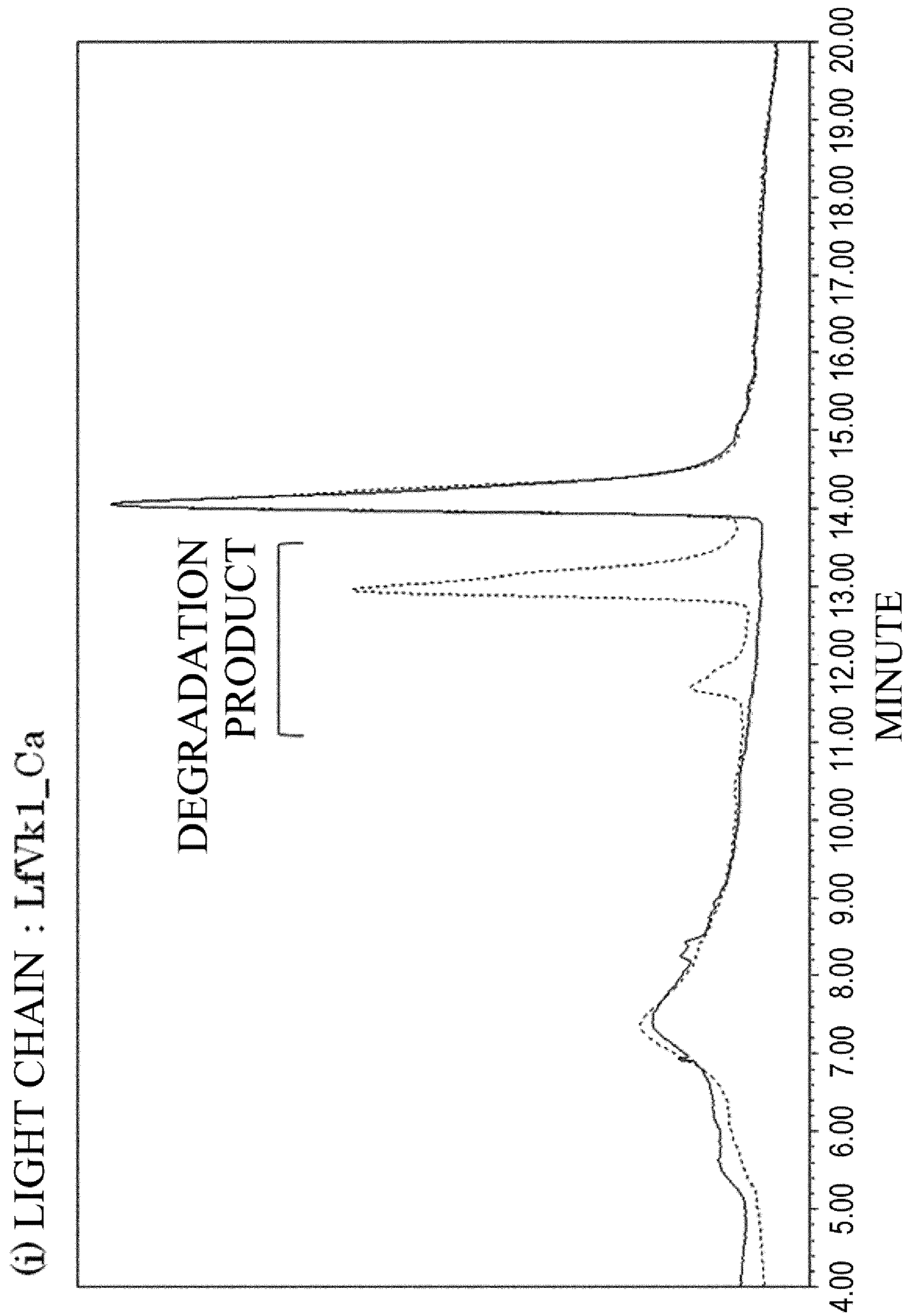
(i) LIGHT CHAIN : LfVk1_Ca
DEGRADATION PRODUCT
4.00
5.00
6.00
7.00
8.00
9.00
10.00
11.00
12.00
13.00
14.00
15.00
16.00
17.00
18.00
19.00
20.00
MINUTE

FIG. 53A

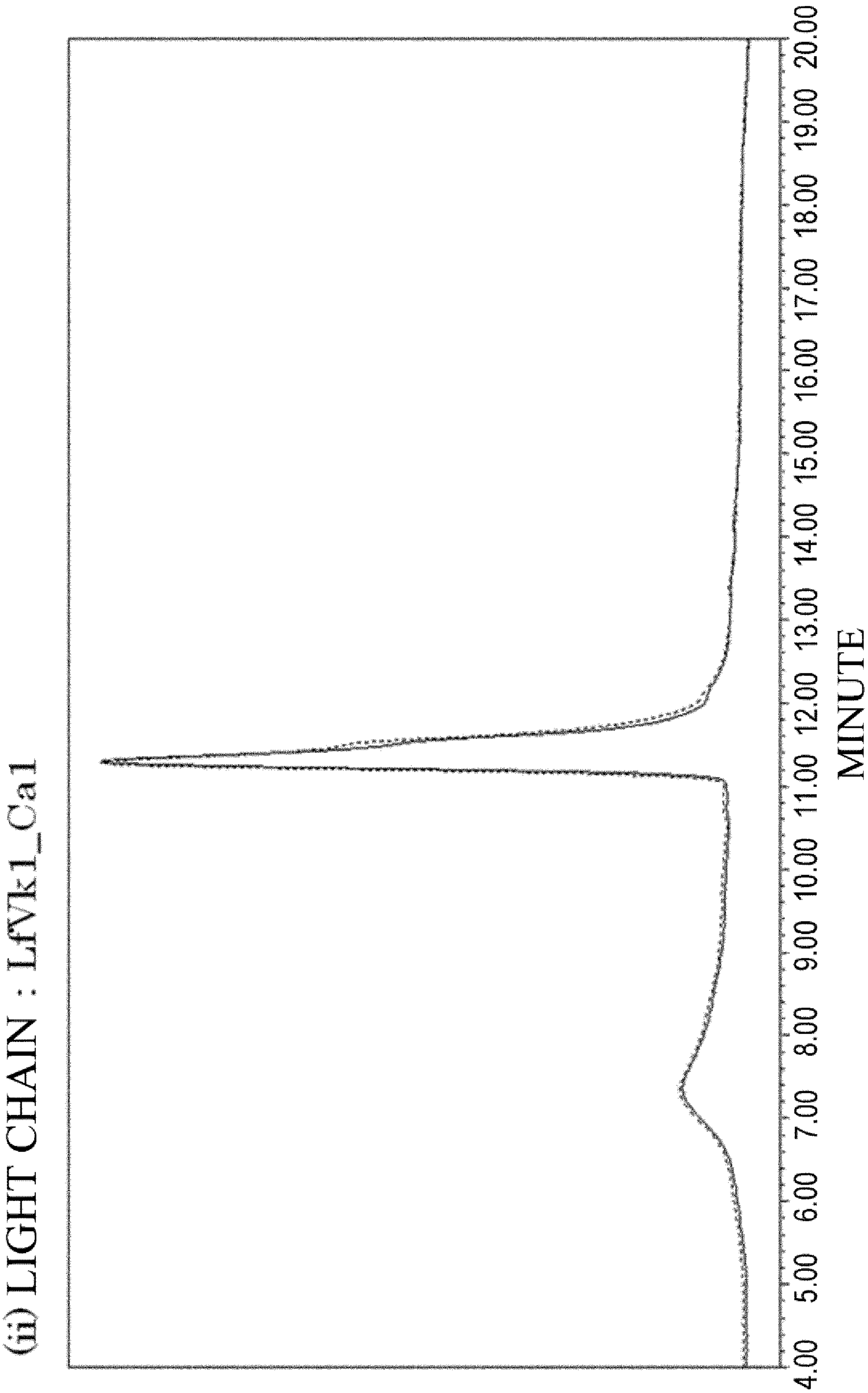
(ii) LIGHT CHAIN : LfVk1_Ca1
4.00
5.00
6.00
7.00
8.00
9.00
10.00
11.00
12.00
13.00
14.00
15.00
16.00
17.00
18.00
19.00
20.00
MINUTE

FIG. 53B

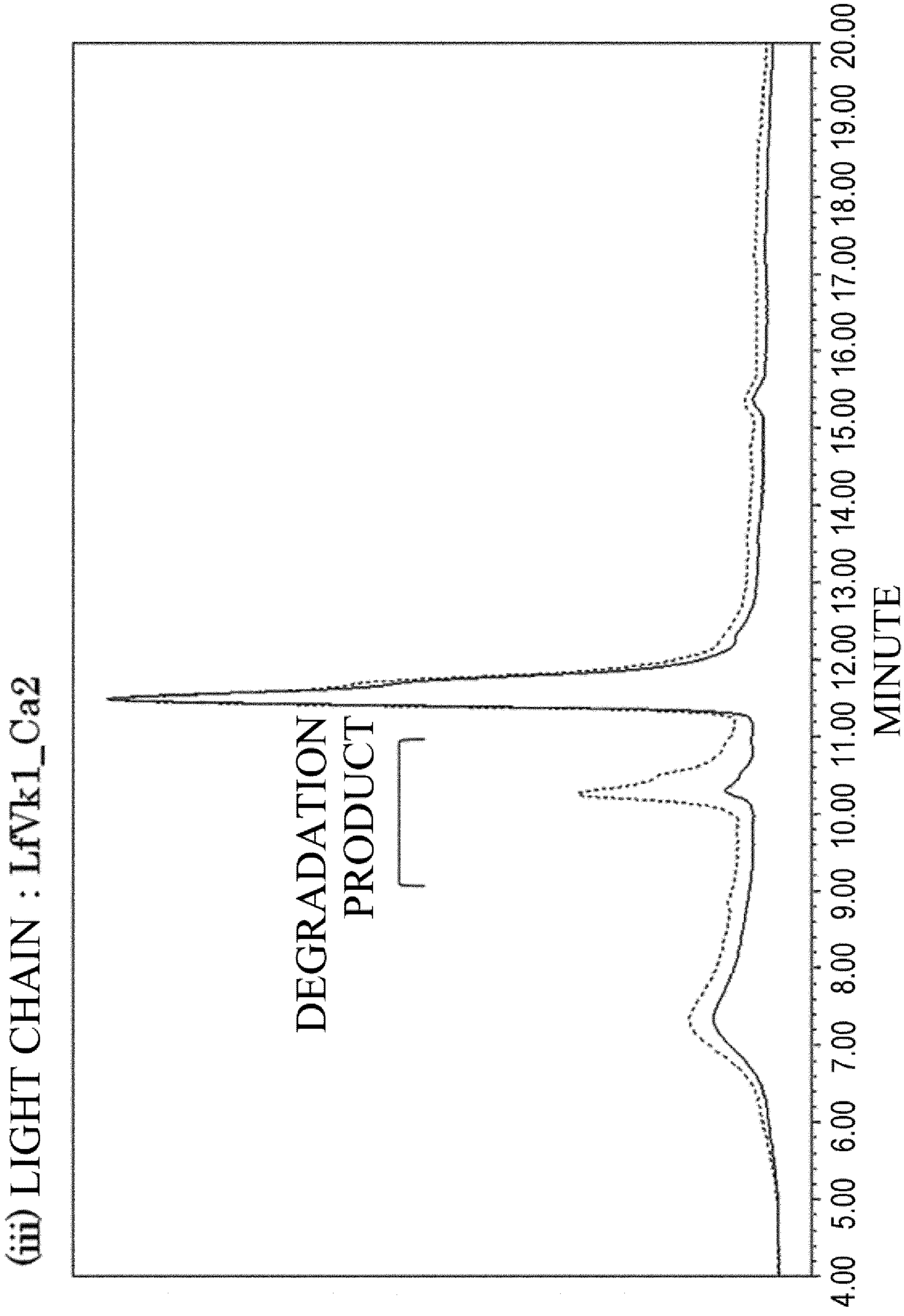
(iii) LIGHT CHAIN : LfVk1_Ca2
DEGRADATION PRODUCT
4.00 5.00 6.00 7.00 8.00 9.00 10.00 11.00 12.00 13.00 14.00 15.00 16.00 17.00 18.00 19.00 20.00
MINUTE

FIG. 53C

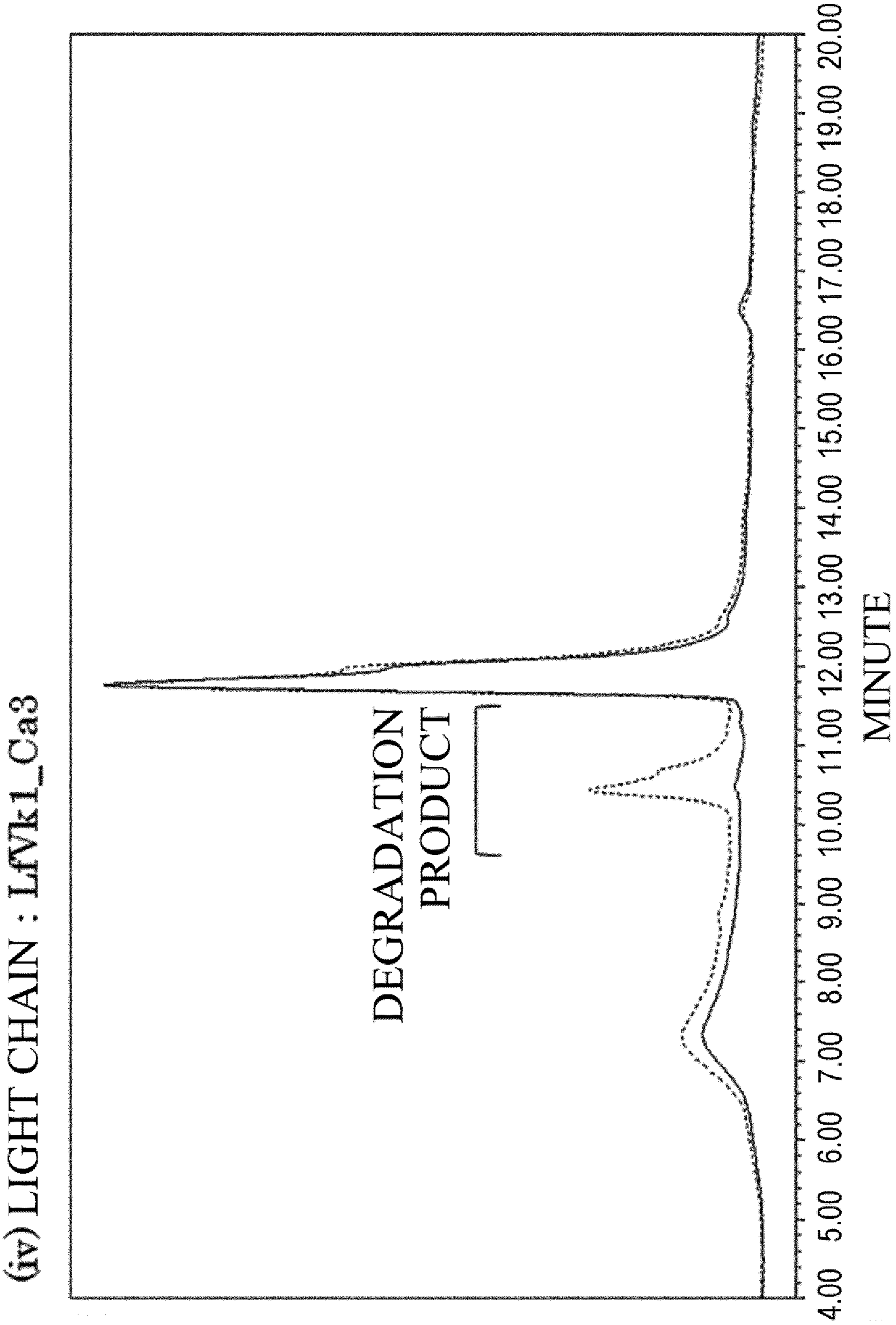
(iv) LIGHT CHAIN : LfVk1_Ca3
DEGRADATION PRODUCT
4.00 5.00 6.00 7.00 8.00 9.00 10.00 11.00 12.00 13.00 14.00 15.00 16.00 17.00 18.00 19.00 20.00
MINUTE

FIG. 53D

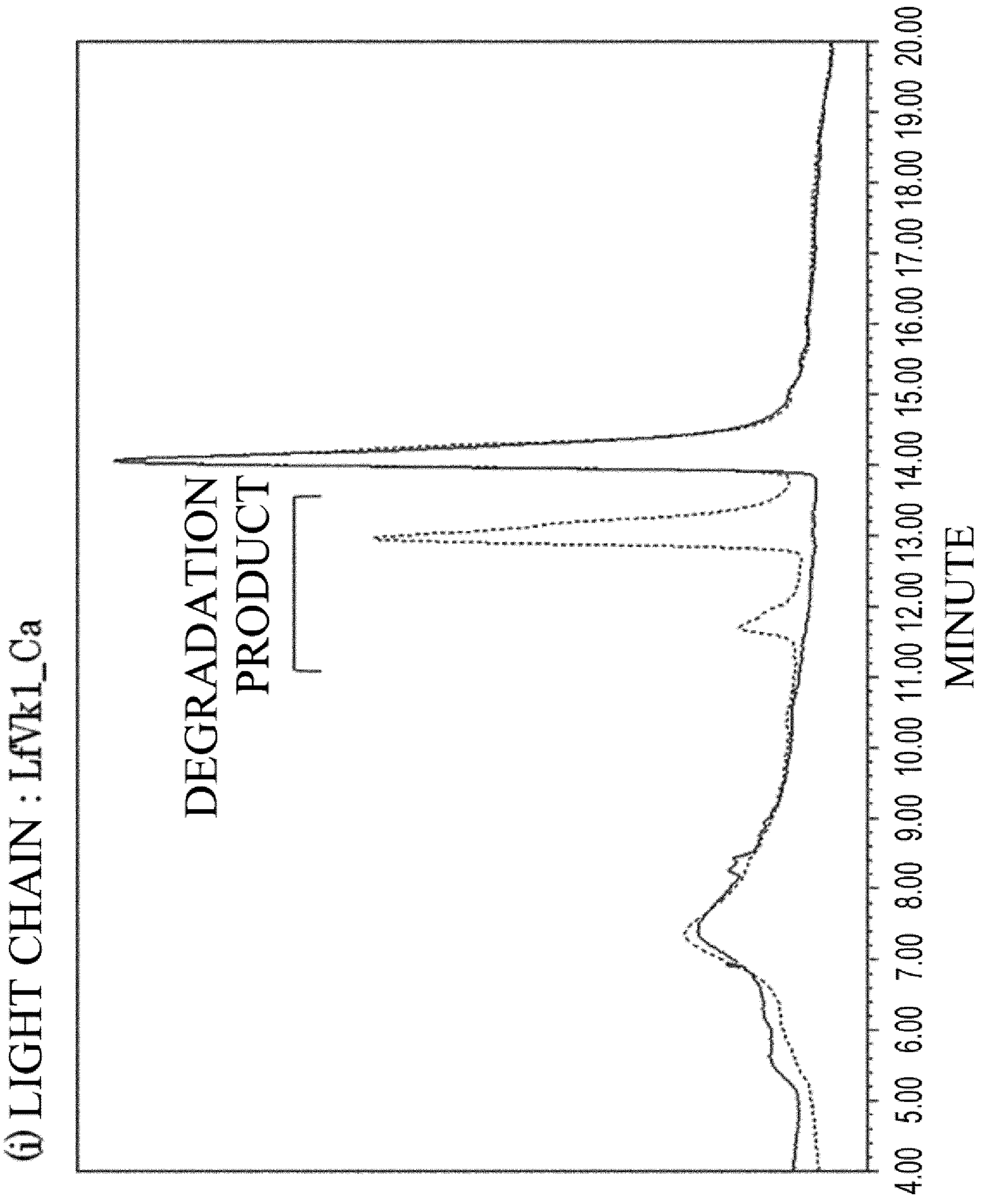
(i) LIGHT CHAIN : LfVk1_Ca
DEGRADATION
PRODUCT
MINUTE
4.00
5.00
6.00
7.00
8.00
9.00
10.00
11.00
12.00
13.00
14.00
15.00
16.00
17.00
18.00
19.00
20.00

FIG. 54A

(ii) LIGHT CHAIN : LfVk1_Ca6

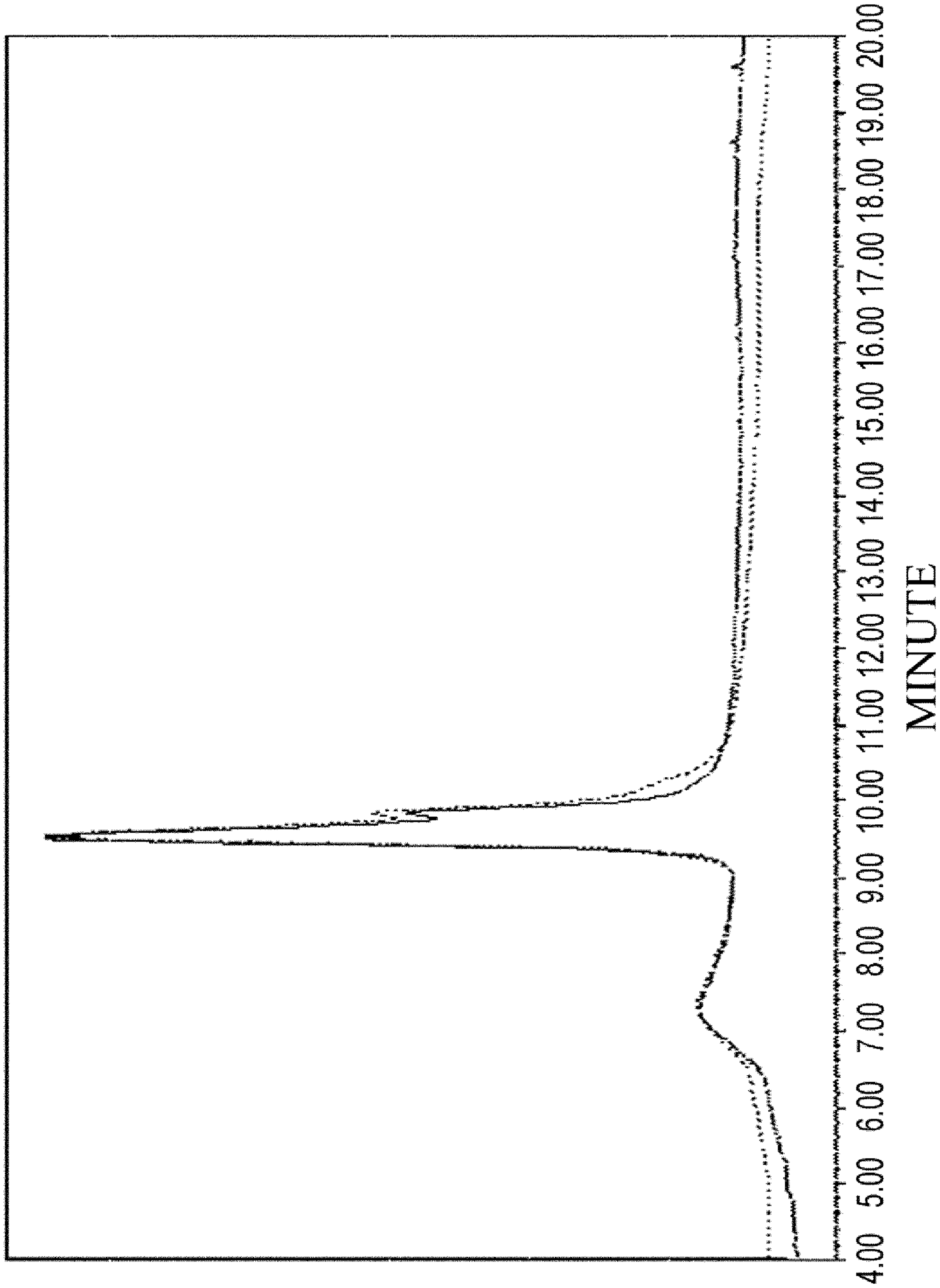


FIG. 54B

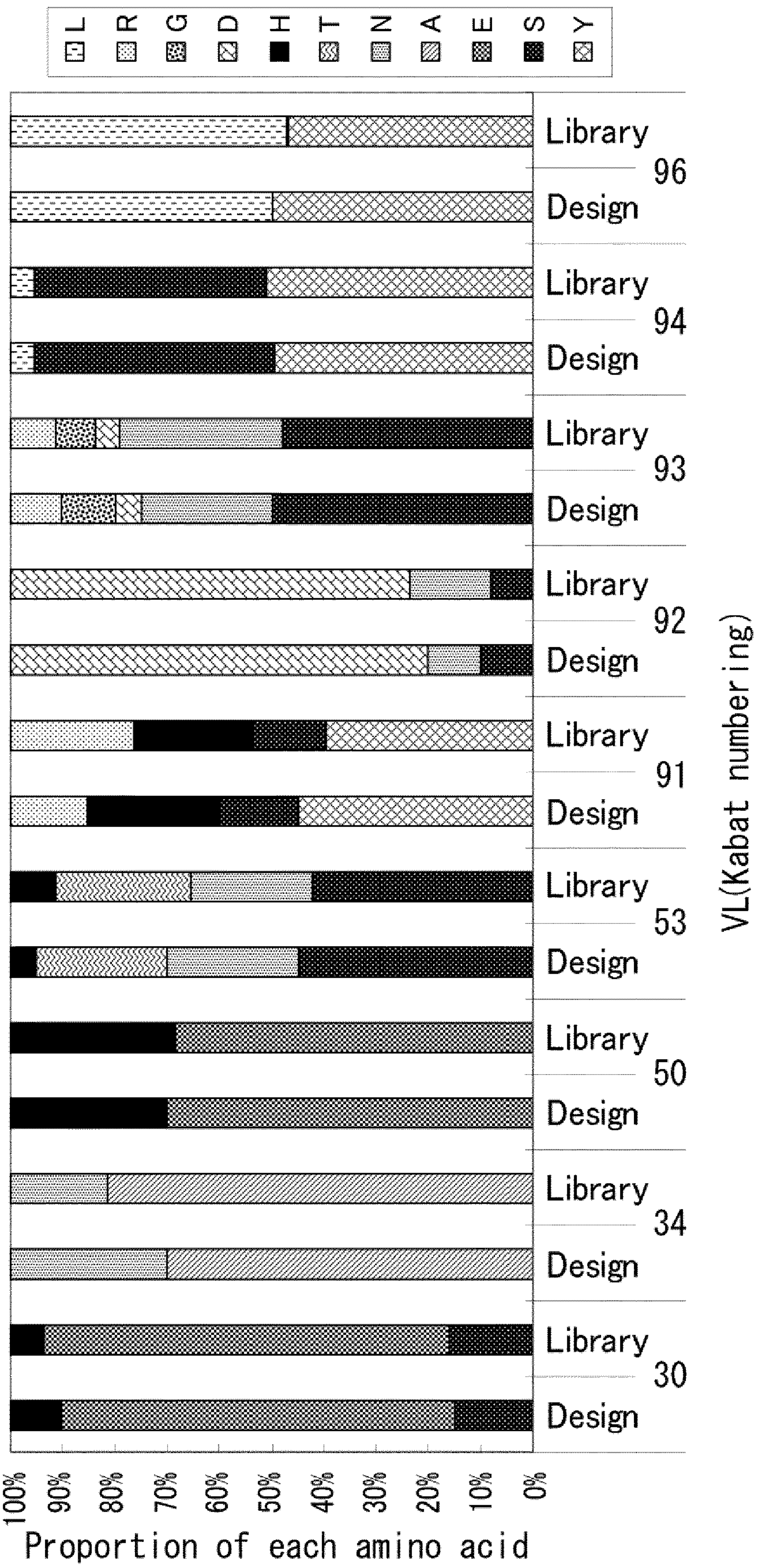
L
R
G
D
H
T
N
A
E
S
Y
Library
96
Design
Library
94
Design
Library
93
Design
Library
92
Design
Library
91
Design
Library
53
Design
Library
50
Design
Library
34
Design
Library
30
Design
VL(Kabat numbering)
100%
90%
80%
70%
60%
50%
40%
30%
20%
10%
0%
Proportion of each amino acid

FIG. 55

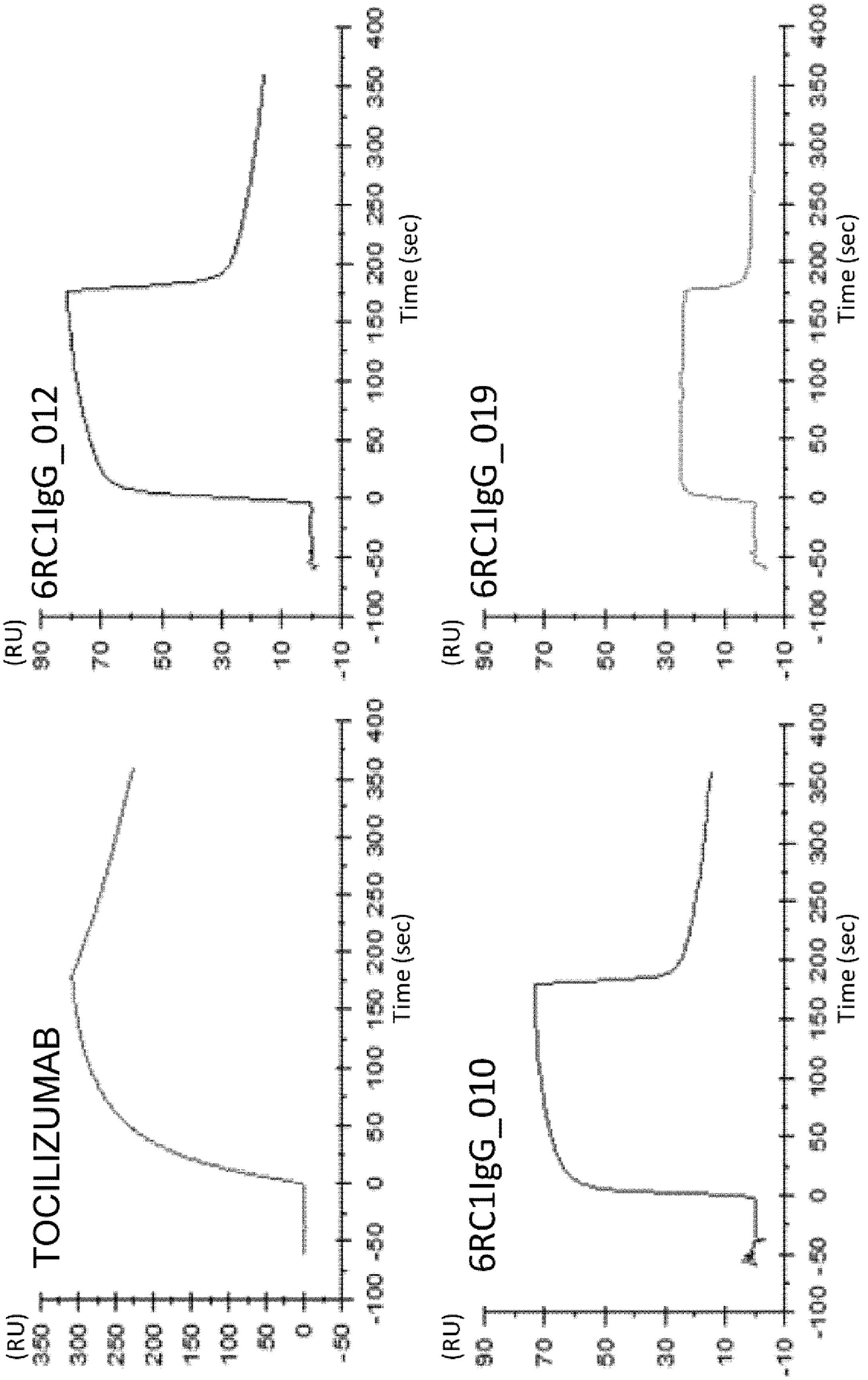
TOCILIZUMAB
(RU)
350
300
250
200
150
100
50
0
-50
-100
0
50
100
150
200
250
300
350
400
Time (sec)
6RC1IgG_012
(RU)
90
70
50
30
10
-10
Time (sec)
6RC1IgG_010
(RU)
Time (sec)
6RC1IgG_019
(RU)
Time (sec)

FIG. 56

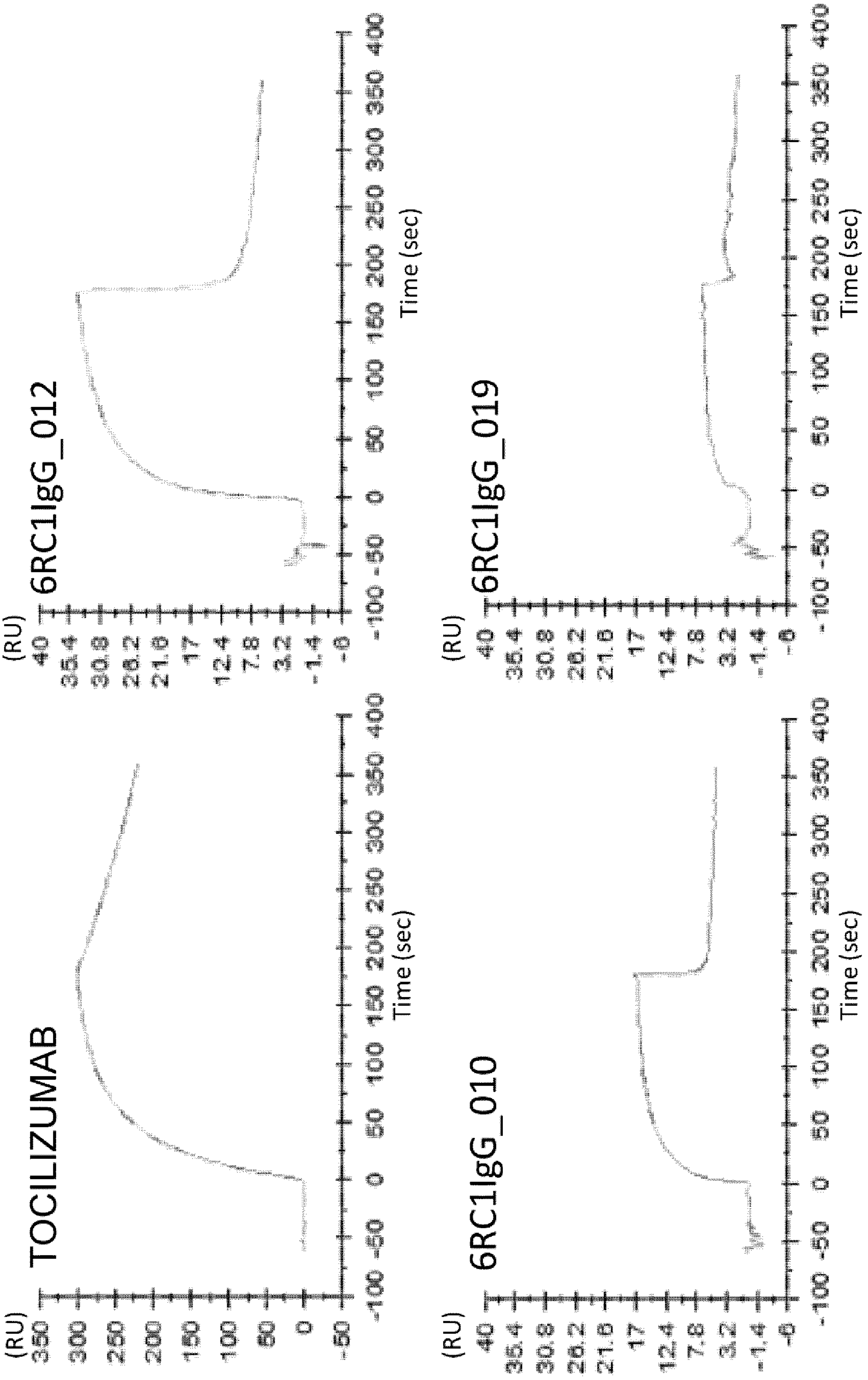

TOCILIZUMAB
6RC1IgG_012
6RC1IgG_010
6RC1IgG_019
(RU)
350
300
250
200
150
100
50
0
-50
40
35.4
30.8
26.2
21.6
17
12.4
7.8
3.2
-1.4
-6
-100 -50 0 50 100 150 200 250 300 350 400
Time (sec)


FIG. 57

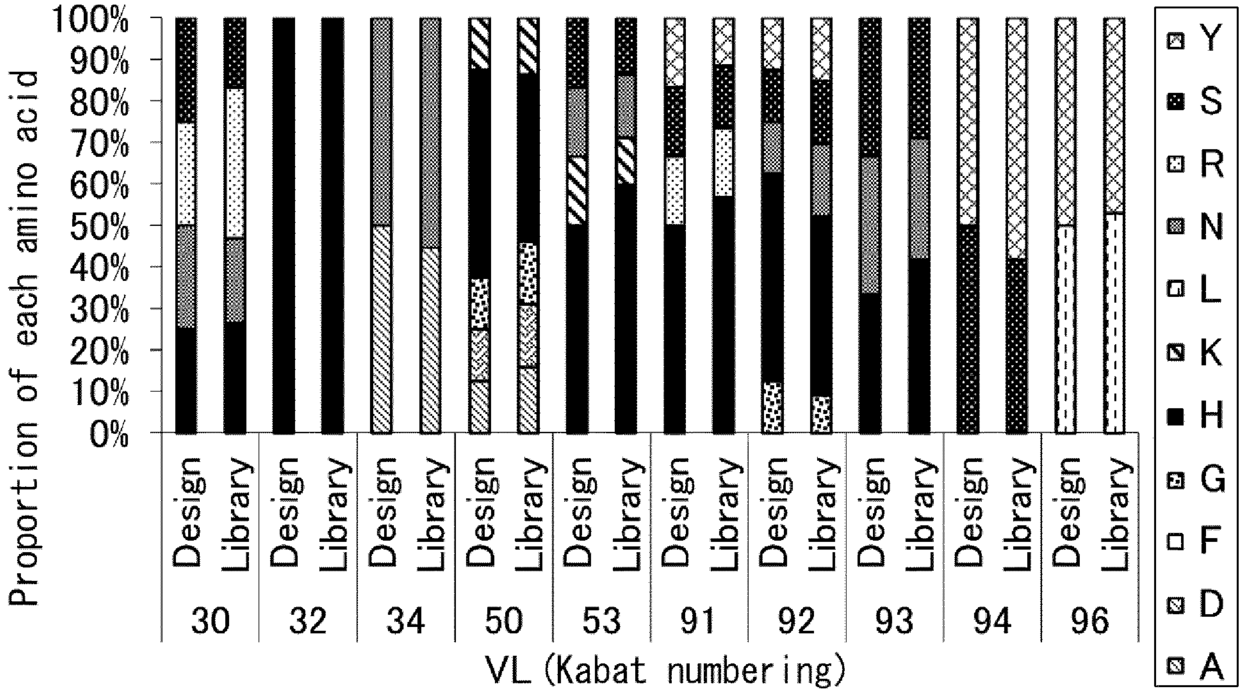
100%
90%
80%
70%
60%
50%
40%
30%
20%
10%
0%
Proportion of each amino acid
Design
Library
30
32
34
50
53
91
92
93
94
96
VL (Kabat numbering)
Y
S
R
N
L
K
H
G
F
D
A

FIG. 58

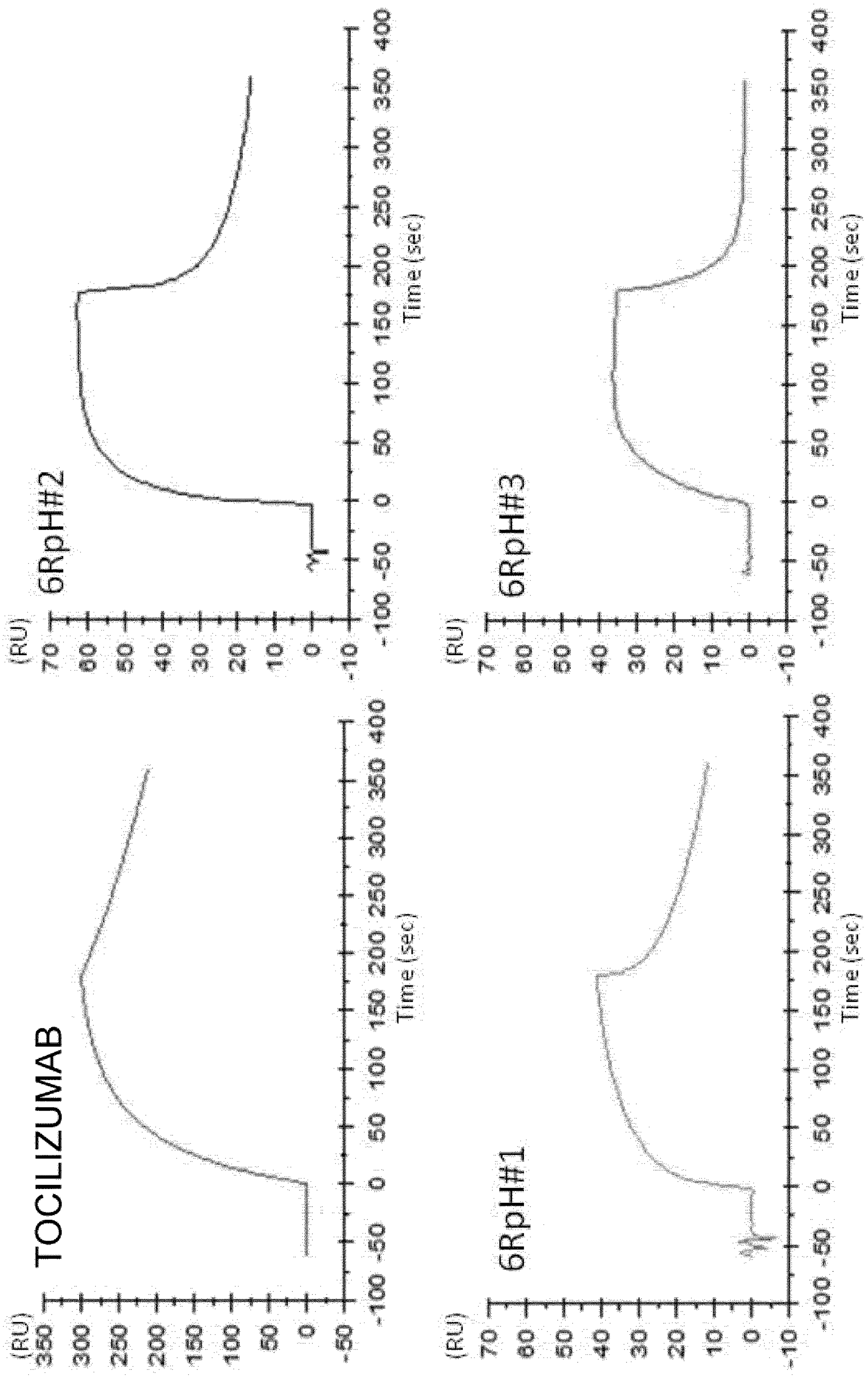


FIG. 59

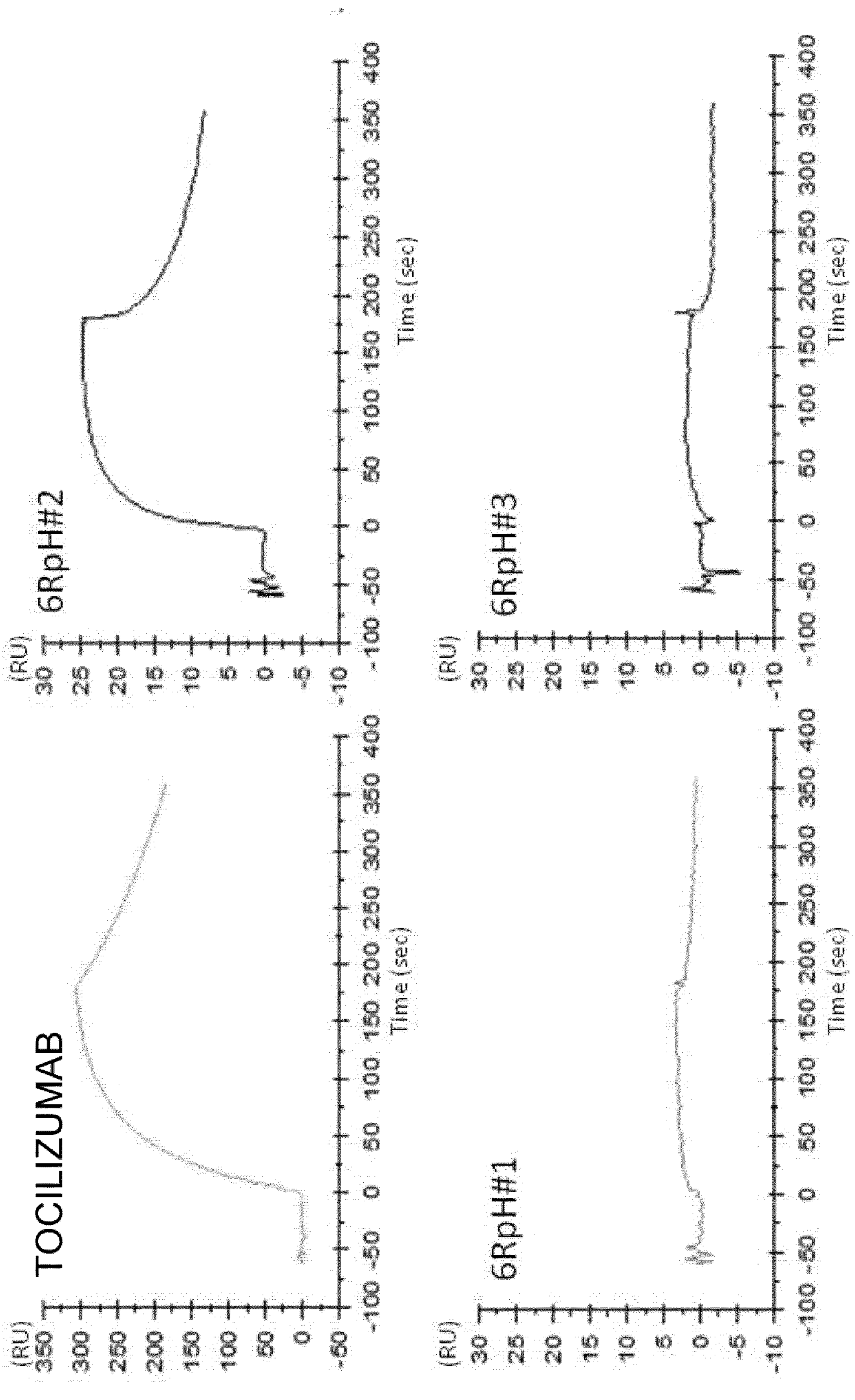


FIG. 60

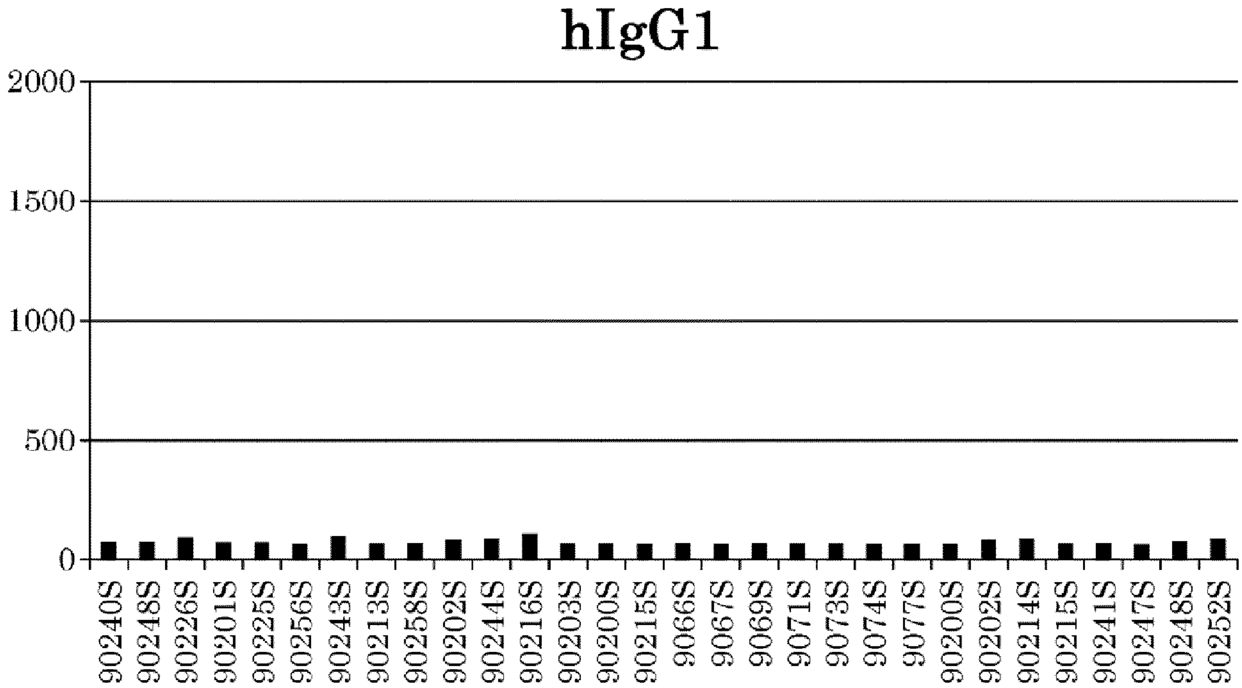
hIgG1
2000
1500
1000
500
0
90240S
90248S
90226S
90201S
90225S
90256S
90243S
90213S
90258S
90202S
90244S
90216S
90203S
90200S
90215S
9066S
9067S
9069S
9071S
9073S
9074S
9077S
90200S
90202S
90214S
90215S
90241S
90247S
90248S
90252S

FIG. 61A

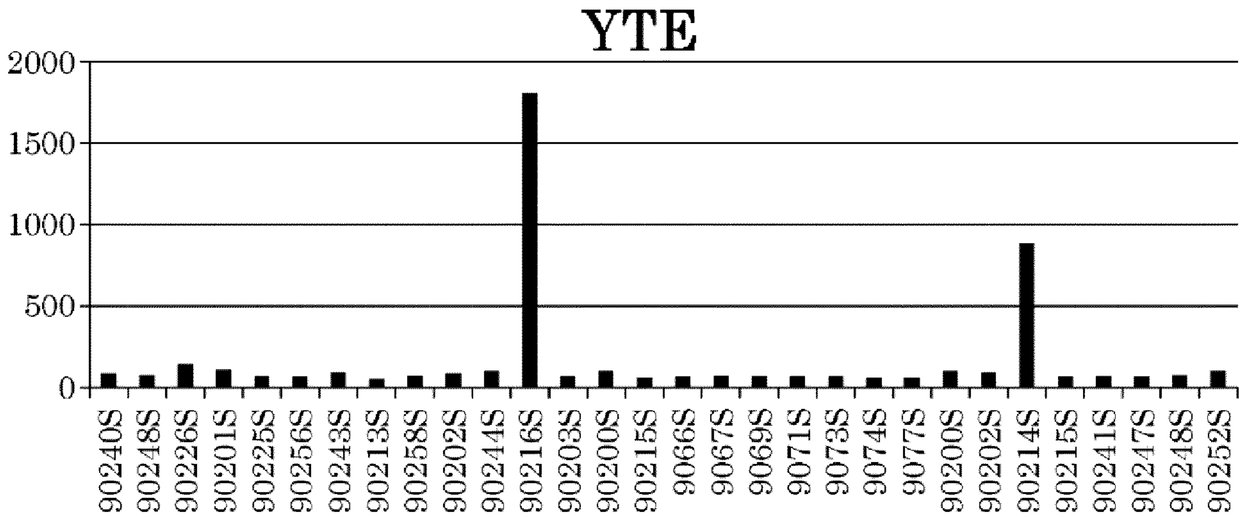
YTE
2000
1500
1000
500
0
90240S
90248S
90226S
90201S
90225S
90256S
90243S
90213S
90258S
90202S
90244S
90216S
90203S
90200S
90215S
9066S
9067S
9069S
9071S
9073S
9074S
9077S
90200S
90202S
90214S
90215S
90241S
90247S
90248S
90252S

FIG. 61B

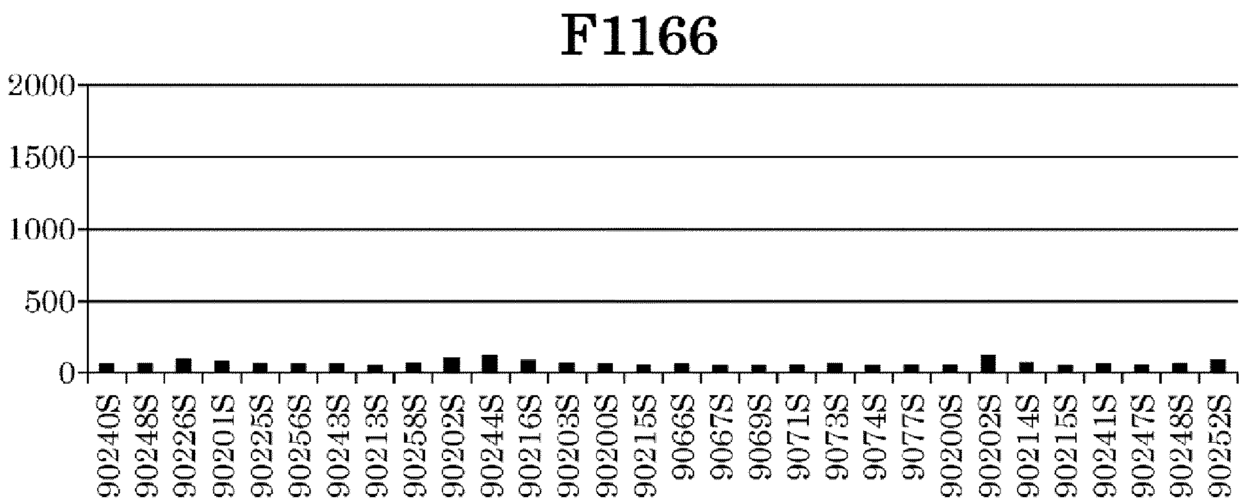
F1166
2000
1500
1000
500
0
90240S
90248S
90226S
90201S
90225S
90256S
90243S
90213S
90258S
90202S
90244S
90216S
90203S
90200S
90215S
9066S
9067S
9069S
9071S
9073S
9074S
9077S
90200S
90202S
90214S
90215S
90241S
90247S
90248S
90252S

FIG. 61C

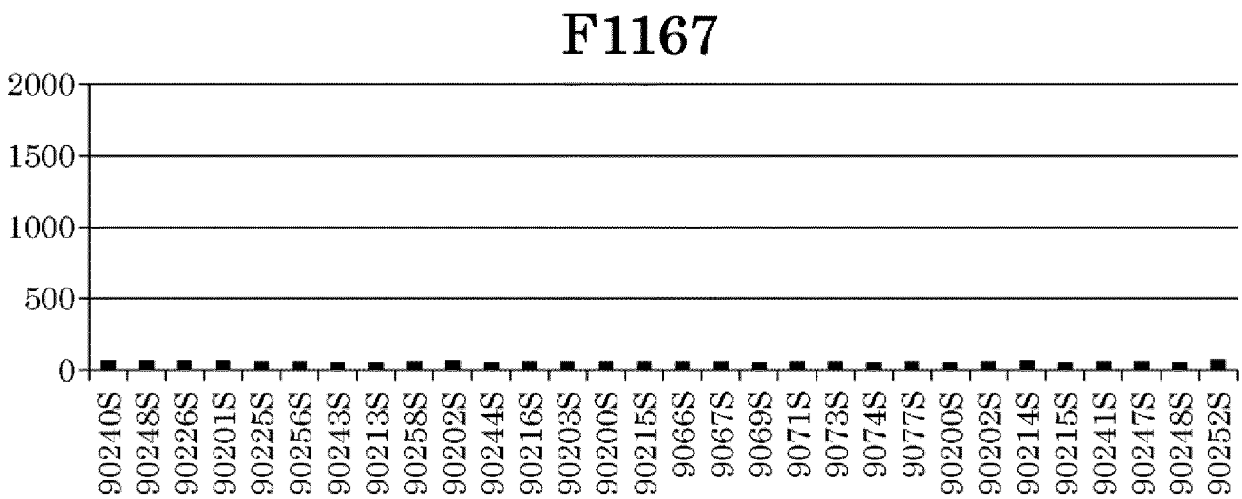
F1167
2000
1500
1000
500
0
90240S
90248S
90226S
90201S
90225S
90256S
90243S
90213S
90258S
90202S
90244S
90216S
90203S
90200S
90215S
9066S
9067S
9069S
9071S
9073S
9074S
9077S
90200S
90202S
90214S
90215S
90241S
90247S
90248S
90252S

FIG. 61D

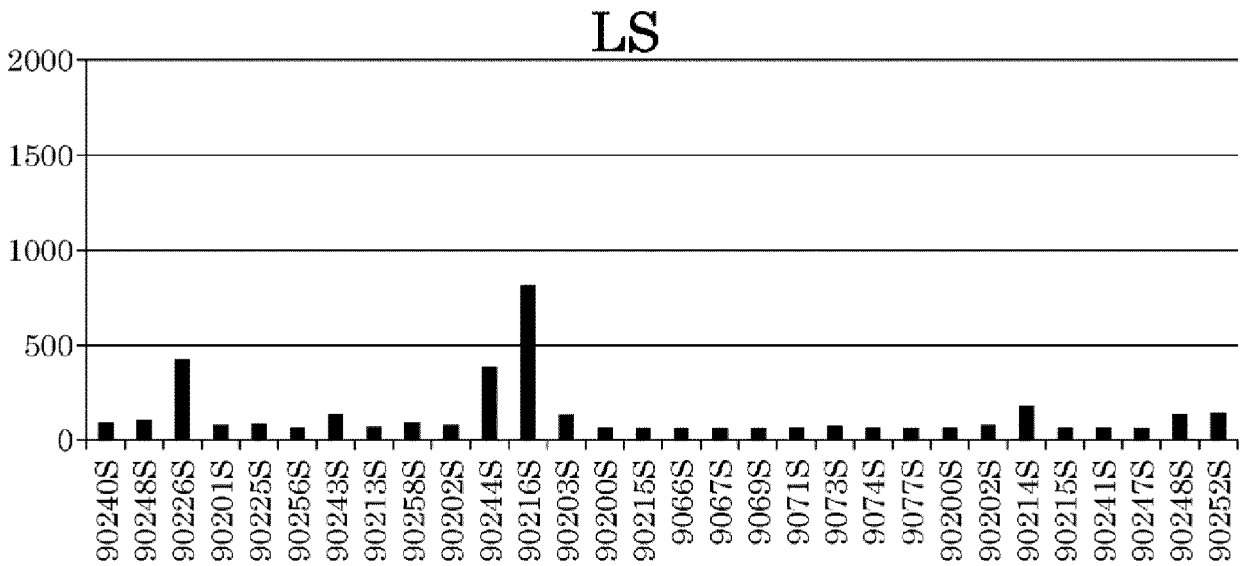
LS
2000
1500
1000
500
0
90240S
90248S
90226S
90201S
90225S
90256S
90243S
90213S
90258S
90202S
90244S
90216S
90203S
90200S
90215S
9066S
9067S
9069S
9071S
9073S
9074S
9077S
90200S
90202S
90214S
90215S
90241S
90247S
90248S
90252S

FIG. 61E

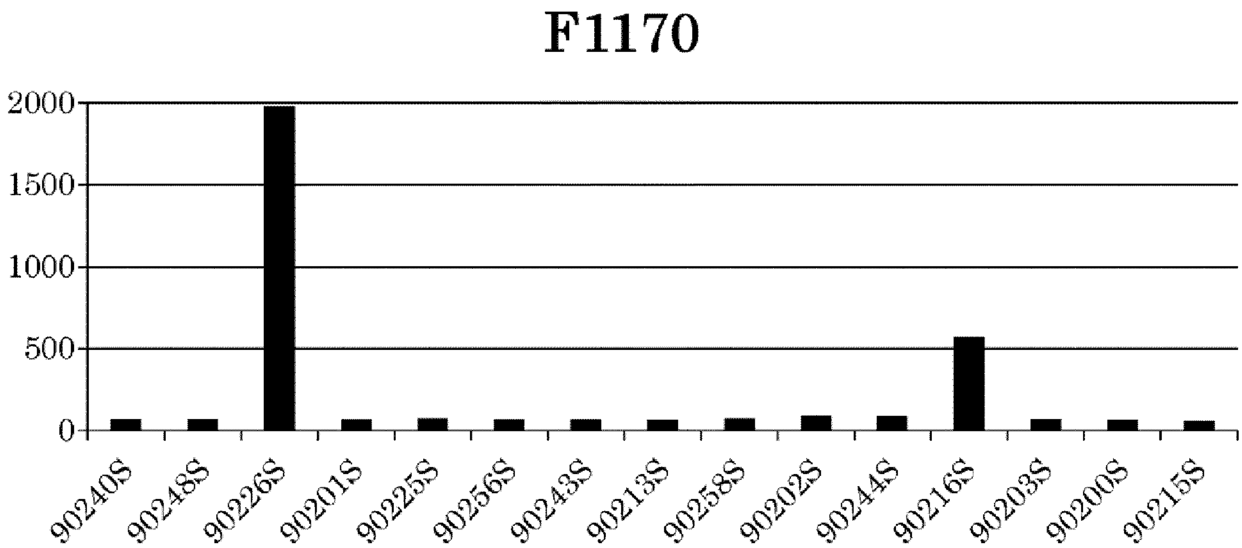
F1170
2000
1500
1000
500
0
90240S
90248S
90226S
90201S
90225S
90256S
90243S
90213S
90258S
90202S
90244S
90216S
90203S
90200S
90215S

FIG. 61F

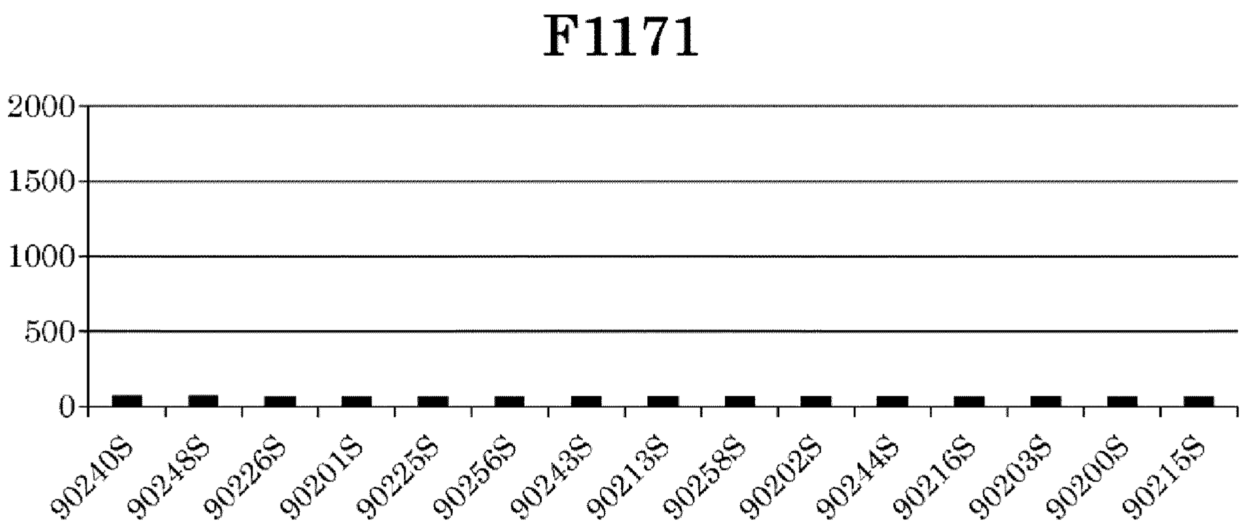
F1171
2000
1500
1000
500
0
90240S
90248S
90226S
90201S
90225S
90256S
90243S
90213S
90258S
90202S
90244S
90216S
90203S
90200S
90215S

FIG. 61G

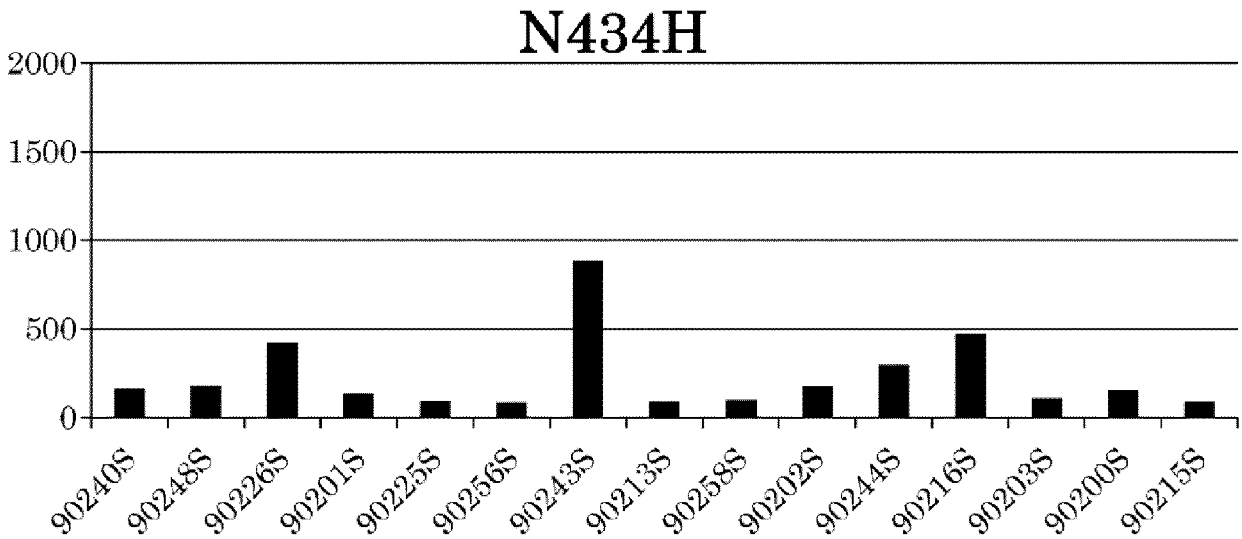
N434H
2000
1500
1000
500
0
90240S
90248S
90226S
90201S
90225S
90256S
90243S
90213S
90258S
90202S
90244S
90216S
90203S
90200S
90215S

FIG. 61H

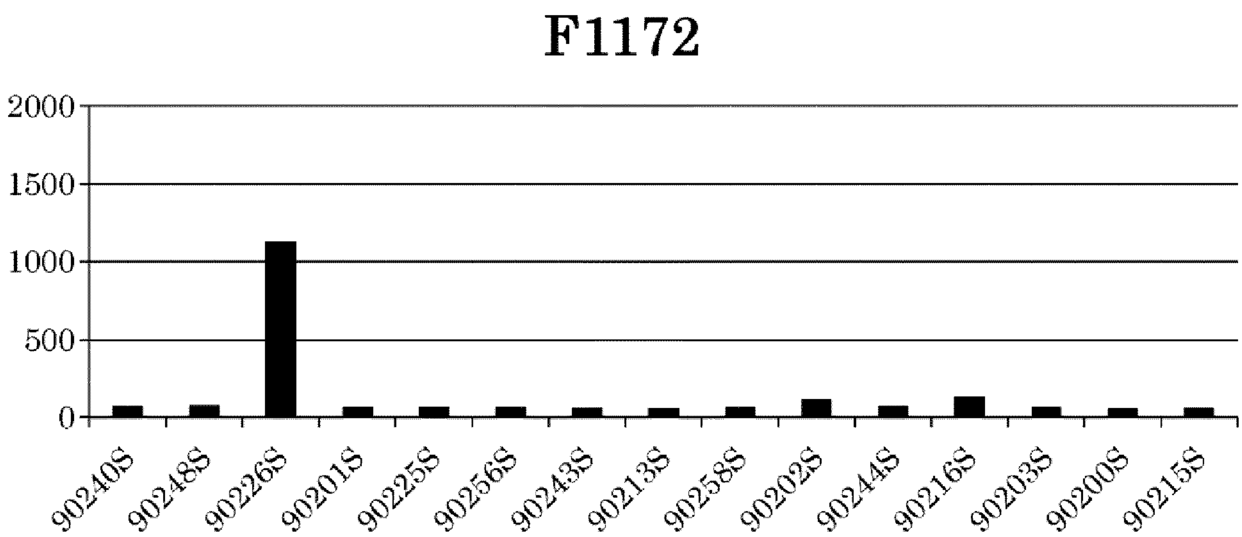
F1172
2000
1500
1000
500
0
90240S
90248S
90226S
90201S
90225S
90256S
90243S
90213S
90258S
90202S
90244S
90216S
90203S
90200S
90215S

FIG. 61I

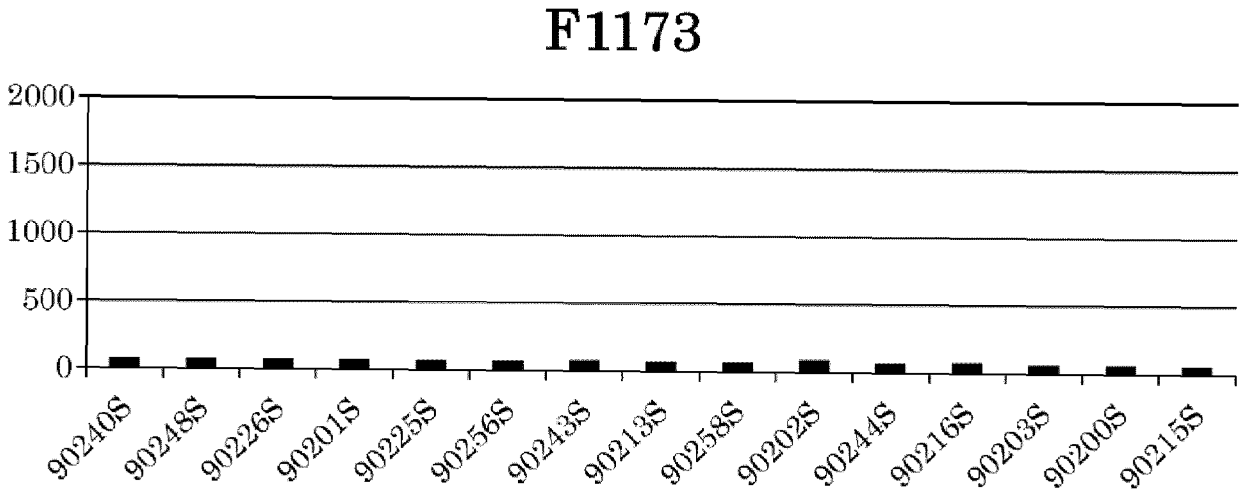
F1173
2000
1500
1000
500
0
90240S
90248S
90226S
90201S
90225S
90256S
90243S
90213S
90258S
90202S
90244S
90216S
90203S
90200S
90215S

FIG. 61J

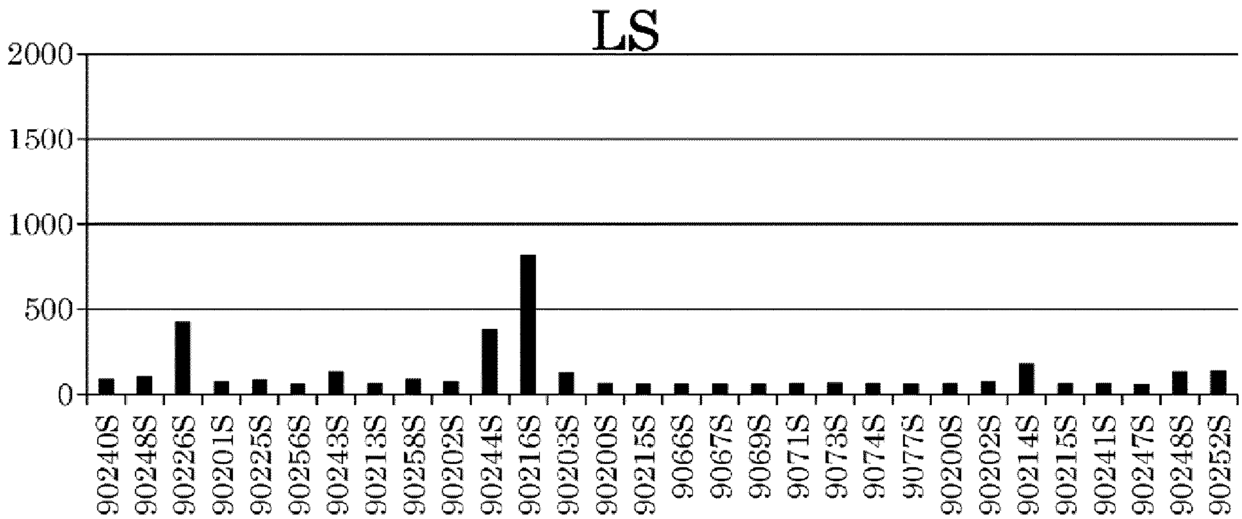
LS
2000
1500
1000
500
0
90240S
90248S
90226S
90201S
90225S
90256S
90243S
90213S
90258S
90202S
90244S
90216S
90203S
90200S
90215S
9066S
9067S
9069S
9071S
9073S
9074S
9077S
90200S
90202S
90214S
90215S
90241S
90247S
90248S
90252S

FIG. 62A

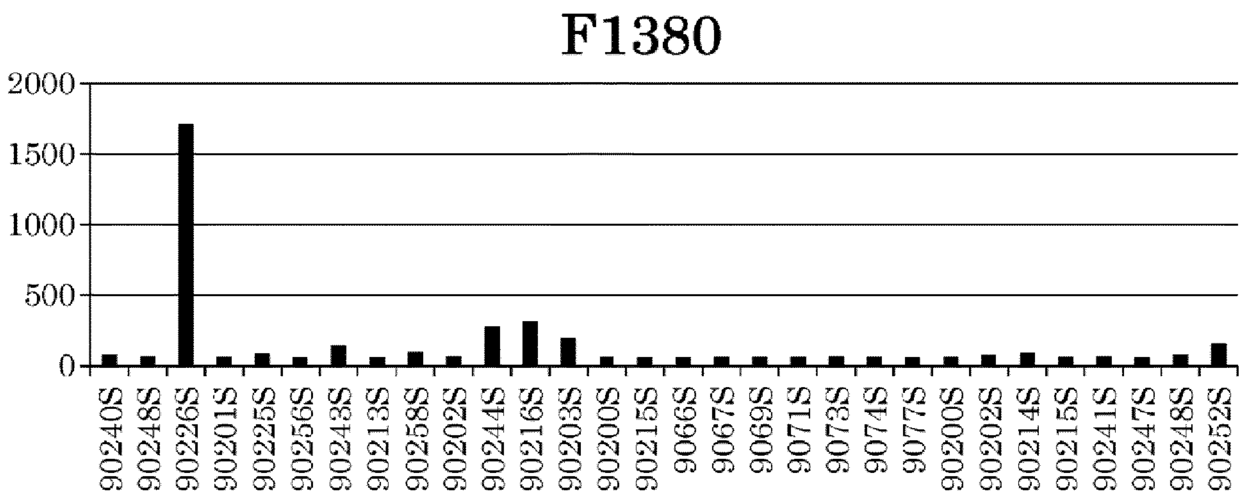
F1380
2000
1500
1000
500
0
90240S
90248S
90226S
90201S
90225S
90256S
90243S
90213S
90258S
90202S
90244S
90216S
90203S
90200S
90215S
9066S
9067S
9069S
9071S
9073S
9074S
9077S
90200S
90202S
90214S
90215S
90241S
90247S
90248S
90252S

FIG. 62B

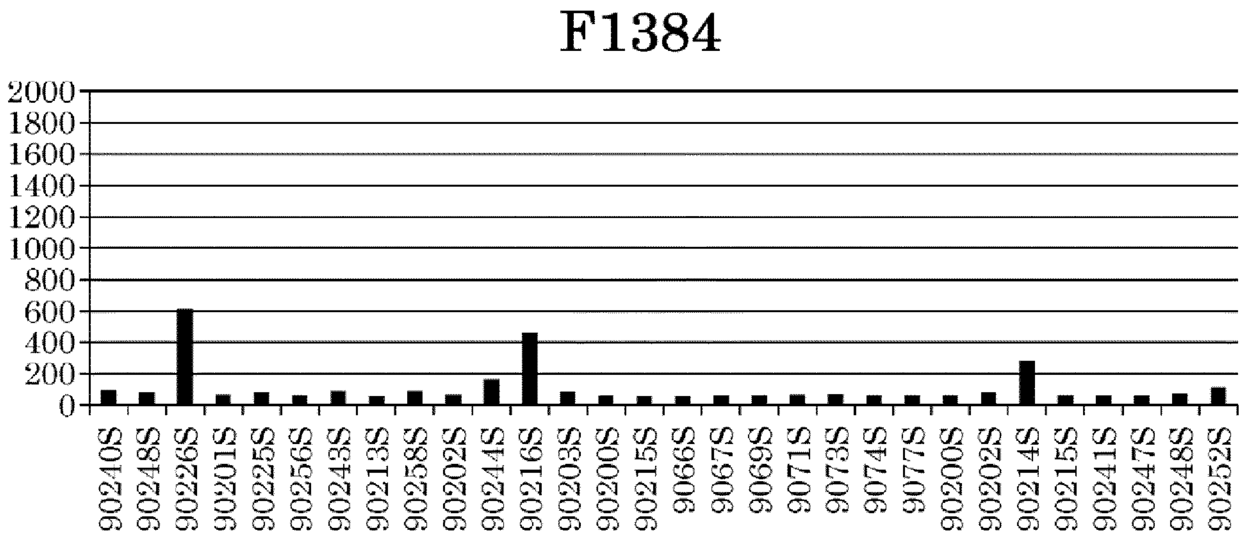
F1384
2000
1800
1600
1400
1200
1000
800
600
400
200
0
90240S
90248S
90226S
90201S
90225S
90256S
90243S
90213S
90258S
90202S
90244S
90216S
90203S
90200S
90215S
9066S
9067S
9069S
9071S
9073S
9074S
9077S
90200S
90202S
90214S
90215S
90241S
90247S
90248S
90252S

FIG. 62C

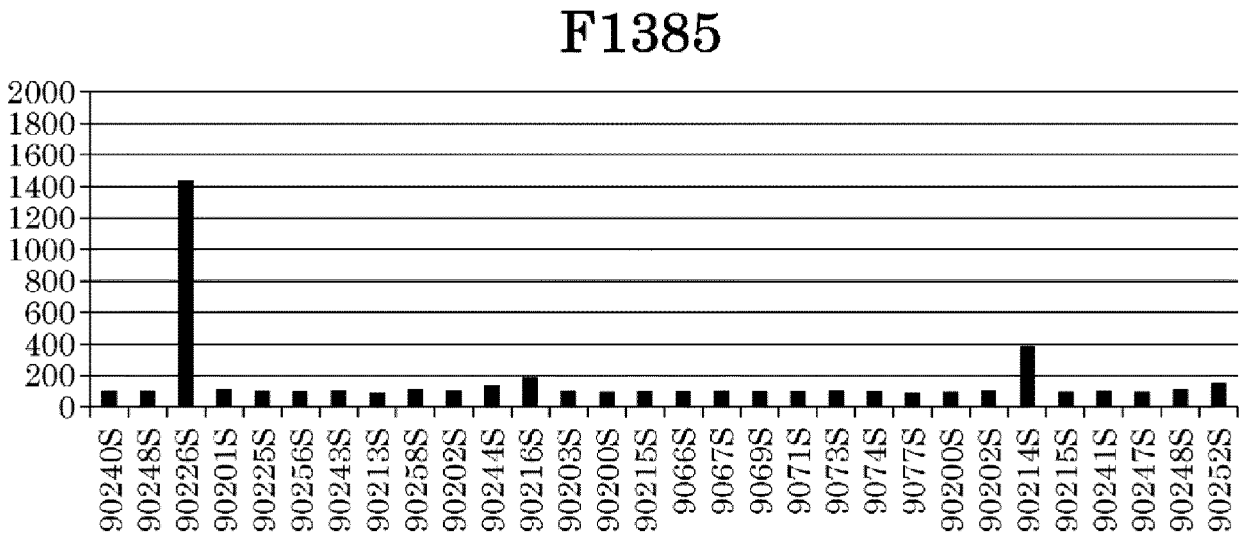
F1385
2000
1800
1600
1400
1200
1000
800
600
400
200
0
90240S
90248S
90226S
90201S
90225S
90256S
90243S
90213S
90258S
90202S
90244S
90216S
90203S
90200S
90215S
9066S
9067S
9069S
9071S
9073S
9074S
9077S
90200S
90202S
90214S
90215S
90241S
90247S
90248S
90252S

FIG. 62D

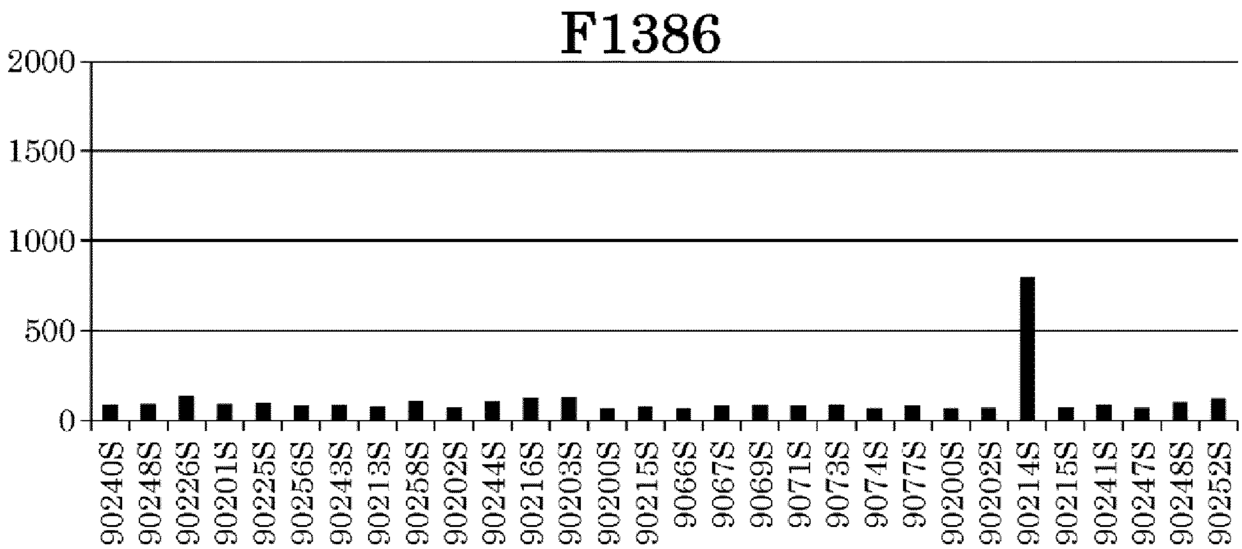
F1386
2000
1500
1000
500
0
90240S
90248S
90226S
90201S
90225S
90256S
90243S
90213S
90258S
90202S
90244S
90216S
90203S
90200S
90215S
9066S
9067S
9069S
9071S
9073S
9074S
9077S
90200S
90202S
90214S
90215S
90241S
90247S
90248S
90252S

FIG. 62E

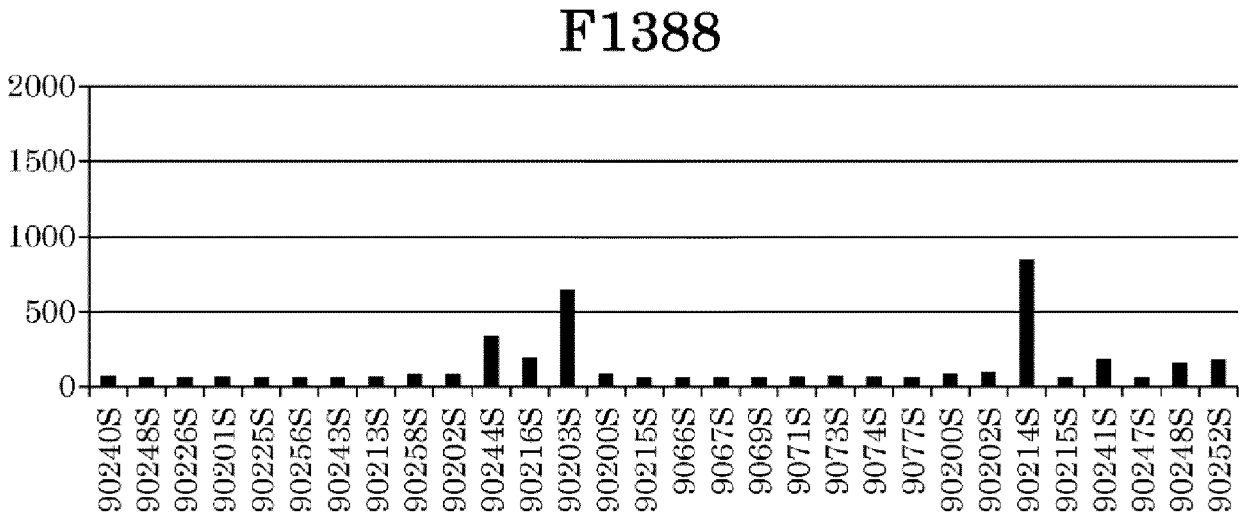
F1388
2000
1500
1000
500
0
90240S
90248S
90226S
90201S
90225S
90256S
90243S
90213S
90258S
90202S
90244S
90216S
90203S
90200S
90215S
9066S
9067S
9069S
9071S
9073S
9074S
9077S
90200S
90202S
90214S
90215S
90241S
90247S
90248S
90252S

FIG. 62F

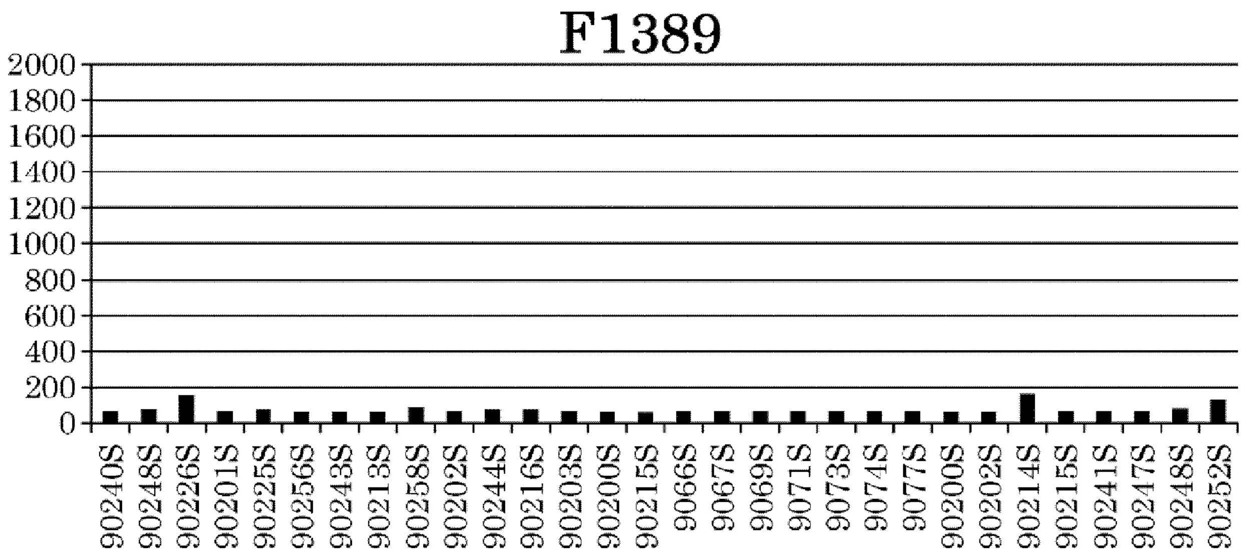
F1389
2000
1800
1600
1400
1200
1000
800
600
400
200
0
90240S
90248S
90226S
90201S
90225S
90256S
90243S
90213S
90258S
90202S
90244S
90216S
90203S
90200S
90215S
9066S
9067S
9069S
9071S
9073S
9074S
9077S
90200S
90202S
90214S
90215S
90241S
90247S
90248S
90252S

FIG. 62G

Europäisches Patentamt
European Patent Office
Office européen des brevets

# EUROPEAN SEARCH REPORT

Application Number
EP 19 19 7872

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 275 443 A1 (CHUGAI PHARMACEUTICAL CO LTD [JP]) 19 January 2011 (2011-01-19) * abstract; claims 1,7,15 * | 1-4,7,8 | INV. C07K16/00 |
| E | EP 2 765 192 A1 (CHUGAI PHARMACEUTICAL CO LTD [JP]) 13 August 2014 (2014-08-13) * par. 0014, 0122, 0159, calims; claims 1-9 * | 1-10 | |
| Y | R REVERBERI ET AL: "Factors affecting the antigen-antibody reaction", BLOOD TRANSFUS, vol. 5, 1 January 2007 (2007-01-01), pages 227-240, XP055069254, DOI: 10.2450/2007.0047-07 * page 230; table 4 * | 1-10 | |
| Y,D | WO 2006/019447 A1 (XENCOR INC [US]; LAZAR GREGORY ALAN [US]; DANG WEI [US]; DESJARLAIS JO) 23 February 2006 (2006-02-23) * paragraphs [0020], [0024], [0255]; figure 41; example 2 * | 1-10 | TECHNICAL FIELDS SEARCHED (IPC) |
| A | WO 2007/024249 A2 (MACROGENICS INC [US]; STAVENHAGEN JEFFREY [US]; KOENIG SCOTT [US]) 1 March 2007 (2007-03-01) * page 42; claims 1, 15, 16, 29; tables 2, 24 * | 1-10 | C07K |

-/--

The present search report has been drawn up for all claims

| Place of search | Date of completion of the search | Examiner |
|---|---|---|
| Munich | 28 May 2020 | Hoesel, Heidi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 19 19 7872

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | IGAWA T ET AL: "Antibody recycling by engineered pH-dependent antigen binding improves the duration of antigen neutralization", NATURE BIOTECHNOLOGY, NATURE PUBLISHING GROUP, NEW YORK, NY, US, vol. 28, no. 11, 1 November 2010 (2010-11-01), pages 1203-1207, XP009153598, ISSN: 1087-0156, DOI: 10.1038/NBT.1691 * abstract * ----- | 1-10 | |
| A | A. DATTA-MANNAN ET AL: "Monoclonal Antibody Clearance: IMPACT OF MODULATING THE INTERACTION OF IgG WITH THE NEONATAL Fc RECEPTOR", JOURNAL OF BIOLOGICAL CHEMISTRY, vol. 282, no. 3, 1 January 2007 (2007-01-01), pages 1709-1717, XP055043665, ISSN: 0021-9258, DOI: 10.1074/jbc.M607161200 * column 2, paragraph 2 - page 1709 * ----- | 1-10 | TECHNICAL FIELDS SEARCHED (IPC) |

The present search report has been drawn up for all claims

2

| Place of search | Date of completion of the search | Examiner |
|---|---|---|
| Munich | 28 May 2020 | Hoesel, Heidi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO. EP 19 19 7872

This annex lists the patent family members relating to the patent documents cited in the above-mentioned European search report.
The members are as contained in the European Patent Office EDP file on
The European Patent Office is in no way liable for these particulars which are merely given for the purpose of information.

28-05-2020

| Patent document cited in search report | Publication date | Patent family member(s) | Publication date |
|---|---|---|---|
| EP 2275443 A1 | 19-01-2011 | AR 071656 A1 | 07-07-2010 |
| | | AU 2009234675 A1 | 15-10-2009 |
| | | BR PI0911431 A2 | 06-10-2015 |
| | | CA 2721052 A1 | 15-10-2009 |
| | | CN 102056946 A | 11-05-2011 |
| | | CN 102993304 A | 27-03-2013 |
| | | CN 103251948 A | 21-08-2013 |
| | | CN 107469077 A | 15-12-2017 |
| | | CN 107488228 A | 19-12-2017 |
| | | CN 107551270 A | 09-01-2018 |
| | | CO 6311005 A2 | 22-08-2011 |
| | | CR 11783 A | 09-12-2010 |
| | | CR 20150655 A | 08-02-2016 |
| | | CR 20150656 A | 27-01-2016 |
| | | DK 2275443 T3 | 08-02-2016 |
| | | DK 2708558 T3 | 14-06-2018 |
| | | DK 2708559 T3 | 14-06-2018 |
| | | EC SP10010600 A | 31-03-2011 |
| | | EP 2275443 A1 | 19-01-2011 |
| | | EP 2708558 A2 | 19-03-2014 |
| | | EP 2708559 A2 | 19-03-2014 |
| | | EP 3056513 A1 | 17-08-2016 |
| | | EP 3514180 A1 | 24-07-2019 |
| | | EP 3521311 A1 | 07-08-2019 |
| | | ES 2563483 T3 | 15-03-2016 |
| | | ES 2671010 T3 | 04-06-2018 |
| | | ES 2671403 T3 | 06-06-2018 |
| | | HR P20160209 T1 | 25-03-2016 |
| | | HR P20180708 T1 | 29-06-2018 |
| | | HU E028718 T2 | 28-12-2016 |
| | | HU E037386 T2 | 28-08-2018 |
| | | IL 208516 A | 30-11-2017 |
| | | IL 237599 A | 31-10-2018 |
| | | JP 4954326 B2 | 13-06-2012 |
| | | JP 4961501 B2 | 27-06-2012 |
| | | JP 5048866 B2 | 17-10-2012 |
| | | JP 5503698 B2 | 28-05-2014 |
| | | JP 5824095 B2 | 25-11-2015 |
| | | JP 6082447 B2 | 15-02-2017 |
| | | JP 6417431 B2 | 07-11-2018 |
| | | JP 2012021004 A | 02-02-2012 |
| | | JP 2012116837 A | 21-06-2012 |
| | | JP 2012224633 A | 15-11-2012 |
| | | JP 2014133746 A | 24-07-2014 |
| | | JP 2016026190 A | 12-02-2016 |
| | | JP 2017081995 A | 18-05-2017 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 19 19 7872

This annex lists the patent family members relating to the patent documents cited in the above-mentioned European search report.
The members are as contained in the European Patent Office EDP file on
The European Patent Office is in no way liable for these particulars which are merely given for the purpose of information.

28-05-2020

| Patent document cited in search report | Publication date | Patent family member(s) | Publication date |
|---|---|---|---|
| | | JP 2019048810 A | 28-03-2019 |
| | | JP WO2009125825 A1 | 04-08-2011 |
| | | KR 20110004435 A | 13-01-2011 |
| | | KR 20170091758 A | 09-08-2017 |
| | | KR 20190140100 A | 18-12-2019 |
| | | KR 20200023536 A | 04-03-2020 |
| | | LT 2708559 T | 11-06-2018 |
| | | MA 32754 B1 | 01-11-2011 |
| | | MX 354670 B | 15-03-2018 |
| | | NO 2708559 T3 | 25-08-2018 |
| | | NZ 588507 A | 30-11-2012 |
| | | NZ 602884 A | 29-08-2014 |
| | | NZ 623716 A | 29-04-2016 |
| | | NZ 717429 A | 27-07-2018 |
| | | PH 12014502054 A1 | 07-12-2015 |
| | | PL 2275443 T3 | 31-05-2016 |
| | | PL 2708558 T3 | 28-09-2018 |
| | | PL 2708559 T3 | 31-08-2018 |
| | | PT 2275443 E | 15-03-2016 |
| | | PT 2708559 T | 16-05-2018 |
| | | RU 2010145939 A | 20-05-2012 |
| | | RU 2014128324 A | 10-02-2016 |
| | | SG 189775 A1 | 31-05-2013 |
| | | SG 2013027206 A | 27-11-2014 |
| | | SG 10201608379Y A | 29-11-2016 |
| | | SI 2275443 T1 | 31-03-2016 |
| | | SI 2708559 T1 | 31-07-2018 |
| | | TR 201808046 T4 | 21-06-2018 |
| | | TR 201808535 T4 | 23-07-2018 |
| | | TW 201000127 A | 01-01-2010 |
| | | TW 201447062 A | 16-12-2014 |
| | | TW 201641514 A | 01-12-2016 |
| | | TW 201920257 A | 01-06-2019 |
| | | US 2011111406 A1 | 12-05-2011 |
| | | US 2013011866 A1 | 10-01-2013 |
| | | US 2013303396 A1 | 14-11-2013 |
| | | US 2013336963 A1 | 19-12-2013 |
| | | US 2018282718 A1 | 04-10-2018 |
| | | US 2018282719 A1 | 04-10-2018 |
| | | US 2020048627 A1 | 13-02-2020 |
| | | WO 2009125825 A1 | 15-10-2009 |
| EP 2765192 A1 | 13-08-2014 | EP 2765192 A1 | 13-08-2014 |
| | | EP 3617313 A1 | 04-03-2020 |
| | | JP 6271251 B2 | 31-01-2018 |
| | | JP 6672251 B2 | 25-03-2020 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 19 19 7872

This annex lists the patent family members relating to the patent documents cited in the above-mentioned European search report.
The members are as contained in the European Patent Office EDP file on
The European Patent Office is in no way liable for these particulars which are merely given for the purpose of information.

28-05-2020

| Patent document cited in search report | Publication date | Patent family member(s) | Publication date |
|---|---|---|---|
| | | JP 2018099118 A | 28-06-2018 |
| | | JP WO2013051294 A1 | 30-03-2015 |
| | | US 2015299313 A1 | 22-10-2015 |
| | | WO 2013051294 A1 | 11-04-2013 |
| WO 2006019447 A1 | 23-02-2006 | AU 2005272993 A1 | 23-02-2006 |
| | | BR PI0510674 A | 26-12-2007 |
| | | CA 2565961 A1 | 23-02-2006 |
| | | CN 101014619 A | 08-08-2007 |
| | | CN 101987870 A | 23-03-2011 |
| | | CN 103172731 A | 26-06-2013 |
| | | CN 103351434 A | 16-10-2013 |
| | | DK 2471813 T3 | 02-03-2015 |
| | | EP 1919950 A1 | 14-05-2008 |
| | | EP 2471813 A1 | 04-07-2012 |
| | | EP 2940043 A1 | 04-11-2015 |
| | | EP 3342782 A1 | 04-07-2018 |
| | | ES 2530340 T3 | 02-03-2015 |
| | | JP 5301611 B2 | 25-09-2013 |
| | | JP 2008505174 A | 21-02-2008 |
| | | JP 2011188869 A | 29-09-2011 |
| | | KR 20070029190 A | 13-03-2007 |
| | | PL 2471813 T3 | 30-09-2015 |
| | | SI 2471813 T1 | 31-03-2015 |
| | | WO 2006019447 A1 | 23-02-2006 |
| WO 2007024249 A2 | 01-03-2007 | AU 2005335714 A1 | 01-03-2007 |
| | | CA 2587766 A1 | 01-03-2007 |
| | | EP 1810035 A2 | 25-07-2007 |
| | | IL 182974 A | 28-11-2013 |
| | | US 2006134709 A1 | 22-06-2006 |
| | | US 2010196362 A1 | 05-08-2010 |
| | | US 2012263711 A1 | 18-10-2012 |
| | | WO 2007024249 A2 | 01-03-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009125825 A
- WO 2000042072 A
- WO 2006019447 A
- WO 2004044011 A
- WO 2005040229 A
- WO 2002032925 A
- WO 1995001937 A
- WO 2002020565 A
- WO 2003029462 A
- WO 2008016854 A
- WO 2003000883 A
- WO 2004022754 A
- WO 2006006693 A
- WO 2003104453 A
- WO 1994011523 A
- EP 239400 A
- WO 1996002576 A
- WO 1993012227 A
- WO 1992003918 A
- WO 1994002602 A
- WO 1994025585 A
- WO 1996034096 A
- WO 1996033735 A
- WO 1992001047 A
- WO 1992020791 A
- WO 1993006213 A
- WO 1993011236 A
- WO 1993019172 A
- WO 1995001438 A
- WO 1995015388 A
- WO 2008081008 A
- US 20090035836 A
- WO 2009086320 A
- WO 2008092117 A
- WO 2007041635 A
- WO 2006105338 A
- WO 2007024249 A
- WO 2007021841 A
- WO 2006031370 A
- WO 2004029207 A
- WO 2004099249 A
- WO 2005070963 A
- WO 2006020114 A
- WO 2006116260 A
- WO 2006023403 A
- WO 2002060919 A
- WO 2004092219 A
- WO 2010045193 A
- WO 1997034631 A
- WO 2006067913 A
- WO 2010106180 A
- WO 2009058492 A
- WO 2008022152 A
- WO 2006050166 A
- WO 2006053301 A
- WO 2005123780 A
- WO 2005047327 A
- WO 2005037867 A
- WO 2004035752 A
- WO 2010088444 A
- WO 2010136598 A
- WO 2010105256 A
- WO 2009139822 A


### Non-patent literature cited in the description


- **JANICE M REICHERT ; CLARK J ROSENSWEIG ; LAURA B FADEN ; MATTHEW C DEWITZ.** Monoclonal antibody successes in the clinic. *Nat. Biotechnol.,* 2005, vol. 23, 1073-1078
- **PAVLOU AK ; BELSEY MJ.** The therapeutic antibodies market to 2008. *Eur J Pharm Biopharm.,* 2005, vol. 59 (3), 389-396
- **KIM SJ ; PARK Y ; HONG HJ.** Antibody engineering for the development of therapeutic antibodies. *Mol Cells.,* 2005, vol. 20 (1), 17-29
- **HINTON PR ; XIONG JM ; JOHLFS MG ; TANG MT ; KELLER S ; TSURUSHITA N.** An engineered human IgG1 antibody with longer serum half-life. *J. Immunol.,* 2006, vol. 176 (1), 346-356
- **GHETIE V ; POPOV S ; BORVAK J ; RADU C ; MATESOI D ; MEDESAN C ; OBER RJ ; WARD ES.** Increasing the serum persistence of an IgG fragment by random mutagenesis. *Nat. Biotechnol.,* 1997, vol. 15 (7), 637-640

- **RAJPAL A ; BEYAZ N ; HABER L ; CAPPUCCILLI G ; YEE H ; BHATT RR ; TAKEUCHI T ; LERNER RA ; CREA R.** A general method for greatly improving the affinity of antibodies by using combinatorial libraries. *Proc. Natl. Acad. Sci. U. S. A.,* 2005, vol. 102 (24), 8466-8471
- **WU H ; PFARR DS ; JOHNSON S ; BREWAH YA ; WOODS RM ; PATEL NK ; WHITE WI ; YOUNG JF ; KIENER PA.** Development of Motavizumab, an Ultra-potent Antibody for the Prevention of Respiratory Syncytial Virus Infection in the Upper and Lower Respiratory Tract. *J. Mol. Biol.,* 2007, vol. 368, 652-665
- **HANSON CV ; NISHIYAMA Y ; PAUL S.** Catalytic antibodies and their applications. *Curr Opin Biotechnol.,* 2005, vol. 16 (6), 631-636
- **RATHANASWAMI P ; ROALSTAD S ; ROSKOS L ; SU QJ ; LACKIE S ; BABCOOK J.** Demonstration of an in vivo generated sub-picomolar affinity fully human monoclonal antibody to interleukin-8. *Biochem. Biophys. Res. Commun.,* 2005, vol. 334 (4), 1004-1013
- **DALL'ACQUA WF ; WOODS RM ; WARD ES ; PALASZYNSKI SR ; PATEL NK ; BREWAH YA ; WU H ; KIENER PA ; LANGERMANN S.** Increasing the affinity of a human IgG1 for the neonatal Fc receptor: biological consequences. *J. Immunol.,* 2002, vol. 169 (9), 5171-5180
- **YEUNG YA ; LEABMAN MK ; MARVIN JS ; QIU J ; ADAMS CW ; LIEN S ; STAROVASNIK MA ; LOWMAN HB.** Engineering human IgG1 affinity to human neonatal Fc receptor: impact of affinity improvement on pharmacokinetics in primates. *J. Immunol.,* 2009, vol. 182 (12), 7663-7671
- **DATTA-MANNAN A ; WITCHER DR ; TANG Y ; WATKINS J ; WROBLEWSKI VJ.** Monoclonal antibody clearance. Impact of modulating the interaction of IgG with the neonatal Fc receptor. *J. Biol. Chem.,* 2007, vol. 282 (3), 1709-1717
- **CLARK, M.** Antibody Engineering IgG Effector Mechanisms. *Chemical Immunology,* 1997, vol. 65, 88-110
- **HORTON HM ; BERNETT MJ ; PONG E ; PEIPP M ; KARKI S ; CHU SY ; RICHARDS JO ; VOSTIAR I ; JOYCE PF ; REPP R.** Potent in vitro and in vivo activity of an Fc-engineered anti-CD 19 monoclonal antibody against lymphoma and leukemia. *Cancer Res.,* 2008, vol. 68, 8049-8057
- **ZALEVSKY J ; LEUNG IW ; KARKI S ; CHU SY ; ZHUKOVSKY EA ; DESJARLAIS JR ; CARMICHAEL DF ; LAWRENCE CE.** The impact of Fc engineering on an anti-CD19 antibody: increased Fcγ receptor affinity enhances B-cell clearing in nonhuman primates. *Blood,* 2009, vol. 113, 3735-3743
- **JEFFERIS R ; LUND J.** Interaction sites on human IgG-Fc for FcgammaR: current models. *Immunol. Lett.,* 2002, vol. 82, 57-65
- **CLYNES, R. ; YOSHIZUMI, T. ; MOROI, Y. ; HOUGHTON, A.N. ; RAVETCH, J.V.** Fc Receptors are required for passive and active immunity to melanoma. *Proc. Natl. Acad. Sci. U. S. A.,* 1998, vol. 95, 652-656
- **CLYNES RA ; TOWERS TL ; PRESTA LG ; RAVETCH JV.** Inhibitory Fc receptors modulate in vivo cytoxicity against tumor targets. *Nat. Med.,* 2000, vol. 6, 443-446
- **CARTRON G ; DACHEUX L ; SALLES G ; SOLAL-CELIGNY P ; BARDOS P ; COLOMBAT P ; WATIER H.** Therapeutic activity of humanized anti-CD20 monoclonal antibody and polymorphism in IgG Fc receptor FcgammaRIIIa gene. *Blood,* 2002, vol. 99, 754-758
- **NIMMERJAHN F ; RAVETCH JV.** Divergent immunoglobulin g subclass activity through selective Fc receptor binding. *Science,* 2005, vol. 310, 1510-1512
- **GREENWOOD J ; CLARK M ; WALDMANN H.** Structural motifs involved in human IgG antibody effector functions. *Eur. J. Immunol.,* 1993, vol. 23, 1098-1104
- **MORGAN A ; JONES ND ; NESBITT AM ; CHAPLIN L ; BODMER MW ; EMTAGE JS.** The N-terminal end of the CH2 domain of chimeric human IgG1 anti-HLA-DR is necessary for C1q, Fc gamma RI and Fc gamma RIII binding. *Immunology,* 1995, vol. 86, 319-324
- **LAZAR GA ; DANG W ; KARKI S ; VAFA O ; PENG JS ; HYUN L ; CHAN C ; CHUNG HS ; EIVAZI A ; YODER SC.** Engineered antibody Fc variants with enhanced effector function. *Proc. Nat. Acad. Sci. U. S. A.,* 2006, vol. 103, 4005-4010
- **SHINKAWA T ; NAKAMURA K ; YAMANE N ; SHOJI-HOSAKA E ; KANDA Y ; SAKURADA M ; UCHIDA K ; ANAZAWA H ; SATOH M ; YAMASAKI M.** The absence of fucose but not the presence of galactose or bisecting N-acetylglucosamine of human IgG1 complex-type oligosaccharides shows the critical role of enhancing antibody-dependent cellular cytotoxicity. *J. Biol. Chem.,* 2003, vol. 278, 3466-3473
- **KUNKEL et al.** *Proc. Natl. Acad. Sci. USA,* 1985, vol. 82, 488-492
- *Annu. Rev. Biophys. Biomol. Struct.,* 2006, vol. 35, 225-249
- *Proc. Natl. Acad. Sci. U.S.A.,* 2003, vol. 100 (11), 6353-6357
- *J. Immunol.,* 1994, vol. 152, 4958-4968
- *Br. J. Haematol.,* 2003, vol. 123 (5), 850-857
- Epitope Mapping Protocols in Methods in Molecular Biology. 1996, vol. 66
- **ED HARLOW ; DAVID LANE.** Antibodies: A Laboratory Manual. Cold Spring Harbor Laboratory, 1988, 359-420
- *Sequences of proteins of immunological interest*

- **MULLBERG et al.** *J. Immunol.,* 1994, vol. 152 (10), 4958-4968
- **SAMBROOK, J et al.** Molecular Cloning. Cold Spring Harbor Lab. Press, 1989, 9.47-9.58
- *J. Immunol.,* 1979, vol. 123 (4), 1548-1550
- *Current Topics in Microbiology and Immunology,* 1978, vol. 81, 1-7
- *C. Eur. J. Immunol.,* 1976, vol. 6 (7), 511-519
- *Cell,* 1976, vol. 8 (3), 405-415
- *Nature,* 1978, vol. 276 (5685), 269-270
- *J. Immunol. Methods,* 1980, vol. 35 (1-2), 1-21
- *J. Exp. Med.,* 1978, vol. 148 (1), 313-323
- *Nature,* 1979, vol. 277 (5692), 131-133
- **KOHLER ; MILSTEIN et al.** *Methods Enzymol.,* 1981, vol. 73, 3-46
- **VANDAMME et al.** *Eur. J. Biochem.,* 1990, vol. 192 (3), 767-775
- *Biochemistry,* 1979, vol. 18 (24), 5294-5299
- *Anal. Biochem.,* 1987, vol. 162 (1), 156-159
- *Proc. Natl. Acad. Sci. USA,* 1988, vol. 85 (23), 8998-9002
- *Nucleic Acids Res.,* 1989, vol. 17 (8), 2919-2932
- **EBERT, K. M. et al.** *Bio/Technology,* 1994, vol. 12 (7), 699-702
- *Cancer Res.,* 1993, vol. 53, 851-856
- **BERNASCONI et al.** *Science,* 2002, vol. 298, 2199-2202
- Sequences of Proteins of Immunological Interest. National Institute of Health, 1987
- **SPRINGER et al.** *Cell,* 2000, vol. 102, 275-277
- **KAWASAKI ; KRETSINGER.** *Protein Prof.,* 1995, vol. 2, 305-490
- **MONCRIEF et al.** *J. Mol. Evol.,* 1990, vol. 30, 522-562
- **CHAUVAUX et al.** *Biochem. J.,* 1990, vol. 265, 261-265
- **BAIROCH ; COX.** *FEBS Lett.,* 1990, vol. 269, 454-456
- **DAVIS.** *New Biol.,* 1990, vol. 2, 410-419
- **SCHAEFER et al.** *Genomics,* 1995, vol. 25, 638-643
- **ECONOMOU et al.** *EMBO J.,* 1990, vol. 9, 349-354
- **WURZBURG et al.** *Structure.,* 2006, vol. 14 (6), 1049-1058
- **TOMLINSON et al.** *J. Mol. Biol.,* 1992, vol. 227, 776-798
- **WILLIAMS ; WINTER.** *Eur. J. Immunol.,* 1993, vol. 23, 1456-1461
- **COX et al.** *Nat. Genetics,* 1994, vol. 7, 162-168
- **MATSUDA et al.** *J. Exp. Med.,* 1998, vol. 188, 1973-1975
- **KAWASAKI et al.** *Eur. J. Immunol.,* 2001, vol. 31, 1017-1028
- **SCHABLE ; ZACHAU.** *Biol. Chem. Hoppe Seyler,* 1993, vol. 374, 1001-1022
- **BRENSING-KUPPERS et al.** *Gene,* 1997, vol. 191, 173-181
- **KAWASAKI et al.** *Genome Res.,* 1997, vol. 7, 250-261
- **WU et al.** *J. Exp. Med.,* 1970, vol. 132, 211-250
- **KABAT.** Sequences of Proteins of Immunological Interest. National Institute of Health, 1987
- **LEE ; RICHARDS.** *J. Mol. Biol.,* 1971, vol. 55, 379-400
- **CONNOLLY.** *J. Appl. Cryst.,* 1983, vol. 16, 548-558
- **PACIOS.** *Comput. Chem.,* 1994, vol. 18 (4), 377-386
- *J. Mol. Model.,* 1995, vol. 1, 46-53
- **GEJIMA et al.** *Human Antibodies,* 2002, vol. 11, 121-129
- **CARDOSO et al.** *Scand. J. Immunol.,* 2000, vol. 51, 337-344
- *Bioorg. Med. Chem.,* 2003, vol. 11 (17), 3761-2768
- **HEINRICH et al.** *Biochem. J.,* 1998, vol. 334, 297-314
- **ISHIKAWA et al.** *J. Clin. Exp. Hematopathol.,* 2006, vol. 46 (2), 55-66
- *Proc. Natl. Acad. Sci. USA,* 2006, vol. 103 (11), 4005-4010
- **DAVIS et al.** *Immunological Reviews,* 2002, vol. 190, 123-136
- *Annu Rev Immunol.,* 2007, vol. 25, 525-60
- *Protein Eng Des Sel.,* March 2009, vol. 22 (3), 175-88
- *Protein Eng Des Sel.,* January 2008, vol. 21 (1), 1-10
- *J Immunol.,* 01 July 2002, vol. 169 (1), 137-44
- *FASEB J.,* February 2009, vol. 23 (2), 575-85
- *Curr. Opin. Biotechnol.,* 2009, vol. 20 (6), 685-91
- *Curr. Opin. Immunol.,* 2008, vol. 20 (4), 460-470
- *Protein Eng. Des. Sel.,* 2010, vol. 23 (4), 195-202
- **WEITZHANDLER et al.** *J. Pharma. Sciences,* 1994, vol. 83 (12), 1670-1675
- **SCHENK et al.** *J. Clin. Investigation,* 2001, vol. 108 (11), 1687-1695
- **BIGGE et al.** *Anal. Biochem.,* 1995, vol. 230 (2), 229-238
- *Nat. Rev. Immunol.,* 2008, vol. 8, 34-47
- *J. Clin. Immunol.,* 2005, vol. 25 (1), 1-18
- *Hum. Genet.,* 2005, vol. 117, 220-227
- *J. Immunol.,* 2004, vol. 172, 7192-7199
- *J. Immunol.,* 2004, vol. 172, 7186-7191
- *Nat. Med.,* 2005, vol. 11, 1056-1058
- *Hum. Mol. Genet.,* 2005, vol. 14, 2881-2892
- *Immunity,* 2000, vol. 13, 277-285

- *J. Exp. Med.,* 2002, vol. 195, 1167-1174
- *Immunogenetics,* 2000, vol. 51, 429-435
- *Int. Immunol.,* 1999, vol. 11, 1685-1691
- *Curr. Biol.,* 2000, vol. 10, 227-230
- *J. Immunol.,* 2002, vol. 169, 4340-4346
- *J. Immunol.,* 1994, vol. 152, 574-585
- *Science,* 1992, vol. 256, 1808-1812
- *Cell,* 1989, vol. 58, 317-327
- *EMBO J.,* 1994, vol. 13 (13), 2963-2969
- *J. Cell. Biol.,* 1992, vol. 116, 875-888
- *J. Cell. Biol.,* 1989, vol. 109, 3291-3302
- *Mol. Immunol.,* 2011, vol. 49, 329-337
- *Protein Engineering,* 1996, vol. 9 (3), 299-305
- **GHETIE.** *Immunol. Today,* 1997, vol. 18 (12), 592-598
- **RAGHAVAN et al.** *Immunity,* 1994, vol. 1, 303-315
- **YEUNG et al.** *J. Immunol.,* 2009, vol. 182, 7663-7671
- *Journal of Immunology,* 2009, vol. 182, 7663-7671
- *J. Immunotoxicol.,* 2005, vol. 3, 131-139
- *mAbs,* 2010, vol. 2 (5), 1-13
- *ARTHRITIS & RHEUMATISM,* 2006, vol. 54, 2387-2392
- *AAPS J.,* 2010, vol. 4, 646-657
- *Blood,* 2008, vol. 112 (10), 3959-64
- *Drug Discov Today,* 2006, vol. 11 (1-2), 81-88
- Human Protein Atlas
- *Int Immunol.,* 2001, vol. 13 (12), 1551-1559
- *Methods Mol Biol.,* 2010, vol. 602, 93-104
- *Clin Pharmacol.,* April 2008, vol. 48 (4), 406-417
- *Pharm Res.,* January 2006, vol. 23 (1), 95-103
- Methods Mol Biol. Jackson Laboratories, 2010, vol. 602, 93-104
- *J. Biol. Chem.,* 2003, vol. 278, 3466-3473
- *Science,* 2005, vol. 310 (5753), 1510-1512
- *Nat. Biotechnol,* 2010, vol. 28, 157-159
- *Drug. Metab. Dispos.,* 2010, vol. 38 (4), 600-605
- *J. Biol. Chem.,* 2006, vol. 281, 23514-23524
- *J. Immunol.,* 2006, vol. 176 (1), 346-356
- *Clin. Pharm. & Ther.,* 2011, vol. 89 (2), 283-290
- *Clin. Pharmacol. Ther.,* 2011, vol. 89 (2), 283-290
- Fcerlg mouse, Taconic. *Cell,* 1994, vol. 76, 519-529
- *Nature,* 1996, vol. 379 (6563), 346-349
- **SEUNG et al.** *Mol. Immunol.,* 2008, vol. 45, 3926-3933
- **GREENWOOD et al.** *Eur. J. Immunol.,* 1993, vol. 23 (5), 1098-1104
- **MEYER et al.** *J. Thromb. Haemost.,* 2009, vol. 7 (1), 171-181
- **ROBLES-CARRILLO et al.** *J. Immunol.,* 2010, vol. 185 (3), 1577-1583
- *Nature,* 2000, vol. 400, 267-273
- *J. Biol. Chem.,* 2011, vol. 276, 16469-16477
- *Proc. Natl. Acad. Sci. USA,* 2011, vol. 108, 12669-126674
- *J. Immunol.,* 2011, vol. 187, 3208-3217
- *Protein Science,* 1996, vol. 5, 2617-2622
- *J. Imunol.,* 2011, vol. 187, 3208-3217
- *J. Appl. Cryst.,* 2010, vol. 43, 186-190
- *Acta Cryst.,* 2010, vol. D66, 125-132
- *Acta Cryst.,* 2006, vol. D62, 72-82
- *J. Appl. Cryst.,* 2007, vol. 40, 658-674
- *Acta Cryst.,* 2011, vol. D67, 355-367
- *Acta Cryst.,* 2010, vol. D66, 486-501
- *J. Immunol.,* 2011, vol. 187 (6), 3208-3217
- *Cancer Res.,* 2007, vol. 67, 8882-8890
- *Protein Science,* 1995, vol. 4, 2411-2423
- *J. Immunol. Methods.,* 2008, vol. 332 (1-2), 2-9
- *J. Immunol. Methods.,* 2001, vol. 247 (1-2), 191-203
- *Biotechnol. Prog.,* 2002, vol. 18 (2), 212-20
- *Mol. Cell Proteomics,* 2003, vol. 2 (2), 61-9
- *Methods Mol. Biol.,* 2002, vol. 178, 133-145
- *J. Biol. Chem.,* 2008, vol. 283 (37), 25140-25149
- *J. Mol. Biol.,* 2000, vol. 296, 57-86
- *Proc. Natl. Acad. Sci.,* 2009, vol. 106 (48), 20216-20221
- *J. Pharm. Biomed. Anal.,* 2008, vol. 47 (1), 23-30
- **KABAT, E.A. et al.** Sequences of proteins of immunological interest. NIH PUBLICATION, 1991, vol. 91
- *Methods Mol Biol.,* 2002, vol. 178, 87-100
- *FEBS Letter 11483,* vol. 309 (1), 85-88
- **KABAT EA et al.** Sequence of Proteins of Immunological Interest. NIH, 1991
- *J. Immunol. Methods,* 2001, vol. 247 (1-2), 191-203
- **WARMERDAM et al.** *J. Exp. Med.,* 1990, vol. 172, 19-25
- **WU et al.** *J. Clin. Invest.,* 1997, vol. 100 (5), 1059-1070
- **ORY et al.** *J. Clin. Invest.,* 1989, vol. 84, 1688-1691
- Biacore T100 Software Handbook BR1006-48